# EUROPEAN PATENT SPECIFICATION

(11) **EP 1 773 874 B1**
(45) Date of publication and mention of the grant of the patent: **24.10.2012**
(21) Application number: 05778839.0
(22) Date of filing: 03.08.2005
(51) Int. Cl.: C07K 14/33

(54) **OPTIMIZING EXPRESSION OF ACTIVE BOTULINUM TOXIN TYPE A**
OPTIMIERUNG DER EXPRESSION VON AKTIVEM BOTULINUMTOXIN A
OPTIMISATION DE L'EXPRESSION DE TOXINE BOTULINIQUE ACTIVE DE TYPE A

(30) Priority: 04.08.2004 US 599121 P
(43) Date of publication of application: 18.04.2007
(73) Proprietor: ALLERGAN, INC., Irvine CA 92612 (US)
(72) Inventor: STEWARD, Iance, E., Irvine, CA 92614 (US); GILMORE, Marcella, A., Santa Ana, CA 92705 (US); FERNANDEZ-SALAS, Ester, E., Fullerton, CA 92831 (US); LI, Shengwen, Irvine, CA 92620 (US); MILLER, Ronald, G., Rancho Santa Margarita, CA 92688 (US); AOKI, Kei, Roger, Coto de Caza, CA 92679 (US)
(74) Representative: HOFFMANN EITLE
(86) International application number: PCT/US2005/027917
(87) International publication number: WO 2006/017749

(56) References cited:
- US-A1- 2003 009 025
- SMITH L A: "DEVELOPMENT OF RECOMBINANT VACCINES FOR BOTULINUM NEUROTOXIN" TOXICON, ELMSFORD, NY, US, vol. 36, no. 11, November 1998 (1998-11), pages 1539-1548, XP001156933 ISSN: 0041-0101
- SMITH LEONARD A ET AL: "Roads from vaccines to therapies." MOVEMENT DISORDERS : OFFICIAL JOURNAL OF THE MOVEMENT DISORDER SOCIETY. MAR 2004, vol. 19 Suppl 8, March 2004 (2004-03), pages S48-S52, XP002356279 ISSN: 0885-3185
- ZHANG LI ET AL: "Complete DNA sequences of the botulinum neurotoxin complex of Clostridium botulinum type A-Hall (Allergan) strain." GENE. 2 OCT 2003, vol. 315, 2 October 2003 (2003-10-02), pages 21-32, XP002361444 ISSN: 0378-1119 -& DATABASE EMBL [Online] 24 October 2003 (2003-10-24), "Clostridium botulinum neurotoxin BoNT gene, complete cds." XP002361452 retrieved from EBI accession no. EM_PRO:AF488749 Database accession no. AF488749

## Description

All GeneBank sequence listings cited this application, as identified by their GenBank accession numbers, are available from the National Center for Biotechnological Information.

The ability of Clostridial toxins, such as, e.g., Botulinum neurotoxins (BoNTs), like, BoNT/A, BoNT/B, BoNT/C1, BoNT/D, BoNT/E, BoNT/F and BoNT/G, and Tetanus neurotoxin (TeNT), to inhibit neuronal transmission are being exploited in a wide variety of therapeutic and cosmetic applications, see e.g., William J. Lipham, COSMETIC AND CLINICAL APPLICATIONS OF BOTULINUM TOXIN (Slack, Inc., 2004). As an example, BOTOX^{®} is currently approved in one or more countries for the following indications: achalasia, adult spasticity, anal fissure, back pain, blepharospasm, bruxism, cervical dystonia, essential tremor, glabellar lines or hyperkinetic facial lines, headache, hemifacial spasm, hyperactivity of bladder, hyperhidrosis, juvenile cerebral palsy, multiple sclerosis, myoclonic disorders, nasal labial lines, spasmodic dysphonia, strabismus and VII nerve disorder. In addition, BoNTs therapies are proposed for treating neuromuscular disorders, see *e.g*., Kei Roger Aoki et al., Method for Treating Neuromuscular Disorders and Conditions with Botulinum Toxin Types A and B, U.S. Patent No. 6,872,397 (Mar. 29, 2005); Rhett M. Schiffman, Methods for Treating Uterine Disorders, U.S. Patent Publication No. 2004/0175399 (Sep. 9, 2004); and Richard L. Barron, Methods for Treating Ulcers and Gastroesophageal Reflux Disease, U.S. Patent Publication No. 2004/0086531 (May. 7, 2004); and Kei Roger Aoki, et al., *Method for Treating Dystonia with Botulinum Toxin C to G,* U.S. Patent No. 6,319,505 (Nov. 20, 2001); eye disorders, see *e.g*., Eric R. First, Methods and Compositions for Treating Eye Disorders, U.S. Patent Publication No. 2004/0234532 (Nov. 25, 2004); Kei Roger Aoki et al., Botulinum Toxin Treatment for Blepharospasm, U.S. Patent Publication No. 2004/0151740 (Aug. 5, 2004); and Kei Roger Aoki et al., Botulinum Toxin Treatment for Strabismus, U.S. Patent Publication No. 2004/0126396 (Jul. 1, 2004); pain, see *e.g*., Kei Roger Aoki et al., Pain Treatment by Peripheral Administration of a Neurotoxin, U.S. Patent No. 6,869,610 (Mar. 22, 2005); Stephen Donovan, Clostridial Toxin Derivatives and Methods to Treat Pain, U.S. Patent No. 6,641,820 (Nov. 4, 2003); Kei Roger Aoki, et al., *Method for Treating Pain by Peripheral Administration of a Neurotoxin,* U.S. Patent No. 6,464,986 (Oct. 15, 2002); Kei Roger Aoki and Minglei Cui, Methods for Treating Pain, U.S. Patent No. 6,113,915 (Sep. 5, 2000); Martin Voet, Botulinum Toxin Therapy for Fibromyalgia, U.S. Patent Publication No. 2004/0062776 (Apr. 1, 2004); and Kei Roger Aoki et al., Botulinum Toxin Therapy for Lower Back Pain, U.S. Patent Publication No. 2004/0037852 (Feb. 26, 2004); muscle injuries, see *e.g.,* Gregory F. Brooks, *Methods for Treating Muscle Injuries,* U.S. Patent No. 6,423,319 (Jul. 23, 2002); headache, see *e.g.,* Martin Voet, Methods for Treating Sinus Headache, U.S. Patent No. 6,838,434 (Jan. 4, 2005); Kei Roger Aoki et al., Methods for Treating Tension Headache, U.S. Patent No. 6,776,992 (Aug. 17, 2004); and Kei Roger Aoki et al., Method for Treating Headache, U.S. Patent No. 6,458,365 (Oct. 1, 2002); cardiovascular diseases, see *e.g.,* Gregory F. Brooks and Stephen Donovan, Methods for Treating Cardiovascular Diseases with Botulinum Toxin, U.S. Patent No. 6,767,544 (Jul. 27, 2004); neurological disorders, see *e.g.,* Stephen Donovan, Parkinson's Disease Treatment, U.S. Patent No. 6,620,415 (Sep. 16, 2003); and Stephen Donovan, Method for Treating Parkinson's Disease with a Botulinum Toxin, U.S. Patent No. 6,306,403 (Oct. 23, 2001); neuropsychiatric disorders, see *e.g*., Stephen Donovan, Botulinum toxin therapy for neuropsychiatric disorders, U.S. Patent Publication No. 2004/0180061 (Sep. 16, 2004); and Steven Donovan, *Therapeutic Treatments for Neuropsychiatric Disorders,* U.S. Patent Publication No. 2003/0211121 (Nov. 13, 2003); endocrine disorders, see e.g., Stephen Donovan, Method for Treating Endocrine Disorders, U.S. Patent No. 6,827,931 (Dec. 7, 2004); Stephen Donovan, Method for Treating Thyroid Disorders with a Botulinum Toxin, U.S. Patent No. 6740321 (May. 25, 2004); Kei Roger Aoki et al., Method for Treating a Cholinergic Influenced Sweat Gland, U.S. Patent No. 6,683,049 (Jan. 27, 2004); Stephen Donovan, Neurotoxin Therapy for Diabetes, U.S. Patent No. 6,416,765 (Jul. 9, 2002); Stephen Donovan, Methods for Treating Diabetes, U.S. Patent No. 6,337,075 (Jan. 8, 2002); Stephen Donovan, Method for Treating a Pancreatic Disorder with a Neurotoxin, U.S. Patent No. 6,261,572 (Jul. 17, 2001); Stephen Donovan, Methods for Treating Pancreatic Disorders, U.S. Patent No. 6,143,306 (Nov. 7, 2000); cancers, see e.g., Stephen Donovan, Methods for Treating Bone Tumors, U.S. Patent No. 6,565,870 (May 20, 2003); Stephen Donovan, Method for Treating Cancer with a Neurotoxin to Improve Patient Function, U.S. Patent No. 6,368,605 (Apr. 9, 2002); Stephen Donovan, Method for Treating Cancer with a Neurotoxin, U.S. Patent No. 6,139,845 (Oct. 31, 2000); and Mitchell F. Brin and Stephen Donovan, Methods for treating diverse cancers, U.S. Patent Publication No. 2005/0031648 (Feb. 10, 2005); otic disorders, see *e.g.,* Stephen Donovan, Neurotoxin therapy for inner ear disorders, U.S. Patent No. 6358926 (Mar. 19, 2002); and Stephen Donovan, Method for Treating Otic Disorders, U.S. Patent No. 6265379 (Jul. 24, 2001); as well as other disorders, see *e.g.,* Stephen Donovan, Use of a Clostridial Toxin to Reduce Appetite, U.S. Patent Publication No. 2004/40253274 (Dec. 16, 2004); and Howard I. Katz and Andrew M. Blumenfeld, Botulinum Toxin Dental Therapies and Procedures, U.S. Patent Publication No. 2004/0115139 (Jun. 17, 2004); Kei Roger Aoki, et al., *Treatment of Neuromuscular Disorders and Conditions with Different Botulinum,* U.S. Patent Publication No. 2002/0010138 (Jan. 24, 2002); and Kei Roger Aoki, et al., *Use of Botulinum Toxins for Treating Various Disorders and Conditions and Associated Pain,* U.S. Patent Publication No. 2004/0013692 (Jan. 22, 2004). In addition, the expected use of BoNTs, such as, *e.g*., BoNT/A, BoNT/B. BoNT/C1, BoNT/D, and BoNT/E, BoNT/F and BoNT/G, in both therapeutic and cosmetic treatments of humans is anticipated to expand to an ever widening range of diseases and aliments that can benefit from the properties of these toxins.
Zhang et al., Gene, 2 October 2003, (315), pages 21-32 disclose the nucleic acid and amino acid sequence of the native ORF of BoNT/A.

The increasing use of BoNTs therapies in treating a wider range of human afflictions necessitates increasing the efficiency with which these toxins are produced. However, meeting the needs for this ever increasing demand for such BoNT treatments may become difficult. One outstanding problem is that methods previously described to express BoNTs using heterologous organisms have failed to achieve optimal levels of BoNTs in commercial quantities. This inefficiency is a problem not only because the amount of BoNTs anticipated for future therapies is increasing, but also because this inefficiency leads to higher overall production costs. Furthermore, this difficulty is exacerbated for BoNTs that require *in vitro* activation by an exogenous protease, such as, *e.g.*, BoNT/A and BoNT/G, since the loss of toxin associated with the activation procedure require even larger amounts of starting material. Therefore, the poor yields using previously described methods is a significant obstacle to the overall commercial production of these BoNTs and is thus a major problem since active forms of these toxins are needed for scientific, therapeutic and cosmetic applications.

### BRIEF DESCRIPTION OF THE DRAWINGS

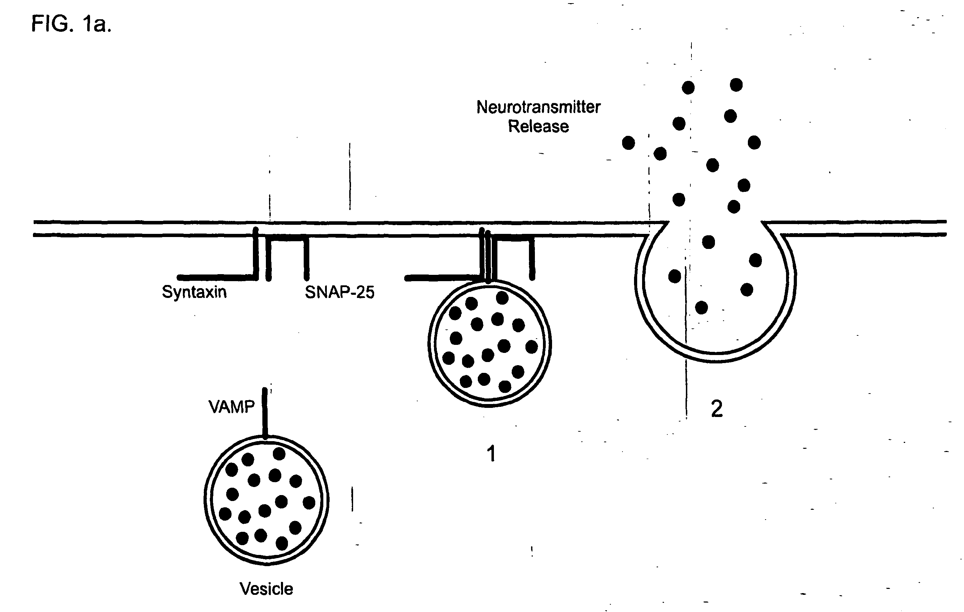

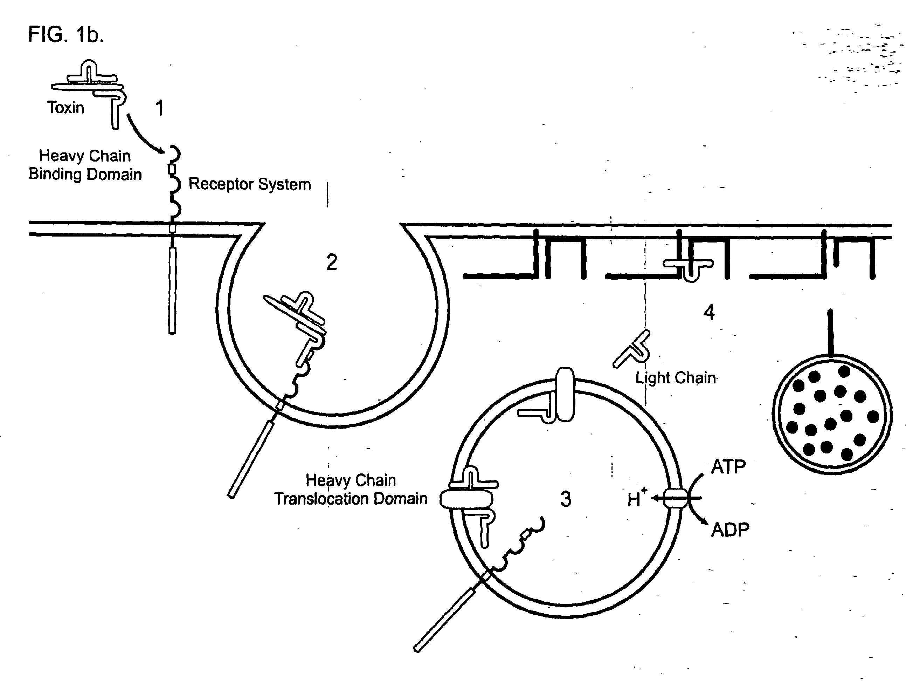

**FIG. 1** shows a schematic of the current paradigm of neurotransmitter release and Clostridial toxin intoxication in a central and peripheral neuron. FIG. 1a shows a schematic for the neurotransmitter release mechanism of a central and peripheral neuron. The release process can be described as comprising two steps: 1) vesicle docking, where the vesicle-bound SNARE protein of a vesicle containing neurotransmitter molecules associates with the membrane-bound SNARE proteins located at the plasma membrane; and 2) neurotransmitter release, where the vesicle fuses with the plasma membrane and the neurotransmitter molecules are exocytosed. FIG. 1b shows a schematic of the intoxication mechanism for tetanus and botulinum toxin activity in a central and peripheral neuron. This intoxication process can be described as comprising four steps: 1) receptor binding, where a Clostridial toxin binds to a Clostridial receptor system and initiates the intoxication process; 2) complex internalization, where after toxin binding, a vesicle containing the toxin/receptor system complex is endocytosed into the cell; 3) light chain translocation, where multiple events are thought to occur, including, *e.g*., changes in the internal pH of the vesicle, formation of a channel pore comprising the H_{N} domain of Clostridial toxin heavy chain, separation of the Clostridial toxin light chain from the heavy chain, and release of the activate light chain and 4) enzymatic target modification, where the activated light chain of Clostridial toxin proteolytically cleaves its target SNARE substrates, such as, *e.g*., SNAP-25, VAMP or Syntaxin, thereby preventing vesicle docking and neurotransmitter release.

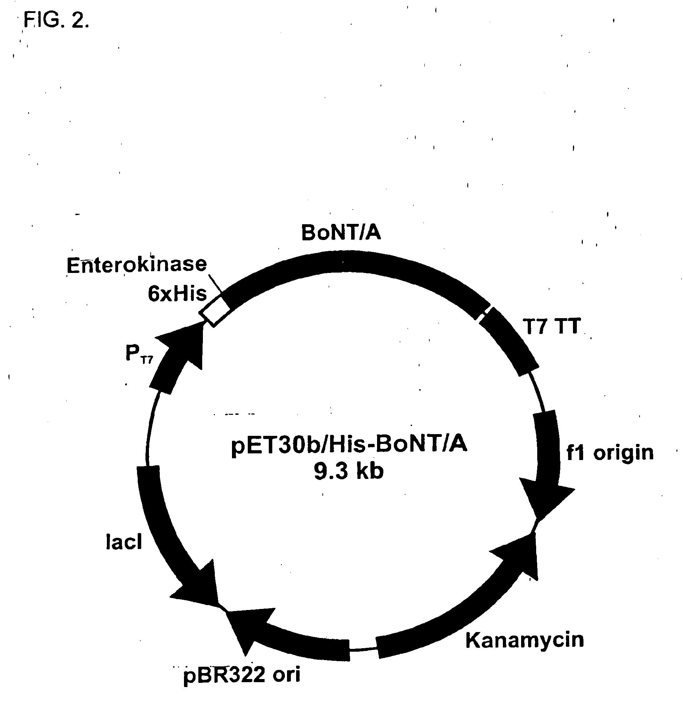

**FIG. 2** shows a plasmid map of prokaryotic expression construct pET30b/His-BoNT/A comprising the modified open reading frame of SEQ ID NO: 110 encoding an active BoNT/A operably-linked to an amino-terminal polyhistidine binding peptide (SEQ ID NO: 111). A Enterokinase protease cleavage site is operably-linked between the polyhistidine binding peptide and BoNT/A. Abbreviations are as follows: P_{T7}, a bacteriophage T7 promoter region; 6xHis, a region encoding a polyhistidine binding peptide sequence; Enterokinase, a region encoding a Enterokinase cleavage site; BoNT/A, a modified open reading frame encoding an active BoNT/A; T7 TT, a bacteriophage T7 transcription termination region; f1 origin, a bacteriophage f1 origin of replication; Kanamycin, a region encoding an aminophosphotransferase peptide that confers Kanamycin resistance; pBR322 ori, a pBR322 origin of plasmid replication region; lacl, a region encoding a lactose I peptide.

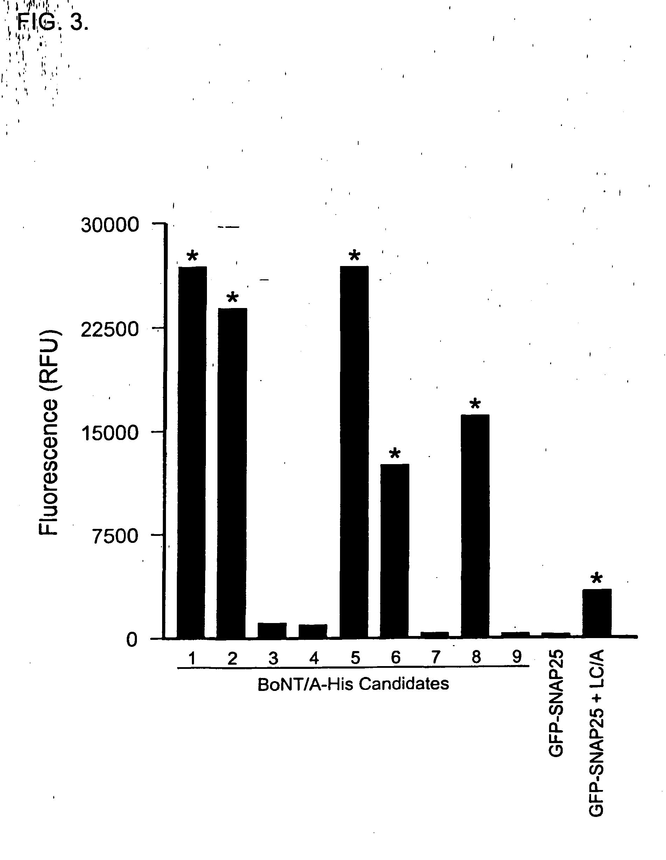

**FIG. 3** shows the results of a GFP-SNAP25 activity assay used to identify constructs expressing active His-BoNT/A. His-BoNT/A candidates 1, 2, 5, 6 and 8 showed statistically significant BoNT/A enzymatic activity.

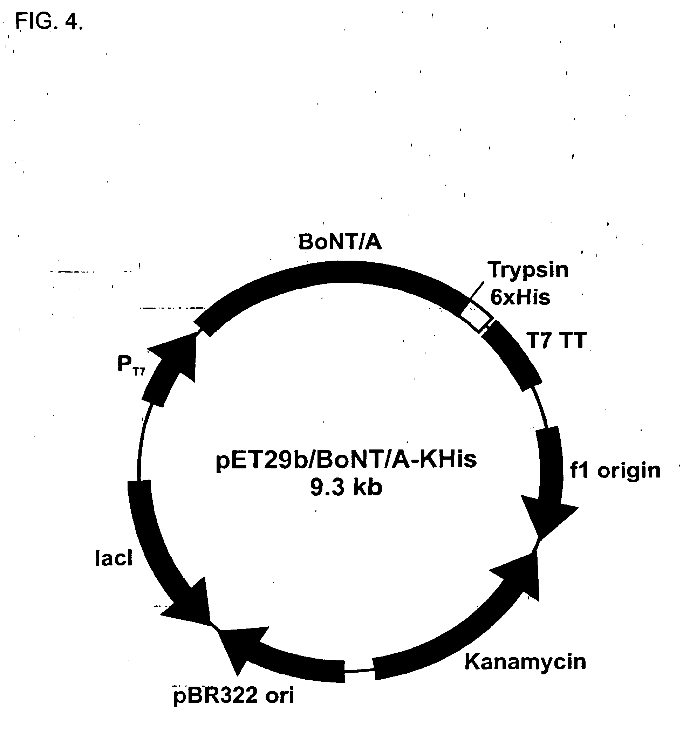

**FIG. 4** shows a plasmid map of prokaryotic expression construct pET29b/BoNT/A-KHis comprising the modified open reading frame of SEQ ID NO: 112 encoding an active BoNT/A operably-linked to a carboxyl-terminal polyhistidine binding peptide (SEQ ID NO: 113). A Trypsin protease cleavage site is operably-linked between the polyhistidine binding peptide and BoNT/A. Abbreviations are as follows: P_{T7}, a bacteriophage T7 promoter region; 6xHis, a region encoding a polyhistidine binding peptide sequence; Trypsin, a region encoding a Trypsin cleavage site; BoNT/A, a modified open reading frame encoding an active BoNT/A; T7 TT, a bacteriophage T7 transcription termination region; f1 origin, a bacteriophage f1 origin of replication; Kanamycin, a region encoding an aminophosphotransferase peptide that confers Kanamycin resistance; pBR322 ori, a pBR322 origin of plasmid replication region; lacl, a region encoding a lactose I peptide.

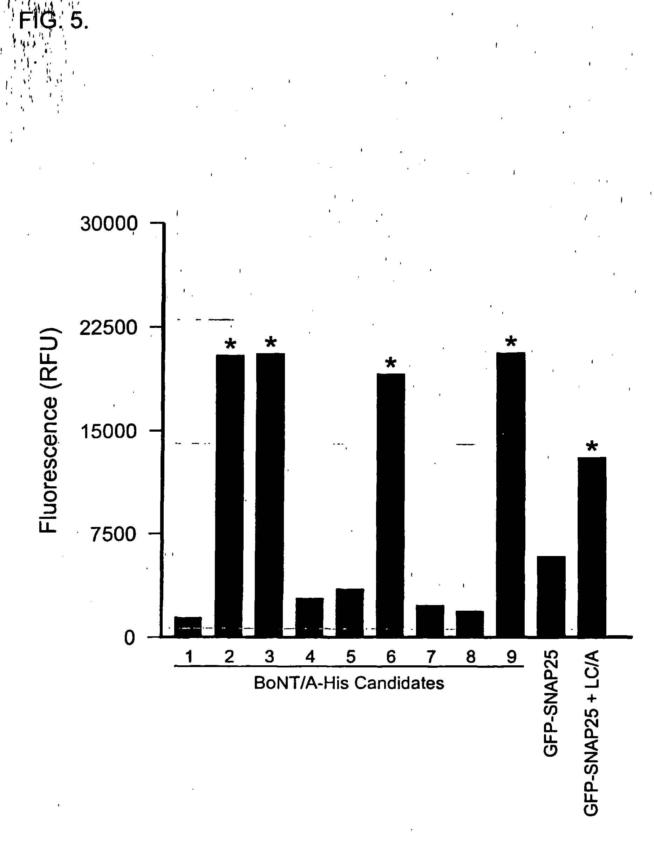

**FIG. 5** shows the results of a GFP-SNAP25 activity assay used to identify constructs expressing active BoNT/A-His. BoNT/A-His candidates 2, 3, 6 and 9 showed statistically significant BoNT/A enzymatic activity.

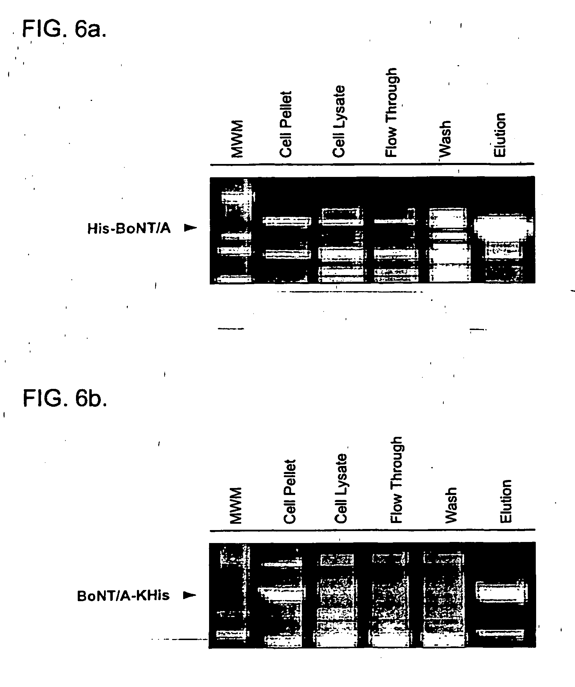

**FIG. 6** shows IMAC purified BoNT/A expressed from modified open reading frames. FIG 6a shows an IMAC purification profile of His-BoNT/A expressed from the pET30b/His-BoNTIA expression construct comprising the modified open reading frame of SEQ ID NO: 110. Amounts of His-BoNT/A obtained averaged approximately 5 mg/L and represents a five-fold increase in protein amounts obtained from an unmodified open reading frame encoding the same active His-BoNT/A. FIG 6b shows an IMAC purification profile of BoNT/A-KHis expressed from the pET29bBoNT/A-KHis expression construct comprising the modified open reading frame of SEQ ID NO: 112. Amounts of BoNT/A-KHis obtained averaged approximately 12 mg/L and represents a 12-fold increase in protein amounts obtained from an unmodified open reading frame encoding the same active BoNT/A-KHis.

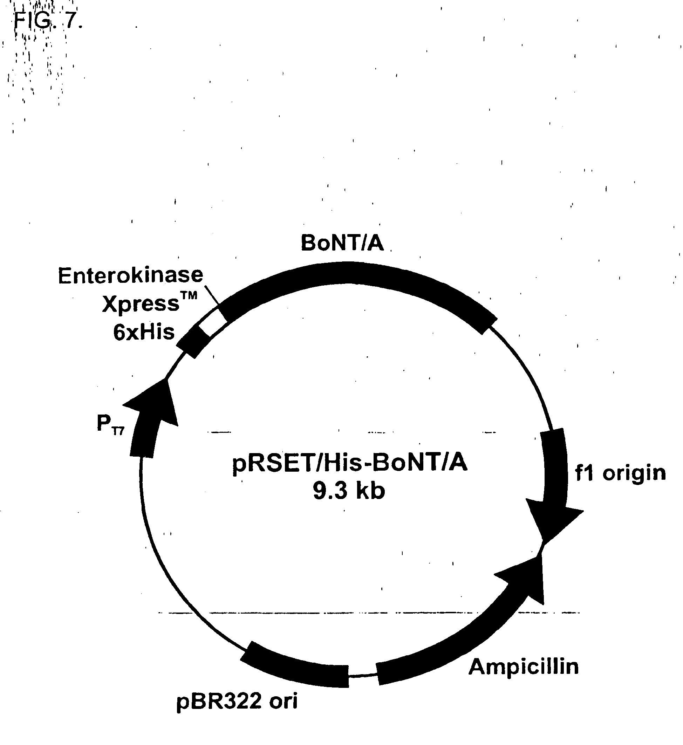

**FIG. 7** shows a plasmid map of prokaryotic expression construct pRSETb/His-BoNT/A comprising the modified open reading frame encoding an active BoNT/A operably-linked to amino-terminal polyhistidine and XpreSS™ binding peptides. An Enterokinase protease cleavage site is operably-linked between the polyhistidine and Xpress™ binding peptides and BoNT/A. Abbreviations are as follows: P_{T7} a bacteriophage T7 promoter region; 6xHis, a region encoding a polyhistidine binding peptide sequence; Xpress™, a region encoding an Xpress™ binding peptide sequence; Enterokinase, a region encoding a EnterokinaseMax™ cleavage site; BoNT/A, modified open reading frame of SEQ ID NO: 6 encoding an active BoNT/A; f1 origin, a bacteriophage f1 origin of replication; Ampicillin, a region encoding a β-lactamase peptide that confers Ampicillin resistance; pBR322 ori, a pBR322 origin of plasmid replication region.

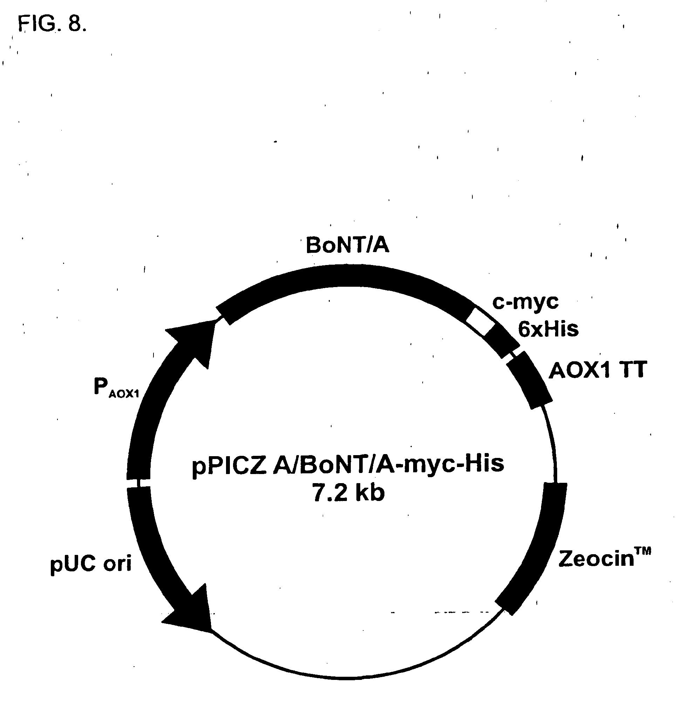

**FIG. 8** shows a plasmid map of yeast expression construct pPICZ A/BoNT/A-myc-His comprising a modified open reading frame encoding an active BoNT/A operably-linked to carboxyl-terminal c-myc and polyhistidine binding peptides. Abbreviations are as follows: P_{AOX1}, an aldehyde oxidase 1 promoter region; BoNT/A, modified open reading frame of SEQ ID NO: 36 encoding an active BoNT/A; c-myc, a region encoding a c-myc binding peptide sequence; 6xHis, a region encoding a polyhistidine binding peptide sequence; AOX1 TT, an aldehyde oxidase 1 transcription termination region; Zeocin™, a region encoding a Zeocin™ resistance peptide; pUC ori, a pUC origin of plasmid replication region.

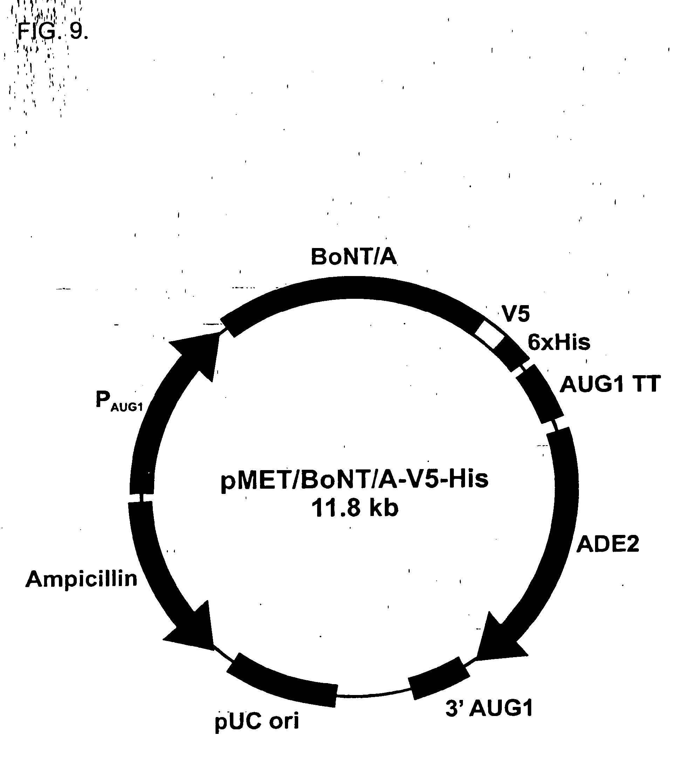

**FIG. 9** shows a plasmid map of yeast expression construct pMET/BoNT/A-V5-His comprising a modified open reading frame encoding an active BoNT/A operably-linked to carboxyl-terminal V5 and polyhistidine binding peptides. Abbreviations are as follows: P_{AUG1}, an alcohol oxidase promoter region; BoNT/A, modified open reading frame of SEQ ID NO: 36 encoding an active BoNT/A; V5, a region encoding a V5 binding peptide sequence; 6xHis, a region encoding a polyhistidine binding peptide sequence; AUG1 TT, an alcohol oxidase transcription termination region; ADE2; ADE2 gene for auxotrophic selection; 3' AUG1; pUC ori, a pUC origin of plasmid replication region; Ampicillin, a region encoding a β-lactamase peptide that confers Ampicillin resistance.

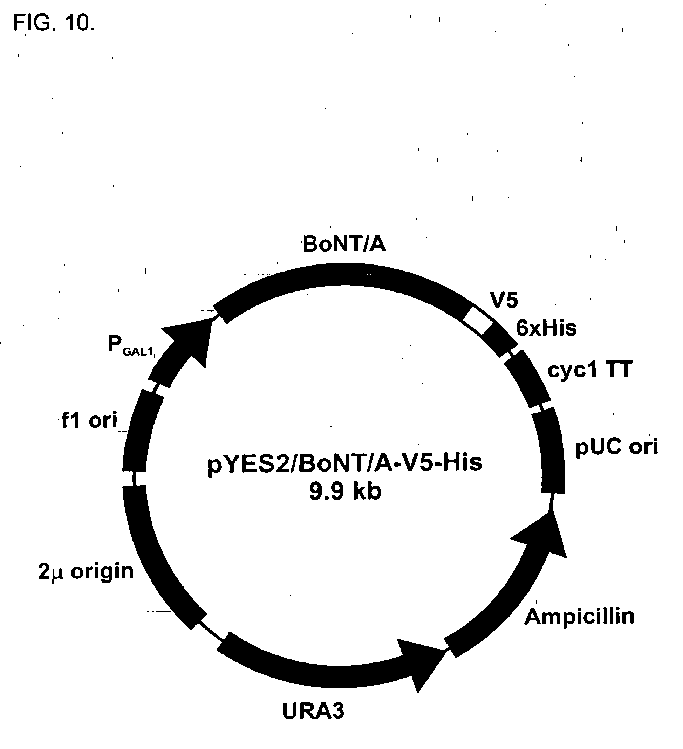

**FIG. 10** shows a plasmid map of yeast expression construct pYES2.1/BoNT/A-V5-His comprising a modified open reading frame encoding an active BoNT/A operably-linked to carboxyl-terminal V5 and polyhistidine binding peptides. Abbreviations are as follows: P_{GAL1}, an galactose-inducible promoter region; BoNT/A, modified open reading frame of SEQ ID NO: 39 encoding an active BoNT/A; V5, a region encoding a V5 binding peptide sequence; 6xHis, a region encoding a polyhistidine binding peptide sequence; cyc1 TT, an alcohol oxidase transcription termination region; pUC ori, a pUC origin of plasmid replication region; Ampicillin, a region encoding a β-lactamase peptide that confers Ampicillin resistance; URA3; URA3 gene for auxotrophic selection; 2µ origin of replication; a 2µ origin of replication; f1 origin, a bacteriophage f1 origin of replication.

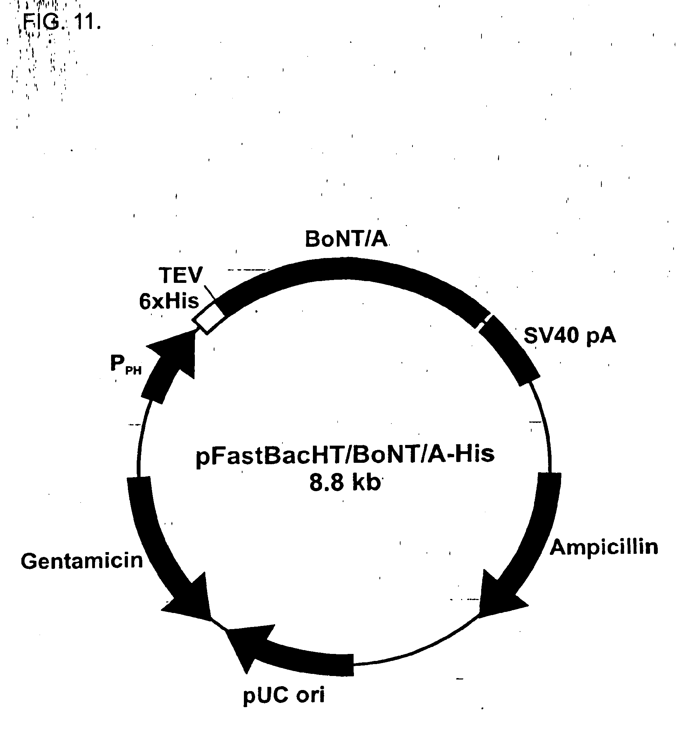

**FIG. 11** shows a plasmid map of baculovirus transfer construct pFastBacHT/His-BoNT/A comprising a modified open reading frame encoding an active BoNT/A operably-linked to amino-terminal polyhistidine binding peptide. A tobacco etch virus (TEV) protease cleavage site is operably-linked between the polyhistidine binding peptide and BoNT/A: Abbreviations are as follows: P_{PH}, an polyhedrin promoter region; 6xHis, a region encoding a polyhistidine binding peptide sequence; TEV, a region encoding a TEV protease cleavage sequence; BoNT/A, modified open reading frame of SEQ ID NO: 63 encoding an active BoNT/A; SV40 pA, a simian virus 40 polyadenylation site; Ampicillin, a region encoding a β-lactamase peptide that confers Ampicillin resistance; pUC ori, a pUC origin of plasmid replication region; Gentamicin, a region encoding an aminophosphotransferase peptide that confers Gentamicin resistance.

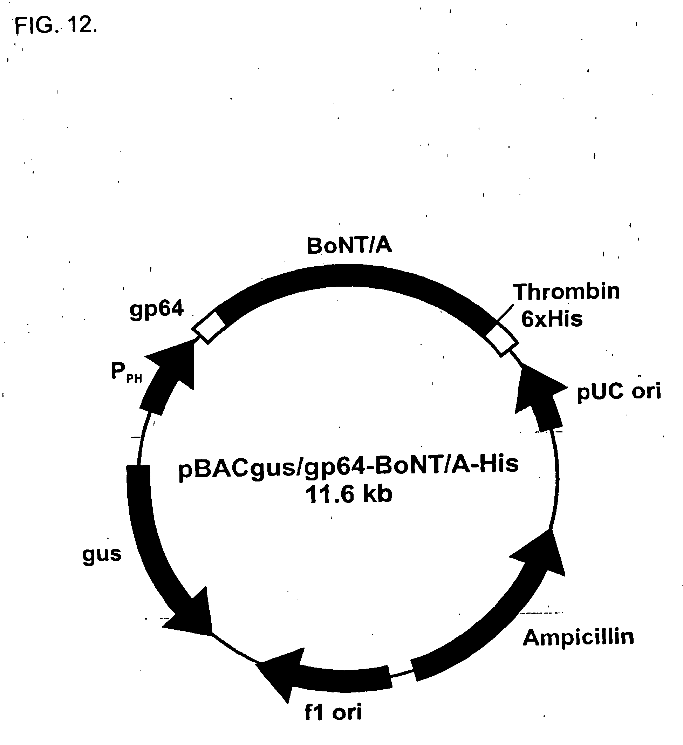

**FIG. 12** shows a plasmid map of baculovirus transfer construct pBACgus3/BoNT/A-His comprising a modified open reading frame encoding an active BoNT/A operably-linked to carboxyl-terminal polyhistidine binding peptide. A thrombin protease cleavage site is operably-linked between the BoNT/A and the polyhistidine binding peptide. Abbreviations are as follows: P_{PH}, an polyhedrin promoter region; gp64, a region encoding a gp64 signal peptide; BoNT/A, modified open reading frame of SEQ ID NO: 63 encoding an active BoNT/A; Thrombin, a region encoding a Thrombin protease cleavage sequence; 6xHis, a region encoding a polyhistidine binding peptide sequence; pUC ori, a pUC origin of plasmid replication region; Ampicillin, a region encoding a ß-lactamase peptide that confers Ampicillin resistance; f1 ori, a bacteriophage f1 origin of replication; gus, a region encoding a ß-glucuronidase peptide.

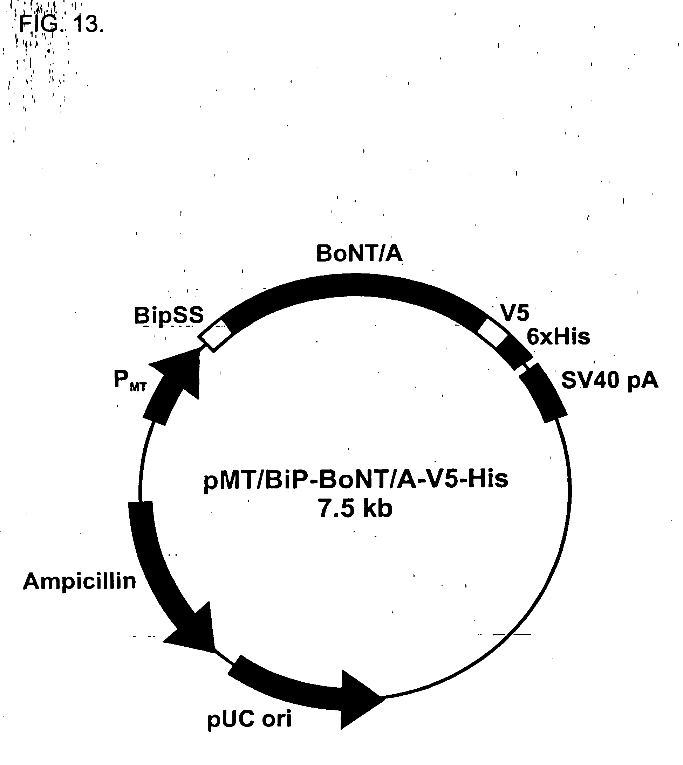

**FIG.13** shows a plasmid map of insect expression construct pMTBiP-BoNT/A-V5-His comprising a modified open reading frame encoding an active BoNT/A operably-linked to carboxyl-terminal V5 and polyhistidine binding peptides. Abbreviations are as follows: P_{MT}, an metallothionein promoter region; BipSS, a region encoding a BiP signal sequence; BoNT/A, modified open reading frame of SEQ ID NO: 60 encoding an active BoNT/A; V5, a region encoding a V5 binding peptide sequence; 6xHis, a region encoding a polyhistidine binding peptide sequence; SV40 pA, a simian virus 40 polyadenylation site; pUC ori, a pUC origin of plasmid replication region; Ampicillin, a region encoding a β-lactamase peptide that confers Ampicillin resistance.

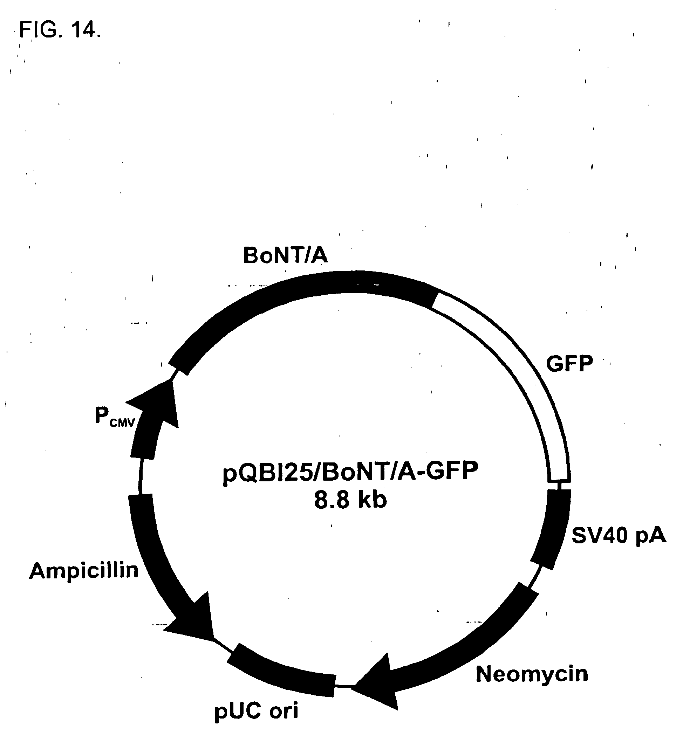

**FIG. 14** shows a plasmid map of mammalian expression construct pQBI25/BoNT/A-GFP comprising a modified open reading frame encoding an active BoNT/A operably-linked to a carboxyl-terminal GFP peptide. Abbreviations are as follows: P_{CMV}, an cytomegalovirus promoter region; BoNT/A, a modified open reading frame of SEQ ID NO: 99 encoding an active BoNT/A; GFP, a region encoding a Green Florescence Protein peptide; BGH pA, a bovine growth hormone polyadenylation site; Neomycin, a region encoding an aminophosphotransferase peptide that confers Neomycin resistance; pUC ori, a pUC origin of plasmid replication region; Ampicillin, a region encoding a β-lactamase peptide that confers Ampicillin resistance.

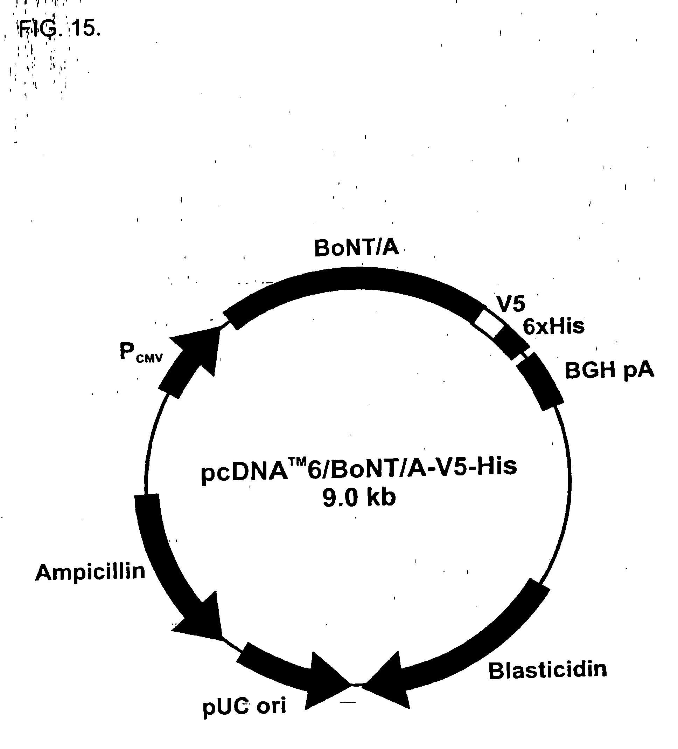

**FIG. 15** shows a plasmid map of mammalian expression construct pcDNA™6/BONT/A-V5-His comprising a modified open reading frame encoding an active BoNT/A operably-linked to carboxyl-terminal V5 and polyhistidine binding peptides. Abbreviations are as follows: P_{CMV}, an cytomegalovirus promoter region; BoNT/A, a modified open reading frame of SEQ ID NO: 99 encoding an active BoNT/A; V5, a region encoding a V5 binding peptide sequence; 6xHis, a region encoding a polyhistidine binding peptide sequence; BGH pA, a bovine growth hormone polyadenylation site; Blasticidin, a region encoding an blasticidin resistance peptide; pUC ori, a pUC origin of plasmid replication region; Ampicillin, a region encoding a β-lactamase peptide that confers Ampicillin resistance.

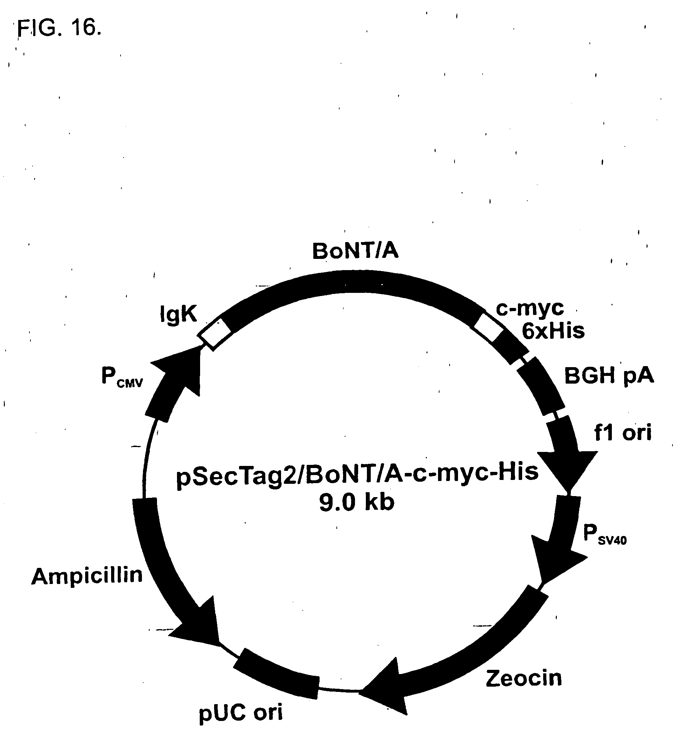

**FIG. 16** shows a plasmid map of mammalian expression construct pSecTag2/BoNT/A-c-myc-His comprising a modified open reading frame encoding an active BoNT/A operably-linked to carboxyl-terminal c-myc and polyhistidine binding peptides. Abbreviations are as follows: P_{CMV}, an cytomegalovirus promoter region; BoNT/A, a modified open reading frame of SEQ ID NO: 99 encoding an active BoNT/A; c-myc, a region encoding a c-myc binding peptide sequence; 6xHis, a region encoding a polyhistidine binding peptide sequence; BGH pA, a bovine growth hormone polyadenylation site; f1 ori, a bacteriophage f1 origin of replication; P_{SV40}, a simian virus 40 promoter region; Zeocin™, a region encoding an Zeocin™ resistance peptide; pUC ori, a pUC origin of plasmid replication region; Ampicillin, a region encoding a p-Iactamase peptide that confers Ampicillin resistance.

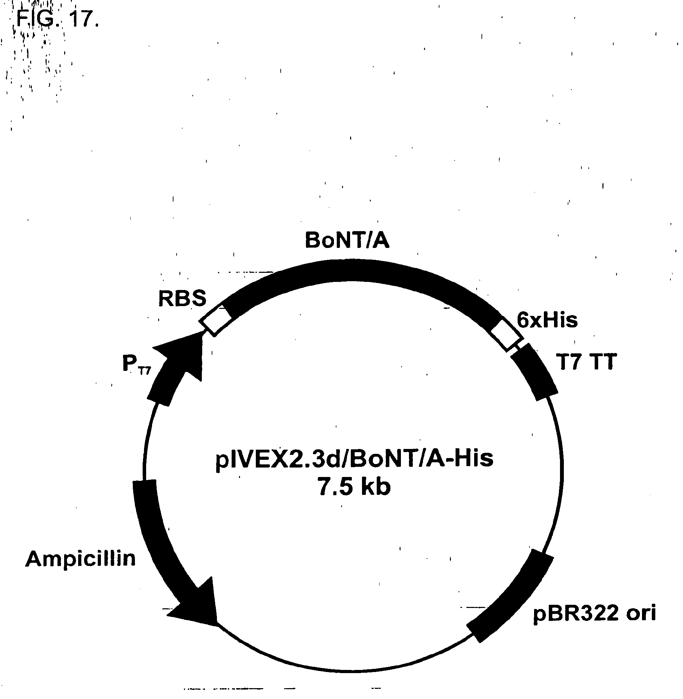

**FIG. 17** shows a plasmid map of cell-free expression construct pIVEX2.3d/BoNT/A-His comprising a modified open reading frame encoding an active BoNT/A operably-linked to a carboxyl-terminal polyhistidine binding peptide. Abbreviations are as follows: P_{T7}, a bacteriophage T7 promoter region; RBS, a ribosomal binding site region; BoNT/A, a modified open reading frame of SEQ ID NO: 3 encoding an active BoNT/A; 6xHis, a region encoding a polyhistidine binding peptide sequence; T7 TT, a bacteriophage T7 transcription termination region; pUC ori, a pUC origin of plasmid replication region; Ampicillin, a region encoding a,β-lactamase peptide that confers Ampicillin resistance.

### DETAILED DESCRIPTION

The present invention recognizes the need for the high-level, high quality commercial production of active Clostridial toxins' using heterologous organisms. In reference aspects, Clostridial toxins useful for scientific, therapeutic and cosmetic applications are envisioned including, without limitation, BoNTs, such as, *e.g*., BoNT/A, BoNT/B, BoNT/C, BoNT/D, BoNT/E, BoNT/F and BoNT/G, and TeNT. High-level production of a Clostridial toxin is achieved by using modified nucleic acid molecules which allows for increased expression of the encoded toxin in a heterologous cell and thus higher protein yields. In aspects of the present invention, nucleic acid molecules encoding a Clostridial toxin comprise modified open reading frames designed to 1) contain codons typically present in the open reading frames of native nucleic acid molecules found in the heterologous cell selected to express that molecule; In reference aspects, 2) contain a G+C content that more closely matches the average G+C content of open reading frames of native nucleic acid molecules found in the heterologous cell selected to express that molecule; 3) reduce polymononucleotide regions found within the open reading frame encoding an active Clostridial toxin; and/or 4) eliminate internal regulatory or structural sites found within the open reading frame encoding an active Clostridial toxin. Because a large number of production factors can influence the selection of a specific heterologous cell, nucleic acid molecules disclosed in the present specification in reference aspects are directed toward a wide range of prokaryotic and eukaryotic cell including, without limitation, bacteria strains, yeast strains, plant cells and cell lines derived from plants, insect cells and cell lines derived from insects and mammalian cells and cell lines derived from mammals. Aspects of the present invention also provide for expression constructs and cell compositions useful for expressing modified nucleic acid molecules disclosed in the present specification. In addition, aspects provide methods for producing Clostridial toxins using the disclosed nucleic acid molecules.

Aspects of the present invention provide nucleic acid molecules comprising a modified open reading frame providing increased expression of the encoded active BoNT/A as defined in claim 1. The modified open reading frame thus includes at least one nucleotide change as compared to the unmodifed open reading frame encoding the same active BoNT/A. Increased active BoNT/A expression from a modified open reading frame in a heterologous cell is determined by comparing the expression level from an unmodified open reading frame encoding the same active BoNT/A in the same type of heterologous cell. In is envisioned that, with the exception of the modified and unmodified open reading frames, the nucleic acid molecules comprising the open reading frames are similar or identical in nature. A nucleotide change may alter a synonymous codon within the open reading frame in order to agree with the endogenous codon usage found in the heterologous cell selected to express the molecule disclosed in the present specification. Additionally, a nucleotide change may alter the G+C content within the open reading frame to better match the average G+C content of open reading frames found in endogenous nucleic acid molecules present in the heterologous cell. A nucleotide change may also alter a polymononucleotide region or an internal regulatory or structural site found within the native nucleic acid molecule. A wide variety of modified nucleic acid molecules are envisioned in reference aspects including, without limitation, molecules comprising a modified open reading frame providing increased expression of the encoded active BoNT/A in a prokaryotic cell; molecules comprising a modified open reading frame providing increased expression of the encoded active BoNT/A in a yeast cell; molecules comprising a modified open reading frame providing increased expression of the encoded active BoNT/A in an insect cell; molecules comprising a modified open reading frame providing increased expression of the encoded active BONT/A in a mammalian cell; and molecules comprising a modified open reading frame providing increased expression of the encoded active BoNT/A in a cell-free extract expression system.

Other aspects of the present invention provide expression constructs comprising a nucleic acid molecule disclosed in the claims, operably-linked to an expression vector useful for expressing BoNT/A in a heterologous cell. A wide variety of expression vectors are envisioned, including, without limitation, a prokaryotic expression construct comprising a modified open reading frame providing increased expression of the encoded active BoNT/A; a yeast expression construct comprising a modified open reading frame providing increased expression of the encoded active BoNT/A; an insect expression construct comprising a modified open reading frame providing increased expression of the encoded active BoNT/A; a mammalian expression construct comprising a modified open reading frame providing increased expression of the encoded active BoNT/A; and an expression construct for a cell-free extract expression system comprising a modified open reading frame providing increased expression of the encoded active BoNT/A.

Aspects of the present invention further provide heterologous cells comprising an expression construct disclosed in the present specification. It is envisioned as defined in the claims that a cell can include, without limitation, a prokaryotic cell containing a prokaryotic expression construct comprising a modified open reading frame providing increased expression of the encoded active BoNT/A; a yeast cell containing a yeast expression construct comprising a modified open reading frame providing increased expression of the encoded active BoNT/A; an insect cell containing an insect expression construct comprising a modified open reading frame providing increased expression of the encoded active BoNT/A; and a mammalian cell containing a mammalian expression construct comprising a modified open reading frame providing increased expression of the encoded active BoNT/A;

Other aspects provide methods of producing an active BoNT/A comprising the step of expressing an active BoNT/A from a nucleic acid molecule in a heterologous cell, the nucleic acid molecule comprising a modified open reading frame encoding the active BoNT/A. Aspects of these methods use nucleic acid molecules, expression constructs and cells disclosed in the present specification. It is envisioned that both cell-free and cell-based expression systems can be used to produce an active BoNT/A disclosed in the present specification according to this method.

Aspects of the present invention provide, in part, nucleic acid molecules comprising a modified open reading frame encoding active BoNT/A in a heterologous cell as defined in the claims. As used herein, the term "open reading frame" is synonymous with "ORF" and means any nucleotide sequence that is potentially able to encode a protein, or a portion of a protein. An open reading frame usually begins with a start codon (represented as, *e.g.* AUG for an RNA molecule and ATG in a DNA molecule in the standard code) and is read in codon-triplets until the frame ends with a STOP codon (represented as, *e.g.* UAA, UGA or UAG for an RNA molecule and TAA, TGA or TAG in a DNA molecule in the standard code). As used herein, the term "codon" means a sequence of three nucleotides in a nucleic acid molecule that specifies a particular amino acid during protein synthesis; also called a triplet or codon-triplet. For example, of the 64 possible codons in the standard genetic code, two codons, GAA and GAG encode the amino acid Glutamine whereas the codons AAA and AAG specify the amino acid Lysine. In the standard genetic code three codons are stop codons, which do not specify an amino acid. As used herein, the term "synonymous codon" means any and all of the codons that code for a single amino acid. Except for Methionine (Met) and Tryptophan (Trp), amino acids are coded by two to six synonymous codons (see e.g., Table 1). For example, in the standard genetic code the four synonymous codons that code for the amino acid Alanine are GCA, GCC, GCG and GCU, the two synonymous codons that specify Glutamine are GAA and GAG and the two synonymous codons that encode Lysine are AAA and AAG (for other non-limiting examples see Table 1).

Thus in an embodiment, a modified open reading frame that encodes an active BoNT/A is changed by altering the nucleotide sequence of native *Clostridia botulinum* codons to better match the synonymous codons used by the heterologous cell selected to express nucleic acid molecules disclosed in the present specification. The *C. botulinum* strain that expresses BoNT/A exhibits a specific preference or bias for one synonymous codon over the others and there is a direct correlation between this *C*. *botulinum* strain-specific codon usage and the cellular concentration of the corresponding isoacceptor tRNA. This unequal presence of synonymous codons in a known or predicted open reading frame in an organism-, cell-, or functional class-specific manner is a phenomenon called codon bias or codon preference. Thus, it can be said that a heterologous cell has a bias for one synonymous codon over another synonymous codon, or that a heterologous cell prefers one synonymous codon over another synonymous codon. In addition, the synonymous codon to which the most abundant isoacceptor tRNA equates is often different between organisms, and, in some cases, between cells comprising different tissue types of the same organism, or between functional classes of proteins of the same organism, *e.g*., proteins expressed during exponential growth phase of a bacterium relative to proteins expressed during stationary growth phase of a bacterium. Different codon bias may also occur through the length of the open reading frame, such as, *e.g*., codons from the 5' third of the open reading frame may use different codons relative the remaining 3' two-thirds of the same open reading frame. For example, as mentioned above, GCA, GCC, GCG and GCU are the four synonymous codons that encode Alanine (Ala). While the most abundant Ala isoacceptor representative in *C. botulinum* recognizes the GCA codon, the bacterium *Escherichia coli* recognizes GCG, the yeast *Pichia pastoris* recognizes GCT and most multicellular eukaryotes appear to recognize GCC (see e.g., Table 1). Thus, certain codons that are normally used in the *Clostridia botulinum* strain that expresses BoNT/A may be rarely present in heterologous cells commonly used in the commercial expression of BoNT/A. Because these heterologous organisms do not produce the corresponding isoacceptor tRNAs at a concentration sufficient to support high-level BoNT/A expression, optimal protein yields are not achieved. Therefore, a modified open reading frame comprising nucleotides changes that increase the number of synonymous codons preferred by a heterologous cell will provide increased expression of the encoded active BoNT/A as compared to an unmodified open reading frame encoding the same active BoNT/A. A synonymous codon of the open reading frame can be changed by substituting a nucleotide at the third position of a codon with a different nucleotide, while still retaining the identity of the amino acid coded by that codon. As a non-limiting example, a 5'-AAATACTTA-3' open reading frame encoding the tripeptide NH₂-lysine-tyrosine-leucine-COOH can be changed to 5'-AAGTATCTG-3' and still encode the tripeptide NH₂-lysine-tyrosine-leucine-COOH.

Thus, in an aspect of this embodiment, at least one nucleotide change is made to a nucleic acid molecule that substitutes a codon in the open reading frame for a synonymous codon providing increased expression of the encoded active BoNT/A in a heterologous cell. In another aspect of this embodiment, a plurality of nucleotide changes are made to a nucleic acid molecule that substitutes a plurality of codons in the open reading frame for a plurality of synonymous codon providing increased expression of the encoded active BoNT/A in a heterologous cell. Thus, aspects of this embodiment can include a modified open reading frame comprising nucleotide changes that alter, *e.g*., at least 10 synonymous codons, at least 25 synonymous codons, at least 50 synonymous codons, at least 75 synonymous codons, at least 100 synonymous codons, at least 200 synonymous codons, at least 300 synonymous codons, at least. 400 synonymous codons, at least 500 synonymous codons, at least 600 synonymous codons, at least 700 synonymous codons, at least 800 synonymous codons, at least 900 synonymous codons, at least 1000 synonymous codons, at least 1100 synonymous codons or at least 1200 synonymous codons. In other aspects of this embodiment a modified open reading frame comprises nucleotide changes that alter, *e.g*., at most 10 synonymous codons, at most 25 synonymous codons, at most 50 synonymous codons, at most 75 synonymous codons, at most 100 synonymous codons, at most 200 synonymous codons, at most 300 synonymous codons, at most 400 synonymous codons, at most 500 synonymous codons, at most 600 synonymous codons, at most 700 synonymous codons, at most 800 synonymous codons, at most 900 synonymous codons, at most 1000 synonymous codons, at most 1100 synonymous codons or at most 1200 synonymous codons. The inventive embodiments are modified open reading frames selected from those comprising SEQ ID NO: 3,11 or 112.

In reference aspect, a modified open reading frame encoding an active BoNT/A is changed by altering the native *Clostridial botulinum* G+C content to better match the G+C content found in the heterologous cell selected to express nucleic acid molecules disclosed in the present specification. The average guanine and cytosine content (referred to as the G+C content) of the C. botulinum nucleic acid molecule comprising the open reading frame encoding BoNT/A is approximately 25%. This very low G+C content is in contrast to the approximately 50% G+C content of endogenous nucleic acid molecules encoding proteins found in heterologous cells commonly used in the commercial expression of BoNT/A (see e.g. Table 2). This unequal G+C content in a known or predicted open reading frame in an organism-specific manner is a phenomenon called G+C content bias or G+C content preference. Thus, it can be said that a heterologous cell has a bias for a certain G+C content level as compared to a different G+C content level, or that a heterologous cell prefers a certain G+C content level as compared to a different G+C content. The low G+C content of the open reading frame encoding BoNT/A conversely results in higher regions of adenine and thymidine content (A+T content). Higher A+T content appears to disrupt protein expression in a heterologous cell because these regions may, for example, mimic regulatory signals that could terminate transcriptional or translational expression, form secondary structures that could hinder transcriptional or translational read-through, or comprise repetitive sequences that could promote transcriptional or translational slippage. Thus, the average G+C content of the open reading frame can influence the expression levels of BoNT/A in a heterologous cell. Therefore, a modified open reading frame comprising nucleotide changes that increase the total G+C content to a level preferred by a heterologous cell will provide increased expression of the encoded active BoNT/A as compared to an unmodified open reading frame encoding the same active BoNT/A. The G+C content of the sequence can be increased by substituting an adenine or thymidine at the third position of a codon with a guanine or cytosine, while still retaining the same amino acid coded by that codon. As a non-limiting example, a 5'-AAATATTTA-3' region in frame with the open reading frame could be changed to 5'-AAGTACCTG-3' and still code for the tripeptide NH₂-lysine-tyrosine-leucine-COOH. Conversely, the G+C content of the sequence can be decreased by substituting a guanine or cytosine at the third position of a codon with an adenine or thymidine, while still retaining the same amino acid coded by that codon. As a non-limiting example, a 5'-AAGTACCTG-3' open reading frame encoding NH₂-lysine-tyrosine-leucine-COOH can be changed to 5'-AAATATTTA-3' and still encode the tripeptide NH₂-lysine-tyrosine-leucine-COOH.

Thus in an aspect thereof, at least one nucleotide change is made to a nucleic acid molecule that alters the G+C content of an open reading frame providing increased expression of the encoded active BoNT/A in a heterologous cell. In another aspect, a plurality of nucleotide substitutions are made to a nucleic acid molecule that alters the G+C content of an open reading frame providing increased expression of the encoded active BoNT/A in a heterologous cell. Therefore, aspects include a modified open reading frame comprising nucleotide changes that increase the total G+C content level to, *e.g.,* at least 30% total G+C content, at least 40% total G+C content, at least 50% total G+C content, at least 60% total G+C content or at least 70% total G+C content. Furthermore, such an open reading frame can include altering the total G+C content to any 50 consecutive nucleotides by, *e.g.,* at least 30% total G+C content, at least 40% total G+C content, at least 50% total G+C content, at least 60% total G+C content or at least 70% total G+C content. In other aspects, a modified open reading frame can include altering the total G+C content to any 75 consecutive nucleotides by, *e.g.,* at least 30% total G+C content, at least 40% total G+C content, at least 50% total G+C content, at least 60% total G+C content or at least 70% total G+C content. In yet other aspects, a modified open reading frame can include altering the total G+C content to any 100 consecutive nucleotides by, *e.g.,* at least 30% total G+C content, at least 40% total G+C content, at least 50% total G+C content, at least 60% total G+C content or at least 70% total G+C content.

Other aspects thereof include a modified open reading frame comprising nucleotide changes that increase the total G+C content level to, *e.g.,* at most 30% total G+C content, at most 40% total G+C content, at most 50% total G+C content, at most 60% total G+C content or at most 70% total G+C content. Furthermore, such an open reading frame can include altering the total G+C content to any 50 consecutive nucleotides by, *e.g.,* at most 30% total G+C content, at most 40% total G+C content, at most 50% total G+C content, at most 60% total G+C content or at most 70% total G+C content. In other aspects, a modified open reading frame can include altering the total G+C content to any 75 consecutive nucleotides by, *e.g.,* at most 30% total G+C content, at most 40% total G+C content, at most 50% total G+C content, at most 60% total G+C content or at most 70% total G+C content. In yet other aspects, a modified open reading frame can include altering the total G+C content to any 100 consecutive nucleotides by, *e.g.,* at most 30% total G+C content, at most 40% total G+C content, at most 50% total G+C content, at most 60% total G+C content or at most 70% total G+C content.

In another aspect, a modified open reading frame encoding an active BoNT/A is changed by altering a polymononucleotide region. Polymononucleotide regions (i.e., polyadenine, polyA; polythymidine, polyT; polyguanine, polyG; and polycytosine, polyC) can be detrimental to protein synthesis, especially if these regions are composed of five or more nucleotides. These regions can, for example, 1) contribute to translational staling which reduces the rate of protein synthesis as well as increase the numbers of incomplete/partial peptides synthesized; and 2) participate in translational skipping where the translational apparatus becomes misaligned with the open reading frame thereby producing aberrant proteins that are, *e.g.,* truncated or contain a different amino acid sequence due to a frame shift. A polymononucleotide region can be changed by substituting a nucleotide different from the one contained in the polymononucleotide region at the third position of a codon that interrupts the region while still maintaining the same amino acid coded by the codon. As a non-limiting example, a polyA region containing nine adenosines (*i.e*., 5'-AAAAAAAAA-3') encoding the tripeptide NH₂-lysine-lysine-lysine-COOH can be eliminated by changing the sequence to 5'-AAGAAGAAG-3' and still encode the tripeptide NH₂-lysine-lysine-lysine-COOH.

Thus in an aspect thereof, at least one nucleotide change may be made to a nucleic acid molecule that alters a polymononucleotide region found in an open reading frame providing increased expression of the encoded active BoNT/A. In another aspect a plurality of nucleotide changes are made to a nucleic acid molecule that alter a plurality of polymononucleotide regions in an open reading frame providing increased expression of the encoded active BoNT/A. In aspects an open reading frame can include, e.g., at least one nucleotide change, at least two nucleotide changes, at least three nucleotide changes, at least four nucleotide changes, at least five nucleotide changes, at least 10 nucleotide, at least 20 nucleotide, or at least 30 nucleotide changes. In other aspects an open reading frame can include, *e.g*., at most one nucleotide change, at most two nucleotide changes, at most three nucleotide changes, at most four nucleotide changes, at most five nucleotide changes, at most 10 nucleotide changes, at most 20 nucleotide changes, or at most 30 nucleotide changes.

In another aspect, a modified open reading frame is changed by altering the nucleotide sequence that alters an internal regulatory or structural site. Internal regulatory or structural sites, include, without limitation, internal or cryptic translational start sites, RNase cleavage sites, out-of-frame stop codons, methylation sites and hairpin-loop structures Internal translational start sites can misdirected the translational apparatus to an incorrect start site, thereby increasing the number of incomplete/partial or abnormal proteins synthesized. The presence of out-of-frame stop codons in the second and third reading frames of an open reading frame can increase translational efficiency and thus protein yields. For example, if the translational apparatus shifts to a reading frame not encoding the desired protein, time, resources and energy will be wasted translating defective proteins. The presence of out-of-frame stop codons reduces the cellular efforts expended in translating these aberrant peptides. RNases are enzymes that cleave RNA molecules, thereby destroying transcripts encoding a protein of interest and reducing yields. Hairpin-loop structures can physically block or disrupt the translational apparatus, thereby preventing protein synthesis or increasing the number of incomplete/partial or abnormal peptides synthesized. An internal regulatory or structural site can be changed by substituting a nucleotide different from the one contained in the consensus sequence, altering the nucleotide identity to the consensus sequence while still maintaining the same amino acid coded by the codon present in the in-frame reading frame.

In an aspect thereof a modified open reading frame is changed by altering the nucleotide sequence that alters an internal translational start site. An internal translational start site can be changed by substituting a nucleotide different from the one contained in the consensus sequence at the third position of a codon, reducing the nucleotide identity to the consensus sequence while still maintaining the same amino acid coded by the codon. As a non-limiting example, the typical translational start site in the insect *Drosophila melanogaster* is 5'-ACAACCAAAATG-3', and is present within an open reading frame would encode the peptide NH₂-threonine-threonine-lysine-methionine-COOH. This translational start site can be eliminated by changing the sequence to 5'-ACGACTAAGATG-3' and still encode the peptide NH₂-threonine-threonine-lysine-methionine-COOH. In another aspect, at least one nucleotide change may be made to a nucleic acid molecule altering the consensus sequence of an internal translational start site found in an open reading frame providing increased expression of the encoded active BoNT/A. In another aspect, a plurality of nucleotide changes are made to a nucleic acid molecule altering one or more internal translational start sites of an open reading frame providing increased expression of the encoded active BoNT/A. Therefore, aspects an open reading frame can include, *e.g.,* at least one nucleotide change, at least two nucleotide changes, at least three nucleotide changes, at least four nucleotide changes, at least five nucleotide changes or at least 10 nucleotide. In other aspects an open reading frame can include, *e.g.,* at most one nucleotide change, at most two nucleotide changes, at most three nucleotide changes, at most four nucleotide changes, at most five nucleotide changes, or at most 10 nucleotide changes.

In another aspect thereof, a modified open reading frame is changed by altering the nucleotide sequence that alters a RNase cleavage site. A RNase cleavage site can be changed by substituting a nucleotide different from the one contained in the consensus sequence at the third position of a codon, reducing the nucleotide identity to the consensus sequence while still maintaining the same amino acid coded by the codon. As a non-limiting example, the typical RNase E cleavage site is 5'-GGTAATTGC-3' is present within an open reading frame and encodes the peptide NH₂-glycine-isoleucine-cysteine-COOH. This RNase cleavage site can be eliminated by changing the sequence to 5'-GGCAACTGC-3' and still encode the peptide NH₂-threonine-threonine-lysine-methionine-COOH. In another aspect, at least one nucleotide change may be made to a nucleic acid molecule altering the consensus sequence of a RNase cleavage site found in an open reading frame providing increased expression of the encoded active BoNT/A. In another aspect, a plurality of nucleotide changes are made to a nucleic acid molecule altering one or more RNase cleavage sites of an open reading frame providing increased expression of the encoded active BoNT/A. Therefore, in aspects thereof an open reading frame can include, *e.g.,* at least one nucleotide change, at least two nucleotide changes, at least three nucleotide changes, at least four nucleotide changes, at least five nucleotide changes or at least 10 nucleotide changes. In other aspects of this embodiment an open reading frame can include, *e.g.,* at most one nucleotide change, at most two nucleotide changes, at most three nucleotide changes, at most four nucleotide changes, at most five nucleotide changes, or at most 10 nucleotide changes. A polymononucleotide region can be changed by substituting a nucleotide different from the one contained in the polymononucleotide region at the third position of a codon that interrupts the region while still maintaining the same amino acid coded by the codon.

In another aspect thereof, a modified open reading frame is changed by altering the nucleotide sequence to add a stop codon to an out-of-frame reading frame. A stop codon in an out-of-frame reading frame can be added by substituting a nucleotide different from the one contained at the third position of a codon which thereby creates a stop codon in an out-of-frame codon while still maintaining the same amino acid coded by the in-frame codon. As a non-limiting example, the in-frame open reading frame of the nucleotide sequence 5'-GGCAACTGC-3' encodes the peptide NH₂-glycine-isoleucine-cysteine-COOH. An out of frame stop codon can be added by changing the sequence to 5'-GGTAACTGC-3' (underlined sequence) and still encode the peptide NH₂-glycine-isoleucine-cysteine-COOH. In another aspect, at least one nucleotide change may be made to a nucleic acid molecule adding a stop codon to an out-of-frame reading frame providing increased expression of the encoded active BoNT/A. In another aspect, a plurality of nucleotide changes are made to a nucleic acid molecule adding one or more stop codons to an out-of-frame reading frame providing increased expression of the encoded active BoNT/A. Therefore, in aspects thereof an out of frame reading frame can include, *e.g.,* at least one nucleotide change, at least two nucleotide changes, at least three nucleotide changes, at least four nucleotide changes, at least five nucleotide changes, at least 10 nucleotide changes, at least 20 nucleotide changes, or at least 30 nucleotide changes. In other aspects an out of frame reading frame can include, *e.g.,* at most one nucleotide change, at most two nucleotide changes, at most three nucleotide changes, at most four nucleotide changes, at most five nucleotide changes, at most 10 nucleotide changes, at most 20 nucleotide changes, or at most 30 nucleotide changes.

In another aspect thereof, a modified open reading frame is changed by altering the nucleotide sequence that alters a hairpin-loop structure. A hairpin-loop structure can be changed by substituting a nucleotide different from the one contained in the consensus sequence at the third position of a codon, reducing the nucleotide identity to the consensus sequence while still maintaining the same amino acid coded by the codon. As a non-limiting example, the hairpin-loop structure 5'-GCTTGGCCAAGC-3' is present within an open reading frame and encodes the peptide NH₂-alanine-tryptophan-proline-serine-COOH. This hairpin-loop structure can be eliminated by changing the sequence to 5'-GCATGGCCTAGC-3' and still encode the peptide NH₂-alanine-tryptophan-proline-serine-COOH. In another aspect, at least one nucleotide change may be made to a nucleic acid molecule altering the consensus sequence of a hairpin-loop structure found in an open reading frame providing increased expression of the encoded active BoNT/A. In another aspect, a plurality of nucleotide changes are made to a nucleic acid molecule altering the consensus sequence of a hairpin-loop structure found in an open reading frame providing increased expression of the encoded active BoNT/A. Therefore, in aspects thereof an open reading frame can include, *e.g.,* at least one nucleotide change, at least two nucleotide changes, at least three nucleotide changes, at least four nucleotide changes, at least five nucleotide changes, at least 10 nucleotide changes, at least 20 nucleotide changes, or at least 30 nucleotide changes. In other aspects an open reading frame can include, *e.g.,* at most one nucleotide change, at most two nucleotide changes, at most three nucleotide changes, at most four nucleotide changes, at most five nucleotide changes, at most 10 nucleotide changes, at most 20 nucleotide changes, or at most 30 nucleotide changes.

In yet another embodiment, a modified open reading frame is changed, as compared to the open reading frame of SEQ ID NO: 2, altering synonymous codons, G+C content, polymononucleotide regions and internal regulatory or structural sites, or any combination thereof, providing increased expression of the encoded active BoNT/A. Inventive embodiments are described in the claims

In an aspect of this embodiment, at least one nucleotide change is made to a nucleic acid molecule that substitutes a codon in the open reading frame for a synonymous codon and alters the G+C content of an open reading frame providing increased expression of the encoded active BoNT/A. In another aspect of this embodiment, a plurality of nucleotide changes are made to a nucleic acid molecule that substitutes a plurality of codons in the open reading frame for a plurality of synonymous codons and alters the G+C content of an open reading frame providing increased expression of the encoded active BoNT/A. In another aspect of this embodiment, at least one nucleotide change is made to a nucleic acid molecule that substitutes a codon in the open reading frame for a synonymous codon and alters a polymononucleotide region found in an open reading frame providing increased expression of the encoded active BoNT/A. In another aspect of this embodiment, a plurality of nucleotide changes are made to a nucleic acid molecule that substitutes a plurality of codons in the open reading frame for a plurality of synonymous codon and alters a plurality of polymononucleotide region found in an open reading frame providing increased expression of the encoded active BoNT/A. In a further aspect of this embodiment, at least one nucleotide change is made to a nucleic acid molecule that substitutes a codon in the open reading frame for a synonymous codon and alters an internal regulatory or structural site found in an open reading frame providing increased expression of the encoded active BoNT/A. In another aspect of this embodiment, a plurality of nucleotide changes are made to a nucleic acid molecule that substitutes a plurality of codons in the open reading frame for a plurality of synonymous codons and alters a plurality of internal regulatory or structural sites found in an open reading frame providing increased expression of the encoded active BoNT/A.

In still another aspect of this embodiment, at least one nucleotide change is made to a nucleic acid molecule that substitutes a codon in the open reading frame for a synonymous codon, alters the G+C content of an open reading frame and alters a polymononucleotide region providing increased expression of the encoded active BoNT/A. In another aspect of this embodiment, a plurality of nucleotide changes are made to a nucleic acid molecule that substitutes a plurality of codons in the open reading frame for a plurality of synonymous codons, alters the G+C content of an open reading frame and alters a plurality of polymononucleotide region providing increased expression of the encoded active BoNT/A. In yet another aspect of this embodiment, at least one nucleotide change is made to a nucleic acid molecule that substitutes a codon in the open reading frame for a synonymous codon, alters the G+C content of an open reading frame and alters an internal regulatory or structural site providing increased expression of the encoded active BoNT/A. In another aspect of this embodiment, a plurality of nucleotide changes are made to a nucleic acid molecule that substitutes a plurality of codons in the open reading frame for a plurality of synonymous codons, alters the G+C content of an open reading frame and alters a plurality of internal regulatory or structural sites providing increased expression of the encoded active BoNT/A.

In an aspect of this embodiment, at least one nucleotide change is made to a nucleic acid molecule that substitutes a codon in the open reading frame for a synonymous codon, alters the G+C content of an open reading frame, alters a polymononucleotide region and alters an internal regulatory or structural site providing increased expression of the encoded active BoNT/A. In another aspect of this embodiment, a plurality of nucleotide changes are made to a nucleic acid molecule that substitutes a plurality of codons in the open reading frame for a plurality of synonymous codons, alters the G+C content of an open reading frame, alters a plurality of polymononucleotide region and alters a plurality of internal regulatory or structural sites providing increased expression of the encoded active BoNT/A.

The nucleic acid molecules disclosed in the present specification include the nucleic acid sequence molecules comprising SEQ ID NO: 3 SEQ ID NO: 110, and SEQ ID NO: 112

It is envisioned in reference aspects that any of a variety of additional nucleotide modifications can be done to assist in the making and using of a nucleic acid molecule and the active BoNT/A encoded by such molecules. In one embodiment, a nucleic acid molecule disclosed in the present specification can be modified to add at least one nucleotide sequence region comprising a restriction endonuclease binding site. In another aspect of this embodiment, a molecule disclosed in the present specification can include a plurality of restriction endonuclease binding sites. Therefore, aspects of this embodiment can include a nucleic acid molecule that includes a nucleic acid region comprising one or more restriction endonuclease binding sites, two or more restriction endonuclease sites, three or more restriction endonuclease sites, four or more restriction endonuclease sites, or five or more restriction endonuclease enzyme sites. It is envisioned that the location of a nucleic acid region comprising a restriction endonuclease binding site can be at the 5' end of a molecule, the 3' end of the molecule, within the molecule, or any combination thereof. In another aspect of this embodiment, regions comprising restriction endonuclease sites are added to both the 5' and 3' ends of the open reading frame contained in a nucleic, acid molecule. In another aspect of this embodiment, restriction endonuclease sites flank each end of an open reading frame encoding the BoNT/A of SELL ID NO: 1. It is envisioned that any of a wide variety of restriction endonuclease binding sites can be used with nucleic acid molecules disclosed in the present specification. The selection, making and use of restriction endonuclease binding sites are routine procedures well within the scope of one skilled in the art and from the teaching herein.

In another embodiment, nucleic acid molecules disclosed in the present specification can include at least one nucleotide change that eliminates a restriction endonuclease binding site from within an open reading frame. In another aspect of this embodiment, a molecule disclosed in the present specification can include a plurality of nucleotide substitutions that eliminate a restriction endonuclease binding site from within an open reading frame. Therefore, aspects of this embodiment can include a nucleic acid molecule that alters the recognition sequence of a restriction endonuclease binding site found within an open reading frame by one or more nucleotides, two or more nucleotides, three or more nucleotides, or four or more nucleotides. A restriction endonuclease binding site can be altered by substituting a nucleotide different from the one contained in the palindrome recognition sequence of that enzyme at the third position of a codon that interrupted the site while still maintaining the same amino acid coded by the codon. As a non-limiting example, an *Eco*RI recognition site of 5'-GAATTC-3', found in the open reading frame, encoding for the dipeptide NH₂-glutamate-phenylalanine-COOH can be changed to 5'-GAGTTC-3' to eliminate the *Eco*RI recognition site and still code for the dipeptide NH₂-glutamate-phenylalanine-COOH. In yet another aspect of this embodiment, a nucleic acid molecule disclosed in the present specification can include the elimination of at least one restriction endonuclease site from an open reading frame. In yet another aspect of this embodiment, a molecule disclosed in the present specification can include the elimination of a plurality of restriction endonuclease binding sites from an open reading frame. Thus, aspects of this embodiment can eliminate one or more restriction endonuclease binding sites from an open reading frame, two or more restriction endonuclease binding sites from an open reading frame, three or more restriction endonuclease binding sites from an open reading frame, or four or more restriction endonuclease binding sites from an open reading frame.

In yet another embodiment, nucleic acid molecules disclosed in the present specification can include at least one nucleic acid region encoding a binding peptide. Such a binding peptide is operably-linked in-frame to an open reading frame encoding a BoNT/A, as a fusion protein. In another aspect of this embodiment, a nucleic acid molecule disclosed in the present specification can include a plurality of nucleic acid regions encoding multiple operably-linked binding peptides. Therefore, aspects of this embodiment can include a nucleic acid molecule including a nucleic acid region encoding one or more operably-linked binding peptides, two or more operably-linked binding peptides, three or more operably-linked binding peptides, four or more operably-linked binding peptides, or five or more operably-linked binding peptides. In another aspect of this embodiment, nucleic acid regions comprising multiple binding peptides can encode multiple copies of the same binding peptide, different binding peptides, or any combination thereof. The location of a nucleic acid region encoding a binding peptide may be in various positions, including, without limitation, before the amino terminus of the BoNT/A, within the BoNT/A, or after the carboxyl terminus of the BoNT/A and a binding peptide. Examples of binding peptides that can be encoded by a nucleic acid region disclosed in the present specification include, without limitation, epitope-binding peptides such as FLAG, Express™, human Influenza virus hemagluttinin (HA), human p62^{c-MyC} protein (c-MYC), Vesicular Stomatitis Virus Glycoprotein (VSV-G), glycoprotein-D precursor of Herpes simplex virus (HSV), V5, and AU1; affinity-binding peptides such as polyhistidine (HIS), streptavidin binding peptide (strep), and biotin; and peptide-binding domains such as the glutathione binding domain of glutathione-S-transferase, the calmodulin binding domain of the calmodulin binding protein, the S-peptide binding domain and the maltose binding domain of the maltose binding protein. Non-limiting examples of specific protocols for selecting, making and using an appropriate binding peptide are described in, *e.g*., MOLECULAR CLONING A LABORATORY MANUAL (Joseph Sambrook & David W. Russell eds., Cold Spring Harbor Laboratory Press, 3rd ed. 2001); ANTIBODIES: A LABORATORY MANUAL (Edward Harlow & David Lane, eds., Cold Spring Harbor Laboratory Press, 2nd ed. 1998); and USING ANTIBODIES: A LABORATORY MANUAL: PORTABLE PROTOCOL NO. I (Edward Harlow & David Lane, Cold Spring Harbor Laboratory Press, 1998), which are hereby incorporated by reference. In addition, non-limiting examples of binding peptides as well as well-characterized reagents, conditions and protocols are readily available from commercial vendors that include, without limitation, BD Biosciences-Clontech, Palo Alto, CA; BD Biosciences Pharmingen, San Diego, CA; Invitrogen, Inc; Carlsbad, CA; QIAGEN, Inc., Valencia, CA; and Stratagene, La Jolla, CA. These protocols are routine procedures within the scope of one skilled in the art and from the teaching herein.

In yet another embodiment, a nucleic acid molecule disclosed in the present specification can include at least one nucleic acid region encoding a protease cleavage site. Such a protease cleavage site is operably-linked in-frame to an open reading frame encoding an active BoNT/A and a binding peptide as a fusion protein. In another aspect of this embodiment, a molecule disclosed in the present specification can comprise a plurality of nucleic acid regions encoding multiple protease cleavage sites. It is further envisioned that in a molecule containing two or more nucleic acid regions, these regions may encode the same protease cleavage sites or may encode for different protease cleavage sites. The location of the nucleic acid region encoding the cleavage site may be in various positions, including, without limitation, between a binding peptide and the amino terminus of the active BoNT/A or between the carboxyl terminus of the active BoNT/A and a binding peptide element. Examples of protease cleavage sites that can be encoded by a nucleic acid region disclosed in the present specification include, without limitation, an enterokinase cleavage site, a thrombin cleavage site, a Factor Xa cleavage site, a human rhinovirus 3C protease cleavage site, a tobacco etch virus (TEV) protease cleavage site, a dipeptidyl aminopeptidase cleavage site and a small ubiquitin-like modifier (SUMO)/ubiquitin-like protein-1(ULP-1) protease cleavage site. Non-limiting examples of protease cleavage site as well as well-characterized reagents, conditions and protocols are readily available from commercial vendors that include, without limitation, BD Biosciences-Clontech, Palo Alto, CA; BD Biosciences Pharmingen, San Diego, CA; Invitrogen, Inc, Carlsbad, CA; QIAGEN, Inc., Valencia, CA; and Stratagene, La Jolla, CA. The selection, making and use of an appropriate protease cleavage site are routine procedures within the scope of one skilled in the art and from the teaching herein.

It is envisioned that any of a variety of means can be used to identify appropriate nucleotides to change in order to make a modified open reading frame providing increased expression of an active BoNT/A. Appropriate nucleotide changes can be identified manually using published codon usage tables, see *e.g*., Codon Usage Database, *supra,* (2004), or codon usage tables developed by one skilled in the art. In addition, computer programs designed to assist in the selection of nucleotide changes. Non-limiting examples of such software include eCodonOpt, Gregory L. Moore and Costas D. Maranas, eCodonOpt: A Systematic Computational Framework for Optimizing Codon Usage in Directed Evolution Experiments, 30(11) Nucleic Acids Res. 2407-2416 (2002); DNA Works, see, *e.g.,* David M. Hoover and Jacek Lubkowski, DNAWorks: An Automated Method for Designing Oligonucleotides for PCR-Based Gene Synthesis, 30(10) Nucleic Acids Res. e43 (2002); DNA2.0, see, *e.g.,* Claes Gustafsson et al., Codon Bias and Heterologous Protein Expression, 22(7) Trends Biotechnol. 346-353 (2004); GeMS, see, *e.g.,* Sarah J. Kodumal et al., Total Synthesis of Long DNA Sequences: Synthesis of a Contiguous 32-Kb Polyketide Synthase Gene Cluster, 101 (44) Proc. Natl. Acad. Sci. U. S. A. 15573-15578 (2004); CAD PAM, see, *e.g.,* Lance Stewart and Alex B. Burgin, *supra,* 2005; and Gene Composer, see, *e.g.,* Lance Stewart and Alex B. Burgin, *supra,* 2005. In addition, publicly available internet sites useful for identifying codon bias are available, such as, Graphical Codon User Analyzer at gcua.schoedl.de, see, *e.g.,* Markus Fuhrmann et al., Monitoring Dynamic Expression of Nuclear Genes in Chlamydomonas Reinhardtii by Using a Synthetic Luciferase Reporter Gene, 55(6) Plant Mol. Biol. 869-881 (2004); and UpGene at URL address vectorcore.pitt.edu/upgene/upgene.html, see, *e.g.,* Wentao Gao et al., UpGene: Application of a Web-based DNA Codon Optimization Algorithm, 20 BIOTECHNOL. PROG. 443-448, (2004). Alternatively, a variety of commercial vendors provide nucleotide optimization services including, but not limited, to Aptagen, Inc. (Hemdon, VA); BlueHeron^{®} Biotechnology (Bothell, WA); deCODE Biostructures, Inc. (Bainbridge Island, WA); DNA 2.0 (Menlo Park, CA); Entelechon, GmbH. (Regensburg, Germany); Genscript Corp. (Piscataway, NJ); Modular Genetics, Inc. (Woburn, MA); and QIAGEN, Inc. (Valencia, CA). The identification of appropriate nucleotide changes to make in a modified open reading frame disclosed in the present specification is a routine procedure within the scope of one skilled in the art and from the teachings herein.

A variety of methods can be used to make a nucleic acid molecule comprising a modified open reading frame disclosed in the present specification, see, *e.g.,* Lance Stewart and Alex B. Burgin, *supra,* 2005. Non-limiting examples of methods include, oligonucleotide ligation methods, *in vivo* repair methods and PCR-based methods. The synthesis of nucleic acid molecules is a routine procedure within the scope of one skilled in the art and from the teachings herein.

Nucleic acid synthesis by sequential assembly of complementary oligonucleotides is a solid phase method involving the sequential hybridization of overlapping complementary oligonucleotides to a starting oligonucleotide that is chemically coupled to an insert support, see, *e.g*., Zdenek Hostomsky and Jiri Smrt, Solid-phase assembly of DNA duplexes from synthetic oligonucleotides, 18 Nucleic Acids Symp Ser. 241-244 (1987); and K L. Beattie and R. F. Fowler, Solid-phase gene assembly, 352(6335) Nature 548-549 (1991). In this oligonucleotide ligation method, oligonucleotide building blocks of approximately 30 nucleotides in length that correspond to the top and bottom strands of the entire gene are individually denatured and purified by denaturing polyacrylamide gel electrophoresis. These purified oligonucleotides are phosphorylated at the 5' end, divided into subgroups and then hybridized to form subassemblies on the solid-phase support. Sequential rounds of subassembly hybridizations to the solid-phase support extend the attached DNA molecule until the full-length gene is constructed.

Nucleic acid synthesis by the *Fok*I method utilizes the *E*. *coli in vivo* repair mechanism of DNA synthesis to construct a synthetic gene from oligonucleotides, see e.g., Wlodek Mandecki & Timothy J. Bolling, Foki Method of Gene Synthesis, 68(1) GENE 101-107, (1988), The method is based on the observation that large (approx. 100 bp long) inserts can be cloned into a plasmid using a technique of oligodeoxynucleotide (oligo)-directed double-strand break repair. The method involves transforming a denatured mixture of oligonucleotides of approximately 40 to 90 nucleotides in length and a linearized plasmid into *E. coli.* The oligonucleotides are designed with terminal sequences which contain a *Fok*I restriction endonuclease site and complement the ends of the linearized plasmid, which also has sites for *Fok*I*.* The nucleotide (nt) sequences are inserted between the two *Fok*I sites of the plasmid. *Fok*I is a class Ils endonuclease which makes a staggered double strand break at a site 9 and 13 nucleotides away from its recognition site. Upon cleavage of the plasmid DNA with *Fok*I, a restriction fragment is liberated that by design contains unique four nucleotide Fokl 5'-overhang sequences that can serve as cohesive ends for subsequent assembly of larger fragments of synthetic DNA until the gene of interest is constructed.

Nucleic acid synthesis by polymerase cycling assembly (PCA) or assembly PCR uses the polymerase chain reaction to construct a gene from oligonucleotides instead of methods involving the ligation of overlapping oligonucleotide, see *e.g.,* Patrick J. Dillon & Craig A. Rosen, A Rapid Method for the Construction of Synthetic Genes Using the Polymerase Chain Reaction, 9(3) BIOTECHNIQUES 298-300, (1990); and Willem P. Stemmer et al., Single-Step Assembly of a Gene and Entire Plasmid from Large Numbers of Oligodeoxyribonucleotides, 164(1) GENE 49-53, (1995). In this method, overlapping, complementary oligonucleotides of approximately 40 to 60 nucleotides in length that correspond to the top and bottom strands of the entire gene are pooled and subjected to multiple cycles of denaturation, renaturation and polymerization. The resulting PCR products are then subjected to PCR amplification using outside flanking primers containing restriction endonuclease sites that facilitate cloning of the final PCR product.

Alternatively, a variety of commercial vendors provide nucleic acid synthesis services through the use of high throughput gene synthesis platforms including, but not limited, to Aptagen, Inc. (Hemdon, VA); BlueHeron^{®} Biotechnology (Bothell, WA); DNA 2.0 (Menlo Park, CA); Entelechon, GmbH. (Regensburg, Germany); Genscript Corp. (Piscataway, NJ); Modular Genetics, Inc. (Woburn, MA); and QIAGEN, Inc. (Valencia, CA). A method of nucleic acid synthesis is illustrated in Example 2. The synthesis of a modified open reading frame disclosed in the present specification is a routine procedure within the scope of one skilled in the art and from the teachings herein.

Seven antigenically-distinct types of Botulinum toxins (BoNTs) have been identified by investigating botulism outbreaks in man (BoNT/A, /B, /E and /F), animals (BoNT/C1 and /D), or isolated from soil (BoNT/G). BoNTs possess approximately 35% amino acid identity with each other and share the same functional domain organization and overall structural architecture. The amino acid sequences of eight Clostridial toxin serotypes have been derived from the corresponding genes, see, *e.g*., Niemann, Molecular Biology of Clostridial Neurotoxins, 303-348 (Sourcebook of Bacterial Protein Toxins, Alouf and Freer, Eds. Academic Press, 1991). It is recognized by those of skill in the art that within each type of Clostridial toxin there can be subtypes that differ somewhat in their amino acid sequence, and also in the nucleic acids encoding these proteins. For example, there are presently four BoNT/A subtypes, BoNT/A1, BoNT/A2, BoNT/A3 and BoNT/A4, with specific subtypes showing approximately 89% amino acid identity when compared to another BoNT/A subtype. While all seven BoNT serotypes have similar structure and pharmacological properties, each also displays heterogeneous bacteriological characteristics. In contrast, tetanus toxin (TeNT) is produced by a uniform group of *C. tetani.* Two other species of clostridia, *C. baratii* and *C. butyricum,* also produce toxins similar to BoNT/F and BoNT/E, respectively.

Clostridia toxins (CoNTs) are each translated as a single chain polypeptide of approximately 150 kDa that is subsequently cleaved by proteolytic scission within a disulphide loop by bacterial or tissue proteases. This posttranslational processing yields a di-chain molecule comprising an approximately 50 kDa light chain (LC) and an approximately 100 kDa heavy chain (HC) held together by a single disulphide bond and noncovalent interactions. Each mature di-chain molecule comprises three functionally distinct domains: 1) an enzymatic domain located in the LC that includes a metalloprotease region containing a zinc-dependent endopeptidase activity which specifically targets core components of the neurotransmitter release apparatus; 2) a translocation domain contained within the amino-terminal half of the HC (H_{N}) that facilitates release of the toxin from intracellular vesicles into the cytoplasm of the target cell; and 3) a binding domain found within the carboxyl-terminal half of the HC (H_{C}) that determines the binding activity and binding specificity of the toxin to the receptor complex located at the surface of the target cell.

The binding, translocation and enzymatic activity of these three functional domains are all necessary for toxicity. While all details of this process are not yet precisely known, the overall cellular intoxication mechanism whereby CoNTs enter a neuron and inhibit neurotransmitter release is similar, regardless of type. Although the applicants have no wish to be limited by the following description, the intoxication mechanism can be described as comprising at least four steps: 1) receptor binding, 2) complex internalization, 3) light chain translocation, and 4) enzymatic target modification (see FIG. 1). The process is initiated when the H_{C} domain of a CoNT binds to CoNT-specific receptor complex located on the plasma membrane surface of a target cell. The binding specificity of a receptor complex is thought to be achieved, in part, by specific combinations of gangliosides and protein receptors that appear to distinctly comprise each Clostridial toxin receptor complex. Once bound, the CoNT/receptor complexes are internalized by endocytosis and the internalized vesicles are sorted to specific intracellular routes. The translocation step appears to be triggered by the acidification of the vesicle compartment. This process seems to initiate two important pH-dependent structural rearrangements that increase hydrophobicity and promote enzymatic activation of the toxin. Once activated, light chain endopeptidase of the toxin is released from the intracellular vesicle into the cytosol where it specifically targets one of three known core components of the neurotransmitter release apparatus. These core proteins, vesicle-associated membrane protein (VAMP)/synaptobrevin, synaptosomal-associated protein of 25 kDa (SNAP-25) and Syntaxin, are necessary for synaptic vesicle docking and fusion at the nerve terminal and constitute members of the soluble *N*-ethylmaleimide-sensitive factor-attachment protein-receptor (SNARE) family. BoNT/A and BoNT/E cleave SNAP-25 in the carboxyl-terminal region, releasing a nine or twenty-six amino acid segment, respectively, and BoNT/C1 also cleaves SNAP-25 near the carboxyl-terminus. The botulinum serotypes BoNT/B, BoNT/D, BoNT/F and BoNT/G, and tetanus toxin, act on the conserved central portion of VAMP, and release the amino-terminal portion of VAMP into the cytosol. BoNT/C1 cleaves syntaxin at a single site near the cytosolic membrane surface. The selective proteolysis of synaptic SNAREs accounts for the block of neurotransmitter release caused by Clostridial toxins *in vivo.* The SNARE protein targets of Clostridial toxins are common to exocytosis in a variety of non-neuronal types; in these cells, as in neurons, light chain peptidase activity inhibits exocytosis, see, *e.g.,* Yann Humeau et al., How Botulinum and Tetanus Neurotoxins Block Neurotransmitter Release, 82(5) Biochimie. 427-446 (2000); Kathryn Turton et al., Botulinum and Tetanus Neurotoxins: Structure, Function and Therapeutic Utility, 27(11) Trends Biochem. Sci. 552-558. (2002); M. Zouhair Atassi, Basic and Therapeutic Aspects of Botulinum and Tetanus Toxins, (Dirk W. Dressler & Joseph J. Jankovic eds., 2003); Giovanna Lalli et al., The Journey of Tetanus and Botulinum Neurotoxins in Neurons, 11 (9) Trends Microbiol. 431-437, (2003) which are hereby incorporated by reference.

Aspects of the present invention provide, in part, an active BoNT/A. As used herein, the term "active BoNT/A" means any protein, or fragment thereof, that can execute the overall cellular mechanism whereby BoNT/A enters a neuron and inhibits neurotransmitter release and encompasses the binding of a BoNT/A to a low or high affinity receptor complex, the internalization of the toxin/receptor complex, the translocation of the BoNT/A light chain into the cytoplasm and the enzymatic modification of a BoNT/A substrate. Thus, active BoNT/A encompass without limitation, naturally occurring active BoNT/A variants, such as, *e.g*., active BoNT/A isoforms and BoNT/A subtypes; non-naturally occurring active BoNT/A variants, such as, *e.g*., conservative BoNT/A variants, non-conservative BoNT/A variants, BoNT/A chimeric variants and active BoNT/A fragments thereof, or any combination thereof. As used herein, the term "BoNT/A variant," whether naturally-occurring or non-naturally-occurring, means an active BoNT/A that has at least one amino acid change from the corresponding region of SEQ ID NO: 1 and can be described in percent identity to the corresponding region of SEQ ID NO: 1. As a non-limiting example, an active BoNT/A variant comprising amino acids 1-1296 of SEQ ID NO: 1 will have at least one amino acid difference, such as, *e.g*., an amino acid substitution, deletion or addition, as compared to the amino acid region 1-1296 of SEQ ID NO: 1. As another non-limiting example, an BoNT/A variant comprising amino acids 15-1290 of SEQ ID NO: 1 will have at least one amino acid difference, such as, *e.g*., an amino acid substitution, deletion or addition, as compared to the amino acid region 15-1290 of SEQ ID NO: 1.

Any of a variety of sequence alignment methods can be used to determine percent identity, including, without limitation, global methods, local methods and hybrid methods, such as, *e.g*., segment approach methods. Protocols to determine percent identity are routine procedures within the scope of one skilled in the art and from the teaching herein.

Global methods align sequences from the beginning to the end of the molecule and determine the best alignment by adding up scores of individual residue pairs and by imposing gap penalties. Non-limiting methods include, *e.g*., CLUSTAL W, see, *e.g*., Julie D. Thompson et al., CLUSTAL W: improving the sensitivity of progressive multiple sequence alignment through sequence weighting, position-specific gap penalties and weight matrix choice, 22(22) Nucleic Acids Research 4673-4680 (1994); and iterative refinement, see, *e.g*., Osamu Gotoh, Significant improvement in accuracy of multiple protein sequence alignments by iterative refinement as assessed by reference to structural alignments, 264(4) J. Mol. Biol. 823-838 (1996).

Local methods align sequences by identifying one or more conserved motifs shared by all of the input sequences. Non-limiting methods include, *e.g*., Match-box, see, *e.g*., Eric Depiereux and Ernest Feytmans, Match-box: a fundamentally new algorithm for the simultaneous alignment of several protein sequences, 8(5) CABIOS 501-509 (1992); Gibbs sampling, see, *e.g*., C. E. Lawrence et al., Detecting subtle sequence signals: a gibbs sampling strategy for multiple alignment, 262(5131) Science 208-214 (1993); Align-M, see, *e.g*., Ivo Van Walle et al., Align-m - a new algorithm for multiple alignment of highly divergent sequences, 20(9) Bioinformatics,:1428-1435 (2004).

Hybrid methods combine functional aspects of both global and local alignment methods. Non-limiting methods include, *e.g*., segment-to-segment comparison, see, *e.g*., Burkhard Morgenstern et al., Multiple DNA and protein sequence alignment based on segment-to-segment comparison, 93(22) Proc. Natl. Acad. Sci. U.S.A. 12098-12103 (1996); T-Coffee, see, *e.g*., Cédric Notredame et al., T-Coffee: a novel algorithm for multiple sequence alignment, 302(1) J. Mol. Biol. 205-217 (2000); MUSCLE, see, *e.g*., Robert C. Edgar, MUSCLE: Multiple sequence alignment with high score accuracy and high throughput, 32(5) Nucleic Acids Res. 1792-1797 (2004); and DIALIGN-T, see, *e.g*., Amarendran R Subramanian et al., DIALIGN-T: An improved algorithm for segment-based multiple sequence alignment, 6(1) BMC Bioinformatics 66 (2005).

As used herein, the term "naturally occurring BoNT/A variant" means any active BoNT/A produced without the aid of any human manipulation, including, without limitation, BoNT/A isoforms produced from alternatively-spliced transcripts and BoNT/A isoforms produced by spontaneous mutation. As used herein, the term "non-naturally occurring BoNT/A variant" means any active BoNT/A produced with the aid of human manipulation, including, without limitation, active BoNT/A produced by genetic engineering using random mutagenesis or rational designed and active BoNT/A produced by chemical synthesis.

As used herein, the term "conservative BoNT/A variant" means an active BoNT/A that has at least one amino acid substituted by another amino acid or an amino acid analog that has at least one property similar to that of the original amino acid. Examples of properties include, without limitation, similar size, topography, charge, hydrophobicity, hydrophilicity, lipophilicity covalent-bonding capacity, hydrogen-bonding capacity, a physicochemically property, of the like, or any combination thereof. A conservative BoNT/A variant can function in substantially the same manner as the active BoNT/A on which the conservative BoNT/A variant is based, and can be substituted for the active BoNT/A in any aspect of the present invention. A conservative BoNT/A variant may substitute one or more amino acids, two or more amino acids, three or more amino acids, four or more amino acids, five or more amino acids, ten or more amino acids, 20 or more amino acids, 30 or more amino acids, 40 or more amino acids, 50 or more amino acids, 100 or more amino acids, 200 or more amino acids, 300 or more amino acids, 400 or more amino acids, or 500 or more amino acids from the active BoNT/A on which the conservative BoNT/A variant is based. A conservative BoNT/A variant can also substitute at least 10 contiguous amino acids, at least 15 contiguous amino acids, at least 20 contiguous amino acids, or at least 25 contiguous amino acids from the active BoNT/A on which the conservative BoNT/A variant is based, that possess at least 50% amino acid identity, 65% amino acid identity, 75% amino acid identity, 85% amino acid identity or 95% amino acid identity to the active BoNT/A on which the conservative BoNT/A variant is based.

As used herein, the term "non-conservative BoNT/A variant means an active BoNT/A in which 1) at least one amino acid is deleted from the active BoNT/A on which the non-conservative BoNT/A variant is based; 2) at least one amino acid added to the active BoNT/A on which the non-conservative BoNT/A variant is based; or 3) at least one amino acid is substituted by another amino acid or an amino acid analog that does not share any property similar to that of the original amino acid. A non-conservative BoNT/A variant can function in substantially the same manner as the active BoNT/A on which the non-conservative BoNT/A variant is based, and can be substituted for the active BoNT/A in any aspect of the present invention. A non-conservative BoNT/A variant can delete one or more amino acids, two or more amino acids, three or more amino acids, four or more amino acids, five or more amino acids, and ten or more amino acids from the active BoNT/A on which the non-conservative BoNT/A variant is based. A non-conservative BoNT/A variant can add one or more amino acids, two or more amino acids, three or more amino acids, four or more amino acids, five or more amino acids, and ten or more amino acids to the active BoNT/A on which the non-conservative BoNT/A variant is based. A non-conservative BoNT/A variant may substitute one or more amino acids, two or more amino acids, three or more amino acids, four or more amino acids, five or more amino acids, ten or more amino acids, 20 or more amino acids, 30 or more amino acids, 40 or more amino acids, 50 or more amino acids, 100 or more amino acids, 200 or more amino acids, 300 or more amino acids, 400 or more amino acids, or 500 or more amino acids from the active BoNT/A on which the non-conservative BoNT/A variant is based. A non-conservative BoNT/A variant can also substitute at least 10 contiguous amino acids, at least 15 contiguous amino acids, at least 20 contiguous amino acids, or at least 25 contiguous amino acids from the active BoNT/A on which the non-conservative BoNT/A variant is based, that possess at least 50% amino acid identity, 65% amino acid identity, 75% amino acid identity, 85% amino acid identity or 95% amino acid identity to the active BoNT/A on which the non-conservative BoNT/A variant is based.

As used herein, the term "BoNT/A chimeric variant" means a molecule comprising at least a portion of an active BoNT/A and at least a portion of at least one other protein to form an active BoNT/A. Such BoNT/A chimeric molecules are described in, *e.g*., Clifford C. Shone et al., Recombinant Toxin Fragments, US 6,461,617 (Oct. 8, 2002); Keith A. Foster et al., Clostridial Toxin Derivatives Able To Modify Peripheral Sensory Afferent Functions, US 6,395,513 (May 28, 2002); Wei-Jin Lin et al., Neurotoxins with Enhanced Target Specificity, US 2002/0137886 (Sep. 26, 2002); Keith A. Foster et al., Inhibition of Secretion from Non-neural Cells, US 2003/0180289 (Sep. 25, 2003); J. Oliver Dolly et al., Activatable Recombinant Neurotoxins, WO 2001/014570 (Mar. 1, 2001); Clifford C. Shone et al., Recombinant Toxin Fragments, WO 2004/024909 (Mar. 25, 2004); and Keith A. Foster et al., Re-targeted Toxin Conjugates, WO 2005/023309 (Mar. 17, 2005).

It is also envisioned that any of a variety of active BoNT/A fragments can be useful in aspects of the present invention with the proviso that these active fragments can execute the overall cellular mechanism whereby an active BoNT/A proteolytically cleaves a substrate. Thus, aspects of this embodiment can include active BoNT/A fragments having a length of, *e.g*., at least 300 amino acids, at least 400 amino acids, at least 500 amino acids, at least 600 amino acids, at least 700 amino acids, at least 800 amino acids, at least 900 amino acids, at least 1000 amino acids, at least 1100 amino acids and at least 1200 amino acids. Other aspects of this embodiment, can include active BoNT/A fragments having a length of, *e.g*., at most 300 amino acids, at most 400 amino acids, at most 500 amino acids, at most 600 amino acids, at most 700 amino acids, at most 800 amino acids, at most 900 amino acids, at most 1000 amino acids, at most 1100 amino acids and at most 1200 amino acids.

Thus, in an embodiment, a nucleic acid molecule comprising a modified open reading frame disclosed in the present specification encodes an active BoNT/A. Other aspects of this embodiment include, without limitation, naturally occurring BoNT/A variants, such as, *e.g*., BoNT/A isoforms, non-naturally occurring BoNT/A variants, such as, *e.g*., conservative BoNT/A variants, non-conservative BoNT/A variants and active BoNT/A fragments, or any combination thereof. In another embodiment, a nucleic acid molecule comprising a modified open reading frame disclosed in the present specification encodes an active BoNT/A comprising SEQ ID NO:1. Other aspects of this embodiment include, without limitation, naturally occurring BoNT/A variants of SEQ ID NO: 1, such as, *e.g*., BoNT/A isoforms of SEQ ID NO: 1, non-naturally occurring BoNT/A variants of SEQ ID NO: 1, such as, *e.g*., conservative BoNT/A variants of SEQ ID NO: 1, non-conservative BoNT/A variants of SEQ ID NO: 1 and active BoNT/A fragments of SEQ ID NO: 1, or any combination thereof.

In still other aspects of this embodiment, an active BoNT/A has, *e.g*., at least 70% amino acid identity with SEQ ID NO:1, at least 75% amino acid identity with the SEQ ID NO:1, at least 80% amino acid identity with SEQ ID NO:1, at least 85% amino acid identity with SEQ ID NO:1, at least 90% amino acid identity with SEQ ID NO:1 or at least 95% amino acid identity with SEQ ID NO:1. In yet other aspects of this embodiment, an active BoNT/A has, *e.g*., at most 70% amino acid identity with SEQ ID NO:1, at most 75% amino acid identity with the SEQ ID NO:1, at most 80% amino acid identity with SEQ ID NO:1, at most 85% amino acid identity with SEQ ID NO:1, at most 90% amino acid identity with SEQ ID NO:1 or at most 95% amino acid identity with SEQ ID NO:1.

In other aspects of this embodiment, an active BoNT/A has, *e.g*., at most one, two, three, four, five, six, seven, eight, nine, 10, 20, 30, 40 , 50, 100, 200 or 500 non-contiguous amino acid substitutions relative to SEQ ID NO:1. In other aspects of this embodiment, an active BoNT/A has, *e.g*., at least one, two, three, four, five, six, seven, eight, nine, 10, 20, 30, 40 , 50, 100, 200 or 500 non-contiguous amino acid substitutions relative to SEQ ID NO:1. In yet other aspects of this embodiment, an active BoNT/A has, *e.g*., at most one, two, three, four, five, six, seven, eight, nine, 10, 20, 30, 40, 50, 100, 200 or 500 non-contiguous amino acid deletions relative to SEQ ID NO:1. In other aspects of this embodiment, an active BoNT/A has, *e.g*., at least one, two, three, four, five, six, seven, eight, nine, 10, 20, 30, 40 , 50, 100, 200 or 500 non-contiguous amino acid deletions relative to SEQ ID NO:1. In still other aspects of this embodiment, an active BoNT/A has, *e.g*., at most one, two, three, four, five, six, seven, eight, nine, 10, 20, 30, 40 , 50, 100, 200 or 500 non-contiguous amino acid additions relative to SEQ ID NO:1. In other aspects of this embodiment, an active BoNT/A has, *e.g*., at least one, two, three, four, five, six, seven, eight, nine, 10, 20, 30, 40 , 50, 100, 200 or 500 non-contiguous amino acid additions relative to SEQ ID NO:1.

In other aspects of this embodiment, an active BoNT/A has, *e.g*., at most one, two, three, four, five, six, seven, eight, nine, 10, 20, 30, 40 , 50, 100, 200 or 500 contiguous amino acid substitutions relative to SEQ ID NO:1. In other aspects of this embodiment, an active BoNT/A has, *e.g*., at least one, two, three, four, five, six, seven, eight, nine, 10, 20, 30, 40 , 50, 100, 200 or 500 contiguous amino acid substitutions relative to SEQ ID NO:1. In yet other aspects of this embodiment, an active BoNT/A has, *e.g*., at most one, two, three, four, five, six, seven, eight, nine, 10, 20, 30, 40 , 50, 100, 200 or 500 contiguous amino acid deletions relative to SEQ ID NO:1. In other aspects of this embodiment, an active BoNT/A has, *e.g*., at least one, two, three, four, five, six, seven, eight, nine, 10, 20, 30, 40 , 50, 100, 200 or 500 contiguous amino acid deletions relative to SEQ ID NO:1. In still other aspects of this embodiment, an active BoNT/A has, *e.g*., at most one, two, three, four, five, six, seven, eight, nine, 10, 20, 30, 40 , 50, 100, 200 or 500 contiguous amino acid additions relative to SEQ ID NO:1. In other aspects of this embodiment, an active BoNT/A has, *e.g*., at least one, two, three, four, five, six, seven, eight, nine, 10, 20, 30, 40 , 50, 100, 200 or 500 contiguous amino acid additions relative to SEQ ID NO:1.

Aspects provide, in part, a heterologous cell. As used herein, the term "heterologous cell" means any cell other than the native strain of Clostridium from which the Clostridial toxin was discovered, that expresses, or can be engineered to express an active BoNT/A disclosed in the present specification. Thus, for example, a heterologous cell that expresses a nucleic acid molecule comprising a modified open reading frame encoding an active BoNT/A would be any prokaryotic or eukaryotic cell other than the *C*. *botulinum* strain that produces the A serotype. The term heterologous cell encompasses cells from a variety of organisms, including, without limitation, bacteria strains, yeast strains, plant cells and cell lines derived from plants, insect cells and cell lines derived from insects and mammalian cells and cell lines derived from mammals. It is understood that cells useful in aspects of the invention can include, without limitation, primary cells; cultured cells; established cells; normal cells; transformed cells; tumor cells; infected cells; proliferating and terminally differentiated cells; and stably or transiently transfected cells. It is further understood that cells useful in aspects of the invention can be in any state such as proliferating or quiescent; intact or permeabilized such as through chemical-mediated transfection such as, *e.g*., calcium phosphate-mediated, diethyl-aminoethyl (DEAE) dextran-mediated, lipid-mediated, polyethyleneimine (PEI)-mediated and polybrene-mediated; physical-mediated tranfection, such as, *e.g*., biolistic particle delivery, microinjection and electroporation; and viral-mediated transfection, such as, *e.g*., retroviral-mediated transfection. It is further understood that cells useful in aspects of the invention may include those which express an active BoNT/A under control of a constitutive, tissue-specific, cell-specific or inducible promoter element, enhancer element or both. The invention provides for increased expression in an E. coli cell.

Because a wide variety of factors could influence the selection of a specific heterologous cell, nucleic acid molecules comprising a modified open reading frame providing increased expression of the encoded active BoNT/A can be designed to be expressed in a range of prokaryotic and eukaryotic cells. Codon usage tables and G+C content information for prokaryotic and eukaryotic organisms are publicly maintained by the Codon Usage Database, The First Laboratory for Plant Gene Research, Kazusa DNA Research Institute (2004), at URL address www.kazusa.or.jp/codon.

Thus nucleic acid molecules comprising a modified open reading frame providing increased expression of the encoded active BpNT/A are expressed in an E. coli cell. Non-limiting examples of prokaryotic cells of reference aspects include strains of aerobic, microaerophilic, capnophilic, facultative, anaerobic, gram-negative and gram-positive bacterial cells such as those derived from, *e.g.,* (*Bacillus subtilis, Bacillus licheniformis, Bacteroides fragilis, Clostridia perfringens, Clostridia difficile, Caulobacter crescentus, Lactococcus lactis, Methylobacterium extorquens, Neisseria meningirulls, Neisseria meningitidis, Pseudomonas fluorescens* and *Salmonella typhimurium.* In an aspect of this embodiment, a nucleic acid molecule disclosed in the present specification is expressed in an *E*. *coli* strain. In other aspects of the invention a nucleic acid molecule is expressed in an *E*. *coli* strain comprises, namely the open reading frame of SEQ ID NO: 3, SEQ ID NO: 110, or SEQ ID NO: 112. In an aspect, a nucleic acid molecule disclosed in the present specification is expressed in a *B*. *fragilis* strain. In other aspects a nucleic acid molecule expressed in a *B*. *fragilis* strain comprises, *e.g*., the open reading frame of SEQ ID NO: 7, SEQ ID NO: 8 or SEQ ID NO: 9. In an aspect, a nucleic acid molecule disclosed in the present specification is expressed in a *B*. *licheniformis* strain. In other aspects, a nucleic acid molecule expressed in a *B*. *licheniformis* strain comprises, *e.g.,* the open reading frame of SEQ ID NO: 10, SEQ ID NO: 11 or SEQ ID NO: 12. In an aspect, a nucleic acid molecule disclosed in the present specification is expressed in a *B. subtilis* strain. In other aspects, a nucleic acid molecule expressed in an *B. subtilis* strain comprises, *e.g*., the open reading frame of SEQ ID NO: 13, SEQ ID NO: 14 or SEQ ID NO: 15. In an aspect, a nucleic acid molecule disclosed in the present specification is expressed in a *C. crescentus* strain. In other aspects, a nucleic acid molecule expressed in a *C*. *crescentus* strain comprises, *e.g*., the open reading frame of SEQ ID NO: 16, SEQ ID NO: 17 or SEQ ID NO: 18. In an aspect a nucleic acid molecule disclosed in the present specification is expressed in a *C*. *difficile* strain. In another aspect, a nucleic acid molecule expressed in a *C*. *difficile* strain comprises, *e.g.,* the open reading frame of SEQ ID NO: 19, SEQ ID NO: 20 or SEQ ID NO: 21. In an aspect, a nucleic acid molecule disclosed in the present specification is expressed in a *C*. *perfringens* strain. In other aspects, a nucleic acid molecule expressed in a *C. perfringens* strain comprises, *e.g*., the open reading frame of SEQ ID NO: 22, SEQ ID NO: 23 or SEQ ID NO: 24. In an aspect, a nucleic acid molecule disclosed in the present specification is expressed in a *L. lactis* strain. In another aspect, a nucleic acid molecule expressed in a *L. lactis* strain comprises, *e.g*., the open reading frame of SEQ ID NO: 25, SEQ ID NO: 26 or SEQ ID NO: 27. In an aspect, a nucleic acid molecule disclosed in the present specification is expressed in a *M*. *extorquens* strain. In another aspect, a nucleic acid molecule expressed in a *M*. *extorquens* strain comprises, *e.g*., the open reading frame of SEQ ID NO: 28, SEQ ID NO: 29 or SEQ ID NO: 30. In an aspect, a nucleic acid molecule disclosed in the present specification is expressed in an *N. meningirulls* strain. In an aspect, a nucleic acid molecule disclosed in the present specification is expressed in a *P*. *fluorescens* strain. In an aspect, a nucleic acid molecule disclosed in the present specification is expressed in a *S*. *typhimurium* strain. In other aspects, a nucleic acid molecule expressed in a *S*. *typhimurium* strain comprises, *e.g*., the open reading frame of SEQ ID NO: 31, SEQ ID NO: 32 or SEQ ID NO: 33.

In another reference aspect, nucleic acid molecules comprising a modified open reading frame providing increased expression of the encoded active BoNT/A is expressed in an eukaryotic cell or cell line derived from an eukaryotic cell. In aspects, a nucleic acid molecule expressed in an eukaryotic cell or cell line derived from an eukaryotic cell comprises, *e.g*., any one of the open reading frames of SEQ ID NO: 34 through SEQ ID NO: 99.

In yet another reference aspect, nucleic acid sequence molecules comprising a modified open reading frame providing increased expression of the encoded active BoNT/A is expressed in a yeast strain. Non-limiting examples of yeast strains include those derived from, *e.g*., *Pichia pastoris, Pichia methanolica, Pichia angusta, Schizosaccharomyces pombe, Saccharomyces cerevisiae* and *Yarrowia lipolytica.* In an aspect of this embodiment, a nucleic acid molecule disclosed in the present specification is expressed in a *P. pastoris* strain. In other aspects of this embodiment, a nucleic acid molecule expressed in a *P*. *pastoris* strain comprises, *e.g*., the open reading frame of SEQ ID NO: 34, SEQ ID NO: 35 or SEQ ID NO: 36. In an aspect of this embodiment, a nucleic acid molecule disclosed in the present specification is expressed in a *P*. *methanolica* strain. In other aspects of this embodiment, a nucleic acid molecule expressed in a *P*. *methanolica* strain comprises, *e.g*., the open reading frame of SEQ ID NO: 34, SEQ ID NO: 35 or SEQ ID NO: 36. In an aspect of this embodiment, a nucleic acid molecule disclosed in the present specification is expressed in a *P*. *angusta* strain. In other aspects of this embodiment, a nucleic acid molecule expressed in a *P*. *angusta* strain comprises, *e.g*., the open reading frame of SEQ ID NO: 34, SEQ ID NO: 35 or SEQ ID NO: 36. In an aspect of this embodiment, a nucleic acid molecule disclosed in the present specification is expressed in a *S*. *cerevisiae* strain. In other aspects of this embodiment, a nucleic acid molecule expressed in a *S*. *cerevisiae* strain comprises, *e.g*., the open reading frame of SEQ ID NO: 37, SEQ ID NO: 38 or SEQ ID NO: 39. In an aspect of this embodiment, a nucleic acid molecule disclosed in the present specification is expressed in a *S*. *pombe* strain. In other aspects of this embodiment, a nucleic acid molecule expressed in a *S*. *pombe* strain comprises, *e.g*., the open reading frame of SEQ ID NO: 40, SEQ ID NO: 41 or SEQ ID NO: 42. In an aspect of this embodiment, a nucleic acid molecule disclosed in the present specification is expressed in a *Y. lipolytica* strain. In other aspects of this embodiment, a nucleic acid molecule expressed in a *Y. lipolytica* strain comprises, *e.g*., the open reading frame of SEQ ID NO: 43, SEQ ID NO: 44 or SEQ ID NO: 45.

In yet another reference aspect, nucleic acid sequence molecules comprising a modified open reading frame providing increased expression of the encoded active BoNT/A is expressed in a slime mold strain. Non-limiting examples of slime mold strains include those derived from, *e.g*., *Dictyostelium discoideum.* In an aspect of this embodiment, a nucleic acid molecule disclosed in the present specification is expressed in a *D. discoideum* strain. In other aspects of this embodiment, a nucleic acid molecule expressed in a *D. discoideum* strain comprises, *e.g*., the open reading frame of SEQ ID NO: 46, SEQ ID NO: 47 or SEQ ID NO: 48.

In yet another reference aspect, nucleic acid sequence molecules comprising a modified open reading frame providing increased expression of the encoded active BoNT/A is expressed in a plant cell. Non-limiting examples of plant cells and cell lines derived from plant cells include those derived from, *e.g*., species of monocots, such as, *e.g*., *Zea mays* and species of dicots, such as, *e.g*., *Arabidopsis thaliana, Triticum aestivum, Lemna gibba* and *Lemna minor.* In an aspect of this embodiment, a nucleic acid molecule disclosed in the present specification is expressed in a monocot cell or cell line derived from a monocot cell. In an aspect of this embodiment, a nucleic acid molecule disclosed in the present specification is expressed in a dicot cell or cell line derived from a dicot cell. In an aspect of this embodiment, a nucleic acid molecule disclosed in the present specification is expressed in an *A*. *thaliana* cell or cell line derived from an *A*. *thaliana* cell. In other aspects of this embodiment, a nucleic acid molecule expressed in an *A*. *thaliana* cell or cell line derived from an A. *thaliana* cell comprises, *e.g.,* the open reading frame of SEQ ID NO: 49, SEQ ID NO: 50 or SEQ ID NO: 51. In an aspect of this embodiment, a nucleic acid molecule disclosed in the present specification is expressed in a *T. aestivium* cell or cell line derived from an *A. thaliana* cell. In other aspects of this embodiment, a nucleic acid molecule expressed in an *A*. *thaliana* cell or cell line derived from a *T*. *aestivum* cell comprises, *e.g.,* the open reading frame of SEQ ID NO: 52, SEQ ID NO: 53 or SEQ ID NO: 54. In an aspect of this embodiment, a nucleic acid molecule disclosed in the present specification is expressed in a *Z*. *mays* cell or cell line derived from a *Z*. *mays* cell. In other aspects of this embodiment, a nucleic acid molecule expressed in a *Z*. *mays* cell or cell line derived from a *Z*. *mays* cell comprises, *e.g.,* the open reading frame of SEQ ID NO: 55, SEQ ID NO: 56 or SEQ ID NO: 57.

In yet another reference aspect, nucleic acid sequence molecules comprising a modified open reading frame providing increased expression of the encoded active BoNT/A is expressed in an insect cell or a cell line derived from insects. Non-limiting examples of insect cells and cell lines derived from insects such as those derived from, *e.g., Spodoptera frugiperda, Trichoplusia ni, Drosophila melanogaster* and *Manduca sexta.* In an aspect of this embodiment, a nucleic acid molecule disclosed in the present specification is expressed in a *D. melanogaster* cell or a cell line derived from *D. melanogaster.* In other aspects of this embodiment, a nucleic acid molecule expressed in a *D. melanogaster* cell or a cell line derived from *D. melanogaster* comprises, *e.g*., the open reading frame of SEQ ID NO: 58, SEQ ID NO: 59 or SEQ ID NO: 60. In an aspect of this embodiment, a nucleic acid molecule disclosed in the present specification is expressed in a *S*. *frugiperda* strain or a cell line derived from *S*. *frugiperda.* In other aspects of this embodiment, a nucleic acid molecule expressed in a *S*. *frugiperda* cell or a cell line derived from *S*. *frugiperda* comprises, *e.g*., the open reading frame of SEQ ID NO: 61, SEQ ID NO: 62 or SEQ ID NO: 63. In an aspect of this embodiment, a nucleic acid molecule disclosed in the present specification is expressed in a *T*. *ni* cell or a cell line derived from *T*. *ni.* In other aspects of this embodiment, a nucleic acid molecule expressed in a *T*. *ni* cell or a cell line derived from *T*. *ni* comprises, *e.g*., the open reading frame of SEQ ID NO: 61, SEQ ID NO: 62 or SEQ ID NO: 63. In an aspect of this embodiment, a nucleic acid molecule disclosed in the present specification is expressed in a *M. sexta* strain or a cell line derived from *M. sexta.* In an aspect of this embodiment, a nucleic acid molecule disclosed in the present specification is expressed in a Sf9 cell line. In other aspects of this embodiment, a nucleic acid molecule expressed in a Sf9 cell line comprises, *e.g*., the open reading frame of SEQ ID NO: 61, SEQ ID NO: 62 or SEQ ID NO: 63. In an aspect of this embodiment, a nucleic acid molecule disclosed in the present specification is expressed in a Sf21 cell line. In other aspects of this embodiment, a nucleic acid molecule expressed in a Sf21 cell line comprises, *e.g*., the open reading frame of SEQ ID NO: 61, SEQ ID NO: 62 or SEQ ID NO: 63. In an aspect of this embodiment, a nucleic acid molecule disclosed in the present specification is expressed in a High-Five cell line. In other aspects of this embodiment, a nucleic acid molecule expressed in a High-Five cell line comprises, *e.g*., the open reading frame of SEQ ID NO: 61, SEQ ID NO: 62 or SEQ ID NO: 63. In an aspect of this embodiment, a nucleic acid molecule disclosed in the present specification is expressed in a Schneider's Drosophila line 2 (S2) cell line. In other aspects of this embodiment, a nucleic acid molecule expressed in a Schneider's Drosophila line 2 (S2) cell line comprises, *e.g*., the open reading frame of SEQ ID NO: 58, SEQ ID NO: 59 or SEQ ID NO: 60. In an aspect of this embodiment, a nucleic acid molecule disclosed in the present specification is expressed in a Kc cell line. In other aspects of this embodiment, a nucleic acid molecule expressed in a Kc cell line comprises, *e.g*., the open reading frame of SEQ ID NO: 58, SEQ ID NO: 59 or SEQ ID NO: 60.

In yet another reference aspect, nucleic acid sequence molecules comprising a modified open reading frame providing increased expression of the encoded active BoNT/A is expressed in a fish cell or a cell line derived from a fish cell. Non-limiting examples of fish cells and cell lines derived from fish cells include those derived from, *e.g., Danio rerio.* In an aspect of this embodiment, a nucleic acid molecule disclosed in the present specification is expressed in a *D. rerio* cell or a cell line derived from *D. rerio.* In other aspects of this embodiment, a nucleic acid molecule expressed in a *D*. *rerio* cell or a cell line derived from *D. rerio* comprises, *e.g*., the open reading frame of SEQ ID NO: 64, SEQ ID NO: 65 or SEQ ID NO: 66.

In yet another reference aspect, nucleic acid sequence molecules comprising a modified open reading frame providing increased expression of the encoded active BoNT/A is expressed in an amphibian cell. Non-limiting examples of amphibian cells and cell lines derived from amphibian cells include those derived from, *e.g., Xenopus.* In an aspect of this embodiment, a nucleic acid molecule disclosed in the present specification is expressed in a *X*. *laevis* cell or a cell line derived from *X*. *laevis.* In other aspects of this embodiment, a nucleic acid molecule expressed in a *X*. *laevis* cell or a cell line derived from *X*. *laevis* comprises, *e.g*., the open reading frame of SEQ ID NO: 67, SEQ ID NO: 68 or SEQ ID NO: 69. In another aspect of this embodiment, a nucleic acid molecule disclosed in the present specification is expressed in a *X*. *tropicalis* cell or a cell line derived from *X*. *tropicalis.* In other aspects of this embodiment, a nucleic acid molecule expressed in a *X. tropicalis* cell or a cell line derived from *X*. *tropicalis* comprises, *e.g*., the open reading frame of SEQ ID NO: 70, SEQ ID NO: 71 or SEQ ID NO: 72.

In yet another reference aspect, nucleic acid sequence molecules comprising a modified open reading frame providing increased expression of the encoded active BoNT/A is expressed in a bird cell. Non-limiting examples of bird cells and cell lines derived from bird cells include those derived from, *e.g., Gallus gallus.* In an aspect of this embodiment, a nucleic acid molecule disclosed in the present specification is expressed in a *G*. *gallus* cell or a cell line derived from *G. gallus.* In other aspects of this embodiment, a nucleic acid molecule expressed in a *G. gallus* cell or a cell line derived from *G. gallus* comprises, *e.g*., the open reading frame of SEQ ID NO: 73, SEQ ID NO: 74 or SEQ ID NO: 75.

In yet another reference aspect, nucleic acid sequence molecules comprising a modified open reading frame providing increased expression of the encoded active BoNT/A is expressed in a mammalian cell. Non-limiting examples of mammalian cells and cell lines derived from mammalian cells include those derived from, *e.g*., mouse, rat, hamster, porcine, bovine, equine, primate and human. In an aspect of this embodiment, a nucleic acid molecule disclosed in the present specification is expressed in a mouse cell or a cell line derived from mouse. In other aspects of this embodiment, a nucleic acid molecule expressed in a mouse cell or a cell line derived from mouse comprises, *e.g*., the open reading frame of SEQ ID NO: 76, SEQ ID NO: 77 or SEQ ID NO: 78. In yet another aspect of this embodiment, a nucleic acid molecule disclosed in the present specification is expressed in a *Mus musculus* cell or a cell line derived from *M. musculus.* In yet other aspects of this embodiment, a nucleic acid molecule expressed in a *M*. *musculus* cell or a cell line derived from *M. musculus* comprises, *e.g*., the open reading frame of SEQ ID NO: 76, SEQ ID NO: 77 or SEQ ID NO: 78. In an aspect of this embodiment, a nucleic acid molecule disclosed in the present specification is expressed in a 10T1/2 cell line. In other aspects of this embodiment, a nucleic acid molecule expressed in a 10T1/2 cell line comprises, *e.g*., the open reading frame of SEQ ID NO: 76, SEQ ID NO: 77 or SEQ ID NO: 78. In another aspect of this embodiment, a nucleic acid molecule disclosed in the present specification is expressed in a BALB/3T3 cell line. In yet other aspects of this embodiment, a nucleic acid molecule expressed in a BALB/3T3 cell line comprises, *e.g*., the open reading frame of SEQ ID NO: 76, SEQ ID NO: 77 or SEQ ID NO: 78. In another aspect of this embodiment, a nucleic acid molecule disclosed in the present specification is expressed in a L-M cell line. In yet other aspects of this embodiment, a nucleic acid molecule expressed in a L-M cell line comprises, *e.g*., the open reading frame of SEQ ID NO: 76, SEQ ID NO: 77 or SEQ ID NO: 78. In another aspect of this embodiment, a nucleic acid molecule disclosed in the present specification is expressed in a NB4 cell line. In yet other aspects of this embodiment, a nucleic acid molecule expressed in a NB4 cell line comprises, *e.g*., the open reading frame of SEQ ID NO: 76, SEQ ID NO: 77 or SEQ ID NO: 78. In another aspect of this embodiment, a nucleic acid molecule disclosed in the present specification is expressed in a 1A3 cell line. In yet other aspects of this embodiment, a nucleic acid molecule expressed in a 1A3 cell line comprises, *e.g*., the open reading frame of SEQ ID NO: 76, SEQ ID NO: 77 or SEQ ID NO: 78. In another aspect of this embodiment, a nucleic acid molecule disclosed in the present specification is expressed in a NIE-115 cell line. In yet other aspects of this embodiment, a nucleic acid molecule expressed in a NIE-115 cell line comprises, *e.g*., the open reading frame of SEQ ID NO: 76, SEQ ID NO: 77 or SEQ ID NO: 78. In another aspect of this embodiment, a nucleic acid molecule disclosed in the present specification is expressed in a NG108-15 cell line. In yet other aspects of this embodiment, a nucleic acid molecule expressed in a NG108-15 cell line comprises, *e.g*., the open reading frame of SEQ ID NO: 76, SEQ ID NO: 77 or SEQ ID NO: 78. In another aspect of this embodiment, a nucleic acid molecule disclosed in the present specification is expressed in a NIH3T3 cell line. In yet other aspects of this embodiment, a nucleic acid molecule expressed in a NIH3T3 cell line comprises, *e.g*., the open reading frame of SEQ ID NO: 76, SEQ ID NO: 77 or SEQ ID NO: 78. In another aspect of this embodiment, a nucleic acid molecule disclosed in the present specification is expressed in a NCTC cell line. In yet other aspects of this embodiment, a nucleic acid molecule expressed in a NCTC cell line comprises, *e.g*., the open reading frame of SEQ ID NO: 76, SEQ ID NO: 77 or SEQ ID NO: 78. In another aspect of this embodiment, a nucleic acid molecule disclosed in the present specification is expressed in a Neuro-2A cell line. In yet other aspects of this embodiment, a nucleic acid molecule expressed in a Neuro-2A cell line comprises, *e.g*., the open reading frame of SEQ ID NO: 76, SEQ ID NO: 77 or SEQ ID NO: 78.

In an aspect, a nucleic acid molecule disclosed in the present specification is expressed in a rat cell or a cell line derived from rat. In other aspects of this embodiment, a nucleic acid molecule expressed in a rat cell or a cell line derived from rat comprises, *e.g*., the open reading frame of SEQ ID NO: 79, SEQ ID NO: 80 or SEQ ID NO: 81. In yet another aspect of this embodiment, a nucleic acid molecule disclosed in the present specification is expressed in a *Rattus norvegicus* cell or a cell line derived from *R. norvegicus.* In yet another aspect of this embodiment, a nucleic acid molecule expressed in a *R. norvegicus* cell or a cell line derived from *R. norvegicus* comprises, *e.g.,* the open reading frame of SEQ ID NO: 79, SEQ ID NO: 80 or SEQ ID NO: 81. In an aspect of this embodiment, a nucleic acid molecule disclosed in the present specification is expressed in a PC12 cell line. In other aspects of this embodiment, a nucleic acid molecule expressed in a PC12 cell line comprises, *e.g*., the open reading frame of SEQ ID NO: 79, SEQ ID NO: 80 or SEQ ID NO: 81. In another aspect of this embodiment, a nucleic acid molecule disclosed in the present specification is expressed in a GH1 cell line. In yet other aspects of this embodiment, a nucleic acid molecule expressed in a GH1 cell line comprises, *e.g*., the open reading frame of SEQ ID NO: 79, SEQ ID NO: 80 or SEQ ID NO: 81. In another aspect of this embodiment, a nucleic acid molecule disclosed in the present specification is expressed in a GH3 cell line. In yet other aspects of this embodiment, a nucleic acid molecule expressed in a GH3 cell line comprises, *e.g*., the open reading frame of SEQ ID NO: 79, SEQ ID NO: 80 or SEQ ID NO: 81. In another aspect of this embodiment, a nucleic acid molecule disclosed in the present specification is expressed in a C6 cell line. In yet other aspects of this embodiment, a nucleic acid molecule expressed in a C6 cell line comprises, *e.g*., the open reading frame of SEQ ID NO: 79, SEQ ID NO: 80 or SEQ ID NO: 81. In another aspect of this embodiment, a nucleic acid molecule disclosed in the present specification is expressed in a L2 cell line. In yet other aspects of this embodiment, a nucleic acid molecule expressed in a L2 cell line comprises, *e.g*., the open reading frame of SEQ ID NO: 79, SEQ ID NO: 80 or SEQ ID NO: 81.

In an aspect, a nucleic acid molecule disclosed in the present specification is expressed in a hamster cell or a cell line derived from hamster. In other aspects of this embodiment, a nucleic acid molecule expressed in a hamster cell or a cell line derived from hamster comprises, *e.g*., the open reading frame of SEQ ID NO: 82, SEQ ID NO: 83 or SEQ ID NO: 84. In yet another aspect of this embodiment, a nucleic acid molecule disclosed in the present specification is expressed in a *Cricetulus griseus* cell or a cell line derived from *C. griseus.* In yet other aspects of this embodiment, a nucleic acid molecule expressed in a *C*. *griseus* cell or a cell line derived from *C. griseus* comprises, *e.g*., the open reading frame of SEQ ID NO: 82, SEQ ID NO: 83 or SEQ ID NO: 84. In an aspect of this embodiment, a nucleic acid molecule disclosed in the present specification is expressed in a CHO cell line. In other aspects of this embodiment, a nucleic acid molecule expressed in a CHO cell line comprises, *e.g*., the open reading frame of SEQ ID NO: 82, SEQ ID NO: 83 or SEQ ID NO: 84. In another aspect of this embodiment, a nucleic acid molecule disclosed in the present specification is expressed in a 6E6 cell line. In yet other aspects of this embodiment, a nucleic acid molecule expressed in a 6E6 cell line comprises, *e.g*., the open reading frame of SEQ ID NO: 82, SEQ ID NO: 83 or SEQ ID NO: 84.

In an aspect, a nucleic acid molecule disclosed in the present specification is expressed in a porcine cell or a cell line derived from porcine. In other aspects of this embodiment, a nucleic acid molecule expressed in a porcine cell or a cell line derived from porcine comprises, *e.g*., the open reading frame of SEQ ID NO: 85, SEQ ID NO: 86 or SEQ ID NO: 87. In yet another aspect of this embodiment, a nucleic acid molecule disclosed in the present specification is expressed in a *Sus scrofa* cell or a cell line derived from *S. scrofa*. In yet other aspects of this embodiment, a nucleic acid molecule expressed in a *S. scrofa* cell or a cell line derived from *S. scrofa* comprises, *e.g*., the open reading frame of SEQ ID NO: 85, SEQ ID NO: 86 or SEQ ID NO: 87. In an aspect of this embodiment, a nucleic acid molecule disclosed in the present specification is expressed in a PK15 cell line. In other aspects of this embodiment, a nucleic acid molecule expressed in a PK15 cell line comprises, *e.g*., the open reading frame of SEQ ID NO: 85, SEQ ID NO: 86 or SEQ ID NO: 87. In another aspect of this embodiment, a nucleic acid molecule disclosed in the present specification is expressed in a LLC-PK1 cell line. In yet other aspects of this embodiment, a nucleic acid molecule expressed in a LLC-PK1 cell line comprises, *e.g*., the open reading frame of SEQ ID NO: 85, SEQ ID NO: 86 or SEQ ID NO: 87. In another aspect of this embodiment, a nucleic acid molecule disclosed in the present specification is expressed in a ST cell line. In yet other aspects of this embodiment, a nucleic acid molecule expressed in a ST cell line comprises, *e.g*., the open reading frame of SEQ ID NO: 85, SEQ ID NO: 86 or SEQ ID NO: 87. In another aspect of this embodiment, a nucleic acid molecule disclosed in the present specification is expressed in a ESK-4 cell line. In yet other aspects of this embodiment, a nucleic acid molecule expressed in a ESK-4 cell line comprises, *e.g*., the open reading frame of SEQ ID NO: 85, SEQ ID NO: 86 or SEQ ID NO: 87.

In an aspect, a nucleic acid molecule disclosed in the present specification is expressed in a bovine cell or a cell line derived from bovine. In other aspects of this embodiment, a nucleic acid molecule expressed in a bovine cell or a cell line derived from bovine comprises, *e.g*., the open reading frame of SEQ ID NO: 88, SEQ ID NO: 89 or SEQ ID NO: 90. In yet another aspect of this embodiment, a nucleic acid molecule disclosed in the present specification is expressed in a Bos *taurus* cell or a cell line derived from *B. taurus.* In yet other aspects of this embodiment, a nucleic acid molecule expressed in a *B. taurus* cell or a cell line derived from *B. taurus* comprises, *e.g*., the open reading frame of SEQ ID NO: 88, SEQ ID NO: 89 or SEQ ID NO: 90. In an aspect of this embodiment, a nucleic acid molecule disclosed in the present specification is expressed in a CPAE cell line. In other aspects of this embodiment, a nucleic acid molecule expressed in a CPAE cell line comprises, *e.g*., the open reading frame of SEQ ID NO: 88, SEQ ID NO: 89 or SEQ ID NO: 90. In another aspect of this embodiment, a nucleic acid molecule disclosed in the present specification is expressed in a BT cell line. In yet other aspects of this embodiment, a nucleic acid molecule expressed in a BT cell line comprises, *e.g*., the open reading frame of SEQ ID NO: 88, SEQ ID NO: 89 or SEQ ID NO: 90. In another aspect of this embodiment, a nucleic acid molecule disclosed in the present specification is expressed in a SBAC cell line. In yet other aspects of this embodiment, a nucleic acid molecule expressed in a SBAC cell line comprises, *e.g*., the open reading frame of SEQ ID NO: 88, SEQ ID NO: 89 or SEQ ID NO: 90. In another aspect of this embodiment, a nucleic acid molecule disclosed in the present specification is expressed in a FB2 cell line. In yet other aspects of this embodiment, a nucleic acid molecule expressed in a FB2 cell line comprises, *e.g*., the open reading frame of SEQ ID NO: 88, SEQ ID NO: 89 or SEQ ID NO: 90.

In an aspect, a nucleic acid molecule disclosed in the present specification is expressed in a equine cell or a cell line derived from equine. In other aspects of this embodiment, a nucleic acid molecule expressed in a equine cell or a cell line derived from equine comprises, *e.g*., the open reading frame of SEQ ID NO: 91, SEQ ID NO: 92 or SEQ ID NO: 93. In yet another aspect of this embodiment, a nucleic acid molecule disclosed in the present specification is expressed in a *Equus caballus* cell or a cell line derived from *E. caballus.* In yet other aspects of this embodiment, a nucleic acid molecule expressed in a *E. cabal*/*us* cell or a cell line derived from *E. caballus* comprises, *e.g*., the open reading frame of SEQ ID NO: 91, SEQ ID NO: 92 or SEQ ID NO: 93. In an aspect of this embodiment, a nucleic acid molecule disclosed in the present specification is expressed in a NBL-6 cell line. In other aspects of this embodiment, a nucleic acid molecule expressed in a NBL-6 cell line comprises, *e.g*., the open reading frame of SEQ ID NO: 91, SEQ ID NO: 92 or SEQ ID NO: 93.

In an aspect, a nucleic acid molecule disclosed in the present specification is expressed in a primate cell or a cell line derived from primate. In other aspects of this embodiment, a nucleic acid molecule expressed in a primate cell or a cell line derived from primate comprises, *e.g*., the open reading frame of SEQ ID NO: 94, SEQ ID NO: 95 or SEQ ID NO: 96. In yet another aspect of this embodiment, a nucleic acid molecule disclosed in the present specification is expressed in a *Cercopithecus aethiops* cell or a cell line derived from *C. aethiops.* In yet other aspects of this embodiment, a nucleic acid molecule expressed in a *C. aethiops* cell or a cell line derived from *C. aethiops* comprises, *e.g*., the open reading frame of SEQ ID NO: 94, SEQ ID NO: 95 or SEQ ID NO: 96. In an aspect of this embodiment, a nucleic acid molecule disclosed in the present specification is expressed in a COS-1 cell line. In other aspects of this embodiment, a nucleic acid molecule expressed in a COS-1 cell line comprises, *e.g*., the open reading frame of SEQ ID NO: 94, SEQ ID NO: 95 or SEQ ID NO: 96. In another aspect of this embodiment, a nucleic acid molecule disclosed in the present specification is expressed in a COS-7 cell line. In yet other aspects of this embodiment, a nucleic acid molecule expressed in a COS-7 cell line comprises, *e.g*., the open reading frame of SEQ ID NO: 94, SEQ ID NO: 95 or SEQ ID NO: 96. In another aspect of this embodiment, a nucleic acid molecule disclosed in the present specification is expressed in a VV-1 cell line. In yet other aspects of this embodiment, a nucleic acid molecule expressed in a VV-1 cell line comprises, *e.g*., the open reading frame of SEQ ID NO: 94, SEQ ID NO: 95 or SEQ ID NO: 96.

In an aspect, a nucleic acid molecule disclosed in the present specification is expressed in a human cell or a cell line derived from human. In another aspect of this embodiment, a nucleic acid molecule expressed in a human cell or a cell line derived from human comprises, *e.g*., the open reading frame of SEQ ID NO: 97, SEQ ID NO: 98 or SEQ ID NO: 99. In yet another aspect of this embodiment, a nucleic acid molecule disclosed in the present specification is expressed in a *Homo sapiens* cell or a cell line derived from *H. sapiens.* In another aspect of this embodiment, a nucleic acid molecule expressed in a *H. sapiens* cell or a cell line derived from *H. sapiens* comprises, *e.g.,* the open reading frame of SEQ ID NO: 97, SEQ ID NO: 98 or SEQ ID NO: 99. In an aspect of this embodiment, a nucleic acid molecule disclosed in the present specification is expressed in a SH-SY5Y cell line. In other aspects of this embodiment, a nucleic acid molecule expressed in a SH-SY5Y cell line comprises, *e.g*., the open reading frame of SEQ ID NO: 97, SEQ ID NO: 98 or SEQ ID NO: 99. In another aspect of this embodiment, a nucleic acid molecule disclosed in the present specification is expressed in a SK-N-DZ cell line. In yet other aspects of this embodiment, a nucleic acid molecule expressed in a SK-N-DZ cell line comprises, *e.g*., the open reading frame of SEQ ID NO: 97, SEQ ID NO: 98 or SEQ ID NO: 99. In another aspect of this embodiment, a nucleic acid molecule disclosed in the present specification is expressed in a SK-N-SH cell line. In yet other aspects of this embodiment, a nucleic acid molecule expressed in a SK-N-SH cell line comprises, *e.g*., the open reading frame of SEQ ID NO: 97, SEQ ID NO: 98 or SEQ ID NO: 99. In another aspect of this embodiment, a nucleic acid molecule disclosed in the present specification is expressed in a BE(2)-C cell line. In yet other aspects of this embodiment, a nucleic acid molecule expressed in a BE(2)-C cell line comprises, *e.g*., the open reading frame of SEQ ID NO: 97, SEQ ID NO: 98 or SEQ ID NO: 99. In another aspect of this embodiment, a nucleic acid molecule disclosed in the present specification is expressed in a HeLa cell line. In yet other aspects of this embodiment, a nucleic acid molecule expressed in a HeLa cell line comprises, *e.g*., the open reading frame of SEQ ID NO: 97, SEQ ID NO: 98 or SEQ ID NO: 99. In another aspect of this embodiment, a nucleic acid molecule disclosed in the present specification is expressed in a HEK 293 cell line. In yet other aspects of this embodiment, a nucleic acid molecule expressed in a HEK 293 cell line comprises, *e.g*., the open reading frame of SEQ ID NO: 97, SEQ ID NO: 98 or SEQ ID NO: 99. In another aspect of this embodiment, a nucleic acid molecule disclosed in the present specification is expressed in a MCF-7 cell line. In yet other aspects of this embodiment, a nucleic acid molecule expressed in a MCF-7 cell line comprises, *e.g*., the open reading frame of SEQ ID NO: 97, SEQ ID NO: 98 or SEQ ID NO: 99. In another aspect of this embodiment, a nucleic acid molecule disclosed in the present specification is expressed in a HepG2 cell line. In yet other aspects of this embodiment, a nucleic acid molecule expressed in a HepG2 cell line comprises, *e.g*., the open reading frame of SEQ ID NO: 97, SEQ ID NO: 98 or SEQ ID NO: 99. In another aspect of this embodiment, a nucleic acid molecule disclosed in the present specification is expressed in a HL-60 cell line. In yet other aspects of this embodiment, a nucleic acid molecule expressed in a HL-60 cell line comprises, *e.g*., the open reading frame of SEQ ID NO: 97, SEQ ID NO: 98 or SEQ ID NO: 99. In another aspect of this embodiment, a nucleic acid molecule disclosed in the present specification is expressed in a IMR-32 cell line. In yet other aspects of this embodiment, a nucleic acid molecule expressed in a IMR-32 cell line comprises, *e.g*., the open reading frame of SEQ ID NO: 97, SEQ ID NO: 98 or SEQ ID NO: 99. In another aspect of this embodiment, a nucleic acid molecule disclosed in the present specification is expressed in a SW-13 cell line. In yet other aspects of this embodiment, a nucleic acid molecule expressed in a SW-13 cell line comprises, *e.g*., the open reading frame of SEQ ID NO: 97, SEQ ID NO: 98 or SEQ ID NO: 99. In another aspect of this embodiment, a nucleic acid molecule disclosed in the present specification is expressed in a CHP3 cell line. In yet other aspects of this embodiment, a nucleic acid molecule expressed in a CHP3 cell line comprises, *e.g*., the open reading frame of SEQ ID NO: 97, SEQ ID NO: 98 or SEQ ID NO: 99.

The nucleic acid molecules disclosed in the present specification include, in part, a modified open reading frame providing increased expression of an encoded active BoNT/A. Increased expression of an active BoNT/A is determined by comparing the amount of an active BoNT/A expressed from a modified open reading frame with the amount of the same active BoNT/A expressed from an unmodified open reading frame in an otherwise identical nucleic acid molecule. As used herein, the term "modified open reading frame" means an open reading frame that contains at least one nucleotide change providing increased quantitative and qualitative expression of the encoded active BoNT/A. As used herein, the term "unmodified open reading frame" means an open reading frame that does not contain any nucleotide changes providing increased expression of the encoded active BoNT/A. As a non-limiting example, SEQ ID NO: 2, SEQ ID NO: 114 and SEQ ID NO: 115 are unmodified open reading frames that will not provide increased expression of the encoded active BoNT/A in a heterologous cell and SEQ ID NO: 3 through SEQ ID NO: 99, SEQ ID NO: 110, SEQ ID NO: 112 and SEQ ID NO: 122 through SEQ ID NO: 125 are modified open reading frames that can provide increased expression of the encoded active BoNT/A in the appropriate heterologous cell. It is further understood by one skilled in the art that the methods and procedures used to express the nucleic acid molecules comprising the modified open reading frame should be the same or similar to the methods and procedures used to express the nucleic acid molecules comprising the unmodified open reading frame to ensure accurate and consistent comparisons.

A wide variety of well-established methods can be used to compare the amount of expressed active BoNT/A from a modified open reading frame to the amount of the same active BoNT/A expressed from an unmodified open reading frame in an otherwise identical nucleic acid molecule. Comparisons of amounts of an active BoNT/A expressed can be either qualitative or quantitative.

Active BoNT/A amounts can be measured using any procedure that can separate and visualize proteins from a cell lysate, such as, *e.g*., procedures involving gel electrophoresis and protein staining, Western blotting, protein-labeling, as well as, other procedures involving protein separation and visualization. Thus, amounts of active BoNT/A can be appraised by labeling active BoNT/A using a radioactive amino acid tracer and visualizing expression by autoradiography after gel electrophoresis. Likewise, incorporation of radiolabeled amino acids into active BoNT/A can be measured by scintillation counting after Trichloroacetic Acid (TCA) precipitation. Amounts of active BoNT/A can also be assessed by staining proteins separated by gel electrophoresis using, *e.g*., dye staining procedures like Coomassie Brilliant Blue and Colloidal Coomassie Brilliant Blue; fluorescence staining procedures like SYPRO^{®} Ruby and ruthenium II; or silver staining procedures. Amounts of active BoNT/A can likewise be determined by antibody staining after Western blot analysis. Furthermore, functional assays that measure the biological activity of active BoNT/A can be used to compare amounts of active BoNT/A expressed from a modified open reading frame to the amount of the same active BoNT/A expressed from an unmodified open reading frame in an otherwise identical nucleic acid molecule, such as, *e.g*., SNAP25 cleavage assay and the GFP-SNAP25 Fluorescence Release Assay. Non-limiting examples of specific procedures to separate and visualize protein amounts, as well as well-characterized reagents, conditions and protocols are readily available from commercial vendors that include, without limitation, Amersham Biosciences, Piscataway, NJ; Bio-Rad Laboratories, Hercules, CA; Pierce Biotechnology, Inc., Rockford, IL; Promega Corporation, Madison, WI, and Stratagene, La Jolla, CA. In addition, non-limiting examples of specific protocols necessary to separate, visualize and quantify a protein are described in *e.g*., MOLECULAR CLONING A LABORATORY MANUAL, supra, (2001); and CURRENT PROTOCOLS IN MOLECULAR BIOLOGY, supra, (2004). These protocols are routine procedures within the scope of one skilled in the art and from the teaching herein.

Active BoNT/A amounts can be measured after one or more purification steps using, without limitation, gel electrophoresis and protein staining, Western blotting, protein-labeling, UV absorbance, the Lowry assay, the biuret assay, the Smith copper/bicinchoninic (BCA) assay, and the Bradford dye assay, see *e.g*., Christine V. Sapan et al., Colorimetric Protein Assay Techniques, 29(2) BIOTECHNOL. APPL. BIOCHEM. 99-108, (1999). Any of a variety of methods can be used for purifying an active BoNT/A disclosed in the present specification. Examples of purification methods include, without limitation, ammonium sulfate or ethanol precipitation, acid extraction, ion exchange chromatography, phosphocellulose chromatography, lectin chromatography, affinity chromatography, hydrophobic interaction chromatography, size exclusion chromatography, gel-filtration chromatography, adsorption chromatography, hydroxyapatite chromatography, fast performance liquid chromatography (FPLC), and high performance liquid (HPLC) chromatography. Binding moieties of the target peptide of interest may be attached to any of a variety of substances including, without limitation resins, agarose, and magnetic beads. In addition, any of a variety of processing techniques can be used including, without limitation, batch-wise processing, and gravity-feed columns. Protein refolding steps may also be necessary to ensure recovery of a functionally active BoNT/A encoded by nucleic acid molecules disclosed in the specification. Non-limiting examples of specific protocols for purifying and recovering proteins are described in, *e.g*., John Abelson et al., GUIDE TO PROTEIN PURIFICATION, (Academic Press, 1990), PROTEIN PURIFICATION: PRINCIPLES AND PRACTICE, (Robert K. Scopes et al. eds., Springer Verlag, 3rd ed. 1994), PROTEIN PURIFICATION TECHNIQUES: A PRACTICAL APPROACH, (Simon Roe ed., Oxford University Press, 2nd ed. 2001), MOLECULAR CLONING A LABORATORY MANUAL, supra, (2001), Ian M. Rosenberg, PROTEIN ANALYSIS & PURIFICATION: BENCHTOP TECHNIQUES, (Springer Verlag, 2002). These protocols are routine procedures within the scope of one skilled in the art and from the teaching herein.

Thus, in an embodiment, the amount of an active BoNT/A expressed from a modified open reading frame is increased as compared to the amount of the same active BoNT/A expressed from an unmodified open reading frame. In is envisioned that, with the exception of the modified and unmodified open reading frames, the nucleic acid molecules comprising the open reading frames are similar or identical in nature. In aspects of this embodiment, the amount of an active BoNT/A expressed from a modified open reading frame is, *e.g*., increased at least 1.5-fold as compared to the amount of the same BoNT/A expressed from an unmodified open reading frame in an otherwise identical nucleic acid molecule; increased at least 2-fold as compared to the amount of the same BoNT/A expressed from an unmodified open reading frame in an otherwise identical nucleic acid molecule; increased at least 3-fold as compared to the amount of the same BoNT/A expressed from an unmodified open reading frame in an otherwise identical nucleic acid molecule; increased at least 4-fold as compared to the amount of the same BoNT/A expressed from an unmodified open reading frame in an otherwise identical nucleic acid molecule; increased at least 5-fold as compared to the amount of the same BoNT/A expressed from an unmodified open reading frame in an otherwise identical nucleic acid molecule; increased at least 10-fold as compared to the amount of the same BoNT/A expressed from an unmodified open reading frame in an otherwise identical nucleic acid molecule; increased at least 25-fold as compared to the amount of the same BoNT/A expressed from an unmodified open reading frame in an otherwise identical nucleic acid molecule; increased at least 50-fold as compared to the amount of the same BoNT/A expressed from an unmodified open reading frame in an otherwise identical nucleic acid molecule; increased at least 100-fold as compared to the amount of the same BoNT/A expressed from an unmodified open reading frame in an otherwise identical nucleic acid molecule; or increased at least 200-fold as compared to the amount of the same BoNT/A expressed from an unmodifed open reading frame in an otherwise identical nucleic acid molecule.

In aspects of this embodiment, the amount of an active BoNT/A expressed from a modified open reading frame is, *e.g*., increased at most 1.5-fold as compared to the amount of the same BoNT/A expressed from an unmodified open reading frame in an otherwise identical nucleic acid molecule; increased at most 2-fold as compared to the amount of the same BoNT/A expressed from an unmodified open reading frame in an otherwise identical nucleic acid molecule; increased at most 3-fold as compared to the amount of the same BoNT/A expressed from an unmodified open reading frame in an otherwise identical nucleic acid molecule; increased at most 4-fold as compared to the amount of the same BoNT/A expressed from an unmodified open reading frame in an otherwise identical nucleic acid molecule; increased at most 5-fold as compared to the amount of the same BoNT/A expressed from an unmodified open reading frame in an otherwise identical nucleic acid molecule; increased at most 10-fold as compared to the amount of the same BoNT/A expressed from an unmodified open reading frame in an otherwise identical nucleic acid molecule; increased at most 25-fold as compared to the amount of the same BoNT/A expressed from an unmodified open reading frame in an otherwise identical nucleic acid molecule; increased at most 50-fold as compared to the amount of the same BoNT/A expressed from an unmodified open reading frame in an otherwise identical nucleic acid molecule; increased at most 100-fold as compared to the amount of the same BoNT/A expressed from an unmodified open reading frame in an otherwise identical nucleic acid molecule; or increased at most 200-fold as compared to the amount of the same BoNT/A expressed from an unmodified open reading frame in an otherwise identical nucleic acid molecule.

Other aspects of the present invention provide expression constructs comprising a nucleic acid molecule disclosed in the present specification, operably-linked to an expression vector useful for expressing the nucleic acid molecule in a heterologous cell. A wide variety of expression vectors are envisioned, including, without limitation, a prokaryotic expression vector useful for expressing a nucleic acid molecule comprising a modified open reading frame providing increased expression of the encoded active BoNT/A in a prokaryotic cell; a yeast expression vector useful for expressing a nucleic acid molecule comprising a modified open reading frame providing increased expression of the encoded active BoNT/A in a yeast cell; an insect expression vector useful for expressing a nucleic acid molecule comprising a modified open reading frame providing increased expression of the encoded active BoNT/A in an insect cell; a mammalian expression vector useful for expressing a nucleic acid molecule comprising a modified open reading frame providing increased expression of the encoded active BoNT/A in a mammalian cell and a expression vector useful for expressing a nucleic acid molecule in a cell-free extract comprising a modified open reading frame providing increased expression of the encoded active BoNT/A in the cell-free extract.

The expression constructs disclosed in the present specification include, in part, a nucleic acid molecule. It is envisioned that any and all nucleic acid molecules disclosed in the present specification can be used. Thus, aspects of this embodiment include, without limitation, nucleic acid molecules comprising a modified open reading frame providing increased expression of the encoded active BoNT/A in a prokaryotic cell; nucleic acid molecules comprising a modified open reading frame providing increased expression of the encoded active BoNT/A in a yeast cell; nucleic acid molecules comprising a modified open reading frame providing increased expression of the encoded active BoNT/A in an slime mold cell; nucleic acid molecules comprising a modified open reading frame providing increased expression of the encoded active BoNT/A in a plant cell or cell line derived from a plant cell; nucleic acid molecules comprising a modified open reading frame providing increased expression of the encoded active BoNT/A in an insect cell or cell line derived from an insect cell; nucleic acid molecules comprising a modified open reading frame providing increased expression of the encoded active BoNT/A in an fish cell or cell line derived from a fish cell; nucleic acid molecules comprising a modified open reading frame providing increased expression of the encoded active BoNT/A in an amphibian cell or cell line derived from an amphibian cell; nucleic acid molecules comprising a modified open reading frame providing increased expression of the encoded active BoNT/A in a bird cell or cell line derived from a bird cell; and nucleic acid molecules comprising a modified open reading frame providing increased expression of the encoded active BoNT/A in a mammalian cell or cell line derived from a mammalian cell, such as, *e.g*., mouse, rat, hamster, porcine, bovine, equine, primate and human.

The expression constructs disclosed in the present specification include, in part, a heterologous cell, which is an E. coli cell. Reference, aspects include, without limitation, prokaryotic cells including, without limitation, strains of aerobic, microaerophilic, capnophilic, facultative, anaerobic, gram-negative and gram-positive bacterial cells such as those derived from, *e.g., Escherichia coli, Bacillus subtilis, Bacillus licheniformis, Bacteroides fragilis, Clostridia perfringens, Clostridia diffcile, Caulobacter crescentus, Lactococcus lactis, Methylobacterium extorquens, Neisseria meningirulls, Neisseria meningitidis, Pseudomonas fluorescens* and *Salmonella typhimurium;* and eukaryotic cells including, without limitation, yeast strains, such as, *e.g.,* those derived from *Pichia pastoris, Pichia methanolica, Pichia angusta, Schizosaccharomyces pombe, Saccharomyces cerevisiae* and *Yarrowia lipolytica;* slime mold strains, such as, *e.g*., those derived from, *e.g., Dictyostelium discoideum;* plant cells and cell lines derived from plant cells, such as, *e.g*., those derived from species of monocots, species of dicots, *Zea mays* and *Arabidopsis thaliana;* insect cells and cell lines derived from insects, such as, *e.g*., those derived from *Spodoptera frugiperda, Trichoplusia ni, Drosophila melanogaster* and *Manduca sexta;* fish cells and cell lines derived from fish cells, such as, *e.g*., those derived from *Denio renia;* amphibian cells and cell lines derived from amphibian cells, such as, *e.g*., those derived from *Xenopus laevis* and *Xenopus tropicalis;* bird cells and cell lines derived from bird cells, such as, *e.g*., those derived from *Gallus gallus;* mammalian cells and cell lines derived from mammalian cells, such as, *e.g*., those derived from mouse, rat, hamster, porcine, bovine, equine, primate and human.

The expression constructs disclosed in the present specification include, in part, a nucleic acid molecule disclosed in the present specification, operably-linked to an expression vector. As used herein, the term "operably linked" means any of a variety of cloning methods that can join a nucleic acid molecule disclosed in the present specification to an expression vector such that a peptide encoded by the nucleic acid molecule is expressed when introduced into a heterologous cell. Well-established molecular biology techniques that may be necessary to make an expression construct disclosed in the present specification including, but not limited to, procedures involving polymerase chain reaction (PCR) amplification restriction enzyme reactions, agarose gel electrophoresis, nucleic acid ligation, bacterial transformation, nucleic acid purification, nucleic acid sequencing and recombination-based techniques are routine procedures well within the scope of one skilled in the art and from the teaching herein. Non-limiting examples of specific protocols necessary to make an expression construct are described in *e.g*., MOLECULAR CLONING A LABORATORY MANUAL, supra, (2001); and CURRENT PROTOCOLS IN MOLECULAR BIOLOGY (Frederick M. Ausubel et al., eds. John Wiley & Sons, 2004), which are hereby incorporated by reference. These protocols are routine procedures well within the scope of one skilled in the art and from the teaching herein.

A wide variety of expression vectors can be employed for expressing an open reading frame encoding an active BoNT/A and include without limitation, viral expression vectors, prokaryotic expression vectors and eukaryotic expression vectors including yeast, insect, plant and mammalian expression vectors. Non-limiting examples of expression vectors, along with well-established reagents and conditions for making and using an expression construct from such expression vectors are readily available from commercial vendors that include, without limitation, BD Biosciences-Clontech, Palo Alto, CA; BD Biosciences Pharmingen, San Diego, CA; Invitrogen, Inc, Carlsbad, CA; EMD Biosciences-Novagen, Madison, WI; QIAGEN, Inc., Valencia, CA; and Stratagene, La Jolla, CA. The selection, making and use of an appropriate expression vector are routine procedures well within the scope of one skilled in the art and from the teachings herein.

It is envisioned that any of a variety of expression systems may be useful for expressing constructs disclosed in the present specification. An expression system encompasses both cell-based systems and cell-free expression systems. Cell-based systems include, without limitation, viral expression systems, prokaryotic expression systems, yeast expression systems, baculoviral expression systems, insect expression systems and mammalian expression systems. Cell-free systems include, without limitation, wheat germ extracts, rabbit reticulocyte extracts and *E. coli* extracts and generally are equivalent to the method disclosed herein. Expression using an expression system can include any of a variety of characteristics including, without limitation, inducible expression, non-inducible expression, constitutive expression, viral-mediated expression, stably-integrated expression, and transient expression. Expression systems that include well-characterized vectors, reagents, conditions and cells are well-established and are readily available from commercial vendors that include, without limitation, Ambion, Inc. Austin, TX; BD Biosciences-Clontech, Palo Alto, CA; BD Biosciences Pharmingen, San Diego, CA; Invitrogen, Inc, Carlsbad, CA; QIAGEN, Inc., Valencia, CA; Roche Applied Science, Indianapolis, IN; and Stratagene, La Jolla, CA. Non-limiting examples on the selection and use of appropriate heterologous expression systems are described in *e.g*., PROTEIN EXPRESSION. A PRACTICAL APPROACH (S. J. Higgins and B. David Hames eds., Oxford University Press, 1999); Joseph M. Fernandez & James P. Hoeffler, GENE EXPRESSION SYSTEMS. USING NATURE FOR THE ART OF EXPRESSION (Academic Press, 1999); and Meena Rai & Harish Padh, Expression Systems for Production of Heterologous Proteins, 80(9) CURRENT SCIENCE 1121-1128, (2001), which are hereby incorporated by reference. These protocols are routine procedures well within the scope of one skilled in the art and from the teaching herein.

Thus, in an aspect, a nucleic acid molecule disclosed in the present specification is operably linked to control sequences from a viral expression vector useful for expressing an encoded active BoNT/A in a viral expression system. Non-limiting examples of viral expression vector include lentivirus vectors, fowl pox virus, pseudorabies virus, retrovirus vectors, semliki forest virus vectors, sindbis virus vectors, vaccinia virus vectors, and adenovirus vectors. In an aspect of this embodiment, an expression construct comprises a viral expression vector operably linked to a codified open reading frame providing increased expression of an encoded active BoNT/A in a mammalian cell.

In another embodiment disclosed in the present invention, a nucleic acid molecule disclosed in the present specification is operably linked to control sequences from a prokaryotic expression vector useful for expressing an encoded active BoNT/A in a prokaryotic cell. This is in particular prokaryotic expression vector namely an *Escherichia coli* expression vector. Reference aspects include a *Bacillus subtilis* expression vector, a *Bacillus licheniformis* expression vector, a *Bacteroides fragilis* Expression vector, a *Clostridia perfringens* expression vector, a *Clostridia difficile* expression vector, a *Caulobacter crescentus* expression vector, a *Lactococcus lactis* expression vector, a *Methylobacterium extorquens* expression vector, a *Neisseria meningirulls* expression vector, *a Neisseria meningitidis* expression vector, a *Pseudomonas fluorescens* expression vector and *a Salmonella typhimurium* expression vector. In an aspect of this embodiment, an expression construct comprises a prokaryotic expression vector operably linked to a modified open reading frame providing increased expression of an encoded active BoNT/A in a prokaryotic cell. In an aspect of this embodiment, an expression construct comprises a pET28 expression vector and a modified open reading frame providing increased expression of an encoded active BoNT/A in an *E. coli* cell. In another aspect of this embodiment, an expression construct comprises a pET28 expression vector operably linked to a modified open reading frame of SEQ ID NO: 3 providing increased expression of the encoded active BoNT/A in an *E. coli* cell. In another aspect of this embodiment, an expression construct comprises a pET28 expression vector operably linked to a modified open reading frame of SEQ ID NO: 4 providing increased expression of the encoded active BoNT/A in an *E. coli* cell. In another aspect of this embodiment, an expression construct comprises a pET28 expression vector operably linked to a modified open reading frame of SEQ ID NO: 5 providing increased expression of the encoded active BoNT/A in an *E. coli* cell. In another aspect of this embodiment, an expression construct comprises a pET28 expression vector operably linked to a modified open reading frame of SEQ ID NO: 6 providing increased expression of the encoded active BoNT/A in an *E. coli* cell. In another aspect of this embodiment, an expression construct comprises a pET29 expression vector operably linked to a modified open reading frame of SEQ ID NO: 3 providing increased expression of the encoded active BoNT/A in an *E. coli* cell. In another aspect of this embodiment, an expression construct comprises a pET29 expression vector operably linked to a modified open reading frame of SEQ ID NO: 4 providing increased expression of the encoded active BoNT/A in an *E. coli* cell. In another aspect of this embodiment, an expression construct comprises a pET29 expression vector operably linked to a modified open reading frame of SEQ ID NO: 5 providing increased expression of the encoded active BoNT/A in an *E. coli* cell. In another aspect of this embodiment, an expression construct comprises a pET29 expression vector operably linked to a modified open reading frame of SEQ ID NO: 6 providing increased expression of the encoded active BoNT/A in an *E. coli* cell. In another aspect of this embodiment, an expression construct comprise a pRSET expression vector operably linked to a modified open reading frame of SEQ ID NO: 3 providing increased expression of the encoded active BoNT/A in an *E. coli* cell. In another aspect of this embodiment, an expression construct comprises a pRSET expression vector operably linked to a modified open reading frame of SEQ-ID NO: 4 providing increased expression of the encoded active BONT/A in an *E. coli* cell. In another aspect of this embodiment, an expression construct comprises a pRSET expression vector operably linked to a modified open reading frame of SEQ ID NO: 5 providing increased expression of the encoded active BoNT/A in an *E. coli* cell. In another aspect of this embodiment, an expression construct comprises a pRSET expression vector operably linked to a modified open reading frame of SEQ ID NO: 6 providing increased expression of the encoded active BoNT/A in an *E. coli* cell.

In yet another aspect, expression constructs disclosed in the present specification are operably linked to control sequences from a eukaryotic expression vector useful for expressing an encoded active BoNT/A in an eukaryotic cell. In an aspect of this embodiment, a nucleic acid molecule disclosed in the present specification is operably linked to control sequences from a yeast expression vector useful for expressing an encoded BoNT/A in a yeast cell. Non-limiting examples of yeast expression vectors include a *Pichia pastoris* expression vector, a *Pichia methanolica* expression vector, a *Pichia angusta* expression vector, a *Schizosaccharomyces pombe* expression vector, a *Saccharomyces cerevisiae* expression vector and a *Yarrowia lipolytica* expression vector. In an aspect of this embodiment, an expression construct comprises a yeast expression vector operably linked to a modified open reading frame providing increased expression of an encoded active BoNT/A in a yeast cell. In an aspect of this embodiment, an expression construct comprises a pPICZ A expression vector and a modified open reading frame providing increased expression of an encoded active BoNT/A in a *P. pastoris* cell. In another aspect of this embodiment, an expression construct comprises a pPICZ A expression vector operably linked to a modified open reading frame of SEQ ID NO: 34 providing increased expression of the encoded active BoNT/A in a *P. pastoris* cell. In another aspect of this embodiment, an expression construct comprises a pPICZ A expression vector operably linked to a modified open reading frame of SEQ ID NO: 35 providing increased expression of the encoded active BoNT/A in a *P. pastoris* cell. In another aspect of this embodiment, an expression construct comprises a pPICZ A expression vector operably linked to a modified open reading frame of SEQ ID NO: 36 providing increased expression of the encoded active BoNT/A in a *P*. *pastoris* cell. In another aspect of this embodiment, an expression construct comprises a pMET expression vector and a modified open reading frame providing increased expression of an encoded active BoNT/A in a *P*. *methanolica* cell. In another aspect of this embodiment, an expression construct comprises a pMET expression vector operably linked to a modified open reading frame of SEQ ID NO: 34 providing increased expression of the encoded active BoNT/A in a *P*. *methanolica* cell. In another aspect of this embodiment, an expression construct comprises a pMET expression vector operably linked to a modified open reading frame of SEQ ID NO: 35 providing increased expression of the encoded active BoNT/A in a *P*. *methanolica* cell. In another aspect of this embodiment, an expression construct comprises a pMET expression vector operably linked to a modified open reading frame of SEQ ID NO: 36 providing increased expression of the encoded active BoNT/A in a *P. methanolica* cell. In yet another aspect of this embodiment, an expression construct comprises a pYES2.1 expression vector and a modified open reading frame providing increased expression of an encoded active BoNT/A in a *S. cerevisiae* cell. In another aspect of this embodiment, an expression construct comprises a pYES2.1 expression vector operably linked to a modified open reading frame of SEQ ID NO: 37 providing increased expression of the encoded active BoNT/A in a *S*. *cerevisiae* cell. In another aspect of this embodiment, an expression construct comprises a pYES2.1 expression vector operably linked to a modified open reading frame of SEQ ID NO: 38 providing increased expression of the encoded active BoNT/A in a *S. cerevisiae* cell. In another aspect of this embodiment, an expression construct comprises a pYES2.1 expression vector operably linked to a modified open reading frame of SEQ ID NO: 39 providing increased expression of the encoded active BoNT/A in a *S*. *cerevisiae* cell.

In yet another aspect, a nucleic acid molecule disclosed in the present specification is operably linked to control sequences from an insect expression vector useful for expressing an encoded active BoNT/A in an insect cell. Non-limiting examples of an insect expression vector include a *Spodoptera frugiperda* expression vector, a *Trichoplusia ni* expression vector, a *Drosophila melanogaster* expression vector and a *Manduca sexta* expression vector. In an aspect of this embodiment, an expression construct comprises an insect expression vector operably linked to a modified open reading frame providing increased expression of an encoded active BoNT/A in an insect cell or cell line derived from an insect cell. In an aspect of this embodiment, an expression construct comprises a pFastBac™HT expression vector and a modified open reading frame providing increased expression of an encoded active BoNT/A in an insect cell line, such as, *e.g*., Sf9, Sf21 and High-Five. In another aspect of this embodiment, an expression construct comprises a pFastBac™HT expression vector operably linked to a modified open reading frame of SEQ ID NO: 61 providing increased expression of the encoded active BoNT/A in an insect cell line, such as, *e.g.,* Sf9, Sf21 and High-Five. In another aspect of this embodiment, an expression construct comprises a pFastBac™HT expression vector operably linked to a modified open reading frame of SEQ ID NO: 62 providing increased expression of the encoded active BoNT/A in an insect cell line, such as, *e.g*., Sf9, Sf21 and High-Five. In another aspect of this embodiment, an expression construct comprises a pFastBac™HT expression vector operably linked to a modified open reading frame of SEQ ID NO: 63 providing increased expression of the encoded active BoNT/A in an insect cell line, such as, *e.g*., Sf9, Sf21 and High-Five. In another aspect of this embodiment, an expression construct comprises a pBACgus3 expression vector and a modified open reading frame providing increased expression of an encoded active BoNT/A in an insect cell line, such as, *e.g*., Sf9, Sf21 and High-Five. In another aspect of this embodiment, an expression construct comprises a pBACgus3 expression vector operably linked to a modified open reading frame of SEQ ID NO: 61 providing increased expression of the encoded active BoNT/A in an insect cell line, such as, *e.g*., Sf9, Sf21 and High-Five. In another aspect of this embodiment, an expression construct comprises a pBACgus3 expression vector operably linked to a modified open reading frame of SEQ ID NO: 62 providing increased expression of the encoded active BoNT/A in an insect cell line, such as, *e.g*., Sf9, Sf21 and High-Five. In another aspect of this embodiment, an expression construct comprises a pBACgus3 expression vector operably linked to a modified open reading frame of SEQ ID NO: 63 providing increased expression of the encoded active BoNT/A in an insect cell line, such as, *e.g*., Sf9, Sf21 and High-Five.

In an aspect, an expression construct comprises a pMT/BiP-V5-His/GFP expression vector and a modified open reading frame providing increased expression of an encoded active BoNT/A in an insect cell line, such as, *e.g*., Schneider's Drosophila line 2 (S2) and Kc. In another aspect of this embodiment, an expression construct comprises a pMT/BiP-V5-His/GFP expression vector operably linked to a modified open reading frame of SEQ ID NO: 58 providing increased expression of the encoded active BoNT/A in an insect cell line, such as, *e.g*., Schneider's Drosophila line 2 (S2) and Kc. In another aspect of this embodiment, an expression construct comprises a pMT/BiP-V5-His/GFP expression vector operably linked to a modified open reading frame of SEQ ID NO: 59 providing increased expression of the encoded active BoNT/A in an insect cell line, such as, *e.g*., Schneider's Drosophila line 2 (S2) and Kc. In another aspect of this embodiment, an expression construct comprises a pMT/BiP-V5-His/GFP expression vector operably linked to a modified open reading frame of SEQ ID NO: 60 providing increased expression of the encoded active BoNT/A in an insect cell line, such as, *e.g*., Schneider's Drosophila line 2 (S2) and Kc.

In yet another aspect, a nucleic acid molecule disclosed in the present specification is operably linked to control sequences from a mammalian expression vector useful for expressing an encoded active BoNT/A in a mammalian cell or cell line derived from a mammalian cell. Expression of a BoNT/A from a mammalian expression vectors can be under the control of a constitutive, tissue-specific, cell-specific or inducible promoter element, enhancer element or both. Non-limiting examples of mammalian expression vectors include a mouse expression vector, a rat expression vector, a hamster expression vector, a porcine expression vector, a bovine expression vector, an equine expression vector, a primate expression vector and a human expression vector. Specific expresion vectors include, without limitation, pCMV-Script, pCMVTNT, pDisplay, pSECTag, pSECTag2, pVAX1 and pQB125. In an aspect of this embodiment, an expression construct comprises a mammalian expression vector operably linked to a modified open reading frame providing increased expression of an encoded active BoNT/A in a mammalian cell or cell line derived from a mammalian cell.

In an aspect an expression construct comprises a mouse expression vector operably linked to a modified open reading frame providing increased expression of an encoded active BoNT/A in a mouse cell or cell line derived from a mouse cell. In an aspect of this embodiment, an expression construct comprises a pSECTag2expression vector and a modified open reading frame providing increased expression of an encoded active BoNT/A in a mouse cell line, such as, *e.g*., 10T1/2, BALB/3T3, L-M, NB4 1A3, NIE-115, NG108-15, NIH3T3, NCTC and Neuro 2A. In another aspect of this embodiment, an expression construct comprises a pSECTag2 expression vector operably linked to a modified open reading frame of SEQ ID NO: 76 providing increased expression of the encoded active BoNT/A in a mouse cell line, such as, *e.g*., 10T1/2, BALB/3T3, L-M, NB4 1A3, NIE-115, NG108-15, NIH3T3, NCTC and Neuro 2A. In another aspect of this embodiment, an expression construct comprises a pSECTag2 expression vector operably linked to a modified open reading frame of SEQ ID NO: 77 providing increased expression of the encoded active BoNT/A in a mouse cell line, such as, *e.g*., 10T1/2, BALB/3T3, L-M, NB4 1A3, NIE-115, NG108-15, NIH3T3, NCTC and Neuro 2A. In another aspect of this embodiment, an expression construct comprises a pSECTag2 expression vector operably linked to a modified open reading frame of SEQ ID NO: 78 providing increased expression of the encoded active BoNT/A in a mouse cell line, such as, *e.g*., 10T1/2, BALB/3T3, L-M, NB4 1A3, NIE-115, NG108-15, NIH3T3, NCTC and Neuro 2A.

In an aspect, an expression construct comprises a rat expression vector operably linked to a modified open reading frame providing increased expression of an encoded active BoNT/A in a rat cell or cell line derived from a rat cell. In an aspect of this embodiment, an expression construct comprises a pSECTag2 expression vector and a modified open reading frame providing increased expression of an encoded active BoNT/A in a rat cell line, such as, *e.g*., PC12, GH1, GH3, C6 and L2. In another aspect of this embodiment, an expression construct comprises a pSECTag2 expression vector operably linked to a modified open reading frame of SEQ ID NO: 79 providing increased expression of the encoded active BoNT/A in a rat cell line, such as, *e.g*., PC12, GH1, GH3, C6 and L2. In another aspect of this embodiment, an expression construct comprises a pSECTag2 expression vector operably linked to a modified open reading frame of SEQ ID NO: 80 providing increased expression of the encoded active BoNT/A in a rat cell line, such as, *e.g*., PC12, GH1, GH3, C6 and L2. In another aspect of this embodiment, an expression construct comprises a pSECTag2 expression vector operably linked to a modified open reading frame of SEQ ID NO: 81 providing increased expression of the encoded active BoNT/A in a rat cell line, such as, *e.g*., PC12, GH1, GH3, C6 and L2.

In an aspect an expression construct comprises a hamster expression vector operably linked to a modified open reading frame providing increased expression of an encoded active BoNT/A in a hamster cell or cell line derived from a hamster cell. In an aspect of this embodiment, an expression construct comprises a pSECTag2 expression vector and a modified open reading frame providing increased expression of an encoded active BoNT/A in a hamster cell line, such as, *e.g*., CHO and 6E6. In another aspect of this embodiment, an expression construct comprises a pSECTag2 expression vector operably linked to a modified open reading frame of SEQ ID NO: 82 providing increased expression of the encoded active BoNT/A in a hamster cell line, such as, *e.g*., CHO and 6E6. In another aspect of this embodiment, an expression construct comprises a pSECTag2 expression vector operably linked to a modified open reading frame of SEQ ID NO: 83 providing increased expression of the encoded active BoNT/A in a hamster cell line, such as, *e.g*., CHO and 6E6. In another aspect of this embodiment, an expression construct comprises a pSECTag2 expression vector operably linked to a modified open reading frame of SEQ ID NO: 84 providing increased expression of the encoded active BoNT/A in a hamster cell line, such as, *e.g*., CHO and 6E6.

In an aspect, an expression construct comprises a porcine expression vector operably linked to a modified open reading frame providing increased expression of an encoded active BoNT/A in a porcine cell or cell line derived from a porcine cell. In an aspect of this embodiment, an expression construct comprises a pSECTag2 expression vector and a modified open reading frame providing increased expression of an encoded active BoNT/A in a porcine cell line, such as, *e.g*., PK15, LLC-PK1, ST and ESK-4. In another aspect of this embodiment, an expression construct comprises a pSECTag2 expression vector operably linked to a modified open reading frame of SEQ ID NO: 85 providing increased expression of the encoded active BoNT/A in a porcine cell line, such as, *e.g*., PK15, LLC-PK1, ST and ESK-4. In another aspect of this embodiment, an expression construct comprises a pSECTag2 expression vector operably linked to a modified open reading frame of SEQ ID NO: 86 providing increased expression of the encoded active BoNT/A in a porcine cell line, such as, *e.g*., PK15, LLC-PK1, ST and ESK-4. In another aspect of this embodiment, an expression construct comprises a pSECTag2 expression vector operably linked to a modified open reading frame of SEQ ID NO: 87 providing increased expression of the encoded active BoNT/A in a porcine cell line, such as, *e.g*., PK15, LLC-PK1, ST and ESK-4.

In an aspect, an expression construct comprises a bovine expression vector operably linked to a modified open reading frame providing increased expression of an encoded active BoNT/A in a bovine cell or cell line derived from a bovine cell. In an aspect of this embodiment, an expression construct comprises a pSECTag2 expression vector and a modified open reading frame providing increased expression of an encoded active BoNT/A in a bovine cell line, such as, *e.g*., CPAE, BT, SBAC and FB2. In another aspect of this embodiment, an expression construct comprises a pSECTag2 expression vector operably linked to a modified open reading frame of SEQ ID NO: 88 providing increased expression of the encoded active BoNT/A in a bovine cell line, such as, *e.g*., CPAE, BT, SBAC and FB2. In another aspect of this embodiment, an expression construct comprises a pSECTag2 expression vector operably linked to a modified open reading frame of SEQ ID NO: 89 providing increased expression of the encoded active BoNT/A in a bovine cell line, such as, *e.g*., CPAE, BT, SBAC and FB2. In another aspect of this embodiment, an expression construct comprises a pSECTag2 expression vector operably linked to a modified open reading frame of SEQ ID NO: 90 providing increased expression of the encoded active BoNT/A in a bovine cell line, such as, *e.g*., CPAE, BT, SBAC and FB2.

In an aspect, an expression construct comprises an equine expression vector operably linked to a modified open reading frame providing increased expression of an encoded active BoNT/A in an equine cell or cell line derived from an equine cell. In an aspect of this embodiment, an expression construct, comprises a pSECTag2 expression vector and a modified open reading frame providing increased expression of an encoded active BoNT/A in an equine cell line, such as, *e.g*., NBL-6. In another aspect of this embodiment, an expression construct comprises a pSECTag2 expression vector operably linked to a modified open reading frame of SEQ ID NO: 91 providing increased expression of the encoded active BoNT/A in an equine cell line, such as, *e.g*., NBL-6. In another aspect of this embodiment, an expression construct comprises a pSECTag2 expression vector operably linked to a modified open reading frame of SEQ ID NO: 92 providing increased expression of the encoded active BoNT/A in an equine cell line, such as, *e.g*., NBL-6. In another aspect of this embodiment, an expression construct comprises a pSECTag2 expression vector operably linked to a modified open reading frame of SEQ ID NO: 93 providing increased expression of the encoded active BoNT/A in an equine cell line, such as, *e.g*., NBL-6.

In an aspect, an expression construct comprises a primate expression vector operably linked to a modified open reading frame providing increased expression of an encoded active BoNT/A in a primate cell or cell line derived from a primate cell. In an aspect of this embodiment, an expression construct comprises a pSECTag2 expression vector and a modified open reading frame providing increased expression of an encoded active BoNT/A in a primate cell line, such as, *e.g*., COS-1, COS-7 and VV-1. In another aspect of this embodiment, an expression construct comprises a pSECTag2 expression vector operably linked to a modified open reading frame of SEQ ID NO: 94 providing increased expression of the encoded active BoNT/A in a primate cell line, such as, *e.g*., COS-1, COS-7 and VV-1. In another aspect of this embodiment, an expression construct comprises a pSECTag2 expression vector operably linked to a modified open reading frame of SEQ ID NO: 95 providing increased expression of the encoded active BoNT/A in a primate cell line, such as, *e.g*., COS-1, COS-7 and W-1. In another aspect of this embodiment, an expression construct comprises a pSECTag2 expression vector operably linked to a modified open reading frame of SEQ ID NO: 96 providing increased expression of the encoded active BoNT/A in a primate cell line, such as, *e.g*., COS-1, COS-7 and VV-1.

In an aspect, an expression construct comprises a human expression vector operably linked to a modified open reading frame providing increased expression of an encoded active BoNT/A in a human cell or cell line derived from a human cell. In an aspect of this embodiment, an expression construct comprises a pSECTag2 expression vector and a modified open reading frame providing increased expression of an encoded active BoNT/A in a primate cell line, such as, *e.g*., SH-SY5Y, SK-N-DZ, SK-N-F1, SK-N-SH, BE (2)-C, HeLa, HEK 293, MCF-7, HepG2, HL-60, IMR-32, SW-13 and CHP3. In another aspect of this embodiment, an expression construct comprises a pSECTag2 expression vector operably linked to a modified open reading frame of SEQ ID NO:97 providing increased expression of the encoded active BoNT/A in a primate cell line, such as, *e.g*., SH-SY5Y, SK-N-DZ, SK-N-F1, SK-N-SH, BE (2)-C, HeLa, HEK 293, MCF-7, HepG2, HL-60, IMR-32, SW-13 and CHP3. In another aspect of this embodiment, an expression construct comprises a pSECTag2 expression vector operably linked to a modified open reading frame of SEQ ID NO: 98 providing increased expression of the encoded active BoNT/A in a primate cell line, such as, *e.g*., SH-SY5Y, SK-N-DZ, SK-N-F1, SK-N-SH, BE (2)-C, HeLa, HEK 293, MCF-7, HepG2, HL-60, IMR-32, SW-13 and CHP3. In another aspect of this embodiment, an expression construct comprises a pSECTag2 expression vector operably linked to a modified open reading frame of SEQ ID NO: 99 providing increased expression of the encoded active BoNT/A in a primate cell line, such as, *e.g*., SH-SY5Y, SK-N-DZ, SK-N-F1, SK-N-SH, BE (2)-C, HeLa, HEK 293, MCF-7, HepG2, HL-60, IMR-32, SW-13 and CHP3.

Reference Aspects further provide cells comprising an expression construct disclosed in the present specification. It is envisioned that a cell can include, without limitation, a prokaryotic cell containing a prokaryotic expression construct useful for expressing a nucleic acid molecule comprising a modified open reading frame providing increased expression of the encoded active BoNT/A in a prokaryotic cell; a yeast cell containing a yeast expression construct useful for expressing a nucleic acid molecule comprising a modified open reading frame providing increased expression of the encoded active BoNT/A in a yeast cell; an insect cell containing an insect expression construct useful for expressing a nucleic acid molecule comprising a modified open reading frame providing increased expression of the encoded active BoNT/A in an insect cell; and a mammalian cell containing a mammalian expression construct useful for expressing a nucleic acid molecule comprising a modified open reading frame providing increased expression of the encoded active BoNT/A in a mammalian cell.

The cells disclosed in the present specification include, in part, an expression construct. It is envisioned that any and all expression constructs disclosed in the present specification can be used. Thus, aspects of this embodiment include, without limitation, cells comprising a viral expression vector operably linked to a modified open reading frame providing increased expression of an encoded active BoNT/A in a mammalian cell; a prokaryotic expression vector operably linked to a modified open reading frame providing increased expression of an encoded active BoNT/A in a prokaryotic cell; cells comprising a yeast expression vector operably linked to a modified open reading frame providing increased expression of an encoded active BoNT/A in a yeast cell; cells comprising a slime mold expression vector operably linked to a modified open reading frame providing increased expression of an encoded active BoNT/A in an slime mold cell; cells comprising a plant expression vector operably linked to a modified open reading frame providing increased expression of an encoded active BoNT/A in a plant cell or cell line derived from a plant cell; cells comprising an insect expression vector operably linked to a modified open reading frame providing increased expression of the encoded active BoNT/A in an insect cell or cell line derived from an insect cell; cells comprising a fish expression vector operably linked to a modified open reading frame providing increased expression of an encoded active BoNT/A in an fish cell or cell line derived from a fish cell; cells comprising an amphibian expression vector operably linked to a modified open reading frame providing increased expression of an encoded active BoNT/A in an amphibian cell or cell line derived from an amphibian cell; cells comprising a bird expression vector operably linked to a modified open reading frame providing increased expression of an encoded active BoNT/A in a bird cell or cell line derived from a bird cell; and cells comprising a mammalian expression vector operably linked to a modified open reading frame providing increased expression of an encoded active BoNT/A in a mammalian cell or cell line derived from a mammalian cell, such as, *e.g*., mouse, rat, hamster, porcine, bovine, equine, primate and human. Other aspects of this embodiment include, without limitation, expression constructs comprising a modified open reading frame that comprises any one of SEQ ID NO: 3 through SEQ ID NO: 99, SEQ ID NO: 110, SEQ ID NO: 112 and SEQ ID NO: 122 through SEQ ID NO: 125.

The cells disclosed in the present specification include, in part, a heterologous cell. It is envisioned that any and all heterologous cells disclosed in the present specification can be used. Thus, aspects of this embodiment include, without limitation, prokaryotic cells including, without limitation, strains of aerobic, microaerophilic, capnophilic, facultative, anaerobic, gram-negative and gram-positive bacterial cells such as those derived from, *e.g., Escherichia coli, Bacillus subtilis, Bacillus licheniformis, Bacteroides fragilis, Clostridia perfringens, Clostridia difficile, Caulobacter crescentus, Lactococcus lactis, Methylobacterium extorquens, Neisseria meningirulls, Neisseria meningitidis, Pseudomonas fluorescens* and *Salmonella typhimurium;* and eukaryotic cells including, without limitation, yeast strains, such as, *e.g.,* those derived from *Pichia pastoris, Pichia methanolica, Pichia angusta, Schizosaccharomyces pombe, Saccharomyces cerevisiae* and *Yarrowia lipolytica;* slime mold strains, such as, *e.g.,* those derived from, *e.g., Dictyostelium discoideum;* plant cells and cell lines derived from plant cells, such as, *e.g.,* those derived from species of monocots, species of dicots, *Zea mays* and *Arabidopsis thaliana;* insect cells and cell lines derived from insects, such as, *e.g.,* those derived from *Spodoptera frugiperda, Trichoplusia ni, Drosophila melanogaster* and *Manduca sexta;* fish cells and cell lines derived from fish cells, such as, *e.g.,* those derived from *Denio renia;* amphibian cells and cell lines derived from amphibian cells, such as, *e.g.,* those derived from *Xenopus laevis* and. *Xenopus tropicalis;* bird cells and cell lines derived from bird cells, such as, *e.g.,* those derived from *Gallus gallus;* mammalian cells and cell lines derived from mammalian cells, such as, *e.g*., those derived from mouse, rat, hamster, porcine, bovine, equine, primate and human. Cell lines may be obtained from the American Type Culture Collection (2004), at URL address www.atcc.org; European Collection of Cell Cultures (2204), at URL address www.ecacc.org.uk; and the German Collection of Microorganisms and Cell Cultures (2004), at URL address www.dsmz.de. Non-limiting examples of specific protocols for selecting, making and using an appropriate cell line are described in *e.g*., INSECT CELL CULTURE ENGINEERING (Mattheus F. A. Goosen et al. eds., Marcel Dekker, 1993); INSECT CELL CULTURES: FUNDAMENTAL AND APPLIED ASPECTS (J. M. Vlak et al. eds., Kluwer Academic Publishers, 1996); Maureen A. Harrison & Ian F. Rae, GENERAL TECHNIQUES OF CELL CULTURE (Cambridge University Press, 1997); CELL AND TISSUE CULTURE: LABORATORY PROCEDURES (Alan Doyle et al eds., John Wiley and Sons, 1998); R. Ian Freshney, CULTURE OF ANIMAL CELLS: A MANUAL OF BASIC TECHNIQUE (Wiley-Liss, 4th ed. 2000); ANIMAL CELL CULTURE: A PRACTICAL APPROACH (John R. W. Masters ed., Oxford University Press, 3rd ed. 2000); MOLECULAR CLONING A LABORATORY MANUAL, supra, (2001); BASIC CELL CULTURE: A PRACTICAL APPROACH (John M. Davis, Oxford Press, 2nd ed. 2002); and CURRENT PROTOCOLS IN MOLECULAR BIOLOGY, supra, (2004). These protocols are routine procedures within the scope of one skilled in the art and from the teaching herein.

It is envisioned that any and all methods for introducing an expression construct disclosed in the present specification into a cell can be used. A cell disclosed in the present specification can maintain an expression construct transiently or stably. Stably-maintained constructs may be extra-chromosomal and replicate autonomously, or they may be integrated into the chromosomal material of the cell and replicate non-autonomously. Methods useful for introducing a nucleic acid molecule into a cell including, without limitation, calcium phosphate-mediated, DEAE dextran-mediated, lipid-mediated, polybrene-mediated, polylysine-mediated, viral-mediated, microinjection, protoplast fusion, biolistic, and electroporation, see, *e.g*., Introducing Cloned Genes into Cultured Mammalian Cells, pp. 16.1-16.62 (Sambrook & Russell, eds., MOLECULAR CLONING: A LABORATORY MANUAL, Vol. 3, 3rd ed. 2001). One skilled in the art understands that selection of a specific method to introduce an expression construct into a cell will depend, in part, on whether the cell will transiently contain an expression construct or whether the cell will stably contain an expression construct. These protocols are routine procedures within the scope of one skilled in the art and from the teaching herein.

In an aspect of this embodiment, a chemical-mediated method, termed transfection, is used to introduce a construct expressing an active BoNT/A into a heterologous cell. In chemical-mediated methods of transfection the chemical reagent forms a complex with the nucleic acid that facilitates its uptake into the cells. Such chemical reagents include, without limitation, calcium phosphate-mediated, see, *e.g*., Martin Jordan & Florian Worm, Transfection of adherent and suspended cells by calcium phosphate, 33(2) Methods 136-143 (2004); diethyl-aminoethyl (DEAE) dextran-mediated, lipid-mediated, cationic polymer-mediated like polyethyleneimine (PEI)-mediated and polylysine-mediated and polybrene-mediated, see, *e.g*., Chun Zhang et al., Polyethylenimine strategies for plasmid delivery to brain-derived cells, 33(2) Methods 144-150 (2004). Such chemical-mediated delivery systems can be prepared by standard methods and are commercially available, see, *e.g*., CellPhect Transfection Kit (Amersham Biosciences, Piscataway, NJ); Mammalian Transfection Kit, Calcium phosphate and DEAE Dextran, (Stratagene, Inc., La Jolla, CA); Lipofectamine™ Transfection Reagent (Invitrogen, Inc., Carlsbad, CA); ExGen 500 Transfection kit (Fermentas, Inc., Hanover, MD), and SuperFect and Effectene Transfection Kits (Qiagen, Inc., Valencia, CA).

In another aspect of this embodiment, a physical-mediated method is used to introduce a construct expressing an active BoNT/A into a heterologous cell. Physical reagents include, without limitation, electroporation, biolistic and microinjection. Biolistics and microinjection techniques perforate the cell wall in order to introduce the nucleic acid molecule into the cell, see, *e.g*., Jeike E. Biewenga et al., Plasmid-mediated gene transfer in neurons using the biolistics technique, 71 (1) J. Neurosci. Methods. 67-75 (1997); and John O'Brien & Sarah C. R. Lummis, Biolistic and diolistic transfection: using the gene gun to deliver DNA and lipophilic dyes into mammalian cells, 33(2) Methods 121-125 (2004). Electroporation, also termed electropermeabilization, uses brief, high-voltage, electrical pulses to create transient pores in the membrane through which the nucleic acid molecules enter and can be used effectively for stable and transient transfections of all cell types, see, *e.g*., M. Golzio et al., In vitro and in vivo electric field-mediated permeabilization, gene transfer, and expression, 33(2) Methods 126-135 (2004); and Oliver Greschet al., New non-viral method for gene transfer into primary cells, 33(2) Methods 151-163 (2004).

In another aspect of this embodiment, a viral-mediated method, termed transduction, is used to introduce a construct expressing an active BoNT/A into a heterologous cell. In viral-mediated methods of transient transduction, the process by which viral particles infect and replicate in a host cell has been manipulated in order to use this mechanism to introduce a nucleic acid molecule into the cell. Viral-mediated methods have been developed from a wide variety of viruses including, without limitation, retroviruses, adenoviruses, adeno-associated viruses, herpes simplex viruses, picomaviruses, alphaviruses and baculoviruses, see, *e.g*., Armin Blesch, Lentiviral and MLV based retroviral vectors for ex vivo and in vivo gene transfer, 33(2) Methods 164-172 (2004); and Maurizio Federico, From lentiviruses to lentivirus vectors, 229 Methods Mol. Biol. 3-15 (2003); E. M. Poeschla, Non-primate lentiviral vectors, 5(5) Curr. Opin. Mol. Ther. 529-540 (2003); Karim Benihoud et al, Adenovirus vectors for gene delivery, 10(5) Curr. Opin. Biotechnol. 440-447 (1999); H. Bueler, Adeno-associated viral vectors for gene transfer and gene therapy, 380(6) Biol. Chem. 613-622 (1999); Chooi M. Lai et al., Adenovirus and adeno-associated virus vectors, 21 (12) DNA Cell Biol. 895-913 (2002); Edward A. Burton et al., Gene delivery using herpes simplex virus vectors, 21(12) DNA Cell Biol. 915-936 (2002); Paola Grandi et al., Targeting HSV amplicon vectors, 33(2) Methods 179-186 (2004); Ilya Frolov et al., Alphavirus-based expression vectors: strategies and applications, 93(21) Proc. Natl. Acad. Sci. U. S. A. 11371-11377 (1996); Markus U. Ehrengruber, Alphaviral gene transfer in neurobiology, 59(1) Brain Res. Bull. 13-22 (2002); Thomas A. Kost & J. Patrick Condreay, Recombinant baculoviruses as mammalian cell gene-delivery vectors, 20(4) Trends Biotechnol. 173-180 (2002); and A. Huser & C. Hofmann, Baculovirus vectors: novel mammalian cell gene-delivery vehicles and their applications, 3(1) Am. J. Pharmacogenomics 53-63 (2003).

Adenoviruses, which are non-enveloped, double-stranded DNA viruses, are often selected for mammalian cell transduction because adenoviruses handle relatively large nucleic acid molecules of about 36 kd, are produced at high titer, and can efficiently infect a wide variety of both dividing and non-dividing cells, see, *e.g*., Wim T. J. M. C. Hermens et al., Transient gene transfer to neurons and glia: analysis of adenoviral vector performance in the CNS and PNS, 71(1) J. Neurosci. Methods 85-98 (1997); and Hiroyuki Mizuguchi et al., Approaches for generating recombinant adenovirus vectors, 52(3) Adv. Drug Deliv. Rev. 165-176 (2001). Transduction using adenoviral-based system do not support prolonged protein expression because the nucleic acid molecule is carried from an episome in the cell nucleus, rather than being integrated into the host cell chromosome. Adenovirual vector systems and specific protocols for how to use such vectors are disclosed in, *e.g*., ViraPower™ Adenoviral Expression System (Invitrogen, Inc., Carlsbad, CA) and ViraPower™ Adenoviral Expression System Instruction Manual 25-0543 version A, Invitrogen, Inc., (Jul. 15, 2002); and AdEasy™ Adenoviral Vector System (Stratagene, Inc., La Jolla, CA) and AdEasy™ Adenoviral Vector System Instruction Manual 064004f, Stratagene, Inc..

Nucleic acid molecule delivery can also use single-stranded RNA retroviruses, such as, *e.g*., oncoretroviruses and lentiviruses. Retroviral-mediated transduction often produce transduction efficiencies close to 100%, can easily control the proviral copy number by varying the multiplicity of infection (MOI), and can be used to either transiently or stably transduce cells, see, *e.g*., Tiziana Tonini et al., Transient production of retroviral- and lentiviral-based vectors for the transduction of Mammalian cells, 285 Methods Mol. Biol. 141-148 (2004); Armin Blesch, Lentiviral and MLV based retroviral vectors for ex vivo and in vivo gene transfer, 33(2) Methods 164-172 (2004); Félix Recillas-Targa, Gene transfer and expression in mammalian cell lines and transgenic animals, 267 Methods Mol. Biol. 417-433 (2004); and Roland Wolkowicz et al., Lentiviral vectors for the delivery of DNA into mammalian cells, 246 Methods Mol. Biol. 391-411 (2004). Retroviral particles consist of an RNA genome packaged in a protein capsid, surrounded by a lipid envelope. The retrovirus infects a host cell by injecting its RNA into the cytoplasm along with the reverse transcriptase enzyme. The RNA template is then reverse transcribed into a linear, double stranded cDNA that replicates itself by integrating into the host cell genome. Viral particles are spread both vertically (from parent cell to daughter cells via the provirus) as well as horizontally (from cell to cell via virions). This replication strategy enables long-term persist expression since the nucleic acid molecules of interest are stably integrated into a chromosome of the host cell, thereby enabling long-term expression of the protein. For instance, animal studies have shown that lentiviral vectors injected into a variety of tissues produced sustained protein expression for more than 1 year, see, *e.g*., Luigi Naldini et al., In vivo gene delivery and stable transduction of non-dividing cells by a lentiviral vector, 272(5259) Science 263-267 (1996). The Oncoretroviruses-derived vector systems, such as, *e.g*., Moloney murine leukemia virus (MoMLV), are widely used and infect many different non-dividing cells. Lentiviruses can also infect many different cell types, including dividing and non-dividing cells and possess complex envelope proteins, which allows for highly specific cellular targeting.

Retroviral vectors and specific protocols for how to use such vectors are disclosed in, *e.g*., U.S. Patent Nos. Manfred Gossen & Hermann Bujard, Tight control of gene expression in eukaryotic cells by tetracycline-responsive promoters, U.S. Patent No. 5,464,758 (Nov. 7, 1995) and Hermann Bujard & Manfred Gossen, Methods for regulating gene expression, U.S. Patent No. 5,814,618 (Sep. 29, 1998) David S. Hogness, Polynucleotides encoding insect steroid hormone receptor polypeptides and cells transformed with same, U.S. Patent No. 5,514,578 (May 7, 1996) and David S. Hogness, Polynucleotide encoding insect ecdysone receptor, U.S. Patent 6,245,531 (Jun. 12, 2001); Elisabetta Vegeto et al., Progesterone receptor having C. terminal hormone binding domain truncations, U.S. Patent No. 5,364,791 (Nov. 15, 1994), Elisabetta Vegeto et al., Mutated steroid hormone receptors, methods for their use and molecular switch for gene therapy. U.S. Patent No. 5,874,534 (Feb. 23, 1999) and Elisabetta Vegeto et al., Mutated steroid hormone receptors, methods for their use and molecular switch for gene therapy, U.S. Patent No. 5,935,934 (Aug. 10, 1999). Furthermore, such viral delivery systems can be prepared by standard methods and are commercially available, see, *e.g*., BD™ Tet-Off and Tet-On Gene Expression Systems (BD Biosciences-Clonetech, Palo Alto, CA) and BD™ Tet-Off and Tet-On Gene Expression Systems User Manual, PT3001-1, BD Biosciences Clonetech, (Mar. 14, 2003), GeneSwitch™ System (Invitrogen, Inc., Carlsbad, CA) and GeneSwitch™ System A Mifepristone-Regulated Expression System for Mammalian Cells version D, 25-0313, Invitrogen, Inc., (Nov. 4, 2002); ViraPower™ Lentiviral Expression System (Invitrogen, Inc., Carlsbad, CA) and ViraPower™ Lentiviral Expression System Instruction Manual 25-0501 version E, Invitrogen, Inc., (Dec. 8, 2003); and Complete Control^{®} Retroviral Inducible Mammalian Expression System (Stratagene, La Jolla, CA) and Complete Control^{®} Retroviral Inducible Mammalian Expression System Instruction Manual, 064005e.

Thus, in an aspect, a cell comprises a mammalian cell comprising an expression construct operably linked to a nucleic acid molecule comprising a modified open reading frame providing increased expression of the encoded active BoNT/A in a mammalian cell. In an aspect of this embodiment, a cell comprises a mammalian cell transiently containing an expression construct operably linked to a nucleic acid molecule comprising a modified open reading frame providing increased expression of the encoded active BoNT/A in a mammalian cell. In another aspect of this embodiment, a cell comprises a mammalian cell stably containing an expression construct operably linked to a nucleic acid molecule comprising a modified open reading frame providing increased expression of the encoded active BoNT/A in a mammalian cell. In yet another aspect of this embodiment, an expression construct is a viral expression construct. In further aspect of this embodiment, a viral expression construct is a lentivirus expression construct, a fowl pox virus expression construct, a pseudorabies virus expression construct, a retrovirus expression construct, a semliki forest virus expression construct, a sindbis virus expression construct, a vaccinia virus expression construct, or an adenovirus expression construct. In yet other aspect of this embodiment, a nucleic acid molecule comprises any of the modified open reading frames of SEQ ID NO: 64 through SEQ ID NO: 99.

Thus, in an embodiment, a cell comprises a prokaryotic E.coli cell comprising an expression construct operably linked to a nucleic acid molecule comprising a modified open reading frame providing increased expression of the encoded active BoNT/A in a prokaryotic cell. In an aspect of this embodiment, a cell comprises a prokaryotic cell transiently containing an expression construct operably linked to a nucleic acid molecule comprising a modified open reading frame providing increased expression of the encoded active BoNT/A in a prokaryotic cell. In another aspect of this embodiment, a cell comprises a prokaryotic cell stably containing an expression construct operably linked to a nucleic acid molecule comprising a modified open reading frame providing increased expression of the encoded active BoNT/A in a prokaryotic cell. In a further aspect of this embodiment, a prokaryotic cell is derived from an aerobic bacterium, a microaerophitic bacterium, a capnophilic bacterium, a facultative bacterium, an anaerobic bacterium, a gram-negative bacterium or a gram-positive bacterium. In a further aspect of this embodiment, a prokaryotic cell is a prokaryotic strain derived from *Escherichia coli* In yet another aspect of this embodiment, an expression construct is a prokaryotic expression construct. In yet another aspect of this embodiment, a nucleic acid molecule comprises any of the modified open reading frames of SEQ ID NO: 3 : 110, and 112

In an aspect, a cell comprises an eukaryotic cell comprising an expression construct operably linked to a nucleic acid molecule comprising a modified open reading frame providing increased expression of the encoded active BoNT/A in an eukaryotic cell. In an aspect of this embodiment, a cell comprises an eukaryotic cell transiently containing an expression construct operably linked to a nucleic acid molecule comprising a modified open reading frame providing increased expression of the encoded active BoNT/A in an eukaryotic cell. In another aspect of this embodiment, a cell comprises an eukaryotic cell stably containing an expression construct operably linked to a nucleic acid molecule comprising a modified open reading frame providing increased expression of the encoded active BoNT/A in an eukaryotic cell. In yet another aspect of this embodiment, an expression construct is an eukaryotic expression construct. In yet other aspect of this embodiment, a nucleic acid molecule comprises any of the modified open reading frames of SEQ ID NO: 34 through SEQ ID NO: 99.

In an aspect, a cell comprises a yeast cell comprising an expression construct operably linked to a nucleic acid molecule comprising a modified open reading frame providing increased expression of the encoded active BoNT/A in a yeast cell. In an aspect of this embodiment, a cell comprises a yeast cell transiently containing an expression construct operably linked to a nucleic acid molecule comprising a modified open reading frame providing increased expression of the encoded active BoNT/A in a yeast cell. In another aspect of this embodiment, a cell comprises a yeast cell stably containing an expression construct operably linked to a nucleic acid molecule comprising a modified open reading frame providing increased expression of the encoded active BoNT/A in a yeast cell. In a further aspect of this embodiment, a yeast cell is a yeast strain derived from *Pichia pastoris, Pichia methanolica, Pichia angusta, Schizosaccharomyces pombe, Saccharomyces cerevisiae* or *Yarrowia lipolytica.* In yet another aspect of this embodiment, an expression, construct is a yeast expression construct. In yet another aspect of this embodiment, a nucleic acid molecule comprises any of the modified open reading frames of SEQ ID NO: 34 through SEQ ID NO: 45.

In an aspect, a cell comprises a slime mold cell comprising an expression constructs operably linked to a nucleic acid molecule comprising a modified open reading frame providing increased expression of the encoded active BoNT/A in a slime mold cell. In an aspect of this embodiment, a cell comprises a slime mold cell transiently containing an expression construct operably linked to a nucleic acid molecule comprising a modified open reading frame providing increased expression of the encoded active BoNT/A in a slime mold cell. In another aspect of this embodiment, a cell comprises a slime mold cell stably containing an expression construct operably linked to a nucleic acid molecule comprising a modified open reading frame providing increased expression of the encoded active BoNT/A in a slime mold cell. In a further aspect of this embodiment, a slime mold cell is a slime mold strain derived from *Dictyostelium discoideum.* In yet another aspect of this embodiment, an expression construct is a slime mold expression construct. In yet another aspect of this embodiment, a nucleic acid molecule comprises any of the modified open reading frames of SEQ ID NO: 46 through SEQ ID NO: 48.

In an aspect, a cell comprises a plant cell comprising an expression construct operably linked to a nucleic acid molecule comprising a modified open reading frame providing increased expression of the encoded active BoNT/A in a plant cell. In an aspect of this embodiment, a cell comprises a plant cell transiently containing an expression construct operably linked to a nucleic acid molecule comprising a modified open reading frame providing increased expression of the encoded active BoNT/A in a plant cell. In another aspect of this embodiment, a cell comprises a plant cell stably containing an expression construct operably linked to a nucleic acid molecule comprising a modified open reading frame providing increased expression of the encoded active BoNT/A in a plant cell. In a further aspect of this embodiment, a plant cell is derived from a monocot cell or cell line derived from a monocot cell or a dicot cell or cell line derived from a dicot cell. In a further aspect of this embodiment, a plant cell or cell line derived from a plant cell is from *Zea mays, Arabidopsis thaliana* or *Triticum aestivum* In yet another aspect of this embodiment, an expression construct is a plant expression construct. In yet another aspect of this embodiment, a nucleic acid molecule comprises any of the modified open reading frames of SEQ ID NO: 49 through SEQ ID NO: 57.

In an aspect a cell comprises an insect cell comprising an expression construct operably linked to a nucleic acid molecule comprising a modified open reading frame providing increased expression of the encoded active BoNT/A in an insect cell. In an aspect of this embodiment, a cell comprises an insect cell transiently containing an expression construct operably linked to a nucleic acid molecule comprising a modified open reading frame providing increased expression of the encoded active BoNT/A in an insect cell. In another aspect of this embodiment, a cell comprises an insect cell stably containing an expression construct operably linked to a nucleic acid molecule comprising a modified open reading frame providing increased expression of the encoded active BoNT/A in an insect cell. In a further aspect of this embodiment, an insect cell is an insect strain derived from *Spodoptera frugiperda, Trichoplusia ni, Drosophila melanogaster or Manduca sexta.* In a further aspect of this embodiment, an insect cell is an insect cell line derived from Sf9, Sf21, High-five, S2 and Kc. In yet another aspect of this embodiment, an expression construct is an insect expression construct. In yet another aspect of this embodiment, a nucleic acid molecule comprises any of the modified open reading frames of SEQ ID NO: 58 through SEQ ID NO: 63. In additional aspects of this embodiment, a Sf9 cell line contains an expression construct operably linked to a nucleic acid molecule comprising a modified open reading frame of SEQ ID NO: 61, SEQ ID NO: 62 or SEQ ID NO: 63; a Sf21 cell line contains an expression construct operably linked to a nucleic acid molecule comprising a modified open reading frame of SEQ ID NO: 61, SEQ ID NO: 62 or SEQ ID NO: 63; a High-Five cell line contains an expression construct operably linked to a nucleic acid molecule comprising a modified open reading frame of SEQ ID NO: 61, SEQ ID NO: 62 or SEQ ID NO: 63; a S2 cell line contains an expression construct operably linked to a nucleic acid molecule comprising a modified open reading frame of SEQ ID NO: 58, SEQ ID NO: 59 or SEQ ID NO: 60; or a Kc cell line contains an expression construct operably linked to a nucleic acid molecule comprising a modified open reading frame of SEQ ID NO: 58, SEQ ID NO: 59 or SEQ ID NO: 60.

In an aspect, a cell comprises a fish cell comprising an expression construct operably linked to a nucleic acid molecule comprising a modified open reading frame providing increased expression of the encoded active BoNT/A in a fish cell. In an aspect of this embodiment, a cell comprises a fish cell transiently containing an expression construct operably linked to a nucleic acid molecule comprising a modified open reading frame providing increased expression of the encoded active BoNT/A in a fish cell. In another aspect of this embodiment, a cell comprises a fish cell stably containing an expression construct operably linked to a nucleic acid molecule comprising a modified open reading frame providing increased expression of the encoded active BoNT/A in a fish cell. In a further aspect of this embodiment, a fish cell is a fish cell or cell line derived from a fish cell from *Denio renia.* In yet another aspect of this embodiment, an expression construct is a fish expression construct. In yet another aspect of this embodiment, a nucleic acid molecule comprises any of the modified open reading frames of SEQ ID NO: 64 through SEQ ID NO: 66.

In an aspect, a cell comprises an amphibian cell comprising an expression construct operably linked to a nucleic acid molecule comprising a modified open reading frame providing increased expression of the encoded active BoNT/A in an amphibian cell. In an aspect of this embodiment, a cell comprises an amphibian cell transiently containing an expression construct operably linked to a nucleic acid molecule comprising a modified open reading frame providing increased expression of the encoded active BoNT/A in an amphibian cell. In another aspect of this embodiment, a cell comprises an amphibian cell stably containing an expression construct operably linked to a. nucleic acid molecule comprising a modified open reading frame providing increased expression of the encoded active BoNT/A in an amphibian cell. In a further aspect of this embodiment, an amphibian cell is an amphibian cell or cell line derived from an amphibian cell from *Xenopus laevis.* In a further aspect of this embodiment, an amphibian cell is an amphibian cell or cell line derived from an amphibian cell from *Xenopus tropicalis.* In yet another aspect of this embodiment, an expression construct is an amphibian expression construct. In yet another aspect of this embodiment, a nucleic acid molecule comprises any of the modified open reading frames of SEQ ID NO: 67 through SEQ ID NO: 72.

In an aspect, a cell comprises a bird cell comprising an expression construct operably linked to a nucleic acid molecule comprising a modified open reading frame providing increased expression of the encoded active BoNT/A in a bird cell. In an aspect of this embodiment, a cell comprises a bird cell transiently containing an expression construct operably linked to a nucleic acid molecule comprising a modified open reading frame providing increased expression of the encoded active BoNT/A in a bird cell. In another aspect of this embodiment, a cell comprises a bird cell stably containing an expression construct operably linked to a nucleic acid molecule comprising a modified open reading frame providing increased expression of the encoded active BoNT/A in a bird cell. In a further aspect of this embodiment, a bird cell is a bird cell or cell line derived from a bird cell from *Gallus gallus.* In yet another aspect of this embodiment, an expression construct is a bird expression construct. In yet another aspect of this embodiment, a nucleic acid molecule comprises any of the modified open reading frames of SEQ ID NO: 73 through SEQ ID NO: 75.

In an aspect, a cell comprises a mammalian cell comprising an expression construct operably linked to a nucleic acid molecule comprising a modified open reading frame providing increased expression of the encoded active BoNT/A in a mammalian cell. In an aspect of this embodiment, a cell comprises a mammalian cell transiently containing an expression construct operably linked to a nucleic acid molecule comprising a modified open reading frame providing increased expression of the encoded active BoNT/A in a mammalian cell. In another aspect of this embodiment, a cell comprises a mammalian cell stably containing an expression construct operably linked to a nucleic acid molecule comprising a modified open reading frame providing increased expression of the encoded active BoNT/A in a mammalian cell. In a further aspect of this embodiment, a mammalian cell is a mammalian cell or cell line derived from a mammalian cell from a mouse, a rat, a hamster, a porcine, a bovine, an equine, a primate or a human. In yet another aspect of this embodiment, an expression construct is a mammalian expression construct. In yet another aspect of this embodiment, a nucleic acid molecule comprises any of the modified open reading frames of SEQ ID NO: 76 through SEQ ID NO: 99.

In an aspect, a cell comprises a mouse cell comprising an expression construct operably linked to a nucleic acid molecule comprising a modified open reading frame providing increased expression of the encoded active BoNT/A in a mouse cell. In an aspect of this embodiment, a cell comprises a mouse cell transiently containing an expression construct operably linked to a nucleic acid molecule comprising a modified open reading frame providing increased expression of the encoded active BoNT/A in a mouse cell. In another aspect of this embodiment, a cell comprises a mouse cell stably containing an expression construct operably linked to a nucleic acid molecule comprising a modified open reading frame providing increased expression of the encoded active BoNT/A in a mouse cell. In a further aspect of this embodiment, a mouse cell is a mouse cell or cell line derived from a mouse cell from *M. musculus.* In a further aspect of this embodiment, a mouse cell is a mouse cell line derived from 10T1/2. BALB/3T3, L-M, NB4 1A3, NIE-115, NG108-15, NIH3T3, NCTC or Neuro 2A. In yet another aspect of this embodiment, an expression construct is a mouse expression construct. In yet another aspect of this embodiment, a nucleic acid molecule comprises any of the modified open reading frames of SEQ ID NO: 76 through SEQ ID NO: 78. In additional aspects of this embodiment, a Neuro 2A cell line contains an expression construct operably linked to a nucleic acid molecule comprising a modified open reading frame of SEQ ID NO: 76, SEQ ID NO: 77 or SEQ ID NO: 78; a 10T1/2 cell line contains an expression construct operably linked to a nucleic acid molecule comprising a modified open reading frame of SEQ ID NO: 76, SEQ ID NO: 77 or SEQ ID NO: 78; a BALB/3T3 cell line contains an expression construct operably linked to a nucleic acid molecule comprising a modified open reading frame of SEQ ID NO: 76, SEQ ID NO: 77 or SEQ ID NO: 78; a NG108-15 cell line contains an expression construct operably linked to a nucleic acid molecule comprising a modified open reading frame of SEQ ID NO: 76, SEQ ID NO: 77 or SEQ ID NO: 78; or a NIE-115 cell line contains an expression construct operably linked to a nucleic acid molecule comprising a modified open reading frame of SEQ ID NO: 76, SEQ ID NO: 77 or SEQ ID NO: 78.

In an aspect, a cell comprises a rat cell comprising an expression construct operably linked to a nucleic acid molecule comprising a modified open reading frame providing increased expression of the encoded active BoNT/A in a rat cell. In an aspect of this embodiment, a cell comprises a rat cell transiently containing an expression construct operably linked to a nucleic acid molecule comprising a modified open reading frame providing increased expression of the encoded active BoNT/A in a rat cell. In another aspect of this embodiment, a cell comprises a rat cell stably containing an expression construct operably linked to a nucleic acid molecule comprising a modified open reading frame providing increased expression of the encoded active BoNT/A in a rat cell. In a further aspect of this embodiment, a rat cell is a rat cell or cell line derived from a rat cell from *R. norvegicus.* In a further aspect of this embodiment, a rat cell is a rat cell line derived from PC12, GH1, GH3, C6 or L2. In yet another aspect of this embodiment, an expression construct is a rat expression construct. In yet another aspect of this embodiment, a nucleic acid molecule comprises any of the modified open reading frames of SEQ ID NO: 79 through SEQ ID NO: 81. In additional aspects of this embodiment, a PC12 cell line contains an expression construct operably linked to a nucleic acid molecule comprising a modified open reading frame of SEQ ID NO: 79, SEQ ID NO: 80 or SEQ ID NO: 81; a GH1 cell line contains an expression construct operably linked to a nucleic acid molecule comprising a modified open reading frame of SEQ ID NO: 79, SEQ ID NO: 80 or SEQ ID NO: 81; a GH3 cell line contains an expression construct operably linked to a nucleic acid molecule comprising a modified open reading frame of SEQ ID NO: 79, SEQ ID NO: 80 or SEQ ID NO: 81; a C6 cell line contains an expression construct operably linked to a nucleic acid molecule comprising a modified open reading frame of SEQ ID NO: 79, SEQ ID NO: 80 or SEQ ID NO: 81; or a L2 cell line contains an expression construct operably linked to a nucleic acid molecule comprising a modified open reading frame of SEQ ID NO: 79, SEQ ID NO: 80 or SEQ ID NO: 81.

In an aspect, a cell comprises a hamster cell comprising an expression construct operably linked to a nucleic acid molecule comprising a modified open reading frame providing increased expression of the encoded active BoNT/A in a hamster cell. In an aspect of this embodiment, a cell comprises a hamster cell transiently containing an expression construct operably linked to a nucleic acid molecule comprising a modified open reading frame providing increased expression of the encoded active BoNT/A in a hamster cell. In another aspect of this embodiment, a cell comprises a hamster cell stably containing an expression construct operably linked to a nucleic acid molecule comprising a modified open reading frame providing increased expression of the encoded active BoNT/A in a hamster cell. In a further aspect of this embodiment, a hamster cell is a hamster cell or cell line derived from a hamster cell from *C*. *griseus.* In a further aspect of this embodiment, a hamster cell is a hamster cell line derived from CHO or 6E6. In yet another aspect of this embodiment, an expression construct is a hamster expression construct. In yet another aspect of this embodiment, a nucleic acid molecule comprises any of the modified open reading frames of SEQ ID NO: 82 through SEQ ID NO: 84. In additional aspects of this embodiment, a CHO cell line contains an expression construct operably linked to a nucleic acid molecule comprising a modified open reading frame of SEQ ID NO: 82, SEQ ID NO: 83 or SEQ ID NO: 84; or a 6E6 cell line contains an expression construct operably linked to a nucleic acid molecule comprising a modified open reading frame of SEQ ID NO: 82, SEQ ID NO: 83 or SEQ ID NO: 84.

In an aspect, a cell comprises a porcine cell comprising an expression construct operably linked to a nucleic acid molecule comprising a modified open reading frame providing increased expression of the encoded active BoNT/A in a porcine cell. In an aspect of this embodiment, a cell comprises a porcine cell transiently containing an expression construct operably linked to a nucleic acid molecule comprising a modified open reading frame providing increased expression of the encoded active BoNT/A in a porcine cell. In another aspect of this embodiment, a cell comprises a porcine cell stably containing an expression construct operably linked to a nucleic acid molecule comprising a modified open reading frame providing increased expression of the encoded active BoNT/A in a porcine cell. In a further aspect of this embodiment, a porcine cell is a porcine cell or cell line derived from a porcine cell from *S*. *scrofa.* In a further aspect of this embodiment, a porcine cell is a porcine cell line derived from PK15, LLC-PK1, ST or ESK-4. In yet another aspect of this embodiment, an expression construct is a porcine expression construct. In yet another aspect of this embodiment, a nucleic acid molecule comprises any of the modified open reading frames of SEQ ID NO: 85 through SEQ ID NO: 87. In additional aspects of this embodiment, a PK15 cell line contains an expression construct operably linked to a nucleic acid molecule comprising a modified open reading frame of SEQ ID NO: 85, SEQ ID NO: 86 or SEQ ID NO: 87; a LLC-PK1 cell line contains an expression construct operably linked to a nucleic acid molecule comprising a modified open reading frame of SEQ ID NO: 85, SEQ ID NO: 86 or SEQ ID NO: 87; a ST cell line contains an expression construct operably linked to a nucleic acid molecule comprising a modified open reading frame of SEQ ID NO: 85, SEQ ID NO: 86 or SEQ ID NO: 87; or a ESK-4 cell line contains an expression construct operably linked to a nucleic acid molecule comprising a modified open reading frame of SEQ ID NO: 85, SEQ ID NO: 86 or SEQ ID NO: 87.

In an aspect a cell comprises a bovine cell comprising an expression construct operably linked to a nucleic acid molecule comprising a modified open reading frame providing increased expression of the encoded active BoNT/A in a bovine cell. In an aspect of this embodiment, a cell comprises a bovine cell transiently containing an expression construct operably linked to a nucleic acid molecule comprising a modified open reading frame providing increased expression of the encoded active BoNT/A in a bovine cell. In another aspect of this embodiment, a cell comprises a bovine cell stably containing an expression construct operably linked to a nucleic acid molecule comprising a modified open reading frame providing increased expression of the encoded active BoNT/A in a bovine cell. In a further aspect of this embodiment, a bovine cell is a bovine cell or cell line derived from a bovine cell from *B. taurus.* In a further aspect of this embodiment, a bovine cell is a bovine cell line derived from CPAE, BT, SBAC or FB2. In yet another aspect of this embodiment, an expression construct is a bovine expression construct. In yet another aspect of this embodiment, a nucleic acid molecule comprises any of the modified open reading frames of SEQ ID NO: 88 through SEQ ID NO: 90. In additional aspects of this embodiment, a CPAE cell line contains an expression construct operably linked to a nucleic acid molecule comprising a modified open reading frame of SEQ ID NO: 88, SEQ ID NO: 89 or SEQ ID NO: 90; a BT cell line contains an expression construct operably linked to a nucleic acid molecule comprising a modified open reading frame of SEQ ID NO: 88, SEQ ID NO: 89 or SEQ ID NO: 90; a SBAC cell line contains an expression construct operably linked to a nucleic acid molecule comprising a modified open reading frame of SEQ ID NO: 88, SEQ ID NO: 89 or SEQ ID NO: 90; or a FB2 cell line contains an expression construct operably linked to a nucleic acid molecule comprising a modified open reading frame of SEQ ID NO: 88, SEQ ID NO: 89 or SEQ ID NO: 90.

In an aspect a cell comprises an equine cell comprising an expression construct operably linked to a nucleic acid molecule comprising a modified open reading frame providing increased expression of the encoded active BoNT/A in an equine cell. In an aspect of this embodiment, a cell comprises an equine cell transiently containing an expression construct operably linked to a nucleic acid molecule comprising a modified open reading frame providing increased expression of the encoded active BoNT/A in an equine cell. In another aspect of this embodiment, a cell comprises an equine cell stably containing an expression construct operably linked to a nucleic acid molecule comprising a modified open reading frame providing increased expression of the encoded active BoNT/A in an equine cell. In a further aspect of this embodiment, an equine cell is an equine cell or cell line derived from an equine cell from *E. caballus.* In a further aspect of this embodiment, an equine cell is an equine cell line derived from NBL-6. In yet another aspect of this embodiment, an expression construct is an equine expression construct. In yet another aspect of this embodiment, a nucleic acid molecule comprises any of the modified open reading frames of SEQ ID NO: 91 through SEQ ID NO: 94. In additional aspects of this embodiment, a NBL-6 cell line contains an expression construct operably linked to a nucleic acid molecule comprising a modified open reading frame of SEQ ID NO: 91, SEQ ID NO: 92 or SEQ ID NO: 93.

In an aspect, a cell comprises a primate cell comprising an expression construct operably linked to a nucleic acid molecule comprising a modified open reading frame providing increased expression of the encoded active BoNT/A in a primate cell. In an aspect of this embodiment, a cell comprises a primate cell transiently containing an expression construct operably linked to a nucleic acid molecule comprising a modified open reading frame providing increased expression of the encoded active BoNT/A in a primate cell. In another aspect of this embodiment, a cell comprises a primate cell stably containing an expression construct operably linked to a nucleic acid molecule comprising a modified open reading frame providing increased expression of the encoded active BoNT/A in a primate cell. In a further aspect of this embodiment, a primate cell is a primate cell or cell line derived from a primate cell from *C*. *aethiops.* In a further aspect of this embodiment, a primate cell is a primate cell line derived from COS-1, COS-7 or VV-1. In yet another aspect of this embodiment, a nucleic acid molecule comprises any of the modified open reading frames of SEQ ID NO: 94 through SEQ ID NO: 96. In additional aspects of this embodiment, a COS-1 cell line contains an expression construct operably linked to a nucleic acid molecule comprising a modified open reading frame of SEQ ID NO: 94, SEQ ID NO: 95 or SEQ ID NO: 96; a COS-7 cell line contains an expression construct operably linked to a nucleic acid molecule comprising a modified open reading frame of SEQ ID NO: 94, SEQ ID NO: 95 or SEQ ID NO: 96; or a W-1 cell line contains an expression construct operably linked to a nucleic acid molecule comprising a modified open reading frame of SEQ ID NO: 94, SEQ ID NO: 95 or SEQ ID NO: 96.

In an aspect, a cell comprises a human cell comprising an expression construct operably linked to a nucleic acid molecule comprising a modified open reading frame providing increased expression of the encoded active BoNT/A in a human cell. In an aspect of this embodiment, a cell comprises a human cell transiently containing an expression construct operably linked to a nucleic acid molecule comprising a modified open reading frame providing increased expression of the encoded active BoNT/A in a human cell. In another aspect of this embodiment, a cell comprises a human cell stably containing an expression construct operably linked to a nucleic acid molecule comprising a modified open reading frame providing increased expression of the encoded active BoNT/A in a human cell. In further aspect of this embodiment, a human cell is a human cell or cell line derived from a human cell from *H*. *sapiens.* In a further aspect of this embodiment, a human cell is a human cell line derived from SH-SY5Y, SK-N-DZ, SK-N-F1, SK-N-SH, BE (2)-C, HeLa, HEK 293, MCF-7, HepG2, HL-60, IMR-32, SW-13 or CHP3. In yet another aspect of this embodiment, an expression construct is a human expression construct. In yet another aspect of this embodiment, nucleic acid molecule comprises any of the modified open reading frames of SEQ ID NO: 97 through SEQ ID NO: 99. In additional aspects of this embodiment, a SH-SY5Y cell line contains an expression construct operably linked to a nucleic acid molecule comprising a modified open reading frame of SEQ ID. NO: 97, SEQ ID NO: 98 or SEQ ID NO: 99; a SK-N-DZ cell line contains an expression construct operably linked to a nucleic acid molecule comprising a modified open reading frame of SEQ ID NO: 97, SEQ ID NO: 98 or SEQ ID NO: 99; a SK-N-F1 cell line contains an expression construct operably linked to a nucleic acid molecule comprising a modified open reading frame of SEQ ID NO: 97, SEQ ID NO: 98 or SEQ ID NO: 99; a SK-N-SH cell line contains an expression construct operably linked to a nucleic acid molecule comprising a modified open reading frame of SEQ ID NO: 97, SEQ ID NO: 98 or SEQ ID NO: 99; a BE (2)-C cell line contains an expression construct operably linked to a nucleic acid molecule comprising a modified open reading frame of SEQ ID NO: 97, SEQ ID NO: 98 or SEQ ID NO: 99; a HeLa cell line contains an expression construct operably linked to a nucleic acid molecule comprising a modified open reading frame of SEQ ID NO: 97, SEQ ID NO: 98 or SEQ ID NO: 99; a HEK 293 cell line contains an expression construct operably linked to a nucleic acid molecule comprising a modified open reading frame of SEQ ID NO: 97, SEQ ID NO: 98 or SEQ ID NO: 99; a MCF-7 cell line contains an expression construct operably linked to a nucleic acid molecule comprising a modified open reading frame of SEQ ID NO: 97, SEQ ID NO: 98 or SEQ ID NO: 99; a HepG2 cell line contains an expression construct operably linked to a nucleic acid molecule comprising a modified open reading frame of SEQ ID NO: 97, SEQ ID NO: 98 or SEQ ID NO: 99; a HL-60 cell line contains an expression construct operably linked to a nucleic acid molecule comprising a modified open reading frame of SEQ ID NO: 97, SEQ ID NO: 98 or SEQ ID NO: 99; a IMR-32 cell line contains an expression construct operably linked to a nucleic acid molecule comprising a modified open reading frame of SEQ ID NO: 97, SEQ ID NO: 98 or SEQ ID NO: 99; a SW-13 cell line contains an expression construct operably linked to a nucleic acid molecule comprising a modified open reading frame of SEQ ID NO: 97, SEQ ID NO: 98 or SEQ ID NO: 99; or a CHP3 cell line contains an expression construct operably linked to a nucleic acid molecule comprising a modified open reading frame of SEQ ID NO: 97, SEQ ID NO: 98 or SEQ ID NO: 99.

Another aspect provides a method of producing an active BoNT/A comprising the step of expressing an expression construct comprising a modified open reading frame providing increased expression of an encoded active BoNT/A in a heterologous cell. Another aspect of the present invention provides a method of producing an active BoNT/A comprising the steps of introducing an expression construct comprising a modified open reading frame providing increased expression of an encoded active BoNT/A into a heterologous cell and expressing the expression construct in the heterologous cell.

The methods disclosed in the present specification include, in part, an active BoNT/A. It is envisioned that any and all active BoNT/A molecules disclosed in the present specification can be produced using the methods disclosed in the present specification. Thus, aspects of this embodiment include producing, without limitation, active BoNT/A, naturally occurring active BoNT/A variants, such as, *e.g*., BoNT/A isoforms, non-naturally occurring active BoNT/A variants, such as, *e.g*., conservative BoNT/A variants, non-conservative BoNT/A variants and active BoNT/A fragments thereof, or any combination thereof. Other aspects of this embodiment include, without limitation, active BoNT/A of SEQ ID NO:1, naturally occurring active BoNT/A variants of SEQ ID NO: 1, such as, *e.g*., active BoNT/A isoforms of SEQ ID NO: 1, non-naturally occurring active BoNT/A variants of SEQ ID NO: 1, such as, *e.g*., conservative BoNT/A variants of SEQ ID NO: 1, non-conservative BoNT/A variants of SEQ ID NO: 1 and active BoNT/A fragments of SEQ ID NO: 1, or any combination thereof.

The methods disclosed in the present specification include, in part, an expression construct. It is envisioned that any and all expression constructs disclosed in the present specification can be used. Thus, aspects of this embodiment include, without limitation, cells comprising a viral expression vector operably linked to a modified open reading frame providing increased expression of an encoded active BoNT/A in a mammalian cell; a prokaryotic expression vector operably linked to a modified open reading frame providing increased expression of an encoded active BoNT/A in a prokaryotic cell; cells comprising a yeast expression vector operably linked to a modified open reading frame providing increased expression of an encoded active BoNT/A in a yeast cell; cells comprising a slime mold expression vector operably linked to a modified open reading frame providing increased expression of an encoded active BoNT/A in a slime mold cell; cells comprising a plant expression vector operably linked to a modified open reading frame providing increased expression of an encoded active BoNT/A in a plant cell or cell line derived from a plant cell; cells comprising an insect expression vector operably linked to a modified open reading frame providing increased expression of the encoded active BoNT/A in an insect cell or cell line derived from an insect cell; cells comprising a fish expression vector operably linked to a modified open reading frame providing increased expression of an encoded active BoNT/A in a fish cell or cell line derived from a fish cell; cells comprising an amphibian expression vector operably linked to a modified open reading frame providing increased expression of an encoded active BoNT/A in an amphibian cell or cell line derived from an amphibian cell; cells comprising a bird expression vector operably linked to a modified open reading frame providing increased expression of an encoded active BoNT/A in a bird cell or cell line derived from a bird cell; and cells comprising a mammalian expression vector operably linked to a modified open reading frame providing increased expression of an encoded active BoNT/A in a mammalian cell or cell line derived from a mammalian cell, such as, *e.g*., mouse, rat, hamster, porcine, bovine, equine, primate and human. Other aspects of this embodiment include, without limitation, expression constructs suitable expressing a BoNT/A disclosed in the present specification usng a cell-free extract comprising an expression vector operably linked to a modified open reading frame providing increased expression of an encoded active BoNT/A in the cell-free extract. Other aspects of this embodiment include, without limitation, expression constructs comprising a modified open reading frame that comprises any one of SEQ ID NO: 3 through SEQ ID NO: 99, SEQ ID NO: 110, SEQ ID NO: 112 and SEQ ID NO: 122 through SEQ ID NO: 125.

The methods disclosed in the present specification include, in part, a heterologous cell. It is envisioned that any and all heterologous cells disclosed in the present specification can be used. Thus, aspects of this embodiment include, without limitation, prokaryotic cells prokaryotic cells including, without limitation, strains of aerobic, microaerophilic, capnophilic, facultative, anaerobic, gram-negative and gram-positive bacterial cells such as those derived from, *e.g., Escherichia coli, Bacillus subtilis, Bacillus licheniformis, Bacteroides fragilis, Clostridia perfringens, Clostridia difficile, Caulobacter crescentus, Lactococcus lactis, Methylobacterium extorquens, Neisseria meningirulls, Neisseria meningitidis, Pseudomonas fluorescens* and *Salmonella typhimurium*; and eukaryotic cells including, without limitation, yeast strains, such as, *e.g*., those derived from *Pichia pastoris, Pichia methanolica, Pichia angusta, Schizosaccharomyces pombe, Saccharomyces cerevisiae* and *Yarrowia lipolytica;* slime mold strains, such as, *e.g*., those derived from, *e.g., Dictyostelium discoideum;* plant cells and cell lines derived from plant cells, such as, *e.g.,* those derived from species of monocots, species of dicots, *Zea mays* and *Arabidopsis thaliana;* insect cells and cell lines derived from insects, such as, *e.g*., those derived from *Spodoptera frugiperda, Trichoplusia ni, Drosophila melanogaster* and *Manduca sexta;* fish cells and cell lines derived from fish cells, such as, *e.g.*, those derived from *Denio renia;* amphibian cells and cell lines derived from amphibian cells, such as, *e.g.,* those derived from *Xenopus laevis* and *Xenopus tropicalis;* bird cells and cell lines derived from bird cells, such as, *e.g.,* those derived from *Gallus gallus;* mammalian cells and cell lines derived from mammalian cells, such as, *e.g*., those derived from mouse, rat, hamster, porcine, bovine, equine, primate and human. Cell lines may be obtained from the American Type Culture Collection (2004), at URL address www.atcc.org; European Collection of Cell Cultures (2204), at URL address www.ecacc.org.uk; and the German Collection of Microorganisms and Cell Cultures (2004), at URL address www.dsmz.de. Non-limiting examples of specific protocols for selecting, making and using an appropriate cell line are described in *e.g*., INSECT CELL CULTURE ENGINEERING (Mattheus F. A. Goosen et al. eds., Marcel Dekker, 1993); INSECT CELL CULTURES: FUNDAMENTAL AND APPLIED ASPECTS (J. M. Vlak et al. eds., Kluwer Academic Publishers, 1996); Maureen A. Harrison & Ian F. Rae, GENERAL TECHNIQUES OF CELL CULTURE (Cambridge University Press, 1997); CELL AND TISSUE CULTURE: LABORATORY PROCEDURES (Alan Doyle et al eds., John Wiley and Sons, 1998); R. Ian Freshney, CULTURE OF ANIMAL CELLS: A MANUAL OF BASIC TECHNIQUE (Wiley-Liss, 4th ed. 2000); ANIMAL CELL CULTURE: A PRACTICAL APPROACH (John R. W. Masters ed., Oxford University Press, 3rd ed. 2000); MOLECULAR CLONING A LABORATORY MANUAL, supra, (2001); BASIC CELL CULTURE: A PRACTICAL APPROACH (John M. Davis, Oxford Press, 2nd ed. 2002); and CURRENT PROTOCOLS IN MOLECULAR BIOLOGY, supra, (2004). These protocols are routine procedures within the scope of one skilled in the art and from the teaching herein.

The methods disclosed in the present specification include, in part, introducing an expression construct into a heterologous cell. It is envisioned that any and all methods for introducing an expression construct disclosed in the present specification into a cell can be used. A cell disclosed in the present specification can maintain an expression construct transiently or stably. Stably-maintained constructs may be extra-chromosomal and replicate autonomously, or they may be integrated into the chromosomal material of the cell and replicate non-autonomously. Methods useful for introducing a nucleic acid molecule into a cell including, without limitation, calcium phosphate-mediated, DEAE dextran-mediated, lipid-mediated, polybrene-mediated, polylysine-mediated, viral-mediated, microinjection, protoplast fusion, biolistic, and electroporation, see, *e.g*., Introducing Cloned Genes into Cultured Mammalian Cells, pp. 16.1-16.62 (Sambrook & Russell, eds., Molecular Cloning A Laboratory Manual, Vol. 3, 3rd ed. 2001). One skilled in the art understands that selection of a specific method to introduce an expression construct into a cell will depend, in part, on whether the cell will transiently contain an expression construct or whether the cell will stably contain an expression construct. These protocols are routine procedures within the scope of one skilled in the art and from the teaching herein.

It is envisioned that both cell-free and cell-based procedures can be used to produce an active BoNT/A using methods disclosed in the present specification. These procedures involve the use of well-characterized vectors, reagents, conditions and cells that are readily available from commercial vendors including, without limitation, BD Biosciences-Clontech, Palo Alto, CA; BD Biosciences Pharmingen, San Diego, CA; Invitrogen, Inc, Carlsbad, CA; QIAGEN, Inc., Valencia, CA; Roche Applied Science, Indianapolis, IN; and Stratagene, La Jolla, CA. The selection and use of appropriate procedures to produce an active BoNT/A are described in *e.g*., PROTEIN EXPRESSION. A PRACTICAL APPROACH, supra, (1999) and Fernandez & Hoeffler, *supra,* (1999). These protocols are routine procedures within the scope of one skilled in the art and from the teaching herein.

One procedure of producing active BoNT/A employs a cell-free expression system such as, without limitation, prokaryotic extracts and eukaryotic extracts. Non-limiting examples of prokaryotic cell extracts include the RTS 100 *E. coli* HY Kit (Roche Applied Science, Indianapolis, IN), the ActivePro In Vitro Translation Kit (Ambion, Inc., Austin, TX), the EcoPro™ System (EMD Biosciences-Novagen, Madison, WI) and the Expressway™ Plus Expression System (Invitrogen, Inc., Carlsbad, CA). Eukaryotic cell extract include, without limitation, the RTS 100 Wheat Germ CECF Kit (Roche Applied Science, Indianapolis, IN), the TnT^{®} Coupled Wheat Germ Extract Systems (Promega Corp., Madison, WI), the Wheat Germ IVT™ Kit (Ambion, Inc., Austin, TX), the Retic Lysate IVT™ Kit (Ambion, Inc., Austin, TX), the PROTEINscript^{®} II System (Ambion, Inc., Austin, TX) and the TnT^{®} Coupled Reticulocyte Lysate Systems (Promega Corp., Madison, WI).

It is also envisioned that any of a variety of cell-based expression procedures are useful for expressing nucleic acid molecules encoding an active BoNT/A disclosed in the present specification. Examples included, without limitation, viral expression systems, prokaryotic expression systems, yeast expression systems, plant expression systems, baculoviral expression systems, insect expression systems and mammalian expression systems. Viral expression systems include, without limitation, the ViraPower™ Lentiviral (Invitrogen, Inc., Carlsbad, CA), the Adenoviral Expression Systems (Invitrogen, Inc., Carlsbad, CA), the AdEasy™ XL Adenoviral Vector System (Stratagene, La Jolla, CA) and the ViraPort® Retroviral Gene Expression System (Stratagene, La Jolla, CA). Non-limiting examples of prokaryotic expression systems include the Champion™ pET Expression System (EMD Biosciences-Novagen, Madison, WI), the TriEx™ Bacterial Expression Systems (EMD Biosciences-Novagen, Madison, WI), the QIAexpress^{®} Expression System (QIAGEN, Inc.), and the Affinity^{®}. Protein Expression and Purification System (Stratagene, La Jolla, CA). Yeast expression systems include, without limitation, the EasySelect™ *Pichia* Expression Kit (Invitrogen, Inc., Carlsbad, CA), the YES-Echo™ Expression Vector Kits (Invitrogen, Inc., Carlsbad, CA) and the SpECTRA™ *S. pombe* Expression System (Invitrogen, Inc., Carlsbad, CA). Non-limiting examples of baculoviral expression systems include the BaculoDireCt™ (Invitrogen, Inc., Carlsbad, CA), the Bac-to-Bac^{®} (Invitrogen, Inc., Carlsbad, CA), and the BD BaculoGold™ (BD Biosciences-Pharmigen, San Diego, CA). Insect expression systems include, without limitation, the *Drosophila* Expression System (DES^{®}) (Invitrogen, Inc., Carlsbad, CA), InsectSelect™ System (Invitrogen, Inc., Carlsbad, CA) and InsectDirect™ System (EMD Biosciences-Novagen, Madison, WI). Non-limiting examples of mammalian expression systems include the T-REx™ (Tetracycline-Regulated Expression) System (Invitrogen, Inc., Carlsbad, CA), the Flp-In™ T-REx™ System (Invitrogen, Inc., Carlsbad, CA), the pcDNA™ system (Invitrogen, Inc., Carlsbad, CA), the pSecTag2 system (Invitrogen, Inc., Carlsbad, CA), the Exchanger^{®} System, InterPlay™ Mammalian TAP System (Stratagene, La Jolla, CA), Complete Control^{®} Inducible Mammalian Expression System (Stratagene, La Jolla, CA) and LacSwitch^{®} II Inducible Mammalian Expression System (Stratagene, La Jolla, CA).

Aspects of the present invention can also be described as follows:
1. A nucleic acid molecule comprising a modified open reading frame encoding an active BoNT/A
   wherein the modified open reading frame comprises SEQ ID NO: 3, SEQ ID NO: 110 or SEQ ID NO: 112
   wherein the active BoNT/A is a BoNT/A that executes the overall cellular mechanism whereby the active BoNT/A enters a neuron and inhibit neurotransmitter release and encompasses the binding of the active BoNT/A to a low or high affinity receptor complex, the internalization of the active BoNT/A/receptor complex, the translocation of the active BoNT/A light chain into the cytoplasm and the enzymatic modification of the active BoNT/A substrate; and
   wherein the modified open reading frame provides increased expression of the encoded active BoNT/A in an *Escherichia coli* cell as compared to an expression level of the same active BoNT/A in the *Escherichia coli* cell from an unmodified open reading frame in an otherwise identical nucleic acid molecule.
2. The molecule according to item 1, wherein the increased expression of the active BoNT/A from the modified open reading frame is at least two-fold higher as compared to the expression level of the same active BoNT/A from the unmodified open reading frame.
3. The molecule according to item 1, wherein the increased expression of the active BoNT/A from the modified open reading frame is at least five-fold higher as compared to the expression level of the same active BoNT/A from the unmodified open reading frame.
4. The molecule according to item 1, wherein the increased expression of the active BoNT/A from the modified open reading frame is at least ten-fold higher as compared to the expression level of the same active BoNT/A from the unmodified open reading frame.
5. The molecule according to any one of items 1-4, wherein the molecule is an expression construct.
6. A heterologous cell comprising an expression construct, wherein the expression construct is from item 5.
7. The cell according to item 6, wherein the expression construct is transiently contained in the heterologous cell.
8. The cell according to item 6, wherein the expression construct is stably contained in the heterologous cell.

### EXAMPLES

The following non-limiting examples are provided for illustrative purposes only in order to facilitate a more complete understanding of disclosed embodiments and are in no way intended to limit any of the embodiments disclosed in the present specification.

### Example 1

Selection of nucleotide alterations for an open reading frame providing increased expression of the encoded active BoNT/A in a heterologous cell.

### 1. Manual selection of nucleotide alterations

To determine codon use of a particular heterologous cell and how it compares to the codon usage found in *C. botulinum,* codon usage for *C. botulinum* and selected heterologous cells were tabulated using information obtained from the publicly maintained Codon Usage Database (URL address www.kazusa.or.jp/codon) to facilitate comparisons among organisms (Table 1).

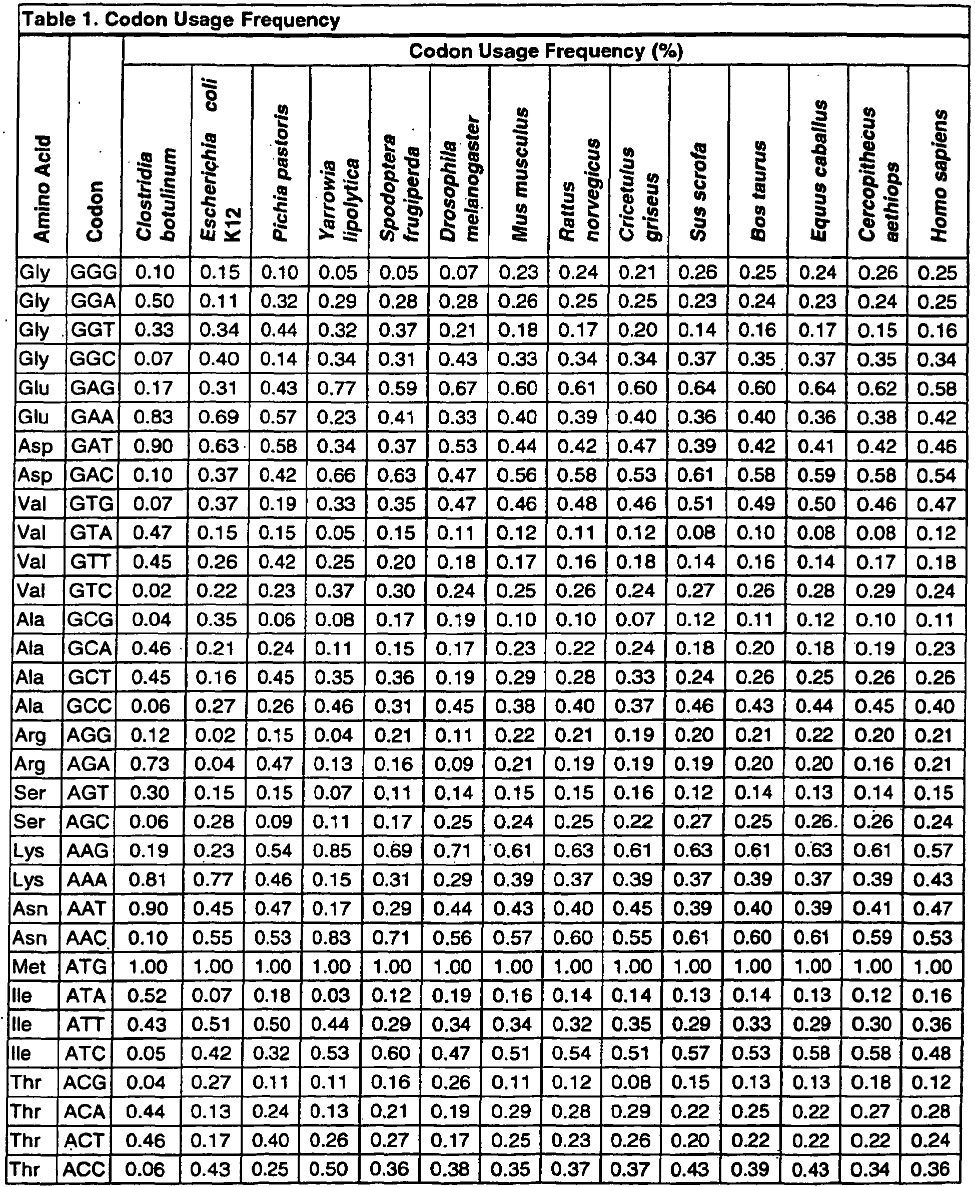

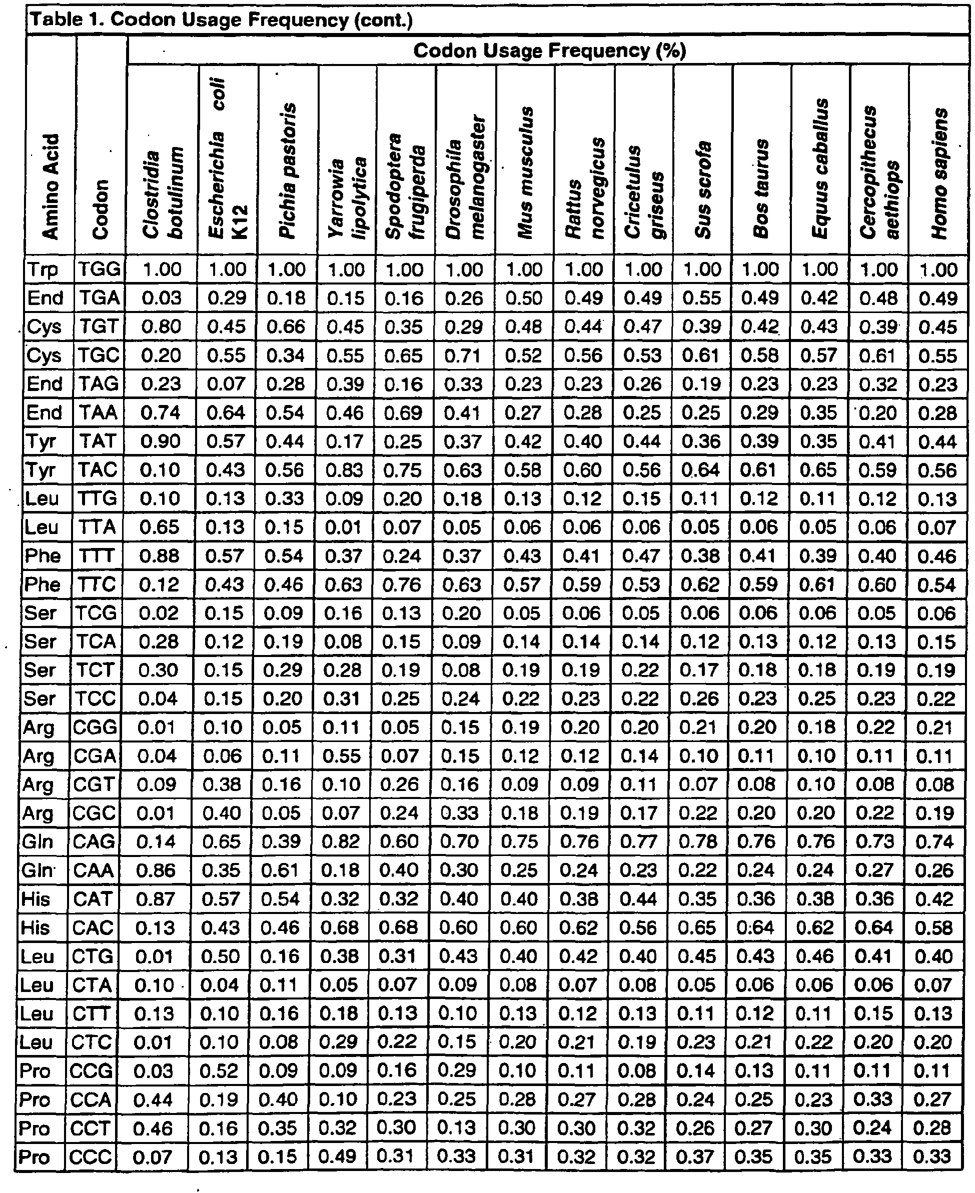

To determine G+C content of a particular heterologous cell and how it compares to the codon usage found in *C. botulinum,* G+C content for *C. botulinum* and selected heterologous cells were tabulated using information obtained from the publicly maintained Codon Usage. Database (URL address www.kazusa.or.jp/codon) to facilitate comparisons among organisms (Table 2).

| **Table 2: G+C content** | | | | |
|---|---|---|---|---|
| **Organism** | **Total G+C Content (%)** | **First Codon Position G+C Content (%)** | **Second Codon Position G+C Content (%)** | **Third Codon Position G+C Content (%)** |
| *Clostridium botulinum* | 25.29 | 33.44 | 28.38 | 14.04 |
| *Escherichia coli* | 51.80 | 58.89 | 40.72 | 55.79 |
| *Pichia pastoris* | 42.99 | 49.16 | 37.49 | 42.32 |
| *Yarrowia lipolytica* | 54.69 | 58.17 | 41.18 | 64.71 |
| *Zea mays* | 54.60 | 57.46 | 43.03 | 63.31 |
| *Spodoptera frugiperda* | 51.44 | 53.92 | 39.52 | 60.88 |
| *Drosophila melanogaster* | 53.99 | 55.90 | 41.51 | 64.57 |
| *Mus musculus* | 52.33 | 55.57 | 42.19 | 59.24 |
| *Rattus norvegicus* | 52.82 | 55.64 | 41.64 | 61.19 |
| *Cricetulus griseus* | 51.26 | 55.29 | 40.43 | 58.07 |
| *Sus scrofa* | 54.68 | 56.47 | 41.95 | 65.63 |
| *Bos taurus* | 53.14 | 55.43 | 41.46 | 62.53 |
| *Equus caballus* | 53.63 | 55.96 | 40.71 | 64.21 |
| *Cercopithecus aethiops* | 52.81 | 53.80 | 42.36 | 62.26 |
| *Homo sapiens* | 52.54 | 56.10 | 42.55 | 58.99 |

Using Tables 1 and 2, one skilled in the art can manually select which nucleotides to alter to the open reading frame of SEQ ID NO: 2 so that the open reading frame now provides synonymous codons preferred by the heterologous cell selected to express this open reading frame and increase the G+C content to better match the G+C content of this heterologous cell.

### 2. Computer-assisted selection of nucleotide alterations

To alter the open reading frame of SEQ ID NO: 2 in order to provide increased expression of the encoded BoNT/A in a heterologous cell, synonymous codon usage for each organism was determined using the publicly available Backtranslate Tool, version 2 (Entelechon, GmbH, Regensburg, Germany, at URL address entelechon.com/eng/backtranslation). The active BoNT/A amino acid sequence of SEQ ID NO: 1 was submitted to this web-based program and prospective modified open reading frames were generated. These modified sequences were subsequently analyzed for G+C content, and substitutions that better matched the G+C content of a specific heterologous cell were made. This procedure resulted in the modified open reading frames SEQ ID NO: 4 through,SEQ ID NO: 99, each encoding an active BoNT/A of SEQ ID NO: 1, but optimized to be expressed in a heterologous cell. Nucleic acid molecule sequences SEQ ID NO: 4, 7, 10, 13, 16, 19, 22, 25, 28, 31, 34, 37, 40, 43, 46, 49, 52, 55, 58, 61, 64, 67, 70, 73, 76, 79, 82, 85, 88, 91, 94 and 97 were generated using codon tables for the indicated species where the codon usage frequencies for each amino acid were maintained when generating the nucleic acid molecule. For example, when generating the nucleic acid molecule SEQ ID NO: 4 for expression in *E. coli,* the codon usage frequencies selected for Arginine were 10% for CGG, 6% for CGA, 38% for CGT, 40% for CGC, 2% for AGG and 4% for AGA; the codon usage frequencies for Alanine were 35% for GCG, 21% for GCA, 16% for GCT and 27% for GCC; and the codon usage frequencies for Cysteine were 45% for TGT and 55% for TGC.

Nucleic acid molecule sequences SEQ ID NO: 5, 8, 11, 14, 17, 20, 23, 26, 29, 32, 35, 38, 41, 44, 47, 50, 53, 56, 59, 62, 65, 68, 71, 74, 77, 80, 83, 86, 89, 92, 95 and 98 were generated using codon tables for the indicated species where only the most frequently used codon for each amino acid was selected to generate a nucleic acid molecule. For example, when generating the nucleic acid molecule SEQ ID NO: 5 for expression in *E. coli,* the codon CGC was used for Arginine; the codon GCG was used for Alanine; and the codon TGC was used for Cysteine.

Nucleic acid molecule sequences SEQ ID NO: 6, 9, 12, 15, 18, 21, 24, 27, 30, 33, 36, 39, 42, 45, 48, 51, 54, 57, 60, 63, 66, 69, 72, 75, 78, 81, 84, 87, 90, 93, 96 and 99 were generated using codon tables for the indicated species where codons below a 50% theoretical ratio for each amino acid were discarded when generating the nucleic acid molecule. Arginine has a theoretical ratio of 16.7% because this amino acid is coded by six different codons, Alanine has a theoretical ratio of 25% because this amino acid is coded by four different codons and Cysteine has a theoretical' ratio of 50% because this amino acid is coded by two different codons. Discarding codons below a 50% theoretical ratio would mean that all codons below a 8.35% usage frequency will be discarded for Arginine; all codons below a 12.5% usage frequency will be discarded for Alanine; and all codons below a 25% usage frequency will be discarded for Cysteine. For example, when generating the nucleic acid molecule SEQ ID NO: 6 for expression in *E. coli,* the codons CGG, CGT and CGC were used for Arginine; the codons GCG, GCA, GCT and GCC were used for Alanine; and the codons TGT and TGC were used for Cysteine.

### Example 2

### Synthesis of a nucleic acid molecule

A nucleic acid molecule encoding a BoNT/A was modified so that particular synonymous codons preferred by *E. coli* were incorporated and the G+C content was increased from about 25% to approximately 40%. Initially, an algorithm generated a modified open reading frame encoding the BoNT/A of SEQ ID NO: 1 (BlueHeron^{®} Biotechnology, Bothell WA). This program 1) reduced the mRNA secondary structure (based on a free energy calculation) of the nucleic acid molecule and 2) altered the synonymous codon usage of the open reading frame of the nucleic acid molecule to an overall codon usage preferred by *E. coli.* The algorithm uses a statistical model to search for improved solutions (*i.e*., combinations of representative codon usage and lower free energy) through an iterative process. This sequence was then modified by one skilled in the art at Allergan, Inc. to add unique restriction endonuclease sites at the 5'-termini (*e.g*., *Eco*RV, *Bam*HI, *Eco*RI, *Sac*I and *Nde*I) and 3'-termini (*e.g*., *Sal*I, *Hind*III, *Not*I, *Eag*I, *Xho*I and *Ava*I) of the nucleic acid molecule in order to facilitate cloning into expression vectors, reduce polymononucleotide regions and remove internal regulatory or structural site sequences.

Based on this sequence information above, BlueHeron^{®} Biotechnology synthesized a nucleic acid molecule of SEQ ID NO: 3. Oligonucleotides of 20 to 50 bases in length were synthesized using standard phosphoramidite synthesis. These oligonucleotides were hybridized into double stranded duplexes that were ligated together to assemble the full-length nucleic acid molecule . This nucleic acid molecule was cloned using standard molecular biology methods into a pUCBHB1 vector at the Smal site to generate pUCBHB1-BoNT/A. The synthesized nucleic acid molecule was versified by sequencing using Big Dye Terminator™ Chemistry 3.1 (Applied Biosystems, Foster City, CA) and an ABI 3100 sequencer (Applied Biosystems, Foster City, CA).

### Example 3

### Construction of pUCBHB1/iBoNT/A (H227Y)

Because of regulatory and safety considerations, initial expression of a construct comprising a modified open reading frame encoding BoNT/A was performed using enzymatically inactive BoNT/A (iBoNT/A). These initial expression attempts allowed development of the protocols and strategies necessary for expressing the constructs encoding an active BoNT/A. Several iBoNT/A molecules were designed based on the knowledge that mutation of the zinc binding motif within the LC disrupts enzymatic activity. In the first, Histidine-227 in BoNT/A was substituted with tyrosine (H227Y). A second point mutation, one in which glutamine replaces Glutamate-224 (E224Q), was also constructed. Unlike the H227Y mutant, in which a zinc binding residue is mutated, the E224Q mutation replaces the residue responsible for coordinating and activating the nucleophilic water molecule that adds to the scissile peptide bond. Both of these inactivating mutations are within the highly conserved zinc binding motif (Table 3).

| **Table 3. Zinc-binding motif inactivating mutations** | |
|---|---|
| **Consensus motif:** | **HExxH** |
| Native BoNT/A | HELIH |
| iBoNT/A(H227Y) | HELIY |
| iBoNT/A(E224Q) | HQLIH |

The pUCBHB1/BoNT/A of Example 3 was used as the starting construct for site-directed in vitro mutagenesis experiments that resulted in the construction of the constructs pUCBHB1/iBoNT/A (H227Y). To construct pUCBHB1/iBoNT/A (H227Y), a 50 µL reaction was assembled using the pUCBHB1-BoNT/A construct as a template, the following H227Y Primer Pair, sense oligonucleotide, 5'-GTGACCTTGGCACA TGAACTTATT**T**ATGCCGGGCATCGCTTGTATGGAATCGCC-3' (SEQ ID NO: 100) and antisense oligonucleotide, 5'-GGCGATTCCATACAAGCGATGCCCGGCAT**A**AATAAGTTCATGTGCCAAGGTCAC-3' (SEQ ID NO: 101); and reagents included with the QuickChange® II XL Site-Directed Mutagenesis kit (Stratagene, La Jolla, CA). The polymerase chain reaction (PCR) mix contained 5 µL of 10x Buffer, 1 µL of deoxyribonucleotides (dNTPs), 1µL of PfuUltra™ High Fidelity DNA polymerase (2.5 units/µL), 125 ng of each primer, 100 ng of template DNA, and nuclease-free water to a final volume of 50 µL. The thermocycler conditions were: one cycle of 95 °C for 60 seconds; 16 cycles of 95 °C for 30 seconds, 55 °C for 60 seconds, and 72 °C for 10 minutes; one cycle of 72 °C for 5 minutes; and 4 °C to hold. Following thermocycling, 1 µL of *Dpn*I restriction enzyme (Stratagene, La Jolla, CA) was added to the reaction and incubated for 1 hour at 37 °C to digest the template DNA. The reaction was purified by QIAquick kit (OIAGEN, Inc., Valencia, CA) and analysis by agarose gel electrophoresis showed that the reaction produced full-length plasmid. The mutagenesis products were transformed into chemically competent *E. coli* DH5a cells (Invitrogen, Inc, Carlsbad, CA) using a heat shock method, plated on 1.5% Luria-Bertani agar plates (pH 7.0) containing 100 µg/mL of Ampicillin, and placed in a 37 °C incubator for overnight growth. Candidate mutagenesis constructs were isolated as Ampicillin resistant colonies and analyzed using an alkaline lysis plasmid mini-preparation procedure to isolate the expression construct and restriction endonuclease digests to determine the presence of the insert. The incorporation of the point mutation was determined by sequence analysis of candidate plasmid constructs. The nucleic acid molecule of SEQ ID NO: 116 encodes iBoNT/A (H227Y) of SEQ ID NO: 117.

### Example 4

### Construction of pRSETb/His-iBoNT/A (H227Y)

To construct pRSETb/His-iBoNT/A (H227Y), a pUCBHB1/iBoNT/A (H227Y) construct was digested with *Bam*HI and *Hind*III to excise the fragment encoding iBoNT/A (H227Y) insert. The resulting restriction fragment was purified by the QIAquick Gel Extraction Kit (QIAGEN, Inc., Valencia, CA), and the fragment containing the entire open reading frame was subcloned into the pRSETb vector (Invitrogen, Inc, Carlsbad, CA) that had been digested with restriction endonucleases *Bam*HI and *Hind*III. The fragment and vector were ligated overnight with T4 DNA ligase at 16 °C to yield pRSETb/His-iBoNT/A (H227Y). An aliquot of the ligation mixture was transformed by a standard heat-shock protocol into chemically competent TOP10 cells (Invitrogen, Inc, Carlsbad, CA), plated onto 1.5% Luria-Bertani agar plates (pH 7.0) containing 100 µg/mL of Ampicillin, and placed in a 37 °C incubator for overnight growth. Candidate expression constructs were selected as Ampicillin-resistant colonies. Resistant colonies were used to inoculate 2 mL of Luria-Bertani media containing 100 µg/mL of Ampicillin that were then grown in a 37 °C incubator, shaking at 250 rpm, overnight. The bacteria cells were harvested by microcentrifugation and the plasmid DNA was isolated using QIAGEN miniprep kits (QIAGEN, Inc., Valencia, CA). Candidate expression constructs were screened by restriction digestion with *Bam*HI and *Pst*I to determine the presence and orientation of the correct insert fragment. Cultures containing the desired expression construct were used to inoculate 1 L baffled flasks containing 200 mL of Luria-Bertani media containing 100 µg/mL of Ampicillin and placed in a 37 °C incubator, shaking at 250 rpm, for overnight growth. Purified plasmid DNA corresponding to an expression construct was isolated using the OIAGEN Maxi-prep method (QIAGEN, Inc., Valencia, CA) and sequenced to verify that the correct expression construct was made. This cloning strategy yielded a pRSETb expression construct comprising the nucleic acid molecule of SEQ ID NO: 118 encoding a iBoNT/A (H227Y) operably-linked to an amino-terminal enterokinase cleavable polyhistidine affinity binding peptide of SEQ ID NO: 119.

### Example 5

### Expression of pRSETb/His-iBoNT/A (H227Y)

The following example illustrates a procedure useful for expressing a BoNT/A from an expression construct disclosed in the present specification. A pRSETb/His-iBoNT/A (H227Y) expression construct was introduced into chemically competent *E. coli BL21* (DE3) cells (Invitrogen, Inc, Carlsbad, CA) using a heat-shock transformation protocol. The heat-shock reaction was plated onto 1.5% Luria-Bertani agar plates (pH 7.0) containing 100 µg/mL of Ampicillin and placed in a 37 °C incubator for overnight growth. Ampicillin-resistant colonies of transformed *E. coli* containing pRSETb/His-iBoNT/A (H227Y) were used to inoculate a baffled flask containing 3.0 mL of PA-0.5G media containing 100 µg/mL of Ampicillin which was then placed in a 37 °C incubator, shaking at 250 rpm, for overnight growth. The resulting overnight starter culture was in turn used to inoculate a 3 L baffled flask containing ZYP-5052 autoinducing media containing 100 µg/mL of Ampicillin at a dilution of 1:1000. Culture volumes ranged from about 600 mL (20% flask volume) to about 750 mL (25% flask volume). These cultures were grown in a 37 °C incubator shaking at 250 rpm for approximately 5.5 hours and were then transferred to a 16 °C incubator shaking at 250 rpm for overnight expression. Cells were harvested by centrifugation (4,000 rpm at 4 °C for 20-30 minutes) and used immediately, or stored dry at -80 °C until needed.

### Example 6

### Purification and quantification of His-iBoNT/A (H227Y)

The following example illustrates methods useful for purification and quantification of BoNT/A disclosed in the present specification. For immobilized metal affinity chromatography (IMAC) protein purification, *E. coli* BL21 (DE3) cell pellets used to express His-iBoNT/A (H227Y), as described in Example 5, were resuspended in Column Binding Buffer (25 mM *N*-(2-hydroxyethy)) piperazine-*N*'-(2-ethanesulfonic acid) (HEPES), pH 7.8; 500 mM sodium chloride; 10 mM imidazole; 2x Protease Inhibitor Cocktail Set III (EMD Biosciences-Calbiochem, San Diego CA); 5 units/mL of Benzonase (EMD Biosciences-Novagen, Madison, WI); 0.1% (v/v) Triton-X^{®} 100, 4-octylphenol polyethoxylate; 10% (v/v) glycerol), and then transferred to a cold Oakridge centrifuge tube. The cell suspension was sonicated on ice (10-12 pulses of 10 seconds at 40% amplitude with 60 seconds cooling intervals on a Branson Digital Sonifier) in order to lyse the cells and release the His-iBoNT/A, and then centrifuged (16,000 rpm at 4 °C for 20 minutes) to clarify the lysate. An immobilized metal affinity chromatography column was prepared using a 20 mL Econo-Pac column support (Bio-Rad Laboratories, Hercules, CA) packed with 2.5-5.0 mL of TALON™ SuperFlow Co²⁺ affinity resin (BD Biosciences-Clontech, Palo Alto, CA), which was then equilibrated by rinsing with 5 column volumes of deionized, distilled water, followed by 5 column volumes of Column Binding Buffer. The clarified lysate was applied slowly to the equilibrated column by gravity flow (approximately 0.25-0.3 mL/minute). The column was then washed with 5 column volumes of Column Wash Buffer (*N*-(2-hydroxyethyl) piperazine-*N'*-(2-ethanesulfonic acid) (HEPES), pH 7.8; 500 mM sodium chloride; 10 mM imidazole; 0.1% (v/v) Triton-X^{®} 100, 4-octylphenol polyethoxylate; 10% (v/v) glycerol). His-iBoNT/A was eluted with 20-30 mL of Column Elution Buffer (25 mM *N*-(2-hydroxyethyl) piperazine-N'-(2-ethanesulfonic acid) (HEPES), pH 7.8; 500 mM sodium chloride; 500 mM imidazole; 0.1% (v/v) Triton-X^{®} 100, 4-octylphenol polyethoxylate; 10% (v/v) glycerol) and collected in approximately twelve 1 mL fractions. The amount of His-iBoNT/A (H227Y) contained in each elution fraction was determined by a Bradford dye assay. In this procedure, 20 µL aliquots of each 1.0 mL fraction was combined with 200 µL of Bio-Rad Protein Reagent (Bio-Rad Laboratories, Hercules, CA), diluted 1 to 4 with deionized, distilled water, and then the intensity of the colorimetric signal was measured using a spectrophotometer. The five fractions with the strongest signal were considered the elution peak and pooled. Total protein yield was determined by estimating the total protein concentration of the pooled peak elution fractions using bovine gamma globulin as a standard (Bio-Rad Laboratories, Hercules, CA).

For purification of a BoNT/A using a FPLC desalting column, a HiPrep™ 26/10 size exclusion column (Amersham Biosciences, Piscataway, NJ) was pre-equilibrated with 80 mL of 4 °C Column Buffer (50 mM sodium phosphate, pH 6.5). After the column was equilibrated, a His-iBoNT/A (H227Y) sample was applied to the size exclusion column with an isocratic mobile phase of 4 °C Column Buffer and at a flow rate of 10 mL/minute using a BioLogic DuoFlow chromatography system (Bio-Rad Laboratories, Hercules, CA). The desalted His-iBoNT/A (H227Y) sample was collected as a single fraction of approximately 7-12 mL.

For purification of a BoNT/A using a FPLC ion exchange column, a His-iBoNT/A (H227Y) sample that had been desalted following elution from an IMAC column was applied to a 1 mL UNO-S1™ cation exchange column (Bio-Rad Laboratories, Hercules, CA) using a BioLogic DuoFlow chromatography system (Bio-Rad Laboratories, Hercules, CA). The sample was applied to the column in 4 °C Column Buffer (50 mM sodium phosphate, pH 6.5) and eluted by linear gradient with 4 °C Elution Buffer (50 mM sodium phosphate, 1 M sodium chloride, pH 6.5) as follows: step 1, 5.0 mL of 5% Elution Buffer at a flow rate of 1 mL/minute; step 2, 20.0 mL of 5-30% Elution Buffer at a flow rate of 1 mL/minute; step 3, 2.0 mL of 50% Elution Buffer at a flow rate of 1.0 mL/minute; step 4, 4.0 mL of 100% Elution Buffer at a flow rate of 1.0 mL/minute: and step 5, 5.0 mL of 0% Elution. Buffer at a flow rate of 1.0 mL/minute. Elution of peptides from the column was monitored at 280, 260, and 214 nm, and peaks absorbing above a minimum threshold (0.01 au) at 280 nm were collected. Most of the His-iBoNT/A eluted at a sodium chloride concentration of approximately 100 to 200 mM. Average total yields of His-iBoNT/A (H227Y) were approximately 1-2 mg/L as determined by a Bradford assay.

Expression of the His-iBoNT/A (H227Y) was analyzed by polyacrylamide gel electrophoresis. Samples purified using the procedure described above were added to 2x LDS Sample Buffer (Invitrogen, Inc, Carlsbad, CA) and peptides separated by MOPS polyacrylamide gel electrophoresis using NuPAGE^{®} Novex 4-12% Bis-Tris precast polyacrylamide gels (Invitrogen, Inc, Carlsbad, CA) under denaturing, reducing conditions. Gels were stained with SYPRO^{®} Ruby (Bio-Rad Laboratories, Hercules, CA) and the separated peptides imaged using a Fluor-S MAX Multilmager (Bio-Rad Laboratories, Hercules, CA) for quantification of peptide expression levels. The size and amount of the His-iBoNT/A (H227Y) was determined by comparison to MagicMark™ protein molecular weight standards (Invitrogen, Inc, Carlsbad, CA). The gels revealed what appeared to be a full-length His-iBoNT/A (H227Y).

Expression of the His-iBoNT/A (H227Y) was also analyzed by Western blot analysis. Protein samples purified using the procedure described above were added to 2x LDS Sample Buffer (Invitrogen, Inc, Carlsbad, CA) and separated by MOPS polyacrylamide gel electrophoresis using NuPAGE^{®} Novex 4-12% Bis-Tris precast polyacrylamide gels (Invitrogen, Inc, Carlsbad, CA) under denaturing, reducing conditions. Separated peptides were transferred from the gel onto polyvinylidene fluoride (PVDF) membranes (Invitrogen; Inc, Carlsbad, CA) by Western blotting using a Trans-Blot^{®} SD semi-dry electrophoretic transfer cell apparatus (Bio-Rad Laboratories, Hercules, CA). PVDF membranes were blocked by incubating at room temperature for 2 hours in a solution containing 25 mM Tris-Buffered Saline (25 mM 2-amino-2-hydroxymethyl-1,3-propanediol hydrochloric acid (Tris-HCl)(pH 7.4), 137 mM sodium chloride, 2.7 mM potassium chloride), 0.1% TWEEN-20^{®}, polyoxyethylene (20) sorbitan monolaureate, 2% bovine serum albumin, 5% nonfat dry milk. Blocked membranes were incubated at 4 °C for overnight in Tris-Buffered Saline TWEEN-20^{®} (25 mM Tris-Buffered Saline, 0.1% TWEEN-20^{®}, polyoxyethylene (20) sorbitan monolaureate) containing one of the following primary antibodies as a probe: a 1:5,000 dilution of rabbit polyclonal anti-BoNT/A antiserum (Allergan, Inc.); or a 1:10,000 dilution of rabbit polyclonal anti-polyhistidine antiserum (Abcam Inc., Cambridge, MA). Primary antibody probed blots were washed three times for 15 minutes each time in Tris-Buffered Saline TWEEN-20^{®}. Washed membranes were incubated at room temperature for 2 hours in Tris-Buffered Saline TWEEN-20^{®} containing a 1:20,000 dilution of goat polyclonal anti-rabbit immunoglobulin G, heavy and light chains (IgG, H+L) antibody conjugated to horseradish peroxidase (HRP; Pierce Biotechnology, Inc., Rockford, IL) as a secondary antibody. Secondary antibody-probed blots were washed three times for 15 minutes each time in Tris-Buffered Saline TWEEN-20^{®}. Signal detection of the labeled His-iBoNT/A (H227Y) was visualized using the ECL Plus™ Western Blot Detection System (Amersham Biosciences, Piscataway, NJ) and imaged with a Typhoon 9410 Variable Mode Imager (Amersham Biosciences, Piscataway, NJ) for quantification of His-iBoNT/A (H227Y) expression levels.

### Example 7

### Construction of pET30b/His-iBoNT/A (H227Y)

To construct pET30b/His-iBoNT/A (H227Y), a pRSETb/iBoNT/A (H227Y) construct was digested with *Bam*HI and *Hind*III to excise the fragment encoding iBoNT/A (H227Y). The resulting restriction fragment was purified by the QIAquick Gel Extraction Kit (QIAGEN, Inc., Valencia, CA), and the fragment containing the entire open reading frame was subcloned into the pET30b vector (EMD Biosciences-Novagen, Madison, WI) that had been digested with restriction endonucleases *Bam*HI and *Hind*III. The fragment and vector were ligated overnight with T4 DNA ligase at 16 °C to yield pET30b/His-iBoNT/A (H227Y). An aliquot of the ligation mixture was transformed by a standard heat-shock protocol into chemically competent TOP10 cells (Invitrogen, Inc, Carlsbad, CA), plated onto 1.5% Luria-Bertani agar plates (pH 7.0) containing 50 µg/mL of Kanamycin, and placed in a 37 °C incubator for overnight growth. Candidate expression constructs were selected as Kanamycin-resistant colonies. Resistant colonies were used to inoculate 2 mL of Luria-Bertani media containing 50 µg/mL of Kanamycin that were then grown in a 37 °C incubator, shaking at 250 rpm, overnight. The bacteria cells were harvested by microcentrifugation and the plasmid DNA was isolated using OIAGEN miniprep kits (OIAGEN, Inc., Valencia, CA). Candidate expression constructs were screened by restriction digestion with *Bam*HI and *Pst*I to determine the presence and orientation of the correct insert fragment. Cultures containing the desired expression construct were used to inoculate 1 L baffled flasks containing 200 mL of Luria-Bertani media containing 50 µg/mL of Kanamycin and placed in a 37 °C incubator, shaking at 250 rpm, for overnight growth. Purified plasmid DNA corresponding to an expression construct was isolated using the QIAGEN Maxi-prep method (QIAGEN, Inc., Valencia, CA) and sequenced to verify that the correct expression construct was made. This cloning strategy yielded a pET30b expression construct comprising the nucleic acid molecule of SEQ ID NO: 118 encoding a iBoNT/A (H227Y) operably-linked to an amino-terminal enterokinase cleavable polyhistidine affinity binding peptide of SEQ ID NO: 119.

### Example 8

### Expression of pET30b/His-iBoNT/A (H227Y) and His-iBoNT/A (H227Y) Purification and Quantification

The following example illustrates a procedure useful for expressing a BoNT/A from an expression construct disclosed in the present specification. A pET30b/His-iBoNT/A (H227Y) expression construct was introduced into chemically competent *E. coli* BL21 (DE3) cells (invitrogen, Inc, Carlsbad, CA) using a heat-shock transformation protocol. The heat-shock reaction was plated onto 1.5% Luria-Bertani agar plates (pH 7.0) containing 50 µg/mL of Kanamycin and placed in a 37 °C incubator for overnight growth. Kanamycin-resistant colonies of transformed *E. coli* containing pET30b/His-iBoNT/A (H227Y) were used to inoculate a baffled flask containing 3.0 mL of PA-0.5G media containing 50 µg/mL of Kanamycin which was then placed in a 37 °C incubator, shaking at 250 rpm, for overnight growth. The resulting overnight starter culture was in turn used to inoculate a 3 L baffled flask containing ZYP-5052 autoinducing media containing 50 µg/mL of Kanamycin at a dilution of 1:1000. Culture volumes ranged from about 600 mL (20% flask volume) to about 750 mL (25% flask volume). These cultures were grown in a 37 °C incubator shaking at 250 rpm for approximately 5.5 hours and were then transferred to a 16 °C incubator shaking at 250 rpm for overnight expression. Cells were harvested by centrifugation (4,000 rpm at 4 °C for 20-30 minutes) and used immediately, or stored dry at -80 °C until needed.

His-iBoNT/A (H227Y) expressed from a pET30b/His-iBoNT/A (H227Y) expression construct was purified and quantified as described above in Example 6. To analyze the His-iBoNT/A (H227Y) expression levels, His-iBoNT/A (H227Y) was purified using the purification procedure, as described in Example 6. Expression from each culture was evaluated by a Bradford dye assay, polyacrylamide gel electrophoresis and Western blot analysis (as described in Example 6; see FIG. 6a). Average total yields of His-iBoNT/A (H227Y) were approximately 4-5 mg/L as determined by a Bradford assay.

### Example 9

### Construction of pET30b/His-BoNT/A

A plasmid comprising a modified open reading frame encoding an active BoNT/A (FIG. 2), was prepared by *in vitro* site-directed mutagenesis of pET30b/His-iBoNT/A (H227Y). Correction of the inactivating H227Y mutation was accomplished in a single site-directed mutagenesis step using the procedure described in Example 3 and the following two oligonucleotides to yield pRSETb/His-BoNT/A: Y227H Primer Pair, sense oligonucleotide, 5'-GTGACCTTGGCACATGAACTTATT**C**ATGCCGGGCATC GCTTGTATGGAATCGCC-3' (SEQ 10 NO: 102) and antisense oligonucleotide, 5'-GGCGATTGCATACA AGCGATGCCCGGCAT**G**AATAAGTTCATGTGCCAAGGTCAC-3' (SEQ ID NO: 103). The amino acid numbering corresponds to native sequence lacking an amino-terminal polyhistidine tag. The nucleotides that were changed to correct H227Y are shown in bold and underlined. This mutagenesis resulted in the modified open reading frame of SEQ ID NO: 110 encoding the active His-BoNT/A of SEQ ID NO: 111.

Activity was identified by proteolytic cleavage of a GFP-SNAP25 substrate using a GFP-SNAP25 Fluorescence Release Assay, see, *e.g*., Lance E. Steward et al., GFP-SNAP25 Fluorescence Release Assay for Botulinum Neurotoxin Protease Activity, U.S. Patent Publication No. 2005/0100973 (May 12, 2005). Candidate pET30b/His-BoNT/A expression constructs were transformed into chemically competent *E. coli* BL21 (DE3) cells (Invitrogen, Inc, Carlsbad, CA) using a heat-shock method, plated onto 1.5% Luria-Bertani agar plates (pH 7.0) containing 50 µg/mL of Kanamycin, and placed in a 37 °C incubator for overnight growth. Kanamycin-resistant colonies containing the pET30b/His-BoNT/A candidates were used to inoculate 1 mL cultures of ZYP-5052 autoinducing media containing 50 µg/mL of Kanamycin in Eppendorf Lid-Bac tubes fitted with membrane lids. The cultures were incubated in a thermomixer (1,400 rpm at 37 °C) located in a biosafety cabinet until turbid (approximately 7-8 hours). The temperature was then reduced to 22 °C and the cultures incubated for approximately 16 hours. The cells were collected by centrifugation (6,000x g at 4 °C for 30 minutes), decanted and frozen briefly at -80 °C to improve lysis. The cell pellets were defrosted on ice, each was resuspended in 350 µL of BugBuster^{®} lysis solution (EMD Biosciences-Novagen, Madison, WI) containing 25 units/mL of benzonase nuclease (EMD Biosciences-Novagen, Madison, WI), 1 KU/mL rLysozyme (EMD Biosciences-Novagen, Madison, WI) and 2 x Protease Inhibitor Cocktail III (EMD Biosciences-Novagen, Madison, WI) and the mixtures were incubated for 30 minutes at 22 °C, 400 rpm in the thermomixer. The lysates were clarified by centrifugation (36,000 xg at 4 °C for 15 minutes) and the supernatant solutions transferred to low-retention microcentrifuge tubes and placed on ice.

Activity of His-BoNT/A candidates was identified by proteolytic cleavage of a GFP-SNAP25 substrate. Each assay reaction contained 25 µL of 2x Toxin Reaction Buffer (100 mM *N*-(2-hydroxyethyl) piperazine-*N'*-(2-ethanesulfonic acid) (HEPES), pH 7.4; 20 µM zinc chloride; 20 mM dithiothreitol; 0.2 % (v/v) TWEEN-20^{®}, polyoxyethylene (20) sorbitan monolaureate), 10 µL of clarified lysate, and 15 µL of 50 µM GFP-SNAP25₍₁₃₄₋₂₀₆₎ substrate. The control reactions contained 10 µL of either water or 0.2 µg/mL of LC/A in lieu of lysate. The reactions were assembled in triplicate, incubated at 37 °C for 1 hour and then quenched with 20 µL of 8 M guanidine hydrochloride. The quenched reactions were transferred to filter-plate wells containing 75 µL of TALON™ SuperFlow Co²⁺ affinity resin (BD Biosciences-Clontech, Palo Alto, CA) that had been conditioned by rinsing with 200 µL of deionized, distilled water and 200 µL of Assay Rinse Buffer (50 mM *N*-(2-hydroxyethyl) piperazine-*N'*-(2-ethanesuifonic acid) (HEPES), pH 7.4). Following 15 minutes incubation on the resin, the reaction solutions were eluted by vacuum filtration, collected in a black 96-well plate, passed over the resin beds twice more and collected after the final pass. Each resin bed was then rinsed with 210 µL of Assay Rinse Buffer which was eluted into the plate containing the reaction solutions. The fluorescence of the eluant reaction solutions was measured with a SpectraMax Gemini XS spectrophotometer (Molecular Devices, λ_{Ex} 474 nm; λ_{Em} 509 nm; 495 nm cutoff filter). The control reactions contained 10 µL of either water or 0.2 µg/mL of LC/A in lieu of lysate. Positive His-BoNT/A candidates showed significant protease activity (see FIG. 3).

### Example 10

### Comparison of His-BoNT/A amounts expressed from modified and unmodified open reading frames

The amount of increased BoNT/A expressed from a modified open reading frame as compared to an unmodified open reading frame can be determined as follows. In separate reactions, a pET30b/His-BoNT/A expression construct comprising the modified open reading frame of SEQ ID NO: 3 and a pET30b/His-BoNT/A construct comprising the unmodified open reading frame of SEQ ID NO: 2 are introduced into chemically competent *E. coli* BL21 (DE3) cells (Invitrogen, Inc, Carlsbad, CA) using a heat-shock transformation protocol. The heat-shock reactions are plated onto 1.5% Luria-Bertani agar plates (pH 7.0) containing 50 µg/mL of Kanamycin and are placed in a 37 °C incubator for overnight growth. Kanamycin-resistant colonies of transformed *E. coli* containing pET30b/His-BoNT/A constructs from both expression constructs are used to inoculate separate 15 mL tubes containing 3.0 mL Kanamycin-resistance selective PA-0.5G media that are then placed in a 37 °C incubator, shaking at 250 rpm, for overnight growth. Approximately 600 µL of the resulting overnight starter culture from each construct is used to inoculate a 3.0 L baffled flask containing 600 mL Kanamycin-resistance, ZYP-5052 autoinducing media. The inoculated cultures are grown in a 37 °C incubator shaking at 250 rpm for approximately 5.5 hours and are then transferred to a 16 °C incubator shaking at 250 rpm for overnight expression. Cells are harvested by centrifugation (4,000 rpm at 4 °C for 20-30 minutes).

To analyze the His-BoNT/A expression levels obtained from both the modified and unmodified open reading frames, His-BoNT/A is purified using the IMAC procedure, as described in Example 8. Expression from each culture is evaluated by a Bradford dye assay, polyacrylamide gel electrophoresis and Western blot analysis (as described in Example 6) in order to determine whether the amounts of His-BoNT/A produced from the modified open reading frame of SEQ ID NO: 3 is greater when compared to the amount of His-BoNT/A expressed from the unmodifed open reading frame of SEQ ID NO: 2. A five-fold increase in the amount of active His-BoNT/A expressed from a modified open reading frame is anticipated. Average amounts of IMAC purified active His-BoNT/A expressed from a modified open reading frame is expected to be approximately 5 mg/L, while IMAC purified active His-BoNT/A expressed from a unmodified open reading frame in an otherwise identical nucleic acid molecule is expected to be approximately 1 mg/L.

### Example 11

### Construction of pET29b/iBoNT/A-KHis (H227Y)

To construct pET29b/iBoNT/A-KHis (H227Y), a three step strategy was employed to first remove two internal *Nde*I restriction endonuclease sites from the open reading frame encoding iBoNT/A (H227Y) from a pRSETb/His-iBoNT/A (H227Y) construct and then, to add a carboxyl terminal lysine residue to iBoNT/A (H227Y), and lastly to subclone this modified fragment into a pET29b vector. The two internal *Nde*I restriction endonuclease sites from the open reading frame encoding iBoNT/A (H227Y) were removed using a site-directed mutagenesis protocol. A 50 µL reaction was assembled with the pRSETb/iBoNT/A (H227Y) expression construct as a template, reagents included with the QuickChange^{®} II XL Site-Directed Mutagenesis kit (Stratagene, La Jolla, CA) and the following four oligonucleotide primers: SL103 Primer Pair, sense oligonucleotide, 5'-GATGAACTCGATGATCCC**T**TA**C**GGTGTGAAAC GTCTGG-3' (SEQ ID NO: 104) and antisense oligonucleotide, 5'-CCAGACGTTTCACACC**G**TA**A**GGGAT CATCGAGTTCATC-3' (SEQ ID NO: 105); and SL104 Primer Pair, sense oligonucleotide, 5'-CCAGACGT TTCACACC**G**TA**A**GGGATCATCGAGTTCATC-3' (SEQ ID NO: 106) and antisense oligonucleotide, 5'-GA TGAACTCGATGATCCC**T**TA**C**GGTGTGAAACGTCTGG-3' (SEQ ID NO: 107). A polymerase chain reaction (PCR) mix contained 5 µL of 10x Buffer, 1 µL of deoxyribonucleotides (dNTPs), 1µL of *PfuUltra*™ High Fidelity DNA polymerase (2.5 units/µL), 125 ng of each primer, 50 ng of template DNA, and nuclease-free water to a final volume of 50 µL. The thermocycler conditions were: one cycle of 95 °C for 120 seconds; 20 cycles of 95 °C for 60 seconds, 55 °C for 30 seconds, and 72 °C for 20 minutes; one cycle of 72 °C for 9 minutes; and 10 °C to hold. Following thermocycling, 1 µL of *Dpn*I restriction enzyme was added to the reaction and incubated for 2 hour at 37 °C to digest the template DNA. The reaction was purified by QIAquick kit (QIAGEN, Inc., Valencia, CA) and analysis by agarose gel electrophoresis showed that the reaction produced full-length plasmid. The mutagenesis products were transformed into chemically competent *E. coli* TOP10 cells (Invitrogen, Inc, Carlsbad, CA) using a heat shock method, plated on 1.5% Luria-Bertani agar plates (pH 7.0) containing 100 µg/mL of Ampicillin, and placed in a 37 °C incubator for overnight growth. Candidate constructs were isolated as Ampicillin-resistant colonies and analyzed using an alkaline lysis plasmid mini-preparation procedure to isolate the expression construct and *Nde*I restriction endonuclease digests to determine the presence of inserts without Ndel sites. Removal of the two internal Ndel sites was confirmed by sequence analysis of the entire open reading frame from candidate plasmid constructs. This cloning strategy yielded a pRSETb/His-iBoNT/A (H227Y) construct comprising an open reading frame lacking two internal *Nde*I restriction endonuclease sites.

The iBoNT/A (H227Y) encoded by the Ndel-modified pRSETb/His-iBoNT/A (H227Y) construct was subcloned into a pET29b vector by PCR amplification of the open reading frame using oligonucleotide primers that added a lysine residue at the carboxyl end of the protein which can serve as a trypsin cleavage site. A 50 µL reaction was assembled with the Ndel-modified pRSETb/His-iBoNT/A (H227Y) expression construct as a template, reagents included with the Expand High Fidelity PCR system (Roche Applied Science, Indianapolis, IN) and the following two oligonucleotide primers: SL101 Primer Pair, *Nde*I sense oligonucleotide, 5'-CGCCATATGCCGTTCGTAAACAAACAGTTC-3' (SEPA ID NO: 108) and *Hind*III-K antisense oligonucleotide, 5'-CCCAAGCTTGTCGACTTTCAATGGGCGTTCTCC CCAACCGTC-3' (SEQ ID NO: 109). A polymerase chain reaction (PCR) mix contained 5 µL of 10x Buffer, 1 µL of deoxyribonucleotides (dNTPs), 1µL of *PfuUltra*™ High Fidelity DNA polymerase (2.5 units/µL), 125 ng of each primer, 100 ng of template DNA, and nuclease-free water to a final volume of 50 µL. The thermocycler conditions were: one cycle of 95 °C for 120 seconds; 25 cycles of 95 °C for 45 seconds, 55 °C for 60 seconds, and 72 °C for 3 minutes; one cycle of 72 °C for 7 minutes; and 10 °C to hold. The PCR-amplified product was digested with *Hind*III and *Nde*I at 37 °C for 2.5 hours to excise the iBoNT/A-K (H227Y) insert. The resulting restriction fragment was purified by the QIAquick Gel Extraction Kit (QIAGEN, Inc., Valencia, CA), and the fragment containing the open reading frame was subcloned into the pET29b vector (EMD Biosciences-Novagen, Madison, WI) that had been digested with restriction endonucleases *Hind*III and Ndel. The fragment and vector were ligated using T4 DNA ligase protocol to yield pET29bBoNT/A-KHis (H227Y). An aliquot from this ligation mixture was transformed by a standard heat-shock protocol into competent TOP10 cells (Invitrogen, Inc, Carlsbad, CA), plated onto 1.5% Luria-Bertani agar plates (pH 7.0) containing 50 µg/mL of Kanamycin, and placed in a 37 °C incubator for overnight growth. Candidate expression constructs were selected as Kanamycin-resistant colonies. Resistant colonies were used to inoculate 2 mL of Luria-Bertani media containing 50 µg/mL of Kanamycin that were then grown in a 37 °C incubator, shaking at 250 rpm, overnight. The bacteria cells were harvested by microcentrifugation and the plasmid DNA was isolated using QIAGEN miniprep kits (QIAGEN, Inc., Valencia, CA). Candidate expression constructs were screened by restriction digestion with *Nde*I and *Hind*III to determine the presence of the correct insert fragment. Cultures containing the desired expression construct were used to inoculate 1 L baffled flasks containing 200 mL of Luria-Bertani media containing 50 µg/mL of Kanamycin and placed in a 37 °C incubator, shaking at 250 rpm, for overnight growth. Purified plasmid DNA corresponding to an expression construct was isolated using the QIAGEN Maxi-prep method (QIAGEN, Inc., Valencia, CA) and sequenced to verify that the correct expression construct was made. This cloning strategy yielded a pET29b expression construct comprising the nucleic acid molecule of SEQ ID NO: 120 encoding a iBoNT/A (H227Y) operably-linked to a carboxyl terminal lysine residue followed by a trypsin cleavable polyhistidine affinity binding peptide of SEQ ID NO: 121.

### Example 12

### Construction of pET29bBoNT/A-KHis

A plasmid comprising a modified open reading frame encoding an active BoNT/A (FIG. 4), was prepared by *in vitro* site-directed mutagenesis of pET29brBoNT/A-KHis (H227Y). Correction of the inactivating H227Y mutation was accomplished in a single site-directed mutagenesis step using the procedure described in Example 3 and the following two oligonucleotides to yield pET29b/BoNT/A-KHis: Y227H Primer Pair, sense oligonucleotide, 5'-GTGACCTTGGCACATGAACTTATTCATGCCGGGCATC GCTTGTATGGAATCGCC-3' (SEQ ID NO: 102) and antisense oligonucleotide, 5'-GGCGATTCCATACA AGCGATGCCCGGCAT**G**AATAAGTTCATGTGCC**AA**GGTC**A**C-3' (SEQ ID NO: 103). The amino acid numbering corresponds to native sequence lacking an amino-terminal polyhistidine tag. The nucleotides that were changed to correct H227Y are shown in bold and underlined. This mutagenesis resulted in the modified open reading frame of SEO ID NO: 112 encoding the active BoNT/A-KHis of SEQ ID NO: 113.

Activity of BoNT/A-KHis candidates was identified by proteolytic cleavage of a GFP-SNAP25 substrate using a GFP-SNAP25 Fluorescence Release Assay, see, *e.g*., Lance E. Steward et al., GFP-SNAP25 Fluorescence Release Assay for Botulinum Neurotoxin Protease Activity, U.S. Patent Publication No. 2005/0100973 (May 12, 2005). Candidate pET29b/BoNT/A-KHis expression construct were transformed into chemically competent *E. coli* BL21 (DE3) cells (Invitrogen, Inc, Carlsbad, CA) using a heat-shock method, plated onto 1.5% Luria-Bertani agar plates (pH 7.0) containing 50 µg/mL of Kanamycin, and placed in a 37 °C incubator for overnight growth. Kanamycin-resistant colonies containing the pET29b/BoNT/A-KHis candidates were used to inoculate 1 mL cultures of ZYP-5052 autoinducing media containing 50 µg/mL of Kanamycin in Eppendorf Lid-Bac tubes fitted with membrane lids. The cultures were incubated in a thermomixer (1,400 rpm at 37 °C) located in a biosafety cabinet until turbid (approximately 7-8 hours). The temperature was then reduced to 22 °C and the cultures incubated for approximately 16 hours. The cells were collected by centrifugation (6,000x g at 4 °C for 30 minutes), decanted and frozen briefly at -80 °C to improve lysis. The cell pellets were defrosted on ice, each was resuspended in 350 µL of BugBuster^{®} lysis solution (EMD Biosciences-Novagen, Madison, WI) containing 25 units/mL of benzonase nuclease (EMD Biosciences-Novagen, Madison, WI), 1 KU/mL rLysozyme (EMD Biosciences-Novagen, Madison, WI) and 2 x Protease Inhibitor Cocktail III (EMD Biosciences-Novagen, Madison, WI) and the mixtures were incubated for 30 minutes at 22 °C, 400 rpm in the thermomixer. The lysates were clarified by centrifugation (36,000 xg at 4 °C for 15 minutes) and the supernatant solutions transferred to low-retention microcentrifuge tubes and placed on ice.

Activity of BoNT/A-KHis candidates was identified by proteolytic cleavage of a GFP-SNAP25 substrate. Each assay reaction contained 25 µL of 2x Toxin Reaction Buffer (100 mM *N*-(2-hydroxyethyl) piperazine-*N'*-(2-ethanesulfonic acid) (HEPES), pH 7.4; 20 µM zinc chloride; 20 mM dithiothreitol; 0.2 % (v/v) TWEEN-20^{®}, polyoxyethylene (20) sorbitan monolaureate), 10 µL of clarified lysate, and 15 µL of 50 µM GFP-SNAP25(₁₃₄₋₂₀₆) substrate. The control reactions contained 10 µL of either water or 0.2 µg/mL of LC/A in lieu of lysate. The reactions were assembled in triplicate, incubated at 37 °C for 1 hour and then quenched with 20 µL of 8 M guanidine hydrochloride. The quenched reactions were transferred to filter-plate wells containing 75 µL of TALON™ SuperFlow Co²⁺ affinity resin (BD Biosciences-Clontech, Palo Alto, CA) that had been conditioned by rinsing with 200 µL of deionized, distilled water and 200 µL of Assay Rinse Buffer (50 mM N-(2-hydroxyethyl) piperazine-*N'*-(2-ethanesulfonic acid) (HEPES), pH 7.4). Following 15 minutes incubation on the resin, the reaction solutions were eluted by vacuum filtration, collected in a black 96-well plate, passed over the resin beds twice more and collected after the final pass. Each resin bed was then rinsed with 210 µL of Assay Rinse Buffer which was eluted into the plate containing the reaction solutions. The fluorescence of the eluant reaction solutions was measured with a SpectraMax Gemini XS spectrophotometer (Molecular Devices, λ_{Ex} 474 nm; λ_{Em} 509 nm; 495 nm cutoff filter). The control reactions contained 10 µL of either water or 0.2 µg/mL of LC/A in lieu of lysate. Positive BoNT/A-KHis candidates showed significant protease activity (see FIG. 5).

### Example 13

### Expression of pET29b/BoNT/A-KHis

The following, example illustrates a procedure useful for expressing a BoNT/A from an expression construct disclosed in the present specification. An pET29b/BoNT/A-KHis expression construct was introduced into chemically competent *E. coli* BL21 (DE3) cells (Invitrogen, Inc, Carlsbad, CA) using a heat-shock transformation protocol. The heat-shock reaction was plated onto 1.5% Luria-Bertani agar plates (pH 7.0) containing 50 µg/mL of Kanamycin and placed in a 37 °C incubator for overnight growth. Kanamycin-resistant colonies of transformed *E. coli* containing pET29b/BoNT/A-KHis were used to inoculate a baffled flask containing 3.0 mL of PA-0.5G media containing 50 µg/mL of Kanamycin which was then placed in a 37 °C incubator, shaking at 250 rpm, for overnight growth. The resulting overnight starter culture was in turn used to inoculate a 3 L baffled flask containing ZYP-5052 autoinducing media containing 50 µg/mL of Kanamycin at a dilution of 1:1000. Culture volumes ranged from about 600 mL (20% flask volume) to about 750 mL (25% flask volume). These cultures were grown in a 37 °C incubator shaking at 250 rpm for approximately 5.5 hours and were then transferred to a 16 °C incubator shaking at 250 rpm for overnight expression. Cells were harvested by centrifugation (4,000 rpm at 4 °C for 20-30 minutes) and used immediately, or stored dry at -80 °C until needed.

### Example 14

### Purification and quantification of BoNT/A-KHis

The following example illustrates methods useful for purification and quantification of BoNT/A disclosed in the present specification. For immobilized metal affinity chromatography (IMAC) protein purification, *E. coli* BL21 (DE3) cell pellets used to express BoNT/A-KHis, as described in Example 8, were resuspended in Column Binding Buffer (25 mM *N*-(2-hydroxyethyl) piperazine-*N'*-(2-ethanesulfonic acid) (HEPES), pH 7.8; 500 mM sodium chloride; 10 mM imidazole; 2x Protease Inhibitor Cocktail Set III (EMD Biosciences-Calbiochem, San Diego CA); 5 units/mL of Benzonase (EMD Biosciences-Novagen, Madison, WI); 0.1% (v/v) Triton-X^{®} 100, 4-octylphenol polyethoxylate; 10% (v/v) glycerol), and then transferred to a cold Oakridge centrifuge tube. The cell suspension was sonicated on ice (10-12 pulses of 10 seconds at 40% amplitude with 60 seconds cooling intervals on a Branson Digital Sonifier) in order to lyse the cells and release the BoNT/A-KHis, and then centrifuged (16,000 rpm at 4 °C for 20 minutes) to clarify the lysate. An immobilized metal affinity chromatography column was prepared using a 20 mL Econo-Pac column support (Bio-Rad Laboratories, Hercules, CA) packed with 2.5-5.0 mL of TALON™ SuperFlow Co²⁺ affinity resin (BD Biosciences-Clontech, Palo Alto, CA), which was then equilibrated by rinsing with 5 column volumes of deionized, distilled water, followed by 5 column volumes of Column Binding Buffer. The clarified lysate was applied slowly to the equilibrated column by gravity flow (approximately 0.25-0.3 mL/minute). The column was then washed with 5 column volumes of Column Wash Buffer (*N*-(2-hydroxyethyl) piperazine-*N'*-(2-ethanesulfonic acid) (HEPES), pH 7.8; 500 mM sodium chloride; 10 mM imidazole; 0.1% (v/v) Triton-X^{®} 100, 4-octylphenol polyethoxylate; 10% (v/v) glycerol). BoNT/A-His was eluted with 20-30 mL of Column Elution Buffer (25 mM *N*-(2-hydroxyethyl) piperazine-*N*'-(2-ethanesulfonic acid) (HEPES), pH 7.8; 500 mM sodium chloride; 500 mM imidazole; 0.1% (v/v) Triton-X^{®} 100, 4-octylphenol polyethoxylate; 10% (v/v) glycerol) and collected in approximately twelve 1 mL fractions. The amount of BoNT/A-KHis contained in each elution fraction was determined by a Bradford dye assay and the five fractions with the strongest signal were considered the elution peak and pooled (see FIG. 6b). Total protein yield was determined by estimating the total protein concentration of the pooled peak elution fractions using bovine gamma globulin as a standard (Bio-Rad Laboratories, Hercules, CA).

For purification of a BoNT/A using a FPLC desalting column, a HiPrep™ 26/10 size exclusion column (Amersham Biosciences, Piscataway, NJ) was pre-equilibrated with 80 mL of 4°C Column Buffer (50 mM sodium phosphate, pH 6.5). After the column was equilibrated, a BoNT/A-KHis sample was applied to the size exclusion column with an isocratic mobile phase of 4 °C Column Buffer and at a flow rate of 10 mL/minute using a BioLogic DuoFlow chromatography system (Bio-Rad Laboratories, Hercules, CA). The desalted BoNT/A-His sample was collected as a single fraction of approximately 7-12 mL.

For purification of a BoNT/A using a FPLC ion exchange column, a BoNT/A-KHis sample that had been desalted following elution from an IMAC column was applied to a 1 mL UNO-S1™ cation exchange column (Bio-Rad Laboratories, Hercules, CA) using a BioLogic DuoFlow chromatography system (Bio-Rad Laboratories, Hercules, CA). The sample was applied to the column in 4 °C Column Buffer (50 mM sodium phosphate, pH 6.5) and eluted by linear gradient with 4 °C Elution Buffer (50 mM sodium phosphate, 1 M sodium chloride, pH 6.5) as follows: step 1, 5.0 mL of 5% Elution Buffer at a flow rate of 1 mL/minute; step 2, 20.0 mL of 5-30% Elution Buffer at a flow rate of 1 mL/minute; step 3, 2.0 mL of 50% Elution Buffer at a flow rate of 1.0 mL/minute; step 4, 4.0 mL of 100% Elution Buffer at a flow rate of 1.0 mL/minute; and step 5, 5.0 mL of 0% Elution Buffer at a flow rate of 1.0 mL/minute. Elution of peptides from the column was monitored at 280, 260, and 214 nm, and peaks absorbing above a minimum threshold (0.01 au) at 280 nm were collected. Most of the BoNT/A-KHis eluted at a sodium chloride concentration of approximately 100 to 200 mM. Average total yields of BoNT/A-His were approximately 7-12 mg/L as determined by a Bradford assay.

Expression of BoNT/A-KHis was analyzed by polyacrylamide gel electrophoresis. Samples purified using the procedure described above were added to 2x LDS Sample Buffer (Invitrogen, Inc, Carlsbad, CA) and peptides separated by MOPS polyacrylamide gel electrophoresis using NuPAGE^{®} Novex 4-12% Bis-Tris precast polyacrylamide gels (Invitrogen, Inc, Carlsbad, CA) under denaturing, reducing conditions. Gels were stained with SYPRO^{®} Ruby (Bio-Rad Laboratories, Hercules, CA) and the separated peptides imaged using a Fluor-S MAX Multilmager (Bio-Rad Laboratories, Hercules, CA) for quantification of peptide expression levels. The size and amount of the BoNT/A-His was determined by comparison to MagicMark™ protein molecular weight standards (Invitrogen, Inc, Carlsbad, CA). The gels revealed what appeared to be a full-length BoNT/A-KHis.

Expression of BoNT/A-KHis was also analyzed by Western blot analysis. Protein samples purified using the procedure described above were added to 2x LDS Sample Buffer (Invitrogen, Inc, Carlsbad, CA) and separated by MOPS polyacrylamide gel electrophoresis using NuPAGE^{®} Novex 4-12% Bis-Tris precast polyacrylamide gels (Invitrogen, Inc, Carlsbad, CA) under denaturing, reducing conditions. Separated peptides were transferred from the gel onto polyvinylidene fluoride (PVDF) membranes (Invitrogen, Inc, Carlsbad, CA) by Western blotting using a Trans-Blot^{®} SD semi-dry electrophoretic transfer cell apparatus (Bio-Rad Laboratories, Hercules, CA). PVDF membranes were blocked by incubating at room temperature for 2 hours in a solution containing 25 mM Tris-Buffered Saline (25 mM 2-amino-2-hydroxymethyl-1,3-propanediol hydrochloric acid (Tris-HCl)(pH 7.4), 137 mM sodium chloride, 2.7 mM potassium chloride), 0.1% TWEEN-20^{®}, polyoxyethylene (20) sorbitan monolaureate, 2% bovine serum albumin, 5% nonfat dry milk. Blocked membranes were incubated at 4 °C for overnight in Tris-Buffered Saline TWEEN-20^{®} (25 mM Tris-Buffered Saline, 0.1% TW EEN-20^{®}, polyoxyethylene (20) sorbitan monolaureate) containing one of the following primary antibodies as a probe: a 1:5,000 dilution of rabbit polyclonal anti-BoNT/A antiserum (Allergan, Inc.); or a 1:10,000 dilution of rabbit polyclonal anti-polyhistidine antiserum (Abcam Inc., Cambridge, MA). Primary antibody probed blots were washed three times for 15 minutes each time in Tris-Buffered Saline TWEEN-20^{®}. Washed membranes were incubated at room temperature for 2 hours in Tris-Buffered Saline TWEEN-20^{®} containing a 1:20,000 dilution of goat polyclonal anti-rabbit immunoglobulin G, heavy and light chains (IgG, H+L) antibody conjugated to horseradish peroxidase (HRP; Pierce Biotechnology, Inc., Rockford, IL) as a secondary antibody. Secondary antibody-probed blots were washed three times for 15 minutes each time in Tris-Buffered Saline TWEEN-20^{®}. Signal detection of the labeled BoNT/A-KHis was visualized using the ECL Plus™ Western Blot Detection System (Amersham Biosciences, Piscataway, NJ) and imaged with a Typhoon 9410 Variable Mode Imager (Amersham Biosciences, Piscataway, NJ) for quantification of peptide expression levels.

### Example 15

### Comparison of BoNT/A-His amounts expressed from modified and unmodified open reading frames

The amount of increased BoNT/A expressed from a modified open reading frame as compared to an unmodified open reading frame can be determined as follows. In separate reactions, a pET29b/BoNT/A-KHis expression construct comprising the modified open reading frame of SEQ ID NO: 112 and a pET29b/BoNT/A-KHis construct comprising the unmodified open reading frame of SEQ ID NO: 115 are introduced into chemically competent *E*. *coli* BL21 (DE3) cells (Invitrogen, Inc, Carlsbad, CA) using a heat-shock transformation protocol. The heat-shock reactions are plated onto 1.5% Luria-Bertani agar plates (pH 7.0) containing 50 µg/mL of Kanamycin and are placed in a 37 °C incubator for overnight growth. Kanamycin-resistant colonies of transformed *E*. *coli* containing pET29bBoNT/A-KHis constructs from both expression construct are used to inoculate separate 15 mL tubes containing 3.0 mL Kanamycin-resistance selective PA-0.5G media that are then placed in a 37 °C incubator, shaking at 250 rpm, for overnight growth. Approximately 600 µL of the resulting overnight starter culture from each construct are used to inoculate a 3.0 L baffled flask containing 600 mL Kanamycin-resistance, ZYP-5052 autoinducing media. The inoculated cultures are grown in a 37 °C incubator shaking at 250 rpm for approximately 5.5 hours and are then transferred to a 16 °C incubator shaking at 250 rpm for overnight expression. Cells are harvested by centrifugation (4,000 rpm at 4 °C for 20-30 minutes).

To analyze the BoNT/A-KHis expression amounts obtained from both the unmodified and modified open reading frames, BoNT/A-KHis is purified using the IMAC procedure (as described in Example 14). Expression from each culture is evaluated by a Bradford dye assay, polyacrylamide gel electrophoresis and Western blot analysis (as described in Example 14) in order to determine whether the amounts of BoNT/A-KHis produced from the modified open reading frame of SEQ ID NO: 112 is greater as compared to the amount of BoNT/A-KHis expressed from the unmodifed open reading frame of SEQ ID NO: 115. An approximately 12-fold increase in the amount of active BoNT/A-KHis expressed from a modified open reading frame is anticipated. Average amounts of IMAC purified active BoNT/A-KHis expressed from a modified open reading frame is expected to be approximately 12 mg/L, while IMAC purified active BoNT/A-KHis expressed from a unmodified open reading frame in an otherwise identical nucleic acid molecule is expected to be approximately 1 mg/L.

### Example 16

### Construction and expression of pRSET/BoNT/A-His

Restriction endonuclease sites suitable for cloning an operably linked nucleic acid molecule into a pRSET vector (Invitrogen, Inc, Carlsbad, CA) are incorporated into the 5'- and 3' ends of modified open reading frame SEQ ID NO: 3. This nucleic acid molecule is synthesized and a pUCBHB1/BoNT/A construct obtained as described in Example 2. This construct is digested with restriction enzymes that 1) excise the insert containing the open reading frame of SEQ ID NO: 3 encoding an active BoNT/A; and 2) enable this insert to be operably-linked to a pRSET vector. This insert is subcloned using a T4 DNA ligase procedure into a pRSET vector that is digested with appropriate restriction endonucleases to yield pRSET/BoNT/A-His (FIG. 7). The ligation mixture is transformed into chemically competent *E*. *coli* DH5α cells (Invitrogen, Inc, Carlsbad, CA) using a heat shock method, plated on 1.5% Luria-Bertani agar plates (pH 7.0) containing 100 µg/mL of Ampicillin, and placed in a 37 °C incubator for overnight growth. Bacteria containing expression constructs are identified as Ampicillin resistant colonies. Candidate constructs are isolated using an alkaline lysis plasmid mini-preparation procedure and analyzed by restriction endonuclease digest mapping to determine the presence and orientation of the insert. This cloning strategy yields a prokaryotic expression construct encoding an active BoNT/A operably linked to carboxyl-terminal polyhistidine binding peptide. A similar cloning strategy is used to make a pRSET construct containing the unmodified open reading frame of SEQ ID NO: 2 used as a control for expression levels, as well as, to produce pRSET expression constructs in which any one of the modified open reading frames of SEQ ID NO: 4 through SEQ ID NO: 33 is operably linked to a pRSET vector.

The amount of increased BoNT/A expression from a modified open reading frame is determined as follows. In separate reactions, a pRSET/BoNT/A-His expression construct comprising a modified open reading frame, such as, *e.g*., SEQ ID NO: 3 through SEQ ID NO: 33, and a pRSET/BoNT/A-His construct comprising an unmodified open reading frame, such as, *e.g*., SEQ ID NO: 2 are introduced into chemically competent bacterial cells suitable for expression of the pRSET expression construct using a standard transformation protocol, such as, *e.g*., a heat-shock transformation protocol. The transformation reactions are plated onto 1.5% Luria-Bertani agar plates (pH 7.0) containing suitable antibiotics and placed in a 37 °C incubator for overnight growth. Antibiotic-resistant colonies of transformed cells containing pRSET/BoNT/A-His constructs from both nucleic acid molecules are used to inoculate separate 15 mL tubes containing 3.0 mL antibiotic-resistance selective PA-0.5G media that are then placed in a 37 °C incubator, shaking at 250 rpm, for overnight growth. Approximately 600 µL of the resulting overnight starter culture from each construct is used to inoculate a 3.0 L baffled flask containing 600 mL of a suitable antibiotic-resistance growth media. The inoculated cultures are grown in a 37 °C incubator shaking at 250 rpm for approximately 5.5 hours and are then induced by adding IPTG to a final concentration of 0.5-1.0 mM, and the cultures are transferred to a 16 °C incubator shaking at 250 rpm for overnight expression. Cells are harvested by centrifugation (4,000 rpm at 4 °C for 20-30 minutes).

To analyze the BoNT/A-His expression levels obtained from both the native and modified nucleic acid molecules, BoNT/A-His is purified using the IMAC procedure (as described in Examples 6 and 14). Expression from each culture is evaluated by a Bradford dye assay, polyacrylamide gel electrophoresis and Western blot analysis using either anti-BoNT/A or anti-His antibodies (as described in Examples 6 and 14) in order to determine whether the amounts of BoNT/A-His produced from the modified open reading frame is greater relative to the amount of BoNT/A-His expressed from the unmodifed open reading frame of SEQ ID NO: 2.

### Example 17

### Construction and expression of pPICZ A/BoNT/A-myc-His

Restriction endonuclease sites suitable for cloning an operably linked nucleic acid molecule into a pPIC A vector (Invitrogen, Inc, Carisbad, CA) are incorporated into the 5'- and 3' ends of modified open reading frame SEQ ID NO: 36. This nucleic acid molecule is synthesized and a pUCBHB1/BoNT/A construct is obtained as described in Example 2. This construct is digested with restriction enzymes that 1) excise the insert containing the open reading frame of SEQ ID NO: 36 encoding an active BoNT/A; and 2) enable this insert to be operably-linked to a pPIC A vector. This insert is subcloned using a T4 DNA ligase procedure into a pPIC A vector that is digested with appropriate restriction endonucleases to yield pPIC A/BoNT/A-myc-His (FIG. 8). The ligation mixture is transformed into chemically competent *E*. *coli* DH5α cells (Invitrogen, Inc, Carlsbad, CA) using a heat shock method, plated on 1.5% low salt Luria-Bertani agar plates (pH 7.5) containing 25 µg/mL of Zeocin™, and placed in a 37°C incubator for overnight growth. Bacteria containing expression constructs are identified as Zeocin™ resistant colonies. Candidate constructs are isolated using an alkaline lysis plasmid mini-preparation procedure and analyzed by restriction endonuclease digest mapping to determine the presence and orientation of the insert. This cloning strategy yields a yeast expression construct encoding an active BoNT/A operably linked to carboxyl-terminal c-myc and polyhistidine binding peptides. A similar cloning strategy is used to make a pPIC A expression construct containing the unmodifed open reading frame of SEQ ID NO: 2 used as a control for expression levels, as well as, to produce pPIC A expression constructs in which any one of the modified open reading frames of SEQ ID NO: 34, SEQ ID NO: 35, SEQ ID NO: 37 through SEQ ID NO: 45 is operably linked to a pPIC A vector.

To construct a yeast cell line expressing an active BoNT/A, pPICZ A/BoNT/A-myc-His is digested with a suitable restriction endonuclease (*i.e., Sac*I, *Pme*I or *Bst*XI) and the resulting linearized expression construct is transformed into an appropriate *P. pastoris* Mut^{s} strain KM71 H using an electroporation method. The transformation mixture is plated on 1.5% YPDS agar plates (pH 7.5) containing 100 µg/mL of Zeocin™ and placed in a 28-30 °C incubator for 1-3 days of growth. Selection of transformants integrating the pPICZ A/BoNT/A-myc-His at the 5' AOX1 locus is determined by colony resistance to Zeocin™. A similar strategy is used to make a cell line containing a pPICZ A expression construct containing SEQ ID NO: 2 used as a control for expression levels. Cell lines integrating a pPICZ A/BoNT/A-myc-His construct is tested for BoNT/A-myc-His expression using a small-scale expression test. Isolated colonies from test cell lines that have integrated pPICZ A/BoNT/A-myc-His are used to inoculate 1.0 L baffled flasks containing 100 mL of MGYH media and grown at about 28-30 °C in a shaker incubator (250 rpm) until the culture reaches an OD₆₀₀=2-6 (approximately 16-18 hours). Cells are harvested by centrifugation (3,000x g at 22 °C for 5 minutes). To induce expression, the cell pellet is resuspended in 15 mL of MMH media and 100% methanol is added to a final concentration of 0.5%. Cultures are grown at about 28-30 °C in a shaker incubator (250 rpm) for six days. Additional 100% methanol is added to the culture every 24 hours to a final concentration of 0.5%. A 1.0 mL test aliquot is taken from the culture every 24 hours starting at time zero and ending at time 144 hours. Cells are harvested from the aliquots by microcentrifugation to pellet the cells and lysed using three freeze-thaw rounds consisting of -80 °C for 5 minutes, then 37 °C for 5 minutes. Lysis samples are added to 2x LDS Sample Buffer (Invitrogen, Inc, Carlsbad, CA) and expression from established cell lines is measured by Western blot analysis (as described in Examples 6 and 14) using either anti-BoNT/A, anti-myc or anti-His antibodies in order to identify lines expressing increased amounts of BoNT/A-myc-His produced from SEQ ID NO: 36 relative to established cell lines expressing BoNT/A-myc-His from the SEQ ID NO: 2 control. The *P. pastoris* Mut^{s} KM71 H cell line showing the highest expression level of BoNT/A-myc-His relative to the SEQ ID NO: 2 control is selected for large-scale expression using commercial fermentation procedures. Procedures for large-scale expression are as outlined above except the culture volume is approximately 2.5 L MGYH media grown in a 5 L BioFlo 3000 fermentor and concentrations of all reagents will be proportionally increased for this volume. For greater details on all procedures described in this example, see EasySeleCt™ Pichia Expression Kit, version G, A Manual of Methods for Expression of Recombinant Proteins Using pPICZ and pPICZα in Pichia pastoris, 122701, 25-0172 (Invitrogen, Inc, Carlsbad, CA).

### Example 18

### Construction and expression of pMET/BoNT/A-V5-His

Restriction endonuclease sites suitable for cloning an operably linked nucleic acid molecule into a pMET vector (Invitrogen, Inc, Carlsbad, CA) are incorporated into the 5'- and 3' ends of modified open reading frame SEQ ID NO: 36. This nucleic acid molecule is synthesized and a pUCBHB1/BoNT/A construct is obtained as described in Example 2. This construct is digested with restriction enzymes that 1) excise the insert containing the open reading frame of SEQ ID NO: 36 encoding an active BoNT/A; and 2) enable this insert to be operably-linked to a pMET vector. This insert is subcloned using a T4 DNA ligase procedure into a pMET vector that is digested with appropriate restriction endonucleases to yield pMET/BoNT/A-V5-His (FIG. 9). The ligation mixture is transformed into chemically competent *E*. *coli* DH5α cells (Invitrogen, Inc, Carlsbad, CA) using a heat shock method, plated on 1.5% low salt Luria-Bertani agar plates (pH 7.5) containing 100 µg/mL of Ampicillin, and placed in a 37 °C incubator for overnight growth. Bacteria containing expression constructs are identified as Ampicillin resistant colonies. Candidate constructs are isolated using an alkaline lysis plasmid mini-preparation procedure and analyzed by restriction endonuclease digest mapping to determine the presence and orientation of the insert. This cloning strategy yields a yeast expression construct encoding an active BoNT/A operably linked to carboxyl-terminal V5 and polyhistidine binding peptides. A similar cloning strategy is used to make a pMET expression construct containing the unmodifed open reading frame of SEQ ID NO: 2 used as a control for expression levels, as well as, to produce pMET expression constructs in which any one of the modified open reading frames of SEQ ID NO: 34, SEQ ID NO: 35, SEQ ID NO: 37 through SEQ ID NO: 45 is operably linked to a pMET vector.

To construct a yeast cell line expressing an active BoNT/A, pMETBoNT/A-V5-His is digested with a suitable restriction endonuclease (*i.e., Apa*I, *Asc*I, *Fse*I, *Pac*I, *Kpn*I or *Pst*I) and the resulting linearized expression construct is transformed into an appropriate *P. methanolica* Mut^{s} strain PMAD16 using an electroporation method. The transformation mixture is plated on 1.5% MD agar plates (pH 7.5) lacking adenine and grown in a 28-30 °C incubator for 3-4 days. Selection of transformants integrating the pMET/BoNT/A-V5-His is determined by colony growth on adenine-deficient media. A similar strategy is used to make a cell line containing a pMET expression construct containing SEQ ID NO: 2 used as a control for expression levels. Ade+ cell lines integrating a pMET/BoNT/A-V5-His construct are tested for BoNT/A-myc-His expression using a small-scale expression test. Isolated Ade+ colonies from test cell lines that have integrated pMETBoNT/A-V5-His are used to inoculate 15 mL of BMDY media and cells are grown at about 28-30 °C in a shaker incubator (250 rpm) until the culture reaches an OD₆₀₀=2-10 (approximately 16-18 hours). Cells are harvested by centrifugation (1,500 x *g* at 22°C for 5 minutes). To induce expression, cell pellets are resuspended in 5 mL of BMMY media and cultures are grown at about 28-30 °C in a shaker incubator (250 rpm). After 24 hours, a 500 µL aliquot is removed, methanol is added to a final concentration of 0.5% and the cultures are grown at about 28-30 °C in a shaker incubator (250 rpm). A 500 µL aliquot is removed and additional methanol is added to a final concentration of 0.5% to the culture every 24 hours for 3-5 days. Harvested cells are centrifuged (1,500x *g* at 4 °C for 5 minutes), washed once in water and cell pellets stored at -80 °C until needed. To detect expression of the induced BoNT/A-V5-His, the cell pellets of each time point are lysed using an acid-washed glass bead method. Lysis samples are added to 2x LDS Sample Buffer (Invitrogen, Inc, Carlsbad, CA) and expression from established cell lines is measured by Western blot analysis (as described in Examples 6 and 14) using either anti-BoNT/A, anti-V5 or anti-His antibodies in order to identify lines expressing increased amounts of BoNT/A-V5-His produced from SEQ ID NO: 36 relative to established cell lines expressing BoNT/A-V5-His from the SEQ ID NO: 2 control. The *P. methanolica* Mut^{s} PMAD16 cell line showing the highest expression level of BoNT/A-V5-His relative to the SEQ ID NO: 2 control is selected for large-scale expression using commercial fermentation procedures. Procedures for large-scale expression are as outlined above except the culture volume is approximately 2.5 L BMDY/BMMY media grown in a 5 L BioFlo 3000 fermentor and concentrations of all reagents will be proportionally increased for this volume. For greater details on all procedures described in this example, see P. methanolica Expression Kit, version C, A Manual of Methods for Expression of Recombinant Proteins in Pichia methanolica, 062101, 25-0288 (Invitrogen, Inc, Carlsbad, CA).

### Example 19

### Construction and expression of pYES2/BoNT/A-V5-His

Restriction endonuclease sites suitable for cloning an operably linked nucleic acid molecule into a pYES2 vector (Invitrogen, Inc, Carlsbad, CA) are incorporated into the 5' and 3' ends of open reading frame SEQ ID NO: 39. This nucleic acid molecule is synthesized and a pUCBHB1/BoNT/A construct is obtained as described in Example 2. This construct is digested with restriction enzymes that 1) excise the insert containing the open reading frame of SEQ ID NO: 39 encoding an active BoNT/A; and 2) enable this insert to be operably-linked to a pYES2 vector. This insert is subcloned using a T4 DNA ligase procedure into a pYES2 vector that is digested with appropriate restriction endonucleases to yield pYES2/BoNT/A-V5-His (FIG. 10). The ligation mixture is transformed into chemically competent *E*. *coli* DH5α cells (Invitrogen, Inc, Carlsbad, CA) using a heat shock method, plated on 1.5% low salt Luria-Bertani agar plates (pH 7.5) containing 100 µg/mL of Ampicillin, and placed in a 37 °C incubator for overnight growth. Bacteria containing expression constructs are identified as Ampicillin resistant colonies. Candidate constructs are isolated using an alkaline lysis plasmid mini-preparation procedure and analyzed by restriction endonuclease digest mapping to determine the presence and orientation of the insert. This cloning strategy yields a yeast expression construct encoding an active BoNT/A operably linked to carboxyl-terminal V5 and polyhistidine binding peptides. A similar cloning strategy is used to make a pYES2 expression construct containing the unmodifed open reading frame of SEQ ID NO: 2 used as a control for expression levels, as well as, to produce pYES2 expression constructs in which any one of the modified open reading frames of SEQ ID NO: 34 through SEQ ID NO: 38 and SEQ ID NO: 40 through SEQ ID NO: 45 is operably linked to a pYES2 vector.

To construct a yeast cell line expressing an active BoNT/A, pYES2/BoNT/A-V5-His is transformed into competent *S. cerevisiae* strain INVSc1 using a Lithium-based transformation method. The transformation mixture is plated on 2% SC minimal media agar plates (pH 7.5) containing 2% glucose, that either have 0.01% uracil or lack uracil and placed in a 28-30 °C incubator for 1-3 days of growth. Selection of transformants containing pYES2/BoNT/A-V5-His is determined by colony growth only on plates containing uracil. A similar strategy is used to make cells containing a pYES2 expression construct containing SEQ ID NO: 2 used as a control for expression levels. Cells containing a pYES2/BoNT/A-V5-His construct are tested for BoNT/A-V5-His expression using a small-scale expression test. Isolated colonies from test cells containing pYES2/BoNT/A-V5-His are used to inoculate 50 mL tubes containing 15 mL of SC media containing 2% glucose and 0.01% uracil and grown overnight at about 28-30 °C in a shaker incubator (250 rpm). The OD₆₀₀ of overnight cultures are determined and aliquoted to obtain a cell concentration of OD₆₀₀ of 0.4 in a 50 mL volume. These aliquots are centrifuged (1,500x g at 22 °C for 5 minutes) and the resulting cell pellet resuspended in SC media containing 20% galactose and 10% raffinose. Cells are grown at about 28-30 °C in a shaker incubator (250 rpm) and 5 mL aliquots are taken at 0 hours, 4 hours, 8 hours, 12 hours, 16 hours and 24 hours and OD₆₀₀ concentrations are determined for each sample. Harvested cells are centrifuged (1,500x *g* at 4 °C for 5 minutes), washed once in water and cell pellets stored at -80 °C until needed. To detect expression of the induced BoNT/A-V5-His, the cell pellets of each time point are lysed using an acid-washed glass bead method. Lysis samples are added to 2x LDS Sample Buffer (Invitrogen, Inc, Carlsbad, CA) and expression from each time point is measured by Western blot analysis (as described in Examples 6 and 14) using either anti-BoNT/A, anti-V5 or anti-His antibodies to identify the optimal induction time necessary to obtain maximal BoNT/A-V5-His expression. The induction conditions resulting in the highest expression level of BoNT/A-V5-His encoded by the modified open reading frame as compared to the unmodified open reading frame of SEQ ID NO: 2 control are selected for large-scale expression using commercial fermentation procedures. Procedures for large-scale expression are as outlined above except the culture volume is approximately 2.5 L SC media grown in a 5 L BioFlo 3000 fermentor and concentrations of all reagents will be proportionally increased for this volume. For greater details on all procedures described in this example, see pYES2/CT, pYES3/CT, and pYC2/CT Yeast Expression Vectors with C-terminal Tags and Auxotrophic Selection Markers, version E, 25-0304, Jan. 27, 2003 (Invitrogen, Inc, Carlsbad, CA).

### Example 20

### Construction and expression of pFastBacHTMis-BoNT/A

Restriction endonuclease sites suitable for cloning an operably linked nucleic acid molecule into a pFastBacHT vector (Invitrogen, Inc, Carlsbad, CA) are incorporated into the 5'- and 3' ends of modified open reading frame SEQ ID NO: 63. This nucleic acid molecule is synthesized and a pUCBHB1/BoNT/A construct is obtained as described in Example 2. This construct is digested with restriction enzymes that 1) excise the insert containing the open reading frame of SEQ ID NO: 63 encoding an active BoNT/A; and 2) enable this insert to be operably-linked to a pFastBacHT vector. This insert is subcloned using a T4 DNA ligase procedure into a pFastBacHT vector that is digested with appropriate restriction endonucleases to yield pFastBacHT/His-BoNT/A (FIG. 11). The ligation mixture is transformed into chemically competent *E. coli* DH5α cells (Invitrogen, Inc, Carlsbad, CA) using a heat shock method, plated on 1.5% Luria-Bertani agar plates (pH 7.0) containing 100 µg/mL of Ampicillin, and placed in a 37 °C incubator for overnight growth. Bacteria containing expression constructs are identified as Ampicillin resistant colonies. Candidate constructs are isolated using an alkaline lysis plasmid mini-preparation procedure and analyzed by restriction endonuclease digest mapping to determine the presence and orientation of the insert. This cloning strategy yields a baculovirus transfer construct encoding an active BoNT/A operably linked to an amino-terminal, TEV cleavable; polyhistidine affinity binding peptide. A similar cloning strategy is used to make a pFastBacHT construct containing the unmodified open reading frame of SEQ ID NO: 2 used as a control for expression levels, as well as, to produce pFastBacHT expression constructs in which any one of the modified open reading frames of SEQ ID NO: 58, SEQ ID NO: 59, SEQ ID NO: 60, SEQ ID NO: 61 or SEQ ID NO: 62 is operably linked to a pFastBacHT vector.

To make a bacmid construct expressing an active BoNT/A, pFastBacHT/His-BoNT/A constructs are transformed by a heat shock method into MAX Efficiency^{®} DH10Bac™ *E*. *coli* cells for transposition into a bacmid. The transformation mixture is plated on 1.5% Luria-Bertani agar plates (pH 7.0) containing 50 µg/mL of Kanamycin, 7 µg/mL of Gentamicin, 10 µg/mL of Tetracycline, 100 µg/mL of Bluo-gal and 40 µg/mL of IPTG and is grown for approximately 48 hours to isolate recombinant bacmid DNA. Candidate bacmid constructs are isolated as white colonies that are Kanamycin, Gentamicin and Tetracycline resistant. Candidate bacmid constructs are isolated using an alkaline lysis plasmid mini-preparation procedure and analyzed by restriction endonuclease digest mapping to determine the presence and orientation of the insert. A similar strategy is used to generate a recombinant baculoviral stock containing the unmodified open reading frame of SEQ ID NO: 2 construct. A P1 recombinant baculovirus stock is isolated by transfecting approximately 5x10⁵ Sf9 cells plated in a 35 mm tissue culture dish containing 2 mL of complete Sf-900 II SFM media with 50 units/mL of penicillin and 50 µg/mL of streptomycin, with 1.0 mL of transfection solution. The transfection solution is prepared by adding 800 µL of unsupplemented Grace's media to 200 µL of unsupplemented Grace's media, containing 1.0 µg of a purified bacmid His-BoNT/A construct and 6 µL of Cellfectin^{®} Reagent preincubated for 30 minutes to allow formation of DNA:lipid complexes. Cells are incubated with this trasfection solution for 5 hours in a 27 °C incubator, after which time this solution is replaced with 2.0 mL of complete Sf-900 II SFM media with 50 units/mL of penicillin and 50 µg/mL of streptomycin. Sf9 cells are grown for approximately 72 hours in a 27 °C incubator to allow for the release of virus into the medium. The virus is harvested by transferring the media from virally-infected insect cells to 15 mL snap-cap tubes and centrifuging tubes at 500x g for 5 minutes to remove debris. The clarified supernatant is transferred to fresh 15 mL snap-cap tubes and should contain approximately 1x10⁶ to 10⁷ plaque forming units (pfu) of baculovirus. This P1 viral stock is then amplified to generate a P2 recombinant baculovirus stock. About 2x10⁶ Sf9 cells are plated in a 35 mm culture dish containing 2 mL of Sf-900 II SFM media, supplemented with 50 units/mL of penicillin and 50 µg/mL of streptomycin, are inoculated with 400 µL of the P1 recombinant baculovirus stock (approximately 5x10⁶ pfu/ml) and incubated for approximately 48 hours in a 27 °C incubator. The virus is harvested by transferring the media to 15 mL snap-cap tubes and centrifuging tubes at 500x *g* for 5 minutes to remove debris. The clarified supernatant is transferred to fresh 15 mL snap-cap tubes and should contain approximately 1x10⁷ to 10⁸ pfu of baculovirus.

To express His-BoNT/A using a baculoviral expression system, about 2x10⁶ Sf9 cells are plated in a 35 mm culture dish containing 2 mL of Sf-900 II SFM media, supplemented with 50 units/mL of penicillin and 50 µg/mL of streptomycin, are inoculated with approximately 4 µL of the P1 recombinant baculovirus stock (approximately 5x10⁷ pfu/ml) and incubated for approximately 48 hours in a 27 °C incubator. Both media and cells are collected for BoNT/A-His expression. Media is harvested by transferring the media to 15 mL snap-cap tubes and centrifuging tubes at 500x g for 5 minutes to remove debris. Cells are harvested by rinsing cells once with 3.0 mL of 100 mM phosphate-buffered saline, pH 7.4 and lysing cells with a buffer containing 62.6 mM 2-amino-2-hydroxymethyl-1,3-propanediol hydrochloric acid (Tris-HCl), pH 6.8 and 2% sodium lauryl sulfate (SDS). Both media and cell samples are added to 2x LDS Sample Buffer (Invitrogen, Inc, Carlsbad, CA) and expression is measured by Western blot analysis (as described in Examples 6 and 14) using either anti-BoNT/A or anti-His antibodies in order to identify P2 baculoviral stocks expressing increased amounts of His-BoNT/A produced from SEQ ID NO: 63 relative to stocks expressing His-BoNT/A from the SEQ ID NO: 2 control. For greater details on all procedures described in this example, see Bac-to-Bac^{®} Baculovirus Expression System, version D, An Efficient Site-specific Transposition System to Generate Baculovirus for High-level Expression of Recombinant Proteins, 10359 (Invitrogen, Inc, Carlsbad, CA).

### Example 21

### Construction and expression of pBACgus3/gp64-BoNT/A-His

Restriction endonuclease sites suitable for cloning an operably linked nucleic acid molecule into a pBACgus3 vector (EMD Biosciences-Novagen, Madison, WI) are incorporated into the 5'- and 3' ends of modified open reading frame SEQ ID NO: 63. This nucleic acid molecule is synthesized and a pUCBHB1/BoNT/A construct is obtained as described in Example 2. This construct is digested with restriction enzymes that 1) excise the insert containing the open reading frame of SEQ ID NO: 63 encoding an active BoNT/A; and 2) enable this insert to be operably-linked to a pBACgus3 vector. This insert is subcloned using a T4 DNA ligase procedure into a pBACgus3 vector that is digested with appropriate restriction endonucleases to yield pBACgus3/BoNT/A-His (FIG. 12). The ligation mixture is transformed into chemically competent *E. coli* DH5α cells (invitrogen, Inc, Carlsbad, CA) using a heat shock method, plated on 1.5% Luria-Bertani agar plates (pH 7.0) containing 100 µg/mL of Ampicillin, and placed in a 37 °C incubator for overnight growth. Bacteria containing expression constructs are identified as Ampicillin resistant colonies. Candidate constructs are isolated using an alkaline lysis plasmid mini-preparation procedure and analyzed by restriction endonuclease digest mapping to determine the presence and orientation of the insert. This cloning strategy yields a baculovirus transfer construct encoding an active BoNT/A operably linked to an amino-terminal gp64 signal peptide and a carboxyl-terminal, thrombin cleavable, polyhistidine affinity binding peptide. A similar cloning strategy is used to make a pBACgus3 construct containing the unmodified open reading frame of SEQ ID NO: 2 used as a control for expression levels, as well as, to produce pBACgus3 expression constructs in which any one of the modified open reading frames of SEQ ID NO: 58, SEQ ID NO: 59, SEQ ID NO: 60, SEQ ID NO: 61 or SEQ ID NO: 62 is operably linked to a pBACgus3 vector.

To express BoNT/A-His using a baculoviral expression system, about 2.5x10⁶ Sf9 cells are plated in four 60 mm culture dishes containing 2 mL of BacVector^{®} Insect media (EMD Biosciences-Novagen, Madison, WI) and incubated for approximately 20 minutes in a 28 °C incubator. For each transfection, a 50 µL transfection solution is prepared in a 6 mL polystyrene tube by adding 25 µL of BacVector^{®} Insect media containing 100 ng pBACgus3/gp64-BoNT/A-His and 500 ng TlowE transfer plasmid to 25 µL of diluted Insect GeneJuice^{®} containing 5 µL Insect GeneJuice^{®} (EMD Biosciences-Novagen, Madison, WI) and 20 µL nuclease-free water and this solution is incubated for approximately 15 minutes. After the 15 minute incubation, add 450 µL BacVector^{®} media to the transfection solution and mix gently. Using this stock transfection solution as the 1/10 dilution make additional transfection solutions of 1/50, 1/250 and 1/1250 dilutions. Add 100 µL of a transfection solution to the Sf9 cells from one of the four 60 mm culture dishes, twice washed with antibiotic-free, serum-free BacVector^{®} Insect media and incubate at 22 °C. After one hour, add 6 mL of 1% BacPlaque agarose-BacVector^{®} Insect media containing 5% bovine serum albumin. After the agarose is solidified, add 2 mL BacVector^{®} Insect media containing 5% bovine serum albumin to the transfected cells and transfer the cells to a 28 °C incubator for 3-5 days until plaques are visible. After 3-5 days post-transfection, plaques in the monolayer will be stained for ß-glucuronidase reporter gene activity to test for the presence of recombinant virus plaques containing pBACgus3BoNT/A-His by incubating the washed monolayer with 2 mL of BacVector^{®} Insect media containing 30 µL of 20 mg/mL X-Gluc Solution (EMD Biosciences-Novagen, Madison, WI) for approximately 2 hours in a 28 °C incubator.

After identifying candidate recombinant virus plaques, several candidate virus plaques are eluted and plaque purified. To elute a recombinant virus, transfer a plug containing a recombinant virus plaque with a sterile Pasteur pipet to 1 mL BacVector^{®} Insect media (EMD Biosciences-Novagen, Madison, WI) in a sterile screw-cap vial. Incubate the vial for approximately 2 hours at 22 °C or for approximately 16 hours at 4 °C. For each recombinant virus plaque, 2.5x10⁵ Sf9 cells are plated in 35 mm culture dishes containing 2 mL of BacVector^{®} Insect media (EMD Biosciences-Novagen, Madison, WI) and incubated for approximately 20 minutes in a 28 °C incubator. Remove the media and add 200 µL of eluted recombinant virus. After one hour, add 2 mL of 1% BacPlaque agarose-BacVector^{®} Insect media containing 5% bovine serum albumin. After the agarose is solidified, add 1 mL BacVector^{®} Insect media containing 5% bovine serum albumin to the transfected cells and transfer the cells to a 28 °C incubator for 3-5 days until plaques are visible. After 3-5 days post-transfection, plaques in the monolayer will be stained for ß-glucuronidase reporter gene activity to test for the presence of recombinant virus plaques containing pBACgus3/BoNT/A-His by incubating the washed monolayer with 2 mL of BacVector^{®} Insect media containing 30 µL of 20 mg/mL X-Gluc Solution (EMD Biosciences-Novagen, Madison, WI) for approximately 2 hours in a 28 °C incubator.

To prepare a seed stock of virus, elute a recombinant virus by transferring a plug containing a recombinant virus plaque with a sterile Pasteur pipet to 1 mL BacVector^{®} Insect media (EMD Biosciences-Novagen, Madison, WI) in a sterile screw-cap vial. Incubate the vial for approximately 16 hours at 4 °C. Approximately 5x10⁵ Sf9 cells are plated in T-25 flask containing 5 mL of BacVector^{®} Insect media (EMD Biosciences-Novagen, Madison, WI) and are incubated for approximately 20 minutes in a 28 °C incubator. Remove the media and add 300 µL of eluted recombinant virus. After one hour, add 5 mL BacVector^{®} Insect media containing 5% bovine serum albumin to the transfected cells and transfer the cells to a 28 °C incubator for 3-5 days until the majority of cells become unattached and unhealthy. The virus is harvested by transferring the media to 15 mL snap-cap tubes and centrifuging tubes at 1000x g for 5 minutes to remove debris. The clarified supernatant is transferred to fresh 15 mL snap-cap tubes and are stored at 4 °C.

To prepare a high titer stock of virus, approximately 2x10⁷ Sf9 cells are plated in T-75 flask containing 10 mL of BacVector^{®} Insect media (EMD Biosciences-Novagen, Madison, WI) and are incubated for approximately 20 minutes in a 28 °C incubator. Remove the media and add 500 µL of virus seed stock. After one hour, add 10 mL BacVector^{®} Insect media containing 5% bovine serum albumin to the transfected cells and transfer the cells to a 28 °C incubator for 3-5 days until the majority of cells become unattached and unhealthy. The virus is harvested by transferring the media to 15 mL snap-cap tubes and centrifuging tubes at 1000x g for 5 minutes to remove debris. The clarified supernatant is transferred to fresh 15 mL snap-cap tubes and are stored at 4 °C . High titer virus stocks should contain approximately 2x10⁸ to 3x10⁹ pfu of baculovirus.

To express gp64-BoNT/A-His using a baculoviral expression system, about 1.25x10⁸ Sf9 cells are seeded in a 1L flask containing 250 mL of BacVector^{®} Insect media and are grown in an orbital shaker (150 rpm) to a cell density of approximately 5x10⁸. The culture is inoculated with approximately 2.5x10⁹ of high titer stock recombinant baculovirus and incubated for approximately 48 hours in a 28°C orbital shaker (150 rpm). Media is harvested by transferring the media to tubes and centrifuging tubes at 500x *g* for 5 minutes to remove debris. Media samples are added to 2x LDS Sample Buffer (Invitrogen, Inc, Carlsbad, CA) and expression is measured by Western blot analysis (as described in Examples 6 and 14) using either anti-BoNT/A or anti-His antibodies in order to identify baculoviral stocks expressing increased amounts of His-BoNT/A produced from SEQ ID NO: 63 relative to stocks expressing gp64-BoNT/A-His from the SEQ ID NO: 2 control. For greater details on all procedures described in this example, see BacVector^{®} Transfection Kits, TB216, revision A 1203 (EMD Biosciences-Novagen, Madison, WI).

### Example 22

### Construction and expression of pMTBiP-BoNT/A-V5-His

Restriction endonuclease sites suitable for cloning an operably linked nucleic acid molecule into a pMT vector (Invitrogen, Inc, Carlsbad, CA) are incorporated into the 5'- and 3' ends of modified open reading frame SEQ ID NO: 60. This nucleic acid molecule is synthesized and a pUCBHB1/BoNT/A construct obtained as described in Example 2. This construct is digested with restriction enzymes that 1) excise the insert containing the open reading frame of SEQ ID NO: 60 encoding an active BoNT/A; and 2) enable this insert to be operably-linked to a pMT vector. This insert is subcloned using a T4 DNA ligase procedure into a pMT vector that is digested with appropriate restriction endonucleases to yield pMT/BiP-BoNT/A-V5-His (FIG. 13). The ligation mixture is transformed into chemically competent *E*. *coli* DH5α cells (Invitrogen, Inc, Carlsbad, CA) using a heat shock method, plated on 1.5% Luria-Bertani agar plates (pH 7.0) containing 100 µg/mL of Ampicillin, and placed in a 37 °C incubator for overnight growth. Bacteria containing expression constructs are identified as Ampicillin resistant colonies. Candidate constructs are isolated using an alkaline lysis plasmid mini-preparation procedure and analyzed by restriction endonuclease digest mapping to determine the presence and orientation of the insert. This cloning strategy will yield an insect expression construct encoding an active BoNT/A operably linked to carboxyl-terminal V5 and polyhistidine binding peptides. A similar cloning strategy is used to make a pMT construct containing the unmodified open reading frame of SEQ ID NO: 2 used as a control for expression levels, as well as, to produce pMT expression constructs in which any one of the modified open reading frames of SEQ ID NO: 58, SEQ ID NO: 59, SEQ ID NO: 61, SEQ ID NO: 62 or SEQ ID NO: 63 is operably linked to a pMT vector.

To transiently express active BoNT/A-V5-His in insect cells, about 3x10⁶ S2 cells are plated in a 35 mm tissue culture dish containing 3 mL of Schneider's Drosophila media and are grown in a 28 °C incubator until cells reach a density of approximately 9x10⁶ cells/ml (6-16 hours). A 600 µL transfection solution is prepared by adding 300 µL of 2x HEPES-Buffered Saline, pH 7.1 (50 mM *N*-(2-hydroxyethyl) piperazine-*N'*-(2-ethanesulfonic acid) (HEPES), pH 7.4; 1.5 mM sodium phosphate (monobasic); 280 mM sodium chloride) to 300 µL of 240 mM calcium chloride containing 19 µg of pMTBiP-BoNT/A-V5-His and this solution is incubated for approximately 30 minutes. The transfection solution is added to S2 cells and the cells are incubated in a 28 °C incubator for approximately 16-24 hours. The transfection media is replaced with 3 mL of fresh Schneider's Drosophila media containing 500 µM copper sulfate to induce expression. Cells are incubated in a 28 °C incubator for an additional 48 hours. Media is harvested by transferring the media to 15 mL snap-cap tubes and centrifuging tubes at 500x *g* for 5 minutes to remove debris. Samples are added to 2x LDS Sample Buffer (Invitrogen, Inc, Carlsbad, CA) and expression is measured by Western blot analysis (as described in Examples 6 and 14) using either anti-BoNT/A, anti-V5 or anti-His antibodies in order to identify pMT constructs expressing increased amounts of BiP-BoNT/A-V5-His produced from SEQ ID NO: 60 relative to constructs expressing BoNT/A-V5-His from the SEX ID NO: 2 control.

To generate a stably-integrated insect cell line expressing active BoNT/A-V5-His, approximately 3x10⁶ S2 cells are plated in a 35 mm tissue culture dish containing 3 mL of Schneider's Drosophila media and grown in a 28 °C incubator until cells reach a density of about 9x10⁶ cells/ml (6-16 hours). A 600 µL transfection solution is prepared by adding 300 µL of 2x HEPES-Buffered Saline, pH 7.1 (50 mM N-(2-hydroxyethyl) piperazine-*N'*-(2-ethanesutfonic acid) (HEPES), pH 7.4; 1.5 mM sodium phosphate (monobasic); 280 mM sodium chloride) to 300 µL of 240 mM calcium chloride containing 19 µg of pMT/BiP-BoNT/A-V5-His and 1 µg of pCoHygro, and this solution is incubated for approximately 30 minutes. The transfection solution is added to S2 cells and incubate in a 28 °C incubator for approximately 16-24 hours. Transfection media is replaced with 3 mL of fresh Schneider's Drosophila media and the cells are incubated in a 28 °C incubator for approximately 48 hours. Media is replaced with 3 mL of fresh Schneider's Drosophila media containing approximately 500 µg/mL of hygromycin-B. Cells are incubated in a 28 °C incubator for approximately 3-4 weeks, and old media is replaced with fresh hygromycin-B selective media every 4 to 5 days. Once hygromycin-B-resistant colonies are established, resistant clones are replated to new 35 mm culture plates containing fresh Schneider's Drosophila media supplemented with approximately 500 µg/mL of hygromycin-B until these cells reach a density of about 6 to 20x10⁶ cells/mL. To test for expression of BoNT/A-V5-His from S2 cell lines that have stably-integrated a pMTBiP-BoNT/A-V5-His, approximately 3x10⁶ S2 cells from each cell line are plated in a 35 mm tissue culture dish containing 3 mL of Schneider's Drosophila media and are grown in a 28 °C incubator until cells reach a density of about 9x10⁶ cells/ml (6-16 hours). Transfection media is replaced with 3 mL of fresh Schneider's Drosophila media containing 500 µM copper sulfate to induce expression. Cells are incubated in a 28 °C incubator for an additional 48 hours. Media is harvested by transferring the media to 15 mL snap-cap tubes and centrifuging tubes at 500x *g* for 5 minutes to remove debris. Samples are added to 2x LDS Sample Buffer (Invitrogen, Inc, Carlsbad, CA) and expression is measured by Western blot analysis (as described in Examples 6 and 14) using either anti-BoNT/A, anti-V5 or anti-His antibodies in order to identify S2 cell lines expressing increased amounts of BoNT/A-V5-His produced from SEQ ID NO: 60 relative to cell lines expressing BoNT/A-V5-His from the SEQ ID NO: 2 control. The established S2 cell line showing the highest expression level of BoNT/A-V5-His relative to the SEQ ID NO: 2 control is selected for large-scale expression using 3 L spinner flasks. Procedures for large-scale expression are as outlined above except the culture volume is approximately 800-1000 mL of Schneider's Drosophila media and concentrations of all reagents are proportionally increased for this volume. For greater details on all procedures described in this example, see Drosophila Expression System, version H, For the Stable Expression and Purification of Heterologous Proteins in Schneider 2 Cells, 25-0191 (Invitrogen, Inc, Carlsbad, CA).

### Example 23

### Construction and expression of pQBI25BoNT/A-GFP

Restriction endonuclease sites suitable for cloning an operably linked nucleic acid molecule into a pQBI25 vector (Qbiogene, Inc., Carlsbad, CA) are incorporated into the 5'- and 3' ends of modified open reading frame SEQ ID NO: 99. This nucleic acid molecule is synthesized and a pUCBHB1/BoNT/A construct is obtained as described in Example 2. This construct is digested with restriction enzymes that 1) excise the insert containing the open reading frame of SEQ ID NO: 99 encoding an active BoNT/A; and 2) enable this insert to be operably-linked to a pQBI25 vector. This insert is subcloned using a T4 DNA ligase procedure into a pOBI25 vector that is digested with appropriate restriction endonucleases to yield pOBI25BoNT/A-GFP (FIG. 14). The ligation mixture is transformed into chemically competent *E*. *coli* DH5α cells (Invitrogen, Inc, Carlsbad, CA) using a heat shock method, plated on 1.5% Luria-Bertani agar plates (pH 7.0) containing 100 µg/mL of Ampicillin, and placed in a 37°C incubator for overnight growth. Bacteria containing expression constructs are identified as Ampicillin resistant colonies. Candidate constructs are isolated using an alkaline lysis plasmid mini-preparation procedure and analyzed by restriction endonuclease digest mapping to determine the presence and orientation of the insert. This cloning strategy yields a mammalian expression construct encoding an active BoNT/A operably linked to carboxyl-terminal GFP peptide. A similar cloning strategy is used to make a pQBI 25 construct containing the unmodified open reading frame of SEQ ID NO: 2 used as a control for expression levels, as well as, to produce pQBI25 expression constructs in which any one of the modified open reading frames of SEQ ID NO: 76 through SEQ ID NO: 98 is operably linked to a pQBI25 vector.

To transiently express an active BoNT/A-GFP in a cell line, about 1.5x10⁵ SH-SY5Y cells are plated in a 35 mm tissue culture dish containing 3 mL of complete Dulbecco's Modified Eagle Media (DMEM), supplemented with 10% fetal bovine serum (FBS), 1x penicillin/streptomycin solution (Invitrogen, Inc, Carlsbad, CA) and 1x MEM non-essential amino acids solution (MEM) (Invitrogen, Inc, Carlsbad, CA), and are grown in a 37 °C incubator under 5% carbon dioxide until cells reach a density of about 5x10⁵ cells/mL (6-16 hours). A 500 µL transfection solution is prepared by adding 250 µL of OPTI-MEM Reduced Serum Medium containing 15 µL of LipofectAmine 2000 (Invitrogen, Carlsbad, CA) incubated at room temperature for 5 minutes to 250 µL of OPTI-MEM Reduced Serum Medium containing 5 µg of a pOBI25/BoNT/A-GFP. This transfection is incubated at room temperature for approximately 20 minutes. The complete, supplemented DMEM media is replaced with 2 mL of OPTI-MEM Reduced Serum Medium and the 500 µL transfection solution is added to the SH-SY5Y cells and the cells are incubated in a 37°C incubator under 5% carbon dioxide for approximately 6 to 18 hours. Transfection media is replaced with 3 mL of fresh complete, supplemented DMEM and the cells are incubated in a 37 °C incubator under 5% carbon dioxide for 48 hours. Cells are harvest by rinsing cells once with 3.0 mL of 100 mM phosphate-buffered saline, pH 7.4 and lysing cells with a buffer containing 50 mM N-(2-hydroxyethyl) piperazine-N'-(2-ethanesulfonic acid) (HEPES), pH 6.8 150 mM sodium chloride, 1.5 mM magnesium chloride, 10% (v/v) glycerol, 1mM ethylene glycol bis(β-aminoethyl ether) *N*, *N, N; N'-*tetraacetic acid (EGTA), 2% (v/v) Triton-X^{®} 100 (4-octylphenol polyethoxylate) and 1 x Complete protease inhibitor cocktail (Roche Applied Science, Indianapolis, IN). Cell samples are added to 2x LDS Sample Buffer (Invitrogen, Inc, Carlsbad, CA) and expression is measured by Western blot analysis (as described in Examples 6 and 14) using either anti-BoNT/A or anti-GFP antibodies in order to identify pQBI 25 constructs expressing increased amounts of BoNT/A-GFP produced from SEQ ID NO: 99 relative to constructs expressing BoNT/A-GFP from the SEQ ID NO: 2 control.

### Example 24

### Construction and expression of pcDNA™6/BoNT/A-V5-His

Restriction endonuclease sites suitable for cloning an operably linked nucleic acid molecule into a pcDNA™6 vector (Invitrogen, Inc, Carlsbad, CA) are incorporated into the 5'- and 3' ends of modified open reading frame SEQ ID NO: 99. This nucleic acid molecule is synthesized and a pUCBHB1/BoNT/A construct obtained as described in Example 2. This construct is digested with restriction enzymes that 1) excise the insert containing the open reading frame of SEQ ID NO: 99 encoding an active BoNT/A; and 2) enable this insert to be operably-linked to a pcDNA™6 vector. This insert is subcloned using a T4 DNA ligase procedure into a pcDNA™6 vector that is digested with appropriate restriction endonucleases to yield pcDNA™6/BoNT/A-V5-His (FIG. 15). The ligation mixture is transformed into chemically competent *E. coli* DH5α cells (Invitrogen, Inc, Carlsbad, CA) using a heat shock method, plated on 1.5% Luria-Bertani agar plates (pH 7.0) containing 100 µg/mL of Ampicillin, and placed in a 37 °C incubator for overnight growth. Bacteria containing expression constructs are identified as Ampicillin resistant colonies. Candidate constructs are isolated using an alkaline lysis plasmid mini-preparation procedure and analyzed by restriction endonuclease digest mapping to determine the presence and orientation of the insert. This cloning strategy yields a mammalian expression construct encoding an active BoNT/A operably linked to carboxyl-terminal V5 and polyhistidine binding peptides. A similar cloning strategy is used to make a pcDNA™6 construct containing the unmodified open reading frame of SEQ ID NO: 2 used as a control for expression levels, as well as, to produce pcDNA™6 expression constructs in which any one of the modified open reading frames of SEQ ID NO: 76 through SEQ ID NO: 98 is operably linked to a pcDNA™6 vector.

To transiently express BoNT/A-V5-His in a cell line, about 1.5x10⁵ SH-SY5Y cells are plated in a 35 mm tissue culture dish containing 3 mL of complete Dulbecco's Modified Eagle Media (DMEM), supplemented with 10% fetal bovine serum (FBS), 1x penicillin/streptomycin solution (Invitrogen, Inc, Carlsbad, CA) and 1x MEM non-essential amino acids solution (MEM) non-essential amino acids solution (Invitrogen, Inc, Carlsbad, CA), and are grown in a 37 °C incubator under 5% carbon dioxide until cells reach a density of about 5x10⁵ cells/ml (6-16 hours). A 500 µL transfection solution is prepared by adding 250 µL of OPTI-MEM Reduced Serum Medium containing 15 µL of LipofectAmine 2000 (Invitrogen, Carlsbad, CA) incubated at room temperature for 5 minutes to 250 µL of OPTI-MEM Reduced Serum Medium containing 5 µg of a pcDNA™6BoNT/A-V5-His. This transfection is incubated at room temperature for approximately 20 minutes. The complete, supplemented DMEM media is replaced with 2 mL of OPTI-MEM Reduced Serum Medium and the 500 µL transfection solution is added to the SH-SY5Y cells and the cells incubated in a 37 °C incubator under 5% carbon dioxide for approximately 6 to 18 hours. Transfection media is replaced with 3 mL of fresh complete, supplemented DMEM and cells are incubated in a 37 °C incubator under 5% carbon dioxide for 48 hours. Both media and cells are collected for expression analysis of BoNT/A-V5-His. Media is harvested by transferring the media to 15 mL snap-cap tubes and centrifuging tubes at 500x g for 5 minutes to remove debris. Cells are harvested by rinsing cells once with 3.0 mL of 100 mM phosphate-buffered saline, pH 7.4 and the cells are lysed with a buffer containing 62.6 mM 2-amino-2-hydroxymethyl-1,3-propanediol hydrochloric acid (Tris-HCl), pH 6.8 and 2% sodium lauryl sulfate (SDS). Both media and cell samples are added to 2x LDS Sample Buffer (Invitrogen, Inc, Carlsbad, CA) and expression is measured by Western blot analysis (as described in Examples 6 and 14) using either anti-BoNT/A, anti-V5 or anti-His antibodies in order to identify pcDNA™6 constructs expressing increased amounts of BoNT/A-V5-His produced from SEQ ID NO: 99 relative to constructs expressing BoNT/A-V5-His from the SEQ ID NO: 2 control.

To generate a stably-integrated cell line expressing BoNT/A-V5-His, approximately 1.5x10⁵ SH-SY5Y cells are plated in a 35 mm tissue culture dish containing 3 mL of complete DMEM, supplemented with 10% FBS, 1x penicillin/streptomycin solution (Invitrogen, Inc, Carlsbad, CA) and 1x MEM non-essential amino acids solution (Invitrogen, Inc, Carlsbad, CA), and grown in a 37 °C incubator under 5% carbon dioxide until cells reach a density of about 5x10⁵ cells/ml (6-16 hours). A 500 µL transfection solution is prepared by adding 250 µL of OPTI-MEM Reduced Serum Medium containing 15 µL of LipofectAmine 2000 (Invitrogen, Carlsbad, CA) incubated at room temperature for 5 minutes to 250 µL of OPTI-MEM Reduced Serum Medium containing 5 µg of a pcDNA™6BoNT/A-V5-His. This transfection is incubated at room temperature for approximately 20 minutes. The complete, supplemented DMEM media is replaced with 2 mL of OPTI-MEM Reduced Serum Medium and the 500 µL transfection solution is added to the SH-SY5Y cells and the cells are incubated in a 37 °C incubator under 5% carbon dioxide for approximately 6 to 18 hours. Transfection media is replaced with 3 mL of fresh complete, supplemented DMEM and the cells are incubated in a 37 °C incubator under 5% carbon dioxide for approximately 48 hours. Media is replaced with 3 mL of fresh complete DMEM, containing approximately 5 µg/mL of blasticidin, 10% FBS, 1x penicillin/streptomycin solution (Invitrogen, Inc, Carlsbad, CA) and 1x MEM non-essential amino acids solution (Invitrogen, Inc, Carlsbad, CA). Cells are incubated in a 37 °C incubator under 5% carbon dioxide for approximately 3-4 weeks, with old media being replaced with fresh blasticidin selective, complete, supplemented DMEM every 4 to 5 days. Once blasticidin-resistant colonies are established, resistant clones are replated to new 35 mm culture plates containing fresh complete DMEM, supplemented with approximately 5 µg/mL of blasticidin, 10% FBS, 1x penicillin/streptomycin solution (Invitrogen, Inc, Carlsbad, CA) and 1x MEM non-essential amino acids solution (Invitrogen, Inc, Carlsbad, CA), until these cells reach a density of 6 to 20x10⁵ cells/mL. To test for expression of BoNT/A-V5-His from SH-SY5Y cell lines that have stably-integrated a pcDNA™6BoNT/A-V5-His, approximately 1.5x10⁵ SH-SY5Y cells from each cell line are plated in a 35 mm tissue culture dish containing 3 mL of blasticidin selective, complete, supplemented DMEM and grown in a 37 °C incubator under 5% carbon dioxide until cells reach a density of about 5x10⁵ cells/ml (6-16 hours). Media is replaced with 3 mL of fresh blasticidin selective, complete, supplemented DMEM and cells are incubated in a 37 °C incubator under 5% carbon dioxide for 48 hours. Both media and cells are collected for expression analysis of BoNT/A-V5-His. Media is harvested by transferring the media to 15 mL snap-cap tubes and centrifuging tubes at 500x g for 5 minutes to remove debris. Cells are harvest by rinsing cells once with 3.0 mL of 100 mM phosphate-buffered saline, pH 7.4 and lysing cells with a buffer containing 62.6 mM 2-amino-2-hydroxymethyl-1,3-propanediol hydrochloric acid (Tris-HCl), pH 6.8 and 2% sodium lauryl sulfate (SDS). Both media and cell samples are added to 2x LDS Sample Buffer (Invitrogen, Inc, Carlsbad, CA) and expression is measured by Western blot analysis (as described in Examples 6 and 14) using either anti-BoNT/A, anti-V5 or anti-His antibodies in order to identify SH-SY5Y cell lines expressing increased amounts of BoNT/A-V5-His produced from SEQ ID NO: 99 relative to cell lines expressing BoNT/A-V5-His from the SEQ ID NO: 2 control. The established SH-SY5Y cell line showing the highest expression level of BoNT/A-V5-His relative to the SEQ ID NO: 2 control is selected for large-scale expression using 3 L flasks. Procedures for large-scale expression are as outlined above except the starting volume is approximately 800-1000 mL of complete DMEM and concentrations of all reagents are proportionally increased for this volume. For greater details on all procedures described in this example, see pcDNA™6/V5-His A, B, and C, version C, 28-0183 (Invitrogen, Inc, Carlsbad, CA).

### Example 25

### Construction and expression of pSecTag2/BoNT/A-c-myc-His

Restriction endonuclease sites suitable for cloning an operably linked nucleic acid molecule into a pSecTag2 vector (Invitrogen, Inc, Carlsbad, CA) are incorporated into the 5'- and 3' ends of modified open reading frame SEQ ID NO: 99. This nucleic acid molecule is synthesized and a pUCBHB1/BoNT/A construct obtained as described in Example 2. This construct is digested with restriction enzymes that 1) excise the insert containing the open reading frame of SEQ ID NO: 99 encoding an active BoNT/A; and 2) enable this insert to be operably-linked to a pSecTag2 vector. This insert is subcloned using a T4 DNA ligase procedure into a pSecTag2 vector that is digested with appropriate restriction endonucleases to yield pSecTag2/BoNT/A-V5-His (FIG. 16). The ligation mixture is transformed into chemically competent *E. coli* DH5α cells (Invitrogen, Inc, Carlsbad, CA) using a heat shock method, plated on 1.5% Luria-Bertani agar plates (pH 7.0) containing 100 µg/mL of Ampicillin, and placed in a 37 °C incubator for overnight growth. Bacteria containing expression constructs are identified as Ampicillin resistant colonies. Candidate constructs are isolated using an alkaline lysis plasmid mini-preparation procedure and analyzed by restriction endonuclease digest mapping to determine the presence and orientation of the insert. This cloning strategy yields a mammalian expression construct encoding an active BoNT/A operably linked to carboxyl-terminal c-myc and polyhistidine binding peptides. A similar cloning strategy is used to make a pSecTag2 construct containing the unmodified open reading frame of SEQ ID NO: 2 used as a control for expression levels, as well as, to produce pSecTag2 expression constructs in which any one of the modified open reading frames of SEQ ID NO: 76 through SEQ ID NO: 98 is operably linked to a pSecTag2 vector.

To transiently express BoNT/A-c-myc-His in a cell line, about 1.5x10⁵ SH-SY5Y cells are plated in a 35 mm tissue culture dish containing 3 mL of complete Dulbecco's Modified Eagle Media (DMEM), supplemented with 10% fetal bovine serum (FBS), 1x penicillin/streptomycin solution (Invitrogen, Inc, Carlsbad, CA) and 1x MEM non-essential amino acids solution (MEM) non-essential amino acids solution (Invitrogen, Inc, Carlsbad, CA), and grown in a 37 °C incubator under 5% carbon dioxide until cells reach a density of about 5x10⁵ cells/ml (6-16 hours). A 500 µL transfection solution is prepared by adding 250 µL of OPTI-MEM Reduced Serum Medium containing 15 µL of LipofectAmine 2000 (Invitrogen, Carlsbad, CA) incubated at room temperature for 5 minutes to 250 µL of OPTI-MEM Reduced Serum Medium containing 5 µg of a pSecTag2/BoNT/A-c-myc-His. This transfection is incubated at room temperature for approximately 20 minutes. The complete, supplemented DMEM media is replaced with 2 mL of OPTI-MEM Reduced Serum Medium and the 500 µL transfection solution is added to the SH-SY5Y cells and the cells are incubated in a 37 °C incubator under 5% carbon dioxide for approximately 6 to 18 hours. Transfection media is replaced with 3 mL of fresh complete, supplemented DMEM and the cells are incubated in a 37 °C incubator under 5% carbon dioxide for 48 hours. Both media and cells are collected for expression analysis of BoNT/A-c-myc-His. Media is harvested by transferring the media to 15 mL snap-cap tubes and centrifuging tubes at 500x g for 5 minutes to remove debris. Cells are harvested by rinsing cells once with 3.0 mL of 100 mM phosphate-buffered saline, pH 7.4 and lysing cells with a buffer containing 62.6 mM 2-amino-2-hydroxymethyl-1,3-propanediol hydrochloric acid (Tris-HCl), pH 6.8 and 2% sodium lauryl sulfate (SDS). Both media and cell samples are added to 2x LDS Sample Buffer (Invitrogen, Inc, Carlsbad, CA) and expression is measured by Western blot analysis (as described in Examples 6 and 14) using either anti-BoNT/A, anti-c-myc or anti-His antibodies in order to identify pSecTag2 constructs expressing increased amounts of BoNT/A-c-myc-His produced from SEQ ID NO: 99 relative to constructs expressing BoNT/A-V5-His from the SEQ ID NO: 2 control.

To generate a stably-integrated cell line expressing BoNT/A-V5-His, approximately 1.5x10⁵ SH-SY5Y cells are plated in a 35 mm tissue culture dish containing 3 mL of complete DMEM, supplemented with 10% FBS, 1x penicillin/streptomycin solution (Invitrogen, Inc, Carlsbad, CA) and 1x MEM non-essential amino acids solution (Invitrogen, Inc, Carlsbad, CA), and grown in a 37 °C incubator under 5% carbon dioxide until cells reach a density of about 5x10⁵ cells/ml (6-16 hours). A 500 µL transfection solution is prepared by adding 250 µL of OPTI-MEM Reduced Serum Medium containing 15 µL of LipofectAmine 2000 (Invitrogen, Carlsbad, CA) incubated at room temperature for 5 minutes to 250 µL of OPTI-MEM Reduced Serum Medium containing 5 µg of a pSecTag2/BoNT/A-c-myc-His. This transfection is incubated at room temperature for approximately 20 minutes. The complete, supplemented DMEM media is replaced with 2 mL of OPTI-MEM Reduced Serum Medium and the 500 µL transfection solution is added to the SH-SY5Y cells and the cells are incubated in a 37 °C incubator under 5% carbon dioxide for approximately 6 to 18 hours. Transfection media is replaced with 3 mL of fresh complete, supplemented DMEM and cells are incubated in a 37 °C incubator under 5% carbon dioxide for approximately 48 hours. Media is replaced with 3 mL of fresh complete DMEM, containing approximately 5 µg/mL of Zeocin™, 10% FBS, 1x penicillin/streptomycin solution (Invitrogen, Inc, Carlsbad, CA) and 1x MEM non-essential amino acids solution (Invitrogen, Inc, Carlsbad, CA). Cells are incubated in a 37 °C incubator under 5% carbon dioxide for approximately 3-4 weeks, with old media being replaced with fresh Zeocin™-selective, complete, supplemented DMEM every 4 to 5 days. Once Zeocin™-resistant colonies are established, resistant clones are replated to new 35 mm culture plates containing fresh complete DMEM, supplemented with approximately 5 µg/mL of Zeocin™, 10% FBS, 1 x penicillin/streptomycin solution (Invitrogen, Inc, Carlsbad, CA) and 1x MEM non-essential amino acids solution (Invitrogen, Inc, Carlsbad, CA), until these cells reach a density of 6 to 20x10⁵ cells/mL. To test for expression of BoNT/A-c-myc-His from SH-SY5Y cell lines that have stably-integrated a pSecTag2/BoNT/A-c-myc-His, approximately 1.5x10⁵ SH-SY5Y cells from each cell line are plated in a 35 mm tissue culture dish containing 3 mL of Zeocin™-selective, complete, supplemented DMEM and grown in a 37 °C incubator under 5% carbon dioxide until cells reach a density of about 5x10⁵ cells/ml (6-16 hours). Media is replaced with 3 mL of fresh Zeocin™-selective, complete, supplemented DMEM and cells are incubated in a 37 °C incubator under 5% carbon dioxide for 48 hours. Both media and cells are collected for expression analysis of BoNT/A-c-myc-His. Media is harvested by transferring the media to 15 mL snap-cap tubes and centrifuging tubes at 500x g for 5 minutes to remove debris. Cells are harvest by rinsing cells once with 3.0 mL of 100 mM phosphate-buffered saline, pH 7.4 and lysing cells with a buffer containing 62.6 mM 2-amino-2-hydroxymethyl-1,3-propanediol hydrochloric acid (Tris-HCl), pH 6.8 and 2% sodium lauryl sulfate (SDS). Both media and cell samples are added to 2x LDS Sample Buffer (Invitrogen, Inc, Carlsbad, CA) and expression is measured by Western blot analysis (as described in Examples 6 and 14) using either anti-BoNT/A, anti-c-myc or anti-His antibodies in order to identify SH-SY5Y cell lines expressing increased amounts of BoNT/A-c-myc-His produced from SEQ ID NO: 99 relative to cell lines expressing BoNT/A-c-myc-His from the SEQ ID NO: 2 control. The established SH-SY5Y cell line showing the highest expression level of BoNT/A-c-myc-His relative to the SEQ ID NO: 2 control is selected for large-scale expression using 3 L flasks. Procedures for large-scale expression are as outlined above except the starting volume is approximately 800-1000 mL of complete DMEM and concentrations of all reagents are proportionally increased for this volume. For greater details on all procedures described in this example, see pSecTag2 A, B, and C, version E, 28-0159 (Invitrogen, Inc, Carlsbad, CA).

### Example 26

### Construction and expression of pIVEX2.3d/BoNT/A-His

Restriction endonuclease sites suitable for cloning an operably linked nucleic acid molecule into a pIVEX2.3d vector (Roche Applied Science, Indianapolis, IN) are incorporated into the 5'- and 3' ends of modified open reading frame SEQ ID NO: 3. This nucleic acid molecule is synthesized and a pUCBHB1/BoNT/A construct obtained as described in Example 2. This construct is digested with restriction enzymes that 1) excise the insert containing the open reading frame of SEQ ID NO: 3 encoding an active BoNT/A; and 2) enable this insert to be operably-linked to a pIVEX2.3d vector. This insert is subcloned using a T4 DNA ligase procedure into a pIVEX2.3d vector that is digested with appropriate restriction endonucleases to yield pIVEX2.3d/BoNT/A-His (FIG. 17). The ligation mixture is transformed into chemically competent E. *coli* DH5α cells (Invitrogen, Inc, Carlsbad, CA) using a heat shock method, plated on 1.5% Luria-Bertani agar plates (pH 7.0) containing 100 µg/mL of Ampicillin, and placed in a 37 °C incubator for overnight growth. Bacteria containing expression constructs are identified as Ampicillin resistant colonies. Candidate constructs are isolated using an alkaline lysis plasmid mini-preparation procedure and analyzed by restriction endonuclease digest mapping to determine the presence and orientation of the insert. This cloning strategy yields a prokaryotic expression construct encoding an active BoNT/A operably linked to a carboxyl-terminal polyhistidine binding peptide. A similar cloning strategy is used to make a pIVEX2.3d construct containing the unmodified open reading frame of SEQ ID NO: 2 used as a control for expression levels, as well as, to produce pIVEX2.3d expression constructs in which any one of the modified open reading frames of SEQ ID NO: 4 through SEQ ID NO: 33 is operably linked to a pIVEX2.3d vector.

The RTS 100 *E*. *coli* HY Kit (Roche Applied Science, Indianapolis, IN) is used to express an active BoNT/A using a cell-free expression system. A 50 µl reaction mixture consisting of 12 µl *E. coli* lysate, 10 µl reaction mix, 12 µl amino acids, 1 µl methionine, 5 µl reconstitution buffer and 0.5 µg of pIVEX2.3d/BoNT/A-His is incubated in a 30 °C thermomixer for 4-6 hours. A 5 µl sample from this reaction mixture is added to 2x LDS Sample Buffer (Invitrogen, Inc, Carlsbad, CA) and expression is measured by Western blot analysis (as described in Examples 6 and 14) using either anti-BoNT/A or anti-His antibodies in order to identify pIVEX2.3d constructs expressing increased amounts of BoNT/A-His produced from SEQ ID NO: 3 relative to constructs expressing BoNT/A-His from SEQ ID NO: 2. Procedures for large-scale expression are as outlined above except the RTS 9000 *E*. *coli* HY Kit (Roche Applied Science, Indianapolis, IN) is used. For greater details on all procedures described in this example, see RTS 100 E. coli HY Kit, In vitro protein synthesis system based on E. coli lysate, Instruction Manual, version 3, Oct. 2003 (Roche Applied Science, Indianapolis, IN) and Rapid Translation System RTS 9000 E. coli HY Kit, In vitro protein synthesis system based on an enhanced E. coli lysate, Instruction Manual, version 3, Nov. 2001 (Roche Applied Science, Indianapolis, IN).

Although aspects of the present invention have been described with reference to the disclosed embodiments, one skilled in the art will readily appreciate that the specific experiments disclosed are only illustrative of these aspects and in no way limit the present invention. Various modifications can be made without departing from the scope of the present invention.

## Claims

1. A nucleic acid molecule comprising a modified open reading frame encoding an active BoNT/A
wherein the modified open reading frame comprises SEQ ID NO: 3, SEQ ID NO: 110 or SEQ ID NO: 112
wherein the active BoNT/A is a BoNT/A that executes the overall cellular mechanism whereby the active BoNT/A enters a neuron and inhibit neurotransmitter release and encompasses the binding of the active BoNT/A to a low or high affinity receptor complex, the internalization of the active BoNT/A/receptor complex, the translocation of the active BoNT/A light chain into the cytoplasm and the enzymatic modification of the active BoNT/A substrate; and
wherein the modified open reading frame provides increased expression of the encoded active BoNT/A in an *Escherichia coli* cell as compared to an expression level of the same active BoNT/A in the *Escherichia coli* cell from an unmodified open reading frame in an otherwise identical nucleic acid molecule.

2. The molecule according to Claim 1, wherein the increased expression of the active BoNT/A from the modified open reading frame is at least two-fold higher as compared to the expression level of the same active BoNT/A from the unmodified open reading frame.

3. The molecule according to Claim 1, wherein the increased expression of the active BoNT/A from the modified open reading frame is at least five-fold higher as compared to the expression level of the same active BoNT/A from the unmodified open reading frame.

4. The molecule according to Claim 1, wherein the increased expression of the active BoNT/A from the modified open reading frame is at least ten-fold higher as compared to the expression level of the same active BoNT/A from the unmodified open reading frame.

5. The molecule according to any one of Claims 1-4, wherein the molecule is an expression construct.

6. A heterologous cell comprising an expression construct, wherein the expression construct is from Claim 5.

7. The cell according to Claim 6, wherein the expression construct is stably contained in the heterologous cell.

## Patentansprüche

1. Nukleinsäuremolekül, umfassend einen modifizierten, offenen Leserahmen, welcher ein aktives BoNT/A kodiert,
wobei der modifizierte, offene Leserahmen SEQ ID NO:3, SEQ ID NO:110 oder SEQ ID NO:112 umfasst,
wobei das aktive BoNT/A ein BoNT/A ist, welches den gesamten zellulären Mechanismus ausführt, mit dem das aktive BoNT/A in ein Neuron gelangt und die Neurotransmitterausschüttung inhibiert und der das Binden des aktiven BoNT/A an einen Rezeptorkomplex mit geringer oder hoher Affinität, die Internalisierung des aktiven BoNT/A-Rezeptorkomplexes, die Translokation der aktiven BoNT/A leichten Kette in das Cytoplasma und die enzymatische Modifizierung des aktiven BoNT/A-Substrats umfasst, und
wobei der modifizierte, offene Leserahmen eine erhöhte Expression von dem kodierten, aktiven BoNT/A in einer Escherichia coli-Zelle im Vergleich zu einem Expressionsniveau desselben aktiven BoNT/A in der Escherichia coli-Zelle von einem unmodifizierten, offenen Leserahmen in einem ansonsten identischen Nukleinsäuremolekül bereitstellt.

2. Molekül gemäß Anspruch 1, wobei die erhöhte Expression des aktiven BoNT/A von dem modifizierten, offenen Leserahmen mindestens zweimal höher ist als das Expressionsniveau desselben aktiven BoNT/A von dem unmodifizierten, offenen Leserahmen.

3. Molekül gemäß Anspruch 1, wobei die erhöhte Expression des aktiven BoNT/A von dem modifizierten, offenen Leserahmen mindestens fünfmal höher ist als das Expressionsniveau desselben aktiven BoNT/A von dem unmodifizierten, offenen Leserahmen.

4. Molekül gemäß Anspruch 1, wobei die erhöhte Expression des aktiven BoNT/A von dem modifizierten, offenen Leserahmen mindestens zehnmal höher ist als das Expressionsniveau desselben aktiven BoNT/A von dem unmodifizierten, offenen Leserahmen.

5. Molekül gemäß einem der Ansprüche 1 bis 4, wobei das Molekül ein Expressionskonstrukt ist.

6. Heterologe Zelle, umfassend ein Expressionskonstrukt, wobei das Expressionskonstrukt das Expressionskonstrukt von Anspruch 5 ist.

7. Zelle gemäß Anspruch 6, wobei das Expressionskonstrukt stabil in der heterologen Zelle enthalten ist.

## Revendications

1. Molécule d'acide nucléique comprenant un cadre ouvert de lecture modifié codant un BoNT/A actif,
dans laquelle le cadre ouvert de lecture modifié comprend la SEQ ID NO : 3, la SEQ ID NO : 110 ou la SEQ ID NO : 112,
dans laquelle le BoNT/A actif est un BoNT/A qui exécute le mécanisme cellulaire global de façon que le BoNT/A actif entre dans un neurone et inhibe la libération de neurotransmetteur, et englobe la liaison du BoNT/A actif à un complexe de récepteur de faible ou forte affinité, l'intemalisation du complexe de BoNT/A actif / récepteur, la translocation de la chaîne légère du BoNT/A actif dans le cytoplasme et la modification enzymatique du substrat du BoNT/A actif; et
dans laquelle le cadre ouvert de lecture modifié permet une expression accrue du BoNT/A actif codé dans une cellule d'*Escherichia coli* en comparaison avec le niveau d'expression du même BoNT/A actif dans la cellule d'*Escherichia coli* provenant d'un cadre ouvert de lecture non modifié dans une molécule d'acide nucléique par ailleurs identique.

2. Molécule selon la revendication 1, dans laquelle l'expression accrue du BoNT/A actif provenant du cadre ouvert de lecture modifié est au moins deux fois plus élevée que le niveau d'expression du même BoNT/A actif provenant du cadre ouvert de lecture non modifié.

3. Molécule selon la revendication 1, dans laquelle l'expression accrue du BoNT/A actif provenant du cadre ouvert de lecture modifié est au moins cinq fois plus élevée que le niveau d'expression du même BoNT/A actif provenant du cadre ouvert de lecture non modifié.

4. Molécule selon la revendication 1, dans laquelle l'expression accrue du BoNT/A actif provenant du cadre ouvert de lecture modifié est au moins dix fois plus élevée que le niveau d'expression du même BoNT/A actif provenant du cadre ouvert de lecture non modifié.

5. Molécule selon l'une quelconque des revendications 1 à 4, laquelle molécule est une construction d'expression.

6. Cellule hétérologue comprenant une construction d'expression, dans laquelle la construction d'expression est celle de la revendication 5.

7. Cellule selon la revendication 6, dans laquelle la construction d'expression est contenue de façon stable dans la cellule hétérologue.


### SEQUENCE LISTING

<110> Steward, Lance E.
   Li, Shengwin
   Gilmore, Marcella
   Fernandez Salas, Ester
   Miller, Ronald G.
   Aoki, Kei Roger
<120> Optimizing Expression of Active Botulinum Toxin Type A
<130> 17741 (BOT)
<160> 125
<170> FastSEQ for Windows Version 4.0
<210> 1
   <211> 1296
   <212> PRT
   <213> Clostridium botulinum
<220>
   <221> DOMAIN
   <222> (1)...(448)
   <223> Light chain, therapeutic domain, catalytic domain, enzymatic domain.
<221> DOMAIN
   <222> (449)...(1296)
   <223> Heavy chain
<221> DOMAIN
   <222> (449)...(860)
   <223> Amino-terminal half of the heavy chain, translocation domain
<221> DOMAIN
   <222> (861)...(1296)
   <223> Carboxy-terminal half of the heavy chain, targeting domain, binding domain, cell binding domain
<221> PEPTIDE
   <222> (1)...(1296)
   <223> BoNT/A, active
<400> 1
   Met Pro Phe Val Asn Lys Gln Phe Asn Tyr Lys Asp Pro Val Asn Gly
    1              5                   10                  15
   Val Asp Ile Ala Tyr Ile Lys Ile Pro Asn Ala Gly Gln Met Gln Pro
               20                  25                  30
   Val Lys Ala Phe Lys Ile His Asn Lys Ile Trp Val Ile Pro Glu Arg
           35                  40                  45
   Asp Thr Phe Thr Asn Pro Glu Glu Gly Asp Leu Asn Pro Pro Pro Glu
       50                  55                  60
   Ala Lys Gln Val Pro Val Ser Tyr Tyr Asp Ser Thr Tyr Leu Ser Thr
   65                  70                  75                  80
   Asp Asn Glu Lys Asp Asn Tyr Leu Lys Gly Val Thr Lys Leu Phe Glu
                   85                  90                  95
   Arg Ile Tyr Ser Thr Asp Leu Gly Arg Met Leu Leu Thr Ser Ile Val
                       100                           105                           110
   Arg Gly Ile Pro Phe Trp Gly Gly Ser Thr Ile Asp Thr Glu Leu Lys
                   115                           120                           125
   Val Ile Asp Thr Asn Cys Ile Asn Val Ile Gln Pro Asp Gly Ser Tyr
           130                           135                           140
   Arg Ser Glu Glu Leu Asn Leu Val Ile Ile Gly Pro Ser Ala Asp Ile
   145                           150                           155                           160
   Ile Gln Phe Glu Cys Lys Ser Phe Gly His Glu Val Leu Asn Leu Thr
                           165                           170                           175
   Arg Asn Gly Tyr Gly Ser Thr Gln Tyr Ile Arg Phe Ser Pro Asp Phe
                       180                           185                           190
   Thr Phe Gly Phe Glu Glu Ser Leu Glu Val Asp Thr Asn Pro Leu Leu
                   195                           200                           205
   Gly Ala Gly Lys Phe Ala Thr Asp Pro Ala Val Thr Leu Ala His Glu
           210                           215                           220
   Leu Ile His Ala Gly His Arg Leu Tyr Gly Ile Ala Ile Asn Pro Asn
   225                           230                           235                           240
   Arg Val Phe Lys Val Asn Thr Asn Ala Tyr Tyr Glu Met Ser Gly Leu
                           245                           250                           255
   Glu Val Ser Phe Glu Glu Leu Arg Thr Phe Gly Gly His Asp Ala Lys
                       260                           265                           270
   Phe Ile Asp Ser Leu Gln Glu Asn Glu Phe Arg Leu Tyr Tyr Tyr Asn
                   275                           280                           285
   Lys Phe Lys Asp Ile Ala Ser Thr Leu Asn Lys Ala Lys Ser Ile Val
           290                           295                           300
   Gly Thr Thr Ala Ser Leu Gln Tyr Met Lys Asn Val Phe Lys Glu Lys
   305                           310                           315                           320
   Tyr Leu Leu Ser Glu Asp Thr Ser Gly Lys Phe Ser Val Asp Lys Leu
                           325                           330                           335
   Lys Phe Asp Lys Leu Tyr Lys Met Leu Thr Glu Ile Tyr Thr Glu Asp
                       340                           345                           350
   Asn Phe Val Lys Phe Phe Lys Val Leu Asn Arg Lys Thr Tyr Leu Asn
                   355                           360                           365
   Phe Asp Lys Ala Val Phe Lys Ile Asn Ile Val Pro Lys Val Asn Tyr
           370                           375                           380
   Thr Ile Tyr Asp Gly Phe Asn Leu Arg Asn Thr Asn Leu Ala Ala Asn
   385                           390                           395                           400
   Phe Asn Gly Gln Asn Thr Glu Ile Asn Asn Met Asn Phe Thr Lys Leu
                           405                           410                           415
   Lys Asn Phe Thr Gly Leu Phe Glu Phe Tyr Lys Leu Leu Cys Val Arg
                       420                           425                           430
   Gly Ile Ile Thr Ser Lys Thr Lys Ser Leu Asp Lys Gly Tyr Asn Lys
                   435                           440                           445
   Ala Leu Asn Asp Leu Cys Ile Lys Val Asn Asn Trp Asp Leu Phe Phe
           450                           455                           460
   Ser Pro Ser Glu Asp Asn Phe Thr Asn Asp Leu Asn Lys Gly Glu Glu
   465                           470                           475                           480
   Ile Thr Ser Asp Thr Asn Ile Glu Ala Ala Glu Glu Asn Ile Ser Leu
                           485                           490                           495
   Asp Leu Ile Gln Gln Tyr Tyr Leu Thr Phe Asn Phe Asp Asn Glu Pro
                       500                           505                           510
   Glu Asn Ile Ser Ile Glu Asn Leu Ser Ser Asp Ile Ile Gly Gln Leu
                   515                           520                           525
   Glu Leu Met Pro Asn Ile Glu Arg Phe Pro Asn Gly Lys Lys Tyr Glu
           530                           535                           540
   Leu Asp Lys Tyr Thr Met Phe His Tyr Leu Arg Ala Gln Glu Phe Glu
   545                           550                           555                           560
   His Gly Lys Ser Arg Ile Ala Leu Thr Asn Ser Val Asn Glu Ala Leu
                           565                           570                           575
   Leu Asn Pro Ser Arg Val Tyr Thr Phe Phe Ser Ser Asp Tyr Val Lys
   580 585 590
   Lys Val Asn Lys Ala Thr Glu Ala Ala Met Phe Leu Gly Trp Val Glu
   595 600 605
   Gln Leu Val Tyr Asp Phe Thr Asp Glu Thr Ser Glu Val Ser Thr Thr
   610 615 620
   Asp Lys Ile Ala Asp Ile Thr Ile Ile Ile Pro Tyr Ile Gly Pro Ala
   625 630 635 640
   Leu Asn Ile Gly Asn Met Leu Tyr Lys Asp Asp Phe Val Gly Ala Leu
   645 650 655
   Ile Phe Ser Gly Ala Val Ile Leu Leu Glu Phe Ile Pro Glu Ile Ala
   660 665 670
   Ile Pro Val Leu Gly Thr Phe Ala Leu Val Ser Tyr Ile Ala Asn Lys
   675 680 685
   Val Leu Thr Val Gln Thr Ile Asp Asn Ala Leu Ser Lys Arg Asn Glu
   690 695 700
   Lys Trp Asp Glu Val Tyr Lys Tyr Ile Val Thr Asn Trp Leu Ala Lys
   705 710 715 720
   Val Asn Thr Gln Ile Asp Leu Ile Arg Lys Lys Met Lys Glu Ala Leu
   725 730 735
   Glu Asn Gln Ala Glu Ala Thr Lys Ala Ile Ile Asn Tyr Gln Tyr Asn
   740 745 750
   Gln Tyr Thr Glu Glu Glu Lys Asn Asn Ile Asn Phe Asn Ile Asp Asp
   755 760 765
   Leu Ser Ser Lys Leu Asn Glu Ser Ile Asn Lys Ala Met Ile Asn Ile
   770 775 780
   Asn Lys Phe Leu Asn Gln Cys Ser Val Ser Tyr Leu Met Asn Ser Met
   785 790 795 800
   Ile Pro Tyr Gly Val Lys Arg Leu Glu Asp Phe Asp Ala Ser Leu Lys
   805 810 815
   Asp Ala Leu Leu Lys Tyr Ile Tyr Asp Asn Arg Gly Thr Leu Ile Gly
   820 825 830
   Gln Val Asp Arg Leu Lys Asp Lys Val Asn Asn Thr Leu Ser Thr Asp
   835 840 845
   Ile Pro Phe Gln Leu Ser Lys Tyr Val Asp Asn Gln Arg Leu Leu Ser
   850 855 860
   Thr Phe Thr Glu Tyr Ile Lys Asn Ile Ile Asn Thr Ser Ile Leu Asn
   865 870 875 880
   Leu Arg Tyr Glu Ser Asn His Leu Ile Asp Leu Ser Arg Tyr Ala Ser
   885 890 895
   Lys Ile Asn Ile Gly Ser Lys Val Asn Phe Asp Pro Ile Asp Lys Asn
   900 905 910
   Gln Ile Gln Leu Phe Asn Leu Glu Ser Ser Lys Ile Glu Val Ile Leu
   915 920 925
   Lys Asn Ala Ile Val Tyr Asn Ser Met Tyr Glu Asn Phe Ser Thr Ser
   930 935 940
   Phe Trp Ile Arg Ile Pro Lys Tyr Phe Asn Ser Ile Ser Leu Asn Asn
   945 950 955 960
   Glu Tyr Thr Ile Ile Asn Cys Met Glu Asn Asn Ser Gly Trp Lys Val
   965 970 975
   Ser Leu Asn Tyr Gly Glu Ile Ile Trp Thr Leu Gln Asp Thr Gln Glu
   980 985 990
   Ile Lys Gln Arg Val Val Phe Lys Tyr Ser Gln Met Ile Asn Ile Ser
   995 1000 1005
   Asp Tyr Ile Asn Arg Trp Ile Phe Val Thr Ile Thr Asn Asn Arg Leu
   1010 1015 1020
   Asn Asn Ser Lys Ile Tyr Ile Asn Gly Arg Leu Ile Asp Gln Lys Pro
   1025 1030 1035 1040
   Ile Ser Asn Leu Gly Asn Ile His Ala Ser Asn Asn Ile Met Phe Lys
                           1045                          1050                          1055
   Leu Asp Gly Cys Arg Asp Thr His Arg Tyr Ile Trp Ile Lys Tyr Phe
                   1060                          1065                          1070
   Asn Leu Phe Asp Lys Glu Leu Asn Glu Lys Glu Ile Lys Asp Leu Tyr
               1075                          1080                          1085
   Asp Asn Gln Ser Asn Ser Gly Ile Leu Lys Asp Phe Trp Gly Asp Tyr
           1090                          1095                          1100
   Leu Gln Tyr Asp Lys Pro Tyr Tyr Met Leu Asn Leu Tyr Asp Pro Asn
   1105                          1110                          1115                          1120
   Lys Tyr Val Asp Val Asn Asn Val Gly Ile Arg Gly Tyr Met Tyr Leu
                           1125                          1130                          1135
   Lys Gly Pro Arg Gly Ser Val Met Thr Thr Asn Ile Tyr Leu Asn Ser
                   1140                          1145                          1150
   Ser Leu Tyr Arg Gly Thr Lys Phe Ile Ile Lys Lys Tyr Ala Ser Gly
               1155                          1160                          1165
   Asn Lys Asp Asn Ile Val Arg Asn Asn Asp Arg Val Tyr Ile Asn Val
           1170                          1175                          1180
   Val Val Lys Asn Lys Glu Tyr Arg Leu Ala Thr Asn Ala Ser Gln Ala
   1185                          1190                          1195                          1200
   Gly Val Glu Lys Ile Leu Ser Ala Leu Glu Ile Pro Asp Val Gly Asn
                           1205                          1210                          1215
   Leu Ser Gln Val Val Val Met Lys Ser Lys Asn Asp Gln Gly Ile Thr
                   1220                          1225                          1230
   Asn Lys Cys Lys Met Asn Leu Gln Asp Asn Asn Gly Asn Asp Ile Gly
               1235                          1240                          1245
   Phe Ile Gly Phe His Gln Phe Asn Asn Ile Ala Lys Leu Val Ala Ser
           1250                          1255                          1260
   Asn Trp Tyr Asn Arg Gln Ile Glu Arg Ser Ser Arg Thr Leu Gly Cys
   1265                          1270                          1275                          1280
   Ser Trp Glu Phe Ile Pro Val Asp Asp Gly Trp Gly Glu Arg Pro Leu
                           1285                          1290                          1295
<210> 2
   <211> 3891
   <212> DNA
   <213> Clostridium botulinum
<220>
   <221> CDS
   <222> (1)...(3891)
   <223> BoNT/A, unmodified
<400> 2
   atg cca ttt gtt aat aaa caa ttt aat tat aaa gat cct gta aat ggt      48
   Met Pro Phe Val Asn Lys Gln Phe Asn Tyr Lys Asp Pro Val Asn Gly
    1               5                  10                  15
   gtt gat att gct tat ata aaa att cca aat gca gga caa atg caa cca      96
   Val Asp Ile Ala Tyr Ile Lys Ile Pro Asn Ala Gly Gln Met Gln Pro
               20                  25                  30
   gta aaa gct ttt aaa att cat aat aaa ata tgg gtt att cca gaa aga     144
   Val Lys Ala Phe Lys Ile His Asn Lys Ile Trp Val Ile Pro Glu Arg
           35                  40                  45
   gat aca ttt aca aat cct gaa gaa gga gat tta aat cca cca cca gaa     192
   Asp Thr Phe Thr Asn Pro Glu Glu Gly Asp Leu Asn Pro Pro Pro Glu
       50                  55                  60
   gca aaa caa gtt cca gtt tca tat tat gat tca aca tat tta agt aca    240
   Ala Lys Gln Val Pro Val Ser Tyr Tyr Asp Ser Thr Tyr Leu Ser Thr
    65                 70                  75                  80
   gat aat gaa aaa gat aat tat tta aag gga gtt aca aaa tta ttt gag    288
   Asp Asn Glu Lys Asp Asn Tyr Leu Lys Gly Val Thr Lys Leu Phe Glu
                   85                  90                  95
   aga att tat tca act gat ctt gga aga atg ttg tta aca tca ata gta    336
   Arg Ile Tyr Ser Thr Asp Leu Gly Arg Met Leu Leu Thr Ser Ile Val
               100                 105                 110
   agg gga ata cca ttt tgg ggt gga agt aca ata gat aca gaa tta aaa    384
   Arg Gly Ile Pro Phe Trp Gly Gly Ser Thr Ile Asp Thr Glu Leu Lys
           115                 120                 125
   gtt att gat act aat tgt att aat gtg ata caa cca gat ggt agt tat    432
   Val Ile Asp Thr Asn Cys Ile Asn Val Ile Gln Pro Asp Gly Ser Tyr
       130                 135                 140
   aga tca gaa gaa ctt aat cta gta ata ata gga ccc tca gct gat att    480
   Arg Ser Glu Glu Leu Asn Leu Val Ile Ile Gly Pro Ser Ala Asp Ile
   145                 150                 155                 160
   ata cag ttt gaa tgt aaa agc ttt gga cat gaa gtt ttg aat ctt acg    528
   Ile Gln Phe Glu Cys Lys Ser Phe Gly His Glu Val Leu Asn Leu Thr
                   165                 170                 175
   cga aat ggt tat ggc tct act caa tac att aga ttt agc cca gat ttt    576
   Arg Asn Gly Tyr Gly Ser Thr Gln Tyr Ile Arg Phe Ser Pro Asp Phe
               180                 185                 190
   aca ttt ggt ttt gag gag tca ctt gaa gtt gat aca aat cct ctt tta    624
   Thr Phe Gly Phe Glu Glu Ser Leu Glu Val Asp Thr Asn Pro Leu Leu
           195                 200                 205
   ggt gca ggc aaa ttt gct aca gat cca gca gta aca tta gca cat gaa    672
   Gly Ala Gly Lys Phe Ala Thr Asp Pro Ala Val Thr Leu Ala His Glu
       210                 215                 220
   ctt ata cat gct gga cat aga tta tat gga ata gca att aat cca aat    720
   Leu Ile His Ala Gly His Arg Leu Tyr Gly Ile Ala Ile Asn Pro Asn
   225                 230                 235                 240
   agg gtt ttt aaa gta aat act aat gcc tat tat gaa atg agt ggg tta    768
   Arg Val Phe Lys Val Asn Thr Asn Ala Tyr Tyr Glu Met Ser Gly Leu
                   245                 250                 255
   gaa gta agc ttt gag gaa ctt aga aca ttt ggg gga cat gat gca aag    816
   Glu Val Ser Phe Glu Glu Leu Arg Thr Phe Gly Gly His Asp Ala Lys
               260                 265                 270
   ttt ata gat agt tta cag gaa aac gaa ttt cgt cta tat tat tat aat    864
   Phe Ile Asp Ser Leu Gln Glu Asn Glu Phe Arg Leu Tyr Tyr Tyr Asn
           275                 280                 285
   aag ttt aaa gat ata gca agt aca ctt aat aaa gct aaa tca ata gta    912
   Lys Phe Lys Asp Ile Ala Ser Thr Leu Asn Lys Ala Lys Ser Ile Val
       290                 295                 300
   ggt act act gct tca tta cag tat atg aaa aat gtt ttt aaa gag aaa    960
   Gly Thr Thr Ala Ser Leu Gln Tyr Met Lys Asn Val Phe Lys Glu Lys
   305                 310                 315                 320
   tat ctc cta tct gaa gat aca tct gga aaa ttt tcg gta gat aaa tta   1008
   Tyr Leu Leu Ser Glu Asp Thr Ser Gly Lys Phe Ser Val Asp Lys Leu
                   325                 330                 335
   aaa ttt gat aag tta tac aaa atg tta aca gag att tac aca gag gat   1056
   Lys Phe Asp Lys Leu Tyr Lys Met Leu Thr Glu Ile Tyr Thr Glu Asp
               340                 345                 350
   aat ttt gtt aag ttt ttt aaa gta ctt aac aga aaa aca tat ttg aat   1104
   Asn Phe Val Lys Phe Phe Lys Val Leu Asn Arg Lys Thr Tyr Leu Asn
           355                 360                 365
   ttt gat aaa gcc gta ttt aag ata aat ata gta cct aag gta aat tac   1152
   Phe Asp Lys Ala Val Phe Lys Ile Asn Ile Val Pro Lys Val Asn Tyr
       370                 375                 380
   aca ata tat gat gga ttt aat tta aga aat aca aat tta gca gca aac   1200
   Thr Ile Tyr Asp Gly Phe Asn Leu Arg Asn Thr Asn Leu Ala Ala Asn
   385                 390                 395                 400
   ttt aat ggt caa aat aca gaa att aat aat atg aat ttt act aaa cta   1248
   Phe Asn Gly Gln Asn Thr Glu Ile Asn Asn Met Asn Phe Thr Lys Leu
                   405                 410                 415
   aaa aat ttt act gga ttg ttt gaa ttt tat aag ttg cta tgt gta aga   1296
   Lys Asn Phe Thr Gly Leu Phe Glu Phe Tyr Lys Leu Leu Cys Val Arg
               420                 425                 430
   ggg ata ata act tct aaa act aaa tca tta gat aaa gga tac aat aag   1344
   Gly Ile Ile Thr Ser Lys Thr Lys Ser Leu Asp Lys Gly Tyr Asn Lys
           435                 440                 445
   gca tta aat gat tta tgt atc aaa gtt aat aat tgg gac ttg ttt ttt   1392
   Ala Leu Asn Asp Leu Cys Ile Lys Val Asn Asn Trp Asp Leu Phe Phe
       450                 455                 460
   agt cct tca gaa gat aat ttt act aat gat cta aat aaa gga gaa gaa   1440
   Ser Pro Ser Glu Asp Asn Phe Thr Asn Asp Leu Asn Lys Gly Glu Glu
   465                 470                 475                 480
   att aca tct gat act aat ata gaa gca gca gaa gaa aat att agt tta   1488
   Ile Thr Ser Asp Thr Asn Ile Glu Ala Ala Glu Glu Asn Ile Ser Leu
                   485                 490                 495
   gat tta ata caa caa tat tat tta acc ttt aat ttt gat aat gaa cct   1536
   Asp Leu Ile Gln Gln Tyr Tyr Leu Thr Phe Asn Phe Asp Asn Glu Pro
               500                 505                 510
   gaa aat att tca ata gaa aat ctt tca agt gac att ata ggc caa tta   1584
   Glu Asn Ile Ser Ile Glu Asn Leu Ser Ser Asp Ile Ile Gly Gln Leu
           515                 520                 525
   gaa ctt atg cct aat ata gaa aga ttt cct aat gga aaa aag tat gag   1632
   Glu Leu Met Pro Asn Ile Glu Arg Phe Pro Asn Gly Lys Lys Tyr Glu
   530 535 540
   tta gat aaa tat act atg ttc cat tat ctt cgt gct caa gaa ttt gaa 1680
   Leu Asp Lys Tyr Thr Met Phe His Tyr Leu Arg Ala Gln Glu Phe Glu
   545 550 555 560
   cat ggt aaa tct agg att gct tta aca aat tct gtt aac gaa gca tta 1728
   His Gly Lys Ser Arg Ile Ala Leu Thr Asn Ser Val Asn Glu Ala Leu
   565 570 575
   tta aat cct agt cgt gtt tat aca ttt ttt tct tca gac tat gta aag 1776
   Leu Asn Pro Ser Arg Val Tyr Thr Phe Phe Ser Ser Asp Tyr Val Lys
   580 585 590
   aaa gtt aat aaa gct acg gag gca gct atg ttt tta ggc tgg gta gaa 1824
   Lys Val Asn Lys Ala Thr Glu Ala Ala Met Phe Leu Gly Trp Val Glu
   595 600 605
   caa tta gta tat gat ttt acc gat gaa act agc gaa gta agt act acg 1872
   Gln Leu Val Tyr Asp Phe Thr Asp Glu Thr Ser Glu Val Ser Thr Thr
   610 615 620
   gat aaa att gcg gat ata act ata att att cca tat ata gga cct gct 1920
   Asp Lys Ile Ala Asp Ile Thr Ile Ile Ile Pro Tyr Ile Gly Pro Ala
   625 630 635 640
   tta aat ata ggt aat atg tta tat aaa gat gat ttt gta ggt gct tta 1968
   Leu Asn Ile Gly Asn Met Leu Tyr Lys Asp Asp Phe Val Gly Ala Leu
   645 650 655
   ata ttt tca gga gct gtt att ctg tta gaa ttt ata cca gag att gca 2016
   Ile Phe Ser Gly Ala Val Ile Leu Leu Glu Phe Ile Pro Glu Ile Ala
   660 665 670
   ata cct gta tta ggt act ttt gca ctt gta tca tat att gcg aat aag 2064
   Ile Pro Val Leu Gly Thr Phe Ala Leu Val Ser Tyr Ile Ala Asn Lys
   675 680 685
   gtt cta acc gtt caa aca ata gat aat gct tta agt aaa aga aat gaa 2112
   Val Leu Thr Val Gln Thr Ile Asp Asn Ala Leu Ser Lys Arg Asn Glu
   690 695 700
   aaa tgg gat gag gtc tat aaa tat ata gta aca aat tgg tta gca aag 2160
   Lys Trp Asp Glu Val Tyr Lys Tyr Ile Val Thr Asn Trp Leu Ala Lys
   705 710 715 720
   gtt aat aca cag att gat cta ata aga aaa aaa atg aaa gaa gct tta 2208
   Val Asn Thr Gln Ile Asp Leu Ile Arg Lys Lys Met Lys Glu Ala Leu
   725 730 735
   gaa aat caa gca gaa gca aca aag gct ata ata aac tat cag tat aat 2256
   Glu Asn Gln Ala Glu Ala Thr Lys Ala Ile Ile Asn Tyr Gln Tyr Asn
   740 745 750
   caa tat act gag gaa gag aaa aat aat att aat ttt aat att gat gat 2304
   Gln Tyr Thr Glu Glu Glu Lys Asn Asn Ile Asn Phe Asn Ile Asp Asp
   755 760 765
   tta agt tcg aaa ctt aat gag tct ata aat aaa gct atg att aat ata 2352
   Leu Ser Ser Lys Leu Asn Glu Ser Ile Asn Lys Ala Met Ile Asn Ile
       770                 775                 780
   aat aaa ttt ttg aat caa tgc tct gtt tca tat tta atg aat tct atg      2400
   Asn Lys Phe Leu Asn Gln Cys Ser Val Ser Tyr Leu Met Asn Ser Met
   785                 790                 795                 800
   atc cct tat ggt gtt aaa cgg tta gaa gat ttt gat gct agt ctt aaa      2448
   Ile Pro Tyr Gly Val Lys Arg Leu Glu Asp Phe Asp Ala Ser Leu Lys
                   805                 810                 815
   gat gca tta tta aag tat ata tat gat aat aga gga act tta att ggt      2496
   Asp Ala Leu Leu Lys Tyr Ile Tyr Asp Asn Arg Gly Thr Leu Ile Gly
               820                 825                 830
   caa gta gat aga tta aaa gat aaa gtt aat aat aca ctt agt aca gat      2544
   Gln Val Asp Arg Leu Lys Asp Lys Val Asn Asn Thr Leu Ser Thr Asp
           835                 840                 845
   ata cct ttt cag ctt tcc aaa tac gta gat aat caa aga tta tta tct      2592
   Ile Pro Phe Gln Leu Ser Lys Tyr Val Asp Asn Gln Arg Leu Leu Ser
       850                 855                 860
   aca ttt act gaa tat att aag aat att att aat act tct ata ttg aat      2640
   Thr Phe Thr Glu Tyr Ile Lys Asn Ile Ile Asn Thr Ser Ile Leu Asn
   865                 870                 875                 880
   tta aga tat gaa agt aat cat tta ata gac tta tct agg tat gca tca      2688
   Leu Arg Tyr Glu Ser Asn His Leu Ile Asp Leu Ser Arg Tyr Ala Ser
                   885                 890                 895
   aaa ata aat att ggt agt aaa gta aat ttt gat cca ata gat aaa aat      2736
   Lys Ile Asn Ile Gly Ser Lys Val Asn Phe Asp Pro Ile Asp Lys Asn
               900                 905                 910
   caa att caa tta ttt aat tta gaa agt agt aaa att gag gta att tta      2784
   Gln Ile Gln Leu Phe Asn Leu Glu Ser Ser Lys Ile Glu Val Ile Leu
           915                 920                 925
   aaa aat gct att gta tat aat agt atg tat gaa aat ttt agt act agc      2832
   Lys Asn Ala Ile Val Tyr Asn Ser Met Tyr Glu Asn Phe Ser Thr Ser
       930                 935                 940
   ttt tgg ata aga att cct aag tat ttt aac agt ata agt cta aat aat      2880
   Phe Trp Ile Arg Ile Pro Lys Tyr Phe Asn Ser Ile Ser Leu Asn Asn
   945                 950                 955                 960
   gaa tat aca ata ata aat tgt atg gaa aat aat tca gga tgg aaa gta      2928
   Glu Tyr Thr Ile Ile Asn Cys Met Glu Asn Asn Ser Gly Trp Lys Val
                   965                 970                 975
   tca ctt aat tat ggt gaa ata atc tgg act tta cag gat act cag gaa      2976
   Ser Leu Asn Tyr Gly Glu Ile Ile Trp Thr Leu Gln Asp Thr Gln Glu
               980                 985                 990
   ata aaa caa aga gta gtt ttt aaa tac agt caa atg att aat ata tca      3024
   Ile Lys Gln Arg Val Val Phe Lys Tyr Ser Gln Met Ile Asn Ile Ser
           995                 1000                1005
   gat tat ata aac aga tgg att ttt gta act atc act aat aat aga tta     3072
   Asp Tyr Ile Asn Arg Trp Ile Phe Val Thr Ile Thr Asn Asn Arg Leu
       1010                1015                1020
   aat aac tct aaa att tat ata aat gga aga tta ata gat caa aaa cca     3120
   Asn Asn Ser Lys Ile Tyr Ile Asn Gly Arg Leu Ile Asp Gln Lys Pro
   1025                1030                1035                1040
   att tca aat tta ggt aat att cat gct agt aat aat ata atg ttt aaa     3168
   Ile Ser Asn Leu Gly Asn Ile His Ala Ser Asn Asn Ile Met Phe Lys
                   1045                1050                1055
   tta gat ggt tgt aga gat aca cat aga tat att tgg ata aaa tat ttt     3216
   Leu Asp Gly Cys Arg Asp Thr His Arg Tyr Ile Trp Ile Lys Tyr Phe
               1060                1065                1070
   aat ctt ttt gat aag gaa tta aat gaa aaa gaa atc aaa gat tta tat     3264
   Asn Leu Phe Asp Lys Glu Leu Asn Glu Lys Glu Ile Lys Asp Leu Tyr
           1075                1080                1085
   gat aat caa tca aat tca ggt att tta aaa gac ttt tgg ggt gat tat     3312
   Asp Asn Gln Ser Asn Ser Gly Ile Leu Lys Asp Phe Trp Gly Asp Tyr
       1090                1095                1100
   tta caa tat gat aaa cca tac tat atg tta aat tta tat gat cca aat     3360
   Leu Gln Tyr Asp Lys Pro Tyr Tyr Met Leu Asn Leu Tyr Asp Pro Asn
   1105                1110                1115                1120
   aaa tat gtc gat gta aat aat gta ggt att aga ggt tat atg tat ctt     3408
   Lys Tyr Val Asp Val Asn Asn Val Gly Ile Arg Gly Tyr Met Tyr Leu
                   1125                1130                1135
   aaa ggg cct aga ggt agc gta atg act aca aac att tat tta aat tca     3456
   Lys Gly Pro Arg Gly Ser Val Met Thr Thr Asn Ile Tyr Leu Asn Ser
               1140                1145                1150
   agt ttg tat agg ggg aca aaa ttt att ata aaa aaa tat gct tct gga     3504
   Ser Leu Tyr Arg Gly Thr Lys Phe Ile Ile Lys Lys Tyr Ala Ser Gly
           1155                1160                1165
   aat aaa gat aat att gtt aga aat aat gat cgt gta tat att aat gta     3552
   Asn Lys Asp Asn Ile Val Arg Asn Asn Asp Arg Val Tyr Ile Asn Val
       1170                1175                1180
   gta gtt aaa aat aaa gaa tat agg tta gct act aat gcg tca cag gca     3600
   Val Val Lys Asn Lys Glu Tyr Arg Leu Ala Thr Asn Ala Ser Gln Ala
   1185                1190                1195                1200
   ggc gta gaa aaa ata cta agt gca tta gaa ata cct gat gta gga aat     3648
   Gly Val Glu Lys Ile Leu Ser Ala Leu Glu Ile Pro Asp Val Gly Asn
                   1205                1210                1215
   cta agt caa gta gta gta atg aag tca aaa aat gat caa gga ata aca     3696
   Leu Ser Gln Val Val Val Met Lys Ser Lys Asn Asp Gln Gly Ile Thr
               1220                1225                1230
   aat aaa tgc aaa atg aat tta caa gat aat aat ggg aat gat ata ggc     3744
   Asn Lys Cys Lys Met Asn Leu Gln Asp Asn Asn Gly Asn Asp Ile Gly
           1235                1240                1245
   ttt ata gga ttt cat cag ttt aat aat ata gct aaa cta gta gca agt    3792
   Phe Ile Gly Phe His Gln Phe Asn Asn Ile Ala Lys Leu Val Ala Ser
       1250                1255                1260
   aat tgg tat aat aga caa ata gaa aga tct agt agg act ttg ggt tgc    3840
   Asn Trp Tyr Asn Arg Gln Ile Glu Arg Ser Ser Arg Thr Leu Gly Cys
   1265                1270                1275                1280
   tca tgg gaa ttt att cct gta gat gat gga tgg gga gaa agg cca ctg    3888
   Ser Trp Glu Phe Ile Pro Val Asp Asp Gly Trp Gly Glu Arg Pro Leu
                   1285                1290                1295
   taa                                                                3891
    *
<210> 3
   <211> 3891
   <212> DNA
   <213> Artificial Sequence
<220>
   <223> BoNT/A, E. coli-modified 1
<400> 3
   atgccgttcg taaacaaaca gttcaactat aaagacccag tcaacggcgt ggacattgcc 60
   tatatcaaaa tcccgaatgc gggtcaaatg cagcccgtga aagcatttaa aatccataac 120
   aaaatttggg tgatcccgga gcgcgatacg ttcacgaacc cggaagaagg agatttaaac 180
   ccaccgcctg aggctaaaca ggtcccggtg tcttactatg atagcacata cctgagtacc 240
   gacaatgaaa aggacaacta cctgaaaggt gttaccaaac tgttcgagcg catttattcg 300
   acagatctcg gtcgcatgtt gctgacttct attgtgcgcg gcattccgtt ttggggtggt 360
   agcaccatcg atacagaact caaagtgatt gacaccaact gcatcaatgt gattcagcct 420
   gatgggagct accggtccga agagcttaac ctcgtaatca ttggcccgag cgcggatatt 480
   atccaattcg aatgtaaatc ttttgggcat gaagtcctga atctgacgcg gaatggctat 540
   ggatcgacgc agtatattcg tttttctcca gatttcacat ttggatttga agaaagcctc 600
   gaagttgata cgaaccctct tttaggcgcg ggaaaattcg cgacggaccc agcggtgacc 660
   ttggcacatg aacttattca tgccgggcat cgcttgtatg gaatcgccat taacccgaac 720
   cgtgttttca aggtgaatac gaacgcgtat tacgagatgt cgggcttaga agtgtccttt 780
   gaagaactgc gcacgtttgg cggtcatgat gcaaaattta ttgatagtct gcaagaaaac 840
   gaatttcggc tgtactatta caataaattc aaagacattg catcaacctt aaacaaggcg 900
   aaaagcattg tgggtaccac ggctagctta caatatatga aaaacgtttt caaagaaaaa 960
   tacctcctta gcgaagacac ttccggcaaa ttctctgtcg ataaactgaa atttgataaa 1020
   ctgtataaaa tgctcaccga gatctacaca gaggataact ttgtcaaatt cttcaaggtc 1080
   ttgaatcgga aaacctatct gaacttcgat aaagccgtct ttaagatcaa catcgtaccg 1140
   aaagttaact acaccatcta tgatggcttt aatctgcgca atacgaatct ggcggcgaac 1200
   tttaacggcc agaacaccga aatcaacaac atgaacttta ctaaactgaa aaattttacc 1260
   ggcttgtttg aattttataa gctcctgtgt gtccgcggta ttatcaccag caaaaccaaa 1320
   tccttggata agggctataa caaggcgctc aatgatttat gcatcaaggt gaacaactgg 1380
   gacttgtttt tctctccatc tgaagataat tttactaacg acttgaacaa aggagaggaa 1440
   attacttccg ataccaacat cgaagcagcg gaagagaata ttagcctgga tcttattcaa 1500
   caatattacc tgacctttaa ttttgataac gagcctgaga acatttccat tgagaatctc 1560
   agctctgaca tcatcggcca gctggaactg atgccgaata tcgaacgctt tcctaatgga 1620
   aagaaatatg aattggacaa atacaccatg ttccactatc tccgcgcgca ggagtttgag 1680
   cacggcaagt ctcgtattgc tctgaccaat tcggtaaacg aagccctttt aaatccttcg 1740
   cgtgtgtaca cctttttctc aagcgattat gttaaaaaag tgaacaaggc gaccgaagcg 1800
   gcgatgtttt tgggatgggt ggaacaactg gtatatgact ttacggatga aacttctgaa 1860
   gtctcgacca ccgacaaaat tgccgatatt accattatca ttccctatat tggccctgca 1920
   ctgaacattg gtaacatgct gtataaagat gattttgtgg gcgccctgat cttttcaggc 1980
   gctgttatcc tgctggaatt tatcccggaa atcgccattc cagtactcgg tacctttgcg 2040
   ctggtgtcct atatcgcaaa caaagttttg actgtccaga cgatcgacaa cgcgctcagt 2100
   aaacgtaacg aaaaatggga tgaggtgtat aagtatattg ttaccaactg gctcgctaaa 2160
   gtaaacaccc agattgacct gattcgcaag aagatgaaag aagcgctgga aaaccaagca 2220
   gaagcgacca aagctattat caactatcaa tataaccagt acacagagga agaaaagaat 2280
   aacatcaact tcaacatcga cgacttatct tcaaagctga atgaatctat taacaaagcg 2340
   atgattaata ttaacaagtt cttgaaccaa tgtagtgtca gctatctgat gaactcgatg 2400
   atcccatatg gtgtgaaacg tctggaagac ttcgatgcaa gccttaaaga tgcccttctg 2460
   aagtatattt acgataatcg cggaactctt attggccaag tggatcgctt aaaagataaa 2520
   gtcaacaaca cgctgagtac agacatccct tttcagctgt ctaaatatgt ggacaatcag 2580
   cgcctgctgt ccacgtttac ggaatacatc aaaaacatca tcaacactag tattctgaac 2640
   ttgcgttacg agagtaacca tctgattgat ctgagccgtt acgcatctaa aatcaacatc 2700
   ggctcgaagg tgaacttcga tcctatcgac aaaaaccaga ttcaattgtt caacttagaa 2760
   tcgtcaaaga ttgaagttat cttaaaaaat gcgattgtat ataattcaat gtacgaaaat 2820
   ttctctacga gcttttggat tcgtattccg aaatatttca acagtatctc tttaaacaac 2880
   gagtatacta tcatcaattg tatggagaat aacagcgggt ggaaagtgag ccttaactat 2940
   ggtgaaatca tctggactct gcaggacact caagaaatta aacaacgcgt ggtgtttaaa 3000
   tactcacaga tgattaacat ctcggattat attaatcgct ggatttttgt gacaattact 3060
   aacaaccggc tgaacaacag caaaatttac attaacggtc gcctgatcga tcagaaacca 3120
   atcagtaatc tcggtaacat tcacgcatcg aataatatca tgttcaaact ggatggttgt 3180
   cgcgacacgc accgttacat ttggatcaaa tacttcaatt tattcgacaa agaactcaac 3240
   gaaaaggaga ttaaggatct ttatgacaat cagtctaatt cgggtattct gaaagacttt 3300
   tggggtgatt accttcagta cgataaaccg tattatatgt taaacttata tgatccgaat 3360
   aaatacgttg acgtcaacaa cgttggcatt cgtggctata tgtatctgaa agggccgcgt 3420
   ggcagcgtga tgaccactaa catttactta aactcctccc tctatcgcgg tactaaattt 3480
   attatcaaga aatatgcctc tggcaacaag gacaatatcg tacgcaataa cgatcgcgtc 3540
   tacattaacg tggtggtgaa gaataaagaa tatcgtctgg cgaccaatgc tagtcaggcg 3600
   ggcgtggaga aaattctgtc tgcacttgaa atcccggatg tgggtaattt atcccaggtg 3660
   gttgtgatga aaagtaaaaa tgaccaaggg atcaccaata aatgcaaaat gaatctgcaa 3720
   gataacaacg gcaacgacat tggttttatc ggcttccacc aattcaataa tatcgcgaaa 3780
   ctggtggcct caaattggta caaccgtcag attgagcgca gctcccgcac tttaggctgt 3840
   agctgggagt tcattccggt agatgacggt tggggagaac gcccattgta a          3891
<210> 4
   <211> 3891
   <212> DNA
   <213> Artificial Sequence
<220>
   <223> BoNT/A, E. coli-modified 2
<400> 4
   atgccgttcg tgaacaaaca gtttaactat aaagaccctg tcaatggggt tgatatagcg 60
   tatattaaaa ttccgaatgc gggacagatg cagccagtga aagctttcaa gatacacaat 120
   aaaatttggg tcattccgga acgtgacaca tttaccaacc cagaggaagg ggatttgaac 180
   cccccaccgg aggcgaaaca ggttcctgtt tcgtactatg attcaacata cctgagtacc 240
   gataacgaaa aggacaacta tcttaaaggc gtaacgaagc tttttgaacg catctactcc 300
   acagatctcg gacgtatgtt gctgacctct attgtgcgcg gcattccgtt ttggggcgga 360
   tccaccatag ataccgaatt aaaagttatt gatacaaatt gtatcaatgt catccaacct 420
   gatggcagtt accgcagcga agagctaaat cttgtaatta tcggcccgag tgctgacata 480
   atccagttcg aatgcaagag ctttggtcat gaggtgttga acctgactcg taacgggtat 540
   gggtctactc aatacattcg cttcagcccg gacttcacct ttggttttga ggaatctctc 600
   gaagttgata cgaatccgct gcttggtgct ggaaagtttg ccactgaccc cgccgtgaca 660
   ctcgcgcatg agctgattca cgccggtcat cgtctgtacg gtatcgcaat caaccctaac 720
   cgcgtgttca aagttaacac taatgcgtat tacgaaatgt ccgggctgga agttagtttc 780
   gaagagctgc ggaccttcgg tggccatgat gcgaagttta ttgattcgct gcaagaaaat 840
   gaatttcgct tgtattacta taacaagttt aaagatatcg catcaaccct taacaaagcc 900
   aaaagcattg tcggcacgac cgcatcgcta caatacatga aaaatgtctt caaagaaaaa 960
   tatctgttat ccgaggatac ctccggtaaa ttctcagtgg acaaacttaa attcgataag 1020
   ctgtataaaa tgctaacgga gatctatacg gaagataact ttgtaaagtt tttcaaagtc 1080
   ttaaatcgca aaacgtatct gaattttgac aaagctgtct tcaaaattaa tattgttccc 1140
   aaagttaact atacgattta cgacggattc aacttaagaa acactaacct tgccgcgaat 1200
   ttcaacgggc agaacacgga aattaataac atgaatttta cgaaattaaa gaactttacc 1260
   gggctgtttg agttttacaa attgctctgc gtgcgcggaa tcattaccag caaaacgaaa 1320
   tcactggata aaggttataa taaagcgctt aacgatctgt gcattaaagt taacaattgg 1380
   gatttattct tttcaccatc tgaggataac tttacaaacg atctcaacaa aggcgaggaa 1440
   attacaagtg atactaatat tgaggcagcg gaagagaata tttcactgga tctgatccag 1500
   caatactatt tgacctttaa cttcgataat gaacccgaaa atatcagtat tgaaaatttg 1560
   agctcagaca tcattggaca gctggaactg atgccaaata tcgagcgttt cccaaacggt 1620
   aaaaagtatg aactggacaa atacaccatg tttcactatc tgcgcgctca agagttcgag 1680
   catggcaagt ctaggatcgc gttgactaat tcggtgaatg aggcactgtt aaaccctagt 1740
   cgggtgtata cattcttttc ctctgattat gttaaaaagg tcaacaaagc cacggaagcc 1800
   gcaatgttcc tgggctgggt cgaacagtta gtgtatgatt ttaccgacga aacttccgag 1860
   gtttcaacca cagacaaaat tgcggatatt accattataa tcccgtacat cggcccggct 1920
   ttaaacattg gtaatatgtt gtacaaagat gactttgtgg gtgccctgat attttccggt 1980
   gcagtaattt tgctcgagtt tatcccagaa atagcaatcc ccgttttagg tacgttcgct 2040
   ttagtgagct atattgcaaa caaagttctg actgtacaaa cgattgataa tgccctgtcg 2100
   aaacgtaatg aaaagtggga tgaggtttat aagtatatcg ttaccaactg gctggccaag 2160
   gtaaatacac agatcgatct gattcggaaa aagatgaaag aagcgctgga aaatcaggcg 2220
   gaagctacta aagcaatcat aaactatcaa tataatcagt ataccgaaga ggaaaaaaac 2280
   aatatcaatt ttaatattga cgatctgagc agtaaactga atgaatcgat caataaggct 2340
   atgattaaca taaacaagtt tctgaaccaa tgctcagtct cttatttaat gaactcaatg 2400
   attccgtacg gcgtaaaaag attggaagac tttgatgcca gcctgaaaga cgccttactt 2460
   aaatatatct acgataatcg tggtaccctg attggtcagg tcgaccgact gaaagataag 2520
   gtgaataaca cgctctcaac cgatattccg tttcagttat ccaaatatgt ggataatcag 2580
   cgcttgctga gtacattcac cgagtatatt aagaacatta taaatacctc catcctgaat 2640
   ctgcgttacg aatcgaacca tctaatcgac ctgagccgtt acgcgtctaa aataaatatc 2700
   ggctctaaag ttaatttgga tccaattgat aaaaatcaga tccagttatt caatctggag 2760
   tctagcaaga ttgaagtcat tctgaagaat gcgattgtgt ataactcaat gtacgaaaac 2820
   tttagcacat cgttttggat tcggatcccg aaatatttca atagcatttc gctgaacaat 2880
   gaatatacga taatcaactg tatggaaaat aacagtggct ggaaagtaag tctgaattac 2940
   ggcgaaatta tatggaccct ccaagacacg caggaaatca aacagcgtgt cgtatttaaa 3000
   tattcccaga tgattaatat tagcgattac attaatcgct ggatctttgt taccattact 3060
   aacaatcgcc tcaataactc taaaatttat atcaatggtc gcttgataga tcagaaacct 3120
   atcagcaatc tcggcaatat ccacgcgtct aataacatta tgtttaaact tgatggttgt 3180
   agggataccc acagatacat atggattaaa tactttaacc tgtttgacaa ggagttgaat 3240
   gaaaaggaaa tcaaagacct gtatgataat cagagcaata gcgggattct gaaagacttc 3300
   tggggagatt accttcagta tgataagccg tactatatgc ttaatctcta tgacccgaac 3360
   aaatacgttg atgtaaacaa tgtggggatc cgtggttata tgtacctgaa gggcccgcgt 3420
   ggaagtgtaa tgactacgaa catctacctg aattccagtt tatatcgagg caccaaattc 3480
   atcataaaaa agtatgcgag cggtaacaaa gacaatattg tacgaaataa cgacagagtg 3540
   tatattaacg ttgtggtcaa aaataaagaa tatcgtttag ctacaaatgc cagccaggca 3600
   ggcgtggaaa aaatcctgag tgcacttgaa atcccggatg tgggcaatct gtcgcaggtg 3660
   gttgtgatga aatctaaaaa tgatcaaggg attactaaca aatgtaaaat gaacctgcag 3720
   gataacaatg gcaacgatat cggctttatc ggtttccatc aattcaacaa cattgccaaa 3780
   cttgtcgcat caaactggta taaccgtcaa attgaacggt cttcgcgcac tctaggttgc 3840
   agctgggaat ttatccctgt ggacgacggc tggggtgaac gtccgctgta a 3891
<210> 5
   <211> 3891
   <212> DNA
   <213> Artificial Sequence
<220>
   <223> BoNT/A, E. coli-modified 3
<400> 5
   atgccgtttg tgaataaaca gtttaattat aaagatccgg tgaatggcgt ggatattgcg 60
<210> 6
   <211> 3891
   <212> DNA
   <213> Artificial Sequence
<220>
   <223> BoNT/A, E. coli-modified 4
<400> 6
   atgcccttcg tgaataaaca gttcaactat aaagaccctg tgaatggtgt agacatcgcc 60
   tatatcaaga ttccgaatgc aggtcagatg cagccagtga aagcgttcaa aattcataat 120
   aagatctggg tgattcctga acgtgatacg ttcacgaacc cggaagaggg tgatttgaac 180
   ccacctcccg aagcaaaaca ggtgccagtg agctattacg actctacgta cctcagtaca 240
   gacaacgaga aagataacta cctgaagggc gtaacaaaac tgttcgaacg gatttatagt 300
   acagacctcg ggcgcatgct gttaaccagt attgtccgtg gaattccgtt ttggggcggt 360
   tcaacaattg ataccgaact gaaggtcatt gataccaact gcattaacgt tatccagcct 420
   gatgggtcct atcgttcgga agagcttaac cttgtgatta tcggtccttc ggccgatatt 480
   atccagtttg aatgtaaatc ctttggccat gaagtattaa atctgacccg caatggctat 540
   gggtctactc agtatattcg ttttagtccg gattttacct ttggctttga agagagttta 600
   gaagttgata caaatccgct gttaggtgca ggcaaattcg ccaccgatcc ggcggtgacc 660
   ctggcacatg aacttatcca tgcagggcac cgtctgtatg gtatcgccat taatccgaat 720
   cgtgttttta aggttaatac taatgcatac tatgagatga gtggtctgga agttagcttt 780
   gaggaattac gcacgttcgg gggtcatgat gcgaaattca ttgactcact ccaggaaaat 840
   gaatttcgcc tgtattacta taataaattt aaagatatcg cgtcgaccct gaataaagca 900
   aaatctattg tgggaacaac cgcatcgttg cagtacatga aaaacgtttt taaagagaaa 960
   tatttactct cagaagatac ttcaggaaag ttctctgtag ataaattgaa attcgataaa 1020
   ttgtacaaga tgttaaccga aatctacact gaagataact ttgttaaatt tttcaaagtt 1080
   ctgaatcgta agacctatct taacttcgat aaagcggttt tcaaaattaa tatcgttccg 1140
   aaagtgaact atactattta tgacggtttt aatctccgca acacgaacct cgcggctaac 1200
   tttaatggcc agaacaccga gatcaataac atgaacttta cgaaactgaa aaattttacc 1260
   ggcctgttcg agttttataa actgctttgt gtgcgtggta ttatcacgtc aaaaaccaaa 1320
   tctctggaca aaggctataa taaggccctc aatgatctgt gtatcaaggt caataactgg 1380
   gatctgtttt tctccccctc agaagataat tttacgaacg acctgaacaa aggagaagag 1440
   atcacttctg ataccaacat cgaggctgcc gaagagaata ttagcctgga cctgattcaa 1500
   cagtattacc tgactttcaa ttttgacaat gaacctgaaa atatttctat cgagaatctg 1560
   tcatccgaca ttatcggtca attagaactg atgccgaata ttgaacgttt tccgaatggt 1620
   aaaaagtacg agttggataa atatacaatg ttccattatt tgcgggccca ggagtttgaa 1680
   catggcaaat cccggatcgc tcttacgaac agcgtcaacg aagcgctgct taatccgtct 1740
   cgtgtctaca cgttctttag tagcgactac gttaaaaagg taaataaggc gacggaagct 1800
   gcgatgttct taggttgggt ggaacagctg gtatatgact tcaccgatga aacgagtgaa 1860
   gtttctacaa ccgacaaaat tgcagatatt actattatca ttccatatat tggtccggca 1920
   ctgaacattg gcaatatgct gtacaaagat gactttgtgg gagccctgat ttttagcggc 1980
   gctgtgattc tgttagagtt cattccggaa attgcgattc ccgtcctggg cacgtttgcg 2040
   cttgttagct acattgctaa taaggtgctg accgttcaaa ccatcgacaa tgccctctcg 2100
   aaacgcaatg aaaaatggga tgaggtgtac aaatatattg ttacaaactg gttagccaaa 2160
   gtgaacaccc agattgatct gattcgcaag aaaatgaaag aggcgttaga aaaccaggcc 2220
   gaggcaacga aagccatcat taactatcag tacaatcaat acactgagga agagaaaaat 2280
   aacattaatt ttaatattga tgacctgagc tccaaactga acgaaagcat taataaggca 2340
   atgattaaca ttaataaatt cctgaatcaa tgttctgttt cgtacctgat gaactcaatg 2400
   atcccatatg gcgttaaacg tctcgaggat tttgacgcat cattgaaaga cgcgctttta 2460
   aaatacatct atgataatcg tgggacgctg atcggccaag tggatcgttt gaaggataaa 2520
   gttaataaca ccctgagtac ggatattcca tttcaactgt ccaaatacgt ggacaaccag 2580
   cggttgcttt caacttttac cgagtatatc aagaatatta tcaacacgag tatccttaat 2640
   ctgcgctatg aaagtaatca cctgattgat ctgagccgct atgcttcgaa aattaatatc 2700
   ggcagcaaag tgaatttcga tcctatcgat aaaaaccaaa ttcagttgtt caacttggaa 2760
   tcgagtaaga tcgaggtgat tcttaagaac gcgattgtct acaacagcat gtacgaaaat 2820
   ttttccacct ccttctggat tcgtattccg aaatatttta actcgattag tttaaacaat 2880
   gaatatacta ttatcaactg catggaaaac aattccggat ggaaagtatc actgaattat 2940
   ggagaaatta tctggacact gcaggatact caagaaatta aacaacgtgt cgtattcaaa 3000
   tatagccaaa tgattaatat ctctgactat atcaaccgtt ggatctttgt gaccattact 3060
   aataaccgtc tgaacaattc gaaaatttac atcaacggcc gccttattga tcaaaagcca 3120
   atttccaacc tgggtaacat tcacgcgtca aataacatca tgtttaaact ggatggttgc 3180
   cgtgataccc accgttacat ttggatcaaa tattttaacc tgttcgataa ggaacttaac 3240
   gaaaaagaaa ttaaagattt atatgacaat cagagcaaca gtggtatctt gaaggatttt 3300
   tggggtgatt atctgcagta cgataagccc tattacatgc tgaatctcta tgacccgaat 3360
   aaatatgtag acgtaaacaa tgtcggaatt cgtggctata tgtatttaaa aggcccgcgc 3420
   gggtcggtta tgacaaccaa tatttactta aatagctccc tgtaccgcgg gaccaaattt 3480
   atcattaaga aatatgcttc tggtaacaag gacaacattg ttcgcaataa cgatcgtgtc 3540
   tatatcaacg tagtcgtaaa aaataaggaa tatcggttgg ctacaaacgc cagccaggcg 3600
   ggtgtcgaaa aaattctcag cgctttggaa atcccggatg tgggcaacct gtctcaggtc 3660
   gttgtgatga aaagcaagaa cgatcaggga atcactaata aatgcaaaat gaacctgcag 3720
   gataataacg gcaatgacat tggcttcatc gggtttcacc aatttaacaa tattgcgaaa 3780
   ctggtcgcct caaattggta caaccgccag attgagcgtt ctagccgtac actggggtgc 3840
   agctgggaat tcattccagt cgatgacggt tggggcgaac gtccgctgta a          3891
<210> 7
   <211> 3891
   <212> DNA
   <213> Artificial Sequence
<220>
   <223> BoNT/A, B. fragilis-modified 1
<400> 7
   atgccgttcg taaacaaaca gtttaactat aaggatccgg tgaacggagt cgatatagca 60
   tacatcaaaa tccctaatgc ggggcagatg cagccggtaa aagcattcaa aatccataat 120
   aaaatttggg taatcccgga gcgtgacact tttacgaacc cggaggaagg tgaccttaat 180
   ccccctccag aagctaaaca agtgccggtc agttactatg atagtacata tttgtcgaca 240
   gataacgaaa aagacaacta tctgaagggc gtgaccaaat tgtttgaacg tatatattcc 300
   acagatttgg gacgtatgct tttgactagc attgtacgcg gaatcccttt ttggggaggc 360
   tccacgattg atactgagct taaagttatc gacacgaatt gtatcaatgt gatacagccc 420
   gatggatcct atcgcagcga agagctgaac ttggtgatca taggacccag tgcagatata 480
   atccaatttg aatgcaaatc gttcggccac gaagtcctta acctgacaag gaatggttat 540
   ggatcaactc agtatatccg gttttctccc gacttcacat tcggtttcga agagtcactg 600
   gaagtggaca ccaatcctct tctgggagct ggaaaattcg ccacagatcc tgctgtgacc 660
   ctagcccatg aacttatcca tgccggacac cgtctgtacg gaatagcaat taacccaaat 720
   agagtgttca aagtaaacac taacgcctat tacgaaatga gcggtctcga agtttctttt 780
   gaggaactgc gtacgttcgg aggtcacgac gctaaattca tcgattcact tcaagaaaac 840
   gagttccgcc tttattacta taataaattt aaagacattg cgtccacact gaacaaagca 900
   aaaagtatag tcggtacaac ggcgtcatta caatacatga aaaatgtttt taaagaaaaa 960
   tacctattga gtgaggacac ttcggggaaa ttctccgtgg acaagctgaa atttgataaa 1020
   ctctacaaga tgctgacaga aatttatacg gaagacaatt ttgttaagtt ctttaaggtc 1080
   ctgaaccgca aaacgtatct aaacttcgac aaagctgttt tcaagattaa tatagtgcct 1140
   aaagttaact ataccattta tgatgggttc aatctgcgga atactaatct cgcagctaac 1200
   tttaacggac aaaataccga aattaacaat atgaatttta ccaagttgaa gaactttact 1260
   ggattgtttg aattttataa actactgtgc gttcggggca ttatcactag taaaaccaaa 1320
   agccttgata agggatataa taaagctttg aatgatttgt gcataaaagt gaataactgg 1380
   gatttgtttt tctctccctc tgaagataat tttacaaacg atctgaataa aggcgaagag 1440
   attaccagcg acacgaacat tgaagccgct gaagagaata tctcgctgga tcttattcaa 1500
   cagtattact taacattcaa ctttgataac gagccggaaa atatttctat cgaaaatctg 1560
   tcgagcgata ttataggtca attagaatta atgccgaata tcgaacgttt tccgaatggt 1620
   aaaaagtatg aattggataa atatactatg ttccattact tgcgggccca agagtttgaa 1680
   catggtaaat cacgcatagc cttgaccaat tcagtcaatg aggcattgct gaaccccagt 1740
   agagtttata ctttctttag ttctgattat gtgaaaaagg taaacaaggc taccgaggca 1800
   gcgatgtttc taggatgggt cgagcagctc gtatatgatt ttactgatga aacaagcgag 1860
   gtcagcacga ctgacaagat agctgatatc actatcatta taccttatat tgggccggcc 1920
   cttaatattg gtaatatgct gtataaggat gacttcgtag gcgcgctgat cttctctggt 1980
   gcagtgatct tacttgaatt tattcctgaa atcgccatcc cggtgctggg aacatttgcc 2040
   ctggtgtcct acattgctaa caaggtgttg acagttcaga cgattgataa tgcactttcg 2100
   aaacggaacg agaagtggga tgaggtgtat aagtacattg taactaattg gctggccaag 2160
   gtcaatacgc agattgacct tatccgtaag aaaatgaaag aagctctgga gaaccaggca 2220
   gaagccacca aggctatcat taattatcaa tacaatcaat atactgaaga ggaaaagaat 2280
   aacatcaatt tcaacatcga cgatttgtcg agcaagttaa atgagtcgat taataaggcc 2340
   atgatcaaca tcaataagtt cctaaatcag tgttccgtaa gttatcttat gaatagtatg 2400
   attccttatg gcgtaaagcg acttgaagac ttcgatgcat cactcaaaga cgcgttgctg 2460
   aaatacatct acgacaatcg tggaacatta atagggcaag tggaccgtct gaaagacaaa 2520
   gtcaataaca ccctgtccac ggatatacct tttcagctga gtaagtatgt tgataaccaa 2580
   agattgctct cgacattcac tgaatatata aaaaatataa ttaatacctc gatcttgaat 2640
   ttaagatacg aatcaaacca tttgattgat ctgtctaggt atgcttctaa aataaatata 2700
   gggtctaagg taaatttcga tcccatcgat aaaaatcaga tccagttatt taatttagag 2760
   tcttccaaaa tcgaagttat tctcaaaaac gcaattgtgt ataactcaat gtacgaaaat 2820
   tttagcacaa gcttttggat ccgtataccg aaatatttca attcgatctc attaaacaat 2880
   gaatatacca ttatcaattg tatggaaaac aattcaggat ggaaggtatc actgaactat 2940
   ggcgaaataa tctggacatt gcaggatacc caggagataa aacagcgtgt tgtattcaaa 3000
   tattcgcaaa tgattaatat atctgattat ataaaccgct ggatttttgt aaccattacc 3060
   aacaatcgat tgaacaattc taagatttat atcaatggac gtttgattga tcagaagccg 3120
   atatccaatc ttggaaacat ccacgctagt aacaatatta tgttcaaatt agatggctgt 3180
   cgcgatacgc atcggtacat atggattaag tattttaatt tgttcgataa agagctgaat 3240
   gaaaaggaaa tcaaagatct ctacgataat cagagtaaca gcggcatttt gaaggatttc 3300
   tggggtgact acctgcaata tgataagccg tattacatgt tgaacttata cgacccaaac 3360
   aaatatgtgg atgtaaacaa tgtaggtatc cgaggttata tgtacttgaa aggcccccgt 3420
   ggttcggtaa tgactaccaa catttatctc aatagttctc tgtatagggg aactaaattc 3480
   attataaaaa agtatgcatc tggaaataaa gacaacattg tacgtaataa cgacagagta 3540
   tatataaatg tcgtagttaa gaataaagag tatcggttag ccaccaatgc aagccaagcc 3600
   ggggtagaaa aaatcctgtc cgcccttgaa attccagacg ttgggaattt atcccaagta 3660
   gtcgttatga aatctaaaaa tgatcaaggt atcaccaata aatgtaaaat gaatttgcag 3720
   gataacaatg gtaatgacat aggctttatc ggatttcatc agtttaacaa tattgctaaa 3780
   ttagtagcca gtaactggta taatagacaa attgaacgct cttcccgcac actcggttgc 3840
   agttgggaat ttatacccgt ggacgacggt tggggtgaac gtcctctcta a 3891
<210> 8
   <211> 3891
   <212> DNA
   <213> Artificial Sequence
<220>
   <223> BoNT/A, B. fragilis-modified 2
<400> 8
   atgccgtttg taaataaaca gtttaattat aaagatccgg taaatggagt agatattgcc 60
   tatattaaaa ttccgaatgc cggacagatg cagccggtaa aagcctttaa aattcataat 120
   aaaatttggg taattccgga acgtgatacc tttaccaatc cggaagaagg agatctgaat 180
   ccgccgccgg aagccaaaca ggtaccggta tcttattatg attctaccta tctgtctacc 240
   gataatgaaa aagataatta tctgaaagga gtaaccaaac tgtttgaacg tatttattct 300
   accgatctgg gacgtatgct gctgacctct attgtacgtg gaattccgtt ttggggagga 360
   tctaccattg ataccgaact gaaagtaatt gataccaatt gtattaatgt aattcagccg 420
   gatggatctt atcgttctga agaactgaat ctggtaatta ttggaccgtc tgccgatatt 480
   attcagtttg aatgtaaatc ttttggacat gaagtactga atctgacccg taatggatat 540
   ggatctaccc agtatattcg tttttctccg gattttacct ttggatttga agaatctctg 600
   gaagtagata ccaatccgct gctgggagcc ggaaaatttg ccaccgatcc ggccgtaacc 660
   ctggcccatg aactgattca tgccggacat cgtctgtatg gaattgccat taatccgaat 720
   cgtgtattta aagtaaatac caatgcctat tatgaaatgt ctggactgga agtatctttt 780
   gaagaactgc gtacctttgg aggacatgat gccaaattta ttgattctct gcaggaaaat 840
   gaatttcgtc tgtattatta taataaattt aaagatattg cctctaccct gaataaagcc 900
   aaatctattg taggaaccac cgcctctctg cagtatatga aaaatgtatt taaagaaaaa 960
   tatctgctgt ctgaagatac ctctggaaaa ttttctgtag ataaactgaa atttgataaa 1020
   ctgtataaaa tgctgaccga aatttatacc gaagataatt ttgtaaaatt ttttaaagta 1080
   ctgaatcgta aaacctatct gaattttgat aaagccgtat ttaaaattaa tattgtaccg 1140
   aaagtaaatt ataccattta tgatggattt aatctgcgta ataccaatct ggccgccaat 1200
   tttaatggac agaataccga aattaataat atgaatttta ccaaactgaa aaattttacc 1260
   ggactgtttg aattttataa actgctgtgt gtacgtggaa ttattacctc taaaaccaaa 1320
   tctctggata aaggatataa taaagccctg aatgatctgt gtattaaagt aaataattgg 1380
   gatctgtttt tttctccgtc tgaagataat tttaccaatg atctgaataa aggagaagaa 1440
   attacctctg ataccaatat tgaagccgcc gaagaaaata tttctctgga tctgattcag 1500
   cagtattatc tgacctttaa ttttgataat gaaccggaaa atatttctat tgaaaatctg 1560
   tcttctgata ttattggaca gctggaactg atgccgaata ttgaacgttt tccgaatgga 1620
   aaaaaatatg aactggataa atataccatg tttcattatc tgcgtgccca ggaatttgaa 1680
   catggaaaat ctcgtattgc cctgaccaat tctgtaaatg aagccctgct gaatccgtct 1740
   cgtgtatata cctttttttc ttctgattat gtaaaaaaag taaataaagc caccgaagcc 1800
   gccatgtttc tgggatgggt agaacagctg gtatatgatt ttaccgatga aacctctgaa 1860
   gtatctacca ccgataaaat tgccgatatt accattatta ttccgtatat tggaccggcc 1920
   ctgaatattg gaaatatgct gtataaagat gattttgtag gagccctgat tttttctgga 1980
   gccgtaattc tgctggaatt tattccggaa attgccattc cggtactggg aacctttgcc 2040
   ctggtatctt atattgccaa taaagtactg accgtacaga ccattgataa tgccctgtct 2100
   aaacgtaatg aaaaatggga tgaagtatat aaatatattg taaccaattg gctggccaaa 2160
   gtaaataccc agattgatct gattcgtaaa aaaatgaaag aagccctgga aaatcaggcc 2220
   gaagccacca aagccattat taattatcag tataatcagt ataccgaaga agaaaaaaat 2280
   aatattaatt ttaatattga tgatctgtct tctaaactga atgaatctat taataaagcc 2340
   atgattaata ttaataaatt tctgaatcag tgttctgtat cttatctgat gaattctatg 2400
   attccgtatg gagtaaaacg tctggaagat tttgatgcct ctctgaaaga tgccctgctg 2460
   aaatatattt atgataatcg tggaaccctg attggacagg tagatcgtct gaaagataaa 2520
   gtaaataata ccctgtctac cgatattccg tttcagctgt ctaaatatgt agataatcag 2580
   cgtctgctgt ctacctttac cgaatatatt aaaaatatta ttaatacctc tattctgaat 2640
   ctgcgttatg aatctaatca tctgattgat ctgtctcgtt atgcctctaa aattaatatt 2700
   ggatctaaag taaattttga tccgattgat aaaaatcaga ttcagctgtt taatctggaa 2760
   tcttctaaaa ttgaagtaat tctgaaaaat gccattgtat ataattctat gtatgaaaat 2820
   ttttctacct ctttttggat tcgtattccg aaatatttta attctatttc tctgaataat 2880
   gaatatacca ttattaattg tatggaaaat aattctggat ggaaagtatc tctgaattat 2940
   ggagaaatta tttggaccct gcaggatacc caggaaatta aacagcgtgt agtatttaaa 3000
   tattctcaga tgattaatat ttctgattat attaatcgtt ggatttttgt aaccattacc 3060
   aataatcgtc tgaataattc taaaatttat attaatggac gtctgattga tcagaaaccg 3120
   atttctaatc tgggaaatat tcatgcctct aataatatta tgtttaaact ggatggatgt 3180
   cgtgataccc atcgttatat ttggattaaa tattttaatc tgtttgataa agaactgaat 3240
   gaaaaagaaa ttaaagatct gtatgataat cagtctaatt ctggaattct gaaagatttt 3300
   tggggagatt atctgcagta tgataaaccg tattatatgc tgaatctgta tgatccgaat 3360
   aaatatgtag atgtaaataa tgtaggaatt cgtggatata tgtatctgaa aggaccgcgt 3420
   ggatctgtaa tgaccaccaa tatttatctg aattcttctc tgtatcgtgg aaccaaattt 3480
   attattaaaa aatatgcctc tggaaataaa gataatattg tacgtaataa tgatcgtgta 3540
   tatattaatg tagtagtaaa aaataaagaa tatcgtctgg ccaccaatgc ctctcaggcc 3600
   ggagtagaaa aaattctgtc tgccctggaa attccggatg taggaaatct gtctcaggta 3660
   gtagtaatga aatctaaaaa tgatcaggga attaccaata aatgtaaaat gaatctgcag 3720
   gataataatg gaaatgatat tggatttatt ggatttcatc agtttaataa tattgccaaa 3780
   ctggtagcct ctaattggta taatcgtcag attgaacgtt cttctcgtac cctgggatgt 3840
   tcttgggaat ttattccggt agatgatgga tggggagaac gtccgctgta a          3891
<210> 9
   <211> 3891
   <212> DNA
   <213> Artificial Sequence
<220>
   <223> BoNT/A, B. fragilis-modified 3
<400> 9
   atgccttttg ttaataaaca attcaattac aaagatcctg taaacggtgt ggatatcgca 60
   tatataaaga tccccaatgc tggccaaatg cagcctgtca aggcttttaa aatacataat 120
   aaaatctggg taattcctga acgggatacc ttcactaatc ctgaagaggg agatctcaac 180
   cctcccccgg aagctaaaca ggttcccgtc agctactatg actccacgta tcttagtacc 240
   gataatgaaa aagacaatta cttgaaaggt gttaccaaat tatttgaacg gatttatagt 300
   accgatttag gccgtatgct cttaacctct atagttcgtg gaataccgtt ttggggaggc 360
   tctactattg acactgagtt gaaagttatc gataccaact gcattaatgt gattcagccc 420
   gatggtagtt accgttctga agagctgaac ctggtaatca taggaccgtc agctgatata 480
   attcagtttg agtgtaaatc ctttggacat gaggtattga acttgactcg taacggatat 540
   ggaagtacgc aatatattag atttagcccg gattttacgt ttggattcga agagtctctg 600
   gaggtggata caaatccgct gttgggagcc ggaaaattcg ccaccgatcc tgcagtaacg 660
   ttggcccacg aacttatcca cgccggacat agattgtacg gtattgcaat aaatccgaat 720
   cgtgtgttta aggtgaatac taatgcctat tacgaaatgt cgggtctcga agtgagcttc 780
   gaagagctta gaacgtttgg tggccatgat gcaaaattta ttgacagcct gcaagaaaat 840
   gaattccgtc tgtactatta caataagttc aaggacatcg ccagcacact gaataaagca 900
   aaaagcattg taggcacaac ggcttcactt cagtacatga aaaacgtgtt caaagaaaag 960
   tacctgctta gtgaagacac ttcgggaaag ttttcggtcg ataagctgaa gtttgacaaa 1020
   ttgtataaaa tgctcacaga aatttatacg gaggacaatt tcgtgaaatt tttcaaggta 1080
   ttaaaccgta aaacgtacct gaactttgac aaagccgtgt tcaaaataaa cattgtgccg 1140
   aaagttaact atacaatata tgatggattt aaccttcgga acacaaatct cgccgctaat 1200
   tttaatggtc aaaacaccga aatcaacaat atgaatttca caaagcttaa aaactttaca 1260
   ggactgtttg agttctataa acttctgtgc gtgcgtggta tcattaccag taagacaaaa 1320
   tccttggata agggttacaa caaagcattg aacgacttgt gtatcaaggt caataactgg 1380
   gatctgttct ttagtcccag tgaagataat tttactaatg atttgaacaa gggagaagag 1440
   atcacgtcag atactaatat tgaagcagct gaagagaata taagccttga cctgattcag 1500
   caatattact tgacgtttaa tttcgataac gagccggaaa atatctcgat agaaaatttg 1560
   tctagtgata ttataggtca gctcgaatta atgccgaaca ttgagcgttt tcctaatggc 1620
   aaaaagtatg aactcgacaa atatactatg tttcattatt tgcgggccca agaatttgaa 1680
   cacggtaaaa gtcggatcgc tttaacgaat tctgttaacg aggccttatt gaatccctca 1740
   cgtgtatata ctttctttag ttcggattac gtgaagaaag tgaataaagc caccgaagct 1800
   gccatgttct tgggctgggt agaacagctg gtatatgatt tcacagatga aacatctgag 1860
   gtatccacaa ccgataaaat tgctgatatc acaattatca taccttatat tggaccggct 1920
   ctgaatatag gaaatatgtt atataaagac gatttcgttg gagcccttat cttttctggt 1980
   gctgttatcc tgcttgaatt catcccggaa attgcaattc ccgtcttggg aaccttcgcc 2040
   ctcgtttctt acattgccaa taaagtcctg acagtgcaga ccatcgataa tgccctgtcg 2100
   aagcgcaacg aaaagtggga cgaagtgtat aaatatatcg ttaccaattg gctggccaaa 2160
   gtaaatacac agattgattt aatacgtaag aaaatgaagg aagccttgga gaatcaagct 2220
   gaagcaacaa aggccatcat taattatcag tataatcagt atactgaaga ggaaaaaaac 2280
   aatattaatt tcaacataga cgatttgtcc agcaaattaa acgaatcaat aaacaaagca 2340
   atgattaata ttaataaatt ccttaatcaa tgttccgtat cctatctgat gaacagcatg 2400
   ataccgtatg gagtaaaacg gttagaggac tttgatgcat ccctgaaaga tgccctgttg 2460
   aaatatatct acgataatcg tggaactctt ataggccaag tggaccgttt gaaagataaa 2520
   gtgaataaca ctcttagtac cgatattccg tttcaattat caaagtatgt tgacaatcaa 2580
   cgtctgcttt cgactttcac cgagtatatc aaaaatatca ttaatacaag tatcttgaat 2640
   ctgagatatg aaagcaatca tttaatcgac ctgtcgagat acgcctctaa gataaatatc 2700
   ggctctaaag tcaactttga cccgattgat aaaaatcaaa tccagctgtt taacctggaa 2760
   agctctaaaa ttgaagtcat cttgaagaat gcaatagtat ataattctat gtatgagaat 2820
   ttcagcacct ccttctggat tcgcatcccg aaatatttca actctatctc tctcaacaat 2880
   gaatacacta ttataaactg tatggaaaat aacagtggat ggaaagtatc gctgaactac 2940
   ggtgaaatca tatggaccct ccaggataca caggagatta aacagcgtgt agtttttaaa 3000
   tactcccaga tgatcaatat atccgactat attaaccgct ggatcttcgt aactattact 3060
   aacaatcgtt taaacaattc gaaaatttac attaatggtc ggcttattga ccaaaagccc 3120
   atatccaacc ttggaaatat acatgcatct aacaatatta tgttcaagct cgatggatgc 3180
   agagatactc accgttatat ttggatcaag tatttcaacc tgtttgacaa agagctgaac 3240
   gagaaggaaa tcaaagatct gtacgataac cagtcaaatt ctggaatctt gaaggatttt 3300
   tggggagatt atctgcagta tgacaaaccg tattacatgt taaaccttta tgaccctaat 3360
   aagtatgtag atgtcaataa cgtgggtata cgcggatata tgtatttaaa gggtccgcgt 3420
   ggttcggtaa tgactacgaa catctatctg aacagttcct tgtatcgcgg taccaagttc 3480
   attatcaaga aatatgcatc gggaaacaag gataatatag tacgtaataa cgaccgcgtc 3540
   tatatcaatg tggttgtaaa aaataaggaa tatcgtttgg ccaccaatgc ctcacaagca 3600
   ggtgtggaga aaatcctgag tgctttggaa atacctgatg taggcaatct gtcacaggtc 3660
   gtagtcatga aatcgaaaaa tgaccaaggt atcacaaaca aatgcaagat gaacctgcag 3720
   gacaataacg gaaacgatat tggattcatc ggcttccatc aatttaacaa tattgctaaa 3780
   ttggtggcta gcaattggta taatcgccaa atcgaacgct catcacgcac gctgggatgt 3840
   tcatgggaat ttataccggt tgacgacggc tggggagaac gtcccctgta a          3891
<210> 10
   <211> 3891
   <212> DNA
   <213> Artificial Sequence
<220>
   <223> BoNT/A, B. licheniformis-modified 1
<400> 10
   atgccgtttg tgaataaaca gtttaattat aaggatcccg tcaatggggt cgatattgcc 60
   tatatcaaaa tccctaatgc cggccagatg caaccggtta aggcattcaa aatccataat 120
   aagatctggg tgataccgga acgcgataca tttacaaacc ctgaagaggg cgatcttaac 180
   ccaccgccag aagcgaaaca agtccctgtc tcctactatg actcgacgta tttgtcaacg 240
   gataatgaaa aagataatta tctgaaaggc gtcactaaac tgttcgaacg gatctatagc 300
   acagacctag gccgcatgct cttgacgagc atcgtaagag gcatcccgtt ttggggaggg 360
   tccaccatcg acacagagct caaggtgatc gatacaaact gcataaacgt tatccagccg 420
   gatggatctt atcgcagcga agagcttaac ctcgtgatca ttgggccgag cgcggacatc 480
   attcagttcg agtgcaagtc gtttggtcac gaagtactca atttgaccag aaacggctac 540
   ggatccactc aatacattag gttttccccc gattttacgt tcggattcga agagtcactt 600
   gaagttgata ctaacccatt acttggcgcc gggaaatttg cgactgaccc cgcggttaca 660
   ctagcacatg aactcatcca tgcaggacac cgcctttatg gaatcgcgat caatccgaac 720
   cgcgtgttta aagtcaatac taatgcatat tacgagatgt caggcctaga agtttcattt 780
   gaagagttgc gtacctttgg gggccacgat gccaaattta tcgacagcct gcaagaaaac 840
   gaatttcgcc tctattacta taacaaattt aaagacattg cgtctacctt aaacaaagcc 900
   aaatccatcg tcggcacgac cgctagcctc caatacatga aaaatgtctt taaagagaaa 960
   tatttgcttt ctgaagacac cagcggtaag ttcagcgtgg ataaattaaa attcgacaag 1020
   ctttataaga tgctgacaga aatatacact gaagataatt ttgttaagtt tttcaaagtt 1080
   ttgaaccgca aaacgtactt gaactttgac aaagcagtct tcaagattaa tattgtcccg 1140
   aaagtgaact ataccattta cgacggtttt aatttacgga atacgaactt agcagctaat 1200
   tttaacggac agaacacgga aatcaacaat atgaatttca cgaagcttaa aaacttcaca 1260
   ggtctgtttg aattctataa attgctctgt gtcaggggga tcataacaag caagacaaag 1320
   agcctggaca agggctataa caaagcactt aatgaccttt gcattaaggt gaacaattgg 1380
   gacttgtttt tcagcccttc tgaagataat ttcacaaatg atttaaacaa aggagaagag 1440
   atcacctcgg acacgaatat cgaggctgcg gaggaaaaca tttcgttaga tttaatccag 1500
   caatattacc tgacatttaa ctttgacaac gagccggaaa acattagcat tgaaaactta 1560
   agctccgata tcataggcca gctcgagctt atgcctaata ttgaacgttt tcctaatgga 1620
   aaaaagtatg aactggacaa atatacaatg tttcattatt tgagagccca agagttcgaa 1680
   catggaaaga gcaggattgc cctgacaaat agtgtaaacg aagccttgtt aaatccgtcc 1740
   cgcgtttaca cgttcttttc atcggactac gtaaaaaagg taaacaaggc gacagaagcg 1800
   gctatgtttc ttggttgggt ggaacagctc gtttacgact tcacggatga gacaagcgaa 1860
   gtctctacga ccgataaaat cgcagatatt acaatcataa tcccgtatat cgggcccgca 1920
   cttaacatcg gcaacatgct gtataaagac gattttgttg gcgcactgat tttcagtggc 1980
   gcggtcattc tcttggaatt cattccggaa attgcgatac ctgtgcttgg aacgtttgcc 2040
   ctggttagtt acatcgcgaa caaggtcttg acagtccaaa ccattgataa tgcactttcg 2100
   aagcgcaatg aaaaatggga tgaagtttat aaatatatcg tcacgaactg gttagcgaaa 2160
   gttaacacgc agatcgactt gattcgtaaa aagatgaaag aagcccttga gaatcaagcc 2220
   gaagctacaa aagctatcat taactatcaa tataatcagt acacggaaga ggaaaaaaat 2280
   aacattaact ttaatataga cgatctctct agcaaactga acgagtccat taacaaggct 2340
   atgatcaaca tcaataaatt ccttaaccag tgcagtgtgt catatctgat gaactcgatg 2400
   atcccgtacg gagttaaaag acttgaagat tttgatgcct ctcttaaaga tgcgctgctt 2460
   aaatatatct acgacaaccg tggtacactg attggtcaag tcgatagact taaggataaa 2520
   gtgaacaata cgctgtcaac ggatattcct tttcaattga gcaaatacgt cgataatcag 2580
   agactgttat ctacctttac ggaatacatc aaaaacatta tcaacacgtc gatccttaat 2640
   ttaagatacg aatcaaacca tctgatcgat ctgtccagat atgcctcaaa aatcaatata 2700
   ggatcaaaag taaattttga cccgatcgat aaaaaccaga tacagctgtt caatctcgag 2760
   tcttcaaaaa tcgaagtaat tcttaagaat gcgatcgtct ataattcgat gtatgaaaac 2820
   tttagcacat cattctggat taggattccg aaatacttta attcgatttc actgaacaat 2880
   gagtacacga ttatcaactg catggagaac aattccgggt ggaaagtatc tctgaattac 2940
   ggagaaatta tctggacatt gcaggacacc caagaaatta aacagcgggt ggtctttaaa 3000
   tactcacaga tgatcaacat ctccgattat ataaatcgat ggatctttgt gaccatcaca 3060
   aacaataggc tgaataactc aaagatctat atcaacggaa gattaattga ccagaaacca 3120
   atttcaaacc tcggcaacat tcacgcttcg aacaatatca tgttcaaact tgatggttgt 3180
   cgggacactc atcggtatat ttggatcaag tattttaatt tattcgacaa ggagctcaac 3240
   gagaaagaaa ttaaagatct ctatgacaat caatctaaca gcgggattct gaaggatttt 3300
   tggggagact atctgcagta cgacaaaccg tactatatgc tgaatctgta tgatcccaat 3360
   aaatatgtgg atgttaataa cgttggcatt cggggatata tgtacttgaa aggcccgcga 3420
   ggatcggtga tgacgactaa catttacctg aatagctcgc tatacagggg aacgaagttt 3480
   atcattaaaa agtatgcgtc cggcaataaa gataatattg tccggaataa cgaccgagtc 3540
   tatatcaatg tcgtagtcaa aaacaaagaa taccgtttag ctacgaacgc ttctcaagct 3600
   ggtgtagaaa agattctttc tgccttggaa atccctgatg tcgggaacct gagccaagtg 3660
   gttgtcatga aaagcaaaaa tgatcaaggc ataacgaata aatgcaaaat gaacttgcag 3720
   gacaacaatg gcaatgatat tggatttatt ggcttccacc aatttaacaa tattgcaaaa 3780
   cttgttgcct ccaattggta taatcgccag attgaaagat caagtcgcac gctcgggtgt 3840
   tcgtgggaat ttatcccggt tgacgatggc tggggcgaac ggccgttgta a          3891
<210> 11
   <211> 3891
   <212> DNA
   <213> Artificial Sequence
<220>
   <223> BoNT/A, B. licheniformis-modified 2
<400> 11
   atgccgtttg tcaacaaaca gtttaactat aaagatccgg tcaacggcgt cgatatcgcg 60
   tatatcaaaa tcccgaacgc gggccagatg cagccggtca aagcgtttaa aatccataac 120
   aaaatctggg tcatcccgga acgcgatacg tttacgaacc cggaagaagg cgatctgaac 180
   ccgccgccgg aagcgaaaca ggtcccggtc agctattatg atagcacgta tctgagcacg 240
   gataacgaaa aagataacta tctgaaaggc gtcacgaaac tgtttgaacg catctatagc 300
   acggatctgg gccgcatgct gctgacgagc atcgtccgcg gcatcccgtt ttggggcggc 360
   agcacgatcg atacggaact gaaagtcatc gatacgaact gcatcaacgt catccagccg 420
   gatggcagct atcgcagcga agaactgaac ctggtcatca tcggcccgag cgcggatatc 480
   atccagtttg aatgcaaaag ctttggccat gaagtcctga acctgacgcg caacggctat 540
   ggcagcacgc agtatatccg ctttagcccg gattttacgt ttggctttga agaaagcctg 600
   gaagtcgata cgaacccgct gctgggcgcg ggcaaatttg cgacggatcc ggcggtcacg 660
   ctggcgcatg aactgatcca tgcgggccat cgcctgtatg gcatcgcgat caacccgaac 720
   cgcgtcttta aagtcaacac gaacgcgtat tatgaaatga gcggcctgga agtcagcttt 780
   gaagaactgc gcacgtttgg cggccatgat gcgaaattta tcgatagcct gcaggaaaac 840
   gaatttcgcc tgtattatta taacaaattt aaagatatcg cgagcacgct gaacaaagcg 900
   aaaagcatcg tcggcacgac ggcgagcctg cagtatatga aaaacgtctt taaagaaaaa 960
   tatctgctga gcgaagatac gagcggcaaa tttagcgtcg ataaactgaa atttgataaa 1020
   ctgtataaaa tgctgacgga aatctatacg gaagataact ttgtcaaatt ttttaaagtc 1080
   ctgaaccgca aaacgtatct gaactttgat aaagcggtct ttaaaatcaa catcgtcccg 1140
   aaagtcaact atacgatcta tgatggcttt aacctgcgca acacgaacct ggcggcgaac 1200
   tttaacggcc agaacacgga aatcaacaac atgaacttta cgaaactgaa aaactttacg 1260
   ggcctgtttg aattttataa actgctgtgc gtccgcggca tcatcacgag caaaacgaaa 1320
   agcctggata aaggctataa caaagcgctg aacgatctgt gcatcaaagt caacaactgg 1380
   gatctgtttt ttagcccgag cgaagataac tttacgaacg atctgaacaa aggcgaagaa 1440
   atcacgagcg atacgaacat cgaagcggcg gaagaaaaca tcagcctgga tctgatccag 1500
   cagtattatc tgacgtttaa ctttgataac gaaccggaaa acatcagcat cgaaaacctg 1560
   agcagcgata tcatcggcca gctggaactg atgccgaaca tcgaacgctt tccgaacggc 1620
   aaaaaatatg aactggataa atatacgatg tttcattatc tgcgcgcgca ggaatttgaa 1680
   catggcaaaa gccgcatcgc gctgacgaac agcgtcaacg aagcgctgct gaacccgagc 1740
   cgcgtctata cgttttttag cagcgattat gtcaaaaaag tcaacaaagc gacggaagcg 1800
   gcgatgtttc tgggctgggt cgaacagctg gtctatgatt ttacggatga aacgagcgaa 1860
   gtcagcacga cggataaaat cgcggatatc acgatcatca tcccgtatat cggcccggcg 1920
   ctgaacatcg gcaacatgct gtataaagat gattttgtcg gcgcgctgat ctttagcggc 1980
   gcggtcatcc tgctggaatt tatcccggaa atcgcgatcc cggtcctggg cacgtttgcg 2040
   ctggtcagct atatcgcgaa caaagtcctg acggtccaga cgatcgataa cgcgctgagc 2100
   aaacgcaacg aaaaatggga tgaagtctat aaatatatcg tcacgaactg gctggcgaaa 2160
   gtcaacacgc agatcgatct gatccgcaaa aaaatgaaag aagcgctgga aaaccaggcg 2220
   gaagcgacga aagcgatcat caactatcag tataaccagt atacggaaga agaaaaaaac 2280
   aacatcaact ttaacatcga tgatctgagc agcaaactga acgaaagcat caacaaagcg 2340
   atgatcaaca tcaacaaatt tctgaaccag tgcagcgtca gctatctgat gaacagcatg 2400
   atcccgtatg gcgtcaaacg cctggaagat tttgatgcga gcctgaaaga tgcgctgctg 2460
   aaatatatct atgataaccg cggcacgctg atcggccagg tcgatcgcct gaaagataaa 2520
   gtcaacaaca cgctgagcac ggatatcccg tttcagctga gcaaatatgt cgataaccag 2580
   cgcctgctga gcacgtttac ggaatatatc aaaaacatca tcaacacgag catcctgaac 2640
   ctgcgctatg aaagcaacca tctgatcgat ctgagccgct atgcgagcaa aatcaacatc 2700
   ggcagcaaag tcaactttga tccgatcgat aaaaaccaga tccagctgtt taacctggaa 2760
   agcagcaaaa tcgaagtcat cctgaaaaac gcgatcgtct ataacagcat gtatgaaaac 2820
   tttagcacga gcttttggat ccgcatcccg aaatatttta acagcatcag cctgaacaac 2880
   gaatatacga tcatcaactg catggaaaac aacagcggct ggaaagtcag cctgaactat 2940
   ggcgaaatca tctggacgct gcaggatacg caggaaatca aacagcgcgt cgtctttaaa 3000
   tatagccaga tgatcaacat cagcgattat atcaaccgct ggatctttgt cacgatcacg 3060
   aacaaccgcc tgaacaacag caaaatctat atcaacggcc gcctgatcga tcagaaaccg 3120
   atcagcaacc tgggcaacat ccatgcgagc aacaacatca tgtttaaact ggatggctgc 3180
   cgcgatacgc atcgctatat ctggatcaaa tattttaacc tgtttgataa agaactgaac 3240
   gaaaaagaaa tcaaagatct gtatgataac cagagcaaca gcggcatcct gaaagatttt 3300
   tggggcgatt atctgcagta tgataaaccg tattatatgc tgaacctgta tgatccgaac 3360
   aaatatgtcg atgtcaacaa cgtcggcatc cgcggctata tgtatctgaa aggcccgcgc 3420
   ggcagcgtca tgacgacgaa catctatctg aacagcagcc tgtatcgcgg cacgaaattt 3480
   atcatcaaaa aatatgcgag cggcaacaaa gataacatcg tccgcaacaa cgatcgcgtc 3540
   tatatcaacg tcgtcgtcaa aaacaaagaa tatcgcctgg cgacgaacgc gagccaggcg 3600
   ggcgtcgaaa aaatcctgag cgcgctggaa atcccggatg tcggcaacct gagccaggtc 3660
   gtcgtcatga aaagcaaaaa cgatcagggc atcacgaaca aatgcaaaat gaacctgcag 3720
   gataacaacg gcaacgatat cggctttatc ggctttcatc agtttaacaa catcgcgaaa 3780
   ctggtcgcga gcaactggta taaccgccag atcgaacgca gcagccgcac gctgggctgc 3840
   agctgggaat ttatcccggt cgatgatggc tggggcgaac gcccgctgta a 3891
<210> 12
   <211> 3891
   <212> DNA
   <213> Artificial Sequence .
<220>
   <223> BoNT/A, B. licheniformis-modified 3
<400> 12
   atgccgttcg ttaataaaca gtttaactat aaagaccctg tgaacggagt ggatattgcg 60
   tatattaaga ttccgaatgc gggccagatg caacctgtca aagcttttaa aatccataac 120
   aaaatctggg ttatcccgga acgtgatacg tttaccaacc cggaagaggg cgatctgaac 180
   cctccgcctg aagcgaaaca ggtgccggtc tcatattacg attcgacgta ccttagcaca 240
   gataacgaaa aagacaacta tctcaaaggg gtgacaaaac tgtttgaacg catttacagc 300
   acagacctgg gccggatgct ccttacatca atcgtgagag gcatcccgtt ctggggtgga 360
   tccaccattg acacggaact gaaagtgatc gacacgaatt gcattaacgt catccagccg 420
   gatgggagct acaggtcgga ggaactgaat cttgttatta tcggtccgtc tgcggacatc 480
   attcaattcg aatgtaaaag ctttgggcat gaagttctca acctgacgcg gaacggttac 540
   ggcagcacac aatatatccg gtttagcccg gactttacat ttggctttga agagagcctt 600
   gaagtggata caaatccgtt gctgggagcg ggaaaatttg caaccgatcc ggccgttaca 660
   cttgcacacg aattaatcca tgcgggccac cgtctgtacg gtatcgccat taatccgaac 720
   agagttttta aagtcaacac caacgcttac tatgaaatgt ccgggctgga ggtcagcttt 780
   gaagagttac gcacattcgg cgggcacgat gcaaaattta tcgattcatt gcaggagaat 840
   gaatttagac tctattacta taataaattt aaggacatcg cgtctaccct gaacaaagca 900
   aaatctatcg ttggcaccac ggcatcatta caatatatga aaaatgtctt caaagaaaaa 960
   tatttgctta gcgaagatac atcgggaaaa ttctccgttg acaaattgaa attcgataaa 1020
   ctttataaaa tgttaacgga aatctatacg gaagataact ttgtaaaatt ctttaaagtc 1080
   ctgaacagga agacgtacct taacttcgat aaagcagtgt tcaaaattaa tatcgtacct 1140
   aaggtcaact acaccatcta cgacggattt aatcttagaa acacgaatct ggcggcaaat 1200
   tttaatggac agaacacgga aattaataac atgaacttta caaagctcaa gaacttcacg 1260
   ggactgtttg agttttacaa gcttctgtgc gtgaggggta tcattacgtc taagacaaaa 1320
   agcctcgaca aaggctataa caaagcattg aacgacttat gcattaaagt aaacaattgg 1380
   gatttatttt tctcaccttc tgaagataat ttcacaaacg atttaaataa gggcgaagag 1440
   attacgagcg atacaaacat tgaggccgca gaagagaata ttagcttgga cctcatccag 1500
   caatattacc ttacgtttaa ttttgataac gaaccggaaa atatctcaat cgagaacctg 1560
   tcatcggaca tcattggcca gttggagctg atgcctaaca ttgaacggtt cccgaatggg 1620
   aaaaagtatg aactggataa gtatacgatg tttcattacc tgcgggccca ggaatttgaa 1680
   catggaaaga gccgcattgc cctcacgaat tcagtcaatg aagccctctt gaacccgtct 1740
   agggtctaca catttttctc ctcagactat gtcaagaaag tcaacaaagc cacagaggcg 1800
   gctatgtttt tgggatgggt cgaacagctc gtttacgatt ttacggatga gacaagcgag 1860
   gtctcaacga cagacaagat cgctgacatt acaatcatta tcccgtatat cggcccggcc 1920
   ttaaacatcg gtaacatgct ttacaaagat gactttgtcg gcgccctgat tttttcgggc 1980
   gcggtcatct tacttgagtt catcccggag atcgccatcc cggtccttgg gacctttgct 2040
   cttgtaagct atattgccaa taaagtgctc acagtgcaaa cgattgataa cgctctgagc 2100
   aagcgtaatg aaaaatggga cgaagtctat aaatatatcg tgacgaattg gcttgcgaaa 2160
   gtaaacacgc agatcgactt gatccgtaaa aagatgaagg aagccctgga aaaccaagcc 2220
   gaagcgacga aggcgattat caattaccag tataaccagt acacggaaga ggaaaagaac 2280
   aatattaatt ttaacatcga cgatttgtct agcaagctca atgaaagcat taataaagca 2340
   atgatcaata ttaataaatt ccttaatcaa tgctctgtca gctatctgat gaactcaatg 2400
   attccgtatg gagtgaagag gctggaggat ttcgacgctt ccctgaaaga cgctttgctt 2460
   aaatacatct acgataacag aggcacgttg atcggacaag ttgacagact taaggataag 2520
   gtcaacaata cgctgtccac ggacatcccg tttcagttaa gcaaatacgt tgataatcag 2580
   cgcctgcttt cgacatttac cgagtacatc aaaaacatca ttaacacgtc tattctgaat 2640
   ctgcgctatg aaagcaatca tctgatcgat ttatccagat acgcatccaa aatcaatatc 2700
   ggatcgaagg ttaacttcga tccgatcgat aaaaaccaga tccaactctt taatctcgaa 2760
   agctctaaga ttgaagtaat cttaaaaaat gccattgtgt ataacagcat gtatgagaat 2820
   ttctcaacgt cattttggat ccgcatccct aaatatttca attccatctc gctgaataac 2880
   gaatatacga ttatcaattg catggaaaat aactcaggat ggaaggtgag cttgaattat 2940
   ggggagatca tttggaccct gcaggatacg caagaaatta aacaacgcgt ggtcttcaag 3000
   tattcacaaa tgattaacat ttcggattac attaacagat ggatcttcgt tacgattacc 3060
   aacaatagac tgaataactc aaagatctac atcaatggcc gcttgattga tcaaaaaccg 3120
   atctctaatc ttgggaacat ccacgcctcg aacaatatca tgtttaaatt ggacggctgc 3180
   cgggacacgc acaggtatat ctggattaaa tatttcaatt tatttgataa agagcttaac 3240
   gaaaaagaaa ttaaagatct ttatgacaat cagagcaatt caggtattct caaggacttt 3300
   tggggtgatt atttgcaata cgacaaaccg tattacatgt tgaatttata tgacccgaac 3360
   aaatatgtag acgtaaataa cgtcgggatc cggggctata tgtacctcaa agggccgcgc 3420
   ggctcggtca tgacaacgaa tatctattta aattcctctc tttatcgcgg aacgaaattt 3480
   atcattaaga aatacgcgag cggaaacaaa gataatattg ttcgcaataa cgacagagtt 3540
   tatattaacg ttgtcgttaa gaacaaagaa tatcgcttgg caacaaacgc gtcccaagct 3600
   ggagtcgaaa aaatcctttc ggctttggaa atcccggatg taggcaacct ctcgcaagta 3660
   gtggttatga aatccaaaaa cgatcagggc atcaccaata aatgtaagat gaatttacag 3720
   gacaataacg gaaacgatat tggcttcatc ggatttcatc agtttaataa catcgctaaa 3780
   ctggttgcgt ctaattggta taatcgccaa atcgaacgct cgtcccggac cctgggctgt 3840
   agctgggaat ttattccggt cgatgatggt tggggagaac gtcctcttta a          3891
<210> 13
   <211> 3891
   <212> DNA
   <213> Artificial Sequence
<220>
   <223> BoNT/A, B. subtilis-modified 1
<400> 13
   atgccatttg tcaataaaca gtttaactat aaggatcctg ttaacggagt cgatattgca 60
   tatattaaaa tacctaatgc aggccagatg caacctgtca aagccttcaa aatccataac 120
   aaaatctggg tcatcccgga acgggataca tttacgaacc ctgaagaggg ggatctgaac 180
   ccgcctccgg aagcgaagca agttcctgtg agctactatg atagcacata tctgtctacg 240
   gataatgaaa aggataatta tctcaaaggt gttaccaagc tttttgaacg gatatactcg 300
   accgacctcg gacggatgtt gctgacatct atcgtgagag gcattccttt ttggggtggc 360
   tctacaattg acactgaatt aaaggtcatc gatactaact gtattaatgt tattcagcct 420
   gatggatcct accgtagtga agagttgaac ttagtaataa ttgggccctc ggccgacatt 480
   atccagttcg agtgcaaaag cttcggtcat gaggttctga atttaacacg taacggctat 540
   ggctcaacac agtatatccg attctctcca gatttcacct ttgggttcga agagagcctg 600
   gaggtagata cgaacccgtt gttaggagcg gggaaattcg cgacggatcc agccgtgaca 660
   cttgcccatg aattaatcca cgcaggccat cgtctttatg gaatcgcaat taaccccaac 720
   cgtgtcttta aagtaaacac taatgcctat tacgaaatgt cagggctgga agtctcgttt 780
   gaggaacttc ggacgttcgg cggacacgac gcaaagttta ttgatagcct tcaagagaac 840
   gaatttcgtc tttattacta taataaattt aaagacatcg ccagcacact aaataaagcg 900
   aaatccatcg taggcacgac agctagcctt caatacatga aaaacgtgtt taaagagaag 960
   tacctgctta gcgaggatac gagcgggaaa tttagcgtcg ataagctgaa atttgataaa 1020
   ctctataaaa tgctaacaga aatttataca gaggataatt ttgtgaaatt ctttaaggtc 1080
   ttaaatagaa aaacatacct taactttgat aaggcggtgt ttaagattaa catcgtaccg 1140
   aaagttaact acaccatcta tgatggattt aacttaagaa acacaaattt ggctgcaaat 1200
   tttaatggcc agaacaccga aattaacaat atgaatttca ccaaactcaa aaattttacg 1260
   ggactgtttg agttctataa attgctatgc gttagaggta tcataacgag caaaaccaaa 1320
   tcgctggaca agggctacaa caaagctctg aatgatcttt gtattaaagt gaataactgg 1380
   gatcttttct tttcaccctc cgaagataac ttcactaacg atttaaataa aggagaagag 1440
   atcacgagtg atactaatat tgaagcggct gaagagaata tcagtcttga cttaatccaa 1500
   cagtattacc tgacatttaa cttcgataac gaacctgaaa acatcagcat cgaaaacttg 1560
   agttcagaca ttatcggcca gctggaatta atgccgaata tcgaaaggtt cccgaatggc 1620
   aaaaagtatg agctggataa atacacaatg tttcactatc ttcgcgcgca ggaatttgag 1680
   catggaaaat cgagaatcgc gcttactaat tccgtcaatg aagctctact taatccatct 1740
   cgtgtatata cgtttttctc atctgattat gtgaagaaag tgaacaaagc aacagaagct 1800
   gccatgtttc ttggatgggt agaacaactt gtttatgact tcacagacga aacgtcggaa 1860
   gtatcaacca cagataaaat tgcggacata acaattatca ttccgtatat tggcccggcc 1920
   cttaacattg gcaacatgct atataaggat gactttgttg gtgcactgat ttttagtggt 1980
   gctgtaatct tgctcgaatt tattccggag atcgctattc cggttcttgg gacatttgct 2040
   ctggtttcat atattgcgaa taaagtcttg actgttcaga ctattgataa tgcattgtca 2100
   aaacgcaatg aaaaatggga cgaagtttat aaatatattg taactaattg gttggctaaa 2160
   gtaaacacgc aaattgacct tattaggaaa aagatgaaag aggccctaga aaatcaagcc 2220
   gaagctacca aagctataat caattatcag tacaatcaat acacagaaga ggaaaaaaac 2280
   aatattaatt ttaatattga tgacttgtct agcaagctta acgaaagcat taataaagca 2340
   atgattaaca tcaacaagtt tttgaaccaa tgctcggtct catatttgat gaattcaatg 2400
   atcccgtacg gtgttaaacg tttggaagat tttgacgcga gcttaaaaga cgccttactc 2460
   aaatacattt acgacaatcg cggtacgctg attggccaag tagatcggct taaagacaaa 2520
   gtgaacaata cgctctccac cgatatacca ttccaattat ctaagtatgt tgacaaccaa 2580
   agactgttgt ctacatttac agaatacatc aaaaacataa ttaacacaag tattctcaat 2640
   ctgcgctatg agagcaacca cctgatcgac ttaagtcggt acgctagcaa gattaatatt 2700
   ggatctaaag ttaatttcga tccgatcgat aaaaatcaga tacagttgtt caacctcgaa 2760
   tcatctaaga ttgaggtgat tttaaagaac gcaatcgttt acaattcgat gtatgaaaat 2820
   ttttccacga gtttttggat cagaatccct aagtatttca attcaatttc cctgaacaat 2880
   gaatacacta ttatcaattg tatggaaaac aattccggtt ggaaggtgag cctgaattat 2940
   ggagaaatca tttggacatt acaagacacc caagaaatta agcagagagt tgtcttcaag 3000
   tactctcaaa tgatcaatat atcagattat attaataggt ggatattcgt gacgatcacg 3060
   aataaccgac ttaataactc caaaatctac ataaatggcc ggctcattga tcagaaacca 3120
   atttccaacc tcggtaatat tcatgcatcc aataacatca tgtttaagtt agacggatgc 3180
   agagatacgc atagatatat ttggattaaa tactttaacc tttttgataa agaacttaat 3240
   gaaaaagaaa taaaggatct gtacgacaac caatctaact caggaatcct taaagatttt 3300
   tggggggact atttgcaata cgacaaaccg tattatatgt taaacctgta tgacccgaat 3360
   aaatatgtag atgtcaacaa tgttggcatc cgtggttata tgtatttaaa aggcccgcgc 3420
   ggctcagtga tgacaactaa tatttatctt aattctagct tatatcgagg aacaaagttt 3480
   attataaaaa agtatgcatc tggcaacaaa gataacattg tccgcaacaa tgatagagtc 3540
   tatattaatg tggtagtcaa aaataaagaa tatcgcttag caacaaatgc gtcacaggct 3600
   ggggtggaaa aaattttaag tgcgctggag attcctgatg ttggaaattt gtctcaagtg 3660
   gttgtgatga aatctaaaaa tgatcagggc attacaaata agtgcaaaat gaacttacaa 3720
   gataataacg gcaatgatat tgggtttatc ggttttcatc agtttaataa tatcgcaaaa 3780
   ctggtggcgt caaattggta taatcgacag attgaacgct cgtcacgcac cctgggatgt 3840
   tcatgggagt ttataccagt cgacgatgga tggggagaga ggccgctcta a          3891
<210> 14
   <211> 3891
   <212> DNA
   <213> Artificial Sequence
<220>
   <223> BoNT/A, B. subtilis-modified 2
<400> 14
   atgccgtttg ttaataaaca atttaattat aaagatccgg ttaatggcgt tgatattgca 60
   tatattaaaa ttccgaatgc aggccaaatg caaccggtta aagcatttaa aattcataat 120
   aaaatttggg ttattccgga aagagataca tttacaaatc cggaagaagg cgatcttaat 180
   ccgccgccgg aagcaaaaca agttccggtt agctattatg atagcacata tcttagcaca 240
   gataatgaaa aagataatta tcttaaaggc gttacaaaac tttttgaaag aatttatagc 300
   acagatcttg gcagaatgct tcttacaagc attgttagag gcattccgtt ttggggcggc 360
   agcacaattg atacagaact taaagttatt gatacaaatt gcattaatgt tattcaaccg 420
   gatggcagct atagaagcga agaacttaat cttgttatta ttggcccgag cgcagatatt 480
   attcaatttg aatgcaaaag ctttggccat gaagttctta atcttacaag aaatggctat 540
   ggcagcacac aatatattag atttagcccg gattttacat ttggctttga agaaagcctt 600
   gaagttgata caaatccgct tcttggcgca ggcaaatttg caacagatcc ggcagttaca 660
   cttgcacatg aacttattca tgcaggccat agactttatg gcattgcaat taatccgaat 720
   agagttttta aagttaatac aaatgcatat tatgaaatga gcggccttga agttagcttt 780
   gaagaactta gaacatttgg cggccatgat gcaaaattta ttgatagcct tcaagaaaat 840
   gaatttagac tttattatta taataaattt aaagatattg caagcacact taataaagca 900
   aaaagcattg ttggcacaac agcaagcctt caatatatga aaaatgtttt taaagaaaaa 960
   tatcttctta gcgaagatac aagcggcaaa tttagcgttg ataaacttaa atttgataaa 1020
   ctttataaaa tgcttacaga aatttataca gaagataatt ttgttaaatt ttttaaagtt 1080
   cttaatagaa aaacatatct taattttgat aaagcagttt ttaaaattaa tattgttccg 1140
   aaagttaatt atacaattta tgatggcttt aatcttagaa atacaaatct tgcagcaaat 1200
   tttaatggcc aaaatacaga aattaataat atgaatttta caaaacttaa aaattttaca 1260
   ggcctttttg aattttataa acttctttgc gttagaggca ttattacaag caaaacaaaa 1320
   agccttgata aaggctataa taaagcactt aatgatcttt gcattaaagt taataattgg 1380
   gatctttttt ttagcccgag cgaagataat tttacaaatg atcttaataa aggcgaagaa 1440
   attacaagcg atacaaatat tgaagcagca gaagaaaata ttagccttga tcttattcaa 1500
   caatattatc ttacatttaa ttttgataat gaaccggaaa atattagcat tgaaaatctt 1560
   agcagcgata ttattggcca acttgaactt atgccgaata ttgaaagatt tccgaatggc 1620
   aaaaaatatg aacttgataa atatacaatg tttcattatc ttagagcaca agaatttgaa 1680
   catggcaaaa gcagaattgc acttacaaat agcgttaatg aagcacttct taatccgagc 1740
   agagtttata cattttttag cagcgattat gttaaaaaag ttaataaagc aacagaagca 1800
   gcaatgtttc ttggctgggt tgaacaactt gtttatgatt ttacagatga aacaagcgaa 1860
   gttagcacaa cagataaaat tgcagatatt acaattatta ttccgtatat tggcccggca 1920
   cttaatattg gcaatatgct ttataaagat gattttgttg gcgcacttat ttttagcggc 1980
   gcagttattc ttcttgaatt tattccggaa attgcaattc cggttcttgg cacatttgca 2040
   cttgttagct atattgcaaa taaagttctt acagttcaaa caattgataa tgcacttagc 2100
   aaaagaaatg aaaaatggga tgaagtttat aaatatattg ttacaaattg gcttgcaaaa 2160
   gttaatacac aaattgatct tattagaaaa aaaatgaaag aagcacttga aaatcaagca 2220
   gaagcaacaa aagcaattat taattatcaa tataatcaat atacagaaga agaaaaaaat 2280
   aatattaatt ttaatattga tgatcttagc agcaaactta atgaaagcat taataaagca 2340
   atgattaata ttaataaatt tcttaatcaa tgcagcgtta gctatcttat gaatagcatg 2400
   attccgtatg gcgttaaaag acttgaagat tttgatgcaa gccttaaaga tgcacttctt 2460
   aaatatattt atgataatag aggcacactt attggccaag ttgatagact taaagataaa 2520
   gttaataata cacttagcac agatattccg tttcaactta gcaaatatgt tgataatcaa 2580
   agacttctta gcacatttac agaatatatt aaaaatatta ttaatacaag cattcttaat 2640
   cttagatatg aaagcaatca tcttattgat cttagcagat atgcaagcaa aattaatatt 2700
   ggcagcaaag ttaattttga tccgattgat aaaaatcaaa ttcaactttt taatcttgaa 2760
   agcagcaaaa ttgaagttat tcttaaaaat gcaattgttt ataatagcat gtatgaaaat 2820
   tttagcacaa gcttttggat tagaattccg aaatatttta atagcattag ccttaataat 2880
   gaatatacaa ttattaattg catggaaaat aatagcggct ggaaagttag ccttaattat 2940
   ggcgaaatta tttggacact tcaagataca caagaaatta aacaaagagt tgtttttaaa 3000
   tatagccaaa tgattaatat tagcgattat attaatagat ggatttttgt tacaattaca 3060
   aataatagac ttaataatag caaaatttat attaatggca gacttattga tcaaaaaccg 3120
   attagcaatc ttggcaatat tcatgcaagc aataatatta tgtttaaact tgatggctgc 3180
   agagatacac atagatatat ttggattaaa tattttaatc tttttgataa agaacttaat 3240
   gaaaaagaaa ttaaagatct ttatgataat caaagcaata gcggcattct taaagatttt 3300
   tggggcgatt atcttcaata tgataaaccg tattatatgc ttaatcttta tgatccgaat 3360
   aaatatgttg atgttaataa tgttggcatt agaggctata tgtatcttaa aggcccgaga 3420
   ggcagcgtta tgacaacaaa tatttatctt aatagcagcc tttatagagg cacaaaattt 3480
   attattaaaa aatatgcaag cggcaataaa gataatattg ttagaaataa tgatagagtt 3540
   tatattaatg ttgttgttaa aaataaagaa tatagacttg caacaaatgc aagccaagca 3600
   ggcgttgaaa aaattcttag cgcacttgaa attccggatg ttggcaatct tagccaagtt 3660
   gttgttatga aaagcaaaaa tgatcaaggc attacaaata aatgcaaaat gaatcttcaa 3720
   gataataatg gcaatgatat tggctttatt ggctttcatc aatttaataa tattgcaaaa 3780
   cttgttgcaa gcaattggta taatagacaa attgaaagaa gcagcagaac acttggctgc 3840
   agctgggaat ttattccggt tgatgatggc tggggcgaaa gaccgcttta a          3891
<210> 15
   <211> 3891
   <212> DNA
   <213> Artificial Sequence
<220>
   <223> BoNT/A, B. subtilis-modified 3
<400> 15
   atgccttttg taaataagca gtttaattat aaggatccgg tgaacggcgt ggatattgct 60
   tatattaaaa ttccgaacgc gggtcaaatg caaccggtaa aagcatttaa aatccataat 120
   aaaatttggg tcattccgga aagagacacc tttacaaatc cggaagaggg ggatctgaac 180
   cctccgccag aagccaaaca ggtgcctgta tcatattacg atagtaccta tttatctacg 240
   gacaatgaaa aagataatta tcttaaaggc gttacaaaac tttttgaacg gatttattcc 300
   acggatctgg ggcgcatgct ccttacatcc attgtcagag gtattccgtt ttggggaggc 360
   tcgacaatcg atactgaact gaaggttatc gacacaaatt gcattaacgt catccagcct 420
   gatggcagct acagaagtga agagcttaat ttggtgatta tcggcccttc agctgatatt 480
   atccaattcg aatgcaaaag cttcgggcat gaagtcttga atcttaccag gaacggctat 540
   ggttctacac agtatattcg ctttagccct gattttacat ttggatttga agagtcgctt 600
   gaagttgaca caaatccgtt actgggcgca ggaaaattcg cgacggatcc ggcggtaacc 660
   cttgcacacg aattaattca tgcgggacat cgcctctacg gaattgctat caaccctaat 720
   agagtgttca aggttaatac gaatgcttac tatgagatga gcggcttgga ggtgagcttc 780
   gaagagttac ggacatttgg cggtcatgat gcgaaattta ttgatagctt gcaagagaac 840
   gaatttaggt tgtattacta taacaaattt aaagacattg catcgacctt aaataaggcg 900
   aaatccatcg tgggaacaac tgcatcgctt cagtacatga aaaacgtttt taaagagaag 960
   tatttattgt cagaggacac ttctggaaaa ttttcagtcg ataaactgaa gttcgataaa 1020
   ttatataaaa tgcttacgga aatttacacg gaggacaatt ttgttaaatt tttcaaagtg 1080
   cttaatcgga aaacatacct caactttgat aaggccgttt ttaaaattaa tatcgttccg 1140
   aaagtcaatt atacaatcta tgatgggttt aatcttagaa acactaacct cgcagccaat 1200
   ttcaacggtc aaaatactga aattaataac atgaacttta cgaaacttaa aaattttact 1260
   ggattgttcg agttctacaa gctgttgtgt gtccgaggaa tcattacaag caagacaaaa 1320
   agtttggata agggatataa caaagctttg aatgatctct gtattaaagt taacaattgg 1380
   gacctgttct tttcgccatc ggaagataat ttcaccaatg atcttaacaa aggcgaagag 1440
   atcacttcag atacaaatat tgaggcagcg gaagagaaca tctctctgga tcttattcag 1500
   caatattacc ttacgttcaa tttcgataat gagccggaga atatttctat cgaaaatttg 1560
   agctcagata ttatcggaca acttgaactg atgcctaata ttgaacgttt ccctaacggt 1620
   aaaaagtacg aactggacaa atacacaatg ttccactacc ttagagctca ggaatttgaa 1680
   cacggcaaat cacgcattgc gctcacaaac agtgtcaacg aagcgttatt gaatccgtcc 1740
   cgcgtgtata cgttctttag ctcagattat gtgaaaaagg tcaacaaagc cacagaagca 1800
   gccatgtttc ttggttgggt ggaacaactc gtctatgact ttacggacga aactagtgaa 1860
   gtgagcacta cggataaaat cgcagatatt acaatcatta tcccatacat cggtccggct 1920
   ttgaatattg gcaacatgct gtataaggat gactttgttg gtgctttgat cttttctggc 1980
   gccgttattc tgttggaatt tattccagaa attgctattc cagtccttgg gacgtttgcc 2040
   ttagttagtt atattgcgaa taaagtactc acggtacaga caattgataa cgcattatcc 2100
   aagagaaacg agaaatggga tgaggtctat aagtatattg taacaaattg gctggcgaaa 2160
   gtcaacactc aaatcgacct catccgtaag aaaatgaaag aagcgcttga gaaccaagcc 2220
   gaggctacaa aagcaatcat taattaccag tacaatcagt ataccgagga agagaaaaat 2280
   aacatcaatt tcaacattga cgatctttcc tctaaactta atgagtctat taataaagcg 2340
   atgattaata tcaataagtt cctgaaccaa tgcagcgtat cttacctgat gaactcaatg 2400
   atcccgtatg gcgttaaaag attagaagac tttgatgcat cactgaaaga cgccctgttg 2460
   aagtatattt acgataacag aggcacatta atcggacagg ttgatagact gaaagataaa 2520
   gtgaataaca cattgtctac cgacattcca tttcaattat ctaaatatgt tgataatcaa 2580
   aggcttttat ctacatttac ggagtatatt aagaatatta tcaatacgtc catcttaaac 2640
   ctgcgttatg aatccaacca tctgattgat ctgtcccgct acgcctctaa gattaacatt 2700
   gggtctaaag tgaatttcga ccctatcgat aaaaatcaaa ttcagctctt taacttggaa 2760
   agttcaaaaa tcgaagttat tcttaaaaat gcaattgtat ataactcaat gtatgaaaac 2820
   tttagcacgt cattttggat tcgtattccg aagtatttta atagcatcag cctcaataac 2880
   gaatacacaa ttatcaactg catggaaaat aactctgggt ggaaagtcag tctgaattat 2940
   ggcgaaatta tctggaccct gcaggacaca caggaaatta agcagcgagt agtgtttaaa 3000
   tactcacaaa tgattaatat cagcgactac atcaaccggt ggatctttgt aaccattaca 3060
   aacaatagat taaacaattc aaaaatctac atcaatggac gtcttattga tcaaaaaccg 3120
   attagtaacc ttggaaacat tcacgcttcg aacaatatta tgttcaaatt agacggatgt 3180
   cgcgatacgc atcggtatat ctggattaaa tattttaatc tgtttgataa agaacttaat 3240
   gaaaaagaaa ttaaagacct ttatgacaat caatccaact caggcattct taaggatttc 3300
   tggggtgatt atttacagta tgataagcct tattacatgc ttaacttata tgatccgaac 3360
   aaatatgtgg atgttaacaa tgtcggcatt cgtggatata tgtatcttaa gggcccgcgc 3420
   gggtcagtaa tgacgactaa catttatctg aacagctctc tttatagggg gacaaagttt 3480
   attatcaaaa agtatgccag cggaaacaaa gacaacatcg tccgtaataa cgatcgagtc 3540
   tatattaatg tggttgtcaa aaacaaggaa taccggctcg ctaccaacgc atcgcaggca 3600
   ggagttgaaa aaattttaag cgctcttgaa attccggacg taggtaattt atctcaagtt 3660
   gtggttatga agtcgaaaaa tgaccaggga attacgaata agtgcaaaat gaatctgcaa 3720
   gacaataacg gaaatgatat tgggttcatc ggctttcatc aatttaataa tattgctaaa 3780
   ctggtagcgt caaattggta caatcgccag atcgaacgga gctcacgaac cttaggttgt 3840
   tcctgggaat ttattccggt ggatgacggc tggggcgaac gtccattata a          3891
<210> 16
   <211> 3891
   <212> DNA
   <213> Artificial Sequence
<220>
   <223> BoNT/A, C. crescentus-modified 1
<400> 16
   atgcctttcg ttaacaagca gtttaactac aaggaccccg tcaacggcgt cgacatcgcc 60
   tatatcaaga tcccgaacgc cggccaaatg cagccggtga aggcgttcaa gattcacaac 120
   aagatttggg tgatacccga gcgcgacacg ttcacaaacc cggaggaagg cgatctgaac 180
   ccaccgcccg aggccaagca ggtgccggtg agctattacg acagcacgta cctgagcacc 240
   gacaacgaga aggacaatta tctgaagggc gtcaccaagc tgttcgaaag aatctacagc 300
   acggacctgg gtcgcatgct gcttaccagc atcgtgcgcg gcatcccgtt ctggggcgga 360
   tccaccatcg acacggagct caaagtcatc gacacgaact gcatcaacgt catccagccg 420
   gacggctcgt atcgctccga agagttgaac ctggtgatca ttggcccctc cgccgacatc 480
   attcagtttg agtgcaagtc gttcggccac gaggtcctga atctcacccg aaatggctat 540
   ggcagcaccc agtacatccg cttctcgccg gacttcacct tcggcttcga ggaatccctg 600
   gaggtcgaca cgaacccgct gctcggcgcc ggcaagttcg ccaccgaccc ggccgtgacc 660
   ctcgcccacg aactgatcca cgcgggccat cgtctgtacg gcatcgccat caaccccaat 720
   cgcgtgttca aggtgaacac caacgcctac tatgaaatgt caggcctcga agtctcgttc 780
   gaggaactgc ggaccttcgg gggccatgat gccaagttca tcgactcgct gcaagagaac 840
   gagttccggc tgtattacta taacaagttc aaggatatcg ccagtaccct gaacaaggcg 900
   aagtcgatcg tcggcaccac ggcgagcctg cagtacatga agaacgtgtt caaggaaaag 960
   tatctgttgt ccgaggacac cagcggaaag ttctccgttg ataagctcaa gtttgacaag 1020
   ctgtacaaga tgttgaccga gatctacacg gaggataact ttgtgaagtt ctttaaagtc 1080
   ctgaatcgta agacgtacct caacttcgac aaggccgtgt tcaagatcaa catcgtgccc 1140
   aaggtcaact atactatcta tgatggcttc aacctgcgaa atacgaacct cgcggccaat 1200
   ttcaacggcc agaataccga gatcaacaat atgaacttca cgaagctgaa aaacttcacc 1260
   gggctgttcg agttctacaa gctgctctgc gttcgcggca tcattacctc gaagacgaaa 1320
   agcctcgaca agggctataa caaagcccta aacgacctgt gcatcaaggt caataactgg 1380
   gacctgttct tttcgccgtc ggaggataac ttcacgaacg acctgaataa gggggaggaa 1440
   atcacctcgg acaccaacat cgaggccgcg gaggaaaaca tctccctgga cctgatccag 1500
   caatactatc tgacgttcaa tttcgacaac gagcccgaga acatctcgat cgagaacctg 1560
   agctcggaca tcattggcca gctggaactg atgcccaaca tcgagcggtt cccgaacggc 1620
   aagaaatacg aactggacaa gtacacgatg ttccactatc tgcgggcgca ggagttcgag 1680
   catggcaagt ctcggatcgc cctgacaaac agcgtcaacg aggccctcct gaatccctcg 1740
   cgcgtgtata ccttctttag ctcagattac gttaagaaag tgaacaaggc caccgaggcc 1800
   gcgatgttct tgggatgggt cgaacagctg gtgtatgact tcacggacga gacctcggag 1860
   gtgtccacga ccgataagat cgccgacatc accatcatta tcccgtatat cggtccggct 1920
   ctgaacatcg gcaacatgct gtataaggat gacttcgtcg gcgctctgat cttctcgggc 1980
   gccgtcatcc tgctcgagtt catcccggag atcgcgatcc ccgtgctggg caccttcgcc 2040
   ctggtctcgt atatcgcgaa caaggtcctc accgtccaga ccatcgacaa tgccctgtcc 2100
   aagcgcaacg agaagtggga cgaagtctat aagtacatcg tcactaactg gctggcgaag 2160
   gttaacaccc agatcgacct gatccgcaag aaaatgaagg aggccttgga gaatcaagcg 2220
   gaagcgacca aggccatcat taactaccag tataatcaat acacggagga agagaagaat 2280
   aacatcaact tcaacatcga cgatctgtcg agcaagctaa acgaatccat caacaaggcc 2340
   atgatcaaca tcaacaagtt cctgaaccag tgctctgtga gctatctgat gaacagcatg 2400
   atcccctacg gggtcaagcg cctggaggac ttcgacgcct cgctcaagga tgccctgttg 2460
   aagtatatct acgacaaccg tggcaccttg atcggccagg tggaccgtct taaggacaag 2520
   gtcaataaca ccctgtctac ggacatcccg ttccagctgt cgaagtacgt cgacaaccag 2580
   cggctgctct cgaccttcac cgagtacatc aagaacatca taaacacctc gatccttaat 2640
   ctgcgctatg aaagcaacca cctcatcgac ctgagccgtt atgcctcgaa gatcaatatc 2700
   gggagcaagg tcaacttcga tcccatcgac aagaaccaga tccagctgtt caacctcgag 2760
   agctcgaaga tcgaggtcat cctgaagaac gcaatcgtct acaactccat gtatgagaac 2820
   ttttcgacca gcttctggat ccgcatcccg aagtacttca actcgatcag cctgaacaat 2880
   gagtatacca tcatcaattg catggagaac aatagcgggt ggaaggtgtc ccttaactac 2940
   ggcgagatca tctggaccct gcaggatacg caggagatca agcagcgcgt cgtgttcaag 3000
   tactcacaga tgatcaacat cagcgactac atcaatcgct ggatcttcgt gaccatcacg 3060
   aacaatcgcc ttaacaattc gaagatctat atcaacggtc gcctgatcga tcagaagccc 3120
   atctcgaacc tgggcaatat ccacgcgtcg aacaacatca tgttcaagct ggacggctgc 3180
   cgcgacacgc accggtatat ctggatcaag tacttcaact tgttcgacaa ggagctgaac 3240
   gaaaaggaga tcaaggacct gtatgacaac cagtccaaca gcggcatcct gaaggacttc 3300
   tggggcgact acctgcagta cgacaagccg tattacatgc tgaacctgta tgacccgaac 3360
   aagtacgtcg atgtgaacaa cgtcggcatc cgcggttata tgtacctgaa gggtccaagg 3420
   ggtagtgtaa tgaccaccaa catctacctg aactcgagcc tttaccgcgg gaccaagttc 3480
   atcatcaaga agtacgcctc gggcaacaag gataacatcg tccgcaacaa cgaccgcgtc 3540
   tacatcaacg tcgtggtcaa gaacaaggag taccgcctgg cgaccaacgc gtcgcaggcg 3600
   ggcgtcgaaa agatcctgtc ggcgctggaa atccctgacg tggggaacct gtcccaggtg 3660
   gtcgtgatga agagcaagaa cgaccagggc atcaccaaca agtgtaagat gaacctccag 3720
   gacaacaacg gcaacgacat cggtttcatc ggcttccatc agttcaacaa catcgccaag 3780
   cttgtggcta gcaactggta taaccgccag atcgagcggt cgtcgcgcac cctgggctgc 3840
   tcgtgggagt tcatcccggt cgacgatggc tggggcgagc ggccgctcta a          3891
<210> 17
   <211> 3891
   <212> DNA
   <213> Artificial Sequence
<220>
   <223> BoNT/A, C. crescentus-modified 2
<400> 17
   atgccgttcg tcaacaagca gttcaactac aaggacccgg tcaacggcgt cgacatcgcc 60
   tacatcaaga tcccgaacgc cggccagatg cagccggtca aggccttcaa gatccacaac 120
   aagatctggg tcatcccgga gcgcgacacc ttcaccaacc cggaggaggg cgacctgaac 180
   ccgccgccgg aggccaagca ggtcccggtc tcgtactacg actcgaccta cctgtcgacc 240
   gacaacgaga aggacaacta cctgaagggc gtcaccaagc tgttcgagcg catctactcg 300
   accgacctgg gccgcatgct gctgacctcg atcgtccgcg gcatcccgtt ctggggcggc 360
   tcgaccatcg acaccgagct gaaggtcatc gacaccaact gcatcaacgt catccagccg 420
   gacggctcgt accgctcgga ggagctgaac ctggtcatca tcggcccgtc ggccgacatc 480
   atccagttcg agtgcaagtc gttcggccac gaggtcctga acctgacccg caacggctac 540
   ggctcgaccc agtacatccg cttctcgccg gacttcacct tcggcttcga ggagtcgctg 600
   gaggtcgaca ccaacccgct gctgggcgcc ggcaagttcg ccaccgaccc ggccgtcacc 660
   ctggcccacg agctgatcca cgccggccac cgcctgtacg gcatcgccat caacccgaac 720
   cgcgtcttca aggtcaacac caacgcctac tacgagatgt cgggcctgga ggtctcgttc 780
   gaggagctgc gcaccttcgg cggccacgac gccaagttca tcgactcgct gcaggagaac 840
   gagttccgcc tgtactacta caacaagttc aaggacatcg cctcgaccct gaacaaggcc 900
   aagtcgatcg tcggcaccac cgcctcgctg cagtacatga agaacgtctt caaggagaag 960
   tacctgctgt cggaggacac ctcgggcaag ttctcggtcg acaagctgaa gttcgacaag 1020
   ctgtacaaga tgctgaccga gatctacacc gaggacaact tcgtcaagtt cttcaaggtc 1080
   ctgaaccgca agacctacct gaacttcgac aaggccgtct tcaagatcaa catcgtcccg 1140
   aaggtcaact acaccatcta cgacggcttc aacctgcgca acaccaacct ggccgccaac 1200
   ttcaacggcc agaacaccga gatcaacaac atgaacttca ccaagctgaa gaacttcacc 1260
   ggcctgttcg agttctacaa gctgctgtgc gtccgcggca tcatcacctc gaagaccaag 1320
   tcgctggaca agggctacaa caaggccctg aacgacctgt gcatcaaggt caacaactgg 1380
   gacctgttct tctcgccgtc ggaggacaac ttcaccaacg acctgaacaa gggcgaggag 1440
   atcacctcgg acaccaacat cgaggccgcc gaggagaaca tctcgctgga cctgatccag 1500
   cagtactacc tgaccttcaa cttcgacaac gagccggaga acatctcgat cgagaacctg 1560
   tcgtcggaca tcatcggcca gctggagctg atgccgaaca tcgagcgctt cccgaacggc 1620
   aagaagtacg agctggacaa gtacaccatg ttccactacc tgcgcgccca ggagttcgag 1680
   cacggcaagt cgcgcatcgc cctgaccaac tcggtcaacg aggccctgct gaacccgtcg 1740
   cgcgtctaca ccttcttctc gtcggactac gtcaagaagg tcaacaaggc caccgaggcc 1800
   gccatgttcc tgggctgggt cgagcagctg gtctacgact tcaccgacga gacctcggag 1860
   gtctcgacca ccgacaagat cgccgacatc accatcatca tcccgtacat cggcccggcc 1920
   ctgaacatcg gcaacatgct gtacaaggac gacttcgtcg gcgccctgat cttctcgggc 1980
   gccgtcatcc tgctggagtt catcccggag atcgccatcc cggtcctggg caccttcgcc 2040
   ctggtctcgt acatcgccaa caaggtcctg accgtccaga ccatcgacaa cgccctgtcg 2100
   aagcgcaacg agaagtggga cgaggtctac aagtacatcg tcaccaactg gctggccaag 2160
   gtcaacaccc agatcgacct gatccgcaag aagatgaagg aggccctgga gaaccaggcc 2220
   gaggccacca aggccatcat caactaccag tacaaccagt acaccgagga ggagaagaac 2280
   aacatcaact tcaacatcga cgacctgtcg tcgaagctga acgagtcgat caacaaggcc 2340
   atgatcaaca tcaacaagtt cctgaaccag tgctcggtct cgtacctgat gaactcgatg 2400
   atcccgtacg gcgtcaagcg cctggaggac ttcgacgcct cgctgaagga cgccctgctg 2460
   aagtacatct acgacaaccg cggcaccctg atcggccagg tcgaccgcct gaaggacaag 2520
   gtcaacaaca ccctgtcgac cgacatcccg ttccagctgt cgaagtacgt cgacaaccag 2580
   cgcctgctgt cgaccttcac cgagtacatc aagaacatca tcaacacctc gatcctgaac 2640
   ctgcgctacg agtcgaacca cctgatcgac ctgtcgcgct acgcctcgaa gatcaacatc 2700
   ggctcgaagg tcaacttcga cccgatcgac aagaaccaga tccagctgtt caacctggag 2760
   tcgtcgaaga tcgaggtcat cctgaagaac gccatcgtct acaactcgat gtacgagaac 2820
   ttctcgacct cgttctggat ccgcatcccg aagtacttca actcgatctc gctgaacaac 2880
   gagtacacca tcatcaactg catggagaac aactcgggct ggaaggtctc gctgaactac 2940
   ggcgagatca tctggaccct gcaggacacc caggagatca agcagcgcgt cgtcttcaag 3000
   tactcgcaga tgatcaacat ctcggactac atcaaccgct ggatcttcgt caccatcacc 3060
   aacaaccgcc tgaacaactc gaagatctac atcaacggcc gcctgatcga ccagaagccg 3120
   atctcgaacc tgggcaacat ccacgcctcg aacaacatca tgttcaagct ggacggctgc 3180
   cgcgacaccc accgctacat ctggatcaag tacttcaacc tgttcgacaa ggagctgaac 3240
   gagaaggaga tcaaggacct gtacgacaac cagtcgaact cgggcatcct gaaggacttc 3300
   tggggcgact acctgcagta cgacaagccg tactacatgc tgaacctgta cgacccgaac 3360
   aagtacgtcg acgtcaacaa cgtcggcatc cgcggctaca tgtacctgaa gggcccgcgc 3420
   ggctcggtca tgaccaccaa catctacctg aactcgtcgc tgtaccgcgg caccaagttc 3480
   atcatcaaga agtacgcctc gggcaacaag gacaacatcg tccgcaacaa cgaccgcgtc 3540
   tacatcaacg tcgtcgtcaa gaacaaggag taccgcctgg ccaccaacgc ctcgcaggcc 3600
   ggcgtcgaga agatcctgtc ggccctggag atcccggacg tcggcaacct gtcgcaggtc 3660
   gtcgtcatga agtcgaagaa cgaccagggc atcaccaaca agtgcaagat gaacctgcag 3720
   gacaacaacg gcaacgacat cggcttcatc ggcttccacc agttcaacaa catcgccaag 3780
   ctggtcgcct cgaactggta caaccgccag atcgagcgct cgtcgcgcac cctgggctgc 3840
   tcgtgggagt tcatcccggt cgacgacggc tggggcgagc gcccgctgta a          3891
<210> 18
   <211> 3891
   <212> DNA
   <213> Artificial Sequence
<220>
   <223> BoNT/A, C. crescentus-modified 3
<400> 18
   atgccgttcg tgaacaagca gttcaactat aaggacccgg tgaacggggt ggacatcgcc 60
   tacatcaaga tcccgaacgc cggccagatg cagccggtca aggcgttcaa gatccataac 120
   aagatctggg tgatcccgga gcgcgacacc ttcaccaacc cggaagaggg cgacctcaac 180
   cccccgcccg aggccaagca ggtcccggtg tcgtattacg actcgaccta tctctccacc 240
   gacaacgaga aggacaacta cctgaagggc gtgaccaagc tgttcgagcg catctactcg 300
   accgacctgg gccgcatgct gctcacgagc atcgtccgcg gcatcccgtt ctggggcggg 360
   tcgacgatcg acaccgaact gaaggtcatc gacacgaact gcatcaacgt gatccagccg 420
   gacgggtcgt atcgctcgga agagctgaac ctcgtcatca tcggcccgtc ggccgacatc 480
   atccagttcg aatgcaagag cttcggccat gaggtcctga acctgacgcg caacggctat 540
   ggcagcaccc agtatatccg cttcagcccg gacttcacct tcggcttcga ggaatccctc 600
   gaggtcgaca ccaaccccct gctcggcgcc ggcaagttcg cgacggaccc ggccgtcacg 660
   ctggcccatg aactgatcca cgcgggccac cgcctgtacg gcatcgcgat caacccgaac 720
   cgcgtcttca aggtcaacac gaacgcctat tacgagatgt cgggcctgga ggtcagcttc 780
   gaggaactgc ggaccttcgg cgggcacgac gcgaagttca tcgacagcct gcaggagaac 840
   gagttccgcc tgtactatta caacaagttc aaggacatcg cctcgaccct gaacaaggcc 900
   aagagcatcg tgggcacgac cgcctcgctg cagtatatga agaacgtgtt caaggagaag 960
   tatctgctct cggaggacac ctcgggcaag ttctccgtcg acaagctgaa gttcgacaag 1020
   ctgtataaga tgctgaccga gatctatacc gaggacaact tcgtgaagtt cttcaaggtc 1080
   ctgaaccgta agacgtatct gaacttcgac aaggcggtct tcaagatcaa catcgtcccg 1140
   aaggtcaact ataccatcta cgacggcttc aacctgcgga acacgaacct ggcggccaac 1200
   ttcaacggcc agaacacgga gatcaacaac atgaacttca ccaagctgaa gaacttcacg 1260
   gggctgttcg aattctacaa gctgctctgc gtccgtggca tcatcacctc gaagaccaag 1320
   tcgctggaca agggctacaa caaggccctg aacgacctgt gcatcaaggt caacaactgg 1380
   gacctgttct tcagcccgag cgaagacaac ttcaccaacg acctgaacaa gggcgaagag 1440
   atcaccagcg acaccaacat cgaggccgcg gaggaaaaca tctcgctgga cctgatccag 1500
   cagtactatc tgaccttcaa cttcgacaac gagccggaga acatcagcat cgaaaacctg 1560
   agctcggaca tcatcggcca gctggagctg atgcccaaca tcgagcggtt ccccaacggc 1620
   aagaagtacg agctggacaa gtataccatg ttccactatc tgcgggccca ggaattcgag 1680
   catggcaaga gccgcatcgc gctgaccaac tcggtgaacg aggccctgct caacccgtcg 1740
   cgcgtgtata ccttcttcag ctccgactat gtcaagaagg tcaacaaggc caccgaggcc 1800
   gcgatgttcc tgggctgggt cgaacagctc gtctacgact tcaccgacga gacgtcggaa 1860
   gtctcgacga ccgacaagat cgccgacatc acgatcatca tcccgtacat cggccccgcg 1920
   ctgaacatcg gcaacatgct gtacaaggac gacttcgtgg gggccctgat cttctcgggc 1980
   gcggtgatcc tgctcgagtt catcccggag atcgcgatcc ccgtcctggg cacgttcgcc 2040
   ctggtctcct atatcgccaa caaggtcctc accgtccaga ccatcgacaa cgccctgtcg 2100
   aagcgcaacg agaagtggga cgaagtgtac aagtacatcg tcacgaactg gctggccaag 2160
   gtcaacaccc agatcgacct gatccggaag aagatgaagg aggccctgga gaaccaggcc 2220
   gaggcgacca aggcgatcat caactaccag tataaccagt atacggagga agagaagaac 2280
   aacatcaact tcaacatcga cgacctgagc tcgaagctga acgagtcgat caacaaggcc 2340
   atgatcaaca tcaacaagtt cctgaaccag tgcagcgtca gctacctgat gaacagcatg 2400
   atcccctacg gcgtgaagcg cctggaggac ttcgacgcct cgctgaagga cgccctgctc 2460
   aagtacatct atgacaaccg cggcacgctg atcggccagg tcgaccgcct gaaggacaag 2520
   gtgaacaaca cgctgtcgac cgacatcccc ttccagctgt ccaagtacgt cgacaaccag 2580
   cgcctgctct ccaccttcac cgaatatatc aagaacatca tcaacacgtc gatcctcaac 2640
   ctccgctacg aaagcaacca cctgatcgac ctgtcgcgtt acgcctcgaa gatcaacatc 2700
   ggctcgaagg tcaacttcga cccgatcgac aagaaccaga tccagctgtt caacctggag 2760
   tccagcaaga tcgaggtgat cctcaagaac gccatcgtgt ataacagcat gtacgagaac 2820
   ttctccacct ccttctggat ccggatcccg aagtacttca actccatctc gctgaacaac 2880
   gagtatacca tcatcaactg catggagaac aactcggggt ggaaggtctc gctgaactat 2940
   ggcgagatca tctggaccct ccaggacacc caggagatca agcagcgcgt ggtcttcaag 3000
   tattcgcaga tgatcaacat ctcggactac atcaaccgtt ggatcttcgt gaccatcacg 3060
   aacaaccgtc tgaacaactc gaagatctac atcaacggcc gcctgatcga ccagaagccg 3120
   atctcgaacc tgggcaacat ccacgcgtcg aacaacatca tgttcaagct ggacggctgc 3180
   cgcgacaccc accgctatat ctggatcaag tatttcaacc tgttcgacaa ggagctgaac 3240
   gaaaaggaga tcaaggacct gtacgacaac cagagcaaca gcggcatcct gaaggacttc 3300
   tggggcgact acctgcagta tgacaagccc tactatatgc tcaacctgta cgaccccaac 3360
   aagtatgtcg acgtcaacaa cgtgggcatc cggggctaca tgtacctgaa gggcccccgc 3420
   ggctcggtga tgaccaccaa catctacctg aactcgtccc tgtaccgcgg caccaagttc 3480
   atcatcaaga agtacgcgag cggcaacaag gacaacatcg tccgcaacaa cgaccgcgtg 3540
   tacatcaacg tggtcgtgaa gaacaaggaa taccgcctgg ccaccaacgc gtcccaggcc 3600
   ggcgtggaaa agatcctgag cgccctggag atccccgacg tcgggaacct gtcccaggtg 3660
   gtcgtgatga agtcgaagaa cgaccagggc atcaccaaca agtgcaagat gaacctgcag 3720
   gacaacaacg gcaacgacat cggcttcatc ggcttccacc agttcaacaa catcgccaag 3780
   ctggtcgcca gcaactggta taaccgccag atcgagcgga gctcgcggac cctgggctgc 3840
   tcgtgggagt tcatcccggt cgacgacggc tggggcgagc gcccgctgta a          3891
<210> 19
   <211> 3891
   <212> DNA
   <213> Artificial Sequence
<220>
   <223> BoNT/A, C. difficile-modified 1
<400> 19
   atgccatttg taaataaaca atttaactat aaggatccag ttaatggagt tgatattgca 60
   tatataaaaa ttccaaatgc tggacagatg caacctgtta aagcttttaa gatacataat 120
   aaaatctggg taataccaga aagagatact tttactaatc ctgaagaggg tgatctaaat 180
   ccacctccag aggccaagca agtaccagtt tcttattacg attctacata cctctcaact 240
   gataatgaaa aagataatta tttaaaagga gtaacaaaac tttttgagcg catatattca 300
   acagatttgg gcagaatgtt actaacttct atcgttagag gaataccatt ttggggggga 360
   tcaacaatag atactgaact aaaagtaatt gataccaatt gtattaacgt aattcaacct 420
   gatggatctt atagatcaga agagttaaac ttagtaatca ttggcccgtc tgcagatata 480
   attcaattcg aatgtaaaag ttttggtcat gaagttctta acttgacacg taacggttat 540
   ggttcaacac aatatatacg ctttagtcca gattttacat ttggatttga agagagtcta 600
   gaggtggaca caaatccact tttaggtgcg ggaaaatttg ctactgatcc agcagtaaca 660
   ttggcacatg agttaataca tgcaggtcac cgtctgtatg gtatagccat aaatcctaat 720
   agagtgttta aagtgaacac aaatgcttat tacgaaatgt ctggacttga agtttctttt 780
   gaagagttaa gaacttttgg agggcatgat gctaaattta ttgatagttt acaagaaaac 840
   gagtttagat tatattacta taataaattt aaagatatag cttctacttt aaataaagct 900
   aaaagtatag taggaacaac tgctagctta caatatatga agaacgtatt caaagaaaaa 960
   tatttactta gtgaagatac tagtggtaag ttttcagtgg ataaattgaa atttgataaa 1020
   ttatataaaa tgcttactga aatttacacc gaagataatt tcgttaaatt tttcaaggtc 1080
   ttgaatagaa aaacttatct taattttgat aaggctgttt ttaaaattaa tatagtacct 1140
   aaagtaaact atactattta tgatggcttc aatttaagaa atacaaattt agctgcaaat 1200
   tttaatggac aaaatacaga aattaacaat atgaatttta ctaaattaaa aaattttaca 1260
   ggattgtttg aattttacaa attactttgt gtgcgtggca ttataacttc taaaacaaaa 1320
   tcattggata aaggttataa taaggcatta aacgatctgt gcataaaagt taataactgg 1380
   gatttatttt tctcaccttc tgaagataat tttactaatg atctaaacaa aggagaagag 1440
   attacgtctg atactaatat agaagctgca gaagagaata ttagtttaga tttgattcaa 1500
   cagtattact taacatttaa tttcgataat gaaccagaaa atatatctat tgaaaatcta 1560
   tcatccgata taatcggtca actagaactt atgcctaata tagaaagatt tcctaatgga 1620
   aaaaagtatg aactggataa atacacaatg tttcattacc taagagctca ggaatttgaa 1680
   catggaaagt ccagaatcgc attaactaat agtgtcaatg aagcattact taatccttca 1740
   agggtgtata ctttctttag ctctgactat gttaaaaagg ttaataaagc tactgaagct 1800
   gcaatgtttt tgggatgggt agaacagtta gtttatgatt ttactgacga aacaagtgaa 1860
   gtttcgacta cagacaaaat agcggatata actattataa tcccttatat agggccagca 1920
   cttaatattg gtaatatgct ttataaggat gactttgttg gtgctttaat attttcaggt 1980
   gcagtaattt tactagaatt tatacccgag attgcaatac cagtattggg tacctttgct 2040
   ttagtatctt atatagcaaa taaagtatta acagtacaaa cgattgataa tgcattaagt 2100
   aaaagaaatg aaaaatggga tgaagtttac aaatatattg taactaattg gttagcaaaa 2160
   gttaatactc aaatagactt aataagaaaa aagatgaaag aagcattaga aaatcaagct 2220
   gaagctacaa aagcaataat taattatcaa tataatcaat atacagaaga ggaaaaaaat 2280
   aacattaatt ttaatataga tgacttatcg tctaaattaa atgaaagtat aaataaagca 2340
   atgataaata taaataagtt tttaaatcaa tgtagtgtaa gttacttgat gaattcaatg 2400
   attccatacg gtgttaaacg actagaagat tttgacgcct ctttgaaaga tgctttacta 2460
   aaatatatat atgacaatag aggaacttta ataggtcaag ttgatagatt aaaagataag 2520
   gtaaataaca cattaagcac tgatatacct ttccaattat ctaaatatgt agataatcaa 2580
   agactgttaa gtacgtttac tgaatatata aagaatatta taaatacttc aatattaaat 2640
   ttacgttatg aatctaatca tcttatagat ttaagcagat atgcttctaa gattaatatt 2700
   ggtagtaaag taaattttga tcctattgat aaaaaccaga tacaactctt taatttagaa 2760
   tcaagtaaaa tcgaagtaat ccttaaaaac gctatagtat ataattctat gtatgaaaat 2820
   ttttctacct cattctggat aagaatacca aaatatttta actctatatc attaaataac 2880
   gaatatacta taattaattg tatggaaaat aactcaggat ggaaggtatc tttaaattat 2940
   ggtgaaataa tttggactct tcaagataca caagagatta aacaaagagt agtttttaaa 3000
   tatagtcaaa tgataaatat atcagattat attaatagat ggatattcgt aacaattact 3060
   aataacagac taaataacag taaaatatat attaatggta gacttattga tcaaaaacca 3120
   atatcaaatc taggaaatat acacgcatcc aataacataa tgttcaaatt agatgggtgt 3180
   agagacacac atcgatatat ttggattaaa tattttaatt tatttgataa agaattaaat 3240
   gaaaaagaaa taaaagattt atatgataat caatcaaatt caggaatttt aaaagatttt 3300
   tggggtgatt atttacaata tgacaagcct tattacatgt taaatttata tgacccaaat 3360
   aaatatgttg atgttaataa cgtaggtata agaggatata tgtatttaaa aggtcctcgt 3420
   ggatcagtta tgacgactaa tatatattta aatagttctt tatatagagg aactaagttt 3480
   ataattaaga aatatgctag cggaaataaa gataatatag ttagaaataa tgatagagtt 3540
   tatataaatg tcgtggtaaa aaataaagaa tatcgacttg caacaaatgc atcacaggca 3600
   ggagttgaaa agatcttatc tgcattagaa atacctgatg ttggaaattt aagtcaagtt 3660
   gtagttatga agtctaaaaa tgaccaagga attacaaata aatgcaaaat gaatttgcag 3720
   gataataatg gaaatgatat tggttttata ggttttcacc aatttaataa tattgctaaa 3780
   cttgtcgcta gtaattggta taatcgtcaa atagagagga gttcaaggac tcttggatgt 3840
   agttgggaat ttattcctgt agatgatggt tggggtgaaa gacctttata a          3891
<210> 20
   <211> 3891
   <212> DNA
   <213> Artificial Sequence
<220>
   <223> BoNT/A, C. difficile-modified 2
<400> 20
   atgccatttg taaataaaca atttaattat aaagatccag taaatggagt agatatagca 60
   tatataaaaa taccaaatgc aggacaaatg caaccagtaa aagcatttaa aatacataat 120
   aaaatatggg taataccaga aagagatact tttactaatc cagaagaagg agatttaaat 180
   ccaccaccag aagcaaaaca agtaccagta tcttattatg attctactta tttatctact 240
   gataatgaaa aagataatta tttaaaagga gtaactaaat tatttgaaag aatatattct 300
   actgatttag gaagaatgtt attaacttct atagtaagag gaataccatt ttggggagga 360
   tctactatag atactgaatt aaaagtaata gatactaatt gtataaatgt aatacaacca 420
   gatggatctt atagatctga agaattaaat ttagtaataa taggaccatc tgcagatata 480
   atacaatttg aatgtaaatc ttttggacat gaagtattaa atttaactag aaatggatat 540
   ggatctactc aatatataag attttctcca gattttactt ttggatttga agaatcttta 600
   gaagtagata ctaatccatt attaggagca ggaaaatttg caactgatcc agcagtaact 660
   ttagcacatg aattaataca tgcaggacat agattatatg gaatagcaat aaatccaaat 720
   agagtattta aagtaaatac taatgcatat tatgaaatgt ctggattaga agtatctttt 780
   gaagaattaa gaacttttgg aggacatgat gcaaaattta tagattcttt acaagaaaat 840
   gaatttagat tatattatta taataaattt aaagatatag catctacttt aaataaagca 900
   aaatctatag taggaactac tgcatcttta caatatatga aaaatgtatt taaagaaaaa 960
   tatttattat ctgaagatac ttctggaaaa ttttctgtag ataaattaaa atttgataaa 1020
   ttatataaaa tgttaactga aatatatact gaagataatt ttgtaaaatt ttttaaagta 1080
   ttaaatagaa aaacttattt aaattttgat aaagcagtat ttaaaataaa tatagtacca 1140
   aaagtaaatt atactatata tgatggattt aatttaagaa atactaattt agcagcaaat 1200
   tttaatggac aaaatactga aataaataat atgaatttta ctaaattaaa aaattttact 1260
   ggattatttg aattttataa attattatgt gtaagaggaa taataacttc taaaactaaa 1320
   tctttagata aaggatataa taaagcatta aatgatttat gtataaaagt aaataattgg 1380
   gatttatttt tttctccatc tgaagataat tttactaatg atttaaataa aggagaagaa 1440
   ataacttctg atactaatat agaagcagca gaagaaaata tatctttaga tttaatacaa 1500
   caatattatt taacttttaa ttttgataat gaaccagaaa atatatctat agaaaattta 1560
   tcttctgata taataggaca attagaatta atgccaaata tagaaagatt tccaaatgga 1620
   aaaaaatatg aattagataa atatactatg tttcattatt taagagcaca agaatttgaa 1680
   catggaaaat ctagaatagc attaactaat tctgtaaatg aagcattatt aaatccatct 1740
   agagtatata cttttttttc ttctgattat gtaaaaaaag taaataaagc aactgaagca 1800
   gcaatgtttt taggatgggt agaacaatta gtatatgatt ttactgatga aacttctgaa 1860
   gtatctacta ctgataaaat agcagatata actataataa taccatatat aggaccagca 1920
   ttaaatatag gaaatatgtt atataaagat gattttgtag gagcattaat attttctgga 1980
   gcagtaatat tattagaatt tataccagaa atagcaatac cagtattagg aacttttgca 2040
   ttagtatctt atatagcaaa taaagtatta actgtacaaa ctatagataa tgcattatct 2100
   aaaagaaatg aaaaatggga tgaagtatat aaatatatag taactaattg gttagcaaaa 2160
   gtaaatactc aaatagattt aataagaaaa aaaatgaaag aagcattaga aaatcaagca 2220
   gaagcaacta aagcaataat aaattatcaa tataatcaat atactgaaga agaaaaaaat 2280
   aatataaatt ttaatataga tgatttatct tctaaattaa atgaatctat aaataaagca 2340
   atgataaata taaataaatt tttaaatcaa tgttctgtat cttatttaat gaattctatg 2400
   ataccatatg gagtaaaaag attagaagat tttgatgcat ctttaaaaga tgcattatta 2460
   aaatatatat atgataatag aggaacttta ataggacaag tagatagatt aaaagataaa 2520
   gtaaataata ctttatctac tgatatacca tttcaattat ctaaatatgt agataatcaa 2580
   agattattat ctacttttac tgaatatata aaaaatataa taaatacttc tatattaaat 2640
   ttaagatatg aatctaatca tttaatagat ttatctagat atgcatctaa aataaatata 2700
   ggatctaaag taaattttga tccaatagat aaaaatcaaa tacaattatt taatttagaa 2760
   tcttctaaaa tagaagtaat attaaaaaat gcaatagtat ataattctat gtatgaaaat 2820
   ttttctactt ctttttggat aagaatacca aaatatttta attctatatc tttaaataat 2880
   gaatatacta taataaattg tatggaaaat aattctggat ggaaagtatc tttaaattat 2940
   ggagaaataa tatggacttt acaagatact caagaaataa aacaaagagt agtatttaaa 3000
   tattctcaaa tgataaatat atctgattat ataaatagat ggatatttgt aactataact 3060
   aataatagat taaataattc taaaatatat ataaatggaa gattaataga tcaaaaacca 3120
   atatctaatt taggaaatat acatgcatct aataatataa tgtttaaatt agatggatgt 3180
   agagatactc atagatatat atggataaaa tattttaatt tatttgataa agaattaaat 3240
   gaaaaagaaa taaaagattt atatgataat caatctaatt ctggaatatt aaaagatttt 3300
   tggggagatt atttacaata tgataaacca tattatatgt taaatttata tgatccaaat 3360
   aaatatgtag atgtaaataa tgtaggaata agaggatata tgtatttaaa aggaccaaga 3420
   ggatctgtaa tgactactaa tatatattta aattcttctt tatatagagg aactaaattt 3480
   ataataaaaa aatatgcatc tggaaataaa gataatatag taagaaataa tgatagagta 3540
   tatataaatg tagtagtaaa aaataaagaa tatagattag caactaatgc atctcaagca 3600
   ggagtagaaa aaatattatc tgcattagaa ataccagatg taggaaattt atctcaagta 3660
   gtagtaatga aatctaaaaa tgatcaagga ataactaata aatgtaaaat gaatttacaa 3720
   gataataatg gaaatgatat aggatttata ggatttcatc aatttaataa tatagcaaaa 3780
   ttagtagcat ctaattggta taatagacaa atagaaagat cttctagaac tttaggatgt 3840
   tcttgggaat ttataccagt agatgatgga tggggagaaa gaccattata a          3891
<210> 21
   <211> 3891
   <212> DNA
   <213> Artificial Sequence
<220>
   <223> BoNT/A, C. difficile-modified 3
<400> 21
   atgccttttg taaataaaca atttaattat aaagatccag taaatggagt agatatagca 60
   tatataaaaa ttcctaatgc aggtcaaatg caaccagtaa aagcatttaa aatacataat 120
   aaaatttggg ttattccaga acgtgatact tttacaaatc ctgaagaagg agatttaaat 180
   cctccacctg aagcaaaaca agtaccagta agttattatg attcaactta tttaagtaca 240
   gataatgaaa aagataatta tttaaaagga gttactaaac tatttgaacg tatatatagt 300
   acagatttag gtagaatgtt attgacttct atagtaagag gaattccatt ttggggaggt 360
   agtacaatag atactgaact aaaagttatt gatacaaatt gtattaatgt aatacaacca 420
   gatggttcat atagatctga agaattaaat ttagttataa ttggaccttc tgcagatata 480
   attcaatttg aatgtaaatc ttttggacat gaagtactaa atttaactag aaatggatat 540
   ggttcaactc aatatattag attttcacca gattttactt ttggttttga agaatcttta 600
   gaagtagata ctaatccatt attgggagct ggtaaatttg caactgatcc tgctgttact 660
   ttagcacatg aattaataca tgctggacac agactttatg gtattgctat taatcctaat 720
   agagtattta aagtaaatac taatgcatat tatgaaatgt ctggattaga agtatctttt 780
   gaagaattaa gaacatttgg tggacatgat gctaaattta tagattcttt acaagaaaat 840
   gaatttagat tatattatta taataaattt aaagatatag catctactct taataaagca 900
   aaatctatag taggaactac agcttcttta caatatatga aaaatgtttt taaagaaaaa 960
   tatttattgt cagaagatac ttcaggtaaa tttagtgttg ataaacttaa atttgataaa 1020
   ttgtataaaa tgttgacaga aatttatact gaagataatt ttgttaaatt ttttaaagta 1080
   ctaaatagaa aaacttattt gaattttgat aaagctgttt ttaaaattaa tatagtacca 1140
   aaagtaaatt atactatata tgatggtttt aatttaagaa atacaaattt agcagctaat 1200
   tttaatggtc aaaatactga aattaataat atgaatttta ctaaactaaa aaattttaca 1260
   ggattatttg aattttataa attactttgt gtaagaggaa ttataactag taaaacaaaa 1320
   tctttagata aaggatataa taaagctttg aatgatttat gtataaaagt taataattgg 1380
   gatttatttt tttctccatc tgaagataat tttacaaatg atttaaataa aggagaagaa 1440
   ataacatctg atacaaatat agaagcagct gaagaaaata taagtcttga tttaattcaa 1500
   caatattatt taacatttaa ttttgataat gaacctgaaa atattagtat tgaaaattta 1560
   tcatctgata taattggaca attagaatta atgccaaata tagaaagatt tcctaatgga 1620
   aaaaaatatg aactagataa atatacaatg tttcattatt tgagagcaca agaatttgaa 1680
   cacggaaaat cacgtattgc tttaacaaat tctgttaatg aagcattact aaatccttct 1740
   agagtttata cttttttttc atctgattat gttaaaaaag taaataaagc aacagaagca 1800
   gctatgtttt taggatgggt tgaacaatta gtttatgatt ttacagatga aacttcagaa 1860
   gttagtacaa ctgataaaat tgctgatata actattataa ttccatatat aggaccagct 1920
   ttaaatatag gaaatatgtt gtataaagat gattttgtag gagcacttat tttttctgga 1980
   gctgttatat tactagaatt tatacctgaa attgctatac ctgtacttgg tacttttgca 2040
   cttgtatctt atatagcaaa taaagtacta actgtacaaa caatagataa tgcattatca 2100
   aaaagaaatg aaaaatggga tgaagtttat aaatatattg ttactaattg gttagcaaaa 2160
   gtaaatactc aaatagattt aattagaaaa aaaatgaaag aagctttaga aaatcaagct 2220
   gaagctacta aagctattat aaattatcaa tataatcaat atactgaaga agaaaaaaat 2280
   aatataaatt ttaatataga tgatctttca agtaaattga atgaaagtat aaataaagca 2340
   atgataaata ttaataaatt tttaaatcaa tgtagtgtat catatcttat gaattctatg 2400
   attccatatg gtgttaaaag attagaagat tttgatgcat cacttaaaga tgctttgctt 2460
   aaatatattt atgataatag aggaacatta attggacaag tagatagatt aaaagataaa 2520
   gtaaataata ctttatctac tgatattcca tttcaattgt caaaatatgt tgataatcaa 2580
   cgtctacttt caacttttac agaatatata aaaaatatta taaatacatc aatactaaat 2640
   ttaagatatg aatctaatca cttaattgat ttatctagat atgcaagtaa aataaatata 2700
   ggaagtaaag taaattttga tccaatagat aaaaatcaaa ttcaattatt taatttagaa 2760
   agttcaaaaa tagaagtaat attaaaaaat gctatagttt ataattctat gtatgaaaat 2820
   ttttctactt cattttggat tagaatacct aaatatttta atagtatttc tcttaataat 2880
   gaatatacta taattaattg tatggaaaat aattctggtt ggaaagttag tttaaattat 2940
   ggtgaaataa tttggacttt acaagataca caagaaataa aacaacgtgt tgtatttaaa 3000
   tattcacaaa tgataaatat atctgattat ataaatagat ggatttttgt aactataact 3060
   aataatagac ttaataatag taaaatatat ataaatggta gacttataga tcaaaaacct 3120
   atttctaatt taggaaatat acatgcttct aataatataa tgtttaaatt ggatggttgt 3180
   agagatacac atagatatat atggataaaa tattttaatt tatttgataa agaacttaat 3240
   gaaaaagaaa ttaaagattt atatgataat caaagtaatt caggaatatt aaaagatttt 3300
   tggggtgatt atttacaata tgataaacct tattatatgc ttaatcttta tgatcctaat 3360
   aaatatgttg atgtaaataa tgtaggtata agaggatata tgtatttaaa aggaccaaga 3420
   ggtagtgtaa tgactacaaa tatatattta aattctagtt tatatagagg aactaaattt 3480
   ataattaaaa aatatgcatc aggtaataaa gataatatag taagaaataa tgatagagta 3540
   tatataaatg tagttgtaaa aaataaagaa tatagattag caactaatgc ttctcaagca 3600
   ggtgtagaaa aaattttatc agcattagaa atacctgatg taggaaatct aagtcaagtt 3660
   gtagttatga aatctaaaaa tgatcaagga ataactaata aatgtaaaat gaatttacaa 3720
   gataataatg gaaatgatat aggttttata ggttttcatc aatttaataa tatagcaaaa 3780
   ttagttgcat caaattggta taatagacaa atagaaagat ctagtcgtac attaggatgt 3840
   agttgggaat ttataccagt agatgatggt tggggagaaa gaccactata a          3891
<210> 22
   <211> 3891
   <212> DNA
   <213> Artificial Sequence
<220>
   <223> BoNT/A, C. perfringens-modified 1
<400> 22
   atgccttttg taaataaaca atttaactat aaagatccag ttaatggagt tgatattgcc 60
   tatataaaaa taccaaatgc agggcaaatg caaccagtaa aagcttttaa gatacacaat 120
   aaaatctggg ttataccaga aagagataca tttacaaacc ctgaagaggg agaccttaac 180
   ccacctccag aagcgaaaca agttccagtt agttattacg attcaaccta tttaagtact 240
   gataatgaaa aagataatta tttaaagggt gttacaaaat tgttcgaaag aatatatagt 300
   acagatttgg gaagaatgtt actaacatct atagttagag gaataccttt ttggggtgga 360
   tcaacaattg atactgaatt aaaggttata gatactaatt gtatcaatgt tatacaacca 420
   gatggaagtt atcgtagtga agagctaaat ttagttatta taggtccatc tgctgatata 480
   attcaatttg aatgtaaatc atttggacat gaagttttaa atttaactag aaacggttat 540
   ggaagtacac aatatattag attctcacct gattttactt ttggattcga agagagtctt 600
   gaagtggata ctaatccact tttaggtgct ggaaaatttg ctacagatcc tgctgtaact 660
   ttagcacatg aacttatcca tgctggacac agattatatg gcattgctat taatccaaat 720
   agagttttca aagtaaacac taatgcttat tacgaaatga gtggattaga ggtatctttc 780
   gaagagctaa gaacattcgg aggtcatgac gctaaattta tagattcact acaagaaaac 840
   gagtttagac tttattacta taataaattt aaagatattg cttcaacact taataaggct 900
   aagagcatag tcggcacaac tgcttcttta caatatatga aaaatgtttt caaagagaaa 960
   taccttttaa gcgaagatac aagcggtaag ttttcagttg ataaattaaa attcgataaa 1020
   ttatataaaa tgcttacaga aatatatact gaagataact ttgtaaagtt tttcaaagtg 1080
   ttaaacagaa agacatatct taattttgat aaagctgttt ttaagatcaa tattgtgcct 1140
   aaggtaaatt atactatata tgatggattc aatttaagaa atactaattt agctgcaaat 1200
   tttaatggtc aaaatacaga gattaataac atgaatttta ctaaattaaa gaattttacg 1260
   ggattatttg aattttataa gttactttgt gttaggggaa ttataacatc taaaacaaaa 1320
   agtttagaca aaggatataa taaagcttta aatgatcttt gcataaaagt taataactgg 1380
   gatttatttt tctctccatc tgaagataat tttacaaatg atttaaataa aggagaagag 1440
   attacatctg atactaatat tgaagcagct gaagagaaca tatctttaga tttaatacaa 1500
   cagtattacc tgacttttaa ttttgataat gagcctgaaa atatttcaat tgaaaattta 1560
   tcatccgata taattggaca attagaatta atgccaaata ttgaaagatt tccgaatggt 1620
   aaaaagtatg aattagataa gtatactatg tttcattatt taagggcaca agagttcgag 1680
   catggaaaat caagaattgc tttaacaaat tctgttaatg aagccctatt aaatccaagt 1740
   agagtttata cttttttcag ttctgattac gttaaaaagg ttaataaagc aactgaagca 1800
   gctatgtttt taggttgggt tgaacagctt gtttatgatt ttacagatga aacaagtgaa 1860
   gtttcaacta ccgacaaaat agctgacatt acaataatta taccatatat aggtccagct 1920
   ttaaacattg gaaatatgtt atataaggac gattttgttg gagctttaat atttagtggg 1980
   gcagttattt tattggaatt tataccagaa atagcaatac cagttttagg aacatttgca 2040
   ttagtatctt acatagcaaa taaggtatta acagttcaaa caatagataa tgctctttct 2100
   aaacgtaatg aaaagtggga tgaagtatat aaatacatag ttactaactg gcttgcaaaa 2160
   gtaaacactc agatagattt aattagaaaa aagatgaagg aagcattaga gaatcaagca 2220
   gaagctacta aagctataat taattatcaa tataatcaat atacagaaga ggaaaaaaac 2280
   aatataaatt ttaatattga tgacttatca agcaaactta atgaatcaat taataaagca 2340
   atgataaata ttaataagtt tcttaatcaa tgtagtgtta gttatttaat gaattcaatg 2400
   ataccatacg gagttaagag actagaagat tttgatgcat ctttaaaaga tgcattacta 2460
   aagtatatat atgataatag aggtactctt ataggacaag tagatagatt aaaagataaa 2520
   gtaaataaca ctttatcaac tgatataccc tttcaattat ctaagtatgt ggataatcaa 2580
   agattattga gtacttttac tgaatacata aaaaacataa ttaatacttc aatattaaat 2640
   cttagatatg aatctaatca tttaatagat ttatcaagat atgcttcaaa aataaatatt 2700
   ggatctaagg taaattttga tcctatagat aagaatcaaa ttcaattatt taatttagaa 2760
   tcaagcaaaa tagaagttat acttaaaaat gcaatagtat acaattccat gtatgaaaat 2820
   ttttccacat cattttggat aagaattcct aaatatttta acagtatttc tctaaataac 2880
   gagtatacta taattaattg tatggaaaat aactctggat ggaaggttag tttaaactat 2940
   ggtgaaataa tctggacctt acaggatact caagaaataa aacaaagagt agtttttaaa 3000
   tattctcaga tgataaatat tagtgattat ataaataggt ggatatttgt aactattact 3060
   aataacagat taaacaattc aaaaatatat ataaatggaa gacttattga tcaaaaacct 3120
   atatcaaatt taggtaatat tcatgctagc aataacataa tgtttaagtt ggatggatgc 3180
   agagacactc atagatatat atggataaag tattttaatt tatttgataa agaattaaat 3240
   gaaaaagaga taaaagatct ttatgacaat caatctaata gtgggatact taaagatttt 3300
   tggggagatt atttacaata tgataaacca tactatatgt taaatttata tgatcctaat 3360
   aaatatgtag atgttaataa tgtaggcata agagggtata tgtatttaaa aggacctaga 3420
   ggatcagtaa tgactacaaa tatatattta aatagctctt tatatagagg tactaaattt 3480
   ataattaaga aatatgcaag tggaaataaa gataatatag taagaaataa tgatagagta 3540
   tatataaatg tagttgtaaa aaataaggaa tatagacttg ccaccaatgc cagtcaagct 3600
   ggtgtagaaa aaatactaag tgctttagaa atacctgatg ttggaaattt aagtcaagta 3660
   gttgtaatga aatcaaaaaa tgatcaaggg ataacaaata aatgtaaaat gaatttacaa 3720
   gataataatg gaaatgacat tggttttata ggttttcatc aatttaataa tatagcaaaa 3780
   ttagtagctt caaattggta taatagacaa atagaaagat caagtagaac tttaggatgt 3840
   tcatgggaat tcattcctgt agatgatgga tggggagaaa ggcctttata a          3891
<210> 23
   <211> 3891
   <212> DNA
   <213> Artificial Sequence
<220>
   <223> BoNT/A, C. perfringens-modified 2
<400> 23
   atgccatttg ttaataaaca atttaattat aaagatccag ttaatggagt tgatatagct 60
   tatataaaaa taccaaatgc tggacaaatg caaccagtta aagcttttaa aatacataat 120
   aaaatatggg ttataccaga aagagatact tttactaatc cagaagaagg agatttaaat 180
   ccaccaccag aagctaaaca agttccagtt tcatattatg attcaactta tttatcaact 240
   gataatgaaa aagataatta tttaaaagga gttactaaat tatttgaaag aatatattca 300
   actgatttag gaagaatgtt attaacttca atagttagag gaataccatt ttggggagga 360
   tcaactatag atactgaatt aaaagttata gatactaatt gtataaatgt tatacaacca 420
   gatggatcat atagatcaga agaattaaat ttagttataa taggaccatc agctgatata 480
   atacaatttg aatgtaaatc atttggacat gaagttttaa atttaactag aaatggatat 540
   ggatcaactc aatatataag attttcacca gattttactt ttggatttga agaatcatta 600
   gaagttgata ctaatccatt attaggagct ggaaaatttg ctactgatcc agctgttact 660
   ttagctcatg aattaataca tgctggacat agattatatg gaatagctat aaatccaaat 720
   agagttttta aagttaatac taatgcttat tatgaaatgt caggattaga agtttcattt 780
   gaagaattaa gaacttttgg aggacatgat gctaaattta tagattcatt acaagaaaat 840
   gaatttagat tatattatta taataaattt aaagatatag cttcaacttt aaataaagct 900
   aaatcaatag ttggaactac tgcttcatta caatatatga aaaatgtttt taaagaaaaa 960
   tatttattat cagaagatac ttcaggaaaa ttttcagttg ataaattaaa atttgataaa 1020
   ttatataaaa tgttaactga aatatatact gaagataatt ttgttaaatt ttttaaagtt 1080
   ttaaatagaa aaacttattt aaattttgat aaagctgttt ttaaaataaa tatagttcca 1140
   aaagttaatt atactatata tgatggattt aatttaagaa atactaattt agctgctaat 1200
   tttaatggac aaaatactga aataaataat atgaatttta ctaaattaaa aaattttact 1260
   ggattatttg aattttataa attattatgt gttagaggaa taataacttc aaaaactaaa 1320
   tcattagata aaggatataa taaagcttta aatgatttat gtataaaagt taataattgg 1380
   gatttatttt tttcaccatc agaagataat tttactaatg atttaaataa aggagaagaa 1440
   ataacttcag atactaatat agaagctgct gaagaaaata tatcattaga tttaatacaa 1500
   caatattatt taacttttaa ttttgataat gaaccagaaa atatatcaat agaaaattta 1560
   tcatcagata taataggaca attagaatta atgccaaata tagaaagatt tccaaatgga 1620
   aaaaaatatg aattagataa atatactatg tttcattatt taagagctca agaatttgaa 1680
   catggaaaat caagaatagc tttaactaat tcagttaatg aagctttatt aaatccatca 1740
   agagtttata cttttttttc atcagattat gttaaaaaag ttaataaagc tactgaagct 1800
   gctatgtttt taggatgggt tgaacaatta gtttatgatt ttactgatga aacttcagaa 1860
   gtttcaacta ctgataaaat agctgatata actataataa taccatatat aggaccagct 1920
   ttaaatatag gaaatatgtt atataaagat gattttgttg gagctttaat attttcagga 1980
   gctgttatat tattagaatt tataccagaa atagctatac cagttttagg aacttttgct 2040
   ttagtttcat atatagctaa taaagtttta actgttcaaa ctatagataa tgctttatca 2100
   aaaagaaatg aaaaatggga tgaagtttat aaatatatag ttactaattg gttagctaaa 2160
   gttaatactc aaatagattt aataagaaaa aaaatgaaag aagctttaga aaatcaagct 2220
   gaagctacta aagctataat aaattatcaa tataatcaat atactgaaga agaaaaaaat 2280
   aatataaatt ttaatataga tgatttatca tcaaaattaa atgaatcaat aaataaagct 2340
   atgataaata taaataaatt tttaaatcaa tgttcagttt catatttaat gaattcaatg 2400
   ataccatatg gagttaaaag attagaagat tttgatgctt cattaaaaga tgctttatta 2460
   aaatatatat atgataatag aggaacttta ataggacaag ttgatagatt aaaagataaa 2520
   gttaataata ctttatcaac tgatatacca tttcaattat caaaatatgt tgataatcaa 2580
   agattattat caacttttac tgaatatata aaaaatataa taaatacttc aatattaaat 2640
   ttaagatatg aatcaaatca tttaatagat ttatcaagat atgcttcaaa aataaatata 2700
   ggatcaaaag ttaattttga tccaatagat aaaaatcaaa tacaattatt taatttagaa 2760
   tcatcaaaaa tagaagttat attaaaaaat gctatagttt ataattcaat gtatgaaaat 2820
   ttttcaactt cattttggat aagaatacca aaatatttta attcaatatc attaaataat 2880
   gaatatacta taataaattg tatggaaaat aattcaggat ggaaagtttc attaaattat 2940
   ggagaaataa tatggacttt acaagatact caagaaataa aacaaagagt tgtttttaaa 3000
   tattcacaaa tgataaatat atcagattat ataaatagat ggatatttgt tactataact 3060
   aataatagat taaataattc aaaaatatat ataaatggaa gattaataga tcaaaaacca 3120
   atatcaaatt taggaaatat acatgcttca aataatataa tgtttaaatt agatggatgt 3180
   agagatactc atagatatat atggataaaa tattttaatt tatttgataa agaattaaat 3240
   gaaaaagaaa taaaagattt atatgataat caatcaaatt caggaatatt aaaagatttt 3300
   tggggagatt atttacaata tgataaacca tattatatgt taaatttata tgatccaaat 3360
   aaatatgttg atgttaataa tgttggaata agaggatata tgtatttaaa aggaccaaga 3420
   ggatcagtta tgactactaa tatatattta aattcatcat tatatagagg aactaaattt 3480
   ataataaaaa aatatgcttc aggaaataaa gataatatag ttagaaataa tgatagagtt 3540
   tatataaatg ttgttgttaa aaataaagaa tatagattag ctactaatgc ttcacaagct 3600
   ggagttgaaa aaatattatc agctttagaa ataccagatg ttggaaattt atcacaagtt 3660
   gttgttatga aatcaaaaaa tgatcaagga ataactaata aatgtaaaat gaatttacaa 3720
   gataataatg gaaatgatat aggatttata ggatttcatc aatttaataa tatagctaaa 3780
   ttagttgctt caaattggta taatagacaa atagaaagat catcaagaac tttaggatgt 3840
   tcatgggaat ttataccagt tgatgatgga tggggagaaa gaccattata a          3891
<210> 24
   <211> 3891
   <212> DNA
   <213> Artificial Sequence
<220>
   <223> BoNT/A, C. perfringens-modified 3
<400> 24
   atgccatttg ttaataagca atttaattat aaagatcctg ttaatggagt tgatattgca 60
   tatattaaga tacctaatgc aggtcaaatg caaccagtta aggcatttaa gattcataat 120
   aaaatatggg taatacctga aagagatact tttactaatc ctgaagaagg tgatttaaat 180
   cctccacctg aagcaaaaca agtaccagta tcttattatg attctactta tcttagtact 240
   gataatgaaa aagataatta tcttaagggt gttacaaaat tatttgaaag aatatatagt 300
   actgatttag gaagaatgtt acttacaagt attgtaagag gtataccttt ttggggaggt 360
   agtacaatag atacagaatt aaaagttata gatacaaatt gtataaatgt tatacaacca 420
   gatggaagtt atagaagtga agaattaaat ttagtaataa ttggtcctag cgcagatata 480
   attcaatttg aatgtaaatc ttttggacat gaagttctaa atttaacaag aaatggatat 540
   ggatctactc aatatattag atttagccct gattttacat ttggttttga agaatcttta 600
   gaagttgata caaatccatt acttggagct ggaaaatttg ctactgatcc tgcagttact 660
   ctagctcatg aactaataca tgctggacat agattatatg gaatagctat aaatccaaat 720
   agggttttta aagtaaatac taatgcttat tatgaaatgt caggattaga agtttctttt 780
   gaagaactta gaacttttgg aggtcatgat gctaagttta ttgatagttt acaagaaaat 840
   gaatttagac tatattatta taataaattt aaagatattg cttcaacatt aaataaagct 900
   aaaagcatag ttggaacaac tgcttcatta caatatatga aaaatgtatt taaggaaaaa 960
   tatttacttt cagaagatac atctggaaaa ttttcagtag ataaattaaa atttgataag 1020
   ttatataaga tgttaacaga aatatatact gaagataatt ttgtaaaatt ttttaaagtt 1080
   ttaaatagaa aaacatattt aaattttgat aaagctgttt ttaaaataaa tatagttcca 1140
   aaagtaaatt atacaatata tgatggtttt aatttaagaa atacaaattt agcagctaat 1200
   tttaatggac aaaatactga aattaataat atgaatttta caaagttaaa aaattttaca 1260
   ggattatttg aattttataa attactatgt gtaagaggta taattacatc aaagactaaa 1320
   tctttagata aaggatataa taaagcttta aatgatttat gtattaaggt taataattgg 1380
   gatttatttt tttcaccatc tgaagataat tttacaaatg atttaaataa aggagaagaa 1440
   ataactagtg atacaaatat tgaagcagct gaagaaaata ttagtttaga tcttatacaa 1500
   caatattatc ttacatttaa ttttgataat gaaccagaaa atatatctat agaaaattta 1560
   agttcagata taattggaca attagaactt atgcctaata tagaaagatt tcctaatggt 1620
   aaaaagtatg aacttgataa atatactatg tttcattatc taagagcaca agaatttgaa 1680
   catggaaaaa gcagaattgc attaactaat tcagttaatg aagctttact aaatccatca 1740
   agagtttata catttttttc atctgattat gtaaaaaagg ttaataaggc tactgaagca 1800
   gctatgtttt taggatgggt agaacaatta gtttatgatt ttacagatga aacatctgaa 1860
   gtatctacaa ctgataaaat agctgatata actattataa ttccatatat aggaccagct 1920
   ttaaatatag gtaatatgtt atataaagat gattttgtag gtgcattaat attttcagga 1980
   gcagttatac ttctagaatt tattccagaa atagctatac cagtattagg aacttttgct 2040
   cttgtttcat atatagctaa taaggtactt acagtacaaa caattgataa tgcattaagc 2100
   aaaaggaatg aaaaatggga tgaagtttat aaatatatag taacaaattg gttagcaaaa 2160
   gttaatactc aaatagattt aataagaaag aaaatgaaag aagctcttga aaatcaagct 2220
   gaagcaacta aggctataat taattatcaa tataatcaat atacagaaga agaaaaaaat 2280
   aatattaatt ttaatataga tgatcttagt agcaaattaa atgaatctat taataaggct 2340
   atgataaata taaataaatt tttaaatcaa tgttcagtaa gttatttaat gaattcaatg 2400
   ataccatatg gagtaaagag acttgaagat tttgatgctt cattaaaaga tgctttactt 2460
   aaatatattt atgataatag aggaacttta attggacaag ttgatagatt aaaagataaa 2520
   gttaataata ctctatcaac agatatacca tttcaattat ctaaatatgt agataatcaa 2580
   agattactta gtacttttac tgaatatata aaaaatataa ttaatactag tatattaaat 2640
   ttaagatatg aaagtaatca tcttatagat ttaagtagat atgcttcaaa gattaatata 2700
   ggaagtaaag ttaattttga tcctatagat aagaatcaaa tacaattatt taatttagaa 2760
   tcaagtaaaa tagaagttat attaaagaat gctatagtat ataattctat gtatgaaaat 2820
   tttagtactt ctttttggat tagaatacca aagtatttta atagtatatc attaaataat 2880
   gaatatacta ttataaattg tatggaaaat aatagtggat ggaaggtatc tttaaattat 2940
   ggagaaataa tttggacttt acaagatact caagaaataa agcaaagagt tgtatttaaa 3000
   tattcacaaa tgataaatat tagcgattat attaatagat ggatttttgt aactataact 3060
   aataatagat taaataattc aaaaatatat attaatggaa gattaataga tcaaaagcca 3120
   atttctaatc ttggaaatat acatgcttct aataatatta tgtttaaatt agatggatgt 3180
   agagatactc atagatatat atggataaaa tattttaatt tatttgataa ggaattaaat 3240
   gaaaaagaaa ttaaagatct ttatgataat caatcaaata gtggtatatt aaaagatttt 3300
   tggggtgatt atttacaata tgataagcct tattatatgt taaatttata tgatcctaat 3360
   aaatatgttg atgttaataa tgttggtata aggggatata tgtatcttaa aggacctaga 3420
   ggatctgtta tgactactaa tatatattta aatagttcat tatatagagg aactaaattt 3480
   attataaaaa agtatgcatc aggaaataaa gataatatag taagaaataa tgatagagta 3540
   tatataaatg tagttgttaa aaataaagaa tatagattag caactaatgc atcacaagca 3600
   ggagttgaaa aaatattatc agctttagaa ataccagatg ttggtaattt atctcaagtt 3660
   gttgttatga aaagtaaaaa tgatcaagga ataactaata aatgtaaaat gaatttacaa 3720
   gataataatg gtaatgatat tggatttata ggatttcatc aatttaataa tatagctaag 3780
   ttagttgctt caaattggta taatagacaa atagaaaggt caagtagaac tttaggatgt 3840
   tcatgggaat ttattccagt tgatgatgga tggggagaaa gaccacttta a          3891
<210> 25
   <211> 3891
   <212> DNA
   <213> Artificial Sequence
<220>
   <223> BoNT/A, L. lactis-modified 1
<400> 25
   atgccatttg tgaataaaca atttaattat aaggatcctg taaatggtgt ggacattgct 60
   tatattaaaa tacctaacgc tggacaaatg caacctgtta aagcgttcaa aatccataat 120
   aaaatttggg ttattccaga acgcgataca tttacaaatc ctgaagaggg agacttaaat 180
   ccacctccag aggctaagca agtaccggtg agttattacg actctactta tctctctaca 240
   gataatgaga aagataatta tctaaagggt gttactaagt tatttgaaag aatttatagt 300
   acagatctag gacgtatgtt acttacaagc attgttcgag gaataccctt ttggggcggt 360
   tctacaattg atactgagtt gaaggtgatt gatacaaatt gtattaacgt tatccaacct 420
   gacggatcat atagatcaga agagttaaat ttagttataa tcggaccaag tgcagatatt 480
   atccaatttg aatgtaaatc ctttggtcat gaagtactta atcttacccg aaatggatat 540
   gggagtacac aatacattcg ttttagccca gattttacat tcggctttga agagtcacta 600
   gaagtagata ctaatcctct cttaggagct gggaagtttg cgacagaccc ggcagtcacg 660
   ctagcacatg aacttattca cgctggacac cgcttatatg gaattgccat caacccaaac 720
   agagttttta aagttaatac taatgcatat tacgaaatgt cgggactcga agtttctttt 780
   gaagagcttc ggactttcgg tgggcatgat gcgaaattta tcgacagctt acaagaaaat 840
   gaattccgat tatattacta taacaaattt aaagatattg cttctacttt aaacaaagca 900
   aaatcaattg ttggaacgac agctagtctt cagtatatga aaaacgtatt caaggaaaaa 960
   tatttacttt ccgaagatac ctctgggaag ttcagtgttg ataaattgaa gttcgacaaa 1020
   ttatataaaa tgttaacgga gatttatact gaggataatt ttgttaaatt tttcaaagta 1080
   ttaaatagaa aaacctattt gaactttgac aaagccgtat ttaaaatcaa tattgttcct 1140
   aaagtgaact atacgatcta tgatgggttt aatttgagaa ataccaatct agctgcaaat 1200
   tttaatggtc aaaacactga aataaacaat atgaatttta caaaacttaa aaacttcact 1260
   ggattatttg aattttataa gttattgtgt gtccgtggta ttatcacttc caaaacaaaa 1320
   agccttgata aaggttacaa taaggccctc aacgacttat gcattaaagt taataactgg 1380
   gacttgttct ttagtccgtc cgaagataat tttacgaatg atttgaataa aggtgaagag 1440
   ataacgagtg atactaatat tgaagccgct gaagagaata ttagtcttga tctaatacaa 1500
   cagtattact taacttttaa ttttgataat gaaccagaaa atatctcaat cgaaaatcta 1560
   tcgtctgaca ttatcggtca actggaactc atgccaaata tagaacgatt tcccaacggc 1620
   aaaaagtacg aattagataa atatactatg tttcattatt tacgtgctca agaatttgaa 1680
   catggaaagt ctcgtattgc ccttacaaat tcggtaaatg aggctcttct gaatccttcg 1740
   agggtctata catttttctc aagcgattat gtaaaaaagg tcaacaaagc tacagaagcc 1800
   gctatgtttt taggttgggt ggaacagctt gtatacgatt tcactgatga aacatcagaa 1860
   gtttctacaa ctgataaaat cgcagatatc acaataatta tcccatatat tggtcccgca 1920
   ttaaatattg gaaatatgtt gtacaaagat gactttgtcg ggcattgatt cttcagtggg 1980
   gcagtcatac tgcttgaatt tattccagaa attgctattc cagtactcgg tacatttgct 2040
   cttgtgagct atatagcaaa taaagtatta acagttcaaa ctattgacaa cgctttatca 2100
   aaacgtaatg aaaaatggga cgaagtttat aaatacattg ttactaattg gttggcaaaa 2160
   gttaatactc aaatagactt gattagaaaa aagatgaaag aagctcttga aaatcaagcg 2220
   gaggctacaa aagctataat taattaccaa tataatcaat ataccgaaga ggaaaaaaat 2280
   aacattaact tcaatatcga tgacttaagt agcaaattaa atgaatcaat taacaaagca 2340
   atgattaata taaataaatt tctgaatcaa tgttctgtta gttatctcat gaactcaatg 2400
   attccttatg gagttaaacg tttggaagat tttgatgctt ctcttaaaga tgcacttctg 2460
   aaatatattt atgataatcg cggaacactt attggtcagg ttgataggct taaagataaa 2520
   gtcaataaca cactttcaac cgacatacct tttcaacttt caaagtatgt tgataatcag 2580
   cggttgttaa gtacctttac agaatatatc aaaaacatta tcaatacaag tattttgaat 2640
   ttgcgttacg aatctaatca tttgattgat ttgtcaagat atgcatcaaa aattaatatt 2700
   ggctctaaag taaatttcga tcctattgat aaaaatcaaa ttcaattatt taatttggaa 2760
   tcaagcaaaa tagaagtgat tctaaaaaat gctattgttt ataatagtat gtatgaaaat 2820
   ttttcaactt cattttggat tcggatacct aagtatttta attcaatttc attaaataac 2880
   gaatatacga ttataaattg tatggaaaac aattctgggt ggaaagtttc tttaaattat 2940
   ggtgaaataa tttggacctt gcaagatacc caagaaataa aacaacgtgt agtttttaaa 3000
   tactctcaga tgattaatat ttctgattac attaatagat ggatttttgt tactattact 3060
   aataacagat tgaacaattc taaaatttat atcaatggac gtttaatcga tcaaaaacca 3120
   attagtaatt taggaaatat tcatgcatct aataacatta tgtttaaatt agacggatgt 3180
   cgtgatactc acagatatat ctggattaag tactttaatc tattcgataa agaattgaat 3240
   gaaaaagaaa ttaaagattt atatgacaat caaagtaatt caggtatttt aaaagatttt 3300
   tggggtgatt atttgcaata tgataagcca tactatatgt tgaatcttta tgatcctaac 3360
   aaatatgtcg atgttaataa cgtcggtatt agaggataca tgtatctcaa aggcccacgc 3420
   ggttcagtta tgacaactaa tatatacctt aattcaagtc tataccgtgg cacaaagttt 3480
   atcattaaaa aatatgcctc aggtaacaaa gataatattg tccgtaataa cgaccgagta 3540
   tatatcaatg ttgtggttaa aaataaagaa tatcgtctgg ccaccaatgc gtctcaagca 3600
   ggagtggaaa aaattttatc tgcacttgaa attccagatg ttggtaatct ttcacaagta 3660
   gtcgttatga aaagtaaaaa tgaccaagga attacgaata aatgcaaaat gaatttacaa 3720
   gataataacg gtaatgatat tggttttatt ggttttcatc agtttaataa tattgctaaa 3780
   ttagttgcat caaattggta taatagacaa attgaacgtt catctcgaac tcttggatgt 3840
   tcgtgggaat tcattccagt cgatgatggc tggggtgaac gacctttgta a          3891
<210> 26
   <211> 3891
   <212> DNA
   <213> Artificial Sequence
<220>
   <223> BoNT/A, L. lactis-modified 2
<400> 26
   atgccatttg ttaataaaca atttaattat aaagatccag ttaatggtgt tgatattgct 60
   tatattaaaa ttccaaatgc tggtcaaatg caaccagtta aagcttttaa aattcataat 120
   aaaatttggg ttattccaga acgtgataca tttacaaatc cagaagaagg tgatttaaat 180
   ccaccaccag aagctaaaca agttccagtt tcatattatg attcaacata tttatcaaca 240
   gataatgaaa aagataatta tttaaaaggt gttacaaaat tatttgaacg tatttattca 300
   acagatttag gtcgtatgtt attaacatca attgttcgtg gtattccatt ttggggtggt 360
   tcaacaattg atacagaatt aaaagttatt gatacaaatt gtattaatgt tattcaacca 420
   gatggttcat atcgttcaga agaattaaat ttagttatta ttggtccatc agctgatatt 480
   attcaatttg aatgtaaatc atttggtcat gaagttttaa atttaacacg taatggttat 540
   ggttcaacac aatatattcg tttttcacca gattttacat ttggttttga agaatcatta 600
   gaagttgata caaatccatt attaggtgct ggtaaatttg ctacagatcc agctgttaca 660
   ttagctcatg aattaattca tgctggtcat cgtttatatg gtattgctat taatccaaat 720
   cgtgttttta aagttaatac aaatgcttat tatgaaatgt caggtttaga agtttcattt 780
   gaagaattac gtacatttgg tggtcatgat gctaaattta ttgattcatt acaagaaaat 840
   gaatttcgtt tatattatta taataaattt aaagatattg cttcaacatt aaataaagct 900
   aaatcaattg ttggtacaac agcttcatta caatatatga aaaatgtttt taaagaaaaa 960
   tatttattat cagaagatac atcaggtaaa ttttcagttg ataaattaaa atttgataaa 1020
   ttatataaaa tgttaacaga aatttataca gaagataatt ttgttaaatt ttttaaagtt 1080
   ttaaatcgta aaacatattt aaattttgat aaagctgttt ttaaaattaa tattgttcca 1140
   aaagttaatt atacaattta tgatggtttt aatttacgta atacaaattt agctgctaat 1200
   tttaatggtc aaaatacaga aattaataat atgaatttta caaaattaaa aaattttaca 1260
   ggtttatttg aattttataa attattatgt gttcgtggta ttattacatc aaaaacaaaa 1320
   tcattagata aaggttataa taaagcttta aatgatttat gtattaaagt taataattgg 1380
   gatttatttt tttcaccatc agaagataat tttacaaatg atttaaataa aggtgaagaa 1440
   attacatcag atacaaatat tgaagctgct gaagaaaata tttcattaga tttaattcaa 1500
   caatattatt taacatttaa ttttgataat gaaccagaaa atatttcaat tgaaaattta 1560
   tcatcagata ttattggtca attagaatta atgccaaata ttgaacgttt tccaaatggt 1620
   aaaaaatatg aattagataa atatacaatg tttcattatt tacgtgctca agaatttgaa 1680
   catggtaaat cacgtattgc tttaacaaat tcagttaatg aagctttatt aaatccatca 1740
   cgtgtttata catttttttc atcagattat gttaaaaaag ttaataaagc tacagaagct 1800
   gctatgtttt taggttgggt tgaacaatta gtttatgatt ttacagatga aacatcagaa 1860
   gtttcaacaa cagataaaat tgctgatatt acaattatta ttccatatat tggtccagct 1920
   ttaaatattg gtaatatgtt atataaagat gattttgttg gtgctttaat tttttcaggt 1980
   gctgttattt tattagaatt tattccagaa attgctattc cagttttagg tacatttgct 2040
   ttagtttcat atattgctaa taaagtttta acagttcaaa caattgataa tgctttatca 2100
   aaacgtaatg aaaaatggga tgaagtttat aaatatattg ttacaaattg gttagctaaa 2160
   gttaatacac aaattgattt aattcgtaaa aaaatgaaag aagctttaga aaatcaagct 2220
   gaagctacaa aagctattat taattatcaa tataatcaat atacagaaga agaaaaaaat 2280
   aatattaatt ttaatattga tgatttatca tcaaaattaa atgaatcaat taataaagct 2340
   atgattaata ttaataaatt tttaaatcaa tgttcagttt catatttaat gaattcaatg 2400
   attccatatg gtgttaaacg tttagaagat tttgatgctt cattaaaaga tgctttatta 2460
   aaatatattt atgataatcg tggtacatta attggtcaag ttgatcgttt aaaagataaa 2520
   gttaataata cattatcaac agatattcca tttcaattat caaaatatgt tgataatcaa 2580
   cgtttattat caacatttac agaatatatt aaaaatatta ttaatacatc aattttaaat 2640
   ttacgttatg aatcaaatca tttaattgat ttatcacgtt atgcttcaaa aattaatatt 2700
   ggttcaaaag ttaattttga tccaattgat aaaaatcaaa ttcaattatt taatttagaa 2760
   tcatcaaaaa ttgaagttat tttaaaaaat gctattgttt ataattcaat gtatgaaaat 2820
   ttttcaacat cattttggat tcgtattcca aaatatttta attcaatttc attaaataat 2880
   gaatatacaa ttattaattg tatggaaaat aattcaggtt ggaaagtttc attaaattat 2940
   ggtgaaatta tttggacatt acaagataca caagaaatta aacaacgtgt tgtttttaaa 3000
   tattcacaaa tgattaatat ttcagattat attaatcgtt ggatttttgt tacaattaca 3060
   aataatcgtt taaataattc aaaaatttat attaatggtc gtttaattga tcaaaaacca 3120
   atttcaaatt taggtaatat tcatgcttca aataatatta tgtttaaatt agatggttgt 3180
   cgtgatacac atcgttatat ttggattaaa tattttaatt tatttgataa agaattaaat 3240
   gaaaaagaaa ttaaagattt atatgataat caatcaaatt caggtatttt aaaagatttt 3300
   tggggtgatt atttacaata tgataaacca tattatatgt taaatttata tgatccaaat 3360
   aaatatgttg atgttaataa tgttggtatt cgtggttata tgtatttaaa aggtccacgt 3420
   ggttcagtta tgacaacaaa tatttattta aattcatcat tatatcgtgg tacaaaattt 3480
   attattaaaa aatatgcttc aggtaataaa gataatattg ttcgtaataa tgatcgtgtt 3540
   tatattaatg ttgttgttaa aaataaagaa tatcgtttag ctacaaatgc ttcacaagct 3600
   ggtgttgaaa aaattttatc agctttagaa attccagatg ttggtaattt atcacaagtt 3660
   gttgttatga aatcaaaaaa tgatcaaggt attacaaata aatgtaaaat gaatttacaa 3720
   gataataatg gtaatgatat tggttttatt ggttttcatc aatttaataa tattgctaaa 3780
   ttagttgctt caaattggta taatcgtcaa attgaacgtt catcacgtac attaggttgt 3840
   tcatgggaat ttattccagt tgatgatggt tggggtgaac gtccattata a          3891
<210> 27
   <211> 3891
   <212> DNA
   <213> Artificial Sequence
<220>
   <223> BoNT/A, L. lactis-modified 3
<400> 27
   atgccatttg tcaataaaca atttaattat aaagatccag taaatggtgt ggatattgca 60
   tatattaaaa tccctaatgc cggtcaaatg caaccagtta aagcttttaa aattcacaat 120
   aaaatctggg ttattccaga acgtgatact tttacaaatc cagaagaagg agacttaaat 180
   cctccacctg aagctaaaca agtgcctgta agttattacg attctacata tttgagtaca 240
   gacaatgaaa aagataatta tcttaaagga gttactaaac tttttgaacg tatttactca 300
   actgatcttg gtcgtatgct attaaccagt attgttcgcg gtattccatt ttggggaggt 360
   tctacaattg atactgaatt aaaagtaatt gatactaatt gtatcaatgt gattcaacct 420
   gacggttcat atagatcaga agaattaaat ttagttatta tcggaccttc tgcagatatt 480
   atccaatttg aatgtaaatc ttttggtcat gaagtactaa atttaactcg taatggttat 540
   ggttcaactc aatatatccg tttttcacca gattttacat ttggttttga agaaagtcta 600
   gaagtagaca caaatccttt attgggtgca ggtaaatttg caacagaccc agcagttacc 660
   ctagctcatg aacttatcca cgctggtcat cgattgtacg gaattgctat taatcctaat 720
   agagttttta aagttaatac taatgcttac tatgaaatgt caggtttaga agttagtttt 780
   gaagaattgc gtacctttgg aggtcatgat gctaaattta ttgattctct tcaagaaaat 840
   gaatttagac tttattacta taataaattt aaagatattg catctacact taataaagct 900
   aaatcaattg ttggtactac agcctcttta caatatatga aaaatgtttt taaagaaaaa 960
   tacctattga gtgaagatac tagcggaaaa ttttctgtag acaaattgaa atttgataaa 1020
   ctttacaaaa tgttaacaga aatttatact gaagacaatt ttgtaaaatt ttttaaagtt 1080
   ttaaatagaa aaacatatct aaattttgac aaagctgtat ttaaaattaa tatcgtgcca 1140
   aaagttaatt atactattta tgacggtttt aatcttagaa ataccaattt agcagctaat 1200
   tttaatggac aaaatacaga aattaataat atgaatttta ctaaattgaa aaattttaca 1260
   ggtctttttg aattttataa attgctttgt gttcgcggaa tcattactag taaaacaaaa 1320
   tctcttgata aaggatataa taaagcttta aatgatttat gtattaaagt gaataattgg 1380
   gatttatttt tttctccttc agaagacaat tttacaaatg acttaaataa aggagaagaa 1440
   attacatctg atacaaatat tgaagcagct gaagaaaata tttctttaga tttaattcaa 1500
   caatattact taacatttaa ttttgataat gaaccagaaa atatttctat tgaaaatttg 1560
   tcaagtgata ttatcggtca attagaatta atgccaaata ttgaaagatt tccaaatggt 1620
   aaaaaatacg aacttgataa atacacaatg tttcattatt tacgtgcaca agaatttgaa 1680
   catggaaaaa gtcgaattgc tttaacaaat agtgtcaatg aagctttgct taatccaagt 1740
   cgtgtgtata cctttttttc ttcagattat gtaaaaaaag tgaataaagc tacagaagca 1800
   gccatgtttc ttggttgggt tgaacaatta gtgtatgatt ttactgatga aacctcagaa 1860
   gtctcaacaa ctgataaaat tgcagatatc acaattatca ttccatatat tggtccagca 1920
   ttaaatattg gaaatatgtt atataaagac gattttgttg gtgctttgat ttttagcggt 1980
   gctgttattt tacttgaatt tattcctgaa attgccattc cagtcttggg aacctttgct 2040
   ttagtaagtt atattgcaaa taaagtgtta acagttcaaa caattgataa tgccttatct 2100
   aaacgaaatg aaaaatggga tgaagtatac aaatacattg ttaccaattg gttggccaaa 2160
   gtaaatacac aaattgactt aattcgcaaa aaaatgaaag aagcattaga aaatcaagcc 2220
   gaagcaacca aagctattat caattatcaa tataatcaat atacagaaga agaaaaaaat 2280
   aatattaatt ttaatattga tgacttaagc tcaaaattga atgaatcaat taataaagct 2340
   atgattaata ttaataaatt tcttaatcaa tgttcagttt catacttaat gaattcaatg 2400
   attccttacg gtgttaaacg tcttgaagat tttgacgcat cattaaaaga tgctttattg 2460
   aaatatattt atgataatcg cggaacactt attggtcaag tcgatagact taaagacaaa 2520
   gtcaataata ctttgtctac cgacatccca tttcaattat caaaatatgt tgataatcaa 2580
   agattacttt caacttttac agaatacatt aaaaatatta tcaatacttc tatcttaaat 2640
   ttacgttatg aatcaaatca tcttattgac ctttctcgat atgcttctaa aattaatatt 2700
   ggatcaaaag ttaattttga tcctattgat aaaaatcaaa ttcaattgtt taatttagaa 2760
   agttctaaaa ttgaagtaat tttaaaaaat gcaattgtct ataattcaat gtatgaaaat 2820
   ttttcaactt cattttggat tcgaatccca aaatatttta attctatctc attaaataat 2880
   gaatatacaa tcattaattg tatggaaaat aatagtggat ggaaagtctc acttaattat 2940
   ggtgaaatta tctggacttt gcaagataca caagaaatca aacaacgtgt tgtgtttaaa 3000
   tatagccaaa tgattaatat tagtgattat attaatcgtt ggatttttgt tacaattact 3060
   aataatagat tgaataatag taaaatttat attaatggac gactaattga tcaaaaacct 3120
   atttcaaatt tgggtaatat tcatgcaagc aataatatca tgtttaaact tgatggttgt 3180
   agagatacac accgatatat ttggatcaaa tactttaatt tatttgacaa agaactaaat 3240
   gaaaaagaaa ttaaagactt atatgataat caatcaaatt ctggtatctt aaaagatttt 3300
   tggggtgatt atcttcaata tgataaacca tattacatgt tgaatttata tgatcctaat 3360
   aaatatgttg atgtaaataa tgttggtatt agaggttata tgtacttgaa aggaccacgt 3420
   ggttcagtta tgacaactaa tatttattta aattcaagtt tatatcgtgg tactaaattt 3480
   attattaaaa aatatgctag cggaaataaa gataatattg tccgtaataa tgaccgtgtc 3540
   tatattaatg ttgtcgttaa aaataaagaa taccgtctag ctacaaatgc tagccaagcc 3600
   ggtgttgaaa aaattttatc agcattagaa attccagatg taggtaattt gagtcaagtt 3660
   gtcgttatga aaagcaaaaa tgatcaagga attactaata aatgtaaaat gaatcttcaa 3720
   gataataatg gtaatgatat tggttttatt ggatttcatc aatttaataa tattgctaaa 3780
   ctagtagctt caaattggta taatcgtcaa attgaaagat caagtcgtac attaggatgt 3840
   tcatgggaat ttattcctgt tgatgatggt tggggagaac gtccattgta a 3891
<210> 28
   <211> 3891
   <212> DNA
   <213> Artificial Sequence
<220>
   <223> BoNT/A, M. extorquens-modified 1
<400> 28
<210> 29
   <211> 3891
   <212> DNA
   <213> Artificial Sequence
<220>
   <223> BoNT/A, M. extorquens-modified 2
<400> 29
   atgccgttcg tcaacaagca gttcaactac aaggacccgg tcaacggcgt cgacatcgcc 60
   tacatcaaga tcccgaacgc cggccagatg cagccggtca aggccttcaa gatccacaac 120
   aagatctggg tcatcccgga gcgcgacacc ttcaccaacc cggaggaggg cgacctcaac 180
   ccgccgccgg aggccaagca ggtcccggtc tcgtactacg actcgaccta cctctcgacc 240
   gacaacgaga aggacaacta cctcaagggc gtcaccaagc tcttcgagcg catctactcg 300
   accgacctcg gccgcatgct cctcacctcg atcgtccgcg gcatcccgtt ctggggcggc 360
   tcgaccatcg acaccgagct caaggtcatc gacaccaact gcatcaacgt catccagccg 420
   gacggctcgt accgctcgga ggagctcaac ctcgtcatca tcggcccgtc ggccgacatc 480
   atccagttcg agtgcaagtc gttcggccac gaggtcctca acctcacccg caacggctac 540
   ggctcgaccc agtacatccg cttctcgccg gacttcacct tcggcttcga ggagtcgctc 600
   gaggtcgaca ccaacccgct cctcggcgcc ggcaagttcg ccaccgaccc ggccgtcacc 660
   ctcgcccacg agctcatcca cgccggccac cgcctctacg gcatcgccat caacccgaac 720
   cgcgtcttca aggtcaacac caacgcctac tacgagatgt cgggcctcga ggtctcgttc 780
   gaggagctcc gcaccttcgg cggccacgac gccaagttca tcgactcgct ccaggagaac 840
   gagttccgcc tctactacta caacaagttc aaggacatcg cctcgaccct caacaaggcc 900
   aagtcgatcg tcggcaccac cgcctcgctc cagtacatga agaacgtctt caaggagaag 960
   tacctcctct cggaggacac ctcgggcaag ttctcggtcg acaagctcaa gttcgacaag 1020
   ctctacaaga tgctcaccga gatctacacc gaggacaact tcgtcaagtt cttcaaggtc 1080
   ctcaaccgca agacctacct caacttcgac aaggccgtct tcaagatcaa catcgtcccg 1140
   aaggtcaact acaccatcta cgacggcttc aacctccgca acaccaacct cgccgccaac 1200
   ttcaacggcc agaacaccga gatcaacaac atgaacttca ccaagctcaa gaacttcacc 1260
   ggcctcttcg agttctacaa gctcctctgc gtccgcggca tcatcacctc gaagaccaag 1320
   tcgctcgaca agggctacaa caaggccctc aacgacctct gcatcaaggt caacaactgg 1380
   gacctcttct tctcgccgtc ggaggacaac ttcaccaacg acctcaacaa gggcgaggag 1440
   atcacctcgg acaccaacat cgaggccgcc gaggagaaca tctcgctcga cctcatccag 1500
   cagtactacc tcaccttcaa cttcgacaac gagccggaga acatctcgat cgagaacctc 1560
   tcgtcggaca tcatcggcca gctcgagctc atgccgaaca tcgagcgctt cccgaacggc 1620
   aagaagtacg agctcgacaa gtacaccatg ttccactacc tccgcgccca ggagttcgag 1680
   cacggcaagt cgcgcatcgc cctcaccaac tcggtcaacg aggccctcct caacccgtcg 1740
   cgcgtctaca ccttcttctc gtcggactac gtcaagaagg tcaacaaggc caccgaggcc 1800
   gccatgttcc tcggctgggt cgagcagctc gtctacgact tcaccgacga gacctcggag 1860
   gtctcgacca ccgacaagat cgccgacatc accatcatca tcccgtacat cggcccggcc 1920
   ctcaacatcg gcaacatgct ctacaaggac gacttcgtcg gcgccctcat cttctcgggc 1980
   gccgtcatcc tcctcgagtt catcccggag atcgccatcc cggtcctcgg caccttcgcc 2040
   ctcgtctcgt acatcgccaa caaggtcctc accgtccaga ccatcgacaa cgccctctcg 2100
   aagcgcaacg agaagtggga cgaggtctac aagtacatcg tcaccaactg gctcgccaag 2160
   gtcaacaccc agatcgacct catccgcaag aagatgaagg aggccctcga gaaccaggcc 2220
   gaggccacca aggccatcat caactaccag tacaaccagt acaccgagga ggagaagaac 2280
   aacatcaact tcaacatcga cgacctctcg tcgaagctca acgagtcgat caacaaggcc 2340
   atgatcaaca tcaacaagtt cctcaaccag tgctcggtct cgtacctcat gaactcgatg 2400
   atcccgtacg gcgtcaagcg cctcgaggac ttcgacgcct cgctcaagga cgccctcctc 2460
   aagtacatct acgacaaccg cggcaccctc atcggccagg tcgaccgcct caaggacaag 2520
   gtcaacaaca ccctctcgac cgacatcccg ttccagctct cgaagtacgt cgacaaccag 2580
   cgcctcctct cgaccttcac cgagtacatc aagaacatca tcaacacctc gatcctcaac 2640
   ctccgctacg agtcgaacca cctcatcgac ctctcgcgct acgcctcgaa gatcaacatc 2700
   ggctcgaagg tcaacttcga cccgatcgac aagaaccaga tccagctctt caacctcgag 2760
   tcgtcgaaga tcgaggtcat cctcaagaac gccatcgtct acaactcgat gtacgagaac 2820
   ttctcgacct cgttctggat ccgcatcccg aagtacttca actcgatctc gctcaacaac 2880
   gagtacacca tcatcaactg catggagaac aactcgggct ggaaggtctc gctcaactac 2940
   ggcgagatca tctggaccct ccaggacacc caggagatca agcagcgcgt cgtcttcaag 3000
   tactcgcaga tgatcaacat ctcggactac atcaaccgct ggatcttcgt caccatcacc 3060
   aacaaccgcc tcaacaactc gaagatctac atcaacggcc gcctcatcga ccagaagccg 3120
   atctcgaacc tcggcaacat ccacgcctcg aacaacatca tgttcaagct cgacggctgc 3180
   cgcgacaccc accgctacat ctggatcaag tacttcaacc tcttcgacaa ggagctcaac 3240
   gagaaggaga tcaaggacct ctacgacaac cagtcgaact cgggcatcct caaggacttc 3300
   tggggcgact acctccagta cgacaagccg tactacatgc tcaacctcta cgacccgaac 3360
   aagtacgtcg acgtcaacaa cgtcggcatc cgcggctaca tgtacctcaa gggcccgcgc 3420
   ggctcggtca tgaccaccaa catctacctc aactcgtcgc tctaccgcgg caccaagttc 3480
   atcatcaaga agtacgcctc gggcaacaag gacaacatcg tccgcaacaa cgaccgcgtc 3540
   tacatcaacg tcgtcgtcaa gaacaaggag taccgcctcg ccaccaacgc ctcgcaggcc 3600
   ggcgtcgaga agatcctctc ggccctcgag atcccggacg tcggcaacct ctcgcaggtc 3660
   gtcgtcatga agtcgaagaa cgaccagggc atcaccaaca agtgcaagat gaacctccag 3720
   gacaacaacg gcaacgacat cggcttcatc ggcttccacc agttcaacaa catcgccaag 3780
   ctcgtcgcct cgaactggta caaccgccag atcgagcgct cgtcgcgcac cctcggctgc 3840
   tcgtgggagt tcatcccggt cgacgacggc tggggcgagc gcccgctcta a          3891
<210> 30
   <211> 3891
   <212> DNA
   <213> Artificial Sequence
<220>
   <223> BoNT/A, M. extorquens-modified 3
<400> 30
   atgccgttcg tcaacaagca gttcaactac aaggatcccg tgaacggcgt cgacatcgcc 60
   tacatcaaga tcccgaacgc cggccagatg cagcccgtca aggccttcaa gatccataac 120
   aagatctggg tgatcccgga gcgcgatacc ttcacgaacc cggaggaggg cgacctcaac 180
   ccgcccccgg aggcgaagca ggtgcccgtg tcctactacg actcgaccta cctctccacg 240
   gacaacgaga aggataacta cctcaagggc gtcaccaagc tgttcgagcg catctactcg 300
   accgacctgg gccgcatgct cctgacgtcg atcgtccggg gcatcccgtt ctggggcggc 360
   tccaccatcg acaccgagct gaaggtcatc gataccaact gcatcaacgt catccagccg 420
   gacggctcct accgctcgga ggagctcaac ctcgtcatca tcggcccctc cgccgacatc 480
   atccagttcg agtgcaagtc cttcggccac gaggtgctca acctgacccg caacggctac 540
   ggctcgaccc agtacatccg gttctcgccg gacttcacgt tcggcttcga ggagagcctc 600
   gaggtcgaca ccaacccgct cctgggcgcc ggcaagttcg cgaccgatcc cgccgtcacc 660
   ctcgcgcacg agctcatcca cgccggccac cggctgtacg gcatcgccat caacccgaac 720
   cgcgtgttca aggtgaacac gaacgcctac tacgagatgt cgggcctgga ggtcagcttc 780
   gaggagctgc gcaccttcgg cggccatgac gccaagttca tcgacagcct ccaggagaac 840
   gagttccggc tgtactacta caacaagttc aaggacatcg cgtccacgct gaacaaggcc 900
   aagagcatcg tgggcacgac cgcgtcgctc cagtacatga agaacgtgtt caaggagaag 960
   tacctcctgt cggaggatac ctcgggcaag ttctcggtgg ataagctcaa gttcgacaag 1020
   ctgtacaaga tgctcacgga gatctacacc gaggacaact tcgtgaagtt cttcaaggtg 1080
   ctgaaccgca agacctacct gaacttcgat aaggccgtct tcaagatcaa catcgtcccg 1140
   aaggtcaact acaccatcta cgacggcttc aacctccgga acaccaacct cgcggccaac 1200
   ttcaacggcc agaacacgga gatcaacaac atgaacttca ccaagctgaa gaacttcacc 1260
   ggcctcttcg agttctacaa gctcctgtgc gtccgcggca tcatcacctc gaagaccaag 1320
   agcctcgaca agggctacaa caaggccctc aacgacctct gcatcaaggt caacaactgg 1380
   gatctcttct tctcgccgtc ggaggataac ttcaccaacg acctcaacaa gggcgaggag 1440
   atcaccagcg acacgaacat cgaggcggcc gaggagaaca tctccctgga cctcatccag 1500
   cagtactacc tcacgttcaa cttcgataac gagccggaga acatctcgat cgagaacctc 1560
   tcgagcgaca tcatcggcca gctcgagctc atgcccaaca tcgagcgctt ccccaacggc 1620
   aagaagtacg agctggacaa gtacaccatg ttccactacc tccgcgccca ggagttcgag 1680
   catggcaagt cccggatcgc gctgacgaac agcgtcaacg aggcgctgct caacccctcg 1740
   cgcgtgtaca cgttcttctc gagcgactac gtgaagaagg tgaacaaggc cacggaggcg 1800
   gccatgttcc tcggctgggt ggagcagctg gtctacgact tcaccgacga gacctcggag 1860
   gtctccacca cggacaagat cgcggacatc accatcatca tcccgtacat cggcccggcc 1920
   ctcaacatcg gcaacatgct ctacaaggac gatttcgtcg gcgcgctcat cttcagcggc 1980
   gccgtgatcc tgctcgagtt catccccgag atcgccatcc cggtcctggg caccttcgcg 2040
   ctggtcagct acatcgccaa caaggtcctg accgtgcaga ccatcgacaa cgccctctcg 2100
   aagcgcaacg agaagtggga tgaggtgtac aagtacatcg tcaccaactg gctcgccaag 2160
   gtcaacacgc agatcgacct gatccggaag aagatgaagg aggccctgga gaaccaggcc 2220
   gaggcgacga aggccatcat caactaccag tacaaccagt acaccgagga ggagaagaac 2280
   aacatcaact tcaacatcga cgatctctcc tcgaagctca acgagtcgat caacaaggcg 2340
   atgatcaaca tcaacaagtt cctgaaccag tgctccgtca gctacctcat gaactcgatg 2400
   atcccgtacg gcgtcaagcg cctggaggac ttcgacgcgt cgctcaagga cgcgctcctg 2460
   aagtacatct acgacaaccg cggcaccctg atcggccagg tcgaccgcct caaggataag 2520
   gtgaacaaca ccctctccac ggacatcccg ttccagctca gcaagtacgt cgacaaccag 2580
   cgcctgctca gcaccttcac cgagtacatc aagaacatca tcaacaccag catcctgaac 2640
   ctgcgctacg agtcgaacca cctcatcgat ctcagccggt acgcctcgaa gatcaacatc 2700
   ggctcgaagg tcaacttcga tccgatcgac aagaaccaga tccagctctt caacctggag 2760
   agctcgaaga tcgaggtgat cctgaagaac gcgatcgtct acaactcgat gtacgagaac 2820
   ttcagcacgt ccttctggat ccggatcccg aagtacttca actccatcag cctgaacaac 2880
   gagtacacga tcatcaactg catggagaac aactccggct ggaaggtctc cctgaactac 2940
   ggcgagatca tctggacgct ccaggacacc caggagatca agcagcgcgt cgtgttcaag 3000
   tacagccaga tgatcaacat ctcggattac atcaaccgct ggatcttcgt gacgatcacc 3060
   aacaaccgcc tgaacaacag caagatctac atcaacggcc gcctgatcga tcagaagccc 3120
   atctcgaacc tcggcaacat ccacgcctcc aacaacatca tgttcaagct cgacggctgc 3180
   cgggataccc accggtacat ctggatcaag tacttcaacc tcttcgacaa ggagctgaac 3240
   gagaaggaga tcaaggatct ctacgacaac cagtcgaact cgggcatcct gaaggacttc 3300
   tggggcgact acctccagta cgacaagccg tactacatgc tcaacctcta cgacccgaac 3360
   aagtacgtcg acgtgaacaa cgtcggcatc cgcggctaca tgtacctgaa gggcccccgc 3420
   ggctccgtga tgaccacgaa catctacctc aactcgtccc tctaccgcgg caccaagttc 3480
   atcatcaaga agtacgcctc cggcaacaag gacaacatcg tccgcaacaa cgaccgcgtg 3540
   tacatcaacg tggtcgtgaa gaacaaggag taccgcctcg cgaccaacgc ctcgcaggcc 3600
   ggcgtggaga agatcctgtc ggccctggag atcccggacg tcggcaacct gtcgcaggtg 3660
   gtcgtgatga agagcaagaa cgaccagggc atcaccaaca agtgcaagat gaacctccag 3720
   gacaacaacg gcaacgacat cggcttcatc ggcttccacc agttcaacaa catcgccaag 3780
   ctcgtggcgt cgaactggta caaccgccag atcgagcgct cctcgcgcac cctgggctgc 3840
   tcgtgggagt tcatcccggt ggacgatggc tggggcgagc gccccctgta a          3891
<210> 31
   <211> 3891
   <212> DNA
   <213> Artificial Sequence
<220>
   <223> BoNT/A, S. typhimurium-modified 1
<400> 31
   atgccgtttg tgaacaaaca gtttaactat aaggaccccg tcaatggggt agatatcgcg 60
   tatatcaaaa tcccaaacgc cggacagatg cagccggtca aggcctttaa aattcacaat 120
   aaaatctggg tgatcccgga aagagacact tttacgaatc cggaagaggg cgatttgaac 180
   ccacccccgg aggctaaaca ggtgccagta tcgtattacg acagtaccta cttgagcacc 240
   gacaacgaaa aagataacta tctgaagggt gttacaaaat tatttgagcg tatctattct 300
   acggatctgg gccgtatgct gcttaccagc atcgtgcgtg gtatcccgtt ctggggcggt 360
   agcaccatcg acaccgaatt gaaggtaatc gacacgaatt gcattaacgt gattcagccg 420
   gatggaagct accgctctga agagttaaat ctagtcatca ttggccccag tgcggacatc 480
   attcagtttg aatgcaaatc gttcgggcac gaagttctga atctcaccag gaatgggtat 540
   ggctctacgc aatatattcg ttttagcccg gatttcacgt tcggctttga ggaatccctt 600
   gaggtggata cgaatcccct gctaggcgcg ggcaaatttg cgacggatcc ggccgtcacg 660
   ctcgcccatg aacttatcca tgctggccac cggttgtacg gcattgcgat taatccgaac 720
   cgtgtgttta aggttaatac gaatgcgtac tatgaaatga gcggcttgga ggtaagcttc 780
   gaagagctgc gcacattcgg gggtcacgat gcgaagttta ttgactctct gcaggaaaac 840
   gagtttcgct tatactatta caacaaattc aaagatatcg cgtcaaccct taacaaggcc 900
   aaaagtattg ttggaacaac ggcatcactg cagtacatga aaaacgtgtt caaagagaaa 960
   tacctgttga gtgaagacac cagcggtaaa ttctccgtgg ataaactaaa atttgataaa 1020
   ttgtacaaaa tgctgaccga gatctataca gaggataatt ttgtaaagtt tttcaaggtg 1080
   cttaatcgca aaacctacct gaatttcgat aaagccgtgt ttaagatcaa tatagttccg 1140
   aaagtgaact ataccattta tgacggcttt aacctgcgga atacaaacct ggccgcgaat 1200
   tttaacggcc agaataccga gatcaataac atgaacttta cgaaactcaa aaattttaca 1260
   ggtctgtttg aattttataa gctgttgtgc gttcgtggca tcataacgag caaaacgaaa 1320
   tcgctcgata aagggtataa taaagctctg aacgatctgt gtattaaagt gaataactgg 1380
   gacttatttt tctcccccag cgaggacaat tttaccaatg atctgaataa aggcgaagag 1440
   ataacgtcgg ataccaatat tgaagcggct gaagagaata tctcgttaga cctgatccaa 1500
   cagtactatc tcacatttaa tttcgacaat gaaccggaaa acatctcgat cgaaaacctg 1560
   agctcggata ttatcgggca gctagaatta atgccgaaca ttgagcggtt ccctaatggg 1620
   aagaaatatg agcttgacaa atatactatg tttcattatc tgcgcgcgca agaatttgaa 1680
   catggtaaat cgcgtattgc actgacgaac agcgtgaatg aagcgctgct caacccgagc 1740
   cgcgtttata cctttttcag ctccgattac gttaagaaag tgaacaaagc tacggaggcg 1800
   gccatgttcc ttggctgggt cgagcagtta gtgtatgact tcaccgatga aacctcagaa 1860
   gtcagcacca ctgacaaaat tgcggacatt acgatcataa tcccttacat tggaccggcg 1920
   ctgaacattg gtaatatgct gtataaagat gactttgtag gcgccctgat cttttccggt 1980
   gcagtaattc tactggaatt catccccgaa atagctattc cggtgttagg cacattcgcc 2040
   ctggtctcgt acattgcgaa taaagtactg acggttcaga ccattgataa cgcgttatcg 2100
   aaacgcaacg aaaaatggga tgaggtgtac aagtacatcg taaccaactg gctcgccaag 2160
   gttaatactc agattgatct gattcgcaaa aagatgaagg aagcgcttga aaatcaggca 2220
   gaagcgacta aagcgatcat taactatcag tacaaccagt atacggaaga ggaaaaaaac 2280
   aatatcaact ttaacatcga cgatctcagc tcaaaattaa atgaatcgat taataaagcg 2340
   atgattaata taaataaatt cctgaatcag tgctcagttt cctatctgat gaacagtatg 2400
   atcccgtacg gtgttaaacg cttggaggat ttcgatgcat cactgaagga cgccctttta 2460
   aaatatatct atgacaatcg gggcaccctg atcggtcagg tcgaccgcct taaagataaa 2520
   gtgaataaca ccctgtccac ggacattccg ttccaactgt ccaaatacgt ggataatcaa 2580
   cgtctcttgt ccacctttac tgaatacatt aaaaacatta tcaacacgag tattctgaac 2640
   ctgcgatatg aatcgaacca tctgatcgac ctgagtcgct atgcgagcaa aattaatatt 2700
   gggagtaaag ttaactttga tccaattgac aaaaaccaga tccagctgtt taacttggaa 2760
   agttctaaga tcgaagtaat tctgaaaaac gccatagtgt acaacagcat gtacgagaat 2820
   tttagcacta gcttttggat tcggatccct aagtatttca acagcattag tctgaacaat 2880
   gaatatacca ttataaactg tatggaaaac aattctggtt ggaaagtgag cctgaactat 2940
   ggcgaaatta tctggaccct gcaggatacc caagaaatca agcaacgtgt cgtgttcaaa 3000
   tattctcaaa tgattaacat ttcggattac attaatcgtt ggatttttgt caccattacc 3060
   aataaccgtc ttaacaattc taaaatttat ataaatggca gattgattga tcaaaaacct 3120
   attagtaacc tggggaatat tcacgcgagc aataacatca tgtttaaact ggatggatgc 3180
   cgagatacgc atcgctacat atggatcaag tacttcaatc tgttcgataa agagctgaat 3240
   gaaaaggaga taaaagatct ttacgacaac cagtccaatt ccggaattct gaaagatttt 3300
   tggggagact atctgcagta tgacaaaccg tattacatgt taaatttata tgacccgaac 3360
   aaatatgtcg atgtgaacaa tgtcggtatc cgtgggtata tgtatctgaa aggcccgcgt 3420
   ggcagcgtca tgactacgaa catttatctt aactcctctc tgtatcgtgg caccaaattt 3480
   atcattaaga aatatgcctc aggcaataaa gataacatcg tacgcaacaa tgatcgcgtt 3540
   tatatcaatg tagtggttaa gaacaaagaa taccgcctgg ccaccaacgc atcccaggcg 3600
   ggcgtcgaaa aaattctgtc cgccttggag attcctgatg tgggcaacct gtcccaagtc 3660
   gttgtcatga aatcaaagaa cgaccagggc attacgaaca agtgtaaaat gaacctgcag 3720
   gataacaatg gtaatgatat cggctttatt ggcttccatc aattcaacaa tattgctaaa 3780
   ttagtcgcat cgaactggta caaccgccag atcgaacgca gctcacgcac tctcggatgt 3840
   tcttgggaat ttattccagt cgatgatggc tggggtgagc gacctctcta a          3891
<210> 32
   <211> 3891
   <212> DNA
   <213> Artificial Sequence
<220>
   <223> BoNT/A, S. typhimurium-modified 2
<400> 32
   atgccgtttg tgaacaaaca gtttaactat aaagatccgg tgaacggcgt ggatattgcg 60
   tatattaaaa ttccgaacgc gggccagatg cagccggtga aagcgtttaa aattcataac 120
   aaaatttggg tgattccgga acgcgatacc tttaccaacc cggaagaagg cgatctgaac 180
   ccgccgccgg aagcgaaaca ggtgccggtg agctattatg atagcaccta tctgagcacc 240
   gataacgaaa aagataacta tctgaaaggc gtgaccaaac tgtttgaacg catttatagc 300
   accgatctgg gccgcatgct gctgaccagc attgtgcgcg gcattccgtt ttggggcggc 360
   agcaccattg ataccgaact gaaagtgatt gataccaact gcattaacgt gattcagccg 420
   gatggcagct atcgcagcga agaactgaac ctggtgatta ttggcccgag cgcggatatt 480
   attcagtttg aatgcaaaag ctttggccat gaagtgctga acctgacccg caacggctat 540
   ggcagcaccc agtatattcg ctttagcccg gattttacct ttggctttga agaaagcctg 600
   gaagtggata ccaacccgct gctgggcgcg ggcaaatttg cgaccgatcc ggcggtgacc 660
   ctggcgcatg aactgattca tgcgggccat cgcctgtatg gcattgcgat taacccgaac 720
   cgcgtgttta aagtgaacac caacgcgtat tatgaaatga gcggcctgga agtgagcttt 780
   gaagaactgc gcacctttgg cggccatgat gcgaaattta ttgatagcct gcaggaaaac 840
   gaatttcgcc tgtattatta taacaaattt aaagatattg cgagcaccct gaacaaagcg 900
   aaaagcattg tgggcaccac cgcgagcctg cagtatatga aaaacgtgtt taaagaaaaa 960
   tatctgctga gcgaagatac cagcggcaaa tttagcgtgg ataaactgaa atttgataaa 1020
   ctgtataaaa tgctgaccga aatttatacc gaagataact ttgtgaaatt ttttaaagtg 1080
   ctgaaccgca aaacctatct gaactttgat aaagcggtgt ttaaaattaa cattgtgccg 1140
   aaagtgaact ataccattta tgatggcttt aacctgcgca acaccaacct ggcggcgaac 1200
   tttaacggcc agaacaccga aattaacaac atgaacttta ccaaactgaa aaactttacc 1260
   ggcctgtttg aattttataa actgctgtgc gtgcgcggca ttattaccag caaaaccaaa 1320
   agcctggata aaggctataa caaagcgctg aacgatctgt gcattaaagt gaacaactgg 1380
   gatctgtttt ttagcccgag cgaagataac tttaccaacg atctgaacaa aggcgaagaa 1440
   attaccagcg ataccaacat tgaagcggcg gaagaaaaca ttagcctgga tctgattcag 1500
   cagtattatc tgacctttaa ctttgataac gaaccggaaa acattagcat tgaaaacctg 1560
   agcagcgata ttattggcca gctggaactg atgccgaaca ttgaacgctt tccgaacggc 1620
   aaaaaatatg aactggataa atataccatg tttcattatc tgcgcgcgca ggaatttgaa 1680
   catggcaaaa gccgcattgc gctgaccaac agcgtgaacg aagcgctgct gaacccgagc 1740
   cgcgtgtata ccttttttag cagcgattat gtgaaaaaag tgaacaaagc gaccgaagcg 1800
   gcgatgtttc tgggctgggt ggaacagctg gtgtatgatt ttaccgatga aaccagcgaa 1860
   gtgagcacca ccgataaaat tgcggatatt accattatta ttccgtatat tggcccggcg 1920
   ctgaacattg gcaacatgct gtataaagat gattttgtgg gcgcgctgat ttttagcggc 1980
   gcggtgattc tgctggaatt tattccggaa attgcgattc cggtgctggg cacctttgcg 2040
   ctggtgagct atattgcgaa caaagtgctg accgtgcaga ccattgataa cgcgctgagc 2100
   aaacgcaacg aaaaatggga tgaagtgtat aaatatattg tgaccaactg gctggcgaaa 2160
   gtgaacaccc agattgatct gattcgcaaa aaaatgaaag aagcgctgga aaaccaggcg 2220
   gaagcgacca aagcgattat taactatcag tataaccagt ataccgaaga agaaaaaaac 2280
   aacattaact ttaacattga tgatctgagc agcaaactga acgaaagcat taacaaagcg 2340
   atgattaaca ttaacaaatt tctgaaccag tgcagcgtga gctatctgat gaacagcatg 2400
   attccgtatg gcgtgaaacg cctggaagat tttgatgcga gcctgaaaga tgcgctgctg 2460
   aaatatattt atgataaccg cggcaccctg attggccagg tggatcgcct gaaagataaa 2520
   gtgaacaaca ccctgagcac cgatattccg tttcagctga gcaaatatgt ggataaccag 2580
   cgcctgctga gcacctttac cgaatatatt aaaaacatta ttaacaccag cattctgaac 2640
   ctgcgctatg aaagcaacca tctgattgat ctgagccgct atgcgagcaa aattaacatt 2700
   ggcagcaaag tgaactttga tccgattgat aaaaaccaga ttcagctgtt taacctggaa 2760
   agcagcaaaa ttgaagtgat tctgaaaaac gcgattgtgt ataacagcat gtatgaaaac 2820
   tttagcacca gcttttggat tcgcattccg aaatatttta acagcattag cctgaacaac 2880
   gaatatacca ttattaactg catggaaaac aacagcggct ggaaagtgag cctgaactat 2940
   ggcgaaatta tttggaccct gcaggatacc caggaaatta aacagcgcgt ggtgtttaaa 3000
   tatagccaga tgattaacat tagcgattat attaaccgct ggatttttgt gaccattacc 3060
   aacaaccgcc tgaacaacag caaaatttat attaacggcc gcctgattga tcagaaaccg 3120
   attagcaacc tgggcaacat tcatgcgagc aacaacatta tgtttaaact ggatggctgc 3180
   cgcgataccc atcgctatat ttggattaaa tattttaacc tgtttgataa agaactgaac 3240
   gaaaaagaaa ttaaagatct gtatgataac cagagcaaca gcggcattct gaaagatttt 3300
   tggggcgatt atctgcagta tgataaaccg tattatatgc tgaacctgta tgatccgaac 3360
   aaatatgtgg atgtgaacaa cgtgggcatt cgcggctata tgtatctgaa aggcccgcgc 3420
   ggcagcgtga tgaccaccaa catttatctg aacagcagcc tgtatcgcgg caccaaattt 3480
   attattaaaa aatatgcgag cggcaacaaa gataacattg tgcgcaacaa cgatcgcgtg 3540
   tatattaacg tggtggtgaa aaacaaagaa tatcgcctgg cgaccaacgc gagccaggcg 3600
   ggcgtggaaa aaattctgag cgcgctggaa attccggatg tgggcaacct gagccaggtg 3660
   gtggtgatga aaagcaaaaa cgatcagggc attaccaaca aatgcaaaat gaacctgcag 3720
   gataacaacg gcaacgatat tggctttatt ggctttcatc agtttaacaa cattgcgaaa 3780
   ctggtggcga gcaactggta taaccgccag attgaacgca gcagccgcac cctgggctgc 3840
   agctgggaat ttattccggt ggatgatggc tggggcgaac gcccgctgta a 3891
<210> 33
   <211> 3891
   <212> DNA
   <213> Artificial Sequence
<220>
   <223> BoNT/A, S. typhimurium-modified 3
<400> 33
   atgccgtttg tcaacaagca gttcaattac aaagacccag tgaatggtgt tgatatcgcg 60
   tatatcaaaa ttcctaatgc gggccagatg caaccggtta aagcgtttaa gatccataac 120
   aagatctggg tgatccctga acgcgatacg tttaccaacc cggaagaggg cgatctgaac 180
   cccccgcccg aagccaaaca ggtgccggtt agctattacg actcgacgta tctgtccacg 240
   gataacgaaa aagataatta tctgaagggg gtcaccaaat tgttcgagcg gatttactcc 300
   accgatctgg gtcgcatgct gttaaccagt attgtccgcg gtattccttt ttggggcggg 360
   tccaccatcg ataccgaact gaaagttatt gatacgaatt gcattaatgt cattcagccg 420
   gatggcagct atcgtagcga agagcttaat ctggttatca ttggcccgtc agccgacatt 480
   atccaattcg agtgcaaaag cttcggccat gaagtgctga acctcacccg caacggctac 540
   ggttctaccc aatacattcg gttttcgccg gattttacgt ttgggtttga ggaaagcctg 600
   gaggtcgaca ccaatcctct gctcggcgcg ggtaaatttg ccacggaccc ggcggtcacg 660
   ttagcgcatg aattaattca cgcgggccat cgcctttatg gtattgcgat caacccgaat 720
   cgggtgttca aagtaaatac caacgcttac tatgaaatgt ctggccttga agttagcttc 780
   gaggaactgc gcacgtttgg cgggcacgac gcgaagttca ttgatagcct ccaggaaaat 840
   gagtttcgct tatactatta caacaaattc aaagatatcg cgtcgaccct gaacaaagcc 900
   aagagtattg tgggtaccac ggcgtctctt cagtatatga agaatgtttt taaggaaaag 960
   tacttactga gcgaggatac ctccggtaaa ttttcggtgg ataaacttaa attcgataag 1020
   ttgtataaaa tgttaaccga gatttatacc gaagacaact tcgtcaaatt tttcaaagtc 1080
   ctgaaccgca aaacctatct taattttgac aaagcagtgt ttaaaatcaa catcgtacca 1140
   aaggtgaact ataccattta cgacggcttc aacctgcgca atacgaacct ggcagccaac 1200
   ttcaatggcc aaaacaccga gatcaacaat atgaacttta ccaagctgaa aaattttacc 1260
   gggctgtttg agttctataa actgctttgt gtccgtggca tcattaccag caaaaccaaa 1320
   agcctggata aaggctataa caaagcgctc aacgatttat gtatcaaggt gaataactgg 1380
   gacctgtttt tcagtccgag tgaggacaat tttaccaacg acttaaataa aggcgaagag 1440
   attaccagtg acaccaatat tgaggccgct gaagagaaca tttctctcga cttgattcag 1500
   caatattacc ttacgttcaa ttttgataac gaaccagaaa acatttccat tgagaacctg 1560
   tcgtcagaca ttatcggcca actggaattg atgccgaaca ttgaacgttt tccgaatggc 1620
   aaaaagtatg aacttgacaa atatacgatg tttcattatc tgcgggccca ggaattcgaa 1680
   catgggaaaa gccggattgc cctgaccaac agcgtgaatg aggctttact caacccttct 1740
   cgtgtttata cgtttttctc aagcgattac gtcaaaaagg taaataaagc gacggaggca 1800
   gcgatgttcc tgggttgggt ggaacaactc gtgtacgatt ttacggatga gacgtcggaa 1860
   gtgtcgacga ccgataaaat tgcggacatt accattatca ttccatatat tggcccggcg 1920
   ctgaatattg gcaatatgct ctataaagac gattttgttg gcgccttgat tttcagtggc 1980
   gccgtgattc tcctggaatt tatcccagag atcgctatcc ccgtactggg gacctttgca 2040
   ttggtatctt atattgctaa caaagtcctt accgtacaga ccatcgataa cgcgctgagt 2100
   aagcgcaatg aaaaatggga tgaagtctac aagtatattg tgacgaattg gctggcgaaa 2160
   gttaacaccc agatcgatct gattcgcaag aaaatgaaag aagcgctgga gaatcaagcc 2220
   gaagcaacca aagcgattat caactatcag tataaccagt atacggagga agagaaaaac 2280
   aatattaatt ttaacatcga cgatctgagc tccaaactga acgaaagcat caataaagct 2340
   atgatcaata ttaataagtt cctgaaccag tgcagtgtga gctatctgat gaactccatg 2400
   atcccgtacg gggtaaaacg cttggaagac ttcgatgcaa gtctgaaaga tgcgctgtta 2460
   aaatatatct atgataaccg cggcaccctg atcggccagg tggatcgcct gaaagataaa 2520
   gttaacaata ccctttcaac cgacatcccg tttcagctgt cgaaatacgt ggataaccag 2580
   cgcctgttgt caacctttac cgaatacatc aaaaatatca ttaatacgtc catcctgaat 2640
   ttgcgttatg aaagtaacca cctgatcgac ctgtcccgct acgcgtcaaa aatcaatatt 2700
   ggctctaagg tcaactttga ccccattgac aagaatcaga tccagttgtt caacctggaa 2760
   tcatctaaga ttgaagtcat tctgaaaaac gcaattgtgt ataactccat gtacgaaaac 2820
   ttctcaacct ccttttggat tcgcattccg aaatacttta actcgatctc cctcaataac 2880
   gaatacacga ttatcaactg catggaaaac aattctggct ggaaagtgtc cttaaattat 2940
   ggcgagatca tttggaccct gcaagacacc caggagatta aacaacgtgt ggtctttaaa 3000
   tatagccaga tgattaatat tagcgattat attaaccgct ggatttttgt tacgatcacc 3060
   aataaccgtt taaacaatag caagatttac attaatggtc gcctcatcga tcagaaaccg 3120
   attagcaacc tgggtaacat tcacgccagt aataacatta tgttcaagct ggacggctgt 3180
   cgtgataccc accgttacat ttggatcaaa tactttaact tattcgataa agaactgaat 3240
   gaaaaagaaa tcaaagatct gtacgacaac cagtccaatt cgggcattct gaaagatttt 3300
   tggggcgact atctgcaata tgacaaaccc tactatatgc ttaatcttta tgatcccaat 3360
   aaatatgtag atgtaaataa cgttggcatc cgcggctata tgtatctgaa aggcccgcgt 3420
   ggcagcgtaa tgaccacgaa tatttatttg aactcatcgt tgtaccgcgg taccaaattc 3480
   attatcaaaa agtacgcgag cgggaataaa gataacattg tacgtaataa cgatcgtgtt 3540
   tatatcaatg tagtcgtgaa aaacaaagaa taccgcctgg ccacgaatgc ttcgcaggcc 3600
   ggtgttgaaa aaattttatc tgccctggag atcccggacg ttggcaactt gtcgcaggtg 3660
   gtcgtgatga aaagcaagaa cgatcagggc atcaccaata agtgcaaaat gaatctgcag 3720
   gacaataacg ggaacgatat tggttttatc ggcttccatc agtttaataa cattgccaaa 3780
   ctggtcgcgt cgaattggta caatcgccag attgaacgta gctcccgcac cctgggctgt 3840
   agctgggaat ttattccggt ggatgatggc tggggtgagc gtcctctgta a 3891
<210> 34
   <211> 3891
   <212> DNA
   <213> Artificial Sequence
<220>
   <223> BoNT/A, P. pastoris-modified 1
<400> 34
   atgcctttcg ttaataagca gttcaattac aaagaccctg tgaacggagt ggatatcgct 60
   tatattaaga ttcctaatgc tggtcaaatg caacctgtta aagccttcaa aatacataac 120
   aagatctggg ttatccctga acgcgatact tttacgaacc ctgaggaagg tgatttgaac 180
   cctccacctg aagctaaaca agttcctgtt tcgtattacg actccactta tttatcaacc 240
   gacaatgaaa aggataacta cctgaaaggc gtcacaaagt tgttcgaaag aatttactca 300
   actgatctgg gtcgtatgtt acttacaagt atcgtgagag gtattccatt ctggggggga 360
   tctactatcg atacagaatt gaaagttatc gatactaatt gtatcaacgt tattcaaccc 420
   gatggaagct accgttcaga ggaactaaat cttgttatca taggaccctc agctgacata 480
   attcagtttg aatgcaagtc ctttggacat gaagttttga atttaacaag aaatggttac 540
   ggttcgactc aatacattag attctctcca gatttcacat tcggttttga agagagttta 600
   gaagtcgata caaacccatt gcttggggcc ggcaaattcg ctacagaccc cgccgtcact 660
   ttggcacacg aacttattca tgctggtcac cgactatacg gtatagctat taacccaaat 720
   agggttttta aagttaacac taacgcatat tacgaaatga gtggactcga agtaagtttt 780
   gaagagctaa ggactttcgg gggtcatgat gctaagttta ttgattctct gcaagaaaat 840
   gaatttaggc tttattacta taataagttt aaggatatcg cctccactct gaacaaggcc 900
   aaatctatcg tgggcacaac cgccagcttg caatacatga aaaatgtgtt caaagagaag 960
   tacctgttgt cagaagatac gagtggcaag tttagtgtag acaagcttaa gttcgacaaa 1020
   ctttataaga tgctgactga gatttacacc gaagacaatt tcgtcaaatt tttcaaagtt 1080
   ctcaaccgga agacctattt gaattttgac aaagccgtct ttaaaataaa cattgttcct 1140
   aaagtcaact ataccattta tgacggattt aatttgagaa acaccaactt ggctgcaaac 1200
   tttaacgggc aaaatacaga gattaataac atgaatttca cgaagttgaa gaacttcaca 1260
   ggtttgttcg agttctacaa actactgtgt gtacggggca ttataacgtc caagaccaag 1320
   tctctagata aaggttacaa taaagctttg aacgatttgt gcattaaggt aaataactgg 1380
   gacttatttt tctctccctc tgaggataac ttcaccaacg atttaaataa aggcgaggaa 1440
   atcacttctg acacaaacat tgaggctgca gaggaaaata tatctcttga tcttattcag 1500
   caatactatt tgacgtttaa ctttgacaac gagccagaga acatttcaat cgaaaatcta 1560
   tcttcggata ttatcggcca gctggaacta atgccaaata ttgagaggtt cccaaatgga 1620
   aagaaatacg agcttgataa gtatacgatg tttcattact tacgtgcaca agaattcgaa 1680
   catggaaagt caagaatcgc tcttactaat tccgttaacg aggctctgtt gaatccttca 1740
   agggtttata cctttttctc atccgattac gttaagaaag tcaataaggc tactgaggct 1800
   gccatgtttc tcggatgggt ggaacagttg gtttatgact ttactgacga aacctccgag 1860
   gtttccacta ccgataaaat agcagatatc actataatta taccctacat tggacctgct 1920
   ctcaatatcg gaaacatgtt atataaggat gactttgttg gtgctctaat tttctccggc 1980
   gccgtaattc tactggagtt tattcccgaa atagctatac ctgtcttggg aacatttgct 2040
   ctggtttcgt acattgctaa taaggttttg actgtccaaa caattgataa cgcactgtca 2100
   aagcgaaacg agaaatggga cgaggtatat aagtatatag taacgaactg gctcgcaaag 2160
   gtgaatactc aaattgatct cattagaaag aaaatgaaag aagcattgga aaaccaagcc 2220
   gaggctacaa aagcgattat aaattaccag tacaaccagt atactgaaga ggaaaagaac 2280
   aatattaatt ttaatatcga tgacttgagt agcaagctca atgaatccat aaataaggct 2340
   atgattaaca taaacaaatt cttgaaccag tgtagcgtta gttatttgat gaactctatg 2400
   ataccatatg gggttaagag actggaagat tttgatgcct cgctgaagga tgcccttttg 2460
   aagtatattt acgacaatcg aggtaccctg atcggacaag tcgacagact aaaggacaag 2520
   gtcaacaata ctttgtctac cgacatccca tttcaacttt ctaaatatgt tgataaccaa 2580
   agattgcttt ctactttcac cgaatacatt aaaaatataa ttaataccag tattttgaat 2640
   ctgagatatg agtctaacca cttaatagat ttaagtcgtt atgcctcgaa gattaacatt 2700
   gggtctaaag tgaactttga tccgatcgac aagaaccaga tccagttgtt taatctcgaa 2760
   tcctcaaaga ttgaggtcat tttgaaaaat gcaattgttt ataacagcat gtacgaaaac 2820
   ttttccacct ctttttggat cagaattcca aagtacttta actcaatttc attgaacaat 2880
   gagtacacta ttatcaattg tatggaaaat aacagtggct ggaaagtttc tttaaattac 2940
   ggtgagatta tctggacatt acaagatact caagaaatca aacagagggt cgtatttaag 3000
   tattctcaaa tgattaatat ttccgactac atcaatcgtt ggatcttcgt gacaatcact 3060
   aataacagac tgaataactc aaaaatttat atcaacggta gattaattga tcaaaaacca 3120
   atttctaact tgggtaatat ccacgcttct aacaatatca tgttcaagtt agacggttgt 3180
   agagatacac accgttatat ctggatcaag tacttcaatc ttttcgataa ggaactaaac 3240
   gagaaggaga tcaaggactt atacgataat caaagtaact ccggtattct taaggatttt 3300
   tggggtgact acttgcagta tgacaaaccg tattacatgt tgaacttgta cgatccaaac 3360
   aaatatgtgg acgttaataa cgtaggaatt agaggataca tgtacttaaa gggtccaaga 3420
   ggatctgtaa tgactacgaa tatctactta aactcctcac tctacagagg taccaaattt 3480
   ataattaaga aatatgcgtc cggtaacaaa gacaatatag tcagaaataa cgatcgagtt 3540
   tacattaacg ttgtagtgaa aaataaggaa taccgcctag ctactaacgc atctcaagca 3600
   ggtgtcgaga aaatattgtc cgctcttgaa attccggacg tgggtaacct gtcgcaagtg 3660
   gtcgttatga agagtaaaaa tgaccaggga attaccaata aatgcaaaat gaaccttcag 3720
   gataataacg gtaatgatat cggatttatc ggttttcatc agttcaacaa cattgcgaag 3780
   ttagtcgcct ctaactggta taacagacaa atcgagagaa gctctaggac tctaggttgt 3840
   agctgggagt tcattccagt ggatgacgga tggggtgaac gtccattgta a 3891
<210> 35
   <211> 3891
   <212> DNA
   <213> Artificial Sequence
<220>
   <223> BoNT/A, P. pastoris-modified 2
<400> 35
   atgccatttg ttaacaagca atttaactac aaggatccag ttaacggtgt tgatattgct 60
   tacattaaga ttccaaacgc tggtcaaatg caaccagtta aggcttttaa gattcataac 120
   aagatttggg ttattccaga aagagatact tttactaacc cagaagaagg tgatttgaac 180
   ccaccaccag aagctaagca agttccagtt tcttactacg attctactta cttgtctact 240
   gataacgaaa aggataacta cttgaagggt gttactaagt tgtttgaaag aatttactct 300
   actgatttgg gtagaatgtt gttgacttct attgttagag gtattccatt ttggggtggt 360
   tctactattg atactgaatt gaaggttatt gatactaact gtattaacgt tattcaacca 420
   gatggttctt acagatctga agaattgaac ttggttatta ttggtccatc tgctgatatt 480
   attcaatttg aatgtaagtc ttttggtcat gaagttttga acttgactag aaacggttac 540
   ggttctactc aatacattag attttctcca gattttactt ttggttttga agaatctttg 600
   gaagttgata ctaacccatt gttgggtgct ggtaagtttg ctactgatcc agctgttact 660
   ttggctcatg aattgattca tgctggtcat agattgtacg gtattgctat taacccaaac 720
   agagttttta aggttaacac taacgcttac tacgaaatgt ctggtttgga agtttctttt 780
   gaagaattga gaacttttgg tggtcatgat gctaagttta ttgattcttt gcaagaaaac 840
   gaatttagat tgtactacta caacaagttt aaggatattg cttctacttt gaacaaggct 900
   aagtctattg ttggtactac tgcttctttg caatacatga agaacgtttt taaggaaaag 960
   tacttgttgt ctgaagatac ttctggtaag ttttctgttg ataagttgaa gtttgataag 1020
   ttgtacaaga tgttgactga aatttacact gaagataact ttgttaagtt ttttaaggtt 1080
   ttgaacagaa agacttactt gaactttgat aaggctgttt ttaagattaa cattgttcca 1140
   aaggttaact acactattta cgatggtttt aacttgagaa acactaactt ggctgctaac 1200
   tttaacggtc aaaacactga aattaacaac atgaacttta ctaagttgaa gaactttact 1260
   ggtttgtttg aattttacaa gttgttgtgt gttagaggta ttattacttc taagactaag 1320
   tctttggata agggttacaa caaggctttg aacgatttgt gtattaaggt taacaactgg 1380
   gatttgtttt tttctccatc tgaagataac tttactaacg atttgaacaa gggtgaagaa 1440
   attacttctg atactaacat tgaagctgct gaagaaaaca tttctttgga tttgattcaa 1500
   caatactact tgacttttaa ctttgataac gaaccagaaa acatttctat tgaaaacttg 1560
   tcttctgata ttattggtca attggaattg atgccaaaca ttgaaagatt tccaaacggt 1620
   aagaagtacg aattggataa gtacactatg tttcattact tgagagctca agaatttgaa 1680
   catggtaagt ctagaattgc tttgactaac tctgttaacg aagctttgtt gaacccatct 1740
   agagtttaca cttttttttc ttctgattac gttaagaagg ttaacaaggc tactgaagct 1800
   gctatgtttt tgggttgggt tgaacaattg gtttacgatt ttactgatga aacttctgaa 1860
   gtttctacta ctgataagat tgctgatatt actattatta ttccatacat tggtccagct 1920
   ttgaacattg gtaacatgtt gtacaaggat gattttgttg gtgctttgat tttttctggt 1980
   gctgttattt tgttggaatt tattccagaa attgctattc cagttttggg tacttttgct 2040
   ttggtttctt acattgctaa caaggttttg actgttcaaa ctattgataa cgctttgtct 2100
   aagagaaacg aaaagtggga tgaagtttac aagtacattg ttactaactg gttggctaag 2160
   gttaacactc aaattgattt gattagaaag aagatgaagg aagctttgga aaaccaagct 2220
   gaagctacta aggctattat taactaccaa tacaaccaat acactgaaga agaaaagaac 2280
   aacattaact ttaacattga tgatttgtct tctaagttga acgaatctat taacaaggct 2340
   atgattaaca ttaacaagtt tttgaaccaa tgttctgttt cttacttgat gaactctatg 2400
   attccatacg gtgttaagag attggaagat tttgatgctt ctttgaagga tgctttgttg 2460
   aagtacattt acgataacag aggtactttg attggtcaag ttgatagatt gaaggataag 2520
   gttaacaaca ctttgtctac tgatattcca tttcaattgt ctaagtacgt tgataaccaa 2580
   agattgttgt ctacttttac tgaatacatt aagaacatta ttaacacttc tattttgaac 2640
   ttgagatacg aatctaacca tttgattgat ttgtctagat acgcttctaa gattaacatt 2700
   ggttctaagg ttaactttga tccaattgat aagaaccaaa ttcaattgtt taacttggaa 2760
   tcttctaaga ttgaagttat tttgaagaac gctattgttt acaactctat gtacgaaaac 2820
   ttttctactt ctttttggat tagaattcca aagtacttta actctatttc tttgaacaac 2880
   gaatacacta ttattaactg tatggaaaac aactctggtt ggaaggtttc tttgaactac 2940
   ggtgaaatta tttggacttt gcaagatact caagaaatta agcaaagagt tgtttttaag 3000
   tactctcaaa tgattaacat ttctgattac attaacagat ggatttttgt tactattact 3060
   aacaacagat tgaacaactc taagatttac attaacggta gattgattga tcaaaagcca 3120
   atttctaact tgggtaacat tcatgcttct aacaacatta tgtttaagtt ggatggttgt 3180
   agagatactc atagatacat ttggattaag tactttaact tgtttgataa ggaattgaac 3240
   gaaaaggaaa ttaaggattt gtacgataac caatctaact ctggtatttt gaaggatttt 3300
   tggggtgatt acttgcaata cgataagcca tactacatgt tgaacttgta cgatccaaac 3360
   aagtacgttg atgttaacaa cgttggtatt agaggttaca tgtacttgaa gggtccaaga 3420
   ggttctgtta tgactactaa catttacttg aactcttctt tgtacagagg tactaagttt 3480
   attattaaga agtacgcttc tggtaacaag gataacattg ttagaaacaa cgatagagtt 3540
   tacattaacg ttgttgttaa gaacaaggaa tacagattgg ctactaacgc ttctcaagct 3600
   ggtgttgaaa agattttgtc tgctttggaa attccagatg ttggtaactt gtctcaagtt 3660
   gttgttatga agtctaagaa cgatcaaggt attactaaca agtgtaagat gaacttgcaa 3720
   gataacaacg gtaacgatat tggttttatt ggttttcatc aatttaacaa cattgctaag 3780
   ttggttgctt ctaactggta caacagacaa attgaaagat cttctagaac tttgggttgt 3840
   tcttgggaat ttattccagt tgatgatggt tggggtgaaa gaccattgta a          3891
<210> 36
   <211> 3891
   <212> DNA
   <213> Artificial Sequence
<220>
   <223> BoNT/A, P. pastoris-modified 3
<400> 36
   atgccattcg ttaacaaaca atttaactat aaagatcctg tgaacggtgt agatatcgcc 60
   tacattaaga ttcctaacgc tggtcagatg cagccagtta aggcctttaa aattcataat 120
   aaaatttggg ttattccaga aagagatacc tttacaaatc cagaagaggg agacttgaat 180
   cctccacctg aggctaaaca agttcctgtc agctattacg actccactta cttgtcaact 240
   gataacgaaa aggataatta cttgaagggt gtgactaaat tgttcgagag aatctacagt 300
   actgatctgg gtcgtatgct gttgaccagt atcgttagag gtatcccttt ctggggaggt 360
   tccacaattg acacagaatt gaaggtaatc gatacaaatt gtattaatgt cattcagcca 420
   gacggttcct acaggagtga agagctgaat ttggttatca ttggacccag tgctgatatt 480
   atccaatttg agtgtaaatc tttcggtcac gaagtcctaa acttgactag aaatggttat 540
   ggcagtaccc agtacatccg attttcacca gactttacct ttggtttcga agagtccttg 600
   gaggtggata ctaatccatt attgggtgct ggcaagtttg ctaccgatcc tgcagtgact 660
   ctagctcacg aacttattca tgctggccac agattgtatg gtattgccat taacccaaat 720
   agagttttta aggttaacac aaacgcctac tatgaaatgt caggacttga agttagtttt 780
   gaagagttga ggaccttcgg tggacacgac gccaagttta ttgattctct acaagagaac 840
   gaattcagac tatactatta caacaaattt aaggatattg cttcgactct taacaaggca 900
   aaatcaatag taggtactac cgcatctctg caatatatga agaatgtgtt taaggaaaag 960
   tacctgttat cagaagacac ttctggtaag ttctctgtag ataagctgaa atttgataaa 1020
   ctatacaaga tgttgacaga aatatataca gaggataact tcgtaaagtt tttcaaagtt 1080
   ctaaaccgaa aaacttatct gaacttcgat aaggcagtct ttaaaattaa tatcgttcca 1140
   aaggttaact acaccattta cgatggtttt aacctgagaa atacaaattt ggctgcaaat 1200
   ttcaacggcc aaaacactga aattaataac atgaacttta ccaagctaaa aaatttcact 1260
   ggattgtttg agttttacaa acttctatgc gttagaggta ttatcacttc taagactaaa 1320
   tctttggaca aaggatataa caaggctttg aacgatcttt gtattaaagt taataactgg 1380
   gacttgttct ttttcccctc ggaagataac ttcaccaatg atctaaataa aggcgaggaa 1440
   atcacttctg ataccaacat tgaggcagct gaagagaaca tctccttgga cttaatacaa 1500
   cagtactatc ttactttcaa ttttgacaac gagcctgaaa acatatcgat tgaaaatttg 1560
   tcctctgaca ttatcggaca acttgaactg atgcccaaca tcgaaagatt tccaaatggt 1620
   aaaaagtacg aattagacaa atatactatg ttccattatc ttagggctca agagtttgag 1680
   catggtaagt caagaatagc cttgactaac tcagtaaatg aagctttgct aaacccatca 1740
   agggtctata ctttcttttc aagcgattac gttaaaaagg ttaacaaagc taccgaagca 1800
   gctatgtttc taggatgggt tgaacaattg gtgtacgact tcactgatga gacttctgaa 1860
   gttagcacta cagacaaaat cgctgacata accattatca ttccttacat tggtccagcc 1920
   cttaacattg gtaacatgtt gtacaaggac gattttgttg gtgctttgat tttcagcgga 1980
   gctgttattc ttctggagtt catcccagaa attgcaatac cagtcttagg tacatttgct 2040
   ctggtctctt atattgccaa taaggtcttg actgtgcaaa ctattgacaa tgctttgagc 2100
   aaacgtaacg agaagtggga cgaggtctat aagtacattg tcaccaactg gttagctaag 2160
   gtaaacacac agatagactt gattcgtaag aaaatgaaag aagcccttga aaaccaagca 2220
   gaggcaacaa aggctataat taactaccaa tataaccagt ataccgaaga ggaaaaaaac 2280
   aatataaatt tcaatattga tgacctttcc tctaaactga atgagtctat aaacaaagct 2340
   atgattaaca ttaacaagtt ccttaatcag tgctctgttt cctacttaat gaattccatg 2400
   attccttatg gtgtcaagag attggaggac tttgatgcat cattgaagga tgcactgcta 2460
   aaatacatct atgataatag aggcactctt atcggtcaag tggacagatt aaaagacaag 2520
   gttaacaata ctttgtcgac agatattcct tttcagcttt ctaagtatgt ggataatcaa 2580
   cgattattgt cgacttttac tgagtacatt aagaatatca taaatacttc catcctaaat 2640
   ctgagatatg aatctaacca tttgatcgat ttatcaagat acgcttctaa gattaatatt 2700
   ggcagtaagg taaatttcga tcccatagat aagaatcaaa tccagttgtt taatctggag 2760
   tcctctaaga ttgaagttat cttgaagaac gccattgtgt acaactcgat gtatgagaac 2820
   ttcagtacaa gtttctggat taggatcccc aaatacttta atagtatttc attgaataac 2880
   gagtacacta taatcaactg catggaaaac aatagtggtt ggaaagtgtc tttgaactat 2940
   ggagaaatca tttggacatt gcaagacact caagaaatca agcaacgtgt agtctttaag 3000
   tattcacaga tgatcaatat aagcgattac atcaacaggt ggatttttgt cactattaca 3060
   aataacagat tgaataactc gaagatatac ataaatggac gattgattga tcaaaaaccc 3120
   atttcaaact taggtaacat tcatgcctcc aacaatatca tgttcaagct ggacggatgt 3180
   cgtgataccc acagatatat ttggatcaaa tatttcaact tgtttgataa agaattaaat 3240
   gaaaaagaga taaaagactt atacgataac caatctaatt ccggaatatt aaaggatttc 3300
   tggggagatt atttgcaata tgacaaacct tactatatgt taaatcttta cgaccctaac 3360
   aaatatgtag atgttaacaa tgttggtatt agaggctaca tgtacctgaa aggtccacgt 3420
   ggtagcgtga tgactaccaa tatttatttg aactcctctt tatacagggg tactaaattt 3480
   attataaaga aatatgcttc aggaaataag gataatatag ttagaaataa cgatcgtgtc 3540
   tacatcaacg ttgtcgtgaa gaacaaggaa tacagattgg ccaccaatgc ttctcaagcc 3600
   ggtgtagaga agatcttatc tgctctggag atcccagacg tgggtaactt gtctcaggtt 3660
   gtcgttatga agtccaaaaa tgaccagggt atcactaaca agtgtaaaat gaatcttcag 3720
   gacaataacg gaaacgatat aggattcatt ggattccatc aattcaataa cattgccaag 3780
   cttgtcgcta gtaattggta caatagacag atcgaaagat ctagtagaac attaggttgt 3840
   tcctgggagt ttatcccagt tgatgacgga tggggcgaaa gaccattgta a 3891
<210> 37
   <211> 3891
   <212> DNA
   <213> Artificial Sequence
<220>
   <223> BoNT/A, S. cerevisiae-modified 1
<400> 37
   atgccttttg tgaataagca gtttaattat aaagacccag tgaatggggt tgacatcgcc 60
   tatataaaga ttccaaacgc tggtcagatg caaccagtca aagcctttaa gattcataat 120
   aaaatttggg tcattccaga acgagatact tttactaatc cggaggaagg cgatctgaat 180
   ccaccgccag aggcgaaaca agttccagtt tcctattacg attcaaccta tttgagcaca 240
   gataatgaaa aggataacta tctgaaaggg gtaactaagt tatttgaaag gatttactcc 300
   actgatcttg ggaggatgtt attgacctct attgtaagag gaattccatt ttggggtggg 360
   agcacgattg ataccgaact taaggtaata gatactaatt gtataaacgt gattcaacca 420
   gatggatcat atagatctga ggaactaaat ctggttataa ttggcccatc agcggatatt 480
   atacagttcg agtgtaaatc atttggtcat gaagttttaa acttgacacg taatggctac 540
   ggcagtactc aatatataag attttccccc gatttcactt ttggtttcga agagtcacta 600
   gaagtggaca cgaaccctct actgggggcg ggtaagtttg ccactgatcc ggcagttacg 660
   cttgctcatg aattaattca cgccggtcat agattatacg gcattgcaat taaccctaat 720
   agggtattta aagtcaatac taacgcatat tacgaaatgt caggattgga ggtatccttt 780
   gaagagctta gaacgttcgg aggtcatgat gcaaaattta tcgattcgtt acaagaaaac 840
   gaattcaggt tgtattacta taacaagttt aaagacattg ctagtacatt aaacaaggcc 900
   aagtctatag tgggaacaac cgctagtttg caatacatga agaacgtctt caaagaaaaa 960
   tacttattgt ccgaggacac ttctggtaag tttagtgtag acaagttaaa attcgataaa 1020
   ctgtataaga tgttgacaga aatttacacg gaagataact ttgtcaaatt ctttaaagtc 1080
   ctgaatagaa agacctactt aaatttcgat aaagcagtgt ttaagattaa cattgttcca 1140
   aaagttaatt atacgatcta cgatggcttt aacctcagaa acaccaatct agctgccaat 1200
   ttcaacggtc aaaatacaga aattaataac atgaacttca ctaagttaaa aaatttcacc 1260
   ggtttatttg aattttacaa actattgtgc gtaaggggca ttatcacgtc aaaaacaaaa 1320
   agtttggata aggggtacaa taaagctttg aacgaccttt gcatcaaagt caataactgg 1380
   gatttgttct tttctccttc agaggataac ttcacaaatg accttaataa gggtgaagag 1440
   attacttctg atacaaatat cgaagctgca gaagagaaca tttctttaga tctaattcaa 1500
   cagtactatt tgacttttaa cttcgataat gagcctgaga atatatcaat tgaaaatttg 1560
   agttctgata tcattggtca actggagtta atgcctaaca tcgaaagatt tcccaacggc 1620
   aaaaagtatg agttggacaa gtacacaatg ttccactatc tgagggcaca agaatttgaa 1680
   cacggtaagt ccaggatcgc tttgaccaac tcagttaacg aagccttact taatccatcc 1740
   agagtgtata cttttttctc gagtgactat gtgaagaaag taaataaagc aacagaagct 1800
   gccatgtttt tgggatgggt agaacaattg gtatatgatt ttactgatga aaccagcgag 1860
   gtttctacta cagacaaaat tgccgatatc actatcataa ttccatatat aggtccggct 1920
   ctaaacatag gtaacatgtt gtataaagat gactttgtag gagctttaat tttctcagga 1980
   gctgttattc tattagaatt tattcctgaa atcgccattc ctgttctagg tacattcgcg 2040
   ctagtctcct acatcgcaaa caaagtgcta actgtccaaa caatagataa tgctttgtct 2100
   aaaagaaatg aaaagtggga cgaagtttac aaatatattg ttactaattg gctggctaag 2160
   gtgaatacac aaatagactt gatccgtaag aaaatgaaag aagctcttga aaatcaggcc 2220
   gaagcaacga aggcaataat caattatcaa tataatcaat acacagaaga ggaaaaaaat 2280
   aacataaact ttaatattga tgacctatca tctaaattaa acgagtccat aaataaggca 2340
   atgatcaaca taaacaaatt ccttaatcaa tgttctgtct cttatctgat gaattccatg 2400
   atcccctacg gtgttaaacg gttagaagac ttcgacgctt ctttgaagga tgctttattg 2460
   aaatacatat atgataaccg cggcacatta ataggtcaag ttgatagact gaaagataaa 2520
   gttaataaca ctttgagcac tgatattcca tttcaactct ctaaatacgt agataatcag 2580
   agactcctta gcacgtttac cgagtatata aagaatatta taaatacctc gatactcaat 2640
   ctccgctatg aatccaacca tcttatcgac ctttcaagat acgcatcgaa aattaatatt 2700
   ggtagtaaag ttaatttcga ccccatagat aaaaatcaaa tccaactatt caacctagag 2760
   tcttcgaaga ttgaggttat tctgaaaaat gccatcgtgt ataatagcat gtatgagaat 2820
   tttagcacta gtttctggat tagaatacct aaatatttta actccatttc attgaataac 2880
   gaatacacga ttatcaactg tatggaaaac aattcaggtt ggaaggtgtc gttaaactat 2940
   ggtgaaatta tctggactct gcaggacacc caagaaatca agcagagagt cgtttttaag 3000
   tatagtcaga tgattaacat aagtgattac attaataggt ggatattcgt gactattaca 3060
   aataaccgtt tgaacaattc gaaaatctat attaatggta gattaatcga tcaaaagcct 3120
   atttctaatt tgggtaatat tcacgcttca aataacatta tgttcaagtt agacggttgc 3180
   agagatacac atagatacat ttggatcaaa tatttcaatt tatttgacaa agagttaaac 3240
   gaaaaggaaa ttaaagattt gtacgacaat caatctaact ccggtatatt aaaagacttt 3300
   tggggcgatt atttacagta cgataagcct tattacatgc ttaacctata cgaccctaat 3360
   aagtatgtcg atgtaaataa cgtcggaatc agaggttaca tgtatttgaa gggtccaaga 3420
   gggtctgtca tgacaaccaa catataccta aactcatccc tttaccgtgg tacaaaattc 3480
   atcattaaaa agtatgcttc tggaaataaa gataatatag ttcgtaataa cgatcgagta 3540
   tatataaatg ttgtcgttaa gaataaagaa tatcgtcttg caaccaatgc gagtcaagcg 3600
   ggagttgaaa agatattgtc tgctttggaa atacccgatg ttggaaactt aagccaggta 3660
   gtggttatga aaagcaaaaa tgaccaagga atcaccaata agtgtaaaat gaatctccaa 3720
   gacaataacg ggaatgatat cggctttatt ggattccatc agtttaataa tattgcaaaa 3780
   ttagttgcat cgaattggta caatagacaa atcgaaaggt ctagtcgaac tttgggttgt 3840
   tcatgggaat ttatacccgt tgatgatggt tggggcgaac ggccattata a 3891
<210> 38
   <211> 3891
   <212> DNA
   <213> Artificial Sequence
<220>
   <223> BoNT/A, S. cerevisiae-modified 2
<400> 38
   atgccatttg ttaataaaca atttaattat aaagatccag ttaatggtgt tgatattgct 60
   tatattaaaa ttccaaatgc tggtcaaatg caaccagtta aagcttttaa aattcataat 120
   aaaatttggg ttattccaga aagagatact tttactaatc cagaagaagg tgatttgaat 180
   ccaccaccag aagctaaaca agttccagtt tcttattatg attctactta tttgtctact 240
   gataatgaaa aagataatta tttgaaaggt gttactaaat tgtttgaaag aatttattct 300
   actgatttgg gtagaatgtt gttgacttct attgttagag gtattccatt ttggggtggt 360
   tctactattg atactgaatt gaaagttatt gatactaatt gtattaatgt tattcaacca 420
   gatggttctt atagatctga agaattgaat ttggttatta ttggtccatc tgctgatatt 480
   attcaatttg aatgtaaatc ttttggtcat gaagttttga atttgactag aaatggttat 540
   ggttctactc aatatattag attttctcca gattttactt ttggttttga agaatctttg 600
   gaagttgata ctaatccatt gttgggtgct ggtaaatttg ctactgatcc agctgttact 660
   ttggctcatg aattgattca tgctggtcat agattgtatg gtattgctat taatccaaat 720
   agagttttta aagttaatac taatgcttat tatgaaatgt ctggtttgga agtttctttt 780
   gaagaattga gaacttttgg tggtcatgat gctaaattta ttgattcttt gcaagaaaat 840
   gaatttagat tgtattatta taataaattt aaagatattg cttctacttt gaataaagct 900
   aaatctattg ttggtactac tgcttctttg caatatatga aaaatgtttt taaagaaaaa 960
   tatttgttgt ctgaagatac ttctggtaaa ttttctgttg ataaattgaa atttgataaa 1020
   ttgtataaaa tgttgactga aatttatact gaagataatt ttgttaaatt ttttaaagtt 1080
   ttgaatagaa aaacttattt gaattttgat aaagctgttt ttaaaattaa tattgttcca 1140
   aaagttaatt atactattta tgatggtttt aatttgagaa atactaattt ggctgctaat 1200
   tttaatggtc aaaatactga aattaataat atgaatttta ctaaattgaa aaattttact 1260
   ggtttgtttg aattttataa attgttgtgt gttagaggta ttattacttc taaaactaaa 1320
   tctttggata aaggttataa taaagctttg aatgatttgt gtattaaagt taataattgg 1380
   gatttgtttt tttctccatc tgaagataat tttactaatg atttgaataa aggtgaagaa 1440
   attacttctg atactaatat tgaagctgct gaagaaaata tttctttgga tttgattcaa 1500
   caatattatt tgacttttaa ttttgataat gaaccagaaa atatttctat tgaaaatttg 1560
   tcttctgata ttattggtca attggaattg atgccaaata ttgaaagatt tccaaatggt 1620
   aaaaaatatg aattggataa atatactatg tttcattatt tgagagctca agaatttgaa 1680
   catggtaaat ctagaattgc tttgactaat tctgttaatg aagctttgtt gaatccatct 1740
   agagtttata cttttttttc ttctgattat gttaaaaaag ttaataaagc tactgaagct 1800
   gctatgtttt tgggttgggt tgaacaattg gtttatgatt ttactgatga aacttctgaa 1860
   gtttctacta ctgataaaat tgctgatatt actattatta ttccatatat tggtccagct 1920
   ttgaatattg gtaatatgtt gtataaagat gattttgttg gtgctttgat tttttctggt 1980
   gctgttattt tgttggaatt tattccagaa attgctattc cagttttggg tacttttgct 2040
   ttggtttctt atattgctaa taaagttttg actgttcaaa ctattgataa tgctttgtct 2100
   aaaagaaatg aaaaatggga tgaagtttat aaatatattg ttactaattg gttggctaaa 2160
   gttaatactc aaattgattt gattagaaaa aaaatgaaag aagctttgga aaatcaagct 2220
   gaagctacta aagctattat taattatcaa tataatcaat atactgaaga agaaaaaaat 2280
   aatattaatt ttaatattga tgatttgtct tctaaattga atgaatctat taataaagct 2340
   atgattaata ttaataaatt tttgaatcaa tgttctgttt cttatttgat gaattctatg 2400
   attccatatg gtgttaaaag attggaagat tttgatgctt ctttgaaaga tgctttgttg 2460
   aaatatattt atgataatag aggtactttg attggtcaag ttgatagatt gaaagataaa 2520
   gttaataata ctttgtctac tgatattcca tttcaattgt ctaaatatgt tgataatcaa 2580
   agattgttgt ctacttttac tgaatatatt aaaaatatta ttaatacttc tattttgaat 2640
   ttgagatatg aatctaatca tttgattgat ttgtctagat atgcttctaa aattaatatt 2700
   ggttctaaag ttaattttga tccaattgat aaaaatcaaa ttcaattgtt taatttggaa 2760
   tcttctaaaa ttgaagttat tttgaaaaat gctattgttt ataattctat gtatgaaaat 2820
   ttttctactt ctttttggat tagaattcca aaatatttta attctatttc tttgaataat 2880
   gaatatacta ttattaattg tatggaaaat aattctggtt ggaaagtttc tttgaattat 2940
   ggtgaaatta tttggacttt gcaagatact caagaaatta aacaaagagt tgtttttaaa 3000
   tattctcaaa tgattaatat ttctgattat attaatagat ggatttttgt tactattact 3060
   aataatagat tgaataattc taaaatttat attaatggta gattgattga tcaaaaacca 3120
   atttctaatt tgggtaatat tcatgcttct aataatatta tgtttaaatt ggatggttgt 3180
   agagatactc atagatatat ttggattaaa tattttaatt tgtttgataa agaattgaat 3240
   gaaaaagaaa ttaaagattt gtatgataat caatctaatt ctggtatttt gaaagatttt 3300
   tggggtgatt atttgcaata tgataaacca tattatatgt tgaatttgta tgatccaaat 3360
   aaatatgttg atgttaataa tgttggtatt agaggttata tgtatttgaa aggtccaaga 3420
   ggttctgtta tgactactaa tatttatttg aattcttctt tgtatagagg tactaaattt 3480
   attattaaaa aatatgcttc tggtaataaa gataatattg ttagaaataa tgatagagtt 3540
   tatattaatg ttgttgttaa aaataaagaa tatagattgg ctactaatgc ttctcaagct 3600
   ggtgttgaaa aaattttgtc tgctttggaa attccagatg ttggtaattt gtctcaagtt 3660
   gttgttatga aatctaaaaa tgatcaaggt attactaata aatgtaaaat gaatttgcaa 3720
   gataataatg gtaatgatat tggttttatt ggttttcatc aatttaataa tattgctaaa 3780
   ttggttgctt ctaattggta taatagacaa attgaaagat cttctagaac tttgggttgt 3840
   tcttgggaat ttattccagt tgatgatggt tggggtgaaa gaccattgta a          3891
<210> 39
   <211> 3891
   <212> DNA
   <213> Artificial Sequence
<220>
   <223> BoNT/A, S. cerevisiae-modified 3
<400> 39
   atgccttttg ttaataaaca gttcaactat aaggatcccg tgaacggtgt agatattgcc 60
   tacattaaaa ttcctaacgc aggtcaaatg caacctgtca aggcttttaa gattcataat 120
   aaaatttggg tcatacctga aagggacacc tttacgaatc cagaggaagg tgacttgaac 180
   ccacctcccg aagctaaaca agtaccagta agttattacg attccaccta cttgtcaact 240
   gacaatgaaa aggataatta cttgaaagga gtcacgaagc ttttcgaaag aatttatagt 300
   acggatttag gccgtatgct gcttacttct atcgtaagag gtataccatt ttggggtgga 360
   agcacgatag atacagagct taaagtcatt gatactaact gcatcaacgt tatacaacca 420
   gacggttcat ataggtctga agagcttaat ttagttatca taggtccatc agccgacata 480
   attcaatttg aatgtaagag tttcggacac gaagtgttga atttgaccag aaacggctat 540
   ggaagtactc aatatatcag attcagccca gattttactt ttggttttga ggaatctttg 600
   gaagttgata ctaatccttt gcttggtgct ggcaagtttg caactgaccc agcagtaaca 660
   ttagcccacg aattgataca tgccggtcac aggctgtatg gtatcgctat taatccaaac 720
   agagtattca aagtgaatac caacgcttat tacgaaatgt ctggtcttga agtcagcttt 780
   gaagagttga gaactttcgg cggtcatgat gcaaaattta ttgatagcct gcaggagaat 840
   gagttccgtt tgtactatta caataagttt aaggatatag cctccacttt aaacaaagct 900
   aaatcaatcg ttggtactac cgccagtcta caatatatga agaatgtgtt taaagaaaag 960
   tatttactat ccgaggacac gtctggcaag tttagtgttg acaagttgaa atttgataag 1020
   ttatataaaa tgttaacaga aatttatacc gaggataact ttgtaaaatt tttcaaagtc 1080
   ttgaatagaa agacctactt gaatttcgat aaagctgtct tcaaaataaa tatcgttccc 1140
   aaagtcaact atactatata cgacggtttt aacttaagaa acactaattt agctgcaaac 1200
   ttcaacggtc aaaacaccga aatcaataac atgaatttca caaaattgaa gaatttcaca 1260
   ggattgttcg agttttataa attgctatgc gttagaggta tcattacatc caagactaaa 1320
   tctttagaca agggttacaa taaggcattg aatgatttat gtattaaagt gaacaattgg 1380
   gatttatttt tctccccatc agaggataac tttacaaacg atttaaataa aggtgaagag 1440
   ataacctctg acactaacat cgaagctgcc gaagagaaca tctctttaga tttgattcag 1500
   caatattacc ttacttttaa tttcgataat gaaccagaaa atatttccat agaaaactta 1560
   tcttcagata ttatcggtca acttgaatta atgcctaata tcgaaagatt tcccaatgga 1620
   aaaaagtacg aactggacaa atacacaatg tttcattatt tgagagctca agaatttgag 1680
   catggaaaat ccagaatagc tttgaccaac tctgtaaatg aagccctttt aaacccaagt 1740
   cgtgtctaca cctttttctc aagtgattac gttaagaaag ttaataaagc aaccgaagct 1800
   gcaatgttcc tgggttgggt ggaacaacta gtgtatgatt ttacggatga gacttccgaa 1860
   gtatccacta cggataaaat agctgacatc actattataa tcccatacat cggtccagca 1920
   ctgaatattg gtaacatgtt atataaggac gatttcgttg gtgctttgat cttctcaggc 1980
   gctgttattt tgcttgaatt tatccccgaa attgctattc ctgttctagg aacatttgct 2040
   ctagtatctt atattgctaa taaagtttta acagttcaaa cgatcgataa cgctctgtcc 2100
   aaaagaaatg aaaagtggga cgaggtatac aagtatatcg ttacaaattg gttagcaaaa 2160
   gttaataccc aaatcgactt gattcgtaaa aagatgaaag aggccttaga aaatcaggca 2220
   gaggctacta aggctattat caactatcaa tataatcagt acacagaaga ggaaaaaaac 2280
   aatattaact tcaatataga cgatttgtct tcgaaattaa atgaatccat taataaagct 2340
   atgatcaata ttaacaaatt cctaaatcag tgctcagttt cgtacttaat gaattctatg 2400
   attccttatg gcgtgaaaag gttggaggac ttcgatgctt ccttgaaaga tgcactatta 2460
   aaatacattt atgacaatag aggaactcta attggtcaag tggatagatt aaaggataaa 2520
   gtcaataaca ctctttcaac agatattcca tttcaattgt ctaaatatgt agataatcaa 2580
   cgtctgttat ccacgttcac agaatacata aagaatataa ttaacaccag tattttgaat 2640
   cttagatatg aatcaaatca tctaatagat ctgtcgagat acgcttcgaa gattaatata 2700
   ggctcgaaag ttaattttga tccaatcgat aagaaccaga tacaactttt taatctagaa 2760
   tcgtctaaaa tagaggtcat tttgaagaat gcaattgttt ataacagcat gtacgaaaat 2820
   ttctctacat ctttctggat tagaattcca aaatatttta acagtatttc tttgaacaat 2880
   gagtacacga tcattaattg tatggaaaat aacagcggct ggaaagtatc tctaaattat 2940
   ggcgaaatca tttggacatt acaagatacc caggaaatta agcagagagt tgtctttaaa 3000
   tacagtcaaa tgatcaatat cagcgattac attaaccgtt ggatttttgt gactataaca 3060
   aataacagac tgaacaatag caagatttat attaacggca gattgataga tcaaaaacct 3120
   atatcaaatt tgggaaacat ccatgcttca aataacatca tgttcaagct ggatggctgt 3180
   agagacactc atagatatat atggattaaa tatttcaact tgtttgataa agagctaaat 3240
   gaaaaggaaa taaaggatct ttatgacaac caatctaact ctggtatact gaaggacttt 3300
   tggggtgatt atctacaata tgataagcct tactatatgt taaacttgta cgaccccaat 3360
   aaatatgttg atgtcaataa cgtgggtata agaggttata tgtacttaaa aggtccaagg 3420
   ggatccgtga tgactacaaa tatttacttg aattcaagtt tatacagagg aacaaaattt 3480
   attatcaaga aatatgcatc aggaaataag gacaacattg ttaggaataa cgatagggtt 3540
   tacataaacg tcgttgtgaa aaataaagaa tatagattgg caacaaacgc ctcacaagcc 3600
   ggtgtagaaa aaatactttc tgcactggaa attccagacg tcggtaatct atcgcaagtt 3660
   gtggttatga aaagcaagaa tgatcaagga ataactaata agtgtaagat gaatctacag 3720
   gacaataacg gtaacgatat tggttttatt ggttttcacc agtttaataa cattgctaag 3780
   ttagttgcct caaattggta caataggcag atagaaagat cgtcaaggac attgggttgt 3840
   agttgggaat tcattcctgt agacgatggt tggggtgaaa gaccattgta a 3891
<210> 40
   <211> 3891
   <212> DNA
   <213> Artificial Sequence
<220>
   <223> BoNT/A, S. pombe-modified 1
<400> 40
   atgcctttcg ttaacaagca atttaactat aaagatcctg taaatggtgt tgatattgct 60
   tatattaaaa ttccgaatgc aggtcaaatg caaccggtta aagcttttaa aatacacaac 120
   aagatctggg ttattccaga aagagatact ttcactaacc ccgaagaggg ggatttgaat 180
   ccacccccag aggcgaagca ggtaccagta agttattacg actcaactta tttgtccact 240
   gataatgaaa aggataatta ccttaaaggt gtgaccaagc tgtttgagag aatttacagt 300
   actgatctag gtcgtatgtt acttacgtct attgtacgcg ggattccgtt ctggggggga 360
   tccacgatag atacagagtt gaaagttatt gacaccaact gtattaacgt gattcaacct 420
   gacggatctt atcgatcaga agagcttaat cttgttatta taggaccttc tgcagacatt 480
   atccaattcg aatgtaagag ttttggtcat gaggtcctca accttactag aaatggctat 540
   ggttctacac aatatatcag attttctcca gattttacgt tcggatttga agagagtctt 600
   gaagttgata ctaatccatt gttaggcgct ggaaaattcg ctacagatcc cgccgtaacc 660
   cttgctcatg aattaatcca tgcaggacac agattgtatg gtattgctat aaaccctaac 720
   cgtgtcttta aggttaatac aaatgcgtac tatgaaatgt ctggccttga ggtatcattc 780
   gaagagttgc gtactttcgg cggacatgat gctaaattca ttgatagctt acaggaaaac 840
   gaatttcgtt tgtattacta taataagttc aaggatattg cttcaacttt aaataaagct 900
   aaatctattg ttggaactac cgcttcattg caatatatga aaaatgtatt taaagaaaag 960
   tatcttttgt ccgaagacac ctctggtaaa ttttctgtag ataaattaaa gtttgataag 1020
   ctatacaaaa tgctcacgga aatatacact gaagataact ttgttaagtt tttcaaagtt 1080
   cttaatcgga aaacgtactt aaatttcgac aaagcagttt ttaaaatcaa tattgtacca 1140
   aaagttaatt atacaatcta tgatggtttc aacttgcgga atactaacct cgcagccaat 1200
   tttaatggtc aaaacaccga aattaataac atgaacttta ctaaacttaa gaattttacg 1260
   ggtctctttg aattctacaa attattgtgc gtccgtggga taatcacctc taaaactaaa 1320
   tccttggata aaggttataa taaagctctg aatgatttgt gcattaaagt taacaattgg 1380
   gatttgtttt tctctccttc cgaggacaat ttcactaatg atctcaacaa aggagaagag 1440
   attactagcg ataccaatat cgaagctgca gaagagaata tttcccttga tcttattcaa 1500
   cagtattacc ttacatttaa ctttgataac gagccagaaa atattagtat tgagaatctc 1560
   tcgagtgata ttatcggtca acttgaatta atgcctaata ttgagcgatt tcccaatggt 1620
   aagaaatacg aacttgataa gtacacaatg tttcattatt tacgcgcaca ggagtttgaa 1680
   catggtaagt cacgaattgc gttgactaat agtgtcaatg aagcattgtt aaatcctagc 1740
   cgagtgtata catttttcag ctctgactat gttaagaaag tgaataaggc aacagaggct 1800
   gcaatgtttt taggttgggt ggaacaatta gtctacgatt tcacagatga aacttctgaa 1860
   gtttctacca cagacaaaat agccgatatt accattataa ttccttatat tggccctgcc 1920
   ttgaatatag gtaatatgtt atataaagac gattttgtcg gcgcattaat tttttcaggc 1980
   gcagttatct tattggagtt tattcctgaa atcgctatac ctgttttagg tacttttgct 2040
   ttagtgagtt atatagctaa taaggttctt acggttcaga ctattgacaa cgctctttct 2100
   aaacgtaatg aaaaatggga tgaggtctac aaatacatag tgactaattg gctagctaag 2160
   gtcaatactc agatcgacct aataagaaag aaaatgaaag aagccttaga gaatcaagct 2220
   gaagccacta aagcaattat aaactatcaa tataatcagt atacagaaga ggaaaaaaat 2280
   aacataaact tcaacattga tgacttaagc tctaaactga atgaaagcat taacaaagcg 2340
   atgattaata ttaacaaatt tctcaatcaa tgttcagttt cgtatttgat gaacagtatg 2400
   atcccgtatg gtgttaaacg tctagaagac tttgatgcct cattaaaaga tgccttgctt 2460
   aaatatattt acgataatcg aggaactcta ataggtcagg ttgatcggct gaaagacaaa 2520
   gttaataaca ccctctctac tgatatcccc ttccaattgt cgaaatacgt tgataaccag 2580
   cgtcttttat cgacatttac tgaatacatt aaaaatatta tcaatacttc tattttgaac 2640
   cttcgttatg aaagcaatca tttgatcgat ttgtcacgct atgcttcaaa gatcaacatc 2700
   ggatctaaag tcaactttga tcctattgat aaaaatcaga ttcaattatt taaccttgaa 2760
   tcatctaaga tcgaagtaat ccttaagaat gctattgttt ataattcaat gtatgaaaat 2820
   ttttctactt cgttttggat taggattcca aagtacttta attccatctc tctgaacaat 2880
   gaatatacaa tcataaattg tatggaaaat aactctggct ggaaggtatc cctaaattat 2940
   ggagaaataa tttggacctt acaagatact caagagatta aacaaagagt tgtgtttaaa 3000
   tattcgcaaa tgattaacat ttctgattat ataaatagat ggatttttgt taccataaca 3060
   aataaccgtt tgaataactc caagatttac attaatggcc gtttgattga tcaaaagcca 3120
   attagcaatc tgggcaacat ccatgcttct aataacataa tgtttaaact ggacggatgt 3180
   agagatactc accgatacat ttggatcaag tattttaatt tatttgataa agaacttaat 3240
   gaaaaagaaa taaaggactt atatgataat caatctaatt caggtattct aaaggatttt 3300
   tggggtgact atttgcaata tgacaaacct tattacatgt taaatcttta tgatcccaat 3360
   aaatatgtcg atgtcaataa cgttgggata aggggttaca tgtatctaaa agggccccgc 3420
   ggatccgtaa tgacaaccaa catttatctt aattcatcgt tatatcgtgg tacaaaattt 3480
   ataattaaaa agtatgctag tggtaacaag gataacattg tgcgaaataa cgaccgcgtt 3540
   tacattaacg tcgttgtaaa gaataaagaa tacagacttg ctacgaatgc tagtcaggcc 3600
   ggtgtagaaa agattttaag tgctctggag atccctgatg tcggcaattt gagtcaagtt 3660
   gtagttatga agtcaaaaaa tgaccaaggt ataacaaata agtgtaaaat gaatcttcaa 3720
   gataataatg gaaatgacat tggatttatt ggatttcatc aatttaataa tattgcaaag 3780
   cttgttgcca gtaattggta caataggcaa atagagaggt cttcccgtac actaggatgc 3840
   tcttgggaat ttattcctgt ggacgatgga tggggagaac gtcctttata a 3891
<210> 41
   <211> 3891
   <212> DNA
   <213> Artificial Sequence
<220>
   <223> BoNT/A, S. pombe-modified 2
<400> 41
   atgccttttg ttaataaaca atttaattat aaagatcctg ttaatggtgt tgatattgct 60
   tatattaaaa ttcctaatgc tggtcaaatg caacctgtta aagcttttaa aattcataat 120
   aaaatttggg ttattcctga acgtgatact tttactaatc ctgaagaagg tgatttaaat 180
   cctcctcctg aagctaaaca agttcctgtt tcttattatg attctactta tttatctact 240
   gataatgaaa aagataatta tttaaaaggt gttactaaat tatttgaacg tatttattct 300
   actgatttag gtcgtatgtt attaacttct attgttcgtg gtattccttt ttggggtggt 360
   tctactattg atactgaatt aaaagttatt gatactaatt gtattaatgt tattcaacct 420
   gatggttctt atcgttctga agaattaaat ttagttatta ttggtccttc tgctgatatt 480
   attcaatttg aatgtaaatc ttttggtcat gaagttttaa atttaactcg taatggttat 540
   ggttctactc aatatattcg tttttctcct gattttactt ttggttttga agaatcttta 600
   gaagttgata ctaatccttt attaggtgct ggtaaatttg ctactgatcc tgctgttact 660
   ttagctcatg aattaattca tgctggtcat cgtttatatg gtattgctat taatcctaat 720
   cgtgttttta aagttaatac taatgcttat tatgaaatgt ctggtttaga agtttctttt 780
   gaagaattac gtacttttgg tggtcatgat gctaaattta ttgattcttt acaagaaaat 840
   gaatttcgtt tatattatta taataaattt aaagatattg cttctacttt aaataaagct 900
   aaatctattg ttggtactac tgcttcttta caatatatga aaaatgtttt taaagaaaaa 960
   tatttattat ctgaagatac ttctggtaaa ttttctgttg ataaattaaa atttgataaa 1020
   ttatataaaa tgttaactga aatttatact gaagataatt ttgttaaatt ttttaaagtt 1080
   ttaaatcgta aaacttattt aaattttgat aaagctgttt ttaaaattaa tattgttcct 1140
   aaagttaatt atactattta tgatggtttt aatttacgta atactaattt agctgctaat 1200
   tttaatggtc aaaatactga aattaataat atgaatttta ctaaattaaa aaattttact 1260
   ggtttatttg aattttataa attattatgt gttcgtggta ttattacttc taaaactaaa 1320
   tctttagata aaggttataa taaagcttta aatgatttat gtattaaagt taataattgg 1380
   gatttatttt tttctccttc tgaagataat tttactaatg atttaaataa aggtgaagaa 1440
   attacttctg atactaatat tgaagctgct gaagaaaata tttctttaga tttaattcaa 1500
   caatattatt taacttttaa ttttgataat gaacctgaaa atatttctat tgaaaattta 1560
   tcttctgata ttattggtca attagaatta atgcctaata ttgaacgttt tcctaatggt 1620
   aaaaaatatg aattagataa atatactatg tttcattatt tacgtgctca agaatttgaa 1680
   catggtaaat ctcgtattgc tttaactaat tctgttaatg aagctttatt aaatccttct 1740
   cgtgtttata cttttttttc ttctgattat gttaaaaaag ttaataaagc tactgaagct 1800
   gctatgtttt taggttgggt tgaacaatta gtttatgatt ttactgatga aacttctgaa 1860
   gtttctacta ctgataaaat tgctgatatt actattatta ttccttatat tggtcctgct 1920
   ttaaatattg gtaatatgtt atataaagat gattttgttg gtgctttaat tttttctggt 1980
   gctgttattt tattagaatt tattcctgaa attgctattc ctgttttagg tacttttgct 2040
   ttagtttctt atattgctaa taaagtttta actgttcaaa ctattgataa tgctttatct 2100
   aaacgtaatg aaaaatggga tgaagtttat aaatatattg ttactaattg gttagctaaa 2160
   gttaatactc aaattgattt aattcgtaaa aaaatgaaag aagctttaga aaatcaagct 2220
   gaagctacta aagctattat taattatcaa tataatcaat atactgaaga agaaaaaaat 2280
   aatattaatt ttaatattga tgatttatct tctaaattaa atgaatctat taataaagct 2340
   atgattaata ttaataaatt tttaaatcaa tgttctgttt cttatttaat gaattctatg 2400
   attccttatg gtgttaaacg tttagaagat tttgatgctt ctttaaaaga tgctttatta 2460
   aaatatattt atgataatcg tggtacttta attggtcaag ttgatcgttt aaaagataaa 2520
   gttaataata ctttatctac tgatattcct tttcaattat ctaaatatgt tgataatcaa 2580
   cgtttattat ctacttttac tgaatatatt aaaaatatta ttaatacttc tattttaaat 2640
   ttacgttatg aatctaatca tttaattgat ttatctcgtt atgcttctaa aattaatatt 2700
   ggttctaaag ttaattttga tcctattgat aaaaatcaaa ttcaattatt taatttagaa 2760
   tcttctaaaa ttgaagttat tttaaaaaat gctattgttt ataattctat gtatgaaaat 2820
   ttttctactt ctttttggat tcgtattcct aaatatttta attctatttc tttaaataat 2880
   gaatatacta ttattaattg tatggaaaat aattctggtt ggaaagtttc tttaaattat 2940
   ggtgaaatta tttggacttt acaagatact caagaaatta aacaacgtgt tgtttttaaa 3000
   tattctcaaa tgattaatat ttctgattat attaatcgtt ggatttttgt tactattact 3060
   aataatcgtt taaataattc taaaatttat attaatggtc gtttaattga tcaaaaacct 3120
   atttctaatt taggtaatat tcatgcttct aataatatta tgtttaaatt agatggttgt 3180
   cgtgatactc atcgttatat ttggattaaa tattttaatt tatttgataa agaattaaat 3240
   gaaaaagaaa ttaaagattt atatgataat caatctaatt ctggtatttt aaaagatttt 3300
   tggggtgatt atttacaata tgataaacct tattatatgt taaatttata tgatcctaat 3360
   aaatatgttg atgttaataa tgttggtatt cgtggttata tgtatttaaa aggtcctcgt 3420
   ggttctgtta tgactactaa tatttattta aattcttctt tatatcgtgg tactaaattt 3480
   attattaaaa aatatgcttc tggtaataaa gataatattg ttcgtaataa tgatcgtgtt 3540
   tatattaatg ttgttgttaa aaataaagaa tatcgtttag ctactaatgc ttctcaagct 3600
   ggtgttgaaa aaattttatc tgctttagaa attcctgatg ttggtaattt atctcaagtt 3660
   gttgttatga aatctaaaaa tgatcaaggt attactaata aatgtaaaat gaatttacaa 3720
   gataataatg gtaatgatat tggttttatt ggttttcatc aatttaataa tattgctaaa 3780
   ttagttgctt ctaattggta taatcgtcaa attgaacgtt cttctcgtac tttaggttgt 3840
   tcttgggaat ttattcctgt tgatgatggt tggggtgaac gtcctttata a 3891
<210> 42
   <211> 3891
   <212> DNA
   <213> Artificial Sequence
<220>
   <223> BoNT/A, S. pombe-modified 3
<400> 42
   atgccatttg ttaataaaca attcaattat aaagaccctg taaatggtgt ggacattgct 60
   tatatcaaaa ttcctaacgc aggacaaatg caacccgtta aagcttttaa gatccataat 120
   aagatttggg tcattcctga gagggacact ttcaccaatc ccgaggaagg tgacttgaat 180
   cctccacctg aagctaaaca agtgcctgta tcatattacg atagtacata tctatctact 240
   gacaatgaaa aagataacta tcttaagggt gtcactaagc tatttgaacg tatttactcc 300
   accgacttag gacgaatgtt gcttacaagt attgttagag gaattccatt ttggggtgga 360
   tctactattg acactgagtt aaaagttatt gatacaaatt gtataaatgt gattcagccc 420
   gacggttcat atagatctga agagttgaat cttgtaatta taggcccttc tgccgatata 480
   attcaatttg aatgtaagtc ttttggtcat gaagttctta atttaacacg taatggatac 540
   ggctcaactc aatacattcg tttttctcct gattttactt tcggctttga agagtcttta 600
   gaggtggata caaacccttt acttggagct ggaaagttcg ctacagatcc tgccgttact 660
   ttagcacatg agttaatcca tgctggtcac cgcttatacg gtattgcaat aaatcctaat 720
   cgtgttttta aagttaatac aaatgcctat tacgaaatgt ccggattaga agtatctttc 780
   gaggaattgc gaacttttgg aggtcatgat gccaaattca ttgattctct acaggaaaac 840
   gaatttcgtt tatactatta caacaagttt aaagatattg cttccacttt gaataaagct 900
   aaatccattg taggaactac agcctcatta caatatatga aaaacgtttt caaagaaaag 960
   tatcttttaa gcgaggatac cagcggtaaa tttagcgtag ataaacttaa attcgacaag 1020
   ttgtataaga tgcttactga aatctatact gaggacaact tcgtcaaatt ctttaaagtc 1080
   ttgaaccgta agacctattt aaacttcgat aaggctgtct tcaaaattaa tattgttccc 1140
   aaagttaatt atactatata cgatggtttt aacttaagga acactaactt agctgcaaat 1200
   ttcaatggtc agaacacaga aataaataac atgaacttta caaagttaaa aaattttaca 1260
   ggtttgtttg aattttacaa acttttatgc gttcgaggta ttatcacaag taaaaccaag 1320
   tctttagata aaggttataa taaagctctt aatgatcttt gcattaaggt taacaattgg 1380
   gacttgtttt tctcacccag tgaagataac ttcaccaatg atctaaacaa aggcgaagag 1440
   ataaccagtg acacaaatat agaagctgca gaagagaata tatccttaga tcttatccaa 1500
   cagtattact tgactttcaa ctttgacaat gaaccagaaa acatttcaat tgagaacttg 1560
   tctagtgata ttataggaca attagaactt atgcctaata ttgaacgttt tccaaatggc 1620
   aagaaatacg agttagataa atatacaatg tttcactatt tacgtgctca agaatttgag 1680
   catggaaagt ctcgtattgc tttaactaat tctgttaatg aagctttact aaatccatca 1740
   agagtttata catttttcag ttctgattat gtcaaaaagg tgaataaagc tactgaagcc 1800
   gctatgtttt taggctgggt tgagcaattg gtctatgatt ttaccgatga aactagtgaa 1860
   gtttccacta ccgataagat agctgatatc actataatca taccatatat tggtcccgct 1920
   ttaaatatag gaaatatgct atacaaagat gactttgttg gagcattaat ttttagtggt 1980
   gctgttattc ttttagaatt tatccctgaa attgcaatcc ctgttttagg aacctttgct 2040
   cttgtttcat atattgctaa taaggtactt actgttcaga ctatagacaa tgcactttct 2100
   aaacgtaacg aaaaatggga tgaagtttac aaatacatcg ttacaaattg gttagcaaag 2160
   gtaaatactc aaattgattt aatcagaaaa aagatgaaag aagctcttga aaatcaggca 2220
   gaagcaacta aagccattat aaactatcaa tataatcaat ataccgagga agagaaaaac 2280
   aatataaact ttaatattga tgacttatcc tctaaattaa atgaatcaat caataaagcc 2340
   atgattaata taaataaatt tcttaaccaa tgcagtgtat cttatttgat gaactcaatg 2400
   atcccttatg gagtgaagcg attagaagac tttgatgctt ccttgaagga tgcattgtta 2460
   aagtatattt acgataatcg aggaactttg atcggtcaag ttgatcgctt aaaagataag 2520
   gtcaacaata ctttaagcac tgatatccct tttcagttaa gtaaatacgt tgataatcag 2580
   agacttttgt ctacttttac cgaatatatt aagaatatta tcaatacttc gattttgaat 2640
   ttaagatatg aaagtaacca tcttatagat ttatcgcgtt acgcctcgaa aattaacatt 2700
   ggatctaaag tcaatttgga tcctattgat aaaaaccaaa ttcagctatt caatcttgag 2760
   tcttcaaaga ttgaggtaat tttgaaaaat gctattgttt acaattcaat gtatgaaaat 2820
   ttttctactt cattttggat tcgtattccc aagtacttta attcgatctc tcttaacaat 2880
   gaatatacca ttatcaactg tatggaaaat aactctggct ggaaagtatc attgaactat 2940
   ggagaaatta tatggactct tcaagatact caagaaatta aacaaagagt agtctttaaa 3000
   tattcacaaa tgattaacat ttccgattat attaatcgtt ggatttttgt gactatcaca 3060
   aataaccgtc taaataactc caagatttat ataaatggtc gcttaattga tcaaaaacct 3120
   ataagtaact tgggtaatat tcatgcttcc aataacataa tgtttaagct tgatggttgt 3180
   cgtgacactc acaggtatat ctggattaaa tattttaatc tttttgataa agaattgaat 3240
   gaaaaagaaa ttaaggatct ttatgataat caatctaatt cgggcatctt gaaagatttt 3300
   tggggtgact acttgcaata tgataagcct tattacatgt taaacttgta tgatcctaac 3360
   aaatatgtag atgttaataa cgtaggcatt agaggttaca tgtaccttaa aggtccaaga 3420
   ggtagtgtta tgactacaaa tatatatctt aactcatctt tatacagagg cactaagttc 3480
   atcattaaga aatatgctag cggtaacaaa gataatatcg tccgcaacaa tgaccgagtt 3540
   tacataaatg tggttgtgaa aaataaggaa tatcgcttgg ccaccaatgc ttctcaagca 3600
   ggtgtcgaaa agattttgag cgctttagag atcccagatg ttggtaactt gagccaagtc 3660
   gttgtgatga aaagcaagaa tgatcaaggt attacaaata agtgtaaaat gaatctacag 3720
   gacaataatg gtaatgacat tggattcatt ggttttcatc agtttaataa tattgctaaa 3780
   ctagttgcat cgaattggta taatcgtcaa attgagcgat cttcgcgtac tttaggttgt 3840
   tcttgggagt ttatacctgt agatgacggt tggggcgaga ggccattata a          3891
<210> 43
   <211> 3891
   <212> DNA
   <213> Artificial Sequence
<220>
   <223> BoNT/A, Y. lipolytica-modified 1
<400> 43
   atgcctttcg tgaataaaca attcaactac aaggatcctg tcaacggcgt cgacattgct 60
   tatattaaga ttcccaacgc tggccagatg cagccggtga aagctttcaa gatccacaat 120
   aagatttggg tgatcccaga gcgggatacc tttactaatc ccgaggaagg agacctgaac 180
   ccccctccgg aggctaaaca ggtccctgtc tcctattacg actcaaccta tctgtcgact 240
   gacaacgaga aagataatta cctgaaggga gtcaccaaac tgttcgagcg catctactcg 300
   accgacttag ggcgaatgct acttacttct attgtccgag ggataccatt ttggggcggt 360
   tcaacaattg acacagagct caaagtcatt gacacgaact gtattaacgt tattcagccc 420
   gatgggtctt accgaagcga agagttgaac ctggttatca ttggtccctc cgccgatatt 480
   atccagttcg agtgtaagtc ttttggtcat gaggtcctga acttgacccg taacggatac 540
   ggttcgaccc agtacatccg tttctcccca gattttactt tcggctttga ggaaagtctg 600
   gaagttgata cgaacccttt gctcggagcc ggcaaattcg caaccgaccc tgctgtcacc 660
   ctcgcacatg agctcattca cgctggccac aggctttacg gaattgctat taaccctaac 720
   cgagttttca aggtcaacac aaacgcctat tacgaaatgt ccggactgga agtgtcgttt 780
   gaagagctgc ggacctttgg aggtcatgat gctaagttta tcgatagcct gcaggagaac 840
   gaattccgac tttactatta caacaaattc aaggacatcg cctcgactct gaacaaggcc 900
   aaatccatcg tgggtaccac ggctagtctg cagtatatga agaacgtgtt caaggagaag 960
   tacctcctta gtgaggacac ttcgggaaag ttctctgttg ataaactgaa gtttgacaag 1020
   ctgtacaaga tgctcactga gatttacacc gaagacaatt tcgtaaagtt tttcaaggtg 1080
   ctcaaccgta agacctacct caactttgac aaagctgtgt ttaaaattaa tatcgtcccc 1140
   aaggtgaact acactattta cgacggtttc aatctccgaa acaccaatct cgcagcgaac 1200
   ttcaacggac agaacacaga gatcaacaat atgaacttta ccaagttgaa gaacttcacc 1260
   ggtctatttg agttctacaa gctgctctgc gtgcggggta ttatcacctc caagactaag 1320
   agcctcgaca agggctacaa caaggccttg aacgacttgt gtatcaaggt gaacaattgg 1380
   gacctgttct tttcaccctc cgaggataac ttcacgaacg acctgaataa gggtgaagag 1440
   attacatccg atacgaacat cgaagcagct gaggaaaata tctctctcga tctgattcag 1500
   caatactatc ttaccttcaa cttcgacaac gagcctgaga acatttctat cgagaacctg 1560
   agttctgata ttataggtca gctcgagctg atgcccaaca tcgagagatt tcccaacggc 1620
   aagaaatacg agcttgacaa gtacactatg tttcactacc tgcgagccca ggagttcgag 1680
   cacggcaagt cgcgaatcgc actaaccaac tctgtgaatg aggctctgct caatccgtct 1740
   cgagtgtaca ctttcttttc tagcgactac gtgaagaaag tgaataaggc gacagaagca 1800
   gctatgtttc tcgggtgggt ggagcagctg gtgtacgact ttacggacga gacctcggaa 1860
   gtgtccacca ctgacaagat tgccgacatc acaatcatta tcccttacat tggccccgcc 1920
   ctcaacatcg gcaatatgct ctacaaggat gacttcgtcg gtgcgctaat cttctctggc 1980
   gccgttatcc tgcttgagtt cattcccgag attgccatcc cggtgctggg aacttttgct 2040
   ctagtcagtt atatcgcaaa caaggtcctg actgttcaga ccatcgacaa cgccctctct 2100
   aagcgcaacg agaagtggga tgaggtctac aagtatattg ttaccaactg gttggctaag 2160
   gttaacactc aaattgacct cattcggaag aaaatgaagg aggccttgga gaaccaggcc 2220
   gaggcaacta aggccattat caattatcaa tacaaccaat acacagagga agagaagaac 2280
   aatattaatt ttaacatcga cgatctgtcc tctaagctca acgagtccat taacaaggcg 2340
   atgatcaaca tcaataagtt tctgaaccag tgctccgtga gctaccttat gaactcgatg 2400
   attccctacg gagttaagcg actggaggac tttgatgctt ccttaaagga cgctctgctt 2460
   aagtacatct acgacaatcg tggcacactg atcggtcagg tggatcgact gaaggacaag 2520
   gttaacaata cgctgtcgac ggacatccca ttccagcttt caaagtacgt cgacaaccag 2580
   cgcttgctat cgacctttac cgagtacatc aagaatatca ttaacacctc tatccttaat 2640
   ctgagatacg aatccaacca tctcatcgac ttgtcccgat acgcctccaa gattaacatt 2700
   ggttctaagg ttaactttga tcccatcgac aagaaccaga tacagctatt caatctggag 2760
   agcagtaaaa tcgaagtcat cttgaagaac gccatagtgt acaactctat gtatgagaac 2820
   ttctcaacgt cgttctggat acggatccca aaatacttca actctatttc cctcaacaat 2880
   gaatatacca ttatcaactg catggagaac aatagcggat ggaaggtgtc acttaactac 2940
   ggcgaaatca tttggaccct gcaggacaca caggagatca agcaaagagt cgtgttcaag 3000
   tacagccaga tgattaacat ttcggactat atcaaccgtt ggattttcgt caccatcacc 3060
   aacaatagac tgaacaatag taagatctac attaacggta gacttatcga tcaaaaacct 3120
   atctctaacc tgggaaatat ccatgcttca aacaatatta tgtttaagct cgacggctgc 3180
   cgagacactc accggtatat ttggattaag tacttcaacc tgttcgataa agaactgaac 3240
   gagaaggaga ttaaagatct ctacgacaac cagtctaact cgggtatcct gaaagatttc 3300
   tggggagact atctccaata tgataagccc tactatatgc tgaaccttta cgatcctaac 3360
   aaatacgtgg acgtgaacaa cgtcggaatt cggggctaca tgtaccttaa gggacctagg 3420
   ggctccgtaa tgacgactaa catctacctt aactccagcc tctaccgagg tacaaagttc 3480
   attatcaaga agtacgcctc tggcaacaag gacaacattg tacgaaacaa cgaccgagta 3540
   tacatcaacg ttgtcgttaa gaacaaggag taccgccttg cgactaacgc ctctcaggcg 3600
   ggagtcgaga agattctctc cgccctggaa attcccgacg tcggcaacct ctcgcaggtt 3660
   gtggttatga agtccaagaa cgatcagggc attaccaaca agtgcaagat gaacctgcaa 3720
   gacaacaacg gcaacgacat cggattcatc ggatttcacc agtttaacaa catcgccaag 3780
   ttggttgcct cgaactggta caaccgacag attgagcgat cttcaagaac attgggctgt 3840
   agctgggaat tcatccccgt ggatgacgga tggggagaga gaccgttgta a 3891
<210> 44
   <211> 3891
   <212> DNA
   <213> Artificial Sequence
<220>
   <223> BoNT/A, Y. lipolytica-modified 2
<400> 44
   atgcccttcg tgaacaagca gttcaactac aaggaccccg tgaacggcgt ggacatcgcc 60
   tacatcaaga tccccaacgc cggccagatg cagcccgtga aggccttcaa gatccacaac 120
   aagatctggg tgatccccga gcgagacacc ttcaccaacc ccgaggaggg cgacctgaac 180
   cccccccccg aggccaagca ggtgcccgtg tcttactacg actctaccta cctgtctacc 240
   gacaacgaga aggacaacta cctgaagggc gtgaccaagc tgttcgagcg aatctactct 300
   accgacctgg gccgaatgct gctgacctct atcgtgcgag gcatcccctt ctggggcggc 360
   tctaccatcg acaccgagct gaaggtgatc gacaccaact gcatcaacgt gatccagccc 420
   gacggctctt accgatctga ggagctgaac ctggtgatca tcggcccctc tgccgacatc 480
   atccagttcg agtgcaagtc tttcggccac gaggtgctga acctgacccg aaacggctac 540
   ggctctaccc agtacatccg attctctccc gacttcacct tcggcttcga ggagtctctg 600
   gaggtggaca ccaaccccct gctgggcgcc ggcaagttcg ccaccgaccc cgccgtgacc 660
   ctggcccacg agctgatcca cgccggccac cgactgtacg gcatcgccat caaccccaac 720
   cgagtgttca aggtgaacac caacgcctac tacgagatgt ctggcctgga ggtgtctttc 780
   gaggagctgc gaaccttcgg cggccacgac gccaagttca tcgactctct gcaggagaac 840
   gagttccgac tgtactacta caacaagttc aaggacatcg cctctaccct gaacaaggcc 900
   aagtctatcg tgggcaccac cgcctctctg cagtacatga agaacgtgtt caaggagaag 960
   tacctgctgt ctgaggacac ctctggcaag ttctctgtgg acaagctgaa gttcgacaag 1020
   ctgtacaaga tgctgaccga gatctacacc gaggacaact tcgtgaagtt cttcaaggtg 1080
   ctgaaccgaa agacctacct gaacttcgac aaggccgtgt tcaagatcaa catcgtgccc 1140
   aaggtgaact acaccatcta cgacggcttc aacctgcgaa acaccaacct ggccgccaac 1200
   ttcaacggcc agaacaccga gatcaacaac atgaacttca ccaagctgaa gaacttcacc 1260
   ggcctgttcg agttctacaa gctgctgtgc gtgcgaggca tcatcacctc taagaccaag 1320
   tctctggaca agggctacaa caaggccctg aacgacctgt gcatcaaggt gaacaactgg 1380
   gacctgttct tctctccctc tgaggacaac ttcaccaacg acctgaacaa gggcgaggag 1440
   atcacctctg acaccaacat cgaggccgcc gaggagaaca tctctctgga cctgatccag 1500
   cagtactacc tgaccttcaa cttcgacaac gagcccgaga acatctctat cgagaacctg 1560
   tcttctgaca tcatcggcca gctggagctg atgcccaaca tcgagcgatt ccccaacggc 1620
   aagaagtacg agctggacaa gtacaccatg ttccactacc tgcgagccca ggagttcgag 1680
   cacggcaagt ctcgaatcgc cctgaccaac tctgtgaacg aggccctgct gaacccctct 1740
   cgagtgtaca ccttcttctc ttctgactac gtgaagaagg tgaacaaggc caccgaggcc 1800
   gccatgttcc tgggctgggt ggagcagctg gtgtacgact tcaccgacga gacctctgag 1860
   gtgtctacca ccgacaagat cgccgacatc accatcatca tcccctacat cggccccgcc 1920
   ctgaacatcg gcaacatgct gtacaaggac gacttcgtgg gcgccctgat cttctctggc 1980
   gccgtgatcc tgctggagtt catccccgag atcgccatcc ccgtgctggg caccttcgcc 2040
   ctggtgtctt acatcgccaa caaggtgctg accgtgcaga ccatcgacaa cgccctgtct 2100
   aagcgaaacg agaagtggga cgaggtgtac aagtacatcg tgaccaactg gctggccaag 2160
   gtgaacaccc agatcgacct gatccgaaag aagatgaagg aggccctgga gaaccaggcc 2220
   gaggccacca aggccatcat caactaccag tacaaccagt acaccgagga ggagaagaac 2280
   aacatcaact tcaacatcga cgacctgtct tctaagctga acgagtctat caacaaggcc 2340
   atgatcaaca tcaacaagtt cctgaaccag tgctctgtgt cttacctgat gaactctatg 2400
   atcccctacg gcgtgaagcg actggaggac ttcgacgcct ctctgaagga cgccctgctg 2460
   aagtacatct acgacaaccg aggcaccctg atcggccagg tggaccgact gaaggacaag 2520
   gtgaacaaca ccctgtctac cgacatcccc ttccagctgt ctaagtacgt ggacaaccag 2580
   cgactgctgt ctaccttcac cgagtacatc aagaacatca tcaacacctc tatcctgaac 2640
   ctgcgatacg agtctaacca cctgatcgac ctgtctcgat acgcctctaa gatcaacatc 2700
   ggctctaagg tgaacttcga ccccatcgac aagaaccaga tccagctgtt caacctggag 2760
   tcttctaaga tcgaggtgat cctgaagaac gccatcgtgt acaactctat gtacgagaac 2820
   ttctctacct ctttctggat ccgaatcccc aagtacttca actctatctc tctgaacaac 2880
   gagtacacca tcatcaactg catggagaac aactctggct ggaaggtgtc tctgaactac 2940
   ggcgagatca tctggaccct gcaggacacc caggagatca agcagcgagt ggtgttcaag 3000
   tactctcaga tgatcaacat ctctgactac atcaaccgat ggatcttcgt gaccatcacc 3060
   aacaaccgac tgaacaactc taagatctac atcaacggcc gactgatcga ccagaagccc 3120
   atctctaacc tgggcaacat ccacgcctct aacaacatca tgttcaagct ggacggctgc 3180
   cgagacaccc accgatacat ctggatcaag tacttcaacc tgttcgacaa ggagctgaac 3240
   gagaaggaga tcaaggacct gtacgacaac cagtctaact ctggcatcct gaaggacttc 3300
   tggggcgact acctgcagta cgacaagccc tactacatgc tgaacctgta cgaccccaac 3360
   aagtacgtgg acgtgaacaa cgtgggcatc cgaggctaca tgtacctgaa gggcccccga 3420
   ggctctgtga tgaccaccaa catctacctg aactcttctc tgtaccgagg caccaagttc 3480
   atcatcaaga agtacgcctc tggcaacaag gacaacatcg tgcgaaacaa cgaccgagtg 3540
   tacatcaacg tggtggtgaa gaacaaggag taccgactgg ccaccaacgc ctctcaggcc 3600
   ggcgtggaga agatcctgtc tgccctggag atccccgacg tgggcaacct gtctcaggtg 3660
   gtggtgatga agtctaagaa cgaccagggc atcaccaaca agtgcaagat gaacctgcag 3720
   gacaacaacg gcaacgacat cggcttcatc ggcttccacc agttcaacaa catcgccaag 3780
   ctggtggcct ctaactggta caaccgacag atcgagcgat cttctcgaac cctgggctgc 3840
   tcttgggagt tcatccccgt ggacgacggc tggggcgagc gacccctgta a          3891
<210> 45
   <211> 3891
   <212> DNA
   <213> Artificial Sequence
<220>
   <223> BoNT/A, Y. lipolytica-modified 3
<400> 45
   atgcccttcg tcaacaagca gtttaactac aaggaccccg tgaacggcgt tgatatcgct 60
   tacatcaaga ttcccaacgc tggccagatg cagcccgtta aggcctttaa gatccacaac 120
   aagatctggg ttatccccga acgggatacc tttacaaacc ctgaggaagg agacctgaac 180
   cctcccccag aggctaagca ggtgccggtg tcctactacg attctaccta cctgtctacc 240
   gataacgaga aggacaacta cctgaagggc gttaccaagc tcttcgaacg aatttactcg 300
   acggatctgg gccggatgct gcttaccagc attgtgcgag gcattccttt ttggggtgga 360
   agcaccatcg acactgagct gaaggtgatt gacacaaact gcatcaacgt tatccagccc 420
   gacggctctt accgatctga agagcttaac ctcgtcatca ttggaccttc ggctgatatc 480
   attcagttcg agtgcaagtc cttcggccac gaagtgctta acctgacacg aaacggttac 540
   ggttccactc agtacattag attttcaccg gactttacct tcggatttga ggaatcgctc 600
   gaggtggata ccaaccctct ccttggagcc ggaaagttcg caacagaccc tgccgtcact 660
   ctggctcatg agctgatcca cgctggtcac agactttacg gcattgccat taaccccaac 720
   cgagtcttta aggtgaacac caacgcttac tacgagatgt caggattgga ggtttcgttc 780
   gaggaactcc gaacctttgg tggacacgac gctaagttca ttgacagcct gcaggagaac 840
   gaatttcggc tttactacta caacaagttt aaggacatcg cctccacact gaacaaggca 900
   aagtccatcg tgggtaccac agcctccctg cagtacatga agaacgtgtt caaggagaag 960
   tacttgctgt cggaagacac atccggcaag ttttctgtgg acaagctgaa gttcgacaag 1020
   ctctacaaga tgctgaccga gatttacact gaggacaact tcgtgaagtt ctttaaggtg 1080
   ctcaaccgta agacttacct taacttcgac aaggccgtgt tcaagatcaa catcgtcccc 1140
   aaggtgaact acactatcta cgacggattt aacctgcgta acacgaacct cgccgctaac 1200
   ttcaacggac agaacaccga gattaacaac atgaacttca ccaagctgaa gaactttact 1260
   ggtctgttcg agttttacaa gttgctttgt gttcggggta tcattacgtc gaagacgaag 1320
   tccctggaca agggttacaa caaggccctc aacgacctct gcatcaaggt caacaactgg 1380
   gacctctttt tctctccatc cgaggacaac ttcactaacg acctcaacaa gggcgaagag 1440
   atcacttcgg acaccaacat cgaggccgct gaagagaaca tctctctgga tcttattcag 1500
   cagtactact tgacctttaa ctttgataac gagccggaga acatttccat cgaaaacttg 1560
   agctccgata ttatcggcca gctggaactg atgccaaaca ttgagcgatt ccctaacggc 1620
   aagaagtacg agctcgacaa gtacactatg tttcactacc ttagagctca ggagtttgag 1680
   cacggcaagt ctcgaatcgc tctcactaac tctgtgaacg aggccctgct caacccctct 1740
   cgagtctaca ctttcttttc atctgactac gtcaagaagg tcaacaaggc aacagaggca 1800
   gccatgttcc tgggctgggt cgagcagctc gtgtacgatt tcacggatga aacgagcgag 1860
   gtttctacga ccgacaagat tgctgacatt actatcatta tcccctacat tggtcccgcc 1920
   ctgaacattg gcaacatgtt gtacaaggat gactttgtcg gcgccttgat tttttctggt 1980
   gccgtcatcc ttctcgaatt tatcccggaa attgctatcc ctgttcttgg cacctttgcc 2040
   ctggtgtctt acatcgccaa caaggttctc accgtgcaga ccatcgataa cgccctgtct 2100
   aagcgcaacg agaagtggga tgaggtgtac aagtacattg tgaccaactg gctggcaaag 2160
   gtgaacacac agatcgacct cattcgtaag aagatgaagg aggccttgga gaaccaggct 2220
   gaggcaacca aggctattat caactaccag tacaaccagt acaccgaaga ggaaaagaac 2280
   aacattaact ttaacatcga cgatctgtct agcaagttga acgagtccat caacaaggct 2340
   atgatcaaca ttaacaagtt cctgaaccag tgcagcgttt catacctcat gaactccatg 2400
   attccatacg gagtgaagcg actggaggat ttcgatgcca gcctgaagga cgcactgctc 2460
   aagtacatct acgataaccg gggcacgctg atcggccagg tcgatcgcct gaaggacaag 2520
   gtcaacaaca cgctctccac cgacattcct ttccagctgt ctaagtacgt cgataaccag 2580
   cgactgcttt caactttcac cgagtacatc aagaacatca ttaacacttc tatcctgaac 2640
   ctccgatacg agtctaacca tctgatcgat ctttcccgat acgcctctaa gattaacatc 2700
   ggttcgaagg tgaactttga ccctattgac aagaaccaga tccagctgtt caacctcgag 2760
   tccagcaaga tcgaggttat tctcaagaac gctattgtct acaactctat gtacgagaac 2820
   ttctcgacat ccttctggat tagaattccc aagtacttca actcgatctc gctgaacaac 2880
   gaatacacca ttatcaactg tatggagaac aactcaggtt ggaaggtctc acttaactac 2940
   ggcgagatta tctggactct gcaggacacc caggagatta agcagagagt cgtgtttaag 3000
   tactctcaga tgattaacat ttcggattac atcaaccggt ggattttcgt cactatcacc 3060
   aacaaccgtc tgaacaactc gaagatttac attaacggcc gacttatcga ccagaagcca 3120
   atttctaacc tgggcaacat ccatgcctcc aacaacatca tgttcaagct cgacggatgc 3180
   cgagatactc atcgatacat ctggatcaag tacttcaacc tgttcgacaa ggagctcaac 3240
   gagaaggaga ttaaggatct gtacgacaac cagtctaact cgggaatcct gaaggacttc 3300
   tggggagatt acctgcagta cgacaagccc tactacatgc tcaaccttta cgacccgaac 3360
   aagtacgttg acgtgaacaa cgtgggcatc cgcggttaca tgtacctgaa gggtccccgg 3420
   ggatctgtca tgacaaccaa catctacttg aactcgtcat tgtaccgagg aactaagttc 3480
   attatcaaga agtacgcaag cggcaacaag gataacattg tgagaaacaa cgacagagtt 3540
   tacatcaacg ttgtggttaa gaacaaggag taccgtctgg ccacaaacgc ctctcaggcc 3600
   ggagtcgaaa agatcctctc tgccctggag attcccgacg tcggaaactt gtcccaggtg 3660
   gtcgtgatga agtccaagaa cgatcaggga attaccaaca agtgtaagat gaacttgcag 3720
   gacaacaacg gaaacgacat cggcttcatt ggcttccatc agttcaacaa cattgccaag 3780
   ttggtggcct ctaactggta caaccgccag atcgaacgat cgtcccgaac gctgggatgt 3840
   tcttgggaat tcatccctgt tgacgacggt tggggtgagc gacctctgta a 3891
<210> 46
   <211> 3891
   <212> DNA
   <213> Artificial Sequence
<220>
   <223> BoNT/A, D. discoideum-modified 1
<400> 46
   atgccatttg tgaataaaca atttaattat aaagatccag ttaatggtgt tgacatagct 60
   tatataaaga ttccaaatgc tggtcaaatg caaccagtta aagcattcaa gatccataat 120
   aaaatatggg taattccaga gagagataca tttacaaacc cagaagaggg tgatttaaat 180
   ccaccgccag aagcaaaaca agtacccgtt tcatattacg attcaacata tttatctaca 240
   gataatgaaa aagataacta tcttaaagga gttacaaaac tcttcgaaag aatttatagt 300
   actgatcttg gtcgtatgtt acttacttca attgtccgtg gtattccatt ttggggtggc 360
   agtaccattg ataccgagtt aaaagtaatc gatactaatt gtattaatgt tatacaacca 420
   gatggttcat atagatcaga agagttaaat cttgttatca ttggtccttc agcagatata 480
   attcaatttg aatgtaaatc ttttggtcac gaggtattaa acttaacacg taatggttat 540
   ggttctacac aatatatcag attttcacca gatttcacat tcggttttga agagtcgtta 600
   gaagtagata caaatccatt actgggagca ggtaaattcg caactgatcc tgcagtaaca 660
   ttagctcatg aattaattca tgcaggtcac agattatatg gtatagccat taatcctaat 720
   agagttttta aagttaatac caatgcatat tacgaaatgt caggtttaga agtctcattt 780
   gaagagttaa gaacattcgg gggacatgat gctaaattta tagattcatt acaagaaaat 840
   gaatttagat tatattacta taataagttt aaagatattg ccagtacctt aaataaagca 900
   aaatcaattg tcggaacaac tgcctcatta caatatatga aaaatgtttt caaagaaaaa 960
   tatctacttt cagaagatac cagtggaaag ttttcagtag ataaattaaa attcgataag 1020
   ttatataaaa tgttgacaga aatttatact gaagataatt ttgttaagtt tttcaaagtg 1080
   ctaaatagaa agacatattt aaatttcgat aaagccgttt ttaaaattaa tattgttcca 1140
   aaagtcaatt atactattta tgatggattt aatttaagaa atacaaatct tgctgcaaat 1200
   tttaatggtc aaaatactga aattaataac atgaatttta caaaacttaa aaattttact 1260
   ggtttattcg aattttacaa attactatgt gtaagaggta ttataacttc taaaactaaa 1320
   agcctcgata aaggttataa taaagcttta aacgatcttt gtattaaagt taataactgg 1380
   gatttattct tttcaccatc cgaagacaat tttacaaatg atttgaataa gggtgaagag 1440
   ataacatcag atacgaatat cgaagcagct gaagagaata tatcattaga tttaattcaa 1500
   cagtattact taacttttaa ttttgacaat gaaccagaaa atatctcaat agaaaatttg 1560
   tcatccgaca ttatcggtca attagaatta atgcctaata ttgaacgttt ccctaatgga 1620
   aaaaagtatg aattagataa atataccatg tttcattatt taagagctca agaatttgaa 1680
   catggtaagt cacgtattgc attaacaaat tcagttaatg aagcattgtt aaatccatca 1740
   agagtttata cattcttttc cagtgattat gttaagaaag ttaacaaagc aacagaagcc 1800
   gctatgtttc taggatgggt tgaacaatta gtatatgatt ttacagatga aacatctgaa 1860
   gtatcaacta cagataaaat tgctgatatt actattatca ttccatatat tggtccagca 1920
   cttaatattg gtaatatgtt atataaagat gactttgttg gtgcattaat attctcaggt 1980
   gctgttattt tacttgaatt tataccagaa attgcgattc cagtattagg tacttttgca 2040
   ttagtttcat acatagctaa taaagttttg accgttcaaa caattgataa tgcactctca 2100
   aagagaaatg aaaaatggga tgaagtttat aaatatatag taacaaattg gttagcaaaa 2160
   gttaatactc aaattgattt aattagaaaa aagatgaaag aagcattaga aaatcaagca 2220
   gaagcaacaa aagctataat taattatcaa tataatcaat ataccgaaga ggaaaaaaat 2280
   aacattaact tcaatattga tgacttaagt tcaaaattaa atgaaagtat aaataaagct 2340
   atgattaata tcaataaatt tttaaatcaa tgttcagtat catatttaat gaatagtatg 2400
   attccatatg gtgttaaaag attggaggat tttgatgcta gtttaaaaga tgctttattg 2460
   aaatacattt atgataatag gggtacttta atcggtcaag ttgatcgttt aaaggataaa 2520
   gttaataaca ctttatctac agatatacca ttccaactat caaaatatgt tgataatcaa 2580
   cgtttattgt caacttttac tgaatatatt aaaaatatta taaatacctc cattcttaat 2640
   ttacgttatg aaagtaatca tttaatcgat ttgagtagat atgcatcgaa aataaatatc 2700
   ggttcaaaag taaatttcga tccaatagac aaaaatcaaa ttcaactttt caatttagaa 2760
   tcatctaaga ttgaagtaat cctcaaaaat gctattgttt ataatagtat gtatgaaaat 2820
   ttttcaacct ctttttggat tcgaattcca aaatacttta actcaatatc attaaataac 2880
   gaatatacta ttatcaattg tatggaaaat aacagcggtt ggaaagtaag tttaaattat 2940
   ggtgaaatta tatggacatt gcaagatact caagaaatca aacaaagagt agtctttaaa 3000
   tatagtcaaa tgataaatat ttcagattac attaatagat ggatctttgt tactattact 3060
   aataacagat tgaataatag taagatttat attaatggaa gattaattga tcaaaaacca 3120
   atttcaaatt taggtaatat tcatgcatct aataatataa tgtttaaatt agatggttgt 3180
   agagatacac atcgttatat ttggattaaa tatttcaatt tgtttgataa agaattaaat 3240
   gaaaaagaaa tcaaagattt atatgataat caatctaatt ctggtatact taaagatttt 3300
   tggggtgatt atttacaata tgataaacca tactatatgt tgaatttata tgatccaaat 3360
   aagtacgttg atgtgaataa tgtaggtata agaggatata tgtatttgaa aggaccaaga 3420
   ggttcagtta tgactacaaa tatttattta aattcatctc tatatcgtgg tacaaaattt 3480
   attataaaaa aatatgcaag tggtaataaa gataatattg tgagaaataa tgatagagtt 3540
   tatattaatg tagttgtaaa aaataaagaa tacagactcg ctacaaatgc ttcacaagca 3600
   ggtgtcgaaa aaattttatc agcattagag attccagatg ttggaaattt aagtcaagtt 3660
   gtagttatga aatctaaaaa tgatcaaggt attacaaata aatgcaaaat gaatttacaa 3720
   gataataatg gtaatgatat tggtttcatt ggttttcatc aattcaataa tattgcaaaa 3780
   ttagttgcca gtaattggta taataggcaa attgaaagaa gttctcgtac tttaggttgt 3840
   tcatgggaat ttattccagt agatgatggc tggggcgaaa gaccattgta a          3891
<210> 47
   <211> 3891
   <212> DNA
   <213> Artificial Sequence
<220>
   <223> BoNT/A, D. discoideum-modified 2
<400> 47
   atgccatttg ttaataaaca atttaattat aaagatccag ttaatggtgt tgatattgca 60
   tatattaaaa ttccaaatgc aggtcaaatg caaccagtta aagcatttaa aattcataat 120
   aaaatttggg ttattccaga aagagataca tttacaaatc cagaagaagg tgatttaaat 180
   ccaccaccag aagcaaaaca agttccagtt tcatattatg attcaacata tttatcaaca 240
   gataatgaaa aagataatta tttaaaaggt gttacaaaat tatttgaaag aatttattca 300
   acagatttag gtagaatgtt attaacatca attgttagag gtattccatt ttggggtggt 360
   tcaacaattg atacagaatt aaaagttatt gatacaaatt gtattaatgt tattcaacca 420
   gatggttcat atagatcaga agaattaaat ttagttatta ttggtccatc agcagatatt 480
   attcaatttg aatgtaaatc atttggtcat gaagttttaa atttaacaag aaatggttat 540
   ggttcaacac aatatattag attttcacca gattttacat ttggttttga agaatcatta 600
   gaagttgata caaatccatt attaggtgca ggtaaatttg caacagatcc agcagttaca 660
   ttagcacatg aattaattca tgcaggtcat agattatatg gtattgcaat taatccaaat 720
   agagttttta aagttaatac aaatgcatat tatgaaatgt caggtttaga agtttcattt 780
   gaagaattaa gaacatttgg tggtcatgat gcaaaattta ttgattcatt acaagaaaat 840
   gaatttagat tatattatta taataaattt aaagatattg catcaacatt aaataaagca 900
   aaatcaattg ttggtacaac agcatcatta caatatatga aaaatgtttt taaagaaaaa 960
   tatttattat cagaagatac atcaggtaaa ttttcagttg ataaattaaa atttgataaa 1020
   ttatataaaa tgttaacaga aatttataca gaagataatt ttgttaaatt ttttaaagtt 1080
   ttaaatagaa aaacatattt aaattttgat aaagcagttt ttaaaattaa tattgttcca 1140
   aaagttaatt atacaattta tgatggtttt aatttaagaa atacaaattt agcagcaaat 1200
   tttaatggtc aaaatacaga aattaataat atgaatttta caaaattaaa aaattttaca 1260
   ggtttatttg aattttataa attattatgt gttagaggta ttattacatc aaaaacaaaa 1320
   tcattagata aaggttataa taaagcatta aatgatttat gtattaaagt taataattgg 1380
   gatttatttt tttcaccatc agaagataat tttacaaatg atttaaataa aggtgaagaa 1440
   attacatcag atacaaatat tgaagcagca gaagaaaata tttcattaga tttaattcaa 1500
   caatattatt taacatttaa ttttgataat gaaccagaaa atatttcaat tgaaaattta 1560
   tcatcagata ttattggtca attagaatta atgccaaata ttgaaagatt tccaaatggt 1620
   aaaaaatatg aattagataa atatacaatg tttcattatt taagagcaca agaatttgaa 1680
   catggtaaat caagaattgc attaacaaat tcagttaatg aagcattatt aaatccatca 1740
   agagtttata catttttttc atcagattat gttaaaaaag ttaataaagc aacagaagca 1800
   gcaatgtttt taggttgggt tgaacaatta gtttatgatt ttacagatga aacatcagaa 1860
   gtttcaacaa cagataaaat tgcagatatt acaattatta ttccatatat tggtccagca 1920
   ttaaatattg gtaatatgtt atataaagat gattttgttg gtgcattaat tttttcaggt 1980
   gcagttattt tattagaatt tattccagaa attgcaattc cagttttagg tacatttgca 2040
   ttagtttcat atattgcaaa taaagtttta acagttcaaa caattgataa tgcattatca 2100
   aaaagaaatg aaaaatggga tgaagtttat aaatatattg ttacaaattg gttagcaaaa 2160
   gttaatacac aaattgattt aattagaaaa aaaatgaaag aagcattaga aaatcaagca 2220
   gaagcaacaa aagcaattat taattatcaa tataatcaat atacagaaga agaaaaaaat 2280
   aatattaatt ttaatattga tgatttatca tcaaaattaa atgaatcaat taataaagca 2340
   atgattaata ttaataaatt tttaaatcaa tgttcagttt catatttaat gaattcaatg 2400
   attccatatg gtgttaaaag attagaagat tttgatgcat cattaaaaga tgcattatta 2460
   aaatatattt atgataatag aggtacatta attggtcaag ttgatagatt aaaagataaa 2520
   gttaataata cattatcaac agatattcca tttcaattat caaaatatgt tgataatcaa 2580
   agattattat caacatttac agaatatatt aaaaatatta ttaatacatc aattttaaat 2640
   ttaagatatg aatcaaatca tttaattgat ttatcaagat atgcatcaaa aattaatatt 2700
   ggttcaaaag ttaattttga tccaattgat aaaaatcaaa ttcaattatt taatttagaa 2760
   tcatcaaaaa ttgaagttat tttaaaaaat gcaattgttt ataattcaat gtatgaaaat 2820
   ttttcaacat cattttggat tagaattcca aaatatttta attcaatttc attaaataat 2880
   gaatatacaa ttattaattg tatggaaaat aattcaggtt ggaaagtttc attaaattat 2940
   ggtgaaatta tttggacatt acaagataca caagaaatta aacaaagagt tgtttttaaa 3000
   tattcacaaa tgattaatat ttcagattat attaatagat ggatttttgt tacaattaca 3060
   aataatagat taaataattc aaaaatttat attaatggta gattaattga tcaaaaacca 3120
   atttcaaatt taggtaatat tcatgcatca aataatatta tgtttaaatt agatggttgt 3180
   agagatacac atagatatat ttggattaaa tattttaatt tatttgataa agaattaaat 3240
   gaaaaagaaa ttaaagattt atatgataat caatcaaatt caggtatttt aaaagatttt 3300
   tggggtgatt atttacaata tgataaacca tattatatgt taaatttata tgatccaaat 3360
   aaatatgttg atgttaataa tgttggtatt agaggttata tgtatttaaa aggtccaaga 3420
   ggttcagtta tgacaacaaa tatttattta aattcatcat tatatagagg tacaaaattt 3480
   attattaaaa aatatgcatc aggtaataaa gataatattg ttagaaataa tgatagagtt 3540
   tatattaatg ttgttgttaa aaataaagaa tatagattag caacaaatgc atcacaagca 3600
   ggtgttgaaa aaattttatc agcattagaa attccagatg ttggtaattt atcacaagtt 3660
   gttgttatga aatcaaaaaa tgatcaaggt attacaaata aatgtaaaat gaatttacaa 3720
   gataataatg gtaatgatat tggttttatt ggttttcatc aatttaataa tattgcaaaa 3780
   ttagttgcat caaattggta taatagacaa attgaaagat catcaagaac attaggttgt 3840
   tcatgggaat ttattccagt tgatgatggt tggggtgaaa gaccattata a          3891
<210> 48
   <211> 3891
   <212> DNA
   <213> Artificial Sequence
<220>
   <223> BoNT/A, D. discoideum-modified 3
<400> 48
   atgccatttg ttaataaaca attcaattat aaagatccag taaatggtgt agatattgca 60
   tatataaaaa ttccaaatgc tggacaaatg caacctgtta aagcattcaa aattcataat 120
   aaaatatggg ttattccaga acgtgatacc tttaccaatc cagaagaagg tgatttaaat 180
   ccacctccag aagcaaaaca agttccagta tcatattatg atagtacata tcttagtact 240
   gataatgaaa aagataatta tttaaaaggt gtaacaaaat tgtttgaacg tatttattca 300
   accgatttag gtagaatgtt attgacatca atagtaagag gtattccatt ttggggtgga 360
   tcaacaattg atacagaatt gaaagttatt gatactaatt gtataaatgt tattcaacct 420
   gatggttctt atagatcaga agaattaaat ttagtaatta taggaccatc tgcagatatt 480
   atacaatttg aatgtaaatc atttggtcat gaagttttaa atttaactag aaatggttat 540
   ggttcaacac aatatattag attctcacca gattttactt ttggatttga agaatcactt 600
   gaagttgata caaatccatt attgggtgca ggaaaatttg ctacagatcc agctgtaact 660
   ttggctcatg aattaattca tgcaggtcat agactttatg gtattgcaat taatccaaat 720
   agagtattta aagttaatac aaatgcatat tatgaaatgt ctggtttaga agtaagtttt 780
   gaagaattac gtactttcgg tggacatgat gcaaaattta tagattcact tcaagaaaat 840
   gaatttagat tatattatta taataaattt aaagatattg catcaacttt aaataaagca 900
   aaatctattg ttggtactac agcttcttta caatatatga aaaatgtttt caaagaaaaa 960
   tatttacttt cagaagatac atctggtaaa ttctcagttg ataaattaaa atttgataaa 1020
   ttatataaaa tgttgacaga aatttatact gaagataatt tcgttaaatt tttcaaagtt 1080
   ttaaatagaa aaacctatct taattttgat aaagctgtat tcaaaattaa tatagttcca 1140
   aaagttaatt atacaattta tgatggtttt aatttaagaa atactaattt agcagctaat 1200
   tttaatggac aaaatactga aattaataat atgaatttta caaaattaaa aaattttaca 1260
   ggtctttttg aattctataa attattgtgt gttagaggta taattactag taaaactaaa 1320
   tcattagata aaggttataa taaagcatta aatgatttgt gtataaaagt taataattgg 1380
   gatttattct tttcaccatc agaagataat tttacaaatg atttaaataa aggtgaagaa 1440
   attacttctg atactaatat tgaagctgca gaagaaaata ttagtttaga tttaatacaa 1500
   caatattatc ttacatttaa tttcgataat gaaccagaaa atattagtat tgaaaattta 1560
   tcaagtgata ttataggtca attggaatta atgcctaata ttgaaagatt cccaaatggt 1620
   aaaaaatatg aattagataa atataccatg tttcattatt taagagcaca agaattcgaa 1680
   catggtaaat caagaattgc tttaaccaat agtgttaatg aagctttact taatccatca 1740
   agagtttata cttttttctc atctgattat gtaaaaaaag ttaataaagc aacagaagca 1800
   gctatgttct tgggttgggt tgaacaattg gtttatgatt ttaccgatga aacttcagaa 1860
   gttagtacaa ctgataaaat tgctgatatt actataatta taccatatat tggtccagca 1920
   ttaaatattg gtaatatgtt atataaagat gattttgtag gtgcattaat tttttctggt 1980
   gcagtaattt tattggaatt tataccagaa attgcaattc cagttttagg tacattcgca 2040
   ttggtttcat atattgctaa taaagtttta acagtacaaa ctattgataa tgcattaagt 2100
   aaaagaaatg aaaaatggga tgaagtttat aaatatattg ttactaattg gttagcaaaa 2160
   gttaatactc aaattgattt aattcgtaaa aaaatgaaag aagctttaga aaatcaagca 2220
   gaagctacaa aagcaataat taattatcaa tataatcaat atactgaaga agaaaaaaat 2280
   aatattaatt tcaatattga tgatttatca tctaaattaa atgaaagtat aaataaagca 2340
   atgattaata ttaataaatt tttaaatcaa tgttcagttt catatttaat gaatagtatg 2400
   attccatatg gtgttaaaag attagaagat tttgatgcat cattaaaaga tgctttactt 2460
   aaatatattt atgataatcg tggaacatta attggtcaag ttgatagatt aaaagataaa 2520
   gttaataata cattatctac agatattcct tttcaattat caaaatatgt tgataatcaa 2580
   agattattga gtacctttac agaatatatt aaaaatatta taaatactag tattttaaat 2640
   ttacgttatg aatctaatca tttaattgat ttgtcacgtt atgcatcaaa aattaatatt 2700
   ggtagtaaag ttaattttga tccaattgat aaaaatcaaa tacaattatt taatttagaa 2760
   tcaagtaaaa ttgaagtaat attaaaaaat gctattgttt ataattcaat gtatgaaaat 2820
   ttcagtactt cattttggat tagaattcca aaatatttta attctatttc tttaaataat 2880
   gaatatacaa ttataaattg tatggaaaat aatagtggtt ggaaagtatc acttaattat 2940
   ggagaaatta tatggacact tcaagataca caagaaatta aacaaagagt agtttttaaa 3000
   tattcacaaa tgattaatat ttcagattat attaatagat ggatatttgt tacaataaca 3060
   aataatagat taaataattc aaaaatatat attaatggta gattaattga tcaaaaacca 3120
   atttcaaatt taggaaatat acatgcatca aataatatta tgtttaaatt agatggttgt 3180
   cgtgatactc atagatatat ttggattaaa tatttcaatt tatttgataa agaattaaat 3240
   gaaaaagaaa ttaaagattt atatgataat caatcaaatt caggtatttt aaaagatttt 3300
   tggggtgatt atttacaata tgataaacca tattatatgt taaatttata tgatccaaat 3360
   aaatatgttg atgttaataa tgttggtatt cgtggttata tgtatttaaa aggtccaaga 3420
   ggttcagtaa tgacaaccaa tatttattta aattcaagtt tatatagagg tacaaaattt 3480
   attataaaaa aatatgcttc aggaaataaa gataatatag ttagaaataa tgatcgtgtt 3540
   tatattaatg ttgtagttaa aaataaagaa tatagattag ctaccaatgc atcacaagca 3600
   ggagttgaaa aaattttatc agcattagaa attccagatg taggtaatct ttcacaagtt 3660
   gtagttatga aatcaaaaaa tgatcaaggt attacaaata aatgtaaaat gaatttacaa 3720
   gataataatg gtaatgatat tggtttcatt ggttttcatc aatttaataa tattgcaaaa 3780
   ttagtagcat caaattggta taatagacaa attgaaagat catcacgtac attaggttgt 3840
   tcatgggaat tcattccagt tgatgatggt tggggtgaaa gaccattata a 3891
<210> 49
   <211> 3891
   <212> DNA
   <213> Artificial Sequence
<220>
   <223> BoNT/A, A. thaliana-modified 1
<400> 49
   atgccttttg tgaacaaaca gttcaattac aaagatccag ttaacggtgt cgacattgct 60
   tacattaaaa ttcctaacgc tggtcagatg cagcctgtca aagcatttaa aatccataat 120
   aaaatatggg tgatacccga aagagatacc ttcacgaacc cagaggaagg tgacctcaac 180
   ccacctccgg aggctaaaca agtaccggtc tcttattacg actcgacgta tctgtccaca 240
   gacaatgaaa aagacaatta cctgaagggt gtaactaaat tgtttgagag aatttatagc 300
   actgatttgg gtagaatgct ccttactagc atcgtgagag gtattccttt ttggggaggt 360
   tccaccatag atactgaact gaaggtaata gacacaaatt gcataaatgt aatacagccg 420
   gatggttcgt atcgctctga agagctgaac ctggtcatta taggtccttc agccgatatt 480
   atacagttcg agtgtaagtc gtttggacat gaggttctga atttaactcg taatggttat 540
   ggttcgaccc agtatattag gttcagtccg gattttacat ttgggttcga agagtcactc 600
   gaggtggata ctaaccctct gttaggagcc ggaaagttcg caacagatcc agctgtcacg 660
   ctcgctcacg aacttattca cgctgggcat agactgtatg gaattgctat caatccaaat 720
   agggttttca aagtgaatac caatgcatat tacgagatgt cgggcctgga agtgtctttt 780
   gaggaacttc gaacttttgg gggacacgat gcgaagttca ttgatagcct tcaagaaaat 840
   gaatttaggc tgtactatta caataaattt aaggatatag ctagcacttt gaacaaagca 900
   aagtctatcg ttggtacgac tgctagcctg caatacatga aaaatgtatt caaggaaaaa 960
   tacttgctta gcgaggatac aagtggtaag ttcagtgttg ataagttgaa atttgataaa 1020
   ctgtataaaa tgttgactga aatatatact gaagataatt ttgtcaaatt tttcaaagtt 1080
   ttaaacagga aaacgtattt gaacttcgat aaagctgttt ttaagattaa cattgtacca 1140
   aaggtgaact ataccatata tgatgggttt aatttgcgga acaccaactt agcggcaaac 1200
   ttcaatgggc agaacaccga aatcaacaat atgaatttta caaaattgaa gaatttcacg 1260
   ggactgttcg agttttacaa gcttctctgc gttcgtggca ttatcacatc caagacaaag 1320
   tctttggata agggatataa taaagctctg aacgatctct gcatcaaggt caacaattgg 1380
   gatctcttct tttcaccatc ggaagataac tttactaacg atttgaacaa gggtgaagag 1440
   attacctcag atacgaatat cgaggcggca gaagagaaca tcagcttgga tttaattcaa 1500
   cagtattacc ttacatttaa cttcgacaat gagccagaga atatttctat agaaaacttg 1560
   tcatctgata tcattggtca actggagctg atgcccaaca tcgaacgatt tccaaacggc 1620
   aagaaatacg aactggataa atacacaatg ttccattacc tgcgagccca agagtttgaa 1680
   cacggtaaga gtagaatagc tttgacaaat agcgtaaatg aggcgctgtt gaatccttct 1740
   agagtctata cttttttcag ttcggactac gttaagaaag ttaataaagc tactgaggcg 1800
   gcaatgtttc ttggttgggt tgaacagctg gtgtatgatt tcacagatga gacatcggag 1860
   gttagcacta cagacaaaat agcagatatt acaatcatta tcccttatat tggaccagcc 1920
   ctgaatatcg gaaacatgct ttataaggat gacttcgtcg gtgctctgat cttttccggc 1980
   gctgtcattt tgcttgagtt tattcctgaa attgctattc cagtgcttgg tactttcgca 2040
   ttggtgtcat atatcgccaa caaggtttta acagtacaga ctatcgacaa cgctttgtca 2100
   aagcgaaacg aaaagtggga cgaagtttac aagtatattg tgacgaattg gttagccaag 2160
   gttaacactc aaattgacct tattaggaag aaaatgaaag aagcccttga gaaccaggcc 2220
   gaagctacaa aggcgataat caattatcaa tacaaccagt acaccgagga agagaaaaat 2280
   aacattaact ttaacattga cgatctgtct agcaagttga acgagtccat taataaggct 2340
   atgattaaca taaacaaatt cttgaatcaa tgttcagtca gttacctgat gaatagtatg 2400
   attccttacg gggtaaaacg tctggaagac tttgacgcat cgcttaagga tgcacttctg 2460
   aaatacatct acgataacag aggaactctg atcggacaag ttgatcggct gaaagataag 2520
   gttaacaata ccttatctac cgatatacct ttccaattga gcaagtatgt ggacaaccag 2580
   aggctgttga gtacatttac ggaatacatt aagaacatta tcaatactag tatattgaat 2640
   ttaaggtacg aaagcaatca tctcatcgat ctttctcggt acgcttctaa aatcaatatt 2700
   ggatcaaaag ttaattttga tcccatcgac aagaatcaaa ttcaactctt taacttggaa 2760
   tccagtaaga tcgaagttat tttaaaaaat gccatagtgt ataacagtat gtacgaaaat 2820
   ttctctacct cattctggat cagaatcccg aagtatttta attcaatctc gctgaacaat 2880
   gagtatacaa taattaattg tatggagaat aactccggat ggaaggtttc actcaattat 2940
   ggtgagataa tttggacact gcaagatact caagagatca agcaaagagt agtcttcaaa 3000
   tattcgcaaa tgatcaatat ctccgactac attaataggt ggatcttcgt tacaatcacg 3060
   aataacagat tgaataacag taaaatctat atcaatggcc gtttaataga ccagaagcct 3120
   atcagtaatc tggggaatat tcatgcttcc aacaatatta tgttcaagct tgatggctgt 3180
   agagataccc atagatatat ttggatcaaa tacttcaacc tgtttgacaa agagctgaac 3240
   gagaaggaaa taaaagacct gtatgataac cagtccaact ctggaattct caaggacttc 3300
   tggggagatt acttgcagta tgataaacca tactatatgc tgaacctgta tgatccgaat 3360
   aagtacgtgg atgtcaacaa tgttggtatc cgtggctaca tgtatctgaa gggaccccgt 3420
   gggtctgtta tgacaactaa tatatacctg aattcgtctt tatacagagg aacgaagttt 3480
   attataaaaa agtacgctag tggaaacaaa gataacatag ttcgaaataa cgatcgggtt 3540
   tatatcaacg tggttgtcaa aaacaaagag tatagattag caactaacgc ttctcaagca 3600
   ggggttgaaa agatattgag tgcgttagaa attccggatg taggtaactt gtcccaagtt 3660
   gtggttatga agtccaaaaa cgatcaagga ataaccaata agtgcaaaat gaatttacaa 3720
   gataataacg gaaatgacat cggctttatt ggattccacc aattcaataa catcgcaaag 3780
   cttgtggcta gtaattggta caatcgccag atcgagaggt cgtcccgcac tttggggtgt 3840
   agctgggaat tcattccagt ggacgatggc tggggagagc gtcctttata a 3891
<210> 50
   <211> 3891
   <212> DNA
   <213> Artificial Sequence
<220>
   <223> BoNT/A, A. thaliana-modified 2
<400> 50
   atgccttttg ttaataagca atttaattat aaggatcctg ttaatggagt tgatattgct 60
   tatattaaga ttcctaatgc tggacaaatg caacctgtta aggcttttaa gattcataat 120
   aagatttggg ttattcctga aagagatact tttactaatc ctgaagaagg agatctgaat 180
   cctcctcctg aagctaagca agttcctgtt tcttattatg attctactta tctgtctact 240
   gataatgaaa aggataatta tctgaaggga gttactaagc tgtttgaaag aatttattct 300
   actgatctgg gaagaatgct gctgacttct attgttagag gaattccttt ttggggagga 360
   tctactattg atactgaact gaaggttatt gatactaatt gtattaatgt tattcaacct 420
   gatggatctt atagatctga agaactgaat ctggttatta ttggaccttc tgctgatatt 480
   attcaatttg aatgtaagtc ttttggacat gaagttctga atctgactag aaatggatat 540
   ggatctactc aatatattag attttctcct gattttactt ttggatttga agaatctctg 600
   gaagttgata ctaatcctct gctgggagct ggaaagtttg ctactgatcc tgctgttact 660
   ctggctcatg aactgattca tgctggacat agactgtatg gaattgctat taatcctaat 720
   agagttttta aggttaatac taatgcttat tatgaaatgt ctggactgga agtttctttt 780
   gaagaactga gaacttttgg aggacatgat gctaagttta ttgattctct gcaagaaaat 840
   gaatttagac tgtattatta taataagttt aaggatattg cttctactct gaataaggct 900
   aagtctattg ttggaactac tgcttctctg caatatatga agaatgtttt taaggaaaag 960
   tatctgctgt ctgaagatac ttctggaaag ttttctgttg ataagctgaa gtttgataag 1020
   ctgtataaga tgctgactga aatttatact gaagataatt ttgttaagtt ttttaaggtt 1080
   ctgaatagaa agacttatct gaattttgat aaggctgttt ttaagattaa tattgttcct 1140
   aaggttaatt atactattta tgatggattt aatctgagaa atactaatct ggctgctaat 1200
   tttaatggac aaaatactga aattaataat atgaatttta ctaagctgaa gaattttact 1260
   ggactgtttg aattttataa gctgctgtgt gttagaggaa ttattacttc taagactaag 1320
   tctctggata agggatataa taaggctctg aatgatctgt gtattaaggt taataattgg 1380
   gatctgtttt tttctccttc tgaagataat tttactaatg atctgaataa gggagaagaa 1440
   attacttctg atactaatat tgaagctgct gaagaaaata tttctctgga tctgattcaa 1500
   caatattatc tgacttttaa ttttgataat gaacctgaaa atatttctat tgaaaatctg 1560
   tcttctgata ttattggaca actggaactg atgcctaata ttgaaagatt tcctaatgga 1620
   aagaagtatg aactggataa gtatactatg tttcattatc tgagagctca agaatttgaa 1680
   catggaaagt ctagaattgc tctgactaat tctgttaatg aagctctgct gaatccttct 1740
   agagtttata cttttttttc ttctgattat gttaagaagg ttaataaggc tactgaagct 1800
   gctatgtttc tgggatgggt tgaacaactg gtttatgatt ttactgatga aacttctgaa 1860
   gtttctacta ctgataagat tgctgatatt actattatta ttccttatat tggacctgct 1920
   ctgaatattg gaaatatgct gtataaggat gattttgttg gagctctgat tttttctgga 1980
   gctgttattc tgctggaatt tattcctgaa attgctattc ctgttctggg aacttttgct 2040
   ctggtttctt atattgctaa taaggttctg actgttcaaa ctattgataa tgctctgtct 2100
   aagagaaatg aaaagtggga tgaagtttat aagtatattg ttactaattg gctggctaag 2160
   gttaatactc aaattgatct gattagaaag aagatgaagg aagctctgga aaatcaagct 2220
   gaagctacta aggctattat taattatcaa tataatcaat atactgaaga agaaaagaat 2280
   aatattaatt ttaatattga tgatctgtct tctaagctga atgaatctat taataaggct 2340
   atgattaata ttaataagtt tctgaatcaa tgttctgttt cttatctgat gaattctatg 2400
   attccttatg gagttaagag actggaagat tttgatgctt ctctgaagga tgctctgctg 2460
   aagtatattt atgataatag aggaactctg attggacaag ttgatagact gaaggataag 2520
   gttaataata ctctgtctac tgatattcct tttcaactgt ctaagtatgt tgataatcaa 2580
   agactgctgt ctacttttac tgaatatatt aagaatatta ttaatacttc tattctgaat 2640
   ctgagatatg aatctaatca tctgattgat ctgtctagat atgcttctaa gattaatatt 2700
   ggatctaagg ttaattttga tcctattgat aagaatcaaa ttcaactgtt taatctggaa 2760
   tcttctaaga ttgaagttat tctgaagaat gctattgttt ataattctat gtatgaaaat 2820
   ttttctactt ctttttggat tagaattcct aagtatttta attctatttc tctgaataat 2880
   gaatatacta ttattaattg tatggaaaat aattctggat ggaaggtttc tctgaattat 2940
   ggagaaatta tttggactct gcaagatact caagaaatta agcaaagagt tgtttttaag 3000
   tattctcaaa tgattaatat ttctgattat attaatagat ggatttttgt tactattact 3060
   aataatagac tgaataattc taagatttat attaatggaa gactgattga tcaaaagcct 3120
   atttctaatc tgggaaatat tcatgcttct aataatatta tgtttaagct ggatggatgt 3180
   agagatactc atagatatat ttggattaag tattttaatc tgtttgataa ggaactgaat 3240
   gaaaaggaaa ttaaggatct gtatgataat caatctaatt ctggaattct gaaggatttt 3300
   tggggagatt atctgcaata tgataagcct tattatatgc tgaatctgta tgatcctaat 3360
   aagtatgttg atgttaataa tgttggaatt agaggatata tgtatctgaa gggacctaga 3420
   ggatctgtta tgactactaa tatttatctg aattcttctc tgtatagagg aactaagttt 3480
   attattaaga agtatgcttc tggaaataag gataatattg ttagaaataa tgatagagtt 3540
   tatattaatg ttgttgttaa gaataaggaa tatagactgg ctactaatgc ttctcaagct 3600
   ggagttgaaa agattctgtc tgctctggaa attcctgatg ttggaaatct gtctcaagtt 3660
   gttgttatga agtctaagaa tgatcaagga attactaata agtgtaagat gaatctgcaa 3720
   gataataatg gaaatgatat tggatttatt ggatttcatc aatttaataa tattgctaag 3780
   ctggttgctt ctaattggta taatagacaa attgaaagat cttctagaac tctgggatgt 3840
   tcttgggaat ttattcctgt tgatgatgga tggggagaaa gacctctgta a 3891
<210> 51
   <211> 3891
   <212> DNA
   <213> Artificial Sequence
<220>
   <223> BoNT/A, A. thaliana-modified 3
<400> 51
   atgccatttg tgaacaaaca atttaattat aaagatcctg tgaacggcgt cgacattgca 60
   tacattaaga tccctaatgc gggacaaatg cagccggtga aagctttcaa gatccataat 120
   aagatttggg tgatacctga acgggataca ttcactaacc cagaagaggg agatctgaat 180
   ccgccaccgg aagctaaaca agtgcctgtc tcttactatg attctactta cctgtctacg 240
   gataacgaga aggataacta cctgaaaggt gttacaaaac tgttcgaaag aatatactct 300
   acggacctgg gtaggatgct gctgacctct atcgtaaggg gaattccatt ttggggtggc 360
   tctacaattg acactgagct gaaagtcatc gatacgaact gtattaatgt cattcagcca 420
   gacggttctt atagatctga agagctgaac ctggtaatca ttgggccatc tgccgatatt 480
   atccaatttg agtgtaagtc ttttggtcat gaggttctga acctgactcg taacggatat 540
   ggttctacac aatatatcag gttttctcca gatttcactt tcgggttcga agagtctctg 600
   gaggtagata ctaaccctct gctgggtgcg ggtaaattcg ccacagaccc agctgtaaca 660
   ctggctcacg aactgattca cgccggacat agactgtacg gtatagcaat taatcctaac 720
   agggttttta aggttaacac caatgcatat tacgagatgt ctggcctgga agtatctttc 780
   gaagagctgc gtacgtttgg tgggcacgac gctaaattta ttgactctct gcaggaaaac 840
   gaattccggc tgtattacta taataaattt aaagatatcg cgtctacact gaacaaggcc 900
   aagtctatcg tgggaactac cgcttctctg caatatatga agaatgtatt taaagaaaaa 960
   tacctgctgt ctgaggacac ttctggtaaa ttttctgttg acaagctgaa atttgataag 1020
   ctgtataaga tgctgacaga gatctatact gaagataact tcgttaagtt ctttaaagtg 1080
   ctgaaccgta agacctacct gaattttgat aaagccgtgt tcaagattaa tatcgtgcct 1140
   aaggtgaact acactattta cgatggattc aatctgcgta atacgaatct ggccgcgaat 1200
   ttcaatggac agaacacaga gatcaataac atgaatttca ccaagctgaa gaactttacg 1260
   ggactgtttg agttctacaa actgctgtgt gttagaggta tcataacatc taaaactaaa 1320
   tctctggaca agggatacaa caaagctctg aatgatctgt gtataaaggt caacaattgg 1380
   gatctgttct tttctccgtc tgaagataac ttcacgaacg acctgaataa aggagaggaa 1440
   attacctctg atacaaatat cgaggccgct gaggaaaata tatctctgga cctgatccaa 1500
   cagtattacc tgactttcaa tttcgataat gaacctgaga atatttctat agaaaatctg 1560
   tcttctgata ttatcggaca actggaactg atgccaaata ttgaacgatt tcctaatggc 1620
   aagaaatatg agctggacaa gtacaccatg ttccattatc tgagagccca agaattcgaa 1680
   cacggcaaat ctcgaatcgc gctgactaac tctgtaaatg aggcactgct gaatccttct 1740
   cgtgtataca cattcttttc ttctgactat gtgaagaaag ttaataaggc aaccgaggca 1800
   gctatgttcc tgggctgggt cgagcaactg gtgtatgatt tcaccgatga aacatctgaa 1860
   gtttctacta cggataaaat tgctgacatt acaatcatta taccttacat cggtcctgct 1920
   ctgaatatag gcaacatgct gtataaagat gactttgtcg gagcgctgat tttctctggt 1980
   gctgttattc tgctggagtt tattccagaa atcgctatcc ctgttctggg gaccttcgcc 2040
   ctggtttctt atattgctaa taaagtcctg acagttcaga ctatcgacaa cgctctgtct 2100
   aagagaaacg agaagtggga tgaggtttac aagtatatcg ttacgaattg gctggcaaaa 2160
   gttaacactc agatcgacct gatcaggaaa aagatgaaag aggcgctgga aaatcaagct 2220
   gaagctacaa aggcaattat aaattatcaa tacaatcagt atactgaaga ggaaaagaat 2280
   aacattaact ttaacattga tgacctgtct tctaaactga acgagtctat aaacaaggca 2340
   atgattaata tcaacaagtt tctgaaccaa tgctctgttt cttacctgat gaactctatg 2400
   atcccttatg gcgttaagag actggaagat tttgatgcat ctctgaaaga cgctctgctg 2460
   aagtacatat atgacaatag aggaactctg attggacagg ttgatcgact gaaagataaa 2520
   gttaataaca ctctgtctac agatattccg tttcaactgt ctaagtacgt tgataatcag 2580
   aggctgctgt ctactttcac agagtatatt aagaacatta taaatacatc tatcctgaac 2640
   ctgagatacg aatctaatca tctgatagat ctgtctaggt acgcatctaa gataaatatt 2700
   gggtctaaag ttaactttga tcctatcgat aagaatcaga tacagctgtt caacctggag 2760
   tcttctaaga ttgaagttat actgaagaat gctatagtct ataattctat gtacgaaaat 2820
   ttctctacat ctttttggat acgaattccg aaatatttca actctatatc tctgaataac 2880
   gagtatacga tcataaattg catggagaat aactctggat ggaaagtgtc tctgaattac 2940
   ggagaaatta tctggactct gcaagatact caggagatca agcaacgggt ggtctttaaa 3000
   tattctcaaa tgattaatat atctgattac attaatagat ggatcttcgt tacaattact 3060
   aataacagac tgaataactc taagatctat attaacggaa gactgatcga ccaaaagcct 3120
   atctctaacc tgggtaacat acatgcatct aacaatatta tgttcaaact ggatggatgt 3180
   agagatacac ataggtatat ttggataaag tattttaacc tgtttgataa agagctgaac 3240
   gagaaagaaa ttaaggacct gtatgataac cagtctaact ctggtatact gaaagatttt 3300
   tggggtgatt acctgcaata cgacaagcca tattacatgc tgaatctgta cgatccaaac 3360
   aagtacgtgg atgtaaataa cgttggaata cggggttaca tgtatctgaa ggggcctaga 3420
   gggtctgtca tgacgactaa catctatctg aattcttctc tgtacagagg gaccaagttt 3480
   attataaaaa agtatgcttc tggtaataaa gataatatcg tcagaaacaa tgacagagtc 3540
   tatatcaatg ttgtggttaa aaacaaagaa tatcgtctgg ctaccaatgc atctcaagct 3600
   ggagttgaaa aaatactgtc tgctctggag atccctgatg ttggtaacct gtctcaagtg 3660
   gtcgtaatga aatctaagaa cgatcaggga attaccaata agtgcaagat gaatctgcaa 3720
   gataacaatg gtaatgatat tgggtttatt ggcttccacc agtttaacaa catcgctaaa 3780
   ctggtagcat ctaactggta caaccgacag attgaacgtt cttctaggac cctgggatgc 3840
   tcttgggaat ttatcccagt ggatgatggg tggggtgaga gaccgctgta a          3891
<210> 52
   <211> 3891
   <212> DNA
   <213> Artificial Sequence
<220>
   <223> BoNT/A, T. aestivum-modified 1
<400> 52
   atgccatttg tcaacaagca attcaattac aaggatccag tgaacggcgt ggatatcgcg 60
   tatatcaaga tacctaacgc ggggcagatg caaccagtta aggcttttaa gatccacaac 120
   aagatctggg ttatccccga gagagatact ttcactaacc ctgaagaggg agacctgaac 180
   ccgcccccag aggccaagca ggtcccggtg tcttactatg actcaactta cttgtccacc 240
   gataacgaga aggacaatta cctgaaggga gtgacaaagc tttttgagcg tatctatagc 300
   actgacctgg gcaggatgct tttaacatct atcgtacgcg ggattccttt ctggggggga 360
   tctaccatcg atactgagct taaggtcata gacacaaatt gcattaacgt catccagcca 420
   gacggttcat acaggtcgga ggaactcaac ctggtgatca ttggcccgag cgcggacatt 480
   atccaattcg aatgcaagag cttcggccac gaggtgctca atttgactcg caacgggtac 540
   ggctccactc agtacatacg gttctcccca gattttacct tcggctttga ggaaagccta 600
   gaagttgaca cgaacccgct cttgggcgct ggcaagtttg ccacggatcc agccgttacg 660
   ctggcacacg agctcatcca tgccggtcat aggctgtacg ggatcgccat caacccaaat 720
   cgtgtgttca aggtaaatac caacgcgtac tatgagatgt ctgggctgga agtcagcttc 780
   gaggaactgc gcaccttcgg cggacacgat gcaaaattca tcgactcgct acaagaaaac 840
   gagttcagac tctactatta caacaagttt aaggacatcg ccagcacact caataaggct 900
   aagtcgattg ttggtacgac agcttccctc caatacatga agaacgtgtt caaggagaag 960
   tacctgttgt ccgaggacac atccggcaag ttcagcgtcg ataagctgaa atttgataaa 1020
   ctctataaaa tgttaacaga gatctataca gaggataatt tcgtcaaatt ctttaaggtt 1080
   ttgaacagga agacctatct aaattttgac aaggcggttt tcaagatcaa catcgtgcca 1140
   aaagtcaact ataccatcta cgatggtttc aacctaagaa acaccaacct cgccgcgaac 1200
   ttcaatggcc aaaatacaga aattaacaat atgaacttca cgaagctcaa gaactttact 1260
   gggctcttcg aattctacaa gcttctgtgc gttcgtggta ttataacctc gaagacaaag 1320
   tctcttgaca agggttacaa caaggctctt aatgatctgt gcatcaaagt aaacaattgg 1380
   gatcttttct tttcgccaag tgaagataac ttcaccaacg accttaacaa gggcgaggaa 1440
   atcacttcgg ataccaacat tgaggccgct gaagagaaca tctcgctcga cctcattcaa 1500
   cagtactatc taaccttcaa tttcgacaat gagcctgaga atatttcaat agaaaacctg 1560
   tccagtgaca tcattggcca actcgagctg atgccgaaca tagagagatt ccccaacggc 1620
   aagaaatacg aacttgataa gtacactatg ttccattacc tgcgtgcaca ggagttcgag 1680
   cacgggaagt ctcggatcgc cctcacgaac agtgtcaacg aggcacttct caacccgtcg 1740
   cgcgtgtaca cctttttcag ctccgactac gttaaaaagg tgaacaaggc caccgaggcg 1800
   gccatgttcc tgggttgggt tgagcaactg gtctatgact ttaccgacga gacctcagag 1860
   gtctccacga ctgacaagat cgcagatatc accatcataa tcccttacat cggcccagcc 1920
   ttgaacatcg gcaacatgtt atacaaagat gactttgtgg gtgccctgat attttccgga 1980
   gcggtcatcc ttctggagtt catacccgag atagcaattc ctgtcctcgg cactttcgct 2040
   ctcgtctcat acatcgcaaa taaggttttg accgtgcaga ccatcgacaa cgccttgagc 2100
   aaaaggaatg agaagtggga cgaagtctac aagtacatag tcacgaattg gctcgcgaag 2160
   gtcaacaccc agatcgatct catccggaag aaaatgaaag aggccctgga gaaccaagcg 2220
   gaggccacca aggcgattat caactaccaa tacaatcagt acacagagga agagaagaac 2280
   aatataaact ttaatataga tgacctcagc tcaaagctga acgagtccat caataaggca 2340
   atgatcaaca ttaacaagtt cctgaatcag tgcagcgtgt cttatctgat gaattccatg 2400
   atcccatatg gtgtgaagcg attggaggac ttcgacgctt cgctcaagga cgcgctccta 2460
   aagtatatat acgataaccg cgggacgttg atcggccaag tggaccgtct caaagacaag 2520
   gtcaacaata cgctttccac ggacatcccg tttcaacttt ctaagtacgt tgataaccaa 2580
   aggttgctat ctacctttac cgaatatatt aagaacatca taaatacatc gatcttaaac 2640
   ttgcgctacg agtctaatca cctcatcgac ctgagccggt acgcatcaaa gattaatatt 2700
   ggttccaaag tgaacttcga ccccattgac aagaaccaaa tccaactctt taacctcgag 2760
   tccagcaaga tcgaggtcat cttgaagaac gctatcgtct acaactcgat gtacgagaac 2820
   ttcagcacct ctttctggat taggatccct aagtacttca actccatctc actcaataac 2880
   gagtatacta tcattaactg catggaaaac aattccggat ggaaagtttc acttaactac 2940
   ggggagatta tctggaccct gcaagatacc caggagataa agcagcgcgt ggtatttaag 3000
   tactcccaga tgatcaacat aagtgactac attaaccgct ggatctttgt aaccatcaca 3060
   aataacagac tcaacaatag caagatttac atcaatggac gactaatcga tcagaaacca 3120
   atcagcaacc tcggaaatat tcatgcctcg aacaatatta tgttcaagct cgacggttgc 3180
   cgggatacac atagatacat ttggataaag tactttaact tgttcgacaa agaactcaac 3240
   gagaaggaga ttaaggacct ctacgacaac cagagtaatt cagggattct taaagacttt 3300
   tggggggact acctccaata cgacaagccg tattacatgc ttaacctgta cgacccaaac 3360
   aagtatgtgg acgttaacaa tgtggggatt aggggctata tgtacctcaa ggggccccga 3420
   ggatctgtaa tgaccactaa catctacttg aacagctcat tgtaccgcgg aacgaaattc 3480
   atcattaaga aatacgccag cggcaacaag gacaacattg tgcgcaataa cgaccgggtg 3540
   tacatcaacg tcgtggtcaa gaacaaggag tataggcttg caacgaatgc tagtcaagcc 3600
   ggtgttgaga agatcctctc agcgctggaa atccccgacg tgggcaacct gtcccaggtg 3660
   gttgtgatga agtccaaaaa tgaccagggc attaccaaca agtgtaagat gaatctgcag 3720
   gacaacaatg gcaacgatat cggattcatc ggcttccacc aattcaacaa tattgccaag 3780
   ttagtcgctt caaactggta taaccgccaa atcgagaggt ccagtcggac gctagggtgt 3840
   agctgggaat ttatcccggt ggatgatggc tggggagaga ggcccctata a 3891
<210> 53
   <211> 3891
   <212> DNA
   <213> Artificial Sequence
<220>
   <223> BoNT/A, T. aestivum-modified 2
<400> 53
   atgccattcg tgaacaagca attcaactac aaggacccag tgaacggcgt ggacatcgcc 60
   tacatcaaga tcccaaacgc cggccaaatg caaccagtga aggccttcaa gatccacaac 120
   aagatctggg tgatcccaga gagggacacc ttcaccaacc cagaggaggg cgacctcaac 180
   ccaccaccag aggccaagca agtgccagtg tcctactacg actccaccta cctctccacc 240
   gacaacgaga aggacaacta cctcaagggc gtgaccaagc tcttcgagag gatctactcc 300
   accgacctcg gcaggatgct cctcacctcc atcgtgaggg gcatcccatt ctggggcggc 360
   tccaccatcg acaccgagct caaggtgatc gacaccaact gcatcaacgt gatccaacca 420
   gacggctcct acaggtccga ggagctcaac ctcgtgatca tcggcccatc cgccgacatc 480
   atccaattcg agtgcaagtc cttcggccac gaggtgctca acctcaccag gaacggctac 540
   ggctccaccc aatacatcag gttctcccca gacttcacct tcggcttcga ggagtccctc 600
   gaggtggaca ccaacccact cctcggcgcc ggcaagttcg ccaccgaccc agccgtgacc 660
   ctcgcccacg agctcatcca cgccggccac aggctctacg gcatcgccat caacccaaac 720
   agggtgttca aggtgaacac caacgcctac tacgagatgt ccggcctcga ggtgtccttc 780
   gaggagctca ggaccttcgg cggccacgac gccaagttca tcgactccct ccaagagaac 840
   gagttcaggc tctactacta caacaagttc aaggacatcg cctccaccct caacaaggcc 900
   aagtccatcg tgggcaccac cgcctccctc caatacatga agaacgtgtt caaggagaag 960
   tacctcctct ccgaggacac ctccggcaag ttctccgtgg acaagctcaa gttcgacaag 1020
   ctctacaaga tgctcaccga gatctacacc gaggacaact tcgtgaagtt cttcaaggtg 1080
   ctcaacagga agacctacct caacttcgac aaggccgtgt tcaagatcaa catcgtgcca 1140
   aaggtgaact acaccatcta cgacggcttc aacctcagga acaccaacct cgccgccaac 1200
   ttcaacggcc aaaacaccga gatcaacaac atgaacttca ccaagctcaa gaacttcacc 1260
   ggcctcttcg agttctacaa gctcctctgc gtgaggggca tcatcacctc caagaccaag 1320
   tccctcgaca agggctacaa caaggccctc aacgacctct gcatcaaggt gaacaactgg 1380
   gacctcttct tctccccatc cgaggacaac ttcaccaacg acctcaacaa gggcgaggag 1440
   atcacctccg acaccaacat cgaggccgcc gaggagaaca tctccctcga cctcatccaa 1500
   caatactacc tcaccttcaa cttcgacaac gagccagaga acatctccat cgagaacctc 1560
   tcctccgaca tcatcggcca actcgagctc atgccaaaca tcgagaggtt cccaaacggc 1620
   aagaagtacg agctcgacaa gtacaccatg ttccactacc tcagggccca agagttcgag 1680
   cacggcaagt ccaggatcgc cctcaccaac tccgtgaacg aggccctcct caacccatcc 1740
   agggtgtaca ccttcttctc ctccgactac gtgaagaagg tgaacaaggc caccgaggcc 1800
   gccatgttcc tcggctgggt ggagcaactc gtgtacgact tcaccgacga gacctccgag 1860
   gtgtccacca ccgacaagat cgccgacatc accatcatca tcccatacat cggcccagcc 1920
   ctcaacatcg gcaacatgct ctacaaggac gacttcgtgg gcgccctcat cttctccggc 1980
   gccgtgatcc tcctcgagtt catcccagag atcgccatcc cagtgctcgg caccttcgcc 2040
   ctcgtgtcct acatcgccaa caaggtgctc accgtgcaaa ccatcgacaa cgccctctcc 2100
   aagaggaacg agaagtggga cgaggtgtac aagtacatcg tgaccaactg gctcgccaag 2160
   gtgaacaccc aaatcgacct catcaggaag aagatgaagg aggccctcga gaaccaagcc 2220
   gaggccacca aggccatcat caactaccaa tacaaccaat acaccgagga ggagaagaac 2280
   aacatcaact tcaacatcga cgacctctcc tccaagctca acgagtccat caacaaggcc 2340
   atgatcaaca tcaacaagtt cctcaaccaa tgctccgtgt cctacctcat gaactccatg 2400
   atcccatacg gcgtgaagag gctcgaggac ttcgacgcct ccctcaagga cgccctcctc 2460
   aagtacatct acgacaacag gggcaccctc atcggccaag tggacaggct caaggacaag 2520
   gtgaacaaca ccctctccac cgacatccca ttccaactct ccaagtacgt ggacaaccaa 2580
   aggctcctct ccaccttcac cgagtacatc aagaacatca tcaacacctc catcctcaac 2640
   ctcaggtacg agtccaacca cctcatcgac ctctccaggt acgcctccaa gatcaacatc 2700
   ggctccaagg tgaacttcga cccaatcgac aagaaccaaa tccaactctt caacctcgag 2760
   tcctccaaga tcgaggtgat cctcaagaac gccatcgtgt acaactccat gtacgagaac 2820
   ttctccacct ccttctggat caggatccca aagtacttca actccatctc cctcaacaac 2880
   gagtacacca tcatcaactg catggagaac aactccggct ggaaggtgtc cctcaactac 2940
   ggcgagatca tctggaccct ccaagacacc caagagatca agcaaagggt ggtgttcaag 3000
   tactcccaaa tgatcaacat ctccgactac atcaacaggt ggatcttcgt gaccatcacc 3060
   aacaacaggc tcaacaactc caagatctac atcaacggca ggctcatcga ccaaaagcca 3120
   atctccaacc tcggcaacat ccacgcctcc aacaacatca tgttcaagct cgacggctgc 3180
   agggacaccc acaggtacat ctggatcaag tacttcaacc tcttcgacaa ggagctcaac 3240
   gagaaggaga tcaaggacct ctacgacaac caatccaact ccggcatcct caaggacttc 3300
   tggggcgact acctccaata cgacaagcca tactacatgc tcaacctcta cgacccaaac 3360
   aagtacgtgg acgtgaacaa cgtgggcatc aggggctaca tgtacctcaa gggcccaagg 3420
   ggctccgtga tgaccaccaa catctacctc aactcctccc tctacagggg caccaagttc 3480
   atcatcaaga agtacgcctc cggcaacaag gacaacatcg tgaggaacaa cgacagggtg 3540
   tacatcaacg tggtggtgaa gaacaaggag tacaggctcg ccaccaacgc ctcccaagcc 3600
   ggcgtggaga agatcctctc cgccctcgag atcccagacg tgggcaacct ctcccaagtg 3660
   gtggtgatga agtccaagaa cgaccaaggc atcaccaaca agtgcaagat gaacctccaa 3720
   gacaacaacg gcaacgacat cggcttcatc ggcttccacc aattcaacaa catcgccaag 3780
   ctcgtggcct ccaactggta caacaggcaa atcgagaggt cctccaggac cctcggctgc 3840
   tcctgggagt tcatcccagt ggacgacggc tggggcgaga ggccactcta a          3891
<210> 54
   <211> 3891
   <212> DNA
   <213> Artificial Sequence
<220>
   <223> BoNT/A, T. aestivum-modified 3
<400> 54
   atgccatttg tgaataagca gtttaactac aaggaccccg ttaacggtgt ggacatcgcg 60
   tacattaaga tccctaatgc tggacaaatg caaccagtta aggccttcaa gattcacaat 120
   aagatctggg tcatccctga gagggacacc tttactaatc ccgaggaagg cgaccttaac 180
   ccgcctccgg aggcgaagca agttccagtt tcatactatg attccacgta cttgtccaca 240
   gataatgaga aggataatta cctcaagggt gttacgaagc tcttcgaaag aatctatagc 300
   accgacctag gacgcatgtt gcttacatcg attgtgcgcg gaatcccatt ctggggcggg 360
   agcaccattg atacagagct taaggtcatc gatacgaact gtattaacgt catccagccg 420
   gacggcagtt accgctccga ggaactcaat ctcgttatca ttggcccgtc ggccgacatc 480
   attcaattcg aatgcaagtc ttttggtcac gaggtgctga atctcaccag gaacggctac 540
   ggatccaccc aatacatccg gtttagccct gactttacat tcggcttcga ggaatccttg 600
   gaggtggaca ctaacccact tctaggcgcc ggcaagtttg ccaccgaccc cgctgtcacc 660
   ctggcccatg agctcatcca tgcgggccac agactctacg gaatcgcgat caacccaaac 720
   cgcgtgttca aggtgaatac taacgcttac tatgaaatgt cagggctgga agtgtctttc 780
   gaggaactcc gcacgttcgg cggtcacgac gcaaagttca tcgactcttt gcaagagaat 840
   gagtttcgct tgtattacta taataagttc aaggacatcg catccaccct gaacaaggcc 900
   aagagcatcg ttggtactac agcttccctc caatatatga agaacgtgtt caaggagaag 960
   tacctgctct cagaggacac gagcggcaag ttttccgtgg acaagctcaa gttcgacaag 1020
   ctatacaaga tgctcactga gatctacacg gaggataatt tcgtgaagtt ctttaaggtg 1080
   ctcaaccgta agacctacct caacttcgac aaggctgtct tcaagatcaa catcgtcccg 1140
   aaggtcaact acactattta cgacggattc aacctacgca acaccaacct agcagccaac 1200
   ttcaatggac agaacacgga aatcaacaat atgaacttca ctaagctgaa gaacttcacg 1260
   ggcctcttcg aattctacaa gctcctttgc gtcaggggta tcattactag taagacaaag 1320
   agcttggata aggggtacaa taaggccttg aacgacctct gcatcaaggt gaacaattgg 1380
   gacctgtttt tctcgccatc tgaagacaac tttaccaacg atcttaacaa gggagaggaa 1440
   atcacctcag acaccaacat cgaggcagcg gaggaaaaca tctcactgga tctgatccaa 1500
   cagtactatc tcaccttcaa tttcgacaac gagccggaga atatttcgat cgagaatctc 1560
   tcctcagaca tcattggcca actcgaactc atgcccaata tcgagcggtt tcctaacggt 1620
   aagaagtacg agcttgacaa gtacacaatg ttccactatc tacgtgcaca ggagttcgaa 1680
   catggaaagt ccaggatcgc cctcacgaac tcggtgaacg aggcgttgct gaacccatct 1740
   agagtctata catttttctc ctcagactac gtcaagaagg ttaacaaggc gaccgaggct 1800
   gcaatgttcc tgggttgggt cgaacagctt gtgtatgact ttaccgatga gacgtcggag 1860
   gttagcacaa ccgataagat tgctgatatc acaatcatta tcccatacat tgggcccgca 1920
   ctcaacatcg gcaatatgct ttacaaggac gatttcgtgg gggccctcat cttttccggc 1980
   gcggtcatcc ttctcgagtt tattccggag atcgcaatcc ctgttctggg caccttcgcc 2040
   ttggtgtcct acatcgccaa caaggttcta accgtgcaaa ctatcgataa cgcactctct 2100
   aagcggaatg agaagtggga cgaggtttac aagtacatcg tcacaaactg gctagccaag 2160
   gtcaatacgc aaatcgatct tatccgcaag aagatgaagg aggctctcga aaaccaagcg 2220
   gaggccacca aggcgatcat taattaccag tacaaccagt acaccgaaga ggaaaagaat 2280
   aacattaact tcaacattga tgacctttca tctaagctga acgagagcat caacaaggcc 2340
   atgatcaaca tcaacaagtt tctcaatcaa tgctcagtga gttacttgat gaactccatg 2400
   atcccatatg gagtcaagcg tctcgaggac ttcgacgcga gcctcaagga tgccctgctc 2460
   aagtacattt atgataacag gggcaccttg attggccagg tcgataggct aaaggacaag 2520
   gttaacaata ctctcagcac cgacatcccc ttccagctgt caaagtacgt cgacaaccaa 2580
   aggcttttgt ccactttcac tgagtacatc aagaacatta tcaataccag catcctgaac 2640
   ctccgttatg agtcgaacca cctgatcgat ctgtcgcgct acgcctcgaa gatcaacatt 2700
   ggaagcaagg tgaacttcga tcccatcgac aagaaccaga tccagctctt taacttggag 2760
   tccagcaaga tcgaggtgat cctgaagaac gctattgtgt ataacagtat gtacgagaat 2820
   ttctccacga gcttctggat taggatcccc aagtatttca actcgatctc cctgaacaat 2880
   gagtacacaa tcattaactg catggagaac aattcagggt ggaaggtcag cctgaactac 2940
   ggcgagatca tttggaccct ccaggacacg caagaaatca agcagcgggt ggttttcaag 3000
   tattcacaga tgatcaatat cagtgactat atcaaccggt ggatctttgt gaccatcacc 3060
   aacaatagac tcaacaattc caagatctac atcaacggca gactcattga tcaaaagcct 3120
   atttctaacc ttggtaatat tcatgcgtcg aacaatatca tgtttaagct tgacgggtgc 3180
   agagacactc accggtacat ctggatcaag tacttcaacc tctttgataa ggagctcaat 3240
   gagaaggaaa ttaaggatct gtatgataat caaagcaact ctgggatcct taaggacttt 3300
   tggggggatt acctgcaata tgataagcca tactatatgt tgaacctgta cgacccgaat 3360
   aagtatgtcg acgttaacaa tgtcgggatt aggggttaca tgtacctgaa ggggccaagg 3420
   ggctccgtga tgaccacaaa catctacttg aacagctctt tgtaccgggg cacgaagttc 3480
   atcattaaga agtacgcctc aggtaacaag gacaatatcg tccgtaacaa tgaccgcgtg 3540
   tacatcaatg tggtcgtgaa gaacaaggag tacaggctgg ctactaacgc gtctcaagcc 3600
   ggcgtggaga agatcctctc ggcactggag atcccagatg tggggaacct cagccaggtt 3660
   gtggtcatga agtctaagaa cgaccaaggc atcacaaaca agtgtaagat gaatctgcag 3720
   gataacaatg ggaacgacat cgggtttatc ggcttccatc aatttaacaa catcgctaag 3780
   ctagtggcct ccaactggta caacaggcag attgagagga gtagccgcac ccttgggtgc 3840
   agttgggaat tcatcccggt ggacgacggt tggggagaga ggccactgta a          3891
<210> 55
   <211> 3891
   <212> DNA
   <213> Artificial Sequence
<220>
   <223> BoNT/A, Z. mays-modified 1
<400> 55
   atgccctttg ttaacaagca gttcaattac aaggaccccg tcaacggtgt cgacattgcg 60
   tacattaaga tcccgaatgc cggtcaaatg cagcctgtca aggccttcaa gatccacaac 120
   aagatctggg tcatcccgga gcgggatacc ttcaccaacc cagaggaagg agatctaaat 180
   cctccgccag aagctaaaca ggtgcccgtg tcttattacg actctaccta cctgtcgacc 240
   gataacgaga aggataatta cctgaagggt gtgacgaaac tatttgagcg tatctacagc 300
   accgatctgg gcaggatgct gttgactagc atcgttagag ggattccatt ctggggcggt 360
   tctacgattg acactgagct caaagtgatc gacactaact gcataaacgt tatacaacca 420
   gacggatcct ataggtccga ggaacttaac cttgtaatta tcggtccatc tgccgacatc 480
   attcagtttg aatgtaaaag ctttggccat gaggtcctta acctcactag gaatggctac 540
   ggctctacac aatatattcg gttttcccct gacttcacct ttgggttcga ggaatcgctg 600
   gaggttgaca cgaaccccct gctcggcgca ggcaagtttg ccaccgatcc ggctgtgacg 660
   ttggcacatg aactgatcca tgcgggccac agactatatg ggatcgctat caatcccaac 720
   cgcgtcttca aggtaaatac aaatgcctac tatgagatgt ccgggctcga ggtctcgttc 780
   gaggaactga gaacattcgg cggacacgac gcgaagttca tagacagtct acaggagaac 840
   gaattccggt tatattacta taataaattt aaagacatag catcaaccct caacaaggcc 900
   aagtccatcg ttggcacgac cgcctcgtta cagtacatga aaaatgtctt caaagaaaaa 960
   tatctactct ccgaggatac cagtggcaag ttctctgttg ataagctgaa gttcgataag 1020
   ctctacaaga tgctgacaga gatctacact gaagacaact tcgtgaaatt ctttaaggtc 1080
   ctcaatagga agacatacct caatttcgat aaagcggtgt tcaagatcaa cattgtgcct 1140
   aaagtgaact acactatcta tgatggattc aatctcagaa atactaacct ggctgccaac 1200
   ttcaatggcc agaacactga gatcaacaat atgaacttca ctaagcttaa gaacttcaca 1260
   ggtctttttg aattctacaa gttgctgtgt gtacgcggca taatcacatc aaaaactaaa 1320
   tcactagaca aaggctacaa caaggcactt aacgatctct gtattaaagt caacaattgg 1380
   gacctttttt tctccccatc tgaagacaac tttacgaatg accttaacaa gggagaggaa 1440
   atcacctccg atacaaatat cgaggcggct gaagagaaca tcagtttgga cctgatccag 1500
   caatactatc tcaccttcaa cttcgataac gagcctgaaa acatttccat agagaatctg 1560
   agcagtgaca tcattggcca actagagctt atgccgaaca tcgagaggtt ccctaacggg 1620
   aagaaatatg agctggacaa gtacacgatg tttcactacc tgcgcgccca ggaatttgaa 1680
   catggcaaga gcaggatagc gttgactaat agcgttaacg aggcactcct gaacccctcg 1740
   cgagtctata cgttcttttc gtccgactac gttaaaaagg taaataaggc cacagaggct 1800
   gccatgtttc tgggatgggt tgaacagctg gtttacgact tcaccgatga aaccagcgag 1860
   gtttcaacaa ccgacaaaat tgcagatatc acaattatca taccctatat cggccctgcg 1920
   ttgaacatag gcaatatgct ctataaggac gattttgtgg gcgcgcttat attcagcggc 1980
   gctgtcatcc tccttgagtt cattccggag atagccatcc ccgtcctggg aactttcgcg 2040
   cttgtcagtt acattgcaaa taaagtgctt acggtgcaga ccattgacaa cgcgctgagt 2100
   aaaaggaacg agaagtggga tgaagtgtac aagtacattg ttaccaactg gcttgcaaag 2160
   gttaacacac aaattgacct catcagaaaa aagatgaagg aggccctcga gaaccaggct 2220
   gaggctacca aggcgattat caactaccag tacaaccagt acactgagga agagaaaaac 2280
   aatatcaatt tcaacatcga tgacctttct tcaaagctca acgagagcat caacaaagcc 2340
   atgataaaca tcaacaagtt cctcaatcag tgcagtgtca gctatttgat gaatagcatg 2400
   atcccctatg gcgtgaagcg ccttgaggac tttgacgcca gcctgaagga cgccttgtta 2460
   aagtacatct acgataaccg gggcacgctc atcggtcagg tggaccgcct gaaggataaa 2520
   gttaataaca cattgtccac ggacattcca ttccaactct cgaagtacgt ggataatcag 2580
   cgactccttt ccaccttcac agagtacatt aagaatatta taaatacttc gatccttaac 2640
   ttgaggtacg agtcgaacca cctcatcgac ctgagccggt acgctagcaa gatcaacatt 2700
   gggtcaaaag ttaatttcga cccgattgac aagaaccaga tccaactctt caatttggag 2760
   tcgtccaaga ttgaagtgat actcaagaat gcaatcgtat acaattctat gtacgagaac 2820
   ttctcaacat cattctggat tcgcattcca aagtatttta acagcatatc tctgaacaat 2880
   gagtacacga tcattaactg catggagaac aattcaggat ggaaggtgag ccttaattac 2940
   ggagagatta tctggaccct gcaggatacc caggaaatca agcaacgagt agtctttaag 3000
   tacagccaaa tgattaacat cagtgattac atcaataggt ggatcttcgt gaccatcacg 3060
   aacaatcgct tgaataactc caagatatat attaatggca gactcatcga ccagaagcca 3120
   attagcaatc tgggaaacat acacgcttct aataacataa tgtttaagtt agatggatgc 3180
   agggataccc acagatacat ttggatcaag tatttcaacc tgtttgacaa agaactgaat 3240
   gagaaggaga tcaaggacct ctacgacaac caaagcaact ctggcatttt aaaggatttt 3300
   tggggggact atttacagta tgacaagcct tactatatgc taaacctgta cgacccgaat 3360
   aaatatgtgg atgtcaacaa tgtgggtatt cgtgggtaca tgtacctaaa gggtcctcgt 3420
   gggtcagtga tgactaccaa catctatttg aacagttcac tctaccgggg gactaagttc 3480
   atcataaaga aatacgcttc cgggaacaag gataacatcg ttaggaacaa tgatcgcgtg 3540
   tacataaacg tcgttgtcaa gaacaaggag taccggttgg ccacaaacgc ttcccaggcc 3600
   ggcgtggaga agatcctctc tgcactcgag attccggatg taggtaactt gtcccaagtg 3660
   gtcgtgatga agtccaagaa tgaccagggc atcacgaata agtgcaagat gaacttacag 3720
   gacaacaatg gtaacgatat tggttttatc ggtttccatc aattcaacaa tatagcgaag 3780
   ctggtcgcgt cgaactggta taaccgtcaa atagaacgct cttcacgcac gttggggtgc 3840
   tcatgggagt tcattccggt cgacgatggg tggggagagc gtccactcta a 3891
<210> 56
   <211> 3891
   <212> DNA
   <213> Artificial Sequence
<220>
   <223> BoNT/A, Z. mays-modified 2
<400> 56
   atgccattcg tgaacaagca gttcaactac aaggacccag tgaacggcgt ggacatcgcc 60
   tacatcaaga tcccaaacgc cggccagatg cagccagtga aggccttcaa gatccacaac 120
   aagatctggg tgatcccaga gagggacacc ttcaccaacc cagaggaggg cgacctgaac 180
   ccaccaccag aggccaagca ggtgccagtg agctactacg acagcaccta cctgagcacc 240
   gacaacgaga aggacaacta cctgaagggc gtgaccaagc tgttcgagag gatctacagc 300
   accgacctgg gcaggatgct gctgaccagc atcgtgaggg gcatcccatt ctggggcggc 360
   agcaccatcg acaccgagct gaaggtgatc gacaccaact gcatcaacgt gatccagcca 420
   gacggcagct acaggagcga ggagctgaac ctggtgatca tcggcccaag cgccgacatc 480
   atccagttcg agtgcaagag cttcggccac gaggtgctga acctgaccag gaacggctac 540
   ggcagcaccc agtacatcag gttcagccca gacttcacct tcggcttcga ggagagcctg 600
   gaggtggaca ccaacccact gctgggcgcc ggcaagttcg ccaccgaccc agccgtgacc 660
   ctggcccacg agctgatcca cgccggccac aggctgtacg gcatcgccat caacccaaac 720
   agggtgttca aggtgaacac caacgcctac tacgagatga gcggcctgga ggtgagcttc 780
   gaggagctga ggaccttcgg cggccacgac gccaagttca tcgacagcct gcaggagaac 840
   gagttcaggc tgtactacta caacaagttc aaggacatcg ccagcaccct gaacaaggcc 900
   aagagcatcg tgggcaccac cgccagcctg cagtacatga agaacgtgtt caaggagaag 960
   tacctgctga gcgaggacac cagcggcaag ttcagcgtgg acaagctgaa gttcgacaag 1020
   ctgtacaaga tgctgaccga gatctacacc gaggacaact tcgtgaagtt cttcaaggtg 1080
   ctgaacagga agacctacct gaacttcgac aaggccgtgt tcaagatcaa catcgtgcca 1140
   aaggtgaact acaccatcta cgacggcttc aacctgagga acaccaacct ggccgccaac 1200
   ttcaacggcc agaacaccga gatcaacaac atgaacttca ccaagctgaa gaacttcacc 1260
   ggcctgttcg agttctacaa gctgctgtgc gtgaggggca tcatcaccag caagaccaag 1320
   agcctggaca agggctacaa caaggccctg aacgacctgt gcatcaaggt gaacaactgg 1380
   gacctgttct tcagcccaag cgaggacaac ttcaccaacg acctgaacaa gggcgaggag 1440
   atcaccagcg acaccaacat cgaggccgcc gaggagaaca tcagcctgga cctgatccag 1500
   cagtactacc tgaccttcaa cttcgacaac gagccagaga acatcagcat cgagaacctg 1560
   agcagcgaca tcatcggcca gctggagctg atgccaaaca tcgagaggtt cccaaacggc 1620
   aagaagtacg agctggacaa gtacaccatg ttccactacc tgagggccca ggagttcgag 1680
   cacggcaaga gcaggatcgc cctgaccaac agcgtgaacg aggccctgct gaacccaagc 1740
   agggtgtaca ccttcttcag cagcgactac gtgaagaagg tgaacaaggc caccgaggcc 1800
   gccatgttcc tgggctgggt ggagcagctg gtgtacgact tcaccgacga gaccagcgag 1860
   gtgagcacca ccgacaagat cgccgacatc accatcatca tcccatacat cggcccagcc 1920
   ctgaacatcg gcaacatgct gtacaaggac gacttcgtgg gcgccctgat cttcagcggc 1980
   gccgtgatcc tgctggagtt catcccagag atcgccatcc cagtgctggg caccttcgcc 2040
   ctggtgagct acatcgccaa caaggtgctg accgtgcaga ccatcgacaa cgccctgagc 2100
   aagaggaacg agaagtggga cgaggtgtac aagtacatcg tgaccaactg gctggccaag 2160
   gtgaacaccc agatcgacct gatcaggaag aagatgaagg aggccctgga gaaccaggcc 2220
   gaggccacca aggccatcat caactaccag tacaaccagt acaccgagga ggagaagaac 2280
   aacatcaact tcaacatcga cgacctgagc agcaagctga acgagagcat caacaaggcc 2340
   atgatcaaca tcaacaagtt cctgaaccag tgcagcgtga gctacctgat gaacagcatg 2400
   atcccatacg gcgtgaagag gctggaggac ttcgacgcca gcctgaagga cgccctgctg 2460
   aagtacatct acgacaacag gggcaccctg atcggccagg tggacaggct gaaggacaag 2520
   gtgaacaaca ccctgagcac cgacatccca ttccagctga gcaagtacgt ggacaaccag 2580
   aggctgctga gcaccttcac cgagtacatc aagaacatca tcaacaccag catcctgaac 2640
   ctgaggtacg agagcaacca cctgatcgac ctgagcaggt acgccagcaa gatcaacatc 2700
   ggcagcaagg tgaacttcga cccaatcgac aagaaccaga tccagctgtt caacctggag 2760
   agcagcaaga tcgaggtgat cctgaagaac gccatcgtgt acaacagcat gtacgagaac 2820
   ttcagcacca gcttctggat caggatccca aagtacttca acagcatcag cctgaacaac 2880
   gagtacacca tcatcaactg catggagaac aacagcggct ggaaggtgag cctgaactac 2940
   ggcgagatca tctggaccct gcaggacacc caggagatca agcagagggt ggtgttcaag 3000
   tacagccaga tgatcaacat cagcgactac atcaacaggt ggatcttcgt gaccatcacc 3060
   aacaacaggc tgaacaacag caagatctac atcaacggca ggctgatcga ccagaagcca 3120
   atcagcaacc tgggcaacat ccacgccagc aacaacatca tgttcaagct ggacggctgc 3180
   agggacaccc acaggtacat ctggatcaag tacttcaacc tgttcgacaa ggagctgaac 3240
   gagaaggaga tcaaggacct gtacgacaac cagagcaaca gcggcatcct gaaggacttc 3300
   tggggcgact acctgcagta cgacaagcca tactacatgc tgaacctgta cgacccaaac 3360
   aagtacgtgg acgtgaacaa cgtgggcatc aggggctaca tgtacctgaa gggcccaagg 3420
   ggcagcgtga tgaccaccaa catctacctg aacagcagcc tgtacagggg caccaagttc 3480
   atcatcaaga agtacgccag cggcaacaag gacaacatcg tgaggaacaa cgacagggtg 3540
   tacatcaacg tggtggtgaa gaacaaggag tacaggctgg ccaccaacgc cagccaggcc 3600
   ggcgtggaga agatcctgag cgccctggag atcccagacg tgggcaacct gagccaggtg 3660
   gtggtgatga agagcaagaa cgaccagggc atcaccaaca agtgcaagat gaacctgcag 3720
   gacaacaacg gcaacgacat cggcttcatc ggcttccacc agttcaacaa catcgccaag 3780
   ctggtggcca gcaactggta caacaggcag atcgagagga gcagcaggac cctgggctgc 3840
   agctgggagt tcatcccagt ggacgacggc tggggcgaga ggccactgta a          3891
<210> 57
   <211> 3891
   <212> DNA
   <213> Artificial Sequence
<220>
   <223> BoNT/A, Z. mays-modified 3
<400> 57
   atgccatttg tgaataagca attcaactac aaggatcccg tcaacggggt tgacatagct 60
   tacatcaaaa tcccgaatgc tggacagatg cagcctgtta aggccttcaa gatccacaac 120
   aagatttggg ttatcccgga aagggatacc tttactaacc cagaagaggg tgatctgaac 180
   ccacctccgg aggcaaagca agttcctgtg agctactatg attcaaccta tctgagtacc 240
   gataatgaaa aggataatta cctgaagggg gtgactaagc tctttgagcg gatctactcc 300
   accgacctgg ggaggatgct tctgacgagc atagtcaggg gtatcccatt ttggggcggt 360
   tccactatcg ataccgagct gaaagtgatc gacacaaatt gcataaacgt gatacaaccc 420
   gacggctcgt acagatccga agagctgaac cttgttatca ttggcccgtc cgccgacatc 480
   atacagttcg aatgtaaatc attcggtcac gaagtgctta acttgaccag aaatggatac 540
   ggctccacgc agtatattcg cttctcccca gactttacct tcggttttga agagagcctg 600
   gaggtggaca cgaatcctct cctgggggca ggcaagttcg ctaccgaccc cgcagtcacc 660
   ctggctcatg agctgattca tgccggacac aggctgtacg gtatcgccat taatccaaat 720
   agagtgttca aagtcaacac caacgcatat tacgagatgt ctggactcga ggtttctttc 780
   gaggaactgc gcacatttgg gggccacgat gcaaagttca tcgacagcct ccaagagaat 840
   gagttccgcc tctactatta caataagttc aaggatatcg cgagtactct gaacaaggcc 900
   aagtcgatcg ttggcacaac ggcctcattg cagtacatga aaaacgtgtt caaggaaaag 960
   tatttgctct cagaggacac ctcagggaag ttttcagttg ataaactcaa gttcgacaag 1020
   ctttacaaga tgctgaccga gatctacacc gaggacaatt ttgtgaagtt ctttaaagtc 1080
   ctgaaccgga agacatattt gaacttcgat aaggctgtct tcaagatcaa tatcgtgccg 1140
   aaggtgaact acaccatcta cgatggattt aacttgcgga acactaacct tgctgcgaac 1200
   tttaatggcc aaaataccga aatcaacaat atgaatttca ccaagcttaa gaacttcaca 1260
   ggcctcttcg agttctacaa gctgttgtgc gtgcgtggta taattacatc taagactaaa 1320
   tcactcgaca agggctacaa caaagcactc aatgacctgt gcattaaagt gaacaattgg 1380
   gacctgttct ttagcccgtc ggaagacaat ttcacaaacg acttgaacaa aggcgaggaa 1440
   atcacatctg ataccaatat cgaggcggct gaagagaaca tatccctcga cctgatacaa 1500
   cagtactatc tcacatttaa tttcgataac gagccggaga acattagcat tgagaacctg 1560
   agctctgaca ttatcgggca acttgagctc atgcctaata tcgaaagatt cccgaatggg 1620
   aagaaatatg agcttgacaa gtatactatg tttcattacc ttagggctca agagtttgag 1680
   catggtaaat cacggatcgc gctcacgaat agtgtcaacg aggcactctt gaacccgtcc 1740
   cgcgtctaca cattcttttc cagcgattac gtgaaaaagg tcaataaggc cacagaagcc 1800
   gcgatgttcc tcggatgggt tgagcagctg gtctacgact tcactgacga aacatcagaa 1860
   gtgtccacta cggataagat tgccgatatt accattatca taccatatat tgggccagct 1920
   ctgaacattg gaaacatgct gtacaaagac gatttcgttg gggcgcttat cttctctgga 1980
   gccgttatac tgcttgaatt tatccccgag atcgcaatac ccgttctcgg cacctttgct 2040
   ctggtttcat atatcgcgaa caaagttctg acggtgcaga caatcgataa tgcgcttagt 2100
   aagaggaacg agaagtggga tgaagtgtat aaatacatag tgactaattg gttggcaaaa 2160
   gtgaatacac agatagacct gatccgtaaa aagatgaagg aggccctcga aaatcaagcg 2220
   gaggcgacca aggccatcat taactaccag tataaccaat acacggagga agagaaaaac 2280
   aatatcaact tcaacattga tgacctctct agtaagctca atgagtctat aaacaaggcg 2340
   atgattaaca tcaataagtt tcttaatcag tgctctgtca gctacctgat gaactctatg 2400
   attccatacg gcgtcaagcg cttggaagac tttgatgcta gcttgaagga cgcccttctc 2460
   aagtacatct acgacaatag gggaacactc atcggtcagg tcgaccgcct caaggacaaa 2520
   gtcaataaca cgctgtccac ggatattccg tttcagctct cgaagtatgt cgacaaccag 2580
   aggttgctgt cgaccttcac tgagtatatc aagaacataa tcaacacatc catcctgaat 2640
   ttgaggtacg agtctaacca ccttatcgat ctgtcaagat acgccagtaa gatcaacatt 2700
   ggaagtaaag tcaatttcga ccccattgac aaaaaccaga tccagctgtt caatctggag 2760
   tcgagcaaaa tcgaggttat tctgaagaac gcgatcgtgt acaacagcat gtacgagaac 2820
   tttagcacct cgttctggat aaggatccct aagtacttca attcgattag cctgaacaat 2880
   gaatacacga tcattaactg tatggagaac aattccggct ggaaggtgtc actcaactat 2940
   ggcgagatta tctggactct ccaggacacg caggagatca agcagcgcgt ggtcttcaag 3000
   tactcccaga tgataaacat tagtgattat attaaccgtt ggatattcgt gactataact 3060
   aacaatcggc tcaataactc taagatctac atcaacggta gactgattga tcaaaaacct 3120
   atcagcaacc ttggcaacat acatgcgagc aacaatatta tgttcaaact ggacggctgt 3180
   agggacacgc accggtacat ttggatcaag tacttcaatt tgttcgacaa ggagctcaac 3240
   gagaaggaga tcaaagatct ctacgacaac caaagcaact cggggatcct caaggacttt 3300
   tggggggact acctgcaata cgataagccc tactatatgc tgaacttgta tgaccctaac 3360
   aagtacgtgg atgtgaacaa tgtcggcatt cgcggctaca tgtacctgaa gggccctaga 3420
   gggagtgtga tgactacgaa catttatctg aactcgtccc tttatcgcgg aaccaaattc 3480
   attatcaaga aatacgcctc tggcaacaag gacaacattg tccgtaacaa tgatcgcgtg 3540
   tatataaacg tggtcgttaa gaacaaggaa tatcgtctgg caacgaacgc ttcgcaggcc 3600
   ggcgtggaga agattttgag cgccctcgag atccccgatg tgggaaatct ttctcaggtt 3660
   gtggtcatga agtccaagaa cgaccaaggt attaccaata agtgcaagat gaacctgcag 3720
   gataacaatg gcaacgacat cggtttcata ggcttccacc agttcaacaa tatcgctaag 3780
   cttgtggcca gcaattggta taacaggcag atcgaaaggt cctcaaggac tctgggctgc 3840
   tcctgggagt tcattcccgt cgacgatggt tggggcgagc ggccactgta a          3891
<210> 58
   <211> 3891
   <212> DNA
   <213> Artificial Sequence
<220>
   <223> BoNT/A, D. melanogaster-modified 1
<400> 58
   atgccgttcg tcaacaagca atttaattat aaagatcccg ttaacggtgt ggatatcgct 60
   tatataaaaa ttccaaacgc tgggcagatg caaccagtga aagcctttaa gatccataat 120
   aagatctggg ttataccgga gcgggacaca tttacaaacc ccgaagaggg agacctgaac 180
   cccccgcccg aagcgaagca agtaccggtt tcctattacg actcgaccta cctgtcgact 240
   gacaatgaga aggataacta cttgaagggt gtgaccaagc tcttcgaacg tatatatagc 300
   accgacctgg gacgcatgtt gctcacgtcc attgtgcgtg gcatcccctt ctggggtggc 360
   tccacgatag acacagagct gaaggtgatc gacactaatt gtatcaatgt aatccagccg 420
   gacggatcgt atcgtagcga ggaactgaat ttagtgatca ttggtccgag tgccgatatc 480
   attcaattcg agtgcaagag tttcggacat gaggtcttga acctgacgcg gaatggctat 540
   ggtagcaccc agtacatccg gtttagcccc gatttcacct tcggctttga ggaaagtctt 600
   gaggtcgata ctaacccact tctcggcgca ggcaagttcg ccaccgaccc tgccgttact 660
   ctagcccacg agctaattca cgcgggccat cgtctgtacg gaattgctat aaaccctaat 720
   cgagtgttca aggtgaacac caatgcttac tatgaaatgt ccgggctcga ggtcagcttc 780
   gaggaactgc gcacgttcgg agggcacgat gcgaaattca ttgatagtct ccaggagaat 840
   gagtttcgct tgtactatta caacaagttc aaggatatcg cctccaccct taacaaggcg 900
   aagtcgatag ttggaaccac tgcatcacta cagtatatga aaaacgtctt taaggaaaag 960
   tatcttctga gcgaggacac ttctggcaag ttcagcgtgg acaagttgaa gttcgacaag 1020
   ctgtacaaga tgctcaccga gatctacacc gaggacaact ttgtgaagtt tttcaaggta 1080
   ctgaaccgca agacatatct gaatttcgac aaagccgtgt tcaagatcaa catcgtgccg 1140
   aaggtgaatt atacaatcta tgatggcttc aacctgcgaa atactaacct cgccgctaat 1200
   ttcaacggac agaatacgga gataaacaat atgaacttca ctaagctcaa aaacttcaca 1260
   ggattattcg aattttacaa gctcctttgt gtaagaggca tcattacgtc taagactaaa 1320
   agtctggata agggttacaa caaggcactg aatgacctct gcataaaggt gaataactgg 1380
   gacttgttct ttagcccgtc ggaggacaac tttacgaatg acctaaataa gggtgaggaa 1440
   ataacgagcg atacaaacat tgaggcggcc gaggaaaata tatcgttgga tctgattcag 1500
   caatactatc ttacttttaa ctttgacaat gagcccgaaa atatttcgat tgagaacctt 1560
   agttccgata tcattggtca gcttgagctg atgccgaaca tcgagcgatt cccaaatggc 1620
   aaaaagtacg aactcgacaa gtacaccatg ttccactatt tgcgcgcaca ggagttcgag 1680
   cacggaaagt cacggatcgc actcaccaac tctgtgaacg aggccttgct gaacccttca 1740
   cgcgtctaca cattcttttc atcggattac gtgaagaaag ttaacaaggc caccgaggcc 1800
   gcaatgttcc tgggctgggt agaacagctg gtctatgatt tcacggacga aacctctgag 1860
   gttagcacca cggataaaat cgccgatatc acaattatca taccatacat cggccccgcg 1920
   ctgaacatcg gcaacatgct gtacaaggat gactttgtgg gtgcacttat cttctccggc 1980
   gccgtcattt tgttagaatt tataccggag atagctattc cagttctggg aacctttgcg 2040
   ctggtatcct acatcgccaa taaggttttg actgtccaaa caattgacaa cgccctctcc 2100
   aagcgaaacg aaaagtggga tgaggtctac aaatatattg ttaccaattg gctagctaag 2160
   gttaacacgc aaatcgatct gattcgcaag aaaatgaagg aggctctgga gaaccaagcc 2220
   gaggcgacca aagctatcat taactaccag tacaatcaat acacagagga agagaagaat 2280
   aacatcaact ttaacatcga cgatctgtcg agcaagttga acgagtcgat taacaaagct 2340
   atgataaaca taaataagtt tttgaaccag tgttcagtga gctatctaat gaactcgatg 2400
   atcccatacg gagtgaagag gttggaggat ttcgatgcgt cactgaagga tgccctccta 2460
   aaatacatct atgacaaccg tggcacgctc atcggccaag tggatagact gaaagataag 2520
   gtaaataaca cgctaagcac agatattccg tttcagctca gcaagtacgt ggataaccag 2580
   cgtttgctgt cgacattcac ggagtatatt aaaaatatca taaacacctc cattctgaac 2640
   ttgcgctacg agagcaacca cctgatcgac ctgtcccggt acgcctccaa gattaacatc 2700
   ggtagtaaag tgaacttcga tcccatcgat aaaaatcaga ttcagctctt caatctggag 2760
   tccagtaaga tcgaggtgat cctgaagaat gcaattgtgt acaattccat gtatgaaaat 2820
   ttcagtacca gcttttggat ccgaatcccc aagtacttta actccatctc attgaataac 2880
   gaatatacga tcattaattg catggagaat aactctggct ggaaagtgag cctgaattac 2940
   ggagaaatta tatggacact tcaggatacg caggaaatta aacagcgcgt ggtttttaag 3000
   tacagccaga tgatcaacat ctcggactat atcaatcgtt ggatcttcgt caccatcacc 3060
   aataacaggt taaacaattc caagatatac ataaatggta ggctgattga ccagaagcca 3120
   atttcgaatc tgggcaacat tcatgcatcg aacaatatca tgttcaagtt ggatggctgc 3180
   cgcgacacgc atcgatacat ttggataaaa tactttaact tattcgacaa ggaacttaat 3240
   gaaaaagaga tcaaggatct gtacgataat cagagcaata gcggaatcct gaaagacttc 3300
   tggggcgatt atctccagta cgataagcct tactatatgc tgaatctgta cgaccccaat 3360
   aaatacgtgg atgtcaataa cgtgggcata cgcggctaca tgtacctgaa gggaccccgc 3420
   gggtccgtga tgacgaccaa tatttatctg aacagttcct tgtaccgggg caccaagttc 3480
   ataattaaaa agtacgcttc tggtaataag gacaacatcg tcagaaacaa tgacagggtg 3540
   tacatcaatg tcgtagtgaa gaacaaagag tatcgactag ccaccaacgc ctcccaggcc 3600
   ggagttgaga aaatcctgtc ggccctggaa atccctgatg tggggaacct gtcccaagtg 3660
   gtcgtgatga agagcaagaa cgaccaggga attactaata agtgcaaaat gaacttgcag 3720
   gataataacg gtaatgatat tggcttcata ggcttccacc aattcaacaa tattgcgaaa 3780
   ctagtcgcct cgaactggta caatcgccag atcgagcgct caagtagaac cctgggatgc 3840
   tcttgggagt ttattcccgt cgacgacggt tggggcgaga ggccattata a          3891
<210> 59
   <211> 3891
   <212> DNA
   <213> Artificial Sequence
<220>
   <223> BoNT/A, D. melanogaster-modified 2
<400> 59
   atgcccttcg tgaacaagca gttcaactac aaggatcccg tgaacggcgt ggatatcgcc 60
   tacatcaaga tccccaacgc cggccagatg cagcccgtga aggccttcaa gatccacaac 120
   aagatctggg tgatccccga gcgcgatacc ttcaccaacc ccgaggaggg cgatctgaac 180
   cccccccccg aggccaagca ggtgcccgtg agctactacg atagcaccta cctgagcacc 240
   gataacgaga aggataacta cctgaagggc gtgaccaagc tgttcgagcg catctacagc 300
   accgatctgg gccgcatgct gctgaccagc atcgtgcgcg gcatcccctt ctggggcggc 360
   agcaccatcg ataccgagct gaaggtgatc gataccaact gcatcaacgt gatccagccc 420
   gatggcagct accgcagcga ggagctgaac ctggtgatca tcggccccag cgccgatatc 480
   atccagttcg agtgcaagag cttcggccac gaggtgctga acctgacccg caacggctac 540
   ggcagcaccc agtacatccg cttcagcccc gatttcacct tcggcttcga ggagagcctg 600
   gaggtggata ccaaccccct gctgggcgcc ggcaagttcg ccaccgatcc cgccgtgacc 660
   ctggcccacg agctgatcca cgccggccac cgcctgtacg gcatcgccat caaccccaac 720
   cgcgtgttca aggtgaacac caacgcctac tacgagatga gcggcctgga ggtgagcttc 780
   gaggagctgc gcaccttcgg cggccacgat gccaagttca tcgatagcct gcaggagaac 840
   gagttccgcc tgtactacta caacaagttc aaggatatcg ccagcaccct gaacaaggcc 900
   aagagcatcg tgggcaccac cgccagcctg cagtacatga agaacgtgtt caaggagaag 960
   tacctgctga gcgaggatac cagcggcaag ttcagcgtgg ataagctgaa gttcgataag 1020
   ctgtacaaga tgctgaccga gatctacacc gaggataact tcgtgaagtt cttcaaggtg 1080
   ctgaaccgca agacctacct gaacttcgat aaggccgtgt tcaagatcaa catcgtgccc 1140
   aaggtgaact acaccatcta cgatggcttc aacctgcgca acaccaacct ggccgccaac 1200
   ttcaacggcc agaacaccga gatcaacaac atgaacttca ccaagctgaa gaacttcacc 1260
   ggcctgttcg agttctacaa gctgctgtgc gtgcgcggca tcatcaccag caagaccaag 1320
   agcctggata agggctacaa caaggccctg aacgatctgt gcatcaaggt gaacaactgg 1380
   gatctgttct tcagccccag cgaggataac ttcaccaacg atctgaacaa gggcgaggag 1440
   atcaccagcg ataccaacat cgaggccgcc gaggagaaca tcagcctgga tctgatccag 1500
   cagtactacc tgaccttcaa cttcgataac gagcccgaga acatcagcat cgagaacctg 1560
   agcagcgata tcatcggcca gctggagctg atgcccaaca tcgagcgctt ccccaacggc 1620
   aagaagtacg agctggataa gtacaccatg ttccactacc tgcgcgccca ggagttcgag 1680
   cacggcaaga gccgcatcgc cctgaccaac agcgtgaacg aggccctgct gaaccccagc 1740
   cgcgtgtaca ccttcttcag cagcgattac gtgaagaagg tgaacaaggc caccgaggcc 1800
   gccatgttcc tgggctgggt ggagcagctg gtgtacgatt tcaccgatga gaccagcgag 1860
   gtgagcacca ccgataagat cgccgatatc accatcatca tcccctacat cggccccgcc 1920
   ctgaacatcg gcaacatgct gtacaaggat gatttcgtgg gcgccctgat cttcagcggc 1980
   gccgtgatcc tgctggagtt catccccgag atcgccatcc ccgtgctggg caccttcgcc 2040
   ctggtgagct acatcgccaa caaggtgctg accgtgcaga ccatcgataa cgccctgagc 2100
   aagcgcaacg agaagtggga tgaggtgtac aagtacatcg tgaccaactg gctggccaag 2160
   gtgaacaccc agatcgatct gatccgcaag aagatgaagg aggccctgga gaaccaggcc 2220
   gaggccacca aggccatcat caactaccag tacaaccagt acaccgagga ggagaagaac 2280
   aacatcaact tcaacatcga tgatctgagc agcaagctga acgagagcat caacaaggcc 2340
   atgatcaaca tcaacaagtt cctgaaccag tgcagcgtga gctacctgat gaacagcatg 2400
   atcccctacg gcgtgaagcg cctggaggat ttcgatgcca gcctgaagga tgccctgctg 2460
   aagtacatct acgataaccg cggcaccctg atcggccagg tggatcgcct gaaggataag 2520
   gtgaacaaca ccctgagcac cgatatcccc ttccagctga gcaagtacgt ggataaccag 2580
   cgcctgctga gcaccttcac cgagtacatc aagaacatca tcaacaccag catcctgaac 2640
   ctgcgctacg agagcaacca cctgatcgat ctgagccgct acgccagcaa gatcaacatc 2700
   ggcagcaagg tgaacttcga tcccatcgat aagaaccaga tccagctgtt caacctggag 2760
   agcagcaaga tcgaggtgat cctgaagaac gccatcgtgt acaacagcat gtacgagaac 2820
   ttcagcacca gcttctggat ccgcatcccc aagtacttca acagcatcag cctgaacaac 2880
   gagtacacca tcatcaactg catggagaac aacagcggct ggaaggtgag cctgaactac 2940
   ggcgagatca tctggaccct gcaggatacc caggagatca agcagcgcgt ggtgttcaag 3000
   tacagccaga tgatcaacat cagcgattac atcaaccgct ggatcttcgt gaccatcacc 3060
   aacaaccgcc tgaacaacag caagatctac atcaacggcc gcctgatcga tcagaagccc 3120
   atcagcaacc tgggcaacat ccacgccagc aacaacatca tgttcaagct ggatggctgc 3180
   cgcgataccc accgctacat ctggatcaag tacttcaacc tgttcgataa ggagctgaac 3240
   gagaaggaga tcaaggatct gtacgataac cagagcaaca gcggcatcct gaaggatttc 3300
   tggggcgatt acctgcagta cgataagccc tactacatgc tgaacctgta cgatcccaac 3360
   aagtacgtgg atgtgaacaa cgtgggcatc cgcggctaca tgtacctgaa gggcccccgc 3420
   ggcagcgtga tgaccaccaa catctacctg aacagcagcc tgtaccgcgg caccaagttc 3480
   atcatcaaga agtacgccag cggcaacaag gataacatcg tgcgcaacaa cgatcgcgtg 3540
   tacatcaacg tggtggtgaa gaacaaggag taccgcctgg ccaccaacgc cagccaggcc 3600
   ggcgtggaga agatcctgag cgccctggag atccccgatg tgggcaacct gagccaggtg 3660
   gtggtgatga agagcaagaa cgatcagggc atcaccaaca agtgcaagat gaacctgcag 3720
   gataacaacg gcaacgatat cggcttcatc ggcttccacc agttcaacaa catcgccaag 3780
   ctggtggcca gcaactggta caaccgccag atcgagcgca gcagccgcac cctgggctgc 3840
   agctgggagt tcatccccgt ggatgatggc tggggcgagc gccccctgta a 3891
<210> 60
   <211> 3891
   <212> DNA
   <213> Artificial Sequence
<220>
   <223> BoNT/A, D. melanogaster-modified 3
<400> 60
   atgcctttcg tgaataaaca gtttaactat aaagatcccg ttaacggtgt ggacattgct 60
   tacattaaaa tacccaacgc cggtcagatg caacctgtta aagccttcaa gatccataat 120
   aagatctggg tgattcccga gcgggacact ttcacaaacc cggaggaagg cgacctaaac 180
   ccgccacccg aggctaaaca ggtgccggtt agctattacg atagcacata cctgagcact 240
   gacaacgaaa aggataacta tctgaagggt gttacgaagt tgttcgagcg catttacagc 300
   actgacctgg gccgcatgct gttgacatcc atcgttcgag gtattccatt ttggggcgga 360
   tccacgatag acactgagtt gaaggtgata gatacgaatt gcataaacgt catccagccc 420
   gatggatctt accgttctga ggaactgaac ttggtgataa tcggtccgtc ggccgacatc 480
   attcagtttg aatgcaagag tttcggccat gaagtcctga acctgactcg caacggctat 540
   ggctcaacac aatatattag gttctcgccg gatttcacct tcggcttcga ggaatcgctg 600
   gaggtcgata ccaacccact gctaggcgcc ggtaagtttg cgaccgaccc cgccgtgacc 660
   cttgcgcacg agctgatcca cgctggacac aggctatatg gaatagctat caacccgaac 720
   cgcgtgttta aggtcaacac caacgcgtac tatgaaatga gtggattgga ggtgagcttc 780
   gaagagctgc gcacatttgg aggccatgac gccaagttca ttgactccct acaagaaaat 840
   gagtttcgtc tctactatta caataagttt aaggatatcg ctagtaccct taataaggca 900
   aaatccattg tgggaacgac cgcgtcgctg cagtatatga agaacgtgtt caaggaaaaa 960
   tatctgctct ccgaagacac aagcggaaag tttagcgttg ataaacttaa gtttgataaa 1020
   ctgtacaaaa tgcttacaga gatctacacc gaggataatt ttgtgaagtt ctttaaggtc 1080
   ctgaatagaa agacgtattt gaactttgat aaggccgtgt tcaagatcaa tattgtgccc 1140
   aaggtgaatt atacaattta tgacggcttc aacctccgga ataccaacct cgctgccaac 1200
   ttcaacggcc agaataccga gattaacaat atgaacttca cgaagctgaa gaattttact 1260
   ggactcttcg aattctataa actgctatgc gtgaggggca tcataacgag caagaccaaa 1320
   agcctggaca agggttacaa taaagccttg aacgatttgt gtatcaaagt gaataactgg 1380
   gatctctttt tctccccgtc tgaagacaac tttaccaatg atctgaacaa gggcgaggaa 1440
   ataacttctg acacgaacat cgaagccgca gaggaaaaca tttcccttga cctgatccag 1500
   caatactatt tgacgtttaa tttcgacaac gagccggaga acattagcat cgaaaacctg 1560
   agttcggata tcataggcca actggaactt atgcccaata tcgagcgatt cccgaatggc 1620
   aaaaagtacg aactggataa atacactatg ttccactatt tgcgagctca ggagtttgag 1680
   cacggcaaga gccgtatcgc cctgactaac tcagtcaatg aggcgctcct gaacccatca 1740
   agagtgtata catttttcag ctctgattac gtgaaaaagg tgaacaaagc aactgaggcg 1800
   gcaatgttcc tcggttgggt ggaacaactg gtgtacgatt tcaccgacga gacgtcggag 1860
   gtgtccacga ccgacaagat tgccgacata accatcatta tcccctacat tggacccgca 1920
   ctaaatatcg gaaacatgct gtacaaggat gactttgtcg gagcactgat cttttccgga 1980
   gccgtgatcc tcctggaatt cattcctgag attgccattc cagtgctcgg cacatttgcc 2040
   ctggtgagct acatcgccaa taaggtgctg acggtccaaa ccatagataa cgcgcttagt 2100
   aagcgaaatg agaaatggga tgaggtctac aaatatatcg tgacaaattg gctggccaag 2160
   gtgaacaccc agattgacct gatccgaaag aaaatgaagg aagccctgga aaatcaagct 2220
   gaggcaacga aggctatcat taattatcag tacaatcaat acaccgagga agagaaaaac 2280
   aatattaact tcaacataga cgatctctcg agtaagttga atgagagtat taacaaagcg 2340
   atgatcaata taaataaatt cctgaaccag tgcagcgtct cctacttgat gaattccatg 2400
   ataccctatg gcgtcaagcg cctggaggat ttcgatgcaa gcctgaagga cgcgctgctc 2460
   aagtacattt acgataatcg cggcaccttg atcggccagg tggaccgcct gaaggataag 2520
   gtgaataaca cgctgagtac cgacattccc ttccagttgt caaagtacgt ggataaccag 2580
   cgactccttt cgaccttcac ggagtatatc aagaatatca ttaataccag tatactgaac 2640
   ctgcggtatg agtccaacca cctaattgat ctgtcgaggt atgctagtaa gattaacatt 2700
   ggcagcaagg tcaattttga tccgatagat aaaaaccaga tccagctgtt caacctggag 2760
   tccagcaaga tcgaggttat actgaagaac gccatcgtgt acaactccat gtatgagaac 2820
   ttcagcacct ccttctggat aagaatcccc aagtacttca attcgatctc gctgaacaat 2880
   gagtacacga taatcaactg tatggagaac aattcgggct ggaaagtttc actcaattac 2940
   ggtgagatca tttggactct gcaggacact caggagatca agcagcgcgt tgtgtttaag 3000
   tattctcaga tgatcaacat ctcggattac atcaatcggt ggatcttcgt gaccatcacg 3060
   aacaatcggc tgaataactc gaagatctat attaacggtc gcctgatcga ccagaaacca 3120
   atcagtaact tgggaaacat acacgccagc aataacatca tgtttaagct cgatggttgt 3180
   cgcgacaccc atcgttacat ctggattaaa tacttcaatc ttttcgataa ggagcttaat 3240
   gagaaggaga ttaaggattt gtacgataat cagtccaatt caggaattct gaaggatttc 3300
   tggggcgact accttcagta cgacaaacca tactatatgc taaatctata cgacccgaac 3360
   aagtacgttg acgtgaacaa tgtcggtatt cgcggctaca tgtacttgaa gggccctcgt 3420
   ggctcggtca tgaccacgaa tatttacctc aactcgtcat tgtaccgcgg aacaaaattc 3480
   ataatcaaga aatacgcctc aggaaacaag gataacatag ttaggaataa cgaccgtgtc 3540
   tacatcaacg tggtcgtgaa gaacaaggag taccgcttgg ccacaaatgc aagccaggcc 3600
   ggcgtggaga agatactgtc cgccctagag atcccagacg tcggtaacct ctcccaggtg 3660
   gttgtgatga agtccaagaa cgatcagggc attaccaata agtgcaagat gaatctgcaa 3720
   gataataacg gcaacgacat cggcttcatt ggattccatc aatttaataa catcgctaag 3780
   ttggtggcgt ctaattggta caatcgtcaa atcgaaagat ccagccggac actgggctgc 3840
   tcgtgggagt tcattcctgt tgacgatggc tggggtgagc gaccactcta a          3891
<210> 61
   <211> 3891
   <212> DNA
   <213> Artificial Sequence
<220>
   <223> BoNT/A, S. frugiperda-modified 1
<400> 61
   atgcctttcg ttaacaagca atttaactac aaggacccgg tcaatggagt tgacattgct 60
   tacatcaaaa ttcccaacgc tgggcagatg cagcctgtta aggcgttcaa aatccacaat 120
   aagatatggg tgatcccaga acgcgatacg ttcactaacc ccgaggaagg cgacttgaac 180
   ccgccacctg aagcgaaaca agttccagtg agctattacg actccacata cttgtctacg 240
   gataacgaga aagataacta tctgaagggc gttactaagc tcttcgaaag gatttattcc 300
   actgacctcg gtcgcatgct gctcacaagc attgtgcgcg gtatcccttt ctggggtgga 360
   tccacgatcg ataccgagct caaggtgatc gataccaact gtatcaacgt tattcagccc 420
   gacggctcct atcgtagtga ggaactgaat ttggtcatca ttggcccctc ggctgatatt 480
   atccaattcg aatgtaagtc gttcgggcac gaggtcttaa acctcacgcg taatggttac 540
   ggttccacgc agtacattcg attctcgccc gacttcactt ttggctttga agagtcgttg 600
   gaggtggaca ctaacccctt gctgggtgcc ggtaaattcg cgacggaccc cgctgtaacg 660
   ctcgcgcacg agctaatcca tgcaggacat aggctttacg gaatcgcgat caaccccaac 720
   cgtgtattta aagttaacac caacgcctac tatgaaatgt ccggacttga ggtcagcttt 780
   gaggaactca ggacctttgg cgggcacgac gcaaagttca tcgactcact gcaagaaaat 840
   gagttcagac tgtactatta caacaagttc aaggatatcg cttctaccct taacaaggcg 900
   aagagcatcg tcggaactac cgcctctctc caatacatga agaacgtctt caaggagaaa 960
   tacctgttat cagaggacac aagtggcaag ttctcagtgg acaagttaaa gttcgacaaa 1020
   ctgtacaaaa tgttgactga aatctacacc gaagataatt tcgtgaagtt ctttaaggtt 1080
   ttgaaccgca agacatactt gaactttgat aaggcagtgt tcaagataaa catcgtacct 1140
   aaggtcaact acacaattta cgacggtttc aaccttcgta acacaaatct cgcggccaac 1200
   tttaacggtc aaaacacaga gatcaataac atgaacttta ccaaactaaa gaatttcacc 1260
   ggtcttttcg agttctacaa attgctatgc gtacgtggaa taatcacgtc caagactaag 1320
   tcacttgata agggttataa taaggctttg aacgacctgt gcatcaaagt taacaattgg 1380
   gacttattct tttctccatc agaggataac ttcacaaacg accttaacaa gggtgaagag 1440
   atcacttctg acaccaatat cgaagcagct gaggaaaaca tttccctgga tctgatccag 1500
   caatactatt taaccttcaa cttcgataac gagcctgaga acatctcgat tgaaaatctc 1560
   agtagcgata tcattggcca actcgaactt atgccaaaca ttgagaggtt cccaaacggc 1620
   aagaaatacg agttggacaa gtacactatg ttccattact tgcgggcgca agaattcgaa 1680
   cacggcaaat cccgaatcgc cctaactaat agcgtcaatg aggcactctt gaacccctca 1740
   cgtgtctaca cattcttttc ttcagactac gtgaagaaag tgaataaggc cacggaggcc 1800
   gcgatgtttc ttggttgggt tgagcaactc gtatacgact tcactgacga gacttccgag 1860
   gtgtctacta ccgacaaaat cgctgacatc accatcatta tcccttacat aggtccagca 1920
   ctgaacattg gaaacatgct gtataaggat gactttgttg gtgctttgat tttctctggt 1980
   gctgtgatcc tgttagagtt catccccgag atcgccatcc ctgtgctagg aaccttcgcc 2040
   ctggtgagct acattgctaa caaggtgctg acagtccaga caatcgataa cgccttgtcg 2100
   aagaggaacg agaagtggga cgaggtctac aaatatattg tgacaaattg gttggcgaag 2160
   gtgaatacac agatcgactt gattcggaaa aagatgaagg aagccctgga gaaccaggct 2220
   gaagctacaa aggccatcat taactaccag tataaccaat acacggagga agagaaaaac 2280
   aatataaatt ttaacatcga tgacctaagt agcaagttaa atgagtccat caacaaagcc 2340
   atgattaaca tcaataaatt cctgaaccag tgctcggtca gctatctgat gaatagtatg 2400
   atcccctatg gtgttaaaag attagaagac ttcgatgcaa gtcttaagga tgccttgctt 2460
   aagtacatct acgacaaccg cggcacgttg atcggtcaag tcgatcgtct gaaggacaag 2520
   gttaacaata ccctctccac cgatatacct tttcagctct cgaagtatgt agacaaccag 2580
   agactcctgt caacttttac cgagtatata aagaacatca ttaacacctc gatcctgaat 2640
   cttcgttacg aatccaatca cctgattgac ctttcccgct acgcttcgaa gataaacata 2700
   ggcagcaaag taaactttga cccgatcgac aagaaccaaa ttcagctctt caatctggag 2760
   agcagtaaga tcgaggtgat cctgaagaac gcaatcgtgt ataacagtat gtacgagaac 2820
   ttctctacct ccttctggat tcgtatccca aagtacttca attcaatcag cctgaacaat 2880
   gagtacacca tcataaactg tatggaaaac aattctggat ggaaagtaag cctaaactac 2940
   ggtgaaatta tctggacact gcaggacact caggagatta agcagagggt cgtattcaag 3000
   tactctcaga tgattaacat atccgattac atcaaccgat ggatattcgt gaccattacc 3060
   aacaatcgac taaacaattc caagatatac attaacggac gcctgatcga ccaaaaaccg 3120
   atctcaaacc tcggaaacat tcacgcttct aacaatataa tgttcaaatt ggacggctgc 3180
   agagacactc atagatatat ttggatcaaa tacttcaacc tgttcgacaa ggaattgaat 3240
   gaaaaagaga ttaaggatct ctacgataat cagtctaact ccggaatcct gaaggatttc 3300
   tggggtgatt atctccagta cgacaagcct tactatatgc taaacttgta cgacccgaac 3360
   aagtacgtgg acgtgaacaa tgtgggcatc cgcggttaca tgtacctgaa aggacctcgt 3420
   ggttcagtca tgaccactaa catctacctg aactctagtc tgtaccgcgg caccaagttc 3480
   atcattaaaa agtacgctag cggaaacaaa gataatatcg tgcgcaacaa tgacagggtc 3540
   tacatcaacg tcgttgtcaa aaacaaggag tacaggctcg ctactaacgc ttcacaggcc 3600
   ggagtggaga agatcctcag cgccctcgaa atccccgacg tgggcaacct gagtcaggtc 3660
   gtagtcatga agtcaaagaa tgaccagggc atcaccaaca agtgcaagat gaaccttcag 3720
   gacaacaatg gcaacgatat tggcttcatc ggattccacc aattcaacaa catcgcaaag 3780
   ctcgtagctt ccaactggta caacaggcag atcgagagat cttcccgtac cctgggctgc 3840
   tcttgggaat tcatcccggt cgacgatgga tggggtgaaa gaccactcta a          3891
<210> 62
   <211> 3891
   <212> DNA
   <213> Artificial Sequence
<220>
   <223> BoNT/A, S. frugiperda-modified 2
<400> 62
   atgcccttcg tgaacaagca gttcaactac aaggaccccg tgaacggtgt ggacatcgct 60
   tacatcaaga tccccaacgc tggtcagatg cagcccgtga aggctttcaa gatccacaac 120
   aagatctggg tgatccccga gcgtgacacc ttcaccaacc ccgaggaggg tgacctgaac 180
   cccccccccg aggctaagca ggtgcccgtg tcctactacg actccaccta cctgtccacc 240
   gacaacgaga aggacaacta cctgaagggt gtgaccaagc tgttcgagcg tatctactcc 300
   accgacctgg gtcgtatgct gctgacctcc atcgtgcgtg gtatcccctt ctggggtggt 360
   tccaccatcg acaccgagct gaaggtgatc gacaccaact gcatcaacgt gatccagccc 420
   gacggttcct accgttccga ggagctgaac ctggtgatca tcggtccctc cgctgacatc 480
   atccagttcg agtgcaagtc cttcggtcac gaggtgctga acctgacccg taacggttac 540
   ggttccaccc agtacatccg tttctccccc gacttcacct tcggtttcga ggagtccctg 600
   gaggtggaca ccaaccccct gctgggtgct ggtaagttcg ctaccgaccc cgctgtgacc 660
   ctggctcacg agctgatcca cgctggtcac cgtctgtacg gtatcgctat caaccccaac 720
   cgtgtgttca aggtgaacac caacgcttac tacgagatgt ccggtctgga ggtgtccttc 780
   gaggagctgc gtaccttcgg tggtcacgac gctaagttca tcgactccct gcaggagaac 840
   gagttccgtc tgtactacta caacaagttc aaggacatcg cttccaccct gaacaaggct 900
   aagtccatcg tgggtaccac cgcttccctg cagtacatga agaacgtgtt caaggagaag 960
   tacctgctgt ccgaggacac ctccggtaag ttctccgtgg acaagctgaa gttcgacaag 1020
   ctgtacaaga tgctgaccga gatctacacc gaggacaact tcgtgaagtt cttcaaggtg 1080
   ctgaaccgta agacctacct gaacttcgac aaggctgtgt tcaagatcaa catcgtgccc 1140
   aaggtgaact acaccatcta cgacggtttc aacctgcgta acaccaacct ggctgctaac 1200
   ttcaacggtc agaacaccga gatcaacaac atgaacttca ccaagctgaa gaacttcacc 1260
   ggtctgttcg agttctacaa gctgctgtgc gtgcgtggta tcatcacctc caagaccaag 1320
   tccctggaca agggttacaa caaggctctg aacgacctgt gcatcaaggt gaacaactgg 1380
   gacctgttct tctccccctc cgaggacaac ttcaccaacg acctgaacaa gggtgaggag 1440
   atcacctccg acaccaacat cgaggctgct gaggagaaca tctccctgga cctgatccag 1500
   cagtactacc tgaccttcaa cttcgacaac gagcccgaga acatctccat cgagaacctg 1560
   tcctccgaca tcatcggtca gctggagctg atgcccaaca tcgagcgttt ccccaacggt 1620
   aagaagtacg agctggacaa gtacaccatg ttccactacc tgcgtgctca ggagttcgag 1680
   cacggtaagt cccgtatcgc tctgaccaac tccgtgaacg aggctctgct gaacccctcc 1740
   cgtgtgtaca ccttcttctc ctccgactac gtgaagaagg tgaacaaggc taccgaggct 1800
   gctatgttcc tgggttgggt ggagcagctg gtgtacgact tcaccgacga gacctccgag 1860
   gtgtccacca ccgacaagat cgctgacatc accatcatca tcccctacat cggtcccgct 1920
   ctgaacatcg gtaacatgct gtacaaggac gacttcgtgg gtgctctgat cttctccggt 1980
   gctgtgatcc tgctggagtt catccccgag atcgctatcc ccgtgctggg taccttcgct 2040
   ctggtgtcct acatcgctaa caaggtgctg accgtgcaga ccatcgacaa cgctctgtcc 2100
   aagcgtaacg agaagtggga cgaggtgtac aagtacatcg tgaccaactg gctggctaag 2160
   gtgaacaccc agatcgacct gatccgtaag aagatgaagg aggctctgga gaaccaggct 2220
   gaggctacca aggctatcat caactaccag tacaaccagt acaccgagga ggagaagaac 2280
   aacatcaact tcaacatcga cgacctgtcc tccaagctga acgagtccat caacaaggct 2340
   atgatcaaca tcaacaagtt cctgaaccag tgctccgtgt cctacctgat gaactccatg 2400
   atcccctacg gtgtgaagcg tctggaggac ttcgacgctt ccctgaagga cgctctgctg 2460
   aagtacatct acgacaaccg tggtaccctg atcggtcagg tggaccgtct gaaggacaag 2520
   gtgaacaaca ccctgtccac cgacatcccc ttccagctgt ccaagtacgt ggacaaccag 2580
   cgtctgctgt ccaccttcac cgagtacatc aagaacatca tcaacacctc catcctgaac 2640
   ctgcgttacg agtccaacca cctgatcgac ctgtcccgtt acgcttccaa gatcaacatc 2700
   ggttccaagg tgaacttcga ccccatcgac aagaaccaga tccagctgtt caacctggag 2760
   tcctccaaga tcgaggtgat cctgaagaac gctatcgtgt acaactccat gtacgagaac 2820
   ttctccacct ccttctggat ccgtatcccc aagtacttca actccatctc cctgaacaac 2880
   gagtacacca tcatcaactg catggagaac aactccggtt ggaaggtgtc cctgaactac 2940
   ggtgagatca tctggaccct gcaggacacc caggagatca agcagcgtgt ggtgttcaag 3000
   tactcccaga tgatcaacat ctccgactac atcaaccgtt ggatcttcgt gaccatcacc 3060
   aacaaccgtc tgaacaactc caagatctac atcaacggtc gtctgatcga ccagaagccc 3120
   atctccaacc tgggtaacat ccacgcttcc aacaacatca tgttcaagct ggacggttgc 3180
   cgtgacaccc accgttacat ctggatcaag tacttcaacc tgttcgacaa ggagctgaac 3240
   gagaaggaga tcaaggacct gtacgacaac cagtccaact ccggtatcct gaaggacttc 3300
   tggggtgact acctgcagta cgacaagccc tactacatgc tgaacctgta cgaccccaac 3360
   aagtacgtgg acgtgaacaa cgtgggtatc cgtggttaca tgtacctgaa gggtccccgt 3420
   ggttccgtga tgaccaccaa catctacctg aactcctccc tgtaccgtgg taccaagttc 3480
   atcatcaaga agtacgcttc cggtaacaag gacaacatcg tgcgtaacaa cgaccgtgtg 3540
   tacatcaacg tggtggtgaa gaacaaggag taccgtctgg ctaccaacgc ttcccaggct 3600
   ggtgtggaga agatcctgtc cgctctggag atccccgacg tgggtaacct gtcccaggtg 3660
   gtggtgatga agtccaagaa cgaccagggt atcaccaaca agtgcaagat gaacctgcag 3720
   gacaacaacg gtaacgacat cggtttcatc ggtttccacc agttcaacaa catcgctaag 3780
   ctggtggctt ccaactggta caaccgtcag atcgagcgtt cctcccgtac cctgggttgc 3840
   tcctgggagt tcatccccgt ggacgacggt tggggtgagc gtcccctgta a 3891
<210> 63
   <211> 3891
   <212> DNA
   <213> Artificial Sequence
<220>
   <223> BoNT/A, S. frugiperda-modified 3
<400> 63
   atgccgttcg taaataagca atttaattat aaagaccctg tgaatggagt ggatatcgct 60
   tacattaaga tcccaaatgc tggtcaaatg cagcccgtca aggcatttaa gatccataat 120
   aaaatttggg tcatcccaga gagggacact ttcaccaatc ctgaggaagg agaccttaac 180
   cccccgcctg aggctaaaca ggtcccagtg tcttattacg atagtacata cctgtccacg 240
   gacaacgaga aagacaatta tctcaaagga gtaactaaac tctttgaaag aatttattcc 300
   accgacctcg gaaggatgct gttgacatcg atcgtacgcg gcatcccctt ctggggaggc 360
   tctacgatcg ataccgaatt gaaagttatc gacactaact gcatcaacgt tattcaaccg 420
   gatggaagct acaggtccga ggaactgaac ctcgtgatca ttggaccttc tgctgacatc 480
   attcaatttg aatgcaagag cttcggtcac gaagttctga atctgactag gaatggctac 540
   ggtagtacgc agtatattcg tttctcccca gacttcacat tcggtttcga ggaatcactt 600
   gaggttgaca ccaacccctt gctcggtgcc ggtaagtttg ctacagatcc cgcggtcaca 660
   ttggcacatg agctgattca tgcgggtcac cgcctgtacg gtattgccat caatcccaac 720
   cgtgtcttca aggtgaacac taatgcctac tatgagatgt caggtctcga agtctctttt 780
   gaagagctgc gcacctttgg cggacatgat gcaaagttta tcgactcttt gcaagagaac 840
   gaattccgcc tctactatta caataagttt aaggacattg ctagcacact gaacaaggcg 900
   aaatctatcg tcggaaccac tgcgagtttg caatacatga aaaacgtctt caaagaaaag 960
   tacctcctgt ccgaggatac ctccggcaag ttcagcgtgg acaagttgaa gttcgataag 1020
   ctctataaaa tgctgaccga aatctacact gaggacaact tcgtgaaatt ctttaaggtc 1080
   ctgaatagga agacctacct gaatttcgac aaagcggtgt ttaagatcaa catcgtccct 1140
   aaggtaaact acactatcta cgacggtttc aatctccgta acacgaacct ggccgctaac 1200
   ttcaacggac aaaacaccga aatcaacaat atgaacttca caaaattgaa gaacttcacc 1260
   ggtttgttcg aattctacaa gctcctgtgc gtgaggggca tcattacctc gaagacaaag 1320
   agcctggaca aaggctacaa caaagccctg aacgacctgt gcattaaggt gaataactgg 1380
   gaccttttct ttagtccatc tgaagataac ttcacaaacg atctcaataa gggagaagag 1440
   atcacctccg ataccaacat cgaggcggca gaggaaaaca tttcactgga tctgatccag 1500
   caatactatc tcaccttcaa cttcgacaat gaacctgaga acatcagtat cgagaatttg 1560
   agctccgaca tcattggcca gctggaattg atgcctaaca tcgaacgttt ccccaacggc 1620
   aaaaagtacg agttggacaa atacacaatg ttccactacc ttagagccca ggagttcgag 1680
   cacggcaaga gccgcatcgc ccttacgaac tccgtcaacg aggccctgct caacccgtct 1740
   cgcgtgtaca cgttcttttc ctcggattac gttaagaaag tgaataaagc tactgaggct 1800
   gccatgttcc tgggatgggt agagcagctt gtctacgatt tcaccgatga gacaagcgag 1860
   gtttctacca cagataaaat cgctgacatt actatcatta tcccgtacat cggtcctgca 1920
   ctgaacatcg gtaacatgct ctataaggac gatttcgtgg gtgctctgat cttttctggc 1980
   gctgtgatcc tgcttgagtt catccccgaa atcgctatcc ccgtgcttgg tactttcgct 2040
   cttgtctctt acatcgctaa caaggtattg actgtccaga ccatcgacaa tgcactgtcc 2100
   aagcgtaacg aaaaatggga cgaggtgtac aagtacattg ttactaactg gctcgccaaa 2160
   gtgaacaccc aaatcgatct gatccgtaag aaaatgaagg aggctctgga aaaccaggcc 2220
   gaagcgacta aggccatcat taactaccaa tacaaccagt atacagaaga ggaaaagaat 2280
   aacattaatt tcaacatcga tgacctctcg tcaaagctca atgagtctat caacaaggcg 2340
   atgatcaaca ttaacaagtt cttgaaccag tgcagtgtct cctatttgat gaattcgatg 2400
   atcccatacg gtgtgaagag gctggaggac ttcgacgcct ctctgaaaga tgcgcttctg 2460
   aagtacatct acgacaatcg tggtacgctt atcggccagg tagaccgcct caaggacaag 2520
   gttaacaata ccctgtcgac cgatatccct ttccagctgt caaagtacgt ggacaaccag 2580
   cgtctgctca gcaccttcac cgagtacatt aagaacatca ttaatacgtc cattctcaac 2640
   ctgagatacg aaagcaacca cttgatcgat ctttcgcgct acgcatctaa gatcaacatc 2700
   ggtagcaaag taaatttcga tcctatcgat aagaaccaga ttcaactgtt caacttggag 2760
   tcaagcaaaa tcgaagttat tctgaaaaac gcgatcgttt ataacagtat gtatgagaac 2820
   ttctccacga gtttctggat caggatcccc aagtatttca actcaatcag cctgaacaat 2880
   gagtacacta tcattaactg catggagaac aattcaggct ggaaggtctc cctgaactac 2940
   ggagagatca tttggacgtt gcaggacacg caagaaatta agcagcgtgt tgtgttcaaa 3000
   tatagccaaa tgatcaacat ctctgactac atcaaccgtt ggatctttgt cactatcaca 3060
   aacaatcgtc tgaacaatag taagatctac atcaacggca gactcatcga ccagaagccc 3120
   atctcgaatc tgggaaacat tcacgcctca aataacatta tgttcaaact tgacggttgt 3180
   cgtgacacac accgttatat ctggatcaag tattttaacc tcttcgataa ggagttgaat 3240
   gaaaaggaga tcaaggactt gtacgacaac cagtcgaact ccggtatcct gaaggacttc 3300
   tggggagatt acctgcagta cgacaagccg tactatatgc tgaacctgta cgacccaaac 3360
   aagtacgttg acgtaaacaa tgttggaatt cgcggctaca tgtacttgaa gggaccaaga 3420
   ggttccgtga tgactaccaa catttacctg aattccagcc tgtacagggg caccaagttt 3480
   attatcaaga aatacgcatc cggcaacaag gacaacatcg tgagaaacaa tgatcgcgtc 3540
   tacatcaacg tcgtggtaaa gaacaaggag taccgtctgg caactaacgc ttcacaggct 3600
   ggtgtggaaa agatcctctc ggccctggag atccccgacg ttggcaacct gtcacaagtg 3660
   gtcgtaatga aatcaaagaa cgaccagggt attactaaca agtgtaagat gaacctccaa 3720
   gataacaacg gtaacgatat cggcttcatc ggcttccacc agttcaacaa cattgctaag 3780
   cttgtggcct ctaactggta caacagacag atcgagcgtt cctcacgtac cttgggttgt 3840
   agttgggagt ttatccctgt cgacgacgga tggggtgagc gtccactgta a          3891
<210> 64
   <211> 3891
   <212> DNA
   <213> Artificial Sequence
<220>
   <223> BoNT/A, D. rerio-modified 1
<400> 64
   atgccgttcg ttaataaaca gttcaactac aaggacccag tgaatggagt ggacatcgca 60
   tacatcaaga tcccgaacgc cgggcagatg cagcccgtaa aggcatttaa aatccacaat 120
   aagatctggg ttataccaga aagagataca tttactaacc cagaggaagg agacctgaac 180
   cctcccccag aggcaaaaca ggtgcccgtg agctattacg actctaccta tctgtcgaca 240
   gacaatgaga aagataacta cctgaagggg gtcaccaaac tgttcgagcg catttactca 300
   acagacctgg gccgaatgct tctgacaagt atcgtgaggg gaataccttt ttggggaggt 360
   tctacaatcg acactgagct caaagtaatc gacacaaact gcattaacgt gatccagccg 420
   gatggctctt atcgaagtga ggaactgaat ctggtcatca ttggtccaag tgccgatatc 480
   attcagttcg agtgtaagtc ttttggtcat gaggttttga atctcacacg gaacgggtac 540
   ggttcaaccc agtacatcag attctctccc gatttcacct ttggcttcga ggaatccctg 600
   gaggtcgaca ctaatcctct gcttggagca gggaaattcg ctacggaccc tgcagtgaca 660
   ttggctcacg agctcatcca tgctggccat cgcctttacg gaattgccat taatccaaac 720
   cgggttttta aagtgaatac gaacgcctat tacgagatgt ccggactaga ggtgtccttt 780
   gaggaactca ggacatttgg gggtcacgat gctaaattta tcgactccct gcaggaaaac 840
   gaattcaggc tgtattacta taacaaattc aaagatatcg cctcgacgct gaacaaggcc 900
   aaatctattg tcggcaccac tgcatctctg caatatatga aaaacgtctt caaggaaaag 960
   tacctcctga gtgaggacac cagcggaaag ttctctgtcg ataaattgaa gtttgataag 1020
   ttgtacaaga tgctgactga gatctatact gaggacaatt tcgtcaaatt ctttaaagtg 1080
   ttaaaccgaa agacatatct gaacttcgac aaagcggttt ttaagattaa cattgtgcct 1140
   aaggtgaact atactatata tgatggcttt aatctgagga acacaaacct tgcggctaac 1200
   tttaacggcc aaaatacaga gattaacaat atgaacttta caaaactcaa gaactttacc 1260
   ggattattcg agttctacaa attgctttgt gttcgcggga ttatcacgag taagactaaa 1320
   agcctcgata agggatataa taaagccctg aacgacctct gtatcaaagt taataactgg 1380
   gatctgtttt tcagcccaag cgaggataac ttcacgaacg acctgaataa gggagaggaa 1440
   attaccagcg acacaaatat cgaggcggcc gaggaaaaca tatccctgga cttgattcag 1500
   caatactatt taaccttcaa cttcgataac gaaccggaga acatctccat agaaaatctg 1560
   tctagtgaca tcattggaca gctggaactg atgcctaaca ttgagcggtt tcccaatggc 1620
   aaaaagtacg agctcgataa gtacactatg tttcactatc tgcgtgcaca ggaattcgaa 1680
   cacggtaaaa gcaggattgc actcaccaac tctgtcaacg aggccttact taacccttct 1740
   cgcgtttaca ctttctttag ctcagactat gtgaagaaag ttaacaaggc cactgaggct 1800
   gccatgtttt tggggtgggt cgagcaactg gtgtacgatt ttaccgacga aacatcagag 1860
   gtcagtacga ctgataagat cgcggatata actataatca ttccatatat aggtcctgct 1920
   ctcaacatcg gtaacatgtt atacaaagat gacttcgtgg gtgcgcttat tttctcaggc 1980
   gcggtcatcc tgctcgaatt cattccagag attgctatcc ccgtgctggg aacttttgct 2040
   ctggtgtctt acatcgccaa caaggtgctt accgtgcaaa ctatcgataa cgccttgtcc 2100
   aagcggaatg agaaatggga cgaggtgtac aagtacatcg taacaaattg gctagccaaa 2160
   gtcaataccc aaatcgattt gatcagaaag aaaatgaaag aggcactaga gaaccaggct 2220
   gaagcaacaa aggcaatcat taattatcag tacaatcagt acaccgagga agagaaaaac 2280
   aatattaatt tcaatatcga tgacttgtct agcaagctaa acgagtcaat caataaagct 2340
   atgattaata tcaataaatt cctgaaccag tgctctgttt cttaccttat gaattcgatg 2400
   ataccttatg gtgtgaagag actggaggac tttgacgcat cgctgaaaga tgcactcttg 2460
   aagtacatct acgataacag aggaactctc atagggcagg tggacaggtt gaaggacaag 2520
   gttaacaata ccctgagtac cgacatcccc ttccagctaa gcaagtacgt ggataaccaa 2580
   aggctgcttt caacattcac cgaatacatt aagaatatca ttaacacaag catcctcaat 2640
   ctaaggtatg agtcaaatca tctcatagac ttatcccgtt atgcctcgaa aattaatatt 2700
   ggatccaaag tgaactttga ccctatcgat aagaaccaaa tacaactttt taacctggag 2760
   tctagcaaga ttgaggtcat tcttaagaat gctatcgtgt acaactctat gtacgagaac 2820
   ttcagcacta gtttctggat ccgcatcccg aagtatttca actcaatttc cttaaacaat 2880
   gagtatacca tcattaactg catggaaaac aatagtggct ggaaagttag cctcaattat 2940
   ggtgaaataa tctggacctt gcaggacaca caggagatta aacagagagt ggtttttaag 3000
   tacagtcaga tgatcaacat ctccgactac attaacagat ggatctttgt tacgattacg 3060
   aacaatcgac tcaataactc caagatttac ataaatggac gcctgattga tcagaaacca 3120
   atctccaacc tgggtaacat ccatgccagt aacaatatca tgttcaaact agatggatgc 3180
   cgtgacacgc accgttacat ctggatcaaa tactttaact tgtttgataa agagctgaac 3240
   gagaaggaaa ttaaagatct ttatgataat caatcaaact ccggcatcct caaggatttt 3300
   tggggcgatt accttcagta cgataagcct tactatatgc tgaacctgta cgaccccaat 3360
   aagtatgtgg acgtcaataa cgtggggatc cgaggatata tgtatctcaa aggtccccgc 3420
   ggatccgtta tgaccactaa catatacctg aatagctcgc tgtatagagg cacaaagttc 3480
   ataatcaaga aatatgctag cggcaataaa gacaacatag tacgtaacaa tgacagagtg 3540
   tacataaacg tcgtagtcaa aaataaggag taccggctgg ctacgaacgc ttcacaggcc 3600
   ggcgtagaaa aaatcctttc agctctggaa attcctgacg taggtaactt aagccaggtc 3660
   gtggttatga agtcaaagaa cgatcagggg atcaccaata agtgtaaaat gaatctgcag 3720
   gataacaatg gcaatgacat cggattcatt ggctttcacc agttcaacaa catcgcgaaa 3780
   ctggtggcta gcaactggta taacagacag atcgaacgta gctccagaac attgggctgc 3840
   agttgggaat tcattccggt ggacgacgga tggggagagc gcccactcta a          3891
<210> 65
   <211> 3891
   <212> DNA
   <213> Artificial Sequence
<220>
   <223> BoNT/A, D. rerio-modified 2
<400> 65
   atgcctttcg tgaacaagca gttcaactac aaggaccctg tgaacggagt ggacatcgct 60
   tacatcaaga tccctaacgc tggacagatg cagcctgtga aggctttcaa gatccacaac 120
   aagatctggg tgatccctga gagagacaca ttcacaaacc ctgaggaggg agacctgaac 180
   cctcctcctg aggctaagca ggtgcctgtg agctactacg acagcacata cctgagcaca 240
   gacaacgaga aggacaacta cctgaaggga gtgacaaagc tgttcgagag aatctacagc 300
   acagacctgg gaagaatgct gctgacaagc atcgtgagag gaatcccttt ctggggagga 360
   agcacaatcg acacagagct gaaggtgatc gacacaaact gcatcaacgt gatccagcct 420
   gacggaagct acagaagcga ggagctgaac ctggtgatca tcggacctag cgctgacatc 480
   atccagttcg agtgcaagag cttcggacac gaggtgctga acctgacaag aaacggatac 540
   ggaagcacac agtacatcag attcagccct gacttcacat tcggattcga ggagagcctg 600
   gaggtggaca caaaccctct gctgggagct ggaaagttcg ctacagaccc tgctgtgaca 660
   ctggctcacg agctgatcca cgctggacac agactgtacg gaatcgctat caaccctaac 720
   agagtgttca aggtgaacac aaacgcttac tacgagatga gcggactgga ggtgagcttc 780
   gaggagctga gaacattcgg aggacacgac gctaagttca tcgacagcct gcaggagaac 840
   gagttcagac tgtactacta caacaagttc aaggacatcg ctagcacact gaacaaggct 900
   aagagcatcg tgggaacaac agctagcctg cagtacatga agaacgtgtt caaggagaag 960
   tacctgctga gcgaggacac aagcggaaag ttcagcgtgg acaagctgaa gttcgacaag 1020
   ctgtacaaga tgctgacaga gatctacaca gaggacaact tcgtgaagtt cttcaaggtg 1080
   ctgaacagaa agacatacct gaacttcgac aaggctgtgt tcaagatcaa catcgtgcct 1140
   aaggtgaact acacaatcta cgacggattc aacctgagaa acacaaacct ggctgctaac 1200
   ttcaacggac agaacacaga gatcaacaac atgaacttca caaagctgaa gaacttcaca 1260
   ggactgttcg agttctacaa gctgctgtgc gtgagaggaa tcatcacaag caagacaaag 1320
   agcctggaca agggatacaa caaggctctg aacgacctgt gcatcaaggt gaacaactgg 1380
   gacctgttct tcagccctag cgaggacaac ttcacaaacg acctgaacaa gggagaggag 1440
   atcacaagcg acacaaacat cgaggctgct gaggagaaca tcagcctgga cctgatccag 1500
   cagtactacc tgacattcaa cttcgacaac gagcctgaga acatcagcat cgagaacctg 1560
   agcagcgaca tcatcggaca gctggagctg atgcctaaca tcgagagatt ccctaacgga 1620
   aagaagtacg agctggacaa gtacacaatg ttccactacc tgagagctca ggagttcgag 1680
   cacggaaaga gcagaatcgc tctgacaaac agcgtgaacg aggctctgct gaaccctagc 1740
   agagtgtaca cattcttcag cagcgactac gtgaagaagg tgaacaaggc tacagaggct 1800
   gctatgttcc tgggatgggt ggagcagctg gtgtacgact tcacagacga gacaagcgag 1860
   gtgagcacaa cagacaagat cgctgacatc acaatcatca tcccttacat cggacctgct 1920
   ctgaacatcg gaaacatgct gtacaaggac gacttcgtgg gagctctgat cttcagcgga 1980
   gctgtgatcc tgctggagtt catccctgag atcgctatcc ctgtgctggg aacattcgct 2040
   ctggtgagct acatcgctaa caaggtgctg acagtgcaga caatcgacaa cgctctgagc 2100
   aagagaaacg agaagtggga cgaggtgtac aagtacatcg tgacaaactg gctggctaag 2160
   gtgaacacac agatcgacct gatcagaaag aagatgaagg aggctctgga gaaccaggct 2220
   gaggctacaa aggctatcat caactaccag tacaaccagt acacagagga ggagaagaac 2280
   aacatcaact tcaacatcga cgacctgagc agcaagctga acgagagcat caacaaggct 2340
   atgatcaaca tcaacaagtt cctgaaccag tgcagcgtga gctacctgat gaacagcatg 2400
   atcccttacg gagtgaagag actggaggac ttcgacgcta gcctgaagga cgctctgctg 2460
   aagtacatct acgacaacag aggaacactg atcggacagg tggacagact gaaggacaag 2520
   gtgaacaaca cactgagcac agacatccct ttccagctga gcaagtacgt ggacaaccag 2580
   agactgctga gcacattcac agagtacatc aagaacatca tcaacacaag catcctgaac 2640
   ctgagatacg agagcaacca cctgatcgac ctgagcagat acgctagcaa gatcaacatc 2700
   ggaagcaagg tgaacttcga ccctatcgac aagaaccaga tccagctgtt caacctggag 2760
   agcagcaaga tcgaggtgat cctgaagaac gctatcgtgt acaacagcat gtacgagaac 2820
   ttcagcacaa gcttctggat cagaatccct aagtacttca acagcatcag cctgaacaac 2880
   gagtacacaa tcatcaactg catggagaac aacagcggat ggaaggtgag cctgaactac 2940
   ggagagatca tctggacact gcaggacaca caggagatca agcagagagt ggtgttcaag 3000
   tacagccaga tgatcaacat cagcgactac atcaacagat ggatcttcgt gacaatcaca 3060
   aacaacagac tgaacaacag caagatctac atcaacggaa gactgatcga ccagaagcct 3120
   atcagcaacc tgggaaacat ccacgctagc aacaacatca tgttcaagct ggacggatgc 3180
   agagacacac acagatacat ctggatcaag tacttcaacc tgttcgacaa ggagctgaac 3240
   gagaaggaga tcaaggacct gtacgacaac cagagcaaca gcggaatcct gaaggacttc 3300
   tggggagact acctgcagta cgacaagcct tactacatgc tgaacctgta cgaccctaac 3360
   aagtacgtgg acgtgaacaa cgtgggaatc agaggataca tgtacctgaa gggacctaga 3420
   ggaagcgtga tgacaacaaa catctacctg aacagcagcc tgtacagagg aacaaagttc 3480
   atcatcaaga agtacgctag cggaaacaag gacaacatcg tgagaaacaa cgacagagtg 3540
   tacatcaacg tggtggtgaa gaacaaggag tacagactgg ctacaaacgc tagccaggct 3600
   ggagtggaga agatcctgag cgctctggag atccctgacg tgggaaacct gagccaggtg 3660
   gtggtgatga agagcaagaa cgaccaggga atcacaaaca agtgcaagat gaacctgcag 3720
   gacaacaacg gaaacgacat cggattcatc ggattccacc agttcaacaa catcgctaag 3780
   ctggtggcta gcaactggta caacagacag atcgagagaa gcagcagaac actgggatgc 3840
   agctgggagt tcatccctgt ggacgacgga tggggagaga gacctctgta a 3891
<210> 66
   <211> 3891
   <212> DNA
   <213> Artificial Sequence
<220>
   <223> BoNT/A, D. rerio-modified 3
<400> 66
   atgccgtttg tcaacaagca attcaattat aaggatccag tcaatggagt ggatatcgct 60
   tatatcaaaa tccctaatgc gggtcagatg cagcccgtca aagcgttcaa aatccacaac 120
   aagatctggg ttatccctga gcgagacacc ttcaccaacc cagaagaggg ggacctgaat 180
   cccccacctg aagcgaaaca ggtgcctgtg agctactatg acagcaccta cctgtcaacc 240
   gataacgaga aagacaatta tctgaaaggc gtgacaaaac tgttcgaacg catttatagc 300
   acagaccttg gaaggatgct gctcactagc atcgttcgtg gcattccctt ttgggggggc 360
   agcaccatcg acactgaact gaaagtcatc gatacaaact gcatcaacgt cattcagcca 420
   gatggatcat atcgaagcga ggaactcaat ctggttatta tcggtccatc agccgacatc 480
   attcagttcg agtgtaaatc tttcggccac gaggtgctga accttactcg caacggatat 540
   gggagtacac agtacatccg cttcagtcca gacttcacat ttggtttcga agagagtctt 600
   gaggtggaca ccaatcccct gctcggtgcc ggaaaattcg ctacggatcc agcagtcacc 660
   ctggcccacg aactgattca cgctggacat agattgtacg gaatcgcaat caacccaaat 720
   agagttttca aagtgaacac taatgcttac tatgagatga gcggcctcga ggtctcattt 780
   gaggaattga ggacctttgg cggtcatgat gcgaagttca ttgactccct ccaggaaaac 840
   gaatttcgtc tttactatta caacaagttc aaggatatcg cttctacctt gaataaagcc 900
   aagtctatcg tgggaacaac ggcatccctc cagtacatga agaacgtgtt caaggagaag 960
   tatctgttgt cagaggacac cagcgggaaa tttagcgttg ataaactgaa gttcgacaag 1020
   ctgtacaaaa tgctgacaga aatctacaca gaggacaatt tcgtgaagtt tttcaaggtg 1080
   cttaacagga agacctatct gaactttgac aaggcagtct ttaaaatcaa cattgttcct 1140
   aaggttaact acaccatcta tgatgggttt aacctcagaa acactaacct tgcagctaac 1200
   tttaatggac agaacactga gatcaacaat atgaacttta caaaacttaa gaatttcaca 1260
   ggtctgttcg agttttataa gctgctctgc gtccgtggga ttatcacgag taagacaaag 1320
   tccctggaca agggatacaa taaggctttg aacgatctgt gcattaaggt gaacaattgg 1380
   gatttgttct ttagcccgag tgaagataat ttcactaacg acctgaataa aggtgaggaa 1440
   atcacaagcg acactaatat tgaggcagct gaagagaaca tctcactcga cttgatccaa 1500
   cagtactatc ttacttttaa tttcgacaat gaaccggaga atatctccat tgagaatctc 1560
   agctctgaca ttatcgggca actggagctg atgccgaaca tcgaacggtt tcccaatggc 1620
   aaaaagtatg aactcgataa gtacactatg tttcactacc tccgagccca ggaatttgaa 1680
   catgggaaga gcaggatcgc acttaccaac agcgtcaacg aggctctgct taacccaagc 1740
   cgcgtgtaca catttttctc ctcagattac gtcaagaaag ttaacaaggc cactgaggcc 1800
   gctatgttcc tcggatgggt tgaacagctg gtttacgatt ttacggatga aacgagcgag 1860
   gtgtctacta cagacaaaat cgctgatatc actatcatta tcccgtacat tggccctgca 1920
   ctcaacatcg gaaacatgct gtacaaggac gatttcgtgg gcgccctgat cttttctgga 1980
   gcagtgattc tgcttgagtt catccctgag atcgccattc ctgtgctggg aacgttcgcc 2040
   ctcgtgtctt acatcgctaa taaggtgctg acagtgcaaa caatcgataa cgccctgtcc 2100
   aaaagaaacg agaaatggga tgaggtgtac aaatacatcg tcacaaactg gttggctaaa 2160
   gttaacacac aaattgacct catccggaag aaaatgaaag aggcgctgga gaaccaggcg 2220
   gaagccacca aagccatcat taattaccag tacaaccagt atacggagga agagaaaaac 2280
   aatattaatt ttaacatcga tgacctgagt tctaagctca atgagagtat caacaaagca 2340
   atgattaaca tcaataagtt ccttaaccag tgctctgtca gctacctgat gaattccatg 2400
   atcccttatg gggtgaagcg actggaggat tttgacgcct ctctgaaaga tgctctgttg 2460
   aagtatattt acgacaacag gggtacactc atcggccagg ttgatcgcct gaaggataag 2520
   gtcaataaca ccctgagcac tgacattcct ttccagctgt caaaatatgt ggataatcag 2580
   cgcttgctga gtacattcac tgagtacatc aaaaacatta tcaatacgtc tatcctgaac 2640
   ctgaggtacg agagcaacca cctgatcgat ttgtcccgtt acgcctctaa aatcaacatt 2700
   ggaagcaaag tgaactttga tccgatcgac aagaaccaga tccagctgtt caacctggag 2760
   tcatctaaaa tcgaggtcat cctgaagaat gctattgtgt acaattcaat gtacgagaat 2820
   tttagcacta gcttctggat cagaatccca aaatacttta attccatcag cctgaacaat 2880
   gagtatacca tcattaattg tatggagaat aactccgggt ggaaagtttc actcaactac 2940
   ggtgaaatca tttggaccct gcaagacact caggaaatca aacagagggt ggttttcaag 3000
   tactcccaga tgattaacat cagtgattat attaatagat ggatctttgt gaccattacg 3060
   aacaatcggc tgaacaatag taaaatctac atcaatggca ggttgattga tcaaaagcca 3120
   atctctaatc tgggaaacat tcatgcctct aacaatatca tgtttaaact tgacggctgt 3180
   agagacacgc atcgctatat ctggattaaa tattttaatc tgtttgataa agagctgaac 3240
   gagaaagaga tcaaggacct gtatgataac cagagcaatt ctggtattct gaaggacttc 3300
   tggggagatt acctgcagta tgacaagccc tactatatgc tgaatctcta cgatcctaac 3360
   aagtatgtgg acgtgaacaa tgtgggaatc cgaggataca tgtacttgaa gggcccccgg 3420
   ggctccgtga tgactacaaa catctatttg aactcttccc tctatcgtgg taccaaattc 3480
   atcattaaga aatacgccag cggtaataag gacaacatcg tgcgcaacaa tgacagagtc 3540
   tatattaacg tggtcgtgaa aaacaaggaa tacagattgg cgaccaacgc atcccaggct 3600
   ggtgtggaga agattctgtc cgcacttgag attcccgacg ttggaaacct tagtcaggtg 3660
   gttgtgatga aatcaaaaaa cgaccaaggt atcacaaaca aatgtaagat gaacctgcaa 3720
   gataacaatg gcaacgacat tggcttcatc ggcttccacc aatttaataa catcgcaaag 3780
   ctggtggctt ccaactggta taacagacag atcgaacgga gttcacgtac cctgggatgc 3840
   agttgggagt tcatccctgt ggacgacggc tggggagaga gacccctgta a          3891
<210> 67
   <211> 3891
   <212> DNA
   <213> Artificial Sequence
<220>
   <223> BoNT/A, X. laevis-modified 1
<400> 67
   atgcctttcg tgaacaaaca gttcaattat aaggaccctg taaatggggt ggatatcgca 60
   tatattaaaa ttcctaatgc aggacagatg caacctgtta aagcctttaa aatacacaat 120
   aagatttggg ttatcccaga gcgcgataca tttactaacc ccgaggaagg agatctgaat 180
   ccacctccag aggcaaagca agtgccggtt tcttactatg actcgactta cctaagtaca 240
   gataacgaga aggataacta ccttaagggg gttaccaagt tatttgaacg gatttattcc 300
   actgacttag gccgaatgtt gctcacttcc atcgtacgtg gaattccatt ttggggaggt 360
   agcaccattg acactgagct gaaggttatt gatacgaatt gtattaacgt gatacagcct 420
   gatggaagct acaggtctga ggaacttaat ctggttatca taggccctag cgccgacatt 480
   atacaatttg agtgtaaaag cttcgggcac gaagtactca atcttactag aaacggctac 540
   ggatccacac aatatattag gttcagccca gactttactt ttggattcga agagtctttg 600
   gaggtggata caaacccatt gctgggcgct ggtaagttcg ccacagatcc agctgttaca 660
   ctggcccatg aactaattca cgcagggcat aggttatatg gcatcgccat aaatccaaac 720
   cgtgtattca aagtcaacac aaacgcctac tatgagatgt ctggacttga ggtgtctttc 780
   gaagagctga ggactttcgg cggtcacgat gcgaaattta ttgattcact ccaggaaaac 840
   gagttccgcc tgtactatta caacaagttt aaagacatag cttctacgtt gaataaagct 900
   aaatctatcg taggaaccac agcatccctc cagtacatga agaacgtctt taaggagaaa 960
   tacctgttga gtgaagacac atcgggaaaa tttagtgttg ataagttgaa atttgataag 1020
   ctgtataaaa tgctgacaga aatctataca gaggacaatt tcgtgaaatt tttcaaagtt 1080
   cttaaccgca aaacctactt gaattttgac aaggctgtgt ttaaaatcaa tattgtgccc 1140
   aaggttaatt acactatata cgatggcttc aacctcagga atactaatct ggccgcgaat 1200
   tttaatggcc aaaataccga gattaacaat atgaatttca caaagcttaa aaactttacc 1260
   ggtcttttcg aattctataa acttctgtgt gtcagaggaa tcattacatc gaaaactaag 1320
   agtcttgata agggctataa taaagccctt aatgacctct gcatcaaagt caacaattgg 1380
   gatttgtttt tctcaccatc agaggataat tttacaaacg acttgaataa aggcgaggaa 1440
   attactagcg acactaacat agaagcagcc gaagagaata tttctcttga tttgatacaa 1500
   cagtattact taacatttaa ttttgataat gaaccagaga acattagtat cgaaaatctt 1560
   agcagtgata tcattggtca gttggagctg atgcccaaca ttgagcgttt tcccaatggc 1620
   aagaaatacg aactcgacaa atatacgatg ttccactatt tgagagctca agaatttgag 1680
   cacgggaaat cccggatagc cctgacaaac tctgtaaacg aagctctttt gaatccctcg 1740
   agagtttata ccttcttttc atctgattac gttaaaaagg tcaataaggc taccgaagcc 1800
   gcgatgtttc ttgggtgggt cgaacaactg gtgtacgatt ttaccgacga gacatcagaa 1860
   gtgagcacca ctgacaaaat tgctgacata actataatta taccctacat cggtccggca 1920
   cttaatattg gtaatatgct ttataaagat gacttcgtcg gagccctgat cttttccgga 1980
   gctgtaatct tgctggagtt catcccagag attgctatac ccgtcctggg gaccttcgcg 2040
   ctcgtgtctt acattgcaaa caaggtcctg actgtacaga caatcgataa cgcactaagc 2100
   aaaagaaatg aaaagtggga tgaggtgtat aagtacatag tcactaattg gttagcaaag 2160
   gtgaacacgc agatcgacct tatcaggaag aaaatgaagg aagcactcga aaaccaggct 2220
   gaggctacaa aggcaatcat taactatcag tataatcaat atactgagga agagaagaat 2280
   aacatcaatt ttaatattga tgacctcagt tcaaaactga atgaaagcat taataaggct 2340
   atgataaaca ttaacaagtt tctgaaccag tgtagcgtgt cctatctaat gaactccatg 2400
   atcccctatg gcgtcaagcg gctggaagac tttgacgcaa gcctaaagga cgctttactg 2460
   aagtatattt acgataaccg aggcactctt ataggacagg ttgataggct gaaagataag 2520
   gtgaataaca cccttagtac agatatccca ttccagctgt ctaagtatgt tgataaccag 2580
   cgcctcctgt caacctttac ggagtacatt aagaatatta taaacacctc catcctgaat 2640
   ctgagatacg agtccaacca tctaatcgat ctgagcagat acgcttcaaa aataaatatc 2700
   ggttccaagg tgaacttcga cccaattgac aagaaccaaa ttcagttatt caatttagaa 2760
   tcctcaaaaa tcgaggttat cttgaagaac gccatagtat ataatagcat gtacgagaat 2820
   ttttccacat ctttctggat ccgtatacct aagtacttta attctatatc cttgaacaat 2880
   gagtatacca tcattaactg catggagaac aattcaggtt ggaaggtaag cctgaattat 2940
   ggtgaaatta tctggaccct gcaggacacc caggagatta aacagagagt ggtttttaag 3000
   tattcacaga tgatcaatat atctgattac atcaaccggt ggatcttcgt aaccattacg 3060
   aataacagat taaacaatag taaaatctac attaacggtc gactcattga tcaaaaacct 3120
   attagtaact taggaaacat ccatgccagt aacaatatta tgttcaaact agatggctgc 3180
   cgggatacac atagatacat ttggattaaa tattttaatc tgttcgacaa ggaactaaac 3240
   gaaaaggaaa tcaaggattt gtatgacaac cagagtaatt ctgggatact aaaagacttc 3300
   tggggggact atttacagta tgataaacca tactatatgc taaaccttta tgatcctaat 3360
   aaatatgtcg atgtaaataa cgtggggatc cgtggctata tgtacctcaa gggacctaga 3420
   ggaagtgtca tgactacaaa catatattta aacagttctc tctaccgcgg aacaaagttt 3480
   attatcaaga aatacgcatc cgggaataag gataacatcg tccgaaataa cgatcgcgtt 3540
   tacattaatg tagtggttaa aaataaagaa taccgactgg ctacaaacgc aagccaggcc 3600
   ggagtggaaa agattttgtc tgctctcgaa atacccgacg tgggaaacct ttctcaagtg 3660
   gttgtgatga aatcaaaaaa cgatcaagga attaccaata aatgtaaaat gaacctgcag 3720
   gataacaatg gaaatgacat tggttttatt gggtttcatc agttcaataa catcgcaaaa 3780
   ctagtggcaa gcaattggta caataggcaa atcgaaagat caagtaggac tctcggatgc 3840
   tcatgggaat ttataccggt ggacgatggg tggggtgaaa ggcctttgta a          3891
<210> 68
   <211> 3891
   <212> DNA
   <213> Artificial Sequence
<220>
   <223> BoNT/A, X. laevis-modified 2
<400> 68
   atgccatttg tgaataaaca gtttaattat aaagatccag tgaatggagt ggatattgct 60
   tatattaaaa ttccaaatgc tggacagatg cagccagtga aagcttttaa aattcataat 120
   aaaatttggg tgattccaga aagagataca tttacaaatc cagaagaagg agatctgaat 180
   ccaccaccag aagctaaaca ggtgccagtg tcttattatg attctacata tctgtctaca 240
   gataatgaaa aagataatta tctgaaagga gtgacaaaac tgtttgaaag aatttattct 300
   acagatctgg gaagaatgct gctgacatct attgtgagag gaattccatt ttggggagga 360
   tctacaattg atacagaact gaaagtgatt gatacaaatt gtattaatgt gattcagcca 420
   gatggatctt atagatctga agaactgaat ctggtgatta ttggaccatc tgctgatatt 480
   attcagtttg aatgtaaatc ttttggacat gaagtgctga atctgacaag aaatggatat 540
   ggatctacac agtatattag attttctcca gattttacat ttggatttga agaatctctg 600
   gaagtggata caaatccact gctgggagct ggaaaatttg ctacagatcc agctgtgaca 660
   ctggctcatg aactgattca tgctggacat agactgtatg gaattgctat taatccaaat 720
   agagtgttta aagtgaatac aaatgcttat tatgaaatgt ctggactgga agtgtctttt 780
   gaagaactga gaacatttgg aggacatgat gctaaattta ttgattctct gcaggaaaat 840
   gaatttagac tgtattatta taataaattt aaagatattg cttctacact gaataaagct 900
   aaatctattg tgggaacaac agcttctctg cagtatatga aaaatgtgtt taaagaaaaa 960
   tatctgctgt ctgaagatac atctggaaaa ttttctgtgg ataaactgaa atttgataaa 1020
   ctgtataaaa tgctgacaga aatttataca gaagataatt ttgtgaaatt ttttaaagtg 1080
   ctgaatagaa aaacatatct gaattttgat aaagctgtgt ttaaaattaa tattgtgcca 1140
   aaagtgaatt atacaattta tgatggattt aatctgagaa atacaaatct ggctgctaat 1200
   tttaatggac agaatacaga aattaataat atgaatttta caaaactgaa aaattttaca 1260
   ggactgtttg aattttataa actgctgtgt gtgagaggaa ttattacatc taaaacaaaa 1320
   tctctggata aaggatataa taaagctctg aatgatctgt gtattaaagt gaataattgg 1380
   gatctgtttt tttctccatc tgaagataat tttacaaatg atctgaataa aggagaagaa 1440
   attacatctg atacaaatat tgaagctgct gaagaaaata tttctctgga tctgattcag 1500
   cagtattatc tgacatttaa ttttgataat gaaccagaaa atatttctat tgaaaatctg 1560
   tcttctgata ttattggaca gctggaactg atgccaaata ttgaaagatt tccaaatgga 1620
   aaaaaatatg aactggataa atatacaatg tttcattatc tgagagctca ggaatttgaa 1680
   catggaaaat ctagaattgc tctgacaaat tctgtgaatg aagctctgct gaatccatct 1740
   agagtgtata catttttttc ttctgattat gtgaaaaaag tgaataaagc tacagaagct 1800
   gctatgtttc tgggatgggt ggaacagctg gtgtatgatt ttacagatga aacatctgaa 1860
   gtgtctacaa cagataaaat tgctgatatt acaattatta ttccatatat tggaccagct 1920
   ctgaatattg gaaatatgct gtataaagat gattttgtgg gagctctgat tttttctgga 1980
   gctgtgattc tgctggaatt tattccagaa attgctattc cagtgctggg aacatttgct 2040
   ctggtgtctt atattgctaa taaagtgctg acagtgcaga caattgataa tgctctgtct 2100
   aaaagaaatg aaaaatggga tgaagtgtat aaatatattg tgacaaattg gctggctaaa 2160
   gtgaatacac agattgatct gattagaaaa aaaatgaaag aagctctgga aaatcaggct 2220
   gaagctacaa aagctattat taattatcag tataatcagt atacagaaga agaaaaaaat 2280
   aatattaatt ttaatattga tgatctgtct tctaaactga atgaatctat taataaagct 2340
   atgattaata ttaataaatt tctgaatcag tgttctgtgt cttatctgat gaattctatg 2400
   attccatatg gagtgaaaag actggaagat tttgatgctt ctctgaaaga tgctctgctg 2460
   aaatatattt atgataatag aggaacactg attggacagg tggatagact gaaagataaa 2520
   gtgaataata cactgtctac agatattcca tttcagctgt ctaaatatgt ggataatcag 2580
   agactgctgt ctacatttac agaatatatt aaaaatatta ttaatacatc tattctgaat 2640
   ctgagatatg aatctaatca tctgattgat ctgtctagat atgcttctaa aattaatatt 2700
   ggatctaaag tgaattttga tccaattgat aaaaatcaga ttcagctgtt taatctggaa 2760
   tcttctaaaa ttgaagtgat tctgaaaaat gctattgtgt ataattctat gtatgaaaat 2820
   ttttctacat ctttttggat tagaattcca aaatatttta attctatttc tctgaataat 2880
   gaatatacaa ttattaattg tatggaaaat aattctggat ggaaagtgtc tctgaattat 2940
   ggagaaatta tttggacact gcaggataca caggaaatta aacagagagt ggtgtttaaa 3000
   tattctcaga tgattaatat ttctgattat attaatagat ggatttttgt gacaattaca 3060
   aataatagac tgaataattc taaaatttat attaatggaa gactgattga tcagaaacca 3120
   atttctaatc tgggaaatat tcatgcttct aataatatta tgtttaaact ggatggatgt 3180
   agagatacac atagatatat ttggattaaa tattttaatc tgtttgataa agaactgaat 3240
   gaaaaagaaa ttaaagatct gtatgataat cagtctaatt ctggaattct gaaagatttt 3300
   tggggagatt atctgcagta tgataaacca tattatatgc tgaatctgta tgatccaaat 3360
   aaatatgtgg atgtgaataa tgtgggaatt agaggatata tgtatctgaa aggaccaaga 3420
   ggatctgtga tgacaacaaa tatttatctg aattcttctc tgtatagagg aacaaaattt 3480
   attattaaaa aatatgcttc tggaaataaa gataatattg tgagaaataa tgatagagtg 3540
   tatattaatg tggtggtgaa aaataaagaa tatagactgg ctacaaatgc ttctcaggct 3600
   ggagtggaaa aaattctgtc tgctctggaa attccagatg tgggaaatct gtctcaggtg 3660
   gtggtgatga aatctaaaaa tgatcaggga attacaaata aatgtaaaat gaatctgcag 3720
   gataataatg gaaatgatat tggatttatt ggatttcatc agtttaataa tattgctaaa 3780
   ctggtggctt ctaattggta taatagacag attgaaagat cttctagaac actgggatgt 3840
   tcttgggaat ttattccagt ggatgatgga tggggagaaa gaccactgta a 3891
<210> 69
   <211> 3891
   <212> DNA
   <213> Artificial Sequence
<220>
   <223> BoNT/A, X. laevis-modified 3
<400> 69
   atgccattcg tgaacaaaca atttaactac aaggatcccg ttaacggggt tgacatagcc 60
   tatatcaaaa ttccaaatgc tgggcagatg caacccgtta aagcttttaa gatacataac 120
   aagatctggg ttatccccga aagagacacc ttcactaatc cagaagaggg ggatcttaat 180
   ccaccccctg aggcaaaaca ggttccagta agctattacg acagcaccta cttatctaca 240
   gacaatgaaa aggacaacta cctcaagggg gtgaccaagc tctttgaacg aatatattcc 300
   acagatctgg gtaggatgtt gctaactagc attgtgcgtg gaatcccatt ctggggaggc 360
   tcaaccatcg acacagaatt gaaagtcatc gacacaaact gcattaacgt catccaacct 420
   gatgggtcct atagaagcga agagctgaat ctggtaatta tcggtccctc agcagatata 480
   attcagtttg agtgcaagtc atttggtcac gaggttctca acctcactag aaatggttac 540
   ggcagtacac agtacatccg attttctccc gactttactt tcggattcga ggaatctttg 600
   gaggtcgaca ctaatccttt gctgggggca ggcaaattcg ccactgatcc agccgttaca 660
   ctcgctcatg agttaattca tgctggtcac aggctgtacg gaattgccat taatcctaac 720
   agagtgttta aggtcaatac taatgcctat tacgaaatgt ccggcctcga agtgagtttc 780
   gaagagctta gaacatttgg aggtcacgac gctaaattta ttgacagttt gcaggagaat 840
   gaattccgcc tgtactatta caacaagttt aaagacatcg cctcaactct gaataaggct 900
   aaatctatag taggtacaac cgcttcctta cagtacatga agaacgtctt taaggagaag 960
   tatctactta gcgaagacac cagtgggaag tttagtgttg ataaacttaa gttcgataag 1020
   ctctataaaa tgttgaccga aatctacaca gaagacaact ttgtaaaatt tttcaaagtc 1080
   ttgaacagaa aaacttacct taatttcgac aaagctgtgt tcaaaatcaa cattgtgcca 1140
   aaagtgaact ataccattta cgatggtttc aacctgcgca atacaaatct ggccgctaac 1200
   tttaacgggc aaaacaccga gataaacaat atgaacttca ccaagctcaa gaacttcact 1260
   ggtctttttg agttttacaa actgctctgt gttagaggaa ttatcacttc taaaacaaaa 1320
   tctctggaca agggttacaa caaggcactg aacgatctgt gtattaaggt gaataactgg 1380
   gatttattct ttagtccaag cgaggacaac tttactaacg atctaaataa gggtgaagag 1440
   atcacaagtg atactaacat cgaggctgca gaagagaata tttccctgga tctgattcaa 1500
   cagtattacc tgaccttcaa tttcgataac gaaccagaga atatctctat cgaaaactta 1560
   tcctctgata ttatcggaca gctggagctg atgccaaaca ttgagcgatt tcctaatggt 1620
   aagaaatatg agctggataa gtatacaatg tttcattacc tacgtgctca ggagtttgaa 1680
   cacggaaaaa gcaggatagc cttgacaaac tcagttaacg aggcattact gaatccaagt 1740
   agggtgtaca ctttcttttc ttccgattac gtaaagaaag tgaataaagc tacagaagct 1800
   gcaatgtttc tgggctgggt ggaacagtta gtttacgatt tcactgacga gacctccgag 1860
   gtaagcacaa ccgacaaaat tgccgatatt acaatcataa ttccttacat tggacctgct 1920
   cttaatatcg gaaatatgct atataaagac gattttgttg gagctcttat cttctcagga 1980
   gctgttattc tgctcgaatt tatccctgag attgccatcc ctgtattggg cacatttgcc 2040
   ttagtgagct acattgctaa caaggttcta acagtacaga caatagataa cgcactgagt 2100
   aaacgaaacg aaaagtggga cgaagtatac aagtacatag ttacaaattg gttagcaaaa 2160
   gtgaacacac aaatcgattt aatacggaaa aagatgaagg aggctttgga aaaccaagca 2220
   gaggcaacaa aggccataat caactatcaa tataaccagt acaccgaaga ggaaaagaat 2280
   aacatcaact ttaatattga tgaccttagt tctaaactta atgagtctat taataaggcc 2340
   atgataaata taaataaatt tcttaaccag tgttctgtat catacctgat gaacagtatg 2400
   atcccttatg gcgtcaaaag gctcgaagat tttgacgcat cacttaaaga cgctcttcta 2460
   aagtatatat atgacaatag aggaacttta attggccagg tggatcgctt gaaagataaa 2520
   gttaataaca ctctctctac agatatacca tttcaactaa gcaaatatgt cgataaccag 2580
   aggctgcttt ctaccttcac agagtacatc aaaaatataa ttaataccag tatcttgaat 2640
   ctgaggtatg aatctaacca cttgattgat ctatcacggt acgcaagcaa gataaatatc 2700
   ggctcaaagg tgaatttcga ccccattgac aagaatcaaa tacagctctt caatcttgaa 2760
   tccagtaaaa tcgaggttat tttgaagaat gcaatagtgt ataattcaat gtacgaaaac 2820
   ttttctacct ctttttggat tagaatccca aaatatttca acagcatcag cttgaataac 2880
   gaatatacaa ttataaactg tatggaaaat aactccgggt ggaaagtgag cctgaattac 2940
   ggggaaatca tttggactct acaggatact caagaaatca agcagcgcgt agtcttcaaa 3000
   tattcccaga tgattaacat ctctgattat ataaatagat ggatatttgt gactattaca 3060
   aataacaggc tgaataactc taagatatac attaacggtc gtttaataga tcagaaacca 3120
   atttccaatc tgggaaatat ccatgctagt aataacatta tgtttaagct ggacggatgc 3180
   cgtgataccc atcggtatat atggattaag tacttcaatc tttttgacaa agaactaaat 3240
   gagaaagaga ttaaggatct ttatgataat cagtctaatt ccggaattct gaaagatttc 3300
   tggggcgact acctccaata cgataaaccc tattacatgc ttaacctgta tgacccaaac 3360
   aagtatgtgg atgtcaataa cgtcggaata cgaggctata tgtatctcaa aggacctcgg 3420
   ggctctgtga tgaccactaa catttatttg aactccagtc tttatagggg cacaaagttt 3480
   atcattaaga aatatgcatc tggaaacaaa gacaacatcg tcagaaataa cgatagagtg 3540
   tatatcaatg tggttgtaaa gaacaaagag tataggcttg caacaaatgc ctcacaagct 3600
   ggagtggaga aaattttgtc cgccttggaa attccagatg tagggaatct gtctcaagtg 3660
   gtcgtgatga agagcaagaa tgatcaggga attacaaata agtgtaaaat gaatcttcag 3720
   gataataatg ggaatgatat cgggttcatt ggatttcacc agttcaataa tattgcaaag 3780
   ctggtcgctt caaattggta taatcgtcag atcgaacgct caagccggac actcggctgc 3840
   agctgggagt tcattcctgt tgacgatggc tggggagaac gccccttgta a          3891
<210> 70
   <211> 3891
   <212> DNA
   <213> Artificial Sequence
<220>
   <223> BoNT/A, X. tropicalis-modified 1
<400> 70
   atgcccttcg tgaacaagca gtttaactac aaggaccccg tgaatggagt ggacatcgca 60
   tacattaaaa ttcctaacgc cggacagatg cagccagtta aagcattcaa gatccataat 120
   aagatatggg tcataccaga gcgggatacg tttacgaacc cagaggaagg ggatctaaat 180
   cctccacccg aggcaaagca agtacctgtc tcgtactatg acagtaccta tcttagtacc 240
   gataatgaga aggataacta tctgaaaggt gttacaaagc tattcgaacg tatttattct 300
   actgacctag ggaggatgct tttaacctcc atagtgcgag gtattccctt ctggggaggg 360
   tccacaatcg acaccgagct gaaagtgatc gacactaatt gtattaatgt aatacagcca 420
   gatggatcgt accgaagcga agagcttaat ctcgtgatta tcgggccatc cgctgacatt 480
   atccaatttg agtgcaagag ttttggccat gaggttttga atctcactcg caatggatat 540
   ggatcaactc agtacatcag gttttctcct gatttcacat ttggctttga ggaatccctg 600
   gaggtggaca ccaacccact tctgggcgcg gggaaatttg ccacagatcc tgcagtcact 660
   ctggctcacg agctgattca tgctgggcac agactgtacg gaattgccat caacccgaat 720
   cgtgtattta aggtgaatac caatgcgtac tatgagatga gcggtctgga agtgagcttt 780
   gaggaactaa gaacgtttgg aggtcatgat gctaagttta tcgactcact tcaggaaaac 840
   gaatttaggc tgtattacta taacaaattc aaagatattg cgagtacgct gaacaaagcc 900
   aagtctatcg tcgggacaac tgcttcattg cagtacatga agaatgtgtt caaagaaaaa 960
   tatctgctct ccgaggacac aagtggaaaa ttctccgtgg acaagcttaa atttgataaa 1020
   ttatataaaa tgctgactga gatttacact gaagataact tcgtaaaatt tttcaaggtg 1080
   ctgaatcgaa aaacatatct caactttgat aaggcagtgt tcaaaattaa cattgttcct 1140
   aaggttaact atactattta tgatggtttt aacctaagaa atacaaatct agcagctaat 1200
   ttcaatggcc agaacaccga gatcaataac atgaacttca ccaagctaaa aaactttaca 1260
   ggacttttcg aattctataa gttgctgtgc gtcaggggca tcattacttc caaaaccaag 1320
   tcactggaca aggggtataa taaggcctta aacgacctct gcattaaggt taacaattgg 1380
   gacctgtttt tctctccaag tgaagataac tttactaacg acttgaacaa gggggaagag 1440
   attacatctg atacaaacat tgaagccgca gaggaaaaca ttagtctaga tctgattcag 1500
   caatactatc taaccttcaa tttcgataac gaaccggaaa acatcagtat tgagaacctt 1560
   agctcagaca tcataggcca attggagctg atgcctaata ttgaaaggtt cccaaatggg 1620
   aagaaatatg agctggacaa gtatacaatg tttcattact tgagggccca ggaattcgaa 1680
   cacggcaaat ctcgtatcgc ccttacaaat agcgttaacg aggctctcct aaatcccagt 1740
   cgcgtataca cgtttttcag ttccgattat gttaagaaag tgaacaaagc aactgaggct 1800
   gccatgttct tgggctgggt ggagcaactc gtgtacgatt ttaccgacga aacctccgaa 1860
   gtctctacaa ccgataagat tgccgacata acgattataa ttccatatat aggccccgca 1920
   ctaaacattg gaaatatgtt gtataaggac gattttgtag gtgccttgat cttcagtgga 1980
   gcagtaatct tgcttgagtt tatccctgaa atagccattc cggttctggg tactttcgca 2040
   ctggtcagct atatcgctaa caaggtactg acagttcaaa ctatagacaa tgctctttcc 2100
   aagcggaatg agaaatggga tgaagtctac aaatacatcg tgaccaattg gctcgctaag 2160
   gtgaatacac agatagactt aatccgcaaa aagatgaagg aagctttaga aaaccaagca 2220
   gaagcaacga aagctattat aaactaccag tacaatcagt acacagaaga ggaaaaaaat 2280
   aacatcaatt ttaatattga tgacctgtca tctaagctta acgagagcat aaataaggca 2340
   atgataaata ttaataaatt tctgaatcag tgctcagtaa gctacctgat gaacagtatg 2400
   ataccgtatg gagttaaacg tttggaggat ttcgatgcat cgctcaaaga tgcactgctt 2460
   aagtatattt acgataatcg cggaacactt atcggtcagg tggatcggct gaaggacaag 2520
   gtcaataaca ctctgtcaac tgacatcccc ttccagctct ccaaatatgt tgataaccag 2580
   cgtctgctct caacctttac cgaatatata aaaaatatta tcaacacctc catactcaat 2640
   ctgaggtatg aaagcaacca cctcattgac ctgagcagat atgcttctaa gatcaacatc 2700
   ggttctaaag tcaacttcga ccctatcgat aagaaccaga ttcagttgtt taaccttgag 2760
   tccagcaaga tagaagtgat tttaaaaaac gcgattgtat acaattcaat gtatgaaaac 2820
   tttagcacct cattctggat taggatccca aaatacttta actccatttc tctgaataac 2880
   gagtacacca ttatcaactg tatggagaat aactccggat ggaaggtcag ccttaactac 2940
   ggcgaaatca tttggacact tcaggataca caggaaataa agcagagggt cgtgttcaag 3000
   tattctcaaa tgatcaatat cagcgattac ataaaccgct ggatattcgt aactattact 3060
   aataaccggt tgaacaattc taagatttat atcaacggga gattgatcga tcaaaagccc 3120
   ataagcaact taggtaacat ccacgcttct aacaatatca tgtttaaact tgatggatgt 3180
   cgggacacac accgctacat ttggattaaa tactttaatc ttttcgacaa agaactgaat 3240
   gaaaaggaga tcaaagacct ctatgacaat caatcaaaca gcggaatcct gaaggacttt 3300
   tggggagatt acctgcagta cgataaacca tattacatgt tgaatttgta cgatccaaat 3360
   aagtatgtcg atgttaacaa tgtgggtata cgaggctaca tgtatttaaa agggcctaga 3420
   ggttcggtta tgaccacaaa tatttactta aattcaagtt tatatagggg aacaaaattt 3480
   ataattaaaa agtacgcctc tggcaataaa gataatatag tcagaaataa cgacagagtt 3540
   tacattaatg ttgtggttaa aaataaggag tatcgactcg ccactaatgc ttctcaggcc 3600
   ggagtggaga aaatcctgag cgccttggag attcctgacg tgggcaactt gagtcaagtg 3660
   gttgtaatga aatctaaaaa tgatcagggc attacaaaca agtgcaagat gaatcttcaa 3720
   gataataacg gcaacgatat cggtttcata ggctttcatc aatttaataa catcgcaaaa 3780
   ctcgttgctt ctaattggta caatcggcag atcgagagaa gctctagaac tttagggtgt 3840
   tcctgggagt ttatacctgt ggatgatggg tggggagaaa gacccctgta a          3891
<210> 71
   <211> 3891
   <212> DNA
   <213> Artificial Sequence
<220>
   <223> BoNT/A, X. tropicalis-modified 2
<400> 71
   atgccatttg tgaataagca gtttaattat aaggatccag tgaatggagt ggatattgca 60
   tatattaaga ttccaaatgc aggacagatg cagccagtga aggcatttaa gattcataat 120
   aagatttggg tgattccaga aagagataca tttacaaatc cagaagaagg agatctgaat 180
   ccaccaccag aagcaaagca ggtgccagtg tcttattatg attctacata tctgtctaca 240
   gataatgaaa aggataatta tctgaaggga gtgacaaagc tgtttgaaag aatttattct 300
   acagatctgg gaagaatgct gctgacatct attgtgagag gaattccatt ttggggagga 360
   tctacaattg atacagaact gaaggtgatt gatacaaatt gcattaatgt gattcagcca 420
   gatggatctt atagatctga agaactgaat ctggtgatta ttggaccatc tgcagatatt 480
   attcagtttg aatgcaagtc ttttggacat gaagtgctga atctgacaag aaatggatat 540
   ggatctacac agtatattag attttctcca gattttacat ttggatttga agaatctctg 600
   gaagtggata caaatccact gctgggagca ggaaagtttg caacagatcc agcagtgaca 660
   ctggcacatg aactgattca tgcaggacat agactgtatg gaattgcaat taatccaaat 720
   agagtgttta aggtgaatac aaatgcatat tatgaaatgt ctggactgga agtgtctttt 780
   gaagaactga gaacatttgg aggacatgat gcaaagttta ttgattctct gcaggaaaat 840
   gaatttagac tgtattatta taataagttt aaggatattg catctacact gaataaggca 900
   aagtctattg tgggaacaac agcatctctg cagtatatga agaatgtgtt taaggaaaag 960
   tatctgctgt ctgaagatac atctggaaag ttttctgtgg ataagctgaa gtttgataag 1020
   ctgtataaga tgctgacaga aatttataca gaagataatt ttgtgaagtt ttttaaggtg 1080
   ctgaatagaa agacatatct gaattttgat aaggcagtgt ttaagattaa tattgtgcca 1140
   aaggtgaatt atacaattta tgatggattt aatctgagaa atacaaatct ggcagcaaat 1200
   tttaatggac agaatacaga aattaataat atgaatttta caaagctgaa gaattttaca 1260
   ggactgtttg aattttataa gctgctgtgc gtgagaggaa ttattacatc taagacaaag 1320
   tctctggata agggatataa taaggcactg aatgatctgt gcattaaggt gaataattgg 1380
   gatctgtttt tttctccatc tgaagataat tttacaaatg atctgaataa gggagaagaa 1440
   attacatctg atacaaatat tgaagcagca gaagaaaata tttctctgga tctgattcag 1500
   cagtattatc tgacatttaa ttttgataat gaaccagaaa atatttctat tgaaaatctg 1560
   tcttctgata ttattggaca gctggaactg atgccaaata ttgaaagatt tccaaatgga 1620
   aagaagtatg aactggataa gtatacaatg tttcattatc tgagagcaca ggaatttgaa 1680
   catggaaagt ctagaattgc actgacaaat tctgtgaatg aagcactgct gaatccatct 1740
   agagtgtata catttttttc ttctgattat gtgaagaagg tgaataaggc aacagaagca 1800
   gcaatgtttc tgggatgggt ggaacagctg gtgtatgatt ttacagatga aacatctgaa 1860
   gtgtctacaa cagataagat tgcagatatt acaattatta ttccatatat tggaccagca 1920
   ctgaatattg gaaatatgct gtataaggat gattttgtgg gagcactgat tttttctgga 1980
   gcagtgattc tgctggaatt tattccagaa attgcaattc cagtgctggg aacatttgca 2040
   ctggtgtctt atattgcaaa taaggtgctg acagtgcaga caattgataa tgcactgtct 2100
   aagagaaatg aaaagtggga tgaagtgtat aagtatattg tgacaaattg gctggcaaag 2160
   gtgaatacac agattgatct gattagaaag aagatgaagg aagcactgga aaatcaggca 2220
   gaagcaacaa aggcaattat taattatcag tataatcagt atacagaaga agaaaagaat 2280
   aatattaatt ttaatattga tgatctgtct tctaagctga atgaatctat taataaggca 2340
   atgattaata ttaataagtt tctgaatcag tgctctgtgt cttatctgat gaattctatg 2400
   attccatatg gagtgaagag actggaagat tttgatgcat ctctgaagga tgcactgctg 2460
   aagtatattt atgataatag aggaacactg attggacagg tggatagact gaaggataag 2520
   gtgaataata cactgtctac agatattcca tttcagctgt ctaagtatgt ggataatcag 2580
   agactgctgt ctacatttac agaatatatt aagaatatta ttaatacatc tattctgaat 2640
   ctgagatatg aatctaatca tctgattgat ctgtctagat atgcatctaa gattaatatt 2700
   ggatctaagg tgaattttga tccaattgat aagaatcaga ttcagctgtt taatctggaa 2760
   tcttctaaga ttgaagtgat tctgaagaat gcaattgtgt ataattctat gtatgaaaat 2820
   ttttctacat ctttttggat tagaattcca aagtatttta attctatttc tctgaataat 2880
   gaatatacaa ttattaattg catggaaaat aattctggat ggaaggtgtc tctgaattat 2940
   ggagaaatta tttggacact gcaggataca caggaaatta agcagagagt ggtgtttaag 3000
   tattctcaga tgattaatat ttctgattat attaatagat ggatttttgt gacaattaca 3060
   aataatagac tgaataattc taagatttat attaatggaa gactgattga tcagaagcca 3120
   atttctaatc tgggaaatat tcatgcatct aataatatta tgtttaagct ggatggatgc 3180
   agagatacac atagatatat ttggattaag tattttaatc tgtttgataa ggaactgaat 3240
   gaaaaggaaa ttaaggatct gtatgataat cagtctaatt ctggaattct gaaggatttt 3300
   tggggagatt atctgcagta tgataagcca tattatatgc tgaatctgta tgatccaaat 3360
   aagtatgtgg atgtgaataa tgtgggaatt agaggatata tgtatctgaa gggaccaaga 3420
   ggatctgtga tgacaacaaa tatttatctg aattcttctc tgtatagagg aacaaagttt 3480
   attattaaga agtatgcatc tggaaataag gataatattg tgagaaataa tgatagagtg 3540
   tatattaatg tggtggtgaa gaataaggaa tatagactgg caacaaatgc atctcaggca 3600
   ggagtggaaa agattctgtc tgcactggaa attccagatg tgggaaatct gtctcaggtg 3660
   gtggtgatga agtctaagaa tgatcaggga attacaaata agtgcaagat gaatctgcag 3720
   gataataatg gaaatgatat tggatttatt ggatttcatc agtttaataa tattgcaaag 3780
   ctggtggcat ctaattggta taatagacag attgaaagat cttctagaac actgggatgc 3840
   tcttgggaat ttattccagt ggatgatgga tggggagaaa gaccactgta a 3891
<210> 72
   <211> 3891
   <212> DNA
   <213> Artificial Sequence
<220>
   <223> BoNT/A, X. tropicalis-modified 3
<400> 72
   atgccattcg ttaataagca gttcaactac aaagacccag tgaacggggt agatattgcc 60
   tatattaaga tcccaaacgc tggacagatg caacccgtca aagcatttaa gattcataac 120
   aaaatctggg ttatccctga gcgagacact tttacaaacc ccgaagaggg cgacctcaat 180
   ccaccccctg aagcaaagca ggttccagtc tcatattacg attccacata tttgtctacc 240
   gacaacgaaa aggataatta cttaaagggg gtgaccaaac tctttgagag aatctatagc 300
   acagacctgg gtcgtatgct tttgacatca atagtcagag ggatcccttt ctggggtgga 360
   tccactattg ataccgaact gaaggtgatc gatactaatt gtatcaacgt aatccaacct 420
   gatggatctt atcgaagcga agagttgaat ttggttatta tcggaccaag cgccgacatt 480
   atccagtttg agtgcaaatc attcggacac gaagtattaa atctaactag gaacggatat 540
   ggttcaacac agtatattcg gttctctcca gattttacat ttgggttcga agagagtctt 600
   gaggtggaca ccaacccact ccttggcgct ggtaagtttg caaccgatcc agctgtaaca 660
   ctagcacatg aactcattca cgctgggcac cgcctgtacg ggatcgccat taacccaaac 720
   agagtcttta aagttaatac taatgcctac tatgaaatga gcgggctgga ggtgagtttc 780
   gaggaattga ggacatttgg aggccacgat gcaaaattca tcgactctct tcaggagaat 840
   gagttccgtc tctattacta taataaattc aaagacatcg cctcaacact taataaggcc 900
   aagagtatcg taggtaccac agcaagtctt caatacatga aaaatgtctt taaggaaaaa 960
   tacctgttat ccgaggatac atctggaaag ttctctgtgg ataagttgaa atttgataaa 1020
   ttgtataaga tgttaaccga aatttatact gaggataatt ttgtcaagtt ctttaaggtt 1080
   cttaatagaa aaacctacct gaacttcgac aaggctgtct ttaaaataaa tattgtgccc 1140
   aaggttaact acacaatcta cgatggattt aacttgcgga acacaaatct cgctgcaaac 1200
   tttaacggac aaaatacaga gataaataac atgaatttta caaagcttaa gaattttaca 1260
   gggctgtttg agttctataa attgctttgc gttaggggca tcattactag taaaaccaag 1320
   agcctggaca agggctacaa taaagcttta aatgacttgt gtataaaagt taataactgg 1380
   gaccttttct tttctccctc cgaagataat tttactaatg atctaaataa gggagaggaa 1440
   atcactagtg atacaaatat agaggctgcc gaagagaaca ttagtcttga cttgatccag 1500
   caatactatc taacttttaa cttcgataat gagccagaaa acatctctat tgaaaacctg 1560
   tcatctgata ttatcggcca gctggagctg atgcctaaca tagagcgatt ccctaacggc 1620
   aaaaagtatg aactcgacaa gtataccatg ttccattacc tgcgcgcaca agagtttgaa 1680
   catggaaaaa gccgcattgc ccttactaat tctgtcaatg aggcattatt gaacccatcc 1740
   cgggtctaca ctttcttttc ctcagattat gtcaagaaag taaataaggc aacagaggct 1800
   gcaatgttcc tcggatgggt ggaacagcta gtctatgatt ttacagatga aacaagcgaa 1860
   gtcagcacca ctgacaagat cgccgacatc accataatta tcccatacat tggaccagca 1920
   ctcaacatcg gcaacatgtt gtacaaagat gacttcgtcg gtgctctcat ttttagcgga 1980
   gcagttattc tcttagagtt tattcctgaa atagcaattc ctgtgttagg caccttcgct 2040
   ctggttagct atattgcaaa taaggttctt accgtgcaga ctatagacaa tgcattgagc 2100
   aaaaggaacg aaaagtggga tgaggtatac aaatacattg ttacaaactg gctcgccaag 2160
   gtaaacactc agatcgatct catacggaag aaaatgaagg aggcccttga gaaccaggct 2220
   gaagcaacaa aagcaatcat aaattatcag tacaatcagt acacagagga agagaagaat 2280
   aacattaatt tcaacataga cgatctgtct tcaaaactaa acgaatctat aaataaggct 2340
   atgatcaata ttaataagtt cctgaatcag tgctccgttt cttatcttat gaacagtatg 2400
   ataccttacg gggtgaagcg actagaagac ttcgacgcat cccttaagga tgccctgttg 2460
   aaatatatat acgacaatag aggcacactg attggtcagg tggatagact gaaagacaaa 2520
   gtgaataaca ctctgagcac cgatattccc ttccagctga gcaaatacgt cgataatcaa 2580
   aggctactgt ctacattcac cgagtatatt aagaatataa ttaatacttc cattctcaac 2640
   ctgagatacg aatctaacca tctgatcgat ctgagtagat atgctagcaa aattaatatc 2700
   ggctcaaaag tgaattttga cccaatcgat aaaaaccaga ttcagctctt taacctggag 2760
   agttccaaga tagaagtaat attaaagaac gccatcgttt ataatagtat gtacgaaaat 2820
   tttagtacat ctttttggat acgcattccc aaatacttta atagcatttc cttgaacaat 2880
   gaatacacta taattaactg tatggagaac aattccggtt ggaaggtgag tctaaactac 2940
   ggtgagatca tttggacact tcaagacaca caggaaatta agcaaagggt tgtatttaaa 3000
   tacagccaaa tgatcaacat ctctgactac attaatcgtt ggatttttgt gaccatcacc 3060
   aataacagac ttaacaatag caaaatatat ataaatggta ggcttattga tcaaaagcct 3120
   atttccaacc tgggaaacat ccacgcatct aacaatatca tgttcaaact ggacggatgt 3180
   cgggacactc acaggtacat ttggatcaaa tactttaacc tgtttgataa ggagttaaat 3240
   gaaaaagaaa ttaaagatct atatgataac cagtcaaact ctggaatatt gaaagatttt 3300
   tggggggatt acctgcaata tgacaaaccc tattacatgc tgaacctgta tgatcctaac 3360
   aagtatgtag atgtaaacaa tgtggggatt cggggctata tgtatctgaa agggccaaga 3420
   ggaagcgtta tgaccacaaa catctatctg aactcatccc tgtatagggg caccaaattc 3480
   atcattaaaa agtatgcatc tggtaacaaa gacaacattg tgcgtaacaa tgatcgcgtg 3540
   tacattaatg tggttgtgaa gaataaggaa tatagattag ctacaaacgc ctcccaagcc 3600
   ggagtggaga agatactatc cgctctggag atcccagatg ttggtaatct gtctcaggtg 3660
   gtggtgatga aaagtaagaa tgaccagggc atcacaaata agtgcaaaat gaacctgcag 3720
   gataacaatg gcaacgatat aggctttata ggattccatc agttcaataa catcgccaag 3780
   ctcgtggctt ctaattggta caatcgacag attgaacgct cttcacgtac tttgggttgc 3840
   tcatgggaat tcatacctgt ggacgacggg tggggggaga ggccccttta a          3891
<210> 73
   <211> 3891
   <212> DNA
   <213> Artificial Sequence
<220>
   <223> BoNT/A, G. gallus-modified 1
<400> 73
   atgcctttcg tgaacaagca gttcaactat aaggatccag tgaacggggt ggacatcgct 60
   tacatcaaga tcccaaacgc aggacagatg caacccgtga aagcttttaa gattcacaat 120
   aaaatttggg tcattccaga acgggacaca ttcactaacc ctgaggaagg cgatctgaac 180
   ccccctccag aggcgaaaca ggtgcccgtc agctattacg acagtaccta cttatctact 240
   gacaatgaga aagacaatta cttgaagggc gtgactaagc ttttcgaaag gatatacagc 300
   actgatcttg gcagaatgct tctgaccagc attgtacgcg gcattccgtt ctggggggga 360
   agtactattg acacagaact taaagttatc gatactaact gtatcaacgt tatccagcct 420
   gacggctcct atcgctccga ggaactaaac cttgttatta taggtccgag tgcggacatc 480
   atacagttcg aatgcaaatc gttcgggcac gaggtcctga atctgacccg caacgggtac 540
   ggatccacac agtatatcag gttttccccc gacttcacct ttggtttcga agagtctttg 600
   gaagtggaca caaaccctct gctcggcgca ggtaaatttg ctacagaccc agcagtgacc 660
   ctcgctcatg aacttattca tgcaggccat cggctctatg ggatagccat caacccaaat 720
   cgggtgttta aggttaatac taacgcctac tatgagatgt cgggcctgga agtcagcttc 780
   gaggaactcc gcacgttcgg cggtcacgat gctaagttta tcgattccct ccaggagaac 840
   gagttccggt tgtactatta caacaagttc aaagacatcg ctagcacatt aaataaggcc 900
   aaatcaatcg tgggaacaac cgcttcgctg cagtatatga agaacgtttt caaagagaag 960
   tatctgttgt cggaagatac aagcggaaag ttctctgtgg ataaattgaa gtttgataaa 1020
   ttgtacaaaa tgttgaccga aatctatacc gaggataatt ttgtgaagtt ctttaaagtc 1080
   ctgaaccgta agacatatct taacttcgat aaagccgtct ttaagattaa cattgtgcct 1140
   aaagttaact acacaatata tgacggattc aatttgagga atactaacct ggctgcgaac 1200
   tttaatggcc agaacaccga aatcaacaat atgaatttta ccaaactcaa gaactttacg 1260
   gggctctttg agttctacaa gctgttatgc gtacgaggga tcattacctc aaagacaaag 1320
   agtttggata agggttataa taaggcccta aacgatctgt gcattaaagt taacaattgg 1380
   gacctttttt tctcaccctc cgaggacaat ttcacgaacg acctcaacaa aggagaagag 1440
   atcacaagcg acaccaatat cgaggcagcg gaggaaaaca tttccctaga cctcattcag 1500
   caatattacc tgacattcaa ttttgataat gaaccggaga atatctctat cgagaatctg 1560
   agttctgaca ttataggaca acttgagctg atgcccaaca tcgagagatt tcctaatggg 1620
   aagaaatacg agctggacaa gtataccatg tttcactacc tgcgcgctca ggaattcgag 1680
   cacggtaaat cgaggattgc acttaccaat agcgtcaacg aggccctgtt gaacccttct 1740
   agggtgtaca ctttcttttc ttccgattat gtgaaaaagg taaacaaggc gaccgaagca 1800
   gctatgttcc tgggctgggt tgagcagctc gtctatgatt ttaccgacga gactagtgag 1860
   gtgtccacaa cggacaagat tgcagatatc actattatca taccatacat aggtcccgcc 1920
   ctgaatatcg gaaacatgct gtacaaggat gacttcgtcg gagccctgat cttctccggg 1980
   gctgtcattc tgctcgagtt catccctgaa atagccatcc cggtcctggg taccttcgca 2040
   ttggtgtcat acatcgctaa taaagttctg accgtgcaga cgatagataa cgctctgagc 2100
   aaacgaaatg agaagtggga tgaggtgtat aagtacatcg taaccaactg gcttgcaaaa 2160
   gtgaacaccc aaattgatct gatcagaaag aaaatgaagg aggccctgga aaaccaagct 2220
   gaagccacaa aggcaattat caactaccaa tacaatcaat atacagagga agagaaaaac 2280
   aatattaact ttaatatcga tgaccttagc agtaaactga atgaaagcat caataaagca 2340
   atgataaata ttaacaaatt tctgaaccaa tgctcagtga gctacctgat gaatagtatg 2400
   ataccatatg gcgtaaaacg gctcgaggac tttgacgcct ccctgaagga cgccctcctt 2460
   aaatacatat acgacaatcg tggtacgctt atcggacagg tggaccgctt aaaggataag 2520
   gttaataaca ctctgtctac cgatatcccc tttcaactga gcaagtatgt ggataaccag 2580
   agactgctct ctacattcac agagtatatc aaaaacataa ttaatactag cattttgaac 2640
   ctccggtacg agtcaaatca tctgatcgac ctgagtaggt acgccagtaa gatcaacatt 2700
   gggagcaagg ttaatttcga tccaatcgat aagaaccaga tccagttatt taacctggag 2760
   tcctcaaaaa tcgaagtaat actcaaaaac gccatcgttt acaatagcat gtacgaaaat 2820
   ttttccactt ctttttggat aaggattccc aagtacttca acagcatctc actgaacaat 2880
   gaatacacaa tcattaactg tatggagaac aattctggct ggaaggtttc tttgaattac 2940
   ggcgagataa tttggaccct acaggatacg caggaaataa aacagcgagt ggtcttcaaa 3000
   tacagccaga tgatcaacat ttccgactac attaacagat ggatattcgt gactataact 3060
   aacaataggc tgaacaatag caaaatctac atcaatggcc ggctcattga ccagaagccg 3120
   atttcgaatc tgggaaacat tcacgcttca aacaatataa tgtttaagct agatggctgc 3180
   cgtgatactc acagatatat atggatcaaa tatttcaatt tatttgataa ggaacttaac 3240
   gagaaggaaa tcaaggattt atacgacaac cagagtaact ctggaattct caaagacttt 3300
   tggggggatt acctccaata cgataaacct tattacatgc tgaatttgta cgacccaaat 3360
   aaatacgtag atgttaacaa tgtcggaatc agagggtata tgtatctaaa aggacccaga 3420
   ggatcagtaa tgacgactaa catctactta aacagctcac tgtatcgggg tacgaaattc 3480
   atcattaaga aatacgcgag cggcaacaag gacaatatcg tgcgaaataa cgacagagtg 3540
   tacattaacg tcgtggtaaa gaacaaagaa tatcgcctcg ctacgaacgc atctcaggca 3600
   ggggttgaaa agattctgtc cgcgctggag attcctgacg tcggaaattt gtcccaagtg 3660
   gtcgtgatga agtctaagaa cgatcagggg attacaaata agtgtaagat gaacctacag 3720
   gacaacaatg gaaatgatat cggctttatt ggcttccatc agttcaataa catcgccaag 3780
   ctggtggcct caaattggta taaccgccag atcgaaagaa gctcccgtac attaggctgt 3840
   agttgggagt ttattcccgt ggatgatggt tgggggagga ggccactgta a 3891
<210> 74
   <211> 3891
   <212> DNA
   <213> Artificial Sequence
<220>
   <223> BoNT/A, G. gallus-modified 2
<400> 74
   atgccattcg tgaacaagca gttcaactac aaggatccag tgaacggcgt ggatatcgcc 60
   tacatcaaga tcccaaacgc cggccagatg cagccagtga aggccttcaa gatccacaac 120
   aagatctggg tgatcccaga gagagatacc ttcaccaacc cagaggaggg cgatctgaac 180
   ccaccaccag aggccaagca ggtgccagtg agctactacg atagcaccta cctgagcacc 240
   gataacgaga aggataacta cctgaagggc gtgaccaagc tgttcgagag aatctacagc 300
   accgatctgg gcagaatgct gctgaccagc atcgtgagag gcatcccatt ctggggcggc 360
   agcaccatcg ataccgagct gaaggtgatc gataccaact gcatcaacgt gatccagcca 420
   gatggcagct acagaagcga ggagctgaac ctggtgatca tcggcccaag cgccgatatc 480
   atccagttcg agtgcaagag cttcggccac gaggtgctga acctgaccag aaacggctac 540
   ggcagcaccc agtacatcag attcagccca gatttcacct tcggcttcga ggagagcctg 600
   gaggtggata ccaacccact gctgggcgcc ggcaagttcg ccaccgatcc agccgtgacc 660
   ctggcccacg agctgatcca cgccggccac agactgtacg gcatcgccat caacccaaac 720
   agagtgttca aggtgaacac caacgcctac tacgagatga gcggcctgga ggtgagcttc 780
   gaggagctga gaaccttcgg cggccacgat gccaagttca tcgatagcct gcaggagaac 840
   gagttcagac tgtactacta caacaagttc aaggatatcg ccagcaccct gaacaaggcc 900
   aagagcatcg tgggcaccac cgccagcctg cagtacatga agaacgtgtt caaggagaag 960
   tacctgctga gcgaggatac cagcggcaag ttcagcgtgg ataagctgaa gttcgataag 1020
   ctgtacaaga tgctgaccga gatctacacc gaggataact tcgtgaagtt cttcaaggtg 1080
   ctgaacagaa agacctacct gaacttcgat aaggccgtgt tcaagatcaa catcgtgcca 1140
   aaggtgaact acaccatcta cgatggcttc aacctgagaa acaccaacct ggccgccaac 1200
   ttcaacggcc agaacaccga gatcaacaac atgaacttca ccaagctgaa gaacttcacc 1260
   ggcctgttcg agttctacaa gctgctgtgc gtgagaggca tcatcaccag caagaccaag 1320
   agcctggata agggctacaa caaggccctg aacgatctgt gcatcaaggt gaacaactgg 1380
   gatctgttct tcagcccaag cgaggataac ttcaccaacg atctgaacaa gggcgaggag 1440
   atcaccagcg ataccaacat cgaggccgcc gaggagaaca tcagcctgga tctgatccag 1500
   cagtactacc tgaccttcaa cttcgataac gagccagaga acatcagcat cgagaacctg 1560
   agcagcgata tcatcggcca gctggagctg atgccaaaca tcgagagatt cccaaacggc 1620
   aagaagtacg agctggataa gtacaccatg ttccactacc tgagagccca ggagttcgag 1680
   cacggcaaga gcagaatcgc cctgaccaac agcgtgaacg aggccctgct gaacccaagc 1740
   agagtgtaca ccttcttcag cagcgattac gtgaagaagg tgaacaaggc caccgaggcc 1800
   gccatgttcc tgggctgggt ggagcagctg gtgtacgatt tcaccgatga gaccagcgag 1860
   gtgagcacca ccgataagat cgccgatatc accatcatca tcccatacat cggcccagcc 1920
   ctgaacatcg gcaacatgct gtacaaggat gatttcgtgg gcgccctgat cttcagcggc 1980
   gccgtgatcc tgctggagtt catcccagag atcgccatcc cagtgctggg caccttcgcc 2040
   ctggtgagct acatcgccaa caaggtgctg accgtgcaga ccatcgataa cgccctgagc 2100
   aagagaaacg agaagtggga tgaggtgtac aagtacatcg tgaccaactg gctggccaag 2160
   gtgaacaccc agatcgatct gatcagaaag aagatgaagg aggccctgga gaaccaggcc 2220
   gaggccacca aggccatcat caactaccag tacaaccagt acaccgagga ggagaagaac 2280
   aacatcaact tcaacatcga tgatctgagc agcaagctga acgagagcat caacaaggcc 2340
   atgatcaaca tcaacaagtt cctgaaccag tgcagcgtga gctacctgat gaacagcatg 2400
   atcccatacg gcgtgaagag actggaggat ttcgatgcca gcctgaagga tgccctgctg 2460
   aagtacatct acgataacag aggcaccctg atcggccagg tggatagact gaaggataag 2520
   gtgaacaaca ccctgagcac cgatatccca ttccagctga gcaagtacgt ggataaccag 2580
   agactgctga gcaccttcac cgagtacatc aagaacatca tcaacaccag catcctgaac 2640
   ctgagatacg agagcaacca cctgatcgat ctgagcagat acgccagcaa gatcaacatc 2700
   ggcagcaagg tgaacttcga tccaatcgat aagaaccaga tccagctgtt caacctggag 2760
   agcagcaaga tcgaggtgat cctgaagaac gccatcgtgt acaacagcat gtacgagaac 2820
   ttcagcacca gcttctggat cagaatccca aagtacttca acagcatcag cctgaacaac 2880
   gagtacacca tcatcaactg catggagaac aacagcggct ggaaggtgag cctgaactac 2940
   ggcgagatca tctggaccct gcaggatacc caggagatca agcagagagt ggtgttcaag 3000
   tacagccaga tgatcaacat cagcgattac atcaacagat ggatcttcgt gaccatcacc 3060
   aacaacagac tgaacaacag caagatctac atcaacggca gactgatcga tcagaagcca 3120
   atcagcaacc tgggcaacat ccacgccagc aacaacatca tgttcaagct ggatggctgc 3180
   agagataccc acagatacat ctggatcaag tacttcaacc tgttcgataa ggagctgaac 3240
   gagaaggaga tcaaggatct gtacgataac cagagcaaca gcggcatcct gaaggatttc 3300
   tggggcgatt acctgcagta cgataagcca tactacatgc tgaacctgta cgatccaaac 3360
   aagtacgtgg atgtgaacaa cgtgggcatc agaggctaca tgtacctgaa gggcccaaga 3420
   ggcagcgtga tgaccaccaa catctacctg aacagcagcc tgtacagagg caccaagttc 3480
   atcatcaaga agtacgccag cggcaacaag gataacatcg tgagaaacaa cgatagagtg 3540
   tacatcaacg tggtggtgaa gaacaaggag tacagactgg ccaccaacgc cagccaggcc 3600
   ggcgtggaga agatcctgag cgccctggag atcccagatg tgggcaacct gagccaggtg 3660
   gtggtgatga agagcaagaa cgatcagggc atcaccaaca agtgcaagat gaacctgcag 3720
   gataacaacg gcaacgatat cggcttcatc ggcttccacc agttcaacaa catcgccaag 3780
   ctggtggcca gcaactggta caacagacag atcgagagaa gcagcagaac cctgggctgc 3840
   agctgggagt tcatcccagt ggatgatggc tggggcgaga gaccactgta a          3891
<210> 75
   <211> 3891
   <212> DNA
   <213> Artificial Sequence
<220>
   <223> BoNT/A, G. gallus-modified 3
<400> 75
   atgcccttcg tgaacaaaca gttcaactat aaggacccgg taaatggggt ggacattgct 60
   tacatcaaga tccctaacgc cgggcagatg cagccagtga aggcgttcaa aatacataac 120
   aaaatctggg tgattcccga acgggatacc tttacaaacc ctgaggaagg cgacctgaat 180
   cctcccccag aagcaaagca ggtcccagta agctattacg attccaccta tctcagcacc 240
   gacaatgaaa aagataatta tctgaagggc gtgaccaaac ttttcgaaag aatatatagc 300
   acagaccttg gccggatgct gttgacttct attgtgaggg ggattccctt ttggggcggg 360
   agcaccatag atacggagct taaggttatc gacacaaact gcataaacgt tatccagcct 420
   gacggatcat atcggagcga ggaactgaat ctggtcatta tcggcccctc tgctgacatt 480
   atccagtttg agtgtaaatc atttggtcac gaggttttga acctcacacg taacgggtac 540
   gggagtactc agtatatccg ctttagccca gatttcactt tcggattcga ggaaagcctg 600
   gaggtcgata ccaatccgct gcttggagcc ggaaagttcg caacggaccc cgcagtaacc 660
   ctggcacacg agctcattca cgcagggcac cgcctgtatg gaattgccat aaatccaaac 720
   cgcgtgttca aggtaaacac aaacgcatat tacgagatga gcggactgga ggttagcttc 780
   gaagagctta gaaccttcgg cgggcacgat gctaaattta tcgacagtct ccaggagaat 840
   gaattccgat tgtattacta taacaagttc aaggatatcg cctctacttt gaataaagct 900
   aagtctatag tgggaacaac cgccagcctt cagtatatga aaaatgtttt caaggagaag 960
   tacctgctca gtgaagacac cagcggaaag ttcagcgtag acaaactgaa gttcgacaag 1020
   ctgtacaaga tgctgacgga aatttataca gaggataact ttgtgaaatt tttcaaggtg 1080
   ctcaaccgca agacgtactt gaattttgac aaggcagtct tcaagatcaa cattgttcca 1140
   aaggtgaatt acactatcta cgacggcttc aacttgagaa acaccaacct ggcggctaac 1200
   tttaacggcc agaacaccga gattaataac atgaacttca ctaagctgaa aaacttcaca 1260
   ggtctcttcg agttctacaa acttctgtgt gtgaggggaa tcattacatc taaaaccaag 1320
   tccctggaca aaggctataa taaagcgctt aatgacctgt gtatcaaagt caataactgg 1380
   gacctgtttt tcagtccgtc tgaggataac ttcaccaacg acctgaacaa aggcgaggaa 1440
   ataacctctg acacgaatat cgaggctgcc gaagagaaca tctcactgga tcttattcag 1500
   caatactatc tcacttttaa ctttgataat gagcctgaga atatctcaat tgaaaacctc 1560
   agtagcgaca ttatcggaca gctggaactg atgccaaaca tcgagaggtt ccccaacggc 1620
   aagaaatacg agctggataa gtacactatg tttcattacc tgagagcaca agagtttgaa 1680
   catggtaagt ccaggatagc cctgacgaac tccgtgaatg aagccttgct gaatcctagc 1740
   agagtttaca cattcttttc aagtgattac gtcaagaaag tgaataaggc gacggaagcc 1800
   gctatgtttc tggggtgggt cgaacaactg gtgtatgatt tcaccgatga gacttccgag 1860
   gtctctacaa ctgacaagat tgctgacatc actattataa tcccttatat cggacccgct 1920
   ctgaatatag gtaacatgct ttacaaagat gactttgtag gtgctctgat atttagcggc 1980
   gccgttatcc tcctggagtt catcccagag atcgccattc ctgtgttggg tactttcgca 2040
   ctggtttctt acatcgctaa taaggtcctc acagtccaga ctatcgacaa cgcgttgagc 2100
   aaacgaaatg aaaaatggga tgaagtgtac aagtacattg tgacgaactg gctggctaaa 2160
   gtgaacaccc aaatagacct gatcagaaaa aagatgaaag aagccctgga aaaccaggct 2220
   gaggccacaa aagccattat caattatcag tataatcaat acacagaaga ggaaaaaaac 2280
   aatatcaatt ttaatatcga cgatctttca tccaagttga atgagagcat caacaaagcg 2340
   atgattaata taaataagtt cctgaaccag tgctccgtga gctaccttat gaattctatg 2400
   attccctacg gtgtcaaaag gctggaggat tttgatgcca gtctgaagga tgcactgctc 2460
   aaatacatct acgacaaccg cggcactctg ataggacaag tagatcgtct gaaagataag 2520
   gttaataaca ccttgtctac tgacattcca tttcagctgt ccaaatacgt tgataaccaa 2580
   aggctgttga gcacgtttac ggagtatatt aaaaacatca taaacacaag cattctgaac 2640
   ctgaggtatg aaagtaatca cctcatcgat ctcagcagat acgcttcaaa aatcaatatt 2700
   ggatccaagg tgaatttcga tcctatagat aagaaccaaa tccagttgtt caatctcgaa 2760
   tcaagcaaga tcgaagtaat cctgaaaaat gcaatcgttt acaattccat gtacgagaat 2820
   tttagtacat ccttttggat taggatacct aaatacttca acagcatttc cttgaacaat 2880
   gagtacacaa ttatcaactg catggagaat aactctggct ggaaagtgag ccttaattat 2940
   ggggaaatca tttggaccct gcaagacaca caggagatta agcagcgagt ggttttcaag 3000
   tactcccaga tgatcaacat ctccgattat attaatagat ggatcttcgt tacaattact 3060
   aacaatcggc tcaacaattc caagatttac atcaatggac gtctgattga tcagaagccc 3120
   atctctaacc tgggtaacat tcacgctagt aacaatatca tgtttaagct ggacggttgt 3180
   cgtgacaccc atcgctacat ttggataaag tattttaatc tgtttgacaa agaactcaac 3240
   gaaaaggaaa taaaggacct gtacgataat cagagcaatt cagggatcct caaagatttt 3300
   tggggagact acctgcagta cgataaaccg tattacatgc tcaatttgta cgatcccaac 3360
   aagtacgtag acgtgaataa cgtcggtatc cgcggctaca tgtacctgaa aggaccgagg 3420
   gggagcgtga tgactacaaa catttacctg aacagctctc tgtatagagg gaccaagttt 3480
   ataattaaga aatatgcatc cggaaacaag gataatatcg tgcgcaataa cgatcgggtg 3540
   tatatcaatg tggtcgtgaa gaacaaagag tatagactgg ccaccaacgc gagtcaagca 3600
   ggagtggaga agatactttc tgccctggag atcccagatg tcggcaacct tagtcaagtg 3660
   gtcgttatga aatccaagaa cgatcagggc attactaaca agtgcaaaat gaacctgcag 3720
   gacaacaatg gtaacgacat cgggttcatt ggctttcatc agtttaacaa tatcgctaag 3780
   ctggtcgcat caaactggta taaccggcag attgaacgaa gctcacggac actgggctgc 3840
   tcatgggagt tcatacctgt ggacgatggc tggggggagc ggccactcta a          3891
<210> 76
   <211> 3891
   <212> DNA
   <213> Artificial Sequence
<220>
   <223> BoNT/A, M. musculus-modified 1
<400> 76
   atgcctttcg tgaataagca gtttaactac aaggaccctg ttaacggtgt ggatatcgcc 60
   tacatcaaga ttccaaatgc tgggcagatg cagccagtca aagcttttaa gatacataac 120
   aagatctggg tcatccccga gagagataca ttcactaatc ccgaggaagg ggacctgaac 180
   ccccctcccg aggcaaagca ggtgcccgta agttactatg actcaacgta cctctccaca 240
   gataacgaga aggacaatta tctgaaggga gtgaccaagc tgtttgagcg aatctattcc 300
   accgatttgg gccgcatgct gctaacgagc atcgtcagag gtataccctt ctggggcgga 360
   agcacaattg atactgagct gaaggtcatt gacactaact gcataaatgt tatccaacct 420
   gatgggtcat ataggagcga agagctcaac ttggtaatca ttggcccgtc agccgacatc 480
   atacaatttg aatgcaagag ctttggtcac gaagtgttga acctcactag gaacggatat 540
   ggaagtaccc agtacatcag atttagccct gactttacct tcggctttga ggaatcactg 600
   gaagtagaca ctaacccact actgggcgcc gggaagttcg ctacagaccc agctgtcacc 660
   ttggcacacg agctgattca tgcgggacac aggctttacg gcatcgctat caacccgaac 720
   cgcgttttca aggtgaacac gaatgcttac tatgagatgt ccggcctgga ggtatcattc 780
   gaggaattga ggacattcgg cggtcatgac gctaagttta tcgatagctt acaggagaac 840
   gagttcaggt tgtattacta taacaagttc aaagatattg ccagcactct gaacaaggcc 900
   aagtccatcg tgggcaccac agcctcgctg cagtacatga agaacgtatt caaggagaag 960
   tatctactct ccgaggatac aagtgggaaa tttagtgtcg ataaactgaa attcgataag 1020
   ctttacaaaa tgctgaccga aatctacact gaagacaatt tcgtgaagtt ctttaaagtt 1080
   ctcaacagaa aaacttacct caacttcgat aaagccgtgt ttaagatcaa catcgtccca 1140
   aaagttaact ataccatcta cgacgggttt aatctccgta atactaatct ggcagccaat 1200
   tttaacggac aaaacacgga aataaataac atgaacttta ccaaactcaa gaacttcact 1260
   gggctttttg agttctataa gctcttgtgt gtgagaggga ttatcacctc taagaccaag 1320
   agcttagata agggatataa taaagcactc aatgatctgt gcatcaaagt gaacaattgg 1380
   gatctcttct tttctccatc cgaggataac tttaccaacg atttaaacaa aggagaggaa 1440
   attacctcag atacaaatat agaagccgct gaggaaaaca tttccttgga cttaatccag 1500
   caatactatc taacttttaa tttcgacaac gagcccgaga atatctccat cgagaattta 1560
   tcttccgata ttatcggcca actggagtta atgcctaata ttgagcgttt tccaaacgga 1620
   aagaaatacg agctcgataa atacactatg ttccattatt tgcgggcaca ggagttcgaa 1680
   cacggcaagt ctcggatcgc cctgacaaac tcagtgaatg aagctttact gaacccatcc 1740
   cgggtctaca cctttttctc ctctgactat gttaaaaagg tgaataaagc gacagaggct 1800
   gcaatgttcc tcggatgggt tgaacagctg gtttacgatt ttactgacga gaccagtgag 1860
   gtgtcgacta ccgacaagat cgcagacatc accataatta tcccctatat aggccctgcc 1920
   ctgaatatcg gtaatatgct ctataaggac gatttcgtcg gcgctcttat cttctctgga 1980
   gccgtgattc ttctggagtt catcccggag atcgctatcc cagtacttgg tacatttgcc 2040
   cttgtgtctt acatcgctaa caaggtgctg accgtgcaga ccattgacaa cgccctaagt 2100
   aaacgcaacg aaaagtggga cgaggtgtat aagtacattg tgactaactg gctggcaaag 2160
   gtcaacacac agattgacct gattagaaaa aagatgaaag aagccctgga aaatcaggct 2220
   gaggccacta aggccattat caattatcag tacaatcaat atactgagga agagaagaat 2280
   aacattaact ttaacataga tgacctatcc tcaaagctga atgaaagtat taataaggct 2340
   atgatcaata tcaacaaatt tctcaaccag tgctcagtgt cttatctcat gaacagtatg 2400
   attccctatg gtgtgaaacg ccttgaggac tttgatgcga gtctgaagga cgctctgttg 2460
   aaatatattt atgataatcg gggacactaa atcggacaag tggaccgcct caaagacaaa 2520
   gtgaacaata ccctcagcac agatatccca ttccagctat ccaagtacgt cgacaaccaa 2580
   cgcttgctgt ccaccttcac cgaatacatt aaaaacataa ttaacaccag catcctgaat 2640
   ctaaggtacg aatctaatca cctcattgat ctgtctcgct atgcctcaaa gatcaacatt 2700
   ggctctaaag tcaatttcga ccctatcgac aagaaccaga tccagctttt caatcttgaa 2760
   tccagcaaaa tagaggtgat attgaaaaat gcgatcgttt acaattcgat gtacgaaaac 2820
   ttttctactt ctttttggat ccgcatcccg aagtatttca atagcatcag tctgaataac 2880
   gagtatacaa tcattaactg catggaaaac aatagcggat ggaaggtgtc cctgaactat 2940
   ggcgagatca tttggaccct gcaggatacg caggaaatca agcagcgtgt ggtcttcaag 3000
   tattcacaga tgatcaacat atccgattat ataaaccggt ggattttcgt gaccattacg 3060
   aataaccgat tgaataacag caaaatttac atcaacggca gacttattga ccagaagccc 3120
   atctcaaacc tcggtaacat tcacgcctcc aataacatca tgtttaagct ggacgggtgt 3180
   cgagacacgc atcggtacat ttggattaag tacttcaatt tgtttgacaa ggagcttaac 3240
   gaaaaagaga ttaaagatct ttacgacaat caatctaatt ctggcatcct gaaagacttt 3300
   tggggcgatt acctgcagta cgacaagccc tactatatgc tcaatcttta cgatccaaac 3360
   aagtacgtgg acgtcaacaa tgttggcatt agagggtaca tgtacctgaa aggtcctcga 3420
   gggtcggtta tgacaacaaa catatacttg aattctagcc tgtacagggg aacaaaattc 3480
   atcataaaga aatacgcaag tggaaataaa gataacatag tgcggaataa cgatcgagta 3540
   tatattaacg tggtcgtaaa gaacaaagag tacaggctcg ccacaaatgc atcccaggcc 3600
   ggagtggaaa agatccttag cgcactggaa atccctgacg tcggcaatct gagccaggtc 3660
   gtagtgatga agagtaaaaa tgatcaggga ataacaaaca agtgtaagat gaacctgcaa 3720
   gacaacaatg ggaatgacat tggtttcatc gggttccacc agttcaacaa tatagcgaag 3780
   ctggtcgcaa gcaactggta caacagacag attgagagga gtagccgtac actggggtgt 3840
   agctgggaat tcatccctgt tgacgacggt tggggcgagc ggcctctgtg a          3891
<210> 77
   <211> 3891
   <212> DNA
   <213> Artificial Sequence
<220>
   <223> BoNT/A, M. musculus-modified 2
<400> 77
   atgcctttcg tgaacaagca gttcaactac aaggaccctg tgaacggcgt ggacatcgcc 60
   tacatcaaga tccctaacgc cggccagatg cagcctgtga aggccttcaa gatccacaac 120
   aagatctggg tgatccctga gagagacacc ttcaccaacc ctgaggaggg cgacctgaac 180
   cctcctcctg aggccaagca ggtgcctgtg agctactacg acagcaccta cctgagcacc 240
   gacaacgaga aggacaacta cctgaagggc gtgaccaagc tgttcgagag aatctacagc 300
   accgacctgg gcagaatgct gctgaccagc atcgtgagag gcatcccttt ctggggcggc 360
   agcaccatcg acaccgagct gaaggtgatc gacaccaact gcatcaacgt gatccagcct 420
   gacggcagct acagaagcga ggagctgaac ctggtgatca tcggccctag cgccgacatc 480
   atccagttcg agtgcaagag cttcggccac gaggtgctga acctgaccag aaacggctac 540
   ggcagcaccc agtacatcag attcagccct gacttcacct tcggcttcga ggagagcctg 600
   gaggtggaca ccaaccctct gctgggcgcc ggcaagttcg ccaccgaccc tgccgtgacc 660
   ctggcccacg agctgatcca cgccggccac agactgtacg gcatcgccat caaccctaac 720
   agagtgttca aggtgaacac caacgcctac tacgagatga gcggcctgga ggtgagcttc 780
   gaggagctga gaaccttcgg cggccacgac gccaagttca tcgacagcct gcaggagaac 840
   gagttcagac tgtactacta caacaagttc aaggacatcg ccagcaccct gaacaaggcc 900
   aagagcatcg tgggcaccac cgccagcctg cagtacatga agaacgtgtt caaggagaag 960
   tacctgctga gcgaggacac cagcggcaag ttcagcgtgg acaagctgaa gttcgacaag 1020
   ctgtacaaga tgctgaccga gatctacacc gaggacaact tcgtgaagtt cttcaaggtg 1080
   ctgaacagaa agacctacct gaacttcgac aaggccgtgt tcaagatcaa catcgtgcct 1140
   aaggtgaact acaccatcta cgacggcttc aacctgagaa acaccaacct ggccgccaac 1200
   ttcaacggcc agaacaccga gatcaacaac atgaacttca ccaagctgaa gaacttcacc 1260
   ggcctgttcg agttctacaa gctgctgtgc gtgagaggca tcatcaccag caagaccaag 1320
   agcctggaca agggctacaa caaggccctg aacgacctgt gcatcaaggt gaacaactgg 1380
   gacctgttct tcagccctag cgaggacaac ttcaccaacg acctgaacaa gggcgaggag 1440
   atcaccagcg acaccaacat cgaggccgcc gaggagaaca tcagcctgga cctgatccag 1500
   cagtactacc tgaccttcaa cttcgacaac gagcctgaga acatcagcat cgagaacctg 1560
   agcagcgaca tcatcggcca gctggagctg atgcctaaca tcgagagatt ccctaacggc 1620
   aagaagtacg agctggacaa gtacaccatg ttccactacc tgagagccca ggagttcgag 1680
   cacggcaaga gcagaatcgc cctgaccaac agcgtgaacg aggccctgct gaaccctagc 1740
   agagtgtaca ccttcttcag cagcgactac gtgaagaagg tgaacaaggc caccgaggcc 1800
   gccatgttcc tgggctgggt ggagcagctg gtgtacgact tcaccgacga gaccagcgag 1860
   gtgagcacca ccgacaagat cgccgacatc accatcatca tcccttacat cggccctgcc 1920
   ctgaacatcg gcaacatgct gtacaaggac gacttcgtgg gcgccctgat cttcagcggc 1980
   gccgtgatcc tgctggagtt catccctgag atcgccatcc ctgtgctggg caccttcgcc 2040
   ctggtgagct acatcgccaa caaggtgctg accgtgcaga ccatcgacaa cgccctgagc 2100
   aagagaaacg agaagtggga cgaggtgtac aagtacatcg tgaccaactg gctggccaag 2160
   gtgaacaccc agatcgacct gatcagaaag aagatgaagg aggccctgga gaaccaggcc 2220
   gaggccacca aggccatcat caactaccag tacaaccagt acaccgagga ggagaagaac 2280
   aacatcaact tcaacatcga cgacctgagc agcaagctga acgagagcat caacaaggcc 2340
   atgatcaaca tcaacaagtt cctgaaccag tgcagcgtga gctacctgat gaacagcatg 2400
   atcccttacg gcgtgaagag actggaggac ttcgacgcca gcctgaagga cgccctgctg 2460
   aagtacatct acgacaacag aggcaccctg atcggccagg tggacagact gaaggacaag 2520
   gtgaacaaca ccctgagcac cgacatccct ttccagctga gcaagtacgt ggacaaccag 2580
   agactgctga gcaccttcac cgagtacatc aagaacatca tcaacaccag catcctgaac 2640
   ctgagatacg agagcaacca cctgatcgac ctgagcagat acgccagcaa gatcaacatc 2700
   ggcagcaagg tgaacttcga ccctatcgac aagaaccaga tccagctgtt caacctggag 2760
   agcagcaaga tcgaggtgat cctgaagaac gccatcgtgt acaacagcat gtacgagaac 2820
   ttcagcacca gcttctggat cagaatccct aagtacttca acagcatcag cctgaacaac 2880
   gagtacacca tcatcaactg catggagaac aacagcggct ggaaggtgag cctgaactac 2940
   ggcgagatca tctggaccct gcaggacacc caggagatca agcagagagt ggtgttcaag 3000
   tacagccaga tgatcaacat cagcgactac atcaacagat ggatcttcgt gaccatcacc 3060
   aacaacagac tgaacaacag caagatctac atcaacggca gactgatcga ccagaagcct 3120
   atcagcaacc tgggcaacat ccacgccagc aacaacatca tgttcaagct ggacggctgc 3180
   agagacaccc acagatacat ctggatcaag tacttcaacc tgttcgacaa ggagctgaac 3240
   gagaaggaga tcaaggacct gtacgacaac cagagcaaca gcggcatcct gaaggacttc 3300
   tggggcgact acctgcagta cgacaagcct tactacatgc tgaacctgta cgaccctaac 3360
   aagtacgtgg acgtgaacaa cgtgggcatc agaggctaca tgtacctgaa gggccctaga 3420
   ggcagcgtga tgaccaccaa catctacctg aacagcagcc tgtacagagg caccaagttc 3480
   atcatcaaga agtacgccag cggcaacaag gacaacatcg tgagaaacaa cgacagagtg 3540
   tacatcaacg tggtggtgaa gaacaaggag tacagactgg ccaccaacgc cagccaggcc 3600
   ggcgtggaga agatcctgag cgccctggag atccctgacg tgggcaacct gagccaggtg 3660
   gtggtgatga agagcaagaa cgaccagggc atcaccaaca agtgcaagat gaacctgcag 3720
   gacaacaacg gcaacgacat cggcttcatc ggcttccacc agttcaacaa catcgccaag 3780
   ctggtggcca gcaactggta caacagacag atcgagagaa gcagcagaac cctgggctgc 3840
   agctgggagt tcatccctgt ggacgacggc tggggcgaga gacctctgtg a 3891
<210> 78
   <211> 3891
   <212> DNA
   <213> Artificial Sequence
<220>
   <223> BoNT/A, M. musculus-modified 3
<400> 78
   atgccattcg tcaataagca gttcaattac aaagaccccg tgaatggagt ggatatcgcc 60
   tatattaaaa tccctaacgc tgggcagatg cagccagtca aggcatttaa aattcataac 120
   aaaatctggg tcatcccaga gcgagacacc ttcaccaatc ccgaggaagg agacctcaat 180
   cctcccccag aagcaaaaca ggtgcctgtt tcatactatg acagcactta cctgagcacc 240
   gacaatgaaa aagacaacta cttgaaaggc gttaccaagc ttttcgagcg tatctacagc 300
   accgatcttg ggaggatgct gctcacatct atcgttcgcg gtatcccctt ctggggcggt 360
   agcactattg ataccgaatt gaaggtcatc gatactaact gcatcaacgt gattcaaccc 420
   gacggaagct atcgcagcga agagctgaat ctggttatca ttggcccctc tgccgacatt 480
   atccaatttg aatgtaaatc ttttggtcac gaggtcctta atttgactcg caacggttac 540
   gggagtaccc agtatattcg cttcagccca gatttcacat ttggcttcga agagtcactg 600
   gaggtcgata caaaccctct tctcggggca gggaagttcg ccaccgaccc agctgtgaca 660
   ctggcccacg aactgatcca tgccggccac cggctgtatg gtatcgctat caatcctaac 720
   cgagtgttca aggtcaacac aaacgcttat tacgagatgt ctggactcga ggtgtccttt 780
   gaagagctga ggaccttcgg gggccacgac gctaagttta ttgactccct gcaggaaaat 840
   gagttccgcc tttattacta taacaagttt aaagatatcg catctactct gaataaggct 900
   aaaagcatcg tgggtacaac cgccagtctc cagtacatga aaaacgtgtt caaggagaag 960
   taccttctgt ccgaagatac tagtggtaag ttttctgtcg acaagttgaa gttcgacaag 1020
   ctttacaaga tgctgaccga gatctacacc gaggataatt ttgtgaaatt tttcaaagtg 1080
   ctgaacagga agacctacct caatttcgac aaggccgtgt ttaagattaa catcgtgcct 1140
   aaagtgaact acaccattta cgatgggttt aatttgcgca acaccaacct ggccgcaaac 1200
   ttcaacgggc agaacaccga gattaataac atgaatttta caaagctgaa aaactttacc 1260
   ggcctcttcg agttttacaa gttgctgtgc gtccggggca tcattacaag caagactaag 1320
   tcactggata agggatataa caaagccctg aatgacctct gtattaaggt gaacaattgg 1380
   gatctgtttt tcagccctag tgaagacaac tttaccaacg atctgaacaa gggagaggaa 1440
   atcacaagcg ataccaacat tgaagccgct gaagagaaca tcagcctgga cctgatccag 1500
   caatactatc tgacatttaa tttcgacaac gaacccgaaa atattagcat cgagaacttg 1560
   agcagtgaca tcattggcca actggagctc atgccaaaca tcgagcggtt ccccaacggt 1620
   aagaaatatg agctggataa atacaccatg tttcattacc tgagggcaca ggaatttgaa 1680
   catggcaaaa gtagaatcgc cctgaccaat tccgtcaacg aggccctgtt gaacccatct 1740
   agagtgtaca cttttttctc ctctgactat gtgaaaaagg tgaacaaggc tacagaggcc 1800
   gcaatgttcc tcgggtgggt ggagcagttg gtctacgatt tcactgatga gacttccgaa 1860
   gtcagcacca cagacaagat tgcagacatc acaatcatta tcccttacat cggcccagcc 1920
   cttaacatcg gaaatatgct ttataaggac gattttgtgg gggccctgat cttctccggc 1980
   gctgttattc tgcttgagtt catccctgag atcgccatcc ctgtgctggg aacttttgct 2040
   ctggtgtcat atatcgctaa caaggtgctg actgttcaga ctatcgacaa cgccctctca 2100
   aagcggaatg agaaatggga cgaagtttat aaatacatcg tgactaattg gctcgcaaag 2160
   gtgaacaccc aaatcgatct gattagaaag aaaatgaaag aggccctcga gaatcaggct 2220
   gaggccacca aagccattat caattaccaa tacaatcagt acaccgagga agagaaaaat 2280
   aacatcaact tcaacatcga tgacctgtcc agcaagctta acgaatctat caataaagct 2340
   atgattaata ttaacaagtt cctgaaccag tgttcagtgt cctacctgat gaactccatg 2400
   atcccctatg gcgtcaagcg gctggaggat tttgatgcct ccctcaaaga cgcactgctc 2460
   aaatacatct acgataaccg cggcacactg attggacaag tggatcgact caaggacaag 2520
   gttaataaca ctctgtccac cgatatccca tttcagctgt ctaagtatgt ggacaaccag 2580
   aggctgttgt ctacctttac tgaatatatc aagaacatta tcaatacatc aatcctgaat 2640
   cttcggtatg agagcaatca cttgatcgac ctctcacgct acgcttctaa gatcaacatc 2700
   ggcagcaaag tgaattttga ccctatcgac aagaaccaaa ttcaactctt caatctggaa 2760
   tcctcaaaga tcgaagtgat ccttaagaat gccatcgtgt ataattccat gtacgaaaac 2820
   ttcagtacca gtttctggat caggattcct aagtacttca acagcatctc tctgaataac 2880
   gagtacacaa tcattaactg tatggagaat aacagtggat ggaaagtcag cctgaactat 2940
   ggcgaaatta tctggacctt gcaggataca caggagatca aacagagggt ggttttcaag 3000
   tactctcaga tgatcaatat ttccgattac atcaacagat ggatctttgt tacaattaca 3060
   aacaatagac tgaacaatag taagatttac attaatggac gactgatcga tcagaagcct 3120
   atctcaaacc tgggcaatat ccacgccagt aataacatca tgtttaagct cgacggttgc 3180
   agggataccc acagatatat ttggatcaag tacttcaact tgttcgacaa agagctgaac 3240
   gagaaggaga tcaaggatct ttatgataat cagagcaatt cagggattct caaggacttc 3300
   tggggagact acctgcagta tgataagcca tattacatgc tgaacctgta tgaccccaat 3360
   aagtatgtcg acgtgaacaa tgtcggaatc cgtggttata tgtatctgaa aggacctcgg 3420
   gggtctgtga tgacaaccaa tatctatctg aattcatcct tgtacagagg gactaaattt 3480
   attatcaaaa agtacgcctc tggcaacaag gacaatatcg tgcgtaataa cgatagagtg 3540
   tatatcaatg ttgtggttaa gaacaaagag tacaggcttg ccacaaatgc ttcccaggcc 3600
   ggcgtggaaa aaatcttgag cgcactggaa attcctgacg tgggcaacct gtcccaggtg 3660
   gtcgtgatga agtccaagaa tgatcaaggg attactaaca agtgcaaaat gaacctgcag 3720
   gacaacaatg gaaacgatat cggattcatt ggattccatc agttcaacaa tattgctaaa 3780
   ctggtggcaa gcaactggta taaccgtcag atcgaaagaa gtagccggac cctcggttgc 3840
   agttgggagt ttatccctgt cgacgacggg tggggcgagc gacccctctg a          3891
<210> 79
   <211> 3891
   <212> DNA
   <213> Artificial Sequence
<220>
   <223> BoNT/A, R. norvegicus-modified 1
<400> 79
   atgcctttcg tgaataaaca gttcaactac aaggaccctg ttaacggcgt ggacattgcg 60
   tacattaaaa ttccaaacgc aggacaaatg cagccagtca aggctttcaa aatccacaac 120
   aagatctggg tgataccaga acgggacacg ttcaccaacc ccgaggaagg tgaccttaat 180
   ccacccccag aggccaaaca ggttcctgta tcatattacg actctactta cctctctaca 240
   gacaacgaaa aagacaatta cctaaaaggc gtgaccaagc tgttcgagcg aatatactct 300
   actgatttag ggcggatgct cctgacaagc atagtccggg gaataccctt ctgggggggc 360
   agtactatcg acactgagtt gaaagtcatc gacactaact gcatcaacgt cattcagccc 420
   gacggcagct atagaagcga ggaactcaac ctggtaatta tcggccctag tgccgacata 480
   atccaatttg agtgcaagag ctttgggcac gaggttttga acctgacacg gaacggctat 540
   ggctcgaccc agtatatccg gttcagcccc gatttcacgt tcggatttga ggaatctctt 600
   gaagtggata caaaccctct actgggcgca ggaaagttcg ctacggaccc cgcagttaca 660
   ctagctcacg agctcatcca tgcaggtcac agactgtacg gcatcgccat taaccccaat 720
   agggtcttca aagtcaacac caacgcctac tatgaaatga gcggattgga agtgagtttc 780
   gaagagttac gtacatttgg cgggcatgac gctaagttta tcgattccct ccaggagaac 840
   gagtttcgcc tgtactatta caacaagttc aaggacatag cctcaaccct caacaaggcg 900
   aaatccatcg tggggaccac agccagtctg caatatatga agaacgtttt caaagagaag 960
   taccttctat ccgaggacac gtcaggcaag ttttccgtcg acaagctgaa gttcgacaaa 1020
   ttgtataaga tgttgactga gatctacaca gaagacaact tcgtgaaatt ctttaaggtg 1080
   ctgaaccgca aaacatactt gaacttcgac aaggctgtct ttaagattaa cattgtgccg 1140
   aaggtaaatt ataccatata cgatgggttc aacctgagga acaccaacct cgctgccaac 1200
   ttcaacggtc agaatactga gatcaacaat atgaacttca ccaagctcaa gaattttaca 1260
   ggcctgtttg agttttacaa gcttctgtgc gtccgtggaa tcattacctc taaaacgaag 1320
   tctttggaca agggttataa caaggctctg aatgacctgt gcatcaaagt gaataactgg 1380
   gaccttttct tttctccatc ggaagacaac ttcaccaatg acttaaataa gggggaggaa 1440
   attactagcg acacgaacat cgaggccgca gaggaaaata tatcactgga ccttatccag 1500
   caatactatt tgacctttaa tttcgacaac gagcctgaga atataagtat cgagaatctt 1560
   tcctcagata ttatcggaca gctcgaactg atgcccaata tcgagcgctt ccctaatggt 1620
   aaaaagtacg agctcgataa gtacacaatg ttccactatt tgcgcgccca ggagtttgag 1680
   cacgggaaga gcaggatcgc gcttacaaat agcgtgaacg aggcactcct gaatcctagt 1740
   cgagtgtaca cgttcttttc tagcgattat gtcaagaaag tgaacaaagc caccgaggcc 1800
   gctatgtttt taggatgggt ggaacagctg gtatacgatt ttaccgacga gacctctgaa 1860
   gtcagcacta cagataagat tgccgatatc accatcatta tcccatatat cggaccggct 1920
   ctgaatatcg ggaacatgct gtataaggac gattttgtgg gcgcactgat tttctctggc 1980
   gctgtgatcc ttctcgagtt tatccccgag atcgcaatcc cggtgctggg tacttttgcg 2040
   cttgtgtcct atatcgcaaa caaggtcctt accgtgcaga caattgataa cgccctgtct 2100
   aaacgcaacg agaaatggga cgaggtttac aaatacatcg tcaccaactg gcttgctaag 2160
   gtcaacaccc agatcgatct gatccgcaag aaaatgaaag aagccctgga aaatcaggcc 2220
   gaggccacca aggctatcat aaattaccag tataaccagt acactgaaga ggaaaaaaac 2280
   aatattaact tcaacatcga cgatttgtcc agcaagctga acgagagtat caataaggcc 2340
   atgatcaata tcaataagtt cctgaatcaa tgttcagtgt cctacctcat gaactccatg 2400
   atcccatacg gggtgaagcg tctggaagac tttgacgcgt ccctgaagga cgcactcctg 2460
   aagtatattt atgataaccg aggcaccttg attggacagg ttgatagact gaaggacaaa 2520
   gtgaacaata ctttatctac tgatatccct ttccagttga gcaaatacgt ggataaccag 2580
   agattactga gcaccttcac tgaatacatt aaaaatatta tcaacaccag tatccttaac 2640
   ctgaggtacg agtctaatca cctcatcgat ctgtccagat acgccagcaa gattaacatt 2700
   ggatccaaag tgaacttcga ccctatcgac aaaaaccaaa tccaactgtt taacttggag 2760
   agctccaaga tcgaggtgat cctcaagaat gccatagtct ataattcgat gtacgaaaac 2820
   ttttccactt cattttggat aaggatcccc aagtatttca actccatttc cctcaacaat 2880
   gagtacacta tcattaactg catggaaaat aactctggtt ggaaagtctc tctcaattac 2940
   ggagagatca tttggaccct gcaagatact caggaaatta agcagcgcgt ggtctttaag 3000
   tactcccaga tgatcaacat tagtgattat attaaccgat ggattttcgt cacaattacc 3060
   aataaccgac tgaacaatag caaaatttac attaatggga ggctaatcga tcagaagccc 3120
   atttcaaatc tgggtaacat ccacgcctcc aacaatatca tgtttaagct ggacggctgt 3180
   cgtgacaccc atagatatat atggatcaaa tacttcaatc tgtttgacaa ggaactaaat 3240
   gaaaaagaga ttaaagatct gtacgataat cagtcaaact ccggtatcct caaggatttc 3300
   tggggcgatt acctgcagta cgacaagccc tactatatgc tgaatctcta cgatcctaat 3360
   aagtatgtgg atgtgaacaa tgtcggtatc cggggctaca tgtacctgaa aggccctcgc 3420
   ggcagcgtca tgaccacaaa tatctacctg aattcctctc tctatagggg gacaaagttc 3480
   atcattaaga aatatgcttc ggggaacaag gacaacattg ttaggaataa cgatagagtg 3540
   tacatcaacg ttgtagtgaa gaacaaagag tatcggttgg caacgaacgc cagccaggct 3600
   ggcgtggaga agatcctctc agctctggag ataccagatg ttggtaatct atctcaggtg 3660
   gtagtgatga agagtaaaaa cgaccagggc attaccaaca agtgtaagat gaacctccag 3720
   gacaataacg gcaacgatat cggattcatc ggattccatc aattcaataa catcgctaaa 3780
   ctggttgcca gcaattggta taacaggcag attgagcgct cctcgagaac actcgggtgt 3840
   agttgggaat ttatcccggt agacgatggt tggggagaac ggccactatg a          3891
<210> 80
   <211> 3891
   <212> DNA
   <213> Artificial Sequence
<220>
   <223> BoNT/A, R. norvegicus-modified 2
<400> 80
   atgcctttcg tgaacaagca gttcaactac aaggaccctg tgaacggcgt ggacatcgcc 60
   tacatcaaga tccctaacgc cggccagatg cagcctgtga aggccttcaa gatccacaac 120
   aagatctggg tgatccctga gagggacacc ttcaccaacc ctgaggaggg cgacctgaac 180
   cctcctcctg aggccaagca ggtgcctgtg tcctactacg actccaccta cctgtccacc 240
   gacaacgaga aggacaacta cctgaagggc gtgaccaagc tgttcgagag gatctactcc 300
   accgacctgg gcaggatgct gctgacctcc atcgtgaggg gcatcccttt ctggggcggc 360
   tccaccatcg acaccgagct gaaggtgatc gacaccaact gcatcaacgt gatccagcct 420
   gacggctcct acaggtccga ggagctgaac ctggtgatca tcggcccttc cgccgacatc 480
   atccagttcg agtgcaagtc cttcggccac gaggtgctga acctgaccag gaacggctac 540
   ggctccaccc agtacatcag gttctcccct gacttcacct tcggcttcga ggagtccctg 600
   gaggtggaca ccaaccctct gctgggcgcc ggcaagttcg ccaccgaccc tgccgtgacc 660
   ctggcccacg agctgatcca cgccggccac aggctgtacg gcatcgccat caaccctaac 720
   agggtgttca aggtgaacac caacgcctac tacgagatgt ccggcctgga ggtgtccttc 780
   gaggagctga ggaccttcgg cggccacgac gccaagttca tcgactccct gcaggagaac 840
   gagttcaggc tgtactacta caacaagttc aaggacatcg cctccaccct gaacaaggcc 900
   aagtccatcg tgggcaccac cgcctccctg cagtacatga agaacgtgtt caaggagaag 960
   tacctgctgt ccgaggacac ctccggcaag ttctccgtgg acaagctgaa gttcgacaag 1020
   ctgtacaaga tgctgaccga gatctacacc gaggacaact tcgtgaagtt cttcaaggtg 1080
   ctgaacagga agacctacct gaacttcgac aaggccgtgt tcaagatcaa catcgtgcct 1140
   aaggtgaact acaccatcta cgacggcttc aacctgagga acaccaacct ggccgccaac 1200
   ttcaacggcc agaacaccga gatcaacaac atgaacttca ccaagctgaa gaacttcacc 1260
   ggcctgttcg agttctacaa gctgctgtgc gtgaggggca tcatcacctc caagaccaag 1320
   tccctggaca agggctacaa caaggccctg aacgacctgt gcatcaaggt gaacaactgg 1380
   gacctgttct tctccccttc cgaggacaac ttcaccaacg acctgaacaa gggcgaggag 1440
   atcacctccg acaccaacat cgaggccgcc gaggagaaca tctccctgga cctgatccag 1500
   cagtactacc tgaccttcaa cttcgacaac gagcctgaga acatctccat cgagaacctg 1560
   tcctccgaca tcatcggcca gctggagctg atgcctaaca tcgagaggtt ccctaacggc 1620
   aagaagtacg agctggacaa gtacaccatg ttccactacc tgagggccca ggagttcgag 1680
   cacggcaagt ccaggatcgc cctgaccaac tccgtgaacg aggccctgct gaacccttcc 1740
   agggtgtaca ccttcttctc ctccgactac gtgaagaagg tgaacaaggc caccgaggcc 1800
   gccatgttcc tgggctgggt ggagcagctg gtgtacgact tcaccgacga gacctccgag 1860
   gtgtccacca ccgacaagat cgccgacatc accatcatca tcccttacat cggccctgcc 1920
   ctgaacatcg gcaacatgct gtacaaggac gacttcgtgg gcgccctgat cttctccggc 1980
   gccgtgatcc tgctggagtt catccctgag atcgccatcc ctgtgctggg caccttcgcc 2040
   ctggtgtcct acatcgccaa caaggtgctg accgtgcaga ccatcgacaa cgccctgtcc 2100
   aagaggaacg agaagtggga cgaggtgtac aagtacatcg tgaccaactg gctggccaag 2160
   gtgaacaccc agatcgacct gatcaggaag aagatgaagg aggccctgga gaaccaggcc 2220
   gaggccacca aggccatcat caactaccag tacaaccagt acaccgagga ggagaagaac 2280
   aacatcaact tcaacatcga cgacctgtcc tccaagctga acgagtccat caacaaggcc 2340
   atgatcaaca tcaacaagtt cctgaaccag tgctccgtgt cctacctgat gaactccatg 2400
   atcccttacg gcgtgaagag gctggaggac ttcgacgcct ccctgaagga cgccctgctg 2460
   aagtacatct acgacaacag gggcaccctg atcggccagg tggacaggct gaaggacaag 2520
   gtgaacaaca ccctgtccac cgacatccct ttccagctgt ccaagtacgt ggacaaccag 2580
   aggctgctgt ccaccttcac cgagtacatc aagaacatca tcaacacctc catcctgaac 2640
   ctgaggtacg agtccaacca cctgatcgac ctgtccaggt acgcctccaa gatcaacatc 2700
   ggctccaagg tgaacttcga ccctatcgac aagaaccaga tccagctgtt caacctggag 2760
   tcctccaaga tcgaggtgat cctgaagaac gccatcgtgt acaactccat gtacgagaac 2820
   ttctccacct ccttctggat caggatccct aagtacttca actccatctc cctgaacaac 2880
   gagtacacca tcatcaactg catggagaac aactccggct ggaaggtgtc cctgaactac 2940
   ggcgagatca tctggaccct gcaggacacc caggagatca agcagagggt ggtgttcaag 3000
   tactcccaga tgatcaacat ctccgactac atcaacaggt ggatcttcgt gaccatcacc 3060
   aacaacaggc tgaacaactc caagatctac atcaacggca ggctgatcga ccagaagcct 3120
   atctccaacc tgggcaacat ccacgcctcc aacaacatca tgttcaagct ggacggctgc 3180
   agggacaccc acaggtacat ctggatcaag tacttcaacc tgttcgacaa ggagctgaac 3240
   gagaaggaga tcaaggacct gtacgacaac cagtccaact ccggcatcct gaaggacttc 3300
   tggggcgact acctgcagta cgacaagcct tactacatgc tgaacctgta cgaccctaac 3360
   aagtacgtgg acgtgaacaa cgtgggcatc aggggctaca tgtacctgaa gggccctagg 3420
   ggctccgtga tgaccaccaa catctacctg aactcctccc tgtacagggg caccaagttc 3480
   atcatcaaga agtacgcctc cggcaacaag gacaacatcg tgaggaacaa cgacagggtg 3540
   tacatcaacg tggtggtgaa gaacaaggag tacaggctgg ccaccaacgc ctcccaggcc 3600
   ggcgtggaga agatcctgtc cgccctggag atccctgacg tgggcaacct gtcccaggtg 3660
   gtggtgatga agtccaagaa cgaccagggc atcaccaaca agtgcaagat gaacctgcag 3720
   gacaacaacg gcaacgacat cggcttcatc ggcttccacc agttcaacaa catcgccaag 3780
   ctggtggcct ccaactggta caacaggcag atcgagaggt cctccaggac cctgggctgc 3840
   tcctgggagt tcatccctgt ggacgacggc tggggcgaga ggcctctgtg a 3891
<210> 81
   <211> 3891
   <212> DNA
   <213> Artificial Sequence
<220>
   <223> BoNT/A, R. norvegicus-modified 3
<400> 81
   atgcctttcg tgaataaaca gtttaactac aaggatcccg tgaacggagt ggatatcgcc 60
   tacatcaaga tccctaacgc cggccagatg cagcctgtga aggctttcaa aatccataat 120
   aagatctggg tgatccctga gagagacaca tttaccaatc cagaagaggg tgacctgaac 180
   ccaccccctg aagctaaaca ggtcccagtt tcttattacg actctaccta tctctctacc 240
   gataacgaga aagacaatta tctcaagggg gtcacaaaac tgttcgaaag aatctatagt 300
   accgatctcg gacgcatgtt gctgacatca atcgtgaggg gaattccttt ctggggcggt 360
   tctactattg atactgagct caaggttatt gacaccaact gtattaacgt gatccagcca 420
   gatggctcct accgctcaga ggaactgaac ttggtcatca ttggtccctc tgccgatatt 480
   atccagttcg aatgcaaatc cttcggccac gaggtgctga acctgacacg gaacggttat 540
   gggagtaccc agtacatccg gttctcaccc gacttcacct tcggctttga agagtccctg 600
   gaggttgaca ccaacccact tctgggtgcc gggaagtttg caaccgatcc cgccgtcacc 660
   ctggctcacg agctgattca cgctggtcac cgactgtacg ggatcgccat taaccccaac 720
   agggtgttca aggtgaacac taacgcttac tatgagatga gcggcctgga ggtctccttt 780
   gaggaactgc gcaccttcgg cgggcacgac gcaaagttta tcgatagtct gcaggagaac 840
   gagtttaggt tgtactatta caacaaattc aaagacatcg caagcactct gaataaggcc 900
   aagtctattg ttggtaccac agctagcctg cagtacatga aaaacgtgtt taaggagaaa 960
   taccttctgt ctgaggacac aagcggaaag ttcagcgttg acaaactgaa attcgataag 1020
   ttgtacaaga tgttgaccga gatctataca gaggataact tcgtgaaatt ctttaaggtg 1080
   ctgaacagaa agacatattt gaacttcgac aaagccgtct tcaagatcaa tattgtgcct 1140
   aaggtgaatt acacaatcta cgacggcttc aacctgcgca acaccaacct tgccgcaaac 1200
   ttcaatgggc agaacaccga gatcaataac atgaatttta ccaaactcaa gaactttact 1260
   ggactgttcg agttctataa actgctctgt gtgaggggaa tcattacaag taaaactaag 1320
   agtctggaca aaggttacaa caaggcactg aatgatcttt gtatcaaggt caataactgg 1380
   gacctgttct ttcccccctc tgaagataac ttcaccaacg acctgaacaa aggagaggaa 1440
   atcaccagtg acactaatat cgaggccgct gaggaaaata tctcactgga cctgatccag 1500
   cagtattacc tgaccttcaa ttttgataac gagcctgaga acatcagcat cgagaatctc 1560
   tccagcgaca tcattggcca gctggaattg atgcctaaca tcgagcgctt ccccaatggc 1620
   aagaaatatg aactggataa gtacactatg tttcactacc ttagggccca ggagtttgaa 1680
   cacggcaaat ctcgaattgc actgaccaat tctgttaatg aggccctgct taacccctcc 1740
   cgagtgtaca cctttttcag ctccgactat gtgaagaaag tcaataaagc cacagaggcc 1800
   gcaatgttcc tgggctgggt cgaacagctg gtgtacgact ttaccgatga aactagcgag 1860
   gtctccacaa ccgataagat cgccgacatc accatcatta tcccttacat tggacccgcc 1920
   ttgaacattg gtaacatgct ctacaaggac gatttcgtcg gcgccttgat tttagcgggg 1980
   gccgtgatcc tcctggagtt tatccctgaa attgctatcc ccgtgttggg caccttcgct 2040
   cttgtctcat acatcgccaa taaagtgctg accgtgcaga ccatcgataa cgctctgtcc 2100
   aagaggaatg aaaaatggga cgaggtgtac aagtacatcg tcaccaactg gctcgcaaag 2160
   gtgaacaccc agattgatct gatcaggaaa aagatgaagg aggccctgga aaaccaggca 2220
   gaggccacaa aagctatcat taactatcag tataaccagt atactgaaga ggaaaaaaac 2280
   aatattaact tcaacatcga cgatctgtcc agtaagctga acgaatccat caataaggcc 2340
   atgatcaaca ttaataagtt tctgaatcag tgctcagtgt cctatctcat gaattccatg 2400
   atcccatacg gagtgaagag actggaagac tttgacgcca gcctgaagga cgctctcctg 2460
   aagtacatct acgataacag aggcactctg atcgggcagg tggatcgtct gaaggacaag 2520
   gtgaacaata ctctgagcac agacattcca ttccagctct ccaaatacgt ggataatcag 2580
   cgacttctga gcaccttcac cgagtacatc aagaacatca ttaacacttc catcctcaac 2640
   ctgcgatacg agagcaacca tttgatcgat ctgagccgtt acgcctcaaa aattaatatc 2700
   ggttccaagg tgaatttcga cccaattgac aagaatcaga tccagctctt taacctggag 2760
   agctctaaga tcgaggtcat tctcaagaac gctatcgtgt ataacagcat gtatgaaaat 2820
   tttagcactt ccttctggat ccggatcccc aagtatttca attccatcag tctcaacaat 2880
   gagtacacca tcattaattg catggaaaac aattccggat ggaaggtttc actgaactac 2940
   ggagagatta tctggacact ccaggacact caggaaatta agcagcgggt ggtttttaag 3000
   tactctcaga tgattaacat ctctgactac atcaaccggt ggatttttgt gacaatcacc 3060
   aataacagac tcaacaattc caaaatctat atcaacggtc gccttatcga ccagaagcct 3120
   atctctaact tgggcaatat ccatgcctcc aataacatta tgttcaaact tgatggatgc 3180
   cgcgacaccc accggtatat ttggattaag tacttcaacc tgtttgacaa agagctgaac 3240
   gagaaggaga tcaaggacct ctacgataat cagtcaaact ctgggatctt gaaagacttt 3300
   tggggcgact atcttcagta cgataagcct tattacatgc tcaatcttta cgatccaaac 3360
   aaatatgtgg acgtcaacaa tgtgggcatt agagggtata tgtatctgaa gggcccacgt 3420
   ggcagcgtta tgacaactaa tatttacctg aattccagtc tgtacagagg gaccaagttc 3480
   atcattaaga aatatgcatc cggaaacaag gacaatatcg tgaggaacaa tgatagggtc 3540
   tatatcaacg tcgtggtcaa gaacaaagaa taccgcctgg ccactaatgc ttctcaggca 3600
   ggagtggaaa agatcctctc tgctctggag atccctgacg ttggtaatct cagccaggtg 3660
   gttgtcatga aatccaagaa tgatcagggg atcacaaaca aatgcaagat gaatttgcag 3720
   gataataacg gcaacgacat cgggtttatc ggatttcatc agttcaacaa tatcgctaag 3780
   cttgtcgcct ccaactggta caaccggcag atcgagcgca gttcacggac cctcggctgt 3840
   agttgggaat tcatccctgt ggatgacggg tggggcgaac gtcctctgtg a          3891
<210> 82
   <211> 3891
   <212> DNA
   <213> Artificial Sequence
<220>
   <223> BoNT/A, C. griseus-modified 1
<400> 82
   atgccattcg tgaacaagca gttcaattat aaagatcccg tgaacggggt ggacattgcc 60
   tatattaaaa tcccaaacgc agggcagatg cagcccgtca aagccttcaa aatccataac 120
   aagatctggg tgattcccga gagagatacg ttcaccaacc ctgaggaagg agatctgaat 180
   ccgccccctg aagccaagca agtcccagtt tcctactatg atagcaccta tctgtctacc 240
   gacaacgaga aggataatta tctaaagggc gtgactaaac tttttgagcg tatctactct 300
   accgatcttg gtcgaatgct gctcacttca attgttaggg gtataccctt ctggggtgga 360
   agcactatcg acaccgagtt gaaggtgatc gacacgaact gcattaatgt aatccagcct 420
   gatggatcct acaggagtga ggaactcaac ctggtgatta tcggcccttc tgccgacatc 480
   attcagtttg aatgcaagag ttttggtcac gaggtcctca atctaacccg gaatgggtat 540
   ggcagcaccc agtacattag attcagccct gacttcactt tcggatttga agagagtcta 600
   gaggtcgaca ccaacccact gttgggggcc ggcaagttcg ccacagaccc tgctgtcaca 660
   cttgcgcatg aactgattca cgccggacac cgtctgtacg gcattgctat caatcccaat 720
   cgtgtattca aggtgaacac caatgcatac tatgaaatga gcgggcttga ggtgtccttt 780
   gaggaactta gaacatttgg tgggcacgat gcaaaattta tagactcttt gcaagaaaac 840
   gagttcaggc tgtactatta caataagttc aaggacatcg cttcaactct gaataaggct 900
   aagtctattg tcggtacaac tgcaagcctg cagtacatga agaatgtgtt caaagagaaa 960
   tacctcttgt ctgaagacac ttctggaaaa ttctctgtgg acaagctgaa gttcgacaaa 1020
   ctgtacaaaa tgttgacaga gatatataca gaagacaatt ttgtgaaatt tttcaaggtc 1080
   ttgaatagaa agacctacct caatttcgac aaggctgtct ttaagattaa catagtgcca 1140
   aaagtcaact acactatcta cgatggattt aatttacgca acaccaatct agcagccaac 1200
   ttcaacggac agaataccga aatcaataac atgaatttta ccaagctgaa gaacttcacg 1260
   ggcttatttg agttttacaa actgctctgc gtccggggca tcattacgag taagactaag 1320
   agcctggata agggctacaa caaggctctg aatgacttgt gtatcaaagt aaataactgg 1380
   gatctcttct ttagtccaag tgaggataac tttacaaatg acttgaataa aggagaggaa 1440
   atcacctctg atacaaacat tgaggccgct gaggaaaata tcagtttaga tttgattcag 1500
   caatattacc ttaccttcaa cttcgacaac gagcccgaga acatctcaat cgaaaacttg 1560
   tcgagcgaca ttatcggaca actcgagctg atgccaaaca ttgaacgttt tcctaatggc 1620
   aaaaagtatg aactcgataa atacaccatg tttcattacc tgcgagctca ggagttcgaa 1680
   catggcaagt cacgcattgc cctgacaaac tctgtcaacg aagcactatt gaacccctca 1740
   cgcgtgtaca ccttcttttc tagcgattat gttaagaaag tgaacaaagc tactgaggcc 1800
   gcaatgtttc tgggatgggt tgagcagctc gtatatgatt tcactgacga gacctctgag 1860
   gtgtcaacaa ccgataagat cgccgatatc actattatca ttccatacat aggccccgcg 1920
   ctaaatatag gcaacatgtt gtacaaagac gatttcgtcg gggcgctaat cttcagcgga 1980
   gcagtgatcc tgttggaatt tatccctgaa attgcaatcc cggttcttgg caccttcgcc 2040
   ttggtgtcct atatcgctaa taaagttctg accgtacaga ccatcgacaa tgctctgtct 2100
   aaacgcaacg agaagtggga cgaagtgtat aaatacatcg tgaccaactg gctggccaag 2160
   gtaaatactc agattgatct gatcaggaag aaaatgaaag aggccttaga gaaccaggct 2220
   gaagccacta aggctatcat aaattaccag tacaatcagt acacagaaga ggaaaagaat 2280
   aacataaatt ttaacattga tgacttgagt tccaagctta acgagagcat caacaaagcg 2340
   atgatcaaca tcaacaaatt cctcaatcag tgtagcgtgt cttacttaat gaattccatg 2400
   attccttacg gcgtcaagag actggaggac tttgatgctt cactgaagga tgcactactg 2460
   aaatacatat atgataaccg agggacatta atcgggcagg ttgacaggct gaaggacaag 2520
   gtgaataaca ctctctcaac ggacatccct tttcaactaa gcaagtatgt tgacaaccag 2580
   agactgctca gtacatttac agagtatatc aagaacatca ttaacaccag tattctgaac 2640
   ctgcggtacg agtccaatca cctcatcgac ctgtccaggt acgcttccaa aattaacatt 2700
   ggttccaagg ttaatttcga tccaattgac aagaaccaga tacaactttt taaccttgag 2760
   tcgagcaaaa ttgaggttat ccttaaaaac gctatcgttt ataattcaat gtacgaaaat 2820
   tttagtacaa gcttctggat ccgaatacca aagtatttta actccatttc tctcaacaat 2880
   gagtacacca taatcaattg catggaaaac aattcaggat ggaaagtatc tctcaattat 2940
   ggtgaaataa tctggacact gcaggatact caggagatca aacaacgtgt ggttttcaag 3000
   tattcccaga tgatcaacat ctcagattat atcaaccgct ggatctttgt gacgatcacc 3060
   aacaatagac tgaataacag caagatttat attaatggtc ggttaatcga ccagaagccc 3120
   atttcgaatc ttggcaatat ccacgcctcc aataacatca tgttcaagct cgacggatgc 3180
   cgcgacacac atagatatat ttggatcaag tacttcaacc ttttcgataa ggagctgaac 3240
   gaaaaggaaa tcaaggatct ttatgacaat cagtccaact caggcatcct gaaggatttt 3300
   tggggcgatt acttgcagta tgataagcca tactatatgc tgaacctgta cgatcccaat 3360
   aaatatgttg atgtaaacaa tgtgggcatt cgcggatata tgtacctgaa gggtccccgc 3420
   ggttccgtga tgactacaaa catatatctg aacagctccc tctaccgggg gactaagttt 3480
   attatcaaga aatacgcttc cggcaacaaa gacaacattg taaggaacaa tgaccgagtg 3540
   tacattaatg tcgtggtcaa aaataaggaa tatcggctcg caacaaatgc cagccaggca 3600
   ggcgtcgaga agatcctgag tgccctcgag atacctgacg tcggtaacct ctcccaggtg 3660
   gtggtgatga aatccaagaa cgaccagggt atcaccaaca agtgtaaaat gaatcttcaa 3720
   gacaataacg ggaatgacat tgggtttatt gggttccacc agtttaataa catagccaag 3780
   ctggtggcat ctaactggta taaccggcaa atcgagcgat cgtctcggac actgggctgt 3840
   agttgggagt tcattccggt ggatgatggc tggggagaga ggcctctgtg a          3891
<210> 83
   <211> 3891
   <212> DNA
   <213> Artificial Sequence
<220>
   <223> BoNT/A, C. griseus-modified 2
<400> 83
   atgcctttcg tgaacaagca gttcaactac aaggaccctg tgaacggcgt ggacatcgcc 60
   tacatcaaga tccctaacgc cggccagatg cagcctgtga aggccttcaa gatccacaac 120
   aagatctggg tgatccctga gagagacacc ttcaccaacc ctgaggaggg cgacctgaac 180
   cctcctcctg aggccaagca ggtgcctgtg tcctactacg actccaccta cctgtccacc 240
   gacaacgaga aggacaacta cctgaagggc gtgaccaagc tgttcgagag aatctactcc 300
   accgacctgg gcagaatgct gctgacctcc atcgtgagag gcatcccttt ctggggcggc 360
   tccaccatcg acaccgagct gaaggtgatc gacaccaact gcatcaacgt gatccagcct 420
   gacggctcct acagatccga ggagctgaac ctggtgatca tcggcccttc cgccgacatc 480
   atccagttcg agtgcaagtc cttcggccac gaggtgctga acctgaccag aaacggctac 540
   ggctccaccc agtacatcag attctcccct gacttcacct tcggcttcga ggagtccctg 600
   gaggtggaca ccaaccctct gctgggcgcc ggcaagttcg ccaccgaccc tgccgtgacc 660
   ctggcccacg agctgatcca cgccggccac agactgtacg gcatcgccat caaccctaac 720
   agagtgttca aggtgaacac caacgcctac tacgagatgt ccggcctgga ggtgtccttc 780
   gaggagctga gaaccttcgg cggccacgac gccaagttca tcgactccct gcaggagaac 840
   gagttcagac tgtactacta caacaagttc aaggacatcg cctccaccct gaacaaggcc 900
   aagtccatcg tgggcaccac cgcctccctg cagtacatga agaacgtgtt caaggagaag 960
   tacctgctgt ccgaggacac ctccggcaag ttctccgtgg acaagctgaa gttcgacaag 1020
   ctgtacaaga tgctgaccga gatctacacc gaggacaact tcgtgaagtt cttcaaggtg 1080
   ctgaacagaa agacctacct gaacttcgac aaggccgtgt tcaagatcaa catcgtgcct 1140
   aaggtgaact acaccatcta cgacggcttc aacctgagaa acaccaacct ggccgccaac 1200
   ttcaacggcc agaacaccga gatcaacaac atgaacttca ccaagctgaa gaacttcacc 1260
   ggcctgttcg agttctacaa gctgctgtgc gtgagaggca tcatcacctc caagaccaag 1320
   tccctggaca agggctacaa caaggccctg aacgacctgt gcatcaaggt gaacaactgg 1380
   gacctgttct tctccccttc cgaggacaac ttcaccaacg acctgaacaa gggcgaggag 1440
   atcacctccg acaccaacat cgaggccgcc gaggagaaca tctccctgga cctgatccag 1500
   cagtactacc tgaccttcaa cttcgacaac gagcctgaga acatctccat cgagaacctg 1560
   tcctccgaca tcatcggcca gctggagctg atgcctaaca tcgagagatt ccctaacggc 1620
   aagaagtacg agctggacaa gtacaccatg ttccactacc tgagagccca ggagttcgag 1680
   cacggcaagt ccagaatcgc cctgaccaac tccgtgaacg aggccctgct gaacccttcc 1740
   agagtgtaca ccttcttctc ctccgactac gtgaagaagg tgaacaaggc caccgaggcc 1800
   gccatgttcc tgggctgggt ggagcagctg gtgtacgact tcaccgacga gacctccgag 1860
   gtgtccacca ccgacaagat cgccgacatc accatcatca tcccttacat cggccctgcc 1920
   ctgaacatcg gcaacatgct gtacaaggac gacttcgtgg gcgccctgat cttctccggc 1980
   gccgtgatcc tgctggagtt catccctgag atcgccatcc ctgtgctggg caccttcgcc 2040
   ctggtgtcct acatcgccaa caaggtgctg accgtgcaga ccatcgacaa cgccctgtcc 2100
   aagagaaacg agaagtggga cgaggtgtac aagtacatcg tgaccaactg gctggccaag 2160
   gtgaacaccc agatcgacct gatcagaaag aagatgaagg aggccctgga gaaccaggcc 2220
   gaggccacca aggccatcat caactaccag tacaaccagt acaccgagga ggagaagaac 2280
   aacatcaact tcaacatcga cgacctgtcc tccaagctga acgagtccat caacaaggcc 2340
   atgatcaaca tcaacaagtt cctgaaccag tgctccgtgt cctacctgat gaactccatg 2400
   atcccttacg gcgtgaagag actggaggac ttcgacgcct ccctgaagga cgccctgctg 2460
   aagtacatct acgacaacag aggcaccctg atcggccagg tggacagact gaaggacaag 2520
   gtgaacaaca ccctgtccac cgacatccct ttccagctgt ccaagtacgt ggacaaccag 2580
   agactgctgt ccaccttcac cgagtacatc aagaacatca tcaacacctc catcctgaac 2640
   ctgagatacg agtccaacca cctgatcgac ctgtccagat acgcctccaa gatcaacatc 2700
   ggctccaagg tgaacttcga ccctatcgac aagaaccaga tccagctgtt caacctggag 2760
   tcctccaaga tcgaggtgat cctgaagaac gccatcgtgt acaactccat gtacgagaac 2820
   ttctccacct ccttctggat cagaatccct aagtacttca actccatctc cctgaacaac 2880
   gagtacacca tcatcaactg catggagaac aactccggct ggaaggtgtc cctgaactac 2940
   ggcgagatca tctggaccct gcaggacacc caggagatca agcagagagt ggtgttcaag 3000
   tactcccaga tgatcaacat ctccgactac atcaacagat ggatcttcgt gaccatcacc 3060
   aacaacagac tgaacaactc caagatctac atcaacggca gactgatcga ccagaagcct 3120
   atctccaacc tgggcaacat ccacgcctcc aacaacatca tgttcaagct ggacggctgc 3180
   agagacaccc acagatacat ctggatcaag tacttcaacc tgttcgacaa ggagctgaac 3240
   gagaaggaga tcaaggacct gtacgacaac cagtccaact ccggcatcct gaaggacttc 3300
   tggggcgact acctgcagta cgacaagcct tactacatgc tgaacctgta cgaccctaac 3360
   aagtacgtgg acgtgaacaa cgtgggcatc agaggctaca tgtacctgaa gggccctaga 3420
   ggctccgtga tgaccaccaa catctacctg aactcctccc tgtacagagg caccaagttc 3480
   atcatcaaga agtacgcctc cggcaacaag gacaacatcg tgagaaacaa cgacagagtg 3540
   tacatcaacg tggtggtgaa gaacaaggag tacagactgg ccaccaacgc ctcccaggcc 3600
   ggcgtggaga agatcctgtc cgccctggag atccctgacg tgggcaacct gtcccaggtg 3660
   gtggtgatga agtccaagaa cgaccagggc atcaccaaca agtgcaagat gaacctgcag 3720
   gacaacaacg gcaacgacat cggcttcatc ggcttccacc agttcaacaa catcgccaag 3780
   ctggtggcct ccaactggta caacagacag atcgagagat cctccagaac cctgggctgc 3840
   tcctgggagt tcatccctgt ggacgacggc tggggcgaga gacctctgtg a          3891
<210> 84
   <211> 3891
   <212> DNA
   <213> Artificial Sequence
<220>
   <223> BoNT/A, C. griseus-modified 3
<400> 84
   atgcccttcg tgaacaagca gttcaactac aaagaccccg tgaatggcgt ggatatcgct 60
   tatatcaaga ttccaaacgc tggacagatg cagcccgtca aagccttcaa gatccataat 120
   aaaatctggg tgatccctga aagggacacc tttaccaacc cagaagaggg cgatctgaac 180
   cctccacctg aagcaaaaca ggtgccagtt tcttattacg actcaaccta cttgagtaca 240
   gacaatgaaa aagataatta tctgaagggc gttaccaagc ttttcgagcg tatctatagc 300
   accgacctgg gaagaatgct ccttacaagt atcgtgagag gtatcccttt ttggggtggg 360
   tctaccatcg atactgaact caaagtgatc gacaccaact gtattaacgt gattcagccc 420
   gatggcagtt acaggtccga agagctgaat ctcgtgatca ttggaccttc tgccgatatc 480
   attcagtttg aatgcaaatc ctttggccac gaggtgctga acctgacaag aaacggctat 540
   ggatctactc agtacattag attttctcca gacttcactt tcgggtttga ggaatccctg 600
   gaggtggaca ccaacccact tctcggcgca ggcaaattcg caactgatcc tgccgtgacc 660
   cttgcccatg agctgatcca cgccgggcat cgactctatg gaattgcaat caaccccaat 720
   cgagtgttta aggtgaatac taacgcttat tacgagatga gtgggctgga ggtgtctttc 780
   gaggaactga ggacattcgg aggtcacgac gcaaagttca ttgacagcct gcaggagaac 840
   gagtttcgcc tgtactatta caataagttt aaggacatcg cttcaaccct gaataaagcc 900
   aagagcatcg tcgggaccac agcttccctg cagtacatga aaaatgtgtt caaagagaaa 960
   tacctcctgt ccgaagatac ctccggcaag ttctccgtgg acaagctgaa atttgacaag 1020
   ctctataaga tgctgactga gatctatacc gaggacaact ttgtgaagtt ctttaaagtt 1080
   ttgaatcgca aaacctactt gaattttgat aaagctgtgt tcaagattaa cattgtgccc 1140
   aaggtgaatt ataccatcta cgacggtttc aacctgcgta ataccaacct ggccgctaac 1200
   tttaatggtc agaacactga aatcaataac atgaacttca ccaagctgaa aaattttact 1260
   ggccttttcg agttctacaa actgctctgc gtgaggggaa ttatcacctc caagaccaaa 1320
   tccttggata aaggatataa caaggctctg aacgatcttt gcattaaagt gaacaattgg 1380
   gacctgttct tttctccaag tgaagacaac tttacaaacg accttaacaa gggggaagag 1440
   attacaagcg acacaaatat cgaggccgct gaggaaaaca tcagtctgga tctgatccag 1500
   cagtactatc tgacattcaa cttcgataac gagcctgaaa acattagtat tgagaatttg 1560
   tccagcgaca ttatcggaca gcttgagctg atgcctaata tcgagcggtt tccaaacggt 1620
   aagaaatatg agcttgataa gtacaccatg tttcactacc ttagggccca ggagttcgag 1680
   cacggcaaat ctcgaattgc cctgaccaac tccgttaacg aggccctgct caatccttcc 1740
   agagtctaca ccttctttag ctccgattac gtcaagaaag ttaataaagc tacagaggcc 1800
   gcaatgttct tgggatgggt cgagcagctg gtctacgatt ttacagatga gacctccgag 1860
   gtctcaacta ccgacaagat tgctgatatc acaattatca ttccctacat cgggcccgcc 1920
   ctgaatatcg gcaatatgct gtacaaggac gatttcgttg gcgcattgat ttttagcggc 1980
   gctgtgatcc tgctcgagtt catccccgag attgccattc ctgtccttgg tacatttgca 2040
   ctggtgtcat atatcgctaa caaagtgctg accgtccaga ctatcgacaa cgcactgtct 2100
   aagcgcaacg aaaagtggga tgaagtctat aagtatatcg tgactaactg gctcgcaaaa 2160
   gttaacacac agatcgatct gatccgcaag aaaatgaaag aagccctcga gaatcaggct 2220
   gaagccacca aggctatcat taattatcag tacaatcagt acactgagga agagaagaat 2280
   aacatcaatt tcaacatcga cgatctctct agtaaactga acgaatccat caacaaggcc 2340
   atgatcaata tcaacaagtt tctcaatcag tgttcagtgt cctacctgat gaacagcatg 2400
   atcccttatg gagtgaagcg actggaagac ttcgacgcct ccctgaagga cgcacttttg 2460
   aaatacattt atgataatcg aggaacactg attggacagg tggacagact gaaagacaag 2520
   gtgaacaata ctctctctac tgacatccct tttcagctgt ccaagtacgt tgacaaccag 2580
   cgcctgcttt ctacatttac agagtatatt aagaatatca ttaataccag catcctgaac 2640
   ttgaggtacg aaagcaacca cctgattgat ctgtcacggt atgccagcaa gatcaatatc 2700
   gggagcaagg tcaatttcga ccccatcgat aagaatcaga ttcagctgtt taacttggag 2760
   agctcaaaga tcgaggtgat cctgaagaat gcaattgtgt ataatagcat gtacgagaac 2820
   ttcagtactt cattctggat ccgtatcccc aagtatttca actccatctc actcaataac 2880
   gaatacacca tcattaattg catggagaac aattctggat ggaaggtctc acttaattat 2940
   ggcgaaatca tttggactct gcaggatact caggaaatca aacagagggt cgtgtttaag 3000
   tacagtcaga tgatcaatat ctccgattat atcaaccggt ggatttttgt cacaatcacc 3060
   aacaatcgat tgaacaattc caagatctac attaacggtc gcttgatcga ccagaaacct 3120
   atctccaatc tggggaatat tcacgcatct aacaatatta tgttcaagct cgatggctgt 3180
   cgggatacac atagatacat ctggatcaag tatttcaatc tcttcgataa agaactgaac 3240
   gagaaggaaa ttaaggatct gtacgacaac cagtcaaaca gtggaattct gaaggacttc 3300
   tggggtgatt atctgcagta tgacaaacct tattacatgc tcaacttgta tgatcccaat 3360
   aaatacgtgg acgttaacaa tgttggtatc cggggctaca tgtatctgaa gggtccacgc 3420
   gggagtgtca tgaccactaa catctacctg aatagctctc tttacagagg gaccaagttt 3480
   atcattaaga aatacgccag cgggaataag gataatatcg tgcgtaacaa tgatcgggtg 3540
   tacatcaacg ttgtcgttaa gaataaggaa tatcgcttgg ccaccaacgc tagccaggcc 3600
   ggtgtggaga agattctgtc cgccctggag atcccagacg tgggcaacct ctcacaggtc 3660
   gtggttatga aatctaagaa cgatcagggc attacaaaca agtgtaagat gaacttgcag 3720
   gacaacaatg gtaatgatat cggctttatc gggtttcatc agtttaataa catcgctaaa 3780
   ctggtggcca gcaactggta caacaggcag atcgaacgga gttctcggac cttgggctgc 3840
   tcttgggaat tcatcccagt ggacgacgga tggggcgagc gtcctctctg a 3891
<210> 85
   <211> 3891
   <212> DNA
   <213> Artificial Sequence
<220>
   <223> BoNT/A, S. scrofa-modified 1
<400> 85.
   atgcccttcg tgaacaaaca gttcaactac aaggacccgg tcaatggagt cgacatagcc 60
   tatatcaaga tccccaacgc gggccagatg caacccgtca aggcattcaa gatccacaat 120
   aagatttggg tgattcccga gagagacaca tttactaacc ctgaggaagg cgacctcaac 180
   cccccacccg aagctaagca ggttccagtc tcatattacg acagcactta cctgagcact 240
   gacaacgaaa aagacaatta cctaaaagga gttaccaagc tgttcgagag aatttactcc 300
   acagatctcg gcagaatgct gctcacaagc atcgtccgtg gcatcccatt ctggggggga 360
   agcactatcg acaccgagtt gaaagtgata gacaccaact gtataaacgt tatccagcct 420
   gacggctcct atcgctccga agagctaaac ttggttatta tcggcccatc cgctgatata 480
   attcagtttg agtgcaagag ctttggacac gaggtcttga atctgaccag gaatggctat 540
   gggtccaccc agtacattcg gttttctccg gacttcacgt tcggcttcga agagtccctc 600
   gaagtggaca caaaccccct gcttggggcc ggcaagttcg ccactgatcc tgccgtgacc 660
   ttagcccacg aactcatcca tgccgggcac cggctctacg gcatagcaat caatccgaac 720
   cgggtgttta aagtaaacac caatgcgtac tatgaaatga gcgggttgga agtctccttc 780
   gaggaactgc gcaccttcgg cgggcacgac gcgaaattca tcgactccct ccaggaaaat 840
   gagttccgtc tgtactatta caataaattc aaggacatcg cctcaacctt aaacaaagct 900
   aaaagtattg tcggtaccac tgcttccctg cagtatatga agaatgtttt taaggagaag 960
   tatctgcttt cagaagatac tagcgggaaa ttcagcgtgg ataagctcaa attcgataaa 1020
   ctctataaga tgctgaccga gatctacact gaggacaatt tcgtgaagtt tttcaaggtg 1080
   ctgaatcgca aaacgtatct caattttgat aaagccgtgt ttaagatcaa tattgttcct 1140
   aaggtcaatt atacgatcta cgatggattt aatcttcgga acacgaatct tgccgctaac 1200
   ttcaacggtc agaacactga aataaacaat atgaacttca ccaaactgaa gaattttaca 1260
   gggttgttcg agttctacaa gctgctctgt gtgcgaggga ttatcacaag taagactaag 1320
   tctcttgaca aaggctataa caaggctctg aatgacctgt gcatcaaggt gaacaattgg 1380
   gacctgttct ttagtccgtc tgaggacaac ttcaccaatg acctgaacaa gggtgaggaa 1440
   ataacgtcgg acacaaacat agaagccgcg gaagagaata tctccttgga tctgatccag 1500
   caatactatc tcaccttcaa cttcgataac gagcccgaga atatctcgat tgagaacctt 1560
   tcttcagata tcattggaca gctggaactg atgccaaaca tcgagcgctt tccaaatggc 1620
   aaaaagtacg aactggacaa atacacaatg ttccactatc tgagggcgca agaattcgag 1680
   catggcaaga gccgaatagc tctcacaaac agtgtgaacg aggcactgct caatcccagt 1740
   cgggtataca ccttctttag ctccgactac gtgaaaaagg tgaacaaggc gacagaggca 1800
   gctatgttcc ttggttgggt cgagcagctg gtgtatgatt tcaccgacga gacaagcgaa 1860
   gttagcacca cggataaaat cgcagacatc acgataatta taccgtacat cgggccagcc 1920
   ctcaatatcg gtaacatgct gtacaaagac gattttgtcg gcgctctcat cttctccgga 1980
   gccgtgatct tgctagaatt catccccgag attgccatcc ctgtcctggg caccttcgcc 2040
   ttagtaagtt atattgccaa taaggtgttg accgtccaga ccatcgataa cgccttaagt 2100
   aagaggaatg aaaagtggga cgaggtttac aaatatatag tgacgaattg gctggccaag 2160
   gtgaacaccc aaatcgatct gatccgaaaa aagatgaagg aagccctgga gaaccaggcc 2220
   gaggcaacca aggctattat caactaccag tacaaccagt acacagagga agagaaaaac 2280
   aatatcaact ttaacatcga cgatctctcc agcaagctga acgagagcat taacaaggcc 2340
   atgatcaata tcaataagtt tctgaatcag tgcagtgtgt catacctgat gaactcaatg 2400
   attccatacg gcgtcaagcg gctcgaggat ttcgatgcct ctctcaagga cgcactctta 2460
   aaatatatct acgacaacag aggtacacta ataggtcagg tggatcggct gaaagacaag 2520
   gtgaacaata cgctgagcac ggacattccc ttccagctgt caaagtacgt cgacaatcag 2580
   cgcctgcttt ctacatttac cgagtacatc aaaaacatta tcaatactag catcctgaac 2640
   ctcaggtacg agtcgaacca tttgatcgac ctttctaggt atgcaagcaa aattaatata 2700
   ggatctaaag tcaactttga ccctatcgac aagaatcaaa tccagttatt taacctggaa 2760
   tcctctaaga ttgaagttat tctgaaaaat gcgatcgtct acaatagcat gtatgagaac 2820
   tttagtacca gcttttggat taggatcccc aaatacttca actctattag cctgaataac 2880
   gagtacacca taatcaactg catggagaac aattctgggt ggaaggtgtc gctaaattac 2940
   ggcgagataa tctggaccct gcaggacacc caggagatca aacaaagagt ggtattcaag 3000
   tatagccaga tgatcaacat ctccgattat atcaacagat ggatctttgt gaccatcacc 3060
   aacaatagac ttaacaattc caagatctat attaacggac gtctgattga tcaaaagcca 3120
   attagtaacc ttggtaacat ccatgcttcc aacaatatta tgttcaaact cgatggctgc 3180
   agggacactc acaggtacat ttggattaag tacttcaact tgtttgacaa ggagctcaat 3240
   gagaaggaga tcaaagatct gtacgataac cagtcgaact caggcattct gaaagacttc 3300
   tggggggact atctgcaata cgataagcct tattacatgc tgaaccttta cgatcccaac 3360
   aagtacgtgg acgtgaacaa tgttggaatt agaggttata tgtacctgaa aggacctcga 3420
   ggaagcgtga tgactacaaa tatttacctc aactcttccc tatacagggg cactaagttc 3480
   atcattaaaa agtacgcatc aggaaataag gataacatcg tgcgcaacaa tgaccgcgtc 3540
   tacatcaatg tggtagtgaa gaacaaggaa taccggctgg ccaccaacgc cagccaggcc 3600
   ggcgtcgaga agatcctgtc tgctctggaa atccccgatg tcgggaactt gagtcaggtg 3660
   gtagtgatga agtccaagaa cgaccagggg attactaaca aatgcaaaat gaacctccaa 3720
   gataacaatg ggaacgacat cggctttatc ggcttccacc agtttaacaa cattgcaaag 3780
   ttggtggctt caaactggta taaccgccag atcgagcgat cctctcgcac acttgggtgt 3840
   tcctgggagt ttatccctgt ggatgacgga tggggagagc ggcctttgtg a          3891
<210> 86
   <211> 3891
   <212> DNA
   <213> Artificial Sequence
<220>
   <223> BoNT/A, S. scrofa-modified 2
<400> 86
   atgcccttcg tgaacaagca gttcaactac aaggaccccg tgaacggcgt ggacatcgcc 60
   tacatcaaga tccccaacgc cggccagatg cagcccgtga aggccttcaa gatccacaac 120
   aagatctggg tgatccccga gagggacacc ttcaccaacc ccgaggaggg cgacctgaac 180
   cccccccccg aggccaagca ggtgcccgtg agctactacg acagcaccta cctgagcacc 240
   gacaacgaga aggacaacta cctgaagggc gtgaccaagc tgttcgagag gatctacagc 300
   accgacctgg gcaggatgct gctgaccagc atcgtgaggg gcatcccctt ctggggcggc 360
   agcaccatcg acaccgagct gaaggtgatc gacaccaact gcatcaacgt gatccagccc 420
   gacggcagct acaggagcga ggagctgaac ctggtgatca tcggccccag cgccgacatc 480
   atccagttcg agtgcaagag cttcggccac gaggtgctga acctgaccag gaacggctac 540
   ggcagcaccc agtacatcag gttcagcccc gacttcacct tcggcttcga ggagagcctg 600
   gaggtggaca ccaaccccct gctgggcgcc ggcaagttcg ccaccgaccc cgccgtgacc 660
   ctggcccacg agctgatcca cgccggccac aggctgtacg gcatcgccat caaccccaac 720
   agggtgttca aggtgaacac caacgcctac tacgagatga gcggcctgga ggtgagcttc 780
   gaggagctga ggaccttcgg cggccacgac gccaagttca tcgacagcct gcaggagaac 840
   gagttcaggc tgtactacta caacaagttc aaggacatcg ccagcaccct gaacaaggcc 900
   aagagcatcg tgggcaccac cgccagcctg cagtacatga agaacgtgtt caaggagaag 960
   tacctgctga gcgaggacac cagcggcaag ttcagcgtgg acaagctgaa gttcgacaag 1020
   ctgtacaaga tgctgaccga gatctacacc gaggacaact tcgtgaagtt cttcaaggtg 1080
   ctgaacagga agacctacct gaacttcgac aaggccgtgt tcaagatcaa catcgtgccc 1140
   aaggtgaact acaccatcta cgacggcttc aacctgagga acaccaacct ggccgccaac 1200
   ttcaacggcc agaacaccga gatcaacaac atgaacttca ccaagctgaa gaacttcacc 1260
   ggcctgttcg agttctacaa gctgctgtgc gtgaggggca tcatcaccag caagaccaag 1320
   agcctggaca agggctacaa caaggccctg aacgacctgt gcatcaaggt gaacaactgg 1380
   gacctgttct tcagccccag cgaggacaac ttcaccaacg acctgaacaa gggcgaggag 1440
   atcaccagcg acaccaacat cgaggccgcc gaggagaaca tcagcctgga cctgatccag 1500
   cagtactacc tgaccttcaa cttcgacaac gagcccgaga acatcagcat cgagaacctg 1560
   agcagcgaca tcatcggcca gctggagctg atgcccaaca tcgagaggtt ccccaacggc 1620
   aagaagtacg agctggacaa gtacaccatg ttccactacc tgagggccca ggagttcgag 1680
   cacggcaaga gcaggatcgc cctgaccaac agcgtgaacg aggccctgct gaaccccagc 1740
   agggtgtaca ccttcttcag cagcgactac gtgaagaagg tgaacaaggc caccgaggcc 1800
   gccatgttcc tgggctgggt ggagcagctg gtgtacgact tcaccgacga gaccagcgag 1860
   gtgagcacca ccgacaagat cgccgacatc accatcatca tcccctacat cggccccgcc 1920
   ctgaacatcg gcaacatgct gtacaaggac gacttcgtgg gcgccctgat cttcagcggc 1980
   gccgtgatcc tgctggagtt catccccgag atcgccatcc ccgtgctggg caccttcgcc 2040
   ctggtgagct acatcgccaa caaggtgctg accgtgcaga ccatcgacaa cgccctgagc 2100
   aagaggaacg agaagtggga cgaggtgtac aagtacatcg tgaccaactg gctggccaag 2160
   gtgaacaccc agatcgacct gatcaggaag aagatgaagg aggccctgga gaaccaggcc 2220
   gaggccacca aggccatcat caactaccag tacaaccagt acaccgagga ggagaagaac 2280
   aacatcaact tcaacatcga cgacctgagc agcaagctga acgagagcat caacaaggcc 2340
   atgatcaaca tcaacaagtt cctgaaccag tgcagcgtga gctacctgat gaacagcatg 2400
   atcccctacg gcgtgaagag gctggaggac ttcgacgcca gcctgaagga cgccctgctg 2460
   aagtacatct acgacaacag gggcaccctg atcggccagg tggacaggct gaaggacaag 2520
   gtgaacaaca ccctgagcac cgacatcccc ttccagctga gcaagtacgt ggacaaccag 2580
   aggctgctga gcaccttcac cgagtacatc aagaacatca tcaacaccag catcctgaac 2640
   ctgaggtacg agagcaacca cctgatcgac ctgagcaggt acgccagcaa gatcaacatc 2700
   ggcagcaagg tgaacttcga ccccatcgac aagaaccaga tccagctgtt caacctggag 2760
   agcagcaaga tcgaggtgat cctgaagaac gccatcgtgt acaacagcat gtacgagaac 2820
   ttcagcacca gcttctggat caggatcccc aagtacttca acagcatcag cctgaacaac 2880
   gagtacacca tcatcaactg catggagaac aacagcggct ggaaggtgag cctgaactac 2940
   ggcgagatca tctggaccct gcaggacacc caggagatca agcagagggt ggtgttcaag 3000
   tacagccaga tgatcaacat cagcgactac atcaacaggt ggatcttcgt gaccatcacc 3060
   aacaacaggc tgaacaacag caagatctac atcaacggca ggctgatcga ccagaagccc 3120
   atcagcaacc tgggcaacat ccacgccagc aacaacatca tgttcaagct ggacggctgc 3180
   agggacaccc acaggtacat ctggatcaag tacttcaacc tgttcgacaa ggagctgaac 3240
   gagaaggaga tcaaggacct gtacgacaac cagagcaaca gcggcatcct gaaggacttc 3300
   tggggcgact acctgcagta cgacaagccc tactacatgc tgaacctgta cgaccccaac 3360
   aagtacgtgg acgtgaacaa cgtgggcatc aggggctaca tgtacctgaa gggccccagg 3420
   ggcagcgtga tgaccaccaa catctacctg aacagcagcc tgtacagggg caccaagttc 3480
   atcatcaaga agtacgccag cggcaacaag gacaacatcg tgaggaacaa cgacagggtg 3540
   tacatcaacg tggtggtgaa gaacaaggag tacaggctgg ccaccaacgc cagccaggcc 3600
   ggcgtggaga agatcctgag cgccctggag atccccgacg tgggcaacct gagccaggtg 3660
   gtggtgatga agagcaagaa cgaccagggc atcaccaaca agtgcaagat gaacctgcag 3720
   gacaacaacg gcaacgacat cggcttcatc ggcttccacc agttcaacaa catcgccaag 3780
   ctggtggcca gcaactggta caacaggcag atcgagagga gcagcaggac cctgggctgc 3840
   agctgggagt tcatccccgt ggacgacggc tggggcgaga ggcccctgtg a          3891
<210> 87
   <211> 3891
   <212> DNA
   <213> Artificial Sequence
<220>
   <223> BoNT/A, S. scrofa-modified 3
<400> 87
   atgcccttcg tgaacaagca gttcaactac aaggaccccg ttaatggagt cgacatcgct 60
   tacatcaaaa ttccaaacgc tgggcagatg cagccggtta aggcttttaa gatccacaat 120
   aagatttggg tgatccccga aagagataca ttcacaaatc cagaggaagg tgacctgaac 180
   cctcccccag aagccaaaca ggtgccagtg tcctactatg atagcaccta cctgtccact 240
   gacaacgaga aagacaacta tctgaaaggg gtgaccaaac tttttgaacg aatctattct 300
   acagacttgg gaaggatgct gcttacctca atcgtgagag gcattccttt ttggggggga 360
   tccaccatcg acaccgagct caaggtgatc gacaccaact gcattaacgt tatccagcca 420
   gacgggagct accgatcaga ggaacttaat cttgtgatta tcggtccgtc tgccgacatt 480
   atccagtttg agtgcaagtc attcggccat gaggttctca acctgacgag gaacggctat 540
   gggtcaacac agtacattag attcagccca gacttcacgt tcggcttcga agagagtctg 600
   gaggtggaca ccaatcccct cttgggcgcc ggaaaattcg caaccgatcc ggccgttact 660
   ctggcacatg agctcatcca cgccggacac cgactttacg gtatcgccat caaccctaac 720
   cgggttttta aggtgaatac aaacgcctat tacgagatgt cagggctcga ggtgtcattc 780
   gaggaactgc gcacttttgg cggtcacgac gccaagttca tcgatagcct tcaggagaat 840
   gaattccgcc tgtactatta caataagttt aaagatattg cctccacttt gaataaagcc 900
   aaaagcattg tcggaacaac ggccagtctg cagtacatga aaaatgtctt taaagagaag 960
   tatcttttgt ccgaggacac gagcggtaag ttcagcgtgg ataagctgaa gttcgataag 1020
   ctctacaaaa tgctgacaga gatctacacc gaagacaatt tcgtcaaatt ctttaaagtg 1080
   ctgaatagga agacttacct caatttcgat aaggccgtgt ttaaaatcaa tatcgtgccc 1140
   aaggtcaact acaccatcta cgatgggttc aacctcagga acaccaacct ggcagccaac 1200
   ttcaatgggc agaacacgga aatcaataac atgaacttta caaagcttaa aaattttaca 1260
   ggcctctttg aattctataa actgctctgc gtgagaggaa ttatcactag taaaactaaa 1320
   tcccttgaca agggatacaa taaggccctg aacgatctct gcatcaaggt gaacaattgg 1380
   gacctgttct tttctccgtc tgaagacaat tttaccaacg atctgaacaa aggcgaagag 1440
   attacgtcag acacgaatat tgaggccgca gaggaaaaca tttccttgga tctgatccag 1500
   cagtactatc tgacttttaa ctttgacaac gaacctgaaa atatctccat cgaaaacctg 1560
   agctccgaca ttatcggcca gcttgagctg atgcctaata ttgagaggtt tcctaacggc 1620
   aaaaagtatg aactggataa atataccatg tttcattacc tgagggctca ggaattcgaa 1680
   cacggaaagt ctaggatcgc tctgactaat agcgtgaatg aagctctgct caacccctct 1740
   cgcgtgtata cattctttag ctccgactac gtcaaaaagg tgaacaaggc taccgaggca 1800
   gccatgttct tgggctgggt ggagcagctg gtctacgatt tcaccgatga gacctccgag 1860
   gtcagcacca ctgataagat cgcagacatc accatcatta tcccttatat cggcccagcc 1920
   cttaacatcg gcaacatgct gtataaagac gatttcgtgg gggctctgat cttctccggc 1980
   gccgtgatct tgctggagtt tattcccgag atcgccatcc ccgtgctggg gaccttcgct 2040
   ctcgtcagtt acatcgccaa caaggtgctg accgtccaga ccattgacaa cgccctgagt 2100
   aagagaaatg aaaagtggga cgaggtctac aagtacatcg ttacaaattg gctcgctaag 2160
   gtcaacacac agatcgacct cattaggaag aaaatgaaag aggccctgga gaatcaggca 2220
   gaggccacca aggccattat caactaccag tacaatcagt acactgagga agagaaaaac 2280
   aatattaact tcaacatcga cgatctgtca agcaaactga acgaatccat caataaggct 2340
   atgattaata tcaataagtt cctgaaccag tgttctgtgt cttatctgat gaacagcatg 2400
   atcccatacg gcgtgaagcg cttggaagac ttcgatgcaa gtctgaaaga tgctttgctg 2460
   aagtacattt acgataaccg cggcaccctc atcggacagg tcgaccggct gaaggacaag 2520
   gtgaataaca ccctgagtac tgatattccc tttcagctct ctaagtatgt cgacaatcag 2580
   aggctgctct ccactttcac agagtatatc aaaaacatta tcaacacgag tattctgaat 2640
   ctgcggtacg agagtaacca cctgatcgac ctcagcagat acgccagcaa gatcaatatt 2700
   ggcagcaagg tgaattttga ccctatcgac aaaaaccaga tccagctgtt caacctcgaa 2760
   agctcaaaga tcgaggtgat cctgaagaac gccattgtgt acaatagcat gtacgagaac 2820
   ttcagcactt ctttctggat ccggatccca aagtacttca atagcatcag cctgaacaat 2880
   gagtatacca tcattaactg tatggagaac aattccggct ggaaggtgtc cctcaattac 2940
   ggggagatca tttggacact tcaggataca caggagatca agcagcgggt cgtgttcaaa 3000
   tactcacaga tgatcaacat ctctgattat atcaaccgct ggatcttcgt gaccattacc 3060
   aacaatcggt tgaacaattc taagatttat atcaatggac ggctgatcga ccagaagcct 3120
   attagtaatc tcggaaacat tcacgcaagc aacaatatca tgttcaaact ggacggatgc 3180
   agagatactc atcgctacat ctggatcaag tactttaatc tgtttgacaa ggagttgaat 3240
   gaaaaggaga ttaaggatct gtacgacaac cagagcaact ccggaatcct gaaggatttc 3300
   tggggcgact atttgcagta cgataagccc tactatatgc ttaacctgta tgaccccaac 3360
   aagtacgttg acgtgaacaa tgtgggcatc cgaggatata tgtatctcaa gggcccccga 3420
   gggagcgtga tgaccacaaa catctacctg aactctagcc tgtacagagg tacgaagttt 3480
   atcattaaga aatatgccag cgggaacaag gacaatatcg ttaggaacaa tgacagggtt 3540
   tacatcaatg tcgtggtcaa aaacaaagag tataggctgg ccaccaacgc aagtcaggcc 3600
   ggtgtggaga agatcctctc cgccctggag atccctgacg tcggtaacct ctcccaggtg 3660
   gtcgtgatga aaagcaaaaa cgaccagggg atcacgaaca agtgcaaaat gaacctgcag 3720
   gataacaacg ggaacgatat cggtttcatc ggctttcacc agttcaacaa catcgccaag 3780
   ctggtggctt ccaactggta caaccggcag atcgagcgct cttcccgcac cttgggctgt 3840
   agctgggagt tcatccccgt cgatgacggg tggggcgaac ggccgctgtg a 3891
<210> 88
   <211> 3891
   <212> DNA
   <213> Artificial Sequence
<220>
   <223> BoNT/A, B. taurus-modified 1
<400> 88
   atgccttttg tgaacaaaca gttcaactac aaggatccgg ttaacggtgt cgacatcgct 60
   tacatcaaaa tccctaacgc aggacaaatg cagccagtaa aagcttttaa aattcacaac 120
   aagatttggg tgatcccgga gcgggatacc tttacaaacc ccgaagaggg agatctaaac 180
   ccacccccgg aggccaaaca ggtgcccgtc tcctactatg actcaaccta tttgagtacg 240
   gacaacgaaa aagacaatta cctgaaggga gttacaaaat tgttcgagag aatctatagc 300
   acagatctcg gaaggatgct cctgacttcc atcgtaagag gaataccctt ctggggtggc 360
   tcaactatcg atacagagct gaaagttatt gatactaact gcatcaacgt catccaaccc 420
   gacggcagct atcgatcaga agagctgaac ctggtcatta tcggcccttc cgccgacatt 480
   atccagttcg agtgtaaaag tttcggccac gaagttctga acctaacccg caacggatac 540
   gggagtaccc agtatatccg cttctcacca gacttcacat tcggcttcga ggaaagctta 600
   gaagtggata ctaaccccct tttaggtgcg gggaagttcg ccactgaccc agcggttacc 660
   ttggctcacg agctgatcca cgcaggccat cgtctatatg ggattgccat taatcccaac 720
   cgggtcttta aggtaaatac caacgcctat tacgagatga gtggactgga ggtgtctttc 780
   gaggaacttc ggacctttgg agggcatgac gcgaagttta tcgactctct gcaggagaac 840
   gaatttcgct tgtactatta caataagttc aaggacatag ctagcacact gaataaagcg 900
   aagagcatag taggcactac agcctccctg cagtacatga agaacgtttt caaggaaaaa 960
   tatctcctgt ccgaggacac ctccggtaag tttagcgttg ataagctgaa attcgataag 1020
   ttgtacaaga tgctcaccga gatttatacc gaggacaact tcgtgaagtt tttcaaggtg 1080
   ctgaatcgaa agacatacct gaatttcgat aaggcagtct tcaaaattaa catcgtgccc 1140
   aaggtgaatt atacaatcta cgacggcttc aaccttagaa acactaactt ggcggcaaac 1200
   ttcaatggcc agaacacgga aatcaacaat atgaacttca ctaaactaaa gaatttcacc 1260
   ggactctttg agttttacaa gctgttgtgc gtgcgcggca tcataactag caagaccaag 1320
   agcctggaca aaggttataa caaggccctg aacgacctct gcatcaaagt caacaattgg 1380
   gatctgtttt tcagcccatc cgaggacaat ttcacgaacg acttaaataa gggggaggaa 1440
   ataacatctg acaccaatat agaggcagct gaagagaata tcagcctcga tctgatccag 1500
   caatactatc tgacatttaa tttcgacaac gagccagaaa atattagcat cgagaatctt 1560
   agctctgaca ttataggcca gctggagctt atgccaaaca tcgaacggtt ccccaatggg 1620
   aagaaatacg aactcgataa atatacgatg ttccattacc tgagagccca ggaatttgaa 1680
   cacggaaagt cgagaattgc tctgactaac tcagtcaacg aggccctgtt gaaccctagc 1740
   cgcgtgtaca cgttctttag tagcgattac gtgaagaaag tgaacaaagc caccgaggcc 1800
   gcaatgttcc tgggctgggt ggagcaactc gtctacgact tcaccgatga gacctccgag 1860
   gtttcaacaa ccgacaaaat tgccgacatc accatcatta tcccttatat cggccccgct 1920
   ttaaacatcg gaaatatgct ctataaagac gattttgtgg gcgcactcat cttcagcggc 1980
   gccgtgatcc tgttggaatt catcccggag atagcgattc ccgtcttggg gaccttcgct 2040
   cttgtgagtt acatcgccaa caaagtgctg acagttcaaa ccattgataa cgctctgtcc 2100
   aagcgaaacg agaagtggga cgaggtgtat aaatacatcg tgacaaactg gctggccaaa 2160
   gtgaatacgc aaatcgacct catccgtaaa aagatgaagg aggcactaga aaaccaagct 2220
   gaagccacta aggccatcat taactatcaa tacaaccagt acaccgagga agagaagaac 2280
   aatataaact ttaacatcga tgacctttcc agcaagctga acgagagtat caataaggct 2340
   atgatcaaca ttaataaatt tttaaatcag tgctctgtgt cttacctgat gaattctatg 2400
   atcccatacg gagtgaagag gttggaggac ttcgacgcat ctctgaagga cgccctcctt 2460
   aagtatatct acgacaaccg cgggactctc attggccagg tggatagatt gaaggataaa 2520
   gtcaacaata cactctccac tgacattcct ttccagctgt ccaaatacgt ggataatcag 2580
   cggctcctga gcaccttcac cgagtacatc aaaaacatta tcaacacgag tatattaaat 2640
   ctgaggtacg agagcaatca tctgatcgat ctatcaaggt acgccagcaa gatcaatata 2700
   ggctctaagg taaattttga cccaatcgac aaaaaccaga tccagctgtt caatctcgaa 2760
   tcctcaaaga ttgaagtgat tctcaagaac gcaatcgtct acaacagcat gtacgaaaac 2820
   ttcagtacct ccttctggat taggattcct aaatacttca attccatctc cctgaacaat 2880
   gagtacacaa ttatcaactg tatggagaac aattccgggt ggaaggttag tcttaactac 2940
   ggcgaaatca tttggaccct ccaggacacg caagagatca agcagcgggt cgtgttcaag 3000
   tattctcaga tgatcaacat ctctgattac attaataggt ggatctttgt gaccatcact 3060
   aacaatagac tgaacaatag taagatatac attaacggtc gacttatcga ccagaaaccg 3120
   atcagtaacc ttggcaacat acacgcctct aataacatta tgtttaaact ggacgggtgt 3180
   cgggataccc acaggtatat ctggatcaag tattttaact tgtttgataa ggaactcaac 3240
   gagaaagaga ttaaggacct ttacgataac cagtctaact ccggtattct caaggacttt 3300
   tggggtgact atctgcagta cgataagccc tactatatgc tgaatctgta cgatccaaat 3360
   aagtacgtgg acgtgaacaa tgtgggcatc cgcggatata tgtatctcaa gggccctcgt 3420
   ggttctgtta tgaccactaa tatctatctg aactccagcc tgtacagggg gaccaagttt 3480
   atcattaaaa agtacgccag cgggaataaa gacaacatcg tcaggaacaa tgatagagtg 3540
   tacattaatg tggtcgtgaa gaataaggag taccgactgg ctacaaatgc atcgcaggcc 3600
   ggggtcgaaa agattctctc cgccctcgaa atacctgacg tcggtaacct ctcacaggta 3660
   gtggtcatga agtcgaaaaa cgatcaggga attacaaaca agtgcaagat gaacctgcag 3720
   gacaacaatg gcaacgatat agggtttata ggatttcacc agtttaacaa tatagctaag 3780
   ctggtagctt cgaactggta taaccggcag attgagcgtt catcgcgcac gcttgggtgt 3840
   tcatgggaat tcatccctgt ggatgacggc tggggtgagc ggcccctgtg a 3891
<210> 89
   <211> 3891
   <212> DNA
   <213> Artificial Sequence
<220>
   <223> BoNT/A, B. taurus-modified 2
<400> 89
<210> 90
   <211> 3891
   <212> DNA
   <213> Artificial Sequence
<220>
   <223> BoNT/A, B. taurus-modified 3
<400> 90
   atgccctttg tgaacaagca gttcaactac aaggaccccg tgaacggcgt tgatattgca 60
   tatatcaaga tcccaaacgc tggccagatg cagcctgtga aggccttcaa aattcacaat 120
   aagatctggg tgattcccga gagagatacc ttcactaacc cagaggaagg cgatctcaat 180
   ccacctcccg aggccaagca ggtccccgtc agctactatg acagcaccta tctgagcacc 240
   gataacgaaa aagacaacta cctgaagggc gtgacaaaac tttttgaacg gatttactcc 300
   acagacctgg gacgcatgct gcttacgtcc atcgttcggg gtatcccctt ttggggcggg 360
   agtacaatcg acaccgagct gaaggtgatc gatactaact gcatcaatgt gatccagccc 420
   gatggcagct accgaagcga ggaactgaac ctggtgatca ttgggccatc cgctgatatt 480
   atccagttcg agtgtaagag ctttggacac gaagtgttga accttacgag aaacggctac 540
   ggtagcaccc agtacattcg cttctctccc gacttcacct ttggtttcga ggaatccctg 600
   gaggtggata cgaacccact tctgggcgcc gggaagttcg caactgaccc cgccgtcact 660
   ctggcccacg agcttattca cgccgggcat aggctctatg ggattgccat caacccaaac 720
   cgagttttca aagtgaatac aaacgcctat tacgaaatgt ccggcctgga ggtctctttc 780
   gaggaactga gaacctttgg cgggcatgac gcaaagttca tcgactcact ccaggagaat 840
   gagtttcgtc tgtattacta taacaaattt aaagacatcg caagcacgct caacaaggca 900
   aaatccatcg tgggtactac cgcctccctg cagtatatga agaacgtctt taaagagaaa 960
   tatttgctct ctgaggacac ctccggtaaa ttcagcgtcg ataaacttaa gttcgataag 1020
   ctgtataaga tgctgactga gatttacacc gaggataatt tcgtcaaatt tttcaaggtg 1080
   ctgaacagga agacctactt gaacttcgac aaagccgtct tcaagatcaa cattgtgcct 1140
   aaagttaact acacaatcta cgacgggttc aaccttcgca acacaaacct cgctgccaac 1200
   ttcaatggac agaataccga gatcaataac atgaatttca ccaagttgaa aaattttacc 1260
   gggcttttcg aattttacaa gctgctctgt gttcgaggga tcattacatc taagaccaag 1320
   agcctggaca aaggatacaa taaagccctg aacgaccttt gtattaaagt caataactgg 1380
   gatctcttct tttcaccctc agaggacaac ttcaccaacg atctgaataa aggagaggaa 1440
   atcacttcag acacaaacat cgaggctgca gaggaaaaca ttagcttgga tttgatccag 1500
   cagtattacc tgacttttaa cttcgataac gagcctgaga atatctcaat cgaaaacctg 1560
   tcttcagaca tcattggcca gctggagttg atgcccaaca tcgagcggtt ccctaatgga 1620
   aaaaagtatg aactggacaa gtacacaatg tttcattatc tccgggccca ggaatttgag 1680
   catggtaagt ccagaattgc tctgactaac tccgtcaatg aggcactgct caaccctagc 1740
   agagtctaca cgttctttag ctccgattac gtgaagaaag tgaacaaggc taccgaggcc 1800
   gctatgtttc tgggttgggt cgaacagctg gtgtacgatt tcaccgacga gacttctgag 1860
   gtgtcaacaa ctgataagat cgccgatatc actattatca ttccctacat cggaccagct 1920
   ttgaatatcg ggaacatgct gtacaaagac gatttcgtgg gtgctctgat cttttctggg 1980
   gccgtcatcc ttctggaatt catcccagag atcgctatcc ctgtgctggg caccttcgcc 2040
   ctggtctcct atatcgccaa taaagttctg accgttcaga caattgacaa tgctctgagc 2100
   aaaaggaacg aaaagtggga cgaagtctac aagtatatcg tgactaattg gctggcaaaa 2160
   gtgaatactc agattgacct tattcgcaag aaaatgaagg aggccctgga gaatcaggct 2220
   gaggcaacca aggcaattat caactaccag tataatcagt ataccgagga agagaaaaac 2280
   aatatcaatt ttaatattga cgatctgtcc agtaagctca acgagagtat taacaaagcc 2340
   atgatcaaca tcaataaatt cctcaaccag tgctctgtgt cttacctgat gaacagcatg 2400
   atcccttatg gggtgaaacg gctcgaggat tttgacgcct ccttgaagga cgccctcctt 2460
   aaatatatct acgacaaccg cggtaccctg atcggacagg ttgacagact gaaagacaag 2520
   gtgaacaata cgctcagtac cgacatcccc tttcagctca gcaagtacgt ggataaccag 2580
   aggttgctct caaccttcac agagtacatt aagaacatca ttaacacatc tattctgaat 2640
   ttgaggtacg aatccaatca cctgatcgac ctgtcaaggt acgctagcaa gatcaacatc 2700
   ggaagcaagg tgaacttcga ccccatcgac aagaaccaga tccagctgtt caatctggaa 2760
   agttccaaaa tcgaggtgat cttgaagaac gccattgtgt ataactctat gtacgaaaat 2820
   ttcagtacca gcttctggat taggatcccc aagtatttca acagcatctc tctcaacaat 2880
   gagtacacaa tcattaactg tatggaaaac aatagtgggt ggaaggttag tctgaactac 2940
   ggtgaaatca tttggacgct gcaggacacg caggagatca agcagagagt ggttttcaag 3000
   tactctcaga tgatcaacat ttccgattat attaataggt ggatctttgt gacaatcaca 3060
   aataaccggc tgaacaatag caagatttat atcaatggcc gactgattga tcagaagccc 3120
   atcagcaatc tcggaaatat ccacgccagc aacaatatca tgtttaaact ggacggttgc 3180
   cgcgataccc accgctacat ctggatcaag tactttaacc tgtttgataa ggagttgaat 3240
   gaaaaggaga ttaaggacct ctacgacaat cagagtaatt cagggatcct taaagacttc 3300
   tggggcgact acctgcagta cgacaagcca tactatatgc tgaacctcta cgaccctaat 3360
   aagtacgtgg atgtgaacaa tgtgggcatt cggggctaca tgtacctgaa gggcccccgg 3420
   ggaagtgtca tgaccacaaa catctacctg aactccagcc tctatagagg aacgaagttc 3480
   atcatcaaga aatatgcctc cgggaacaag gataatatcg tgaggaataa cgaccgtgtg 3540
   tatatcaacg tggtcgtgaa gaacaaagag taccgtctcg ctacaaacgc ctcccaggca 3600
   ggcgtggaaa agatcctgag tgccctggag atcccagacg tgggaaatct gtctcaggtg 3660
   gttgtgatga agagcaagaa cgaccagggc atcactaaca aatgcaagat gaacctccag 3720
   gacaacaatg gaaacgatat cggcttcatc ggatttcacc agttcaataa catcgccaag 3780
   ctggtcgcca gcaactggta taaccgacag atcgaacgta gcagtcgcac cctcggctgc 3840
   tcatgggaat ttatccccgt ggatgatggc tggggcgaac ggcctctgtg a          3891
<210> 91
   <211> 3891
   <212> DNA
   <213> Artificial Sequence
<220>
   <223> BoNT/A, E. caballus-modified 1
<400> 91
   atgccgtttg tcaataaaca gtttaactac aaagatcccg tcaacggagt ggacatcgcc 60
   tacatcaaaa ttccgaatgc cgggcagatg cagcccgtga aggctttcaa gattcacaac 120
   aagatctggg ttatccctga gcgcgacacc ttcaccaacc ctgaggaagg agacctgaac 180
   cctccccctg aggctaagca agttcccgtg tcgtactatg actctacgta cttatctaca 240
   gacaatgaaa aggacaatta cctaaagggt gtgaccaaat tgttcgagcg gatctactcc 300
   accgatctcg gtagaatgct gttaacctct atcgtccgcg gtatcccctt ctggggcggt 360
   agcacgatcg atacagagtt gaaagtgata gatacgaact gtatcaacgt tatacaacca 420
   gacggctcgt acaggtctga ggaactaaat ctcgtgatta tcgggccatc agccgacatc 480
   attcagttcg agtgcaagtc attcgggcac gaggtgttga atttgacaag gaacggctac 540
   gggtcaactc agtacattcg cttcagcccg gattttacat ttggatttga agagtccctc 600
   gaggtggaca ccaaccctct gttgggagca ggaaagttcg ctaccgaccc tgctgtgacc 660
   ctcgcacatg aacttataca cgccggccac agactgtacg gcattgctat aaaccccaac 720
   agggtgttta aggttaatac gaacgcgtac tatgaaatga gtggactgga ggtttccttt 780
   gaggaactga ggacgttcgg agggcatgat gccaaattta tcgattctct tcaggagaac 840
   gagtttcgct tgtattacta taacaagttt aaggatatcg ccagcacact gaacaaagcc 900
   aaatctatcg tggggacaac tgctagcctg cagtatatga agaacgtctt taaggagaag 960
   tatctgctta gcgaggacac tagtgggaaa ttctctgtcg acaaactgaa gtttgataaa 1020
   ctttacaaaa tgctgaccga gatctacacc gaggataatt tcgtgaaatt ctttaaggta 1080
   ctgaatcgga aaacctacct caacttcgat aaggcggtct tcaagataaa cattgtccct 1140
   aaggtgaatt atactatcta cgacggattt aacttgcgta atactaatct tgctgccaat 1200
   ttcaacggcc agaacactga aatcaacaat atgaatttta ctaaactgaa aaactttacc 1260
   ggcctctttg aattttacaa acttctgtgc gtccggggca tcattacttc aaagaccaaa 1320
   agtctggaca aggggtacaa taaggccctg aatgacctgt gtatcaaggt gaacaattgg 1380
   gatttatttt tctccccatc tgaggacaat ttcactaatg acctgaacaa aggtgaagag 1440
   ataacatccg atacaaacat cgaggctgcc gaagagaata tcagcctcga tctcatccag 1500
   caatattacc tcacgttcaa cttcgacaac gaacctgaga acatttccat agagaatctt 1560
   agttcagaca tcattggcca gctggagttg atgcctaaca ttgaacggtt tcccaacggg 1620
   aagaaatatg aattggacaa atacacaatg tttcactatc tgagagccca ggagttcgag 1680
   catggcaaat ccagaatcgc cctgacgaat agcgttaacg aggctctgtt aaacccatcc 1740
   agagtctaca ctttcttttc cagcgactac gtcaagaaag taaacaaggc caccgaagca 1800
   gcgatgttcc tggggtgggt ggaacaactt gtgtatgatt tcaccgacga gacatccgaa 1860
   gtcagcacca cagataagat tgctgacatc accatcatta tcccctacat tggccccgct 1920
   ctgaacatcg gaaatatgct gtacaaggac gatttcgttg gggccttaat ttttagcgga 1980
   gccgtaatcc tcctggagtt tataccagag atcgccattc ccgtcctggg aactttcgca 2040
   ctggtgagtt acatcgccaa caaagtactc acagtgcaaa ccattgacaa tgccctaagc 2100
   aagcgaaatg aaaaatggga tgaggtgtac aagtatatcg tgacaaattg gctggcgaag 2160
   gtgaacactc aaatcgacct gattcgtaaa aagatgaagg aggcactgga gaatcaggcc 2220
   gaagcaacaa aagcgattat aaattaccag tacaatcagt acacggaaga ggaaaagaac 2280
   aatatcaact tcaacatcga cgatctgtcg agcaagctta acgagtctat taacaaggcc 2340
   atgatcaata ttaacaagtt tctgaatcag tgcagtgtga gctacttgat gaactctatg 2400
   attccttatg gcgtaaagag actcgaggac ttcgacgcca gtctgaagga cgcactgctc 2460
   aagtacatct acgataaccg cgggactctg atcggtcagg tggatcgcct aaaggacaaa 2520
   gtgaataaca ccctgagcac cgacataccc ttccaacttt ctaagtacgt agataaccag 2580
   cgtctcctgt caactttcac cgagtatata aagaacatta taaatacttc tattttgaac 2640
   ttgcggtatg aaagcaacca cctcattgat ctgtcaagat atgcatccaa gatcaatatt 2700
   gggtctaaag ttaatttcga ccctattgat aagaaccaaa ttcagctctt caacctggaa 2760
   tcatccaaaa tcgaggttat cctgaaaaat gccatcgtct acaactcaat gtacgagaac 2820
   ttcagcacat cattctggat ccggatacct aagtatttca attccatctc cctgaacaat 2880
   gagtacacga tcattaactg tatggagaac aatagcggct ggaaagtcag tctgaattac 2940
   ggtgaaataa tctggaccct ccaggacacc caggaaatca aacagagggt ggtcttcaag 3000
   tattcacaga tgatcaacat cagtgactat attaacaggt ggatctttgt gaccatcacc 3060
   aacaatcgac tcaacaattc caaaatctac atcaacggca ggctgatcga tcaaaagcca 3120
   atctcgaacc tggggaacat tcatgcttcc aataacatca tgtttaagct cgatggctgt 3180
   cgagacactc acagatatat ctggattaag tatttcaacc tctttgacaa ggagctcaac 3240
   gagaaagaga tcaaagacct atatgataat cagtctaaca gtggcatctt aaaggacttc 3300
   tggggagact acctacaata cgacaaaccc tattacatgc ttaacttgta tgacccaaat 3360
   aagtacgtcg atgtgaacaa tgtgggcatc cgcggataca tgtatctgaa gggtccccga 3420
   ggaagcgtga tgacaaccaa catttatctg aattcttcct tgtaccgcgg caccaaattc 3480
   attatcaaga aatatgcctc cggtaataag gacaacatag tgcggaacaa tgaccgtgtt 3540
   tacatcaatg tcgtggttaa gaataaggaa tatagactcg caaccaatgc tagtcaggcg 3600
   ggcgtggaaa agatactctc agctcttgaa atcccagacg tggggaatct ttcccaggtc 3660
   gtggtcatga agtccaagaa cgatcaggga atcactaata agtgcaagat gaacctgcag 3720
   gataacaatg gcaacgatat cggcttcatt ggcttccatc agtttaacaa tatcgccaaa 3780
   cttgtggcaa gtaactggta caacaggcag atagagagga gcagccggac actcggttgc 3840
   agctgggaat tcattccagt ggatgacggc tggggagaga ggccgctgtg a          3891
<210> 92
   <211> 3891
   <212> DNA
   <213> Artificial Sequence
<220>
   <223> BoNT/A, E. caballus-modified 2
<400> 92
   atgcccttcg tgaacaagca gttcaactac aaggaccccg tgaacggcgt ggacatcgcc 60
   tacatcaaga tccccaacgc cggccagatg cagcccgtga aggccttcaa gatccacaac 120
   aagatctggg tgatccccga gagggacacc ttcaccaacc ccgaggaggg cgacctgaac 180
   cccccccccg aggccaagca ggtgcccgtg agctactacg acagcaccta cctgagcacc 240
   gacaacgaga aggacaacta cctgaagggc gtgaccaagc tgttcgagag gatctacagc 300
   accgacctgg gcaggatgct gctgaccagc atcgtgaggg gcatcccctt ctggggcggc 360
   agcaccatcg acaccgagct gaaggtgatc gacaccaact gcatcaacgt gatccagccc 420
   gacggcagct acaggagcga ggagctgaac ctggtgatca tcggccccag cgccgacatc 480
   atccagttcg agtgcaagag cttcggccac gaggtgctga acctgaccag gaacggctac 540
   ggcagcaccc agtacatcag gttcagcccc gacttcacct tcggcttcga ggagagcctg 600
   gaggtggaca ccaaccccct gctgggcgcc ggcaagttcg ccaccgaccc cgccgtgacc 660
   ctggcccacg agctgatcca cgccggccac aggctgtacg gcatcgccat caaccccaac 720
   agggtgttca aggtgaacac caacgcctac tacgagatga gcggcctgga ggtgagcttc 780
   gaggagctga ggaccttcgg cggccacgac gccaagttca tcgacagcct gcaggagaac 840
   gagttcaggc tgtactacta caacaagttc aaggacatcg ccagcaccct gaacaaggcc 900
   aagagcatcg tgggcaccac cgccagcctg cagtacatga agaacgtgtt caaggagaag 960
   tacctgctga gcgaggacac cagcggcaag ttcagcgtgg acaagctgaa gttcgacaag 1020
   ctgtacaaga tgctgaccga gatctacacc gaggacaact tcgtgaagtt cttcaaggtg 1080
   ctgaacagga agacctacct gaacttcgac aaggccgtgt tcaagatcaa catcgtgccc 1140
   aaggtgaact acaccatcta cgacggcttc aacctgagga acaccaacct ggccgccaac 1200
   ttcaacggcc agaacaccga gatcaacaac atgaacttca ccaagctgaa gaacttcacc 1260
   ggcctgttcg agttctacaa gctgctgtgc gtgaggggca tcatcaccag caagaccaag 1320
   agcctggaca agggctacaa caaggccctg aacgacctgt gcatcaaggt gaacaactgg 1380
   gacctgttct tcagccccag cgaggacaac ttcaccaacg acctgaacaa gggcgaggag 1440
   atcaccagcg acaccaacat cgaggccgcc gaggagaaca tcagcctgga cctgatccag 1500
   cagtactacc tgaccttcaa cttcgacaac gagcccgaga acatcagcat cgagaacctg 1560
   agcagcgaca tcatcggcca gctggagctg atgcccaaca tcgagaggtt ccccaacggc 1620
   aagaagtacg agctggacaa gtacaccatg ttccactacc tgagggccca ggagttcgag 1680
   cacggcaaga gcaggatcgc cctgaccaac agcgtgaacg aggccctgct gaaccccagc 1740
   agggtgtaca ccttcttcag cagcgactac gtgaagaagg tgaacaaggc caccgaggcc 1800
   gccatgttcc tgggctgggt ggagcagctg gtgtacgact tcaccgacga gaccagcgag 1860
   gtgagcacca ccgacaagat cgccgacatc accatcatca tcccctacat cggccccgcc 1920
   ctgaacatcg gcaacatgct gtacaaggac gacttcgtgg gcgccctgat cttcagcggc 1980
   gccgtgatcc tgctggagtt catccccgag atcgccatcc ccgtgctggg caccttcgcc 2040
   ctggtgagct acatcgccaa caaggtgctg accgtgcaga ccatcgacaa cgccctgagc 2100
   aagaggaacg agaagtggga cgaggtgtac aagtacatcg tgaccaactg gctggccaag 2160
   gtgaacaccc agatcgacct gatcaggaag aagatgaagg aggccctgga gaaccaggcc 2220
   gaggccacca aggccatcat caactaccag tacaaccagt acaccgagga ggagaagaac 2280
   aacatcaact tcaacatcga cgacctgagc agcaagctga acgagagcat caacaaggcc 2340
   atgatcaaca tcaacaagtt cctgaaccag tgcagcgtga gctacctgat gaacagcatg 2400
   atcccctacg gcgtgaagag gctggaggac ttcgacgcca gcctgaagga cgccctgctg 2460
   aagtacatct acgacaacag gggcaccctg atcggccagg tggacaggct gaaggacaag 2520
   gtgaacaaca ccctgagcac cgacatcccc ttccagctga gcaagtacgt ggacaaccag 2580
   aggctgctga gcaccttcac cgagtacatc aagaacatca tcaacaccag catcctgaac 2640
   ctgaggtacg agagcaacca cctgatcgac ctgagcaggt acgccagcaa gatcaacatc 2700
   ggcagcaagg tgaacttcga ccccatcgac aagaaccaga tccagctgtt caacctggag 2760
   agcagcaaga tcgaggtgat cctgaagaac gccatcgtgt acaacagcat gtacgagaac 2820
   ttcagcacca gcttctggat caggatcccc aagtacttca acagcatcag cctgaacaac 2880
   gagtacacca tcatcaactg catggagaac aacagcggct ggaaggtgag cctgaactac 2940
   ggcgagatca tctggaccct gcaggacacc caggagatca agcagagggt ggtgttcaag 3000
   tacagccaga tgatcaacat cagcgactac atcaacaggt ggatcttcgt gaccatcacc 3060
   aacaacaggc tgaacaacag caagatctac atcaacggca ggctgatcga ccagaagccc 3120
   atcagcaacc tgggcaacat ccacgccagc aacaacatca tgttcaagct ggacggctgc 3180
   agggacaccc acaggtacat ctggatcaag tacttcaacc tgttcgacaa ggagctgaac 3240
   gagaaggaga tcaaggacct gtacgacaac cagagcaaca gcggcatcct gaaggacttc 3300
   tggggcgact acctgcagta cgacaagccc tactacatgc tgaacctgta cgaccccaac 3360
   aagtacgtgg acgtgaacaa cgtgggcatc aggggctaca tgtacctgaa gggccccagg 3420
   ggcagcgtga tgaccaccaa catctacctg aacagcagcc tgtacagggg caccaagttc 3480
   atcatcaaga agtacgccag cggcaacaag gacaacatcg tgaggaacaa cgacagggtg 3540
   tacatcaacg tggtggtgaa gaacaaggag tacaggctgg ccaccaacgc cagccaggcc 3600
   ggcgtggaga agatcctgag cgccctggag atccccgacg tgggcaacct gagccaggtg 3660
   gtggtgatga agagcaagaa cgaccagggc atcaccaaca agtgcaagat gaacctgcag 3720
   gacaacaacg gcaacgacat cggcttcatc ggcttccacc agttcaacaa catcgccaag 3780
   ctggtggcca gcaactggta caacaggcag atcgagagga gcagcaggac cctgggctgc 3840
   agctgggagt tcatccccgt ggacgacggc tggggcgaga ggcccctgtg a 3891
<210> 93
   <211> 3891
   <212> DNA
   <213> Artificial Sequence
<220>
   <223> BoNT/A, E. caballus-modified 3
<400> 93
   atgccattcg tcaataagca atttaactac aaggaccccg ttaacggagt ggatatcgcc 60
   tacatcaaaa tccccaatgc tggccagatg cagcctgtta aagccttcaa gattcataac 120
   aagatctggg ttattcctga gcgagacacc ttcaccaatc ctgaagaggg agacctcaac 180
   ccacctccag aagcaaagca ggtgccagtt agctactatg acagcaccta cctgtctacc 240
   gataacgaga aagacaatta cctgaaaggc gtgactaagc ttttcgaacg gatttactct 300
   actgacctcg ggaggatgct gttgacttcc atcgtgcgtg gcatcccctt ctggggcgga 360
   tccaccatcg atacagaact gaaagtgatc gatacaaatt gtattaacgt gatccagcct 420
   gacggaagct acaggagtga ggaactgaat ctggtgatca ttgggcccag cgccgacatt 480
   atccaattcg agtgtaagtc cttcggtcac gaagtgctga acctgacccg caacggctat 540
   ggttctaccc agtatatccg cttctcacca gacttcacct tcggcttcga agagtctctc 600
   gaggtggaca ccaaccccct cctgggtgct gggaagttcg caactgaccc agccgtcacc 660
   ctcgctcatg agctgattca cgctggccac cgcctgtatg gtatcgccat taaccctaac 720
   agagtcttca aagtgaacac taacgcctac tatgaaatgt ccggactgga agtcagtttt 780
   gaggaactta ggacattcgg cggacatgac gccaagttca ttgatagcct tcaggagaat 840
   gagttccgcc tgtactatta caataagttc aaggacatcg ccagtactct gaataaagct 900
   aaaagtatcg tgggcaccac agcctccctg cagtatatga agaacgtttt caaggagaag 960
   tacctgttga gtgaagatac ctctggaaag ttttccgtgg ataaactgaa attcgacaaa 1020
   ttgtacaaga tgctgacaga gatctacacc gaagataact ttgtgaagtt tttcaaagtg 1080
   ctgaatagaa agacatatct gaacttcgac aaagctgtct ttaaaatcaa catcgtcccc 1140
   aaagtgaatt ataccatcta cgacggtttt aatctgagga atacaaacct cgcagccaac 1200
   tttaacggac agaataccga gatcaacaat atgaatttta caaaacttaa gaactttacc 1260
   gggttgtttg aattttacaa gttgctgtgc gtcaggggga tcattacaag caagactaag 1320
   agcttggata agggctataa taaggccctc aatgatctct gcatcaaggt gaacaattgg 1380
   gatctgttct ttagccccag cgaggataat tttacaaacg acctcaataa gggagaagag 1440
   attacatcag ataccaatat tgaagccgct gaagagaaca tctccctgga tctgattcaa 1500
   cagtattacc tgactttcaa tttcgacaat gaacccgaaa acatctccat cgagaatctg 1560
   tcttccgata ttatcggcca gctcgagctg atgcctaaca tcgagcgctt tcccaatgga 1620
   aagaaatacg agctcgacaa gtacaccatg tttcactatc tcagggctca ggagttcgaa 1680
   cacggtaaga gcagaatcgc cttgaccaat tctgtgaacg aggccttgct gaacccctct 1740
   cgagtgtata ctttcttttc cagcgactac gtcaagaaag tgaacaaggc aaccgaggct 1800
   gccatgttcc tcgggtgggt ggagcagctc gtgtacgact tcaccgatga gacctctgag 1860
   gtgtcaacaa ctgacaagat cgcagacatc accattatca ttccctacat cggacctgct 1920
   ctgaacattg gcaacatgct gtataaagac gattttgttg gagccctgat tttcagcggc 1980
   gcagtcattc tgttggagtt catccccgag atcgccatcc ccgtcctggg tacattcgcc 2040
   ctggtctcat acatcgccaa caaagttctc actgtgcaaa ccattgacaa tgctctctca 2100
   aagcgtaacg agaaatggga cgaggtttac aagtatattg tcaccaactg gctggccaaa 2160
   gtgaacactc agatcgacct cattcggaaa aagatgaaag aggcccttga gaaccaggcc 2220
   gaggccacca aagccatcat taactaccag tataaccagt acactgaaga ggaaaagaat 2280
   aacattaact tcaacatcga tgacctttct tcaaaactca acgaatcaat caacaaagcc 2340
   atgatcaata ttaacaaatt ccttaaccag tgtagcgtca gctacctgat gaactccatg 2400
   atcccctacg gggtgaagag actggaagat tttgacgctt ccctgaagga tgcactcctg 2460
   aagtatatct acgataatcg cggcactctg atcgggcagg tcgaccggct gaaggacaaa 2520
   gtgaacaata ccttgtctac cgatatccca ttccagctta gcaagtacgt tgacaatcag 2580
   cgcttgctca gcacatttac cgagtatatt aagaatatta tcaatacaag tatcctgaat 2640
   ctgcgctacg agagcaatca tttgattgat ctgtcccggt acgcatccaa gatcaacatc 2700
   ggtagcaagg tcaactttga tcctattgac aagaatcaga tccaactgtt caacctggaa 2760
   agttcaaaaa ttgaagtcat ccttaagaac gccatcgtgt acaactcaat gtatgagaac 2820
   ttcagcacct cattttggat caggattccc aaatatttta atagtatcag ccttaacaat 2880
   gagtatacta tcattaactg catggagaat aactccgggt ggaaggtgtc cctcaattat 2940
   ggagagatca tttggactct ccaagacaca caagaaatca aacagagagt tgtgttcaag 3000
   tacagccaga tgatcaacat ctccgattac atcaaccggt ggatttttgt caccatcaca 3060
   aataaccgtc tgaacaattc caagatctac atcaacggcc gacttatcga tcagaagcct 3120
   atctcaaatc tggggaacat ccacgcttcc aataacatca tgttcaagtt ggacggctgc 3180
   cgagacaccc accggtacat ttggatcaag tatttcaacc tgtttgacaa ggagctgaac 3240
   gaaaaggaga tcaaagatct gtatgataat cagtcaaata gcggaatcct taaggacttc 3300
   tggggcgact acctgcagta cgacaagcca tattacatgt tgaacctgta cgatcctaat 3360
   aagtacgtcg acgtgaacaa tgtgggcatc agaggttata tgtacctgaa ggggcccaga 3420
   gggtctgtga tgaccactaa catttatctt aatagtagcc tgtatcgggg caccaaattt 3480
   atcatcaaaa agtacgctag cggaaacaaa gataacatcg tgaggaacaa tgacagagtg 3540
   tacatcaatg tggtcgtgaa gaacaaagag taccgtctgg caaccaatgc ctcccaagcc 3600
   ggagttgaga aaatcctgtc tgccctggaa atccctgacg tgggcaacct gagtcaggtg 3660
   gtggtgatga agtctaagaa cgatcagggc atcaccaaca aatgcaaaat gaaccttcaa 3720
   gacaataacg ggaacgatat cgggtttatc ggctttcatc agttcaataa catcgcaaag 3780
   ctcgtggcca gcaattggta caataggcaa atcgagcggt ctagtagaac cctcggctgt 3840
   agttgggagt ttatcccagt ggacgacggt tggggggaaa ggcccctgtg a          3891
<210> 94
   <211> 3891
   <212> DNA
   <213> Artificial Sequence
<220>
   <223> BoNT/A, C. aethiops-modified 1
<400> 94
   atgccttttg ttaataagca attcaattac aaagatcctg taaatggtgt ggacatcgcc 60
   tacatcaaga ttccaaacgc cggacagatg cagccagtta aggcattcaa gattcataac 120
   aaaatctggg tcatcccaga acgagacaca ttcaccaacc ctgaggaagg ggatcttaac 180
   ccgcccccag aagcaaagca agtgccagtc tcctactatg actctacgta cctatccaca 240
   gataatgaga aagacaacta cctaaagggc gtcacgaaac ttttcgaaag aatatattcc 300
   acagatctgg gccgcatgct tttgacttct attgttcggg gcatcccatt ctggggtggc 360
   agcactatag atacagagct gaaggttatt gacaccaact gcatcaacgt cattcagcct 420
   gatgggtcct accggtctga agagctgaac ctggtcatca ttggcccctc cgccgatata 480
   atccagtttg agtgcaagtc tttcggacat gaggtgctta atctgactcg caacgggtac 540
   ggatctacac agtatatccg cttttcaccg gatttcacgt ttggtttcga ggaatctttg 600
   gaggtcgata ccaatcctct acttggcgct gggaagtttg caaccgaccc agccgtgaca 660
   ctggcacacg agctgattca cgctggacat cgactatacg gtatcgcaat caatcccaat 720
   cgagtgttca aggtaaatac aaacgcttac tatgaaatga gcggattgga agtgagcttt 780
   gaggaactcc gaacgttcgg gggccacgat gctaaattca tagatagcct gcaggagaat 840
   gagttccgtc tgtattacta taataaattc aaagatatcg ccagtactct caacaaggca 900
   aagtcgatcg tggggactac agctagtctg caatacatga agaacgtttt taaggagaag 960
   tacttgctgt cagaagacac ctcagggaag ttttcggtgg acaagcttaa gtttgataaa 1020
   ttgtacaaga tgctcacaga aatatataca gaggacaact ttgtcaagtt ctttaaggtg 1080
   cttaacagaa agacctattt gaacttcgat aaagccgttt tcaaaatcaa tattgtccct 1140
   aaggtgaact atacaatcta cgacggattc aatcttagga atacgaacct ggctgcgaac 1200
   ttcaacggcc agaacacaga gatcaacaat atgaatttca caaagctcaa aaattttacg 1260
   ggtctattcg aattttataa actgctctgc gtcaggggca tcattactag taaaaccaaa 1320
   tccctcgata aagggtacaa taaagcccta aatgatctct gtataaaggt taacaattgg 1380
   gaccttttct tttcaccctc ggaggacaac ttcaccaatg atctgaataa aggggaagag 1440
   atcacctccg ataccaacat tgaagctgcg gaagagaata tcagcctcga cctaattcaa 1500
   cagtactatc tgaccttcaa ctttgacaat gaacccgaga atatctccat agagaacctg 1560
   agctcagaca taatcggaca gctcgagttg atgcctaata tcgaaaggtt ccccaacggg 1620
   aagaaatatg agttagacaa gtacacgatg tttcattatc tgagggctca agagtttgag 1680
   cacggcaagt cccggatcgc actaaccaac agtgtgaacg aggccctcct gaatccaagc 1740
   agagtatata ccttcttttc ttccgactac gtgaaaaagg ttaacaaagc gactgaggct 1800
   gcaatgttct taggttgggt ggagcagctg gtatacgact tcactgacga gacgagcgaa 1860
   gtctctacta cagataagat tgcagatatc acaatcatta taccttatat aggccccgcc 1920
   ctgaacattg gaaacatgtt atataaggat gactttgttg gggcactgat ctttagcggt 1980
   gccgtcattc ttctggagtt tatccccgag atcgccattc ccgtcctcgg aactttcgcc 2040
   ctcgtgagct acatcgcaaa taaggttctg acggtccaaa caatcgacaa cgctttatca 2100
   aaacgaaatg aaaaatggga cgaggtgtac aaatacattg tgactaattg gctggctaag 2160
   gtaaataccc agatcgacct catcaggaag aaaatgaagg aggctctgga aaatcaagcc 2220
   gaagctacca aagccatcat taattaccag tataatcagt atacggaaga ggaaaaaaat 2280
   aacataaact tcaacattga cgatctcagc tccaaattga acgagagcat caacaaggcg 2340
   atgatcaata tcaataaatt tcttaatcaa tgctccgtta gctatttgat gaacagcatg 2400
   atcccatacg gcgtaaaacg cttggaggat ttcgatgctt ctcttaagga cgccctgttg 2460
   aaatacattt atgataacag gggcacactg ataggccagg tcgatagatt aaaggataaa 2520
   gtgaacaata ccctctctac ggacattccc ttccagctta gtaagtacgt ggacaatcaa 2580
   cggttgctga gtacatttac agagtatatt aagaacatca taaataccag cattttgaac 2640
   ctccggtacg aatccaacca cctgattgac ctgagtcgtt atgccagtaa gattaacatc 2700
   ggctcgaaag ttaattttga tcccatcgac aaaaaccaga ttcagctgtt taacttggaa 2760
   agctccaaaa tcgaagtaat cctgaagaat gccatcgtgt ataactctat gtatgaaaat 2820
   tttagtacct cattttggat caggatcccc aagtacttca actctatttc actgaacaat 2880
   gaatatacta tcattaactg tatggagaat aactctgggt ggaaagtctc actgaactac 2940
   ggcgaaatta tatggactct gcaggacacc caggagatta agcagcgcgt ggtcttcaaa 3000
   tattctcaga tgattaatat aagcgactac attaaccgct ggatttttgt gacaataaca 3060
   aataaccgcc ttaataacag taagatctat attaacggga gactcatcga tcagaagccg 3120
   attagtaacc tgggaaacat acacgcctcc aataacatca tgttcaaact ggacggttgt 3180
   cgcgacaccc atcgttacat ctggatcaaa tatttcaatt tatttgataa agaattaaac 3240
   gagaaagaaa ttaaggacct ttacgataac cagtctaact ctggcattct gaaggacttt 3300
   tggggagact atcttcaata tgataaaccg tattacatgc tcaacctcta cgatcctaat 3360
   aagtacgtcg acgtcaataa cgtgggcatt agaggatata tgtatctgaa gggaccaaga 3420
   ggctcagtga tgactacgaa tatctacctg aacagctctc tctaccgggg cactaagttt 3480
   atcataaaga aatacgcgtc cggtaataag gacaacattg tgagaaacaa tgacagagtg 3540
   tatatcaacg tggttgtgaa gaataaagag tacaggctgg ccactaacgc ctcacaggct 3600
   ggagtggaga aaattcttag cgcactggag atcccagatg tgggaaacct gtcacaggtg 3660
   gtcgttatga agagcaagaa tgatcagggg atcaccaaca aatgtaaaat gaatctccag 3720
   gataacaatg gaaacgacat cgggttcatc gggttccacc aattcaataa catcgctaag 3780
   ctggtggcct caaattggta caatcgtcaa attgagcggt ccagtagaac cctcggttgc 3840
   tcctgggagt tcatcccagt tgacgatggc tggggcgagc ggcctctgtg a          3891
<210> 95
   <211> 3891
   <212> DNA
   <213> Artificial Sequence
<220>
   <223> BoNT/A, C. aethiops-modified 2
<400> 95
   atgccattcg tgaacaagca gttcaactac aaggacccag tgaacggcgt ggacatcgcc 60
   tacatcaaga tcccaaacgc cggccagatg cagccagtga aggccttcaa gatccacaac 120
   aagatctggg tgatcccaga gagagacacc ttcaccaacc cagaggaggg cgacctgaac 180
   ccaccaccag aggccaagca ggtgccagtg agctactacg acagcaccta cctgagcacc 240
   gacaacgaga aggacaacta cctgaagggc gtgaccaagc tgttcgagag aatctacagc 300
   accgacctgg gcagaatgct gctgaccagc atcgtgagag gcatcccatt ctggggcggc 360
   agcaccatcg acaccgagct gaaggtgatc gacaccaact gcatcaacgt gatccagcca 420
   gacggcagct acagaagcga ggagctgaac ctggtgatca tcggcccaag cgccgacatc 480
   atccagttcg agtgcaagag cttcggccac gaggtgctga acctgaccag aaacggctac 540
   ggcagcaccc agtacatcag attcagccca gacttcacct tcggcttcga ggagagcctg 600
   gaggtggaca ccaacccact gctgggcgcc ggcaagttcg ccaccgaccc agccgtgacc 660
   ctggcccacg agctgatcca cgccggccac agactgtacg gcatcgccat caacccaaac 720
   agagtgttca aggtgaacac caacgcctac tacgagatga gcggcctgga ggtgagcttc 780
   gaggagctga gaaccttcgg cggccacgac gccaagttca tcgacagcct gcaggagaac 840
   gagttcagac tgtactacta caacaagttc aaggacatcg ccagcaccct gaacaaggcc 900
   aagagcatcg tgggcaccac cgccagcctg cagtacatga agaacgtgtt caaggagaag 960
   tacctgctga gcgaggacac cagcggcaag ttcagcgtgg acaagctgaa gttcgacaag 1020
   ctgtacaaga tgctgaccga gatctacacc gaggacaact tcgtgaagtt cttcaaggtg 1080
   ctgaacagaa agacctacct gaacttcgac aaggccgtgt tcaagatcaa catcgtgcca 1140
   aaggtgaact acaccatcta cgacggcttc aacctgagaa acaccaacct ggccgccaac 1200
   ttcaacggcc agaacaccga gatcaacaac atgaacttca ccaagctgaa gaacttcacc 1260
   ggcctgttcg agttctacaa gctgctgtgc gtgagaggca tcatcaccag caagaccaag 1320
   agcctggaca agggctacaa caaggccctg aacgacctgt gcatcaaggt gaacaactgg 1380
   gacctgttct tcagcccaag cgaggacaac ttcaccaacg acctgaacaa gggcgaggag 1440
   atcaccagcg acaccaacat cgaggccgcc gaggagaaca tcagcctgga cctgatccag 1500
   cagtactacc tgaccttcaa cttcgacaac gagccagaga acatcagcat cgagaacctg 1560
   agcagcgaca tcatcggcca gctggagctg atgccaaaca tcgagagatt cccaaacggc 1620
   aagaagtacg agctggacaa gtacaccatg ttccactacc tgagagccca ggagttcgag 1680
   cacggcaaga gcagaatcgc cctgaccaac agcgtgaacg aggccctgct gaacccaagc 1740
   agagtgtaca ccttcttcag cagcgactac gtgaagaagg tgaacaaggc caccgaggcc 1800
   gccatgttcc tgggctgggt ggagcagctg gtgtacgact tcaccgacga gaccagcgag 1860
   gtgagcacca ccgacaagat cgccgacatc accatcatca tcccatacat cggcccagcc 1920
   ctgaacatcg gcaacatgct gtacaaggac gacttcgtgg gcgccctgat cttcagcggc 1980
   gccgtgatcc tgctggagtt catcccagag atcgccatcc cagtgctggg caccttcgcc 2040
   ctggtgagct acatcgccaa caaggtgctg accgtgcaga ccatcgacaa cgccctgagc 2100
   aagagaaacg agaagtggga cgaggtgtac aagtacatcg tgaccaactg gctggccaag 2160
   gtgaacaccc agatcgacct gatcagaaag aagatgaagg aggccctgga gaaccaggcc 2220
   gaggccacca aggccatcat caactaccag tacaaccagt acaccgagga ggagaagaac 2280
   aacatcaact tcaacatcga cgacctgagc agcaagctga acgagagcat caacaaggcc 2340
   atgatcaaca tcaacaagtt cctgaaccag tgcagcgtga gctacctgat gaacagcatg 2400
   atcccatacg gcgtgaagag actggaggac ttcgacgcca gcctgaagga cgccctgctg 2460
   aagtacatct acgacaacag aggcaccctg atcggccagg tggacagact gaaggacaag 2520
   gtgaacaaca ccctgagcac cgacatccca ttccagctga gcaagtacgt ggacaaccag 2580
   agactgctga gcaccttcac cgagtacatc aagaacatca tcaacaccag catcctgaac 2640
   ctgagatacg agagcaacca cctgatcgac ctgagcagat acgccagcaa gatcaacatc 2700
   ggcagcaagg tgaacttcga cccaatcgac aagaaccaga tccagctgtt caacctggag 2760
   agcagcaaga tcgaggtgat cctgaagaac gccatcgtgt acaacagcat gtacgagaac 2820
   ttcagcacca gcttctggat cagaatccca aagtacttca acagcatcag cctgaacaac 2880
   gagtacacca tcatcaactg catggagaac aacagcggct ggaaggtgag cctgaactac 2940
   ggcgagatca tctggaccct gcaggacacc caggagatca agcagagagt ggtgttcaag 3000
   tacagccaga tgatcaacat cagcgactac atcaacagat ggatcttcgt gaccatcacc 3060
   aacaacagac tgaacaacag caagatctac atcaacggca gactgatcga ccagaagcca 3120
   atcagcaacc tgggcaacat ccacgccagc aacaacatca tgttcaagct ggacggctgc 3180
   agagacaccc acagatacat ctggatcaag tacttcaacc tgttcgacaa ggagctgaac 3240
   gagaaggaga tcaaggacct gtacgacaac cagagcaaca gcggcatcct gaaggacttc 3300
   tggggcgact acctgcagta cgacaagcca tactacatgc tgaacctgta cgacccaaac 3360
   aagtacgtgg acgtgaacaa cgtgggcatc agaggctaca tgtacctgaa gggcccaaga 3420
   ggcagcgtga tgaccaccaa catctacctg aacagcagcc tgtacagagg caccaagttc 3480
   atcatcaaga agtacgccag cggcaacaag gacaacatcg tgagaaacaa cgacagagtg 3540
   tacatcaacg tggtggtgaa gaacaaggag tacagactgg ccaccaacgc cagccaggcc 3600
   ggcgtggaga agatcctgag cgccctggag atcccagacg tgggcaacct gagccaggtg 3660
   gtggtgatga agagcaagaa cgaccagggc atcaccaaca agtgcaagat gaacctgcag 3720
   gacaacaacg gcaacgacat cggcttcatc ggcttccacc agttcaacaa catcgccaag 3780
   ctggtggcca gcaactggta caacagacag atcgagagaa gcagcagaac cctgggctgc 3840
   agctgggagt tcatcccagt ggacgacggc tggggcgaga gaccactgtg a 3891
<210> 96
   <211> 3891
   <212> DNA
   <213> Artificial Sequence
<220>
   <223> BoNT/A, C. aethiops-modified 3
<400> 96
   atgccatttg ttaacaagca gttcaactat aaagatcctg tgaatggggt tgacattgcc 60
   tacatcaaaa tccctaacgc cggccagatg cagccagtga aagcatttaa gatccacaat 120
   aaaatctggg tcattcctga acgcgatacc ttcaccaacc ctgaagaggg agatctgaat 180
   cctccccctg aggctaagca ggttcccgtg tcttactatg atagcaccta cctcagtacc 240
   gataacgaaa aggataatta ccttaagggc gtgaccaaac tcttcgagag gatttactct 300
   actgacctgg gtagaatgtt gctgactagc atcgttagag gaatcccttt ctggggcgga 360
   tccaccatcg acacagaact taaggtgatc gacacaaact gcatcaatgt tattcagcca 420
   gacggttcct atagaagcga ggaactgaat ctggtgatca ttgggccctc tgcagacatt 480
   atccaattcg agtgtaagtc tttcggccac gaggtgctga acctgacccg caatggttac 540
   ggcagcactc agtacatccg cttttctcct gacttcactt ttggattcga agagtccctg 600
   gaggtcgata ccaaccctct tctgggcgct ggcaagttcg ccacggaccc cgctgtgaca 660
   ctggcccacg agttgatcca tgctggacac agactgtacg gcattgccat taaccccaac 720
   cgcgtgttca aagtgaatac taacgcctac tatgagatga gtggtctgga agtgtccttc 780
   gaagagctca ggaccttcgg gggtcacgac gccaaattta tcgactcact tcaagaaaat 840
   gagttcaggc tgtattacta taataagttc aaagatatcg ccagtacact caacaaggca 900
   aaatctatcg tcggcacgac agcctctctc caatacatga aaaatgtctt caaggagaaa 960
   tacctcttga gcgaagacac gagcggaaaa ttctccgtgg ataaactcaa attcgacaaa 1020
   ctttataaga tgctgacgga aatctataca gaagacaatt tcgtgaaatt tttcaaagtt 1080
   ctgaatagga agacctatct gaactttgac aaggcagttt tcaagatcaa cattgtcccc 1140
   aaagtgaact atactatcta cgatggcttt aacttgcgaa atactaatct ggccgctaat 1200
   tttaacggac aaaatactga aatcaataac atgaatttta caaaactgaa aaactttacg 1260
   ggccttttcg agttctataa actcctgtgt gtccgcggta tcattacttc taaaacgaaa 1320
   agtctggata agggatataa caaggctctc aacgacctgt gcattaaggt taacaattgg 1380
   gacttgttct ttagtccaag cgaagacaac ttcactaatg atcttaacaa aggcgaggaa 1440
   atcaccagcg acacgaacat tgaagccgca gaggaaaata tctctctcga ccttatccag 1500
   caatattact tgacattcaa ctttgacaac gaaccagaga atatttccat cgagaatttg 1560
   tcctcagaca tcattggcca gttggaactg atgccaaata tcgagaggtt tcccaacggc 1620
   aaaaagtatg agctggacaa gtatacgatg tttcactacc tgcgcgctca ggaatttgag 1680
   catgggaaga gccgcattgc cctgaccaac agcgtcaacg aagccctgtt gaatccaagt 1740
   cgtgtgtaca catttttcag tagcgactac gtgaagaaag tgaataaagc tactgaggcc 1800
   gcaatgtttc tggggtgggt cgaacagctg gtgtatgatt ttaccgacga gacctctgag 1860
   gtgagcacaa cggacaaaat cgctgacatc acaatcatta tcccctacat cggtccagca 1920
   ctgaatatcg ggaatatgct gtacaaggac gatttcgtcg gcgcactcat tttctccggc 1980
   gctgtcatcc tgttggagtt tatccctgaa atcgccattc cagtgctggg gacatttgcc 2040
   ctggttagct atatcgctaa taaggtgctg accgtgcaaa caattgataa tgccctgtct 2100
   aaacggaacg aaaaatggga tgaggtgtac aagtatatcg tgacaaactg gcttgccaaa 2160
   gtgaacacac agattgatct catccggaag aaaatgaagg aggcactcga gaaccaggcc 2220
   gaagccacta aagcaatcat taactatcag tacaaccagt ataccgagga agagaagaat 2280
   aacatcaact ttaacatcga cgatctttcc tctaagctta acgagtccat caacaaggcc 2340
   atgattaata ttaataaatt tctgaaccaa tgcagcgttt catacctgat gaatagtatg 2400
   atcccctacg gagtcaagag actggaggac ttcgatgcct ctttgaagga tgctcttctc 2460
   aagtatatct atgacaacag agggacactg attgggcagg tggatcgcct caaagataaa 2520
   gttaacaata cactgagtac tgatattccc tttcaactca gcaaatatgt ggataatcaa 2580
   cggcttctct caaccttcac cgagtacatt aagaatatta tcaacactag cattctgaac 2640
   ctcaggtacg aaagcaacca tttgattgac ctgtcccgtt atgcatcaaa gatcaatatt 2700
   ggctcaaagg tgaacttcga tccaatcgac aaaaatcaga tccagttgtt caacctggag 2760
   tcctctaaga ttgaagttat ccttaagaac gctatcgtgt acaacagcat gtatgagaat 2820
   ttttctacat cattttggat tcgtatcccc aaatatttta attcaatcag cctcaataac 2880
   gagtatacga tcattaactg catggagaat aactcaggtt ggaaagtgag cctgaactac 2940
   ggcgagatca tttggacgct gcaggatact caggaaatca agcagcgagt tgtgtttaag 3000
   tactcccaga tgattaacat cagcgattac atcaatcggt ggatcttcgt gacgatcacc 3060
   aacaatagac tgaataactc caagatttat atcaatggca gactgattga tcagaaacca 3120
   atctccaatc tgggcaatat ccatgcaagt aataacatca tgttcaagct ggatggatgt 3180
   agggacaccc acagatacat ctggattaag tattttaacc tgtttgataa ggagcttaat 3240
   gaaaaggaga tcaaggatct ttacgacaat cagtccaatt caggaatcct gaaagatttc 3300
   tggggtgact acctgcagta cgacaagcca tactatatgc tgaatctgta tgacccaaat 3360
   aagtacgtgg atgttaacaa tgtggggatc cgagggtaca tgtatctcaa gggaccacgt 3420
   ggtagtgtga tgacaaccaa catctacctg aacagctctc tctaccgagg cacaaagttc 3480
   atcattaaga aatacgcctc tggaaataag gacaatattg tgcggaacaa tgatcgagtc 3540
   tacatcaacg ttgtcgtgaa gaacaaggaa tatcggctcg ccacaaatgc ttcacaggcc 3600
   ggggtggaga aaatcctgag cgctcttgag atcccagatg tcgggaatct gtcccaggtc 3660
   gtcgtcatga agagtaaaaa cgatcaaggc atcaccaaca agtgtaagat gaacctgcaa 3720
   gacaacaatg ggaacgacat tgggttcatc ggattccatc aattcaacaa catcgctaaa 3780
   ctggtcgcca gcaactggta taaccggcag atcgagcggt catccaggac tctgggatgc 3840
   tcatgggagt ttatcccagt cgacgatggg tggggagaga gacccttgtg a          3891
<210> 97
   <211> 3891
   <212> DNA
   <213> Artificial Sequence
<220>
   <223> BoNT/A, H. sapiens-modified 1
<400> 97
   atgccgtttg tgaataagca gttcaactat aaggatcctg tgaacggtgt ggacatcgct 60
   tatattaaga tcccaaatgc aggccagatg cagcccgtga aggcttttaa gatacacaac 120
   aaaatctggg tcatcccaga gcgcgacaca ttcacgaatc cagaagaggg cgacctcaat 180
   ccccctccag aagctaaaca ggtcccagtg agttattacg acagtaccta tttgtccact 240
   gataacgaga aagacaatta tctgaagggc gtgacaaagc tgttcgaaag aatatacagt 300
   accgatctcg ggagaatgct cctgacatcc attgtccggg ggattccgtt ttggggtgga 360
   agtaccatcg acaccgaact gaaggtgatt gatacaaact gtataaacgt tatccagccc 420
   gacggaagct ataggtccga ggaactgaat ctggtgatca ttggaccttc tgccgacatt 480
   atccagttcg aatgtaagtc cttcggccat gaggttctga acctgactcg caacggatac 540
   gggagtaccc aatatattag attctccccc gactttacct tcggatttga agagtcactc 600
   gaggtggaca cgaatcccct ccttggagct gggaaatttg ccaccgatcc agccgtgact 660
   ctggcccatg aactcataca cgccgggcat aggctgtacg ggatcgcaat caatcccaat 720
   cgagttttta aggtcaatac taacgcatac tatgaaatgt ccgggctgga ggtctcattt 780
   gaagagcttc gaacatttgg cgggcacgat gccaaattca tcgactctct gcaggaaaat 840
   gagttcagac tgtattacta taataaattc aaagatatcg cctccacact caacaaggcc 900
   aaatccatcg tgggtacaac tgctagcctg cagtacatga agaatgtgtt taaagagaaa 960
   taccttctct ctgaagatac cagcgggaaa ttttctgtcg ataagctcaa gtttgacaag 1020
   ctttacaaaa tgctgacaga aatctacaca gaagataact tcgtaaagtt ctttaaagta 1080
   ttgaaccgca aaacctattt gaactttgac aaggcagtct tcaaaattaa catcgtgcct 1140
   aaagtcaact atactatcta tgacggtttt aatctgcgta atactaacct ggctgcaaac 1200
   tttaacggtc agaacaccga aatcaataac atgaacttca caaaactgaa gaatttcacg 1260
   gggctgtttg agttctataa gctgttgtgc gttcgtggca tcattacatc caaaactaag 1320
   tcattggaca aaggctataa taaagctctg aatgacctct gcatcaaggt gaacaattgg 1380
   gacctgttct ttagcccttc cgaagacaat tttacgaatg acctgaataa gggcgaggaa 1440
   atcacatctg ataccaacat tgaggccgca gaggaaaaca tatcccttga cctgattcaa 1500
   cagtactatt tgaccttcaa cttcgataat gagcccgaaa atatcagcat tgaaaacctt 1560
   tcctctgaca tcattggtca gctggaactg atgccaaata ttgaaaggtt tccgaatgga 1620
   aaaaagtacg agcttgataa gtacacgatg ttccattacc tcagggcaca agagttcgag 1680
   cacggaaagt cccgcatcgc actgactaac tctgtcaatg aggccctgct caacccatct 1740
   agagtctaca cttttttctc cagcgactac gtaaagaaag tgaacaaggc caccgaggct 1800
   gcaatgtttc tgggctgggt cgaacagctg gtatacgact tcacagatga gacaagcgaa 1860
   gtctccacta ccgacaaaat tgccgacatt actattatca ttccatacat tggacctgct 1920
   ctgaacatcg gcaatatgct ttacaaagat gacttcgtgg gggcactgat attctccggc 1980
   gctgtgatct tgctggagtt tattcctgag attgcaatcc ccgtgctggg taccttcgct 2040
   ctcgtgtctt acattgctaa taaagtgctg acggtgcaga cgatcgataa tgccctctct 2100
   aaaagaaatg agaagtggga tgaagtgtac aagtacattg tgacaaactg gctcgccaaa 2160
   gtaaacacac agatcgacct gatacgaaag aaaatgaaag aggccctgga gaatcaagcc 2220
   gaggccacca aggccatcat taactaccaa tacaatcagt acactgagga agagaagaat 2280
   aacattaact tcaatatcga tgacctttct tcaaaactga atgagagcat caataaggcc 2340
   atgataaata ttaacaaatt cctgaatcaa tgttctgtta gttacctgat gaatagcatg 2400
   atcccttacg gcgtgaagag gcttgaggat ttcgacgcct cactgaagga tgctctgctt 2460
   aagtatatct acgataatag gggcaccttg atcgggcagg tagataggct gaaggataag 2520
   gtgaacaata ctttgtcaac cgatatccca tttcaactga gcaagtatgt ggataaccag 2580
   cgcctgttgt ctacattcac agagtatatc aaaaacatca ttaacacttc aattttgaac 2640
   ctgaggtatg aatcaaacca cttgattgac ctcagcagat acgcttctaa gataaatatt 2700
   ggaagtaagg ttaacttcga cccgatcgat aagaaccaga tccagctgtt taatttggag 2760
   tccagtaaaa tcgaagtcat actgaaaaac gccatcgttt ataacagtat gtacgagaat 2820
   ttttctacca gcttctggat acgcattccc aagtacttta actctatctc cctgaataac 2880
   gagtatacca ttatcaattg catggagaat aactctggtt ggaaagtttc actcaattat 2940
   ggagaaatta tctggaccct gcaggatacg caggaaatca agcagcgggt agtcttcaag 3000
   tacagccaga tgattaacat ttctgactac attaaccgat ggatcttcgt caccataacc 3060
   aataaccgcc tgaacaattc caaaatttac ataaacggtc gcctgatcga tcagaagcct 3120
   ataagcaacc tgggcaacat ccatgccagc aacaatataa tgtttaagct cgatggatgt 3180
   cgggacacac acagatatat ttggataaag tattttaact tgtttgataa ggagctcaac 3240
   gagaaagaga tcaaagacct gtacgacaat cagtccaata gcggaattct gaaggatttt 3300
   tggggtgatt atctccagta tgacaaaccc tactatatgc tcaacctgta cgaccccaat 3360
   aaatacgttg atgtgaacaa tgttgggata cggggctata tgtatcttaa gggcccaagg 3420
   ggtagcgtta tgactaccaa catttatctt aacagcagtc tgtatcgggg aaccaagttc 3480
   attatcaaaa agtacgcatc cggcaataag gataatatcg tccgaaacaa tgatagagtg 3540
   tacatcaacg tagtggtcaa gaacaaagag taccggcttg ctaccaatgc cagtcaggct 3600
   ggcgtggaga aaattctgag tgcactggag attcctgacg ttggcaactt gagtcaggtg 3660
   gttgtgatga agtccaagaa cgaccaagga atcactaata agtgcaagat gaacctgcaa 3720
   gacaataacg gcaacgacat aggcttcata ggattccacc aattcaacaa tatagctaaa 3780
   ttggtggcct caaactggta taaccggcag atcgaacggt cctcacgtac actgggttgc 3840
   tcatgggagt ttatccccgt ggacgatggc tggggtgagc ggcctctttg a          3891
<210> 98
   <211> 3891
   <212> DNA
   <213> Artificial Sequence
<220>
   <223> BoNT/A, H. sapiens-modified 2
<400> 98
   atgcccttcg tgaacaagca gttcaactac aaggaccccg tgaacggcgt ggacatcgcc 60
   tacatcaaga tccccaacgc cggccagatg cagcccgtga aggccttcaa gatccacaac 120
   aagatctggg tgatccccga gagagacacc ttcaccaacc ccgaggaggg cgacctgaac 180
   cccccccccg aggccaagca ggtgcccgtg tcctactacg actccaccta cctgtccacc 240
   gacaacgaga aggacaacta cctgaagggc gtgaccaagc tgttcgagag aatctactcc 300
   accgacctgg gcagaatgct gctgacctcc atcgtgagag gcatcccctt ctggggcggc 360
   tccaccatcg acaccgagct gaaggtgatc gacaccaact gcatcaacgt gatccagccc 420
   gacggctcct acagatccga ggagctgaac ctggtgatca tcggcccctc cgccgacatc 480
   atccagttcg agtgcaagtc cttcggccac gaggtgctga acctgaccag aaacggctac 540
   ggctccaccc agtacatcag attctccccc gacttcacct tcggcttcga ggagtccctg 600
   gaggtggaca ccaaccccct gctgggcgcc ggcaagttcg ccaccgaccc cgccgtgacc 660
   ctggcccacg agctgatcca cgccggccac agactgtacg gcatcgccat caaccccaac 720
   agagtgttca aggtgaacac caacgcctac tacgagatgt ccggcctgga ggtgtccttc 780
   gaggagctga gaaccttcgg cggccacgac gccaagttca tcgactccct gcaggagaac 840
   gagttcagac tgtactacta caacaagttc aaggacatcg cctccaccct gaacaaggcc 900
   aagtccatcg tgggcaccac cgcctccctg cagtacatga agaacgtgtt caaggagaag 960
   tacctgctgt ccgaggacac ctccggcaag ttctccgtgg acaagctgaa gttcgacaag 1020
   ctgtacaaga tgctgaccga gatctacacc gaggacaact tcgtgaagtt cttcaaggtg 1080
   ctgaacagaa agacctacct gaacttcgac aaggccgtgt tcaagatcaa catcgtgccc 1140
   aaggtgaact acaccatcta cgacggcttc aacctgagaa acaccaacct ggccgccaac 1200
   ttcaacggcc agaacaccga gatcaacaac atgaacttca ccaagctgaa gaacttcacc 1260
   ggcctgttcg agttctacaa gctgctgtgc gtgagaggca tcatcacctc caagaccaag 1320
   tccctggaca agggctacaa caaggccctg aacgacctgt gcatcaaggt gaacaactgg 1380
   gacctgttct tctccccctc cgaggacaac ttcaccaacg acctgaacaa gggcgaggag 1440
   atcacctccg acaccaacat cgaggccgcc gaggagaaca tctccctgga cctgatccag 1500
   cagtactacc tgaccttcaa cttcgacaac gagcccgaga acatctccat cgagaacctg 1560
   tcctccgaca tcatcggcca gctggagctg atgcccaaca tcgagagatt ccccaacggc 1620
   aagaagtacg agctggacaa gtacaccatg ttccactacc tgagagccca ggagttcgag 1680
   cacggcaagt ccagaatcgc cctgaccaac tccgtgaacg aggccctgct gaacccctcc 1740
   agagtgtaca ccttcttctc ctccgactac gtgaagaagg tgaacaaggc caccgaggcc 1800
   gccatgttcc tgggctgggt ggagcagctg gtgtacgact tcaccgacga gacctccgag 1860
   gtgtccacca ccgacaagat cgccgacatc accatcatca tcccctacat cggccccgcc 1920
   ctgaacatcg gcaacatgct gtacaaggac gacttcgtgg gcgccctgat cttctccggc 1980
   gccgtgatcc tgctggagtt catccccgag atcgccatcc ccgtgctggg caccttcgcc 2040
   ctggtgtcct acatcgccaa caaggtgctg accgtgcaga ccatcgacaa cgccctgtcc 2100
   aagagaaacg agaagtggga cgaggtgtac aagtacatcg tgaccaactg gctggccaag 2160
   gtgaacaccc agatcgacct gatcagaaag aagatgaagg aggccctgga gaaccaggcc 2220
   gaggccacca aggccatcat caactaccag tacaaccagt acaccgagga ggagaagaac 2280
   aacatcaact tcaacatcga cgacctgtcc tccaagctga acgagtccat caacaaggcc 2340
   atgatcaaca tcaacaagtt cctgaaccag tgctccgtgt cctacctgat gaactccatg 2400
   atcccctacg gcgtgaagag actggaggac ttcgacgcct ccctgaagga cgccctgctg 2460
   aagtacatct acgacaacag aggcaccctg atcggccagg tggacagact gaaggacaag 2520
   gtgaacaaca ccctgtccac cgacatcccc ttccagctgt ccaagtacgt ggacaaccag 2580
   agactgctgt ccaccttcac cgagtacatc aagaacatca tcaacacctc catcctgaac 2640
   ctgagatacg agtccaacca cctgatcgac ctgtccagat acgcctccaa gatcaacatc 2700
   ggctccaagg tgaacttcga ccccatcgac aagaaccaga tccagctgtt caacctggag 2760
   tcctccaaga tcgaggtgat cctgaagaac gccatcgtgt acaactccat gtacgagaac 2820
   ttctccacct ccttctggat cagaatcccc aagtacttca actccatctc cctgaacaac 2880
   gagtacacca tcatcaactg catggagaac aactccggct ggaaggtgtc cctgaactac 2940
   ggcgagatca tctggaccct gcaggacacc caggagatca agcagagagt ggtgttcaag 3000
   tactcccaga tgatcaacat ctccgactac atcaacagat ggatcttcgt gaccatcacc 3060
   aacaacagac tgaacaactc caagatctac atcaacggca gactgatcga ccagaagccc 3120
   atctccaacc tgggcaacat ccacgcctcc aacaacatca tgttcaagct ggacggctgc 3180
   agagacaccc acagatacat ctggatcaag tacttcaacc tgttcgacaa ggagctgaac 3240
   gagaaggaga tcaaggacct gtacgacaac cagtccaact ccggcatcct gaaggacttc 3300
   tggggcgact acctgcagta cgacaagccc tactacatgc tgaacctgta cgaccccaac 3360
   aagtacgtgg acgtgaacaa cgtgggcatc agaggctaca tgtacctgaa gggccccaga 3420
   ggctccgtga tgaccaccaa catctacctg aactcctccc tgtacagagg caccaagttc 3480
   atcatcaaga agtacgcctc cggcaacaag gacaacatcg tgagaaacaa cgacagagtg 3540
   tacatcaacg tggtggtgaa gaacaaggag tacagactgg ccaccaacgc ctcccaggcc 3600
   ggcgtggaga agatcctgtc cgccctggag atccccgacg tgggcaacct gtcccaggtg 3660
   gtggtgatga agtccaagaa cgaccagggc atcaccaaca agtgcaagat gaacctgcag 3720
   gacaacaacg gcaacgacat cggcttcatc ggcttccacc agttcaacaa catcgccaag 3780
   ctggtggcct ccaactggta caacagacag atcgagagat cctccagaac cctgggctgc 3840
   tcctgggagt tcatccccgt ggacgacggc tggggcgaga gacccctgtg a          3891
<210> 99
   <211> 3891
   <212> DNA
   <213> Artificial Sequence
<220>
   <223> BoNT/A, H. sapiens-modified 3
<400> 99
   atgccctttg tgaataagca gtttaattat aaggatccag tcaacggcgt tgatatcgcc 60
   tacataaaaa tccctaacgc cggccagatg cagccagtta aggccttcaa gattcacaat 120
   aaaatctggg ttatcccaga gagggacacc tttaccaacc ctgaggaggg cgatctgaat 180
   ccaccccctg aggccaagca ggtgcctgtg tcctattacg actccaccta tctgtccacc 240
   gacaacgaga aggataatta cctgaagggc gtcaccaagc tgtttgagag aatatactcc 300
   acagatctgg gccggatgct gctgacttcc atagtgagag gcatcccctt ttggggcggc 360
   tccaccatag ataccgagct gaaagttata gacaccaact gcatcaacgt catccagccc 420
   gatggctcct ataggtccga ggagctgaat ctggtgatca ttggcccatc cgccgatatt 480
   atccaattcg agtgcaaatc ctttggccac gaggtcctga atctgacaag aaatggctac 540
   ggctccacac agtatattcg attttcccct gattttacat tcggctttga ggagtccctg 600
   gaggtggaca ccaatcccct gctgggcgcc ggcaaatttg ccaccgaccc agccgtgacc 660
   ctggcccatg agctgatcca cgccggccac cgcctgtacg gcattgccat caatcccaat 720
   agggtgttta aggtcaacac caacgcctac tatgagatgt ccggcctgga ggtgtccttt 780
   gaggagctgc gaacctttgg cggccatgat gccaagttca ttgactccct gcaggagaat 840
   gagttccgcc tgtactatta caataaattt aaagatattg cctccacact gaataaggcc 900
   aagtccattg tgggcaccac agcctccctg caatacatga agaacgtgtt caaggagaag 960
   tacctgctgt ccgaggacac atccggcaaa ttttccgtgg acaagctgaa gtttgacaag 1020
   ctgtacaaaa tgctgactga gatttatact gaggataact tcgtgaagtt ctttaaagtg 1080
   ctgaaccgca aaacctacct gaattttgac aaggccgttt ttaaaattaa catagtgcca 1140
   aaggtgaact acacaattta tgacggcttc aacctgcgga acaccaacct ggccgccaac 1200
   ttcaatggcc agaacacaga gatcaacaat atgaatttta caaagctgaa gaacttcacc 1260
   ggcctgttcg agttctacaa gctgctgtgc gtgaggggca ttatcacctc caaaactaag 1320
   tccctggata agggctacaa caaggccctg aacgacctgt gcatcaaagt caataactgg 1380
   gacctgtttt tctccccttc cgaggataac tttaccaatg acctgaataa aggcgaggag 1440
   ataacttccg acactaacat cgaggccgcc gaggagaata tttccctgga tctgattcag 1500
   caatactatc tgaccttcaa cttcgacaat gagcccgaga acatttccat cgagaatctg 1560
   tcctccgata tcattggcca gctggagctg atgcccaaca ttgagcgatt ccccaatggc 1620
   aagaaatacg agctggataa gtataccatg ttccactatc tgagggccca ggagttcgag 1680
   catggcaaat ccagaatcgc cctgactaac tccgtcaatg aggccctgct gaatccttcc 1740
   agagtctata ctttcttttc ctccgactac gtgaaaaagg tgaataaagc caccgaggcc 1800
   gccatgttcc tgggctgggt cgagcagctg gtgtatgact tcactgatga gacctccgag 1860
   gtctccacca ctgacaaaat cgccgacata acaataatta tcccctacat cggccctgcc 1920
   ctgaatatcg gcaatatgct gtataaggat gacttcgtgg gcgccctgat tttctccggc 1980
   gccgtgatcc tgctggagtt cattccagag atcgccatcc ctgtgctggg cactttcgcc 2040
   ctggtgtcct atatcgccaa caaggtcctg acagtgcaga ccatcgacaa tgccctgtcc 2100
   aaaaggaatg agaaatggga cgaggtttat aagtatatcg tgactaactg gctggccaag 2160
   gtgaacactc agattgatct gatacgcaag aaaatgaagg aggccctgga gaaccaggcc 2220
   gaggccacaa aggccattat aaactatcaa tacaaccagt acacagagga ggagaaaaat 2280
   aacatcaatt tcaatatcga cgatctgtcc tccaagctga atgagtccat caacaaagcc 2340
   atgataaata tcaataagtt cctgaaccag tgttccgtct cctatctgat gaactccatg 2400
   attccctacg gcgtgaaacg cctggaggac ttcgacgcct ccctgaaaga cgccctgctg 2460
   aaatacattt acgataacag aggcacactg attggccaag tggatagact gaaagacaaa 2520
   gtgaacaata ccctgtccac tgacattccc tttcagctgt ccaagtacgt tgacaatcag 2580
   aggctgctgt ccaccttcac cgagtacatc aagaacatca ttaatacttc catactgaac 2640
   ctgcggtatg agtccaatca tctgatcgat ctgtccagat atgcctccaa gataaatatc 2700
   ggctccaagg ttaattttga tcctattgac aagaatcaaa tacaactgtt taacctggag 2760
   tcctccaaaa ttgaggttat cctgaaaaac gccatcgtgt acaactccat gtatgagaac 2820
   ttctccacct ccttctggat acggatccca aagtatttta actccatatc cctgaataac 2880
   gagtacacaa tcattaattg catggagaac aattccggct ggaaggtgtc cctgaactac 2940
   ggcgagatca tttggacact gcaggatacc caggagataa agcagcgagt ggtctttaaa 3000
   tattcccaga tgatcaacat ttccgattac attaacaggt ggatttttgt caccatcacc 3060
   aacaatcgcc tgaataactc caaaatttat atcaacggcc ggctgatcga tcagaaaccc 3120
   atctccaatc tgggcaatat ccacgcctcc aacaatataa tgttcaagct ggatggctgt 3180
   agagacaccc atagatatat ttggattaag tatttcaacc tgtttgacaa ggagctgaac 3240
   gagaaagaga ttaaggacct gtatgataat caatccaact ccggcatcct gaaggatttc 3300
   tggggcgatt acctgcagta cgacaaacca tactatatgc tgaacctgta cgatcccaac 3360
   aagtacgttg acgtcaacaa tgtgggcatc cgcggctata tgtacctgaa aggccctagg 3420
   ggctccgtga tgactaccaa catctacctg aactcctccc tgtaccgggg cacaaagttc 3480
   atcataaaga aatacgcctc cggcaacaaa gataatattg tccgcaataa cgacagagtg 3540
   tatatcaatg tggtcgttaa aaacaaggag tatagactgg ccacaaacgc ctcccaggcc 3600
   ggcgttgaga aaattctgtc cgccctggag atccccgacg ttggcaatct gtcccaagtg 3660
   gtggtgatga agtccaagaa cgaccagggc attacaaata aatgtaaaat gaatctgcag 3720
   gataacaatg gcaatgacat cggctttatt ggctttcacc aattcaacaa tatcgccaag 3780
   ctggtggcct ccaactggta caaccgacag attgagcggt cctcccggac tctgggctgt 3840
   tcctgggagt tcatcccagt ggacgatggc tggggcgagc ggcccctgtg a          3891
<210> 100
   <211> 54
   <212> DNA
   <213> Artificial Sequence
<220>
   <223> Synthetic DNA oligonucleotide used as sense primer for H227Y site-directed mutagenesis
<400> 100
   gtgaccttgg cacatgaact tatttatgcc gggcatcgct tgtatggaat cgcc 54
<210> 101
   <211> 54
   <212> DNA
   <213> Artificial Sequence
<220>
   <223> Synthetic DNA oligonucleotide used as antisense primer for H227Y site-directed mutagenesis
<400> 101
   ggcgattcca tacaagcgat gcccggcata aataagttca tgtgccaagg tcac 54
<210> 102
   <211> 54
   <212> DNA
   <213> Artificial Sequence
<220>
   <223> Synthetic DNA oligonucleotide used as sense primer for Y227H site-directed mutagenesis
<400> 102
   gtgaccttgg cacatgaact tattcatgcc gggcatcgct tgtatggaat cgcc 54
<210> 103
   <211> 54
   <212> DNA
   <213> Artificial Sequence
<220>
   <223> Synthetic DNA oligonucleotide used as antisense primer for Y227H site-directed mutagenesis
<400> 103
   ggcgattcca tacaagcgat gcccggcatg aataagttca tgtgccaagg tcac 54
<210> 104
   <211> 38
   <212> DNA -
   <213> Artificial Sequence
<220>
   <223> SL103 sense primer to remove first NdeI restriction endonuclease site
<400> 104
   gatgaactcg atgatccctt acggtgtgaa acgtctgg 38
<210> 105
   <211> 38
   <212> DNA
   <213> Artificial Sequence
<220>
   <223> SL103 antisense primer to remove first NdeI restriction endonuclease site
<400> 105
   ccagacgttt cacaccgtaa gggatcatcg agttcatc 38
<210> 106
   <211> 38
   <212> DNA
   <213> Artificial Sequence
<220>
   <223> SL104 sense primer to remove second NdeI restriction endonuclease site
<400> 106
   ccagacgttt cacaccgtaa gggatcatcg agttcatc 38
<210> 107
   <211> 38
   <212> DNA
   <213> Artificial Sequence
<220>
   <223> SL104 antiense primer to remove second NdeI restriction endonuclease site
<400> 107
   gatgaactcg atgatccctt acggtgtgaa acgtctgg 38
<210> 108
   <211> 30
   <212> DNA
   <213> Artificial Sequence
<220>
   <223> SL101 NdeI sense primer for PCR
<400> 108
   cgccatatgc cgttcgtaaa caaacagttc 30
<210> 109
   <211> 42
   <212> DNA
   <213> Artificial Sequence
<220>
   <223> SL101 HindII, lysine addition antisense primer for PCR
<400> 109
   cccaagcttg tcgactttca atgggcgttc tccccaaccg tc 42
<210> 110
   <211> 4005
   <212> DNA
   <213> Artificial Sequence
<220>
   <223> His-BoNT/A, E. coli-modiefied
<400> 110
   atgcggggtt ctcatcatca tcatcatcat ggtatggcta gcatgactgg tggacagcaa 60
   atgggtcggg atctgtacga cgatgacgat aaggatccga attcgagctc ccatatgccg 120
   ttcgtaaaca aacagttcaa ctataaagac ccagtcaacg gcgtggacat tgcctatatc 180
   aaaatcccga atgcgggtca aatgcagccc gtgaaagcat ttaaaatcca taacaaaatt 240
   tgggtgatcc cggagcgcga tacgttcacg aacccggaag aaggagattt aaacccaccg 300
   cctgaggcta aacaggtccc ggtgtcttac tatgatagca catacctgag taccgacaat 360
   gaaaaggaca actacctgaa aggtgttacc aaactgttcg agcgcattta ttcgacagat 420
   ctcggtcgca tgttgctgac ttctattgtg cgcggcattc cgttttgggg tggtagcacc 480
   atcgatacag aactcaaagt gattgacacc aactgcatca atgtgattca gcctgatggg 540
   agctaccggt ccgaagagct taacctcgta atcattggcc cgagcgcgga tattatccaa 600
   ttcgaatgta aatcttttgg gcatgaagtc ctgaatctga cgcggaatgg ctatggatcg 660
   acgcagtata ttcgtttttc tccagatttc acatttggat ttgaagaaag cctcgaagtt 720
   gatacgaacc ctcttttagg cgcgggaaaa ttcgcgacgg acccagcggt gaccttggca 780
   catgaactta ttcatgccgg gcatcgcttg tatggaatcg ccattaaccc gaaccgtgtt 840
   ttcaaggtga atacgaacgc gtattacgag atgtcgggct tagaagtgtc ctttgaagaa 900
   ctgcgcacgt ttggcggtca tgatgcaaaa tttattgata gtctgcaaga aaacgaattt 960
   cggctgtact attacaataa attcaaagac attgcatcaa ccttaaacaa ggcgaaaagc 1020
   attgtgggta ccacggctag cttacaatat atgaaaaacg ttttcaaaga aaaatacctc 1080
   cttagcgaag acacttccgg caaattctct gtcgataaac tgaaatttga taaactgtat 1140
   aaaatgctca ccgagatcta cacagaggat aactttgtca aattcttcaa ggtcttgaat 1200
   cggaaaacct atctgaactt cgataaagcc gtctttaaga tcaacatcgt accgaaagtt 1260
   aactacacca tctatgatgg ctttaatctg cgcaatacga atctggcggc gaactttaac 1320
   ggccagaaca ccgaaatcaa caacatgaac tttactaaac tgaaaaattt taccggcttg 1380
   tttgaatttt ataagctcct gtgtgtccgc ggtattatca ccagcaaaac caaatccttg 1440
   gataagggct ataacaaggc gctcaatgat ttatgcatca aggtgaacaa ctgggacttg 1500
   tttttctctc catctgaaga taattttact aacgacttga acaaaggaga ggaaattact 1560
   tccgatacca acatcgaagc agcggaagag aatattagcc tggatcttat tcaacaatat 1620
   tacctgacct ttaattttga taacgagcct gagaacattt ccattgagaa tctcagctct 1680
   gacatcatcg gccagctgga actgatgccg aatatcgaac gctttcctaa tggaaagaaa 1740
   tatgaattgg acaaatacac catgttccac tatctccgcg cgcaggagtt tgagcacggc 1800
   aagtctcgta ttgctctgac caattcggta aacgaagccc ttttaaatcc ttcgcgtgtg 1860
   tacacctttt tctcaagcga ttatgttaaa aaagtgaaca aggcgaccga agcggcgatg 1920
   tttttgggat gggtggaaca actggtatat gactttacgg atgaaacttc tgaagtctcg 1980
   accaccgaca aaattgccga tattaccatt atcattccct atattggccc tgcactgaac 2040
   attggtaaca tgctgtataa agatgatttt gtgggcgccc tgatcttttc aggcgctgtt 2100
   atcctgctgg aatttatccc ggaaatcgcc attccagtac tcggtacctt tgcgctggtg 2160
   tcctatatcg caaacaaagt tttgactgtc cagacgatcg acaacgcgct cagtaaacgt 2220
   aacgaaaaat gggatgaggt gtataagtat attgttacca actggctcgc taaagtaaac 2280
   acccagattg acctgattcg caagaagatg aaagaagcgc tggaaaacca agcagaagcg 2340
   accaaagcta ttatcaacta tcaatataac cagtacacag aggaagaaaa gaataacatc 2400
   aacttcaaca tcgacgactt atcttcaaag ctgaatgaat ctattaacaa agcgatgatt 2460
   aatattaaca agttcttgaa ccaatgtagt gtcagctatc tgatgaactc gatgatccca 2520
   tatggtgtga aacgtctgga agacttcgat gcaagcctta aagatgccct tctgaagtat 2580
   atttacgata atcgcggaac tcttattggc caagtggatc gcttaaaaga taaagtcaac 2640
   aacacgctga gtacagacat cccttttcag ctgtctaaat atgtggacaa tcagcgcctg 2700
   ctgtccacgt ttacggaata catcaaaaac atcatcaaca ctagtattct gaacttgcgt 2760
   tacgagagta accatctgat tgatctgagc cgttacgcat ctaaaatcaa catcggctcg 2820
   aaggtgaact tcgatcctat cgacaaaaac cagattcaat tgttcaactt agaatcgtca 2880
   aagattgaag ttatcttaaa aaatgcgatt gtatataatt caatgtacga aaatttctct 2940
   acgagctttt ggattcgtat tccgaaatat ttcaacagta tctctttaaa caacgagtat 3000
   actatcatca attgtatgga gaataacagc gggtggaaag tgagccttaa ctatggtgaa 3060
   atcatctgga ctctgcagga cactcaagaa attaaacaac gcgtggtgtt taaatactca 3120
   cagatgatta acatctcgga ttatattaat cgctggattt ttgtgacaat tactaacaac 3180
   cggctgaaca acagcaaaat ttacattaac ggtcgcctga tcgatcagaa accaatcagt 3240
   aatctcggta acattcacgc atcgaataat atcatgttca aactggatgg ttgtcgcgac 3300
   acgcaccgtt acatttggat caaatacttc aatttattcg acaaagaact caacgaaaag 3360
   gagattaagg atctttatga caatcagtct aattcgggta ttctgaaaga cttttggggt 3420
   gattaccttc agtacgataa accgtattat atgttaaact tatatgatcc gaataaatac 3480
   gttgacgtca acaacgttgg cattcgtggc tatatgtatc tgaaagggcc gcgtggcagc 3540
   gtgatgacca ctaacattta cttaaactcc tccctctatc gcggtactaa atttattatc 3600
   aagaaatatg cctctggcaa caaggacaat atcgtacgca ataacgatcg cgtctacatt 3660
   aacgtggtgg tgaagaataa agaatatcgt ctggcgacca atgctagtca ggcgggcgtg 3720
   gagaaaattc tgtctgcact tgaaatcccg gatgtgggta atttatccca ggtggttgtg 3780
   atgaaaagta aaaatgacca agggatcacc aataaatgca aaatgaatct gcaagataac 3840
   aacggcaacg acattggttt tatcggcttc caccaattca ataatatcgc gaaactggtg 3900
   gcctcaaatt ggtacaaccg tcagattgag cgcagctccc gcactttagg ctgtagctgg 3960
   gagttcattc cggtagatga cggttgggga gaacgcccat tgtaa                  4005
<210> 111
   <211> 1334
   <212> PRT
   <213> Artificial Sequence
<220>
   <223> His-BoNT/A, E. coli-modiefied
<400> 111
   Met Arg Gly Ser His His His His His His Gly Met Ala Ser Met Thr
   1 5 10 15
   Gly Gly Gln Gln Met Gly Arg Asp Leu Tyr Asp Asp Asp Asp Lys Asp
   20 25 30
   Pro Asn Ser Ser Ser His Met Pro Phe Val Asn Lys Gln Phe Asn Tyr
   35 40 45
   Lys Asp Pro Val Asn Gly Val Asp Ile Ala Tyr Ile Lys Ile Pro Asn
   50 55 60
   Ala Gly Gln Met Gln Pro Val Lys Ala Phe Lys Ile His Asn Lys Ile
   65 70 75 80
   Trp Val Ile Pro Glu Arg Asp Thr Phe Thr Asn Pro Glu Glu Gly Asp
   85 90 95
   Leu Asn Pro Pro Pro Glu Ala Lys Gln Val Pro Val Ser Tyr Tyr Asp
   100 105 110
   Ser Thr Tyr Leu Ser Thr Asp Asn Glu Lys Asp Asn Tyr Leu Lys Gly
   115 120 125
   Val Thr Lys Leu Phe Glu Arg Ile Tyr Ser Thr Asp Leu Gly Arg Met
   130 135 140
   Leu Leu Thr Ser Ile Val Arg Gly Ile Pro Phe Trp Gly Gly Ser Thr
   145 150 155 160
   Ile Asp Thr Glu Leu Lys Val Ile Asp Thr Asn Cys Ile Asn Val Ile
   165 170 175
   Gln Pro Asp Gly Ser Tyr Arg Ser Glu Glu Leu Asn Leu Val Ile Ile
   180 185 190
   Gly Pro Ser Ala Asp Ile Ile Gln Phe Glu Cys Lys Ser Phe Gly His
   195 200 205
   Glu Val Leu Asn Leu Thr Arg Asn Gly Tyr Gly Ser Thr Gln Tyr Ile
   210 215 220
   Arg Phe Ser Pro Asp Phe Thr Phe Gly Phe Glu Glu Ser Leu Glu Val
   225 230 235 240
   Asp Thr Asn Pro Leu Leu Gly Ala Gly Lys Phe Ala Thr Asp Pro Ala
   245 250 255
   Val Thr Leu Ala His Glu Leu Ile His Ala Gly His Arg Leu Tyr Gly
   260 265 270
   Ile Ala Ile Asn Pro Asn Arg Val Phe Lys Val Asn Thr Asn Ala Tyr
   275 280 285
   Tyr Glu Met Ser Gly Leu Glu Val Ser Phe Glu Glu Leu Arg Thr Phe
   290 295 300
   Gly Gly His Asp Ala Lys Phe Ile Asp Ser Leu Gln Glu Asn Glu Phe
   305 310 315 320
   Arg Leu Tyr Tyr Tyr Asn Lys Phe Lys Asp Ile Ala Ser Thr Leu Asn
   325 330 335
   Lys Ala Lys Ser Ile Val Gly Thr Thr Ala Ser Leu Gln Tyr Met Lys
   340 345 350
   Asn Val Phe Lys Glu Lys Tyr Leu Leu Ser Glu Asp Thr Ser Gly Lys
   355 360 365
   Phe Ser Val Asp Lys Leu Lys Phe Asp Lys Leu Tyr Lys Met Leu Thr
   370 375 380
   Glu Ile Tyr Thr Glu Asp Asn Phe Val Lys Phe Phe Lys Val Leu Asn
   385 390 395 400
   Arg Lys Thr Tyr Leu Asn Phe Asp Lys Ala Val Phe Lys Ile Asn Ile
   405 410 415
   Val Pro Lys Val Asn Tyr Thr Ile Tyr Asp Gly Phe Asn Leu Arg Asn
   420 425 430
   Thr Asn Leu Ala Ala Asn Phe Asn Gly Gln Asn Thr Glu Ile Asn Asn
           435                 440                 445
   Met Asn Phe Thr Lys Leu Lys Asn Phe Thr Gly Leu Phe Glu Phe Tyr
       450                 455                 460
   Lys Leu Leu Cys Val Arg Gly Ile Ile Thr Ser Lys Thr Lys Ser Leu
   465                 470                 475                 480
   Asp Lys Gly Tyr Asn Lys Ala Leu Asn Asp Leu Cys Ile Lys Val Asn
                   485                 490                 495
   Asn Trp Asp Leu Phe Phe Ser Pro Ser Glu Asp Asn Phe Thr Asn Asp
               500                 505                 510
   Leu Asn Lys Gly Glu Glu Ile Thr Ser Asp Thr Asn Ile Glu Ala Ala
           515                 520                 525
   Glu Glu Asn Ile Ser Leu Asp Leu Ile Gln Gln Tyr Tyr Leu Thr Phe
       530                 535                 540
   Asn Phe Asp Asn Glu Pro Glu Asn Ile Ser Ile Glu Asn Leu Ser Ser
   545                 550                 555                 560
   Asp Ile Ile Gly Gln Leu Glu Leu Met Pro Asn Ile Glu Arg Phe Pro
                   565                 570                 575
   Asn Gly Lys Lys Tyr Glu Leu Asp Lys Tyr Thr Met Phe His Tyr Leu
               580                 585                 590
   Arg Ala Gln Glu Phe Glu His Gly Lys Ser Arg Ile Ala Leu Thr Asn
           595                 600                 605
   Ser Val Asn Glu Ala Leu Leu Asn Pro Ser Arg Val Tyr Thr Phe Phe
       610                 615                 620
   Ser Ser Asp Tyr Val Lys Lys Val Asn Lys Ala Thr Glu Ala Ala Met
   625                 630                 635                 640
   Phe Leu Gly Trp Val Glu Gln Leu Val Tyr Asp Phe Thr Asp Glu Thr
                   645                 650                 655
   Ser Glu Val Ser Thr Thr Asp Lys Ile Ala Asp Ile Thr Ile Ile Ile
               660                 665                 670
   Pro Tyr Ile Gly Pro Ala Leu Asn Ile Gly Asn Met Leu Tyr Lys Asp
           675                 680                 685
   Asp Phe Val Gly Ala Leu Ile Phe Ser Gly Ala Val Ile Leu Leu Glu
       690                 695                 700
   Phe Ile Pro Glu Ile Ala Ile Pro Val Leu Gly Thr Phe Ala Leu Val
   705                 710                 715                 720
   Ser Tyr Ile Ala Asn Lys Val Leu Thr Val Gln Thr Ile Asp Asn Ala
                   725                 730                 735
   Leu Ser Lys Arg Asn Glu Lys Trp Asp Glu Val Tyr Lys Tyr Ile Val
               740                 745                 750
   Thr Asn Trp Leu Ala Lys Val Asn Thr Gln Ile Asp Leu Ile Arg Lys
           755                 760                 765
   Lys Met Lys Glu Ala Leu Glu Asn Gln Ala Glu Ala Thr Lys Ala Ile
       770                 775                 780
   Ile Asn Tyr Gln Tyr Asn Gln Tyr Thr Glu Glu Glu Lys Asn Asn Ile
   785                 790                 795                 800
   Asn Phe Asn Ile Asp Asp Leu Ser Ser Lys Leu Asn Glu Ser Ile Asn
                   805                 810                 815
   Lys Ala Met Ile Asn Ile Asn Lys Phe Leu Asn Gln Cys Ser Val Ser
               820                 825                 830
   Tyr Leu Met Asn Ser Met Ile Pro Tyr Gly Val Lys Arg Leu Glu Asp
           835                 840                 845
   Phe Asp Ala Ser Leu Lys Asp Ala Leu Leu Lys Tyr Ile Tyr Asp Asn
       850                 855                 860
   Arg Gly Thr Leu Ile Gly Gln Val Asp Arg Leu Lys Asp Lys Val Asn
   865                 870                 875                 880
   Asn Thr Leu Ser Thr Asp Ile Pro Phe Gln Leu Ser Lys Tyr Val Asp
                   885                 890                 895
   Asn Gln Arg Leu Leu Ser Thr Phe Thr Glu Tyr Ile Lys Asn Ile Ile
               900                 905                 910
   Asn Thr Ser Ile Leu Asn Leu Arg Tyr Glu Ser Asn His Leu Ile Asp
   915 920 925
   Leu Ser Arg Tyr Ala Ser Lys Ile Asn Ile Gly Ser Lys Val Asn Phe
   930 935 940
   Asp Pro Ile Asp Lys Asn Gln Ile Gln Leu Phe Asn Leu Glu Ser Ser
   945 950 955 960
   Lys Ile Glu Val Ile Leu Lys Asn Ala Ile Val Tyr Asn Ser Met Tyr
   965 970 975
   Glu Asn Phe Ser Thr Ser Phe Trp Ile Arg Ile Pro Lys Tyr Phe Asn
   980 985 990
   Ser Ile Ser Leu Asn Asn Glu Tyr Thr Ile Ile Asn Cys Met Glu Asn
   995 1000 1005
   Asn Ser Gly Trp Lys Val Ser Leu Asn Tyr Gly Glu Ile Ile Trp Thr
   1010 1015 1020
   Leu Gln Asp Thr Gln Glu Ile Lys Gln Arg Val Val Phe Lys Tyr Ser
   1025 1030 1035 1040
   Gln Met Ile Asn Ile Ser Asp Tyr Ile Asn Arg Trp Ile Phe Val Thr
   1045 1050 1055
   Ile Thr Asn Asn Arg Leu Asn Asn Ser Lys Ile Tyr Ile Asn Gly Arg
   1060 1065 1070
   Leu Ile Asp Gln Lys Pro Ile Ser Asn Leu Gly Asn Ile His Ala Ser
   1075 1080 1085
   Asn Asn Ile Met Phe Lys Leu Asp Gly Cys Arg Asp Thr His Arg Tyr
   1090 1095 1100
   Ile Trp Ile Lys Tyr Phe Asn Leu Phe Asp Lys Glu Leu Asn Glu Lys
   1105 1110 1115 1120
   Glu Ile Lys Asp Leu Tyr Asp Asn Gln Ser Asn Ser Gly Ile Leu Lys
   1125 1130 1135
   Asp Phe Trp Gly Asp Tyr Leu Gln Tyr Asp Lys Pro Tyr Tyr Met Leu
   1140 1145 1150
   Asn Leu Tyr Asp Pro Asn Lys Tyr Val Asp Val Asn Asn Val Gly Ile
   1155 1160 1165
   Arg Gly Tyr Met Tyr Leu Lys Gly Pro Arg Gly Ser Val Met Thr Thr
   1170 1175 1180
   Asn Ile Tyr Leu Asn Ser Ser Leu Tyr Arg Gly Thr Lys Phe Ile Ile
   1185 1190 1195 1200
   Lys Lys Tyr Ala Ser Gly Asn Lys Asp Asn Ile Val Arg Asn Asn Asp
   1205 1210 1215
   Arg Val Tyr Ile Asn Val Val Val Lys Asn Lys Glu Tyr Arg Leu Ala
   1220 1225 1230
   Thr Asn Ala Ser Gln Ala Gly Val Glu Lys Ile Leu Ser Ala Leu Glu
   1235 1240 1245
   Ile Pro Asp Val Gly Asn Leu Ser Gln Val Val Val Met Lys Ser Lys
   1250 1255 1260
   Asn Asp Gln Gly Ile Thr Asn Lys Cys Lys Met Asn Leu Gln Asp Asn
   1265 1270 1275 1280
   Asn Gly Asn Asp Ile Gly Phe Ile Gly Phe His Gln Phe Asn Asn Ile
   1285 1290 1295
   Ala Lys Leu Val Ala Ser Asn Trp Tyr Asn Arg Gln Ile Glu Arg Ser
   1300 1305 1310
   Ser Arg Thr Leu Gly Cys Ser Trp Glu Phe Ile Pro Val Asp Asp Gly
   1315 1320 1325
   Trp Gly Glu Arg Pro Leu
   1330
<210> 112
   <211> 3939
   <212> DNA
   <213> Artificial Sequence
<220>
   <223> BoNT/A-KHis, E. coli-modified
<400> 112
   atgccgttcg taaacaaaca gttcaactat aaagacccag tcaacggcgt ggacattgcc 60
   tatatcaaaa tcccgaatgc gggtcaaatg cagcccgtga aagcatttaa aatccataac 120
   aaaatttggg tgatcccgga gcgcgatacg ttcacgaacc cggaagaagg agatttaaac 180
   ccaccgcctg aggctaaaca ggtcccggtg tcttactatg atagcacata cctgagtacc 240
   gacaatgaaa aggacaacta cctgaaaggt gttaccaaac tgttcgagcg catttattcg 300
   acagatctcg gtcgcatgtt gctgacttct attgtgcgcg gcattccgtt ttggggtggt 360
   agcaccatcg atacagaact caaagtgatt gacaccaact gcatcaatgt gattcagcct 420
   gatgggagct accggtccga agagcttaac ctcgtaatca ttggcccgag cgcggatatt 480
   atccaattcg aatgtaaatc ttttgggcat gaagtcctga atctgacgcg gaatggctat 540
   ggatcgacgc agtatattcg tttttctcca gatttcacat ttggatttga agaaagcctc 600
   gaagttgata cgaaccctct tttaggcgcg ggaaaattcg cgacggaccc agcggtgacc 660
   ttggcacatg aacttattca tgccgggcat cgcttgtatg gaatcgccat taacccgaac 720
   cgtgttttca aggtgaatac gaacgcgtat tacgagatgt cgggcttaga agtgtccttt 780
   gaagaactgc gcacgtttgg cggtcatgat gcaaaattta ttgatagtct gcaagaaaac 840
   gaatttcggc tgtactatta caataaattc aaagacattg catcaacctt aaacaaggcg 900
   aaaagcattg tgggtaccac ggctagctta caatatatga aaaacgtttt caaagaaaaa 960
   tacctcctta gcgaagacac ttccggcaaa ttctctgtcg ataaactgaa atttgataaa 1020
   ctgtataaaa tgctcaccga gatctacaca gaggataact ttgtcaaatt cttcaaggtc 1080
   ttgaatcgga aaacctatct gaacttcgat aaagccgtct ttaagatcaa catcgtaccg 1140
   aaagttaact acaccatcta tgatggcttt aatctgcgca atacgaatct ggcggcgaac 1200
   tttaacggcc agaacaccga aatcaacaac atgaacttta ctaaactgaa aaattttacc 1260
   ggcttgtttg aattttataa gctcctgtgt gtccgcggta ttatcaccag caaaaccaaa 1320
   tccttggata agggctataa caaggcgctc aatgatttat gcatcaaggt gaacaactgg 1380
   gacttgtttt tctctccatc tgaagataat tttactaacg acttgaacaa aggagaggaa 1440
   attacttccg ataccaacat cgaagcagcg gaagagaata ttagcctgga tcttattcaa 1500
   caatattacc tgacctttaa ttttgataac gagcctgaga acatttccat tgagaatctc 1560
   agctctgaca tcatcggcca gctggaactg atgccgaata tcgaacgctt tcctaatgga 1620
   aagaaatatg aattggacaa atacaccatg ttccactatc tccgcgcgca ggagtttgag 1680
   cacggcaagt ctcgtattgc tctgaccaat tcggtaaacg aagccctttt aaatccttcg 1740
   cgtgtgtaca cctttttctc aagcgattat gttaaaaaag tgaacaaggc gaccgaagcg 1800
   gcgatgtttt tgggatgggt ggaacaactg gtatatgact ttacggatga aacttctgaa 1860
   gtctcgacca ccgacaaaat tgccgatatt accattatca ttccctatat tggccctgca 1920
   ctgaacattg gtaacatgct gtataaagat gattttgtgg gcgccctgat cttttcaggc 1980
   gctgttatcc tgctggaatt tatcccggaa atcgccattc cagtactcgg tacctttgcg 2040
   ctggtgtcct atatcgcaaa caaagttttg actgtccaga cgatcgacaa cgcgctcagt 2100
   aaacgtaacg aaaaatggga tgaggtgtat aagtatattg ttaccaactg gctcgctaaa 2160
   gtaaacaccc agattgacct gattcgcaag aagatgaaag aagcgctgga aaaccaagca 2220
   gaagcgacca aagctattat caactatcaa tataaccagt acacagagga agaaaagaat 2280
   aacatcaact tcaacatcga cgacttatct tcaaagctga atgaatctat taacaaagcg 2340
   atgattaata ttaacaagtt cttgaaccaa tgtagtgtca gctatctgat gaactcgatg 2400
   atcccttacg gtgtgaaacg tctggaagac ttcgatgcaa gccttaaaga tgcccttctg 2460
   aagtatattt acgataatcg cggaactctt attggccaag tggatcgctt aaaagataaa 2520
   gtcaacaaca cgctgagtac agacatccct tttcagctgt ctaaatatgt ggacaatcag 2580
   cgcctgctgt ccacgtttac ggaatacatc aaaaacatca tcaacactag tattctgaac 2640
   ttgcgttacg agagtaacca tctgattgat ctgagccgtt acgcatctaa aatcaacatc 2700
   ggctcgaagg tgaacttcga tcctatcgac aaaaaccaga ttcaattgtt caacttagaa 2760
   tcgtcaaaga ttgaagttat cttaaaaaat gcgattgtat ataattcaat gtacgaaaat 2820
   ttctctacga gcttttggat tcgtattccg aaatatttca acagtatctc tttaaacaac 2880
   gagtatacta tcatcaattg tatggagaat aacagcgggt ggaaagtgag ccttaactat 2940
   ggtgaaatca tctggactct gcaggacact caagaaatta aacaacgcgt ggtgtttaaa 3000
   tactcacaga tgattaacat ctcggattat attaatcgct ggatttttgt gacaattact 3060
   aacaaccggc tgaacaacag caaaatttac attaacggtc gcctgatcga tcagaaacca 3120
   atcagtaatc tcggtaacat tcacgcatcg aataatatca tgttcaaact ggatggttgt 3180
   cgcgacacgc accgttacat ttggatcaaa tacttcaatt tattcgacaa agaactcaac 3240
   gaaaaggaga ttaaggatct ttatgacaat cagtctaatt cgggtattct gaaagacttt 3300
   tggggtgatt accttcagta cgataaaccg tattatatgt taaacttata tgatccgaat 3360
   aaatacgttg acgtcaacaa cgttggcatt cgtggctata tgtatctgaa agggccgcgt 3420
   ggcagcgtga tgaccactaa catttactta aactcctccc tctatcgcgg tactaaattt 3480
   attatcaaga aatatgcctc tggcaacaag gacaatatcg tacgcaataa cgatcgcgtc 3540
   tacattaacg tggtggtgaa gaataaagaa tatcgtctgg cgaccaatgc tagtcaggcg 3600
   ggcgtggaga aaattctgtc tgcacttgaa atcccggatg tgggtaattt atcccaggtg 3660
   gttgtgatga aaagtaaaaa tgaccaaggg atcaccaata aatgcaaaat gaatctgcaa 3720
   gataacaacg gcaacgacat tggttttatc ggcttccacc aattcaataa tatcgcgaaa 3780
   ctggtggcct caaattggta caaccgtcag attgagcgca gctcccgcac tttaggctgt 3840
   agctgggagt tcattccggt agatgacggt tggggagaac gcccattgaa agtcgacaag 3900
   cttgcggccg cactcgagca ccaccaccac caccactga                        3939
<210> 113
   <211> 1312
   <212> PRT
   <213> Artificial Sequence
<220>
   <223> BoNT/A-KHis, E. coli-modified
<400> 113
   Met Pro Phe Val Asn Lys Gln Phe Asn Tyr Lys Asp Pro Val Asn Gly
   1               5                   10                  15
   Val Asp Ile Ala Tyr Ile Lys Ile Pro Asn Ala Gly Gln Met Gln Pro
               20                  25                  30
   Val Lys Ala Phe Lys Ile His Asn Lys Ile Trp Val Ile Pro Glu Arg
           35                  40                  45
   Asp Thr Phe Thr Asn Pro Glu Glu Gly Asp Leu Asn Pro Pro Pro Glu
       50                  55                  60
   Ala Lys Gln Val Pro Val Ser Tyr Tyr Asp Ser Thr Tyr Leu Ser Thr
   65                  70                  75                  80
   Asp Asn Glu Lys Asp Asn Tyr Leu Lys Gly Val Thr Lys Leu Phe Glu
                   85                  90                  95
   Arg Ile Tyr Ser Thr Asp Leu Gly Arg Met Leu Leu Thr Ser Ile Val
               100                 105                 110
   Arg Gly Ile Pro Phe Trp Gly Gly Ser Thr Ile Asp Thr Glu Leu Lys
           115                 120                 125
   Val Ile Asp Thr Asn Cys Ile Asn Val Ile Gln Pro Asp Gly Ser Tyr
       130                 135                 140
   Arg Ser Glu Glu Leu Asn Leu Val Ile Ile Gly Pro Ser Ala Asp Ile
   145                 150                 155                 160
   Ile Gln Phe Glu Cys Lys Ser Phe Gly His Glu Val Leu Asn Leu Thr
                   165                 170                 175
   Arg Asn Gly Tyr Gly Ser Thr Gln Tyr Ile Arg Phe Ser Pro Asp Phe
               180                 185                 190
   Thr Phe Gly Phe Glu Glu Ser Leu Glu Val Asp Thr Asn Pro Leu Leu
           195                 200                 205
   Gly Ala Gly Lys Phe Ala Thr Asp Pro Ala Val Thr Leu Ala His Glu
       210                 215                 220
   Leu Ile His Ala Gly His Arg Leu Tyr Gly Ile Ala Ile Asn Pro Asn
   225                 230                 235                 240
   Arg Val Phe Lys Val Asn Thr Asn Ala Tyr Tyr Glu Met Ser Gly Leu
                   245                 250                 255
   Glu Val Ser Phe Glu Glu Leu Arg Thr Phe Gly Gly His Asp Ala Lys
               260                 265                 270
   Phe Ile Asp Ser Leu Gln Glu Asn Glu Phe Arg Leu Tyr Tyr Tyr Asn
           275                 280                 285
   Lys Phe Lys Asp Ile Ala Ser Thr Leu Asn Lys Ala Lys Ser Ile Val
   290 295 300
   Gly Thr Thr Ala Ser Leu Gln Tyr Met Lys Asn Val Phe Lys Glu Lys
   305 310 315 320
   Tyr Leu Leu Ser Glu Asp Thr Ser Gly Lys Phe Ser Val Asp Lys Leu
   325 330 335
   Lys Phe Asp Lys Leu Tyr Lys Met Leu Thr Glu Ile Tyr Thr Glu Asp
   340 345 350
   Asn Phe Val Lys Phe Phe Lys Val Leu Asn Arg Lys Thr Tyr Leu Asn
   355 360 365
   Phe Asp Lys Ala Val Phe Lys Ile Asn Ile Val Pro Lys Val Asn Tyr
   370 375 380
   Thr Ile Tyr Asp Gly Phe Asn Leu Arg Asn Thr Asn Leu Ala Ala Asn
   385 390 395 400
   Phe Asn Gly Gln Asn Thr Glu Ile Asn Asn Met Asn Phe Thr Lys Leu
   405 410 415
   Lys Asn Phe Thr Gly Leu Phe Glu Phe Tyr Lys Leu Leu Cys Val Arg
   420 425 430
   Gly Ile Ile Thr Ser Lys Thr Lys Ser Leu Asp Lys Gly Tyr Asn Lys
   435 440 445
   Ala Leu Asn Asp Leu Cys Ile Lys Val Asn Asn Trp Asp Leu Phe Phe
   450 455 460
   Ser Pro Ser Glu Asp Asn Phe Thr Asn Asp Leu Asn Lys Gly Glu Glu
   465 470 475 480
   Ile Thr Ser Asp Thr Asn Ile Glu Ala Ala Glu Glu Asn Ile Ser Leu
   485 490 495
   Asp Leu Ile Gln Gln Tyr Tyr Leu Thr Phe Asn Phe Asp Asn Glu Pro
   500 505 510
   Glu Asn Ile Ser Ile Glu Asn Leu Ser Ser Asp Ile Ile Gly Gln Leu
   515 520 525
   Glu Leu Met Pro Asn Ile Glu Arg Phe Pro Asn Gly Lys Lys Tyr Glu
   530 535 540
   Leu Asp Lys Tyr Thr Met Phe His Tyr Leu Arg Ala Gln Glu Phe Glu
   545 550 555 560
   His Gly Lys Ser Arg Ile Ala Leu Thr Asn Ser Val Asn Glu Ala Leu
   565 570 575
   Leu Asn Pro Ser Arg Val Tyr Thr Phe Phe Ser Ser Asp Tyr Val Lys
   580 585 590
   Lys Val Asn Lys Ala Thr Glu Ala Ala Met Phe Leu Gly Trp Val Glu
   595 600 605
   Gln Leu Val Tyr Asp Phe Thr Asp Glu Thr Ser Glu Val Ser Thr Thr
   610 615 620
   Asp Lys Ile Ala Asp Ile Thr Ile Ile Ile Pro Tyr Ile Gly Pro Ala
   625 630 635 640
   Leu Asn Ile Gly Asn Met Leu Tyr Lys Asp Asp Phe Val Gly Ala Leu
   645 650 655
   Ile Phe Ser Gly Ala Val Ile Leu Leu Glu Phe Ile Pro Glu Ile Ala
   660 665 670
   Ile Pro Val Leu Gly Thr Phe Ala Leu Val Ser Tyr Ile Ala Asn Lys
   675 680 685
   Val Leu Thr Val Gln Thr Ile Asp Asn Ala Leu Ser Lys Arg Asn Glu
   690 695 700
   Lys Trp Asp Glu Val Tyr Lys Tyr Ile Val Thr Asn Trp Leu Ala Lys
   705 710 715 720
   Val Asn Thr Gln Ile Asp Leu Ile Arg Lys Lys Met Lys Glu Ala Leu
   725 730 735
   Glu Asn Gln Ala Glu Ala Thr Lys Ala Ile Ile Asn Tyr Gln Tyr Asn
   740 745 750
   Gln Tyr Thr Glu Glu Glu Lys Asn Asn Ile Asn Phe Asn Ile Asp Asp
   755 760 765
   Leu Ser Ser Lys Leu Asn Glu Ser Ile Asn Lys Ala Met Ile Asn Ile
   770 775 780
   Asn Lys Phe Leu Asn Gln Cys Ser Val Ser Tyr Leu Met Asn Ser Met
   785 790 795 800
   Ile Pro Tyr Gly Val Lys Arg Leu Glu Asp Phe Asp Ala Ser Leu Lys
   805 810 815
   Asp Ala Leu Leu Lys Tyr Ile Tyr Asp Asn Arg Gly Thr Leu Ile Gly
   820 825 830
   Gln Val Asp Arg Leu Lys Asp Lys Val Asn Asn Thr Leu Ser Thr Asp
   835 840 845
   Ile Pro Phe Gln Leu Ser Lys Tyr Val Asp Asn Gln Arg Leu Leu Ser
   850 855 860
   Thr Phe Thr Glu Tyr Ile Lys Asn Ile Ile Asn Thr Ser Ile Leu Asn
   865 870 875 880
   Leu Arg Tyr Glu Ser Asn His Leu Ile Asp Leu Ser Arg Tyr Ala Ser
   885 890 895
   Lys Ile Asn Ile Gly Ser Lys Val Asn Phe Asp Pro Ile Asp Lys Asn
   900 905 910
   Gln Ile Gln Leu Phe Asn Leu Glu Ser Ser Lys Ile Glu Val Ile Leu
   915 920 925
   Lys Asn Ala Ile Val Tyr Asn Ser Met Tyr Glu Asn Phe Ser Thr Ser
   930 935 940
   Phe Trp Ile Arg Ile Pro Lys Tyr Phe Asn Ser Ile Ser Leu Asn Asn
   945 950 955 960
   Glu Tyr Thr Ile Ile Asn Cys Met Glu Asn Asn Ser Gly Trp Lys Val
   965 970 975
   Ser Leu Asn Tyr Gly Glu Ile Ile Trp Thr Leu Gln Asp Thr Gln Glu
   980 985 990
   Ile Lys Gln Arg Val Val Phe Lys Tyr Ser Gln Met Ile Asn Ile Ser
   995 1000 1005
   Asp Tyr Ile Asn Arg Trp Ile Phe Val Thr Ile Thr Asn Asn Arg Leu
   1010 1015 1020
   Asn Asn Ser Lys Ile Tyr Ile Asn Gly Arg Leu Ile Asp Gln Lys Pro
   1025 1030 1035 1040
   Ile Ser Asn Leu Gly Asn Ile His Ala Ser Asn Asn Ile Met Phe Lys
   1045 1050 1055
   Leu Asp Gly Cys Arg Asp Thr His Arg Tyr Ile Trp Ile Lys Tyr Phe
   1060 1065 1070
   Asn Leu Phe Asp Lys Glu Leu Asn Glu Lys Glu Ile Lys Asp Leu Tyr
   1075 1080 1085
   Asp Asn Gln Ser Asn Ser Gly Ile Leu Lys Asp Phe Trp Gly Asp Tyr
   1090 1095 1100
   Leu Gln Tyr Asp Lys Pro Tyr Tyr Met Leu Asn Leu Tyr Asp Pro Asn
   1105 1110 1115 1120
   Lys Tyr Val Asp Val Asn Asn Val Gly Ile Arg Gly Tyr Met Tyr Leu
   1125 1130 1135
   Lys Gly Pro Arg Gly Ser Val Met Thr Thr Asn Ile Tyr Leu Asn Ser
   1140 1145 1150
   Ser Leu Tyr Arg Gly Thr Lys Phe Ile Ile Lys Lys Tyr Ala Ser Gly
   1155 1160 1165
   Asn Lys Asp Asn Ile Val Arg Asn Asn Asp Arg Val Tyr Ile Asn Val
   1170 1175 1180
   Val Val Lys Asn Lys Glu Tyr Arg Leu Ala Thr Asn Ala Ser Gln Ala
   1185 1190 1195 1200
   Gly Val Glu Lys Ile Leu Ser Ala Leu Glu Ile Pro Asp Val Gly Asn
   1205 1210 1215
   Leu Ser Gln Val Val Val Met Lys Ser Lys Asn Asp Gln Gly Ile Thr
   1220 1225 1230
   Asn Lys Cys Lys Met Asn Leu Gln Asp Asn Asn Gly Asn Asp Ile Gly
           1235                1240                1245
   Phe Ile Gly Phe His Gln Phe Asn Asn Ile Ala Lys Leu Val Ala Ser
       1250                1255                1260
   Asn Trp Tyr Asn Arg Gln Ile Glu Arg Ser Ser Arg Thr Leu Gly Cys
   1265                1270                1275                1280
   Ser Trp Glu Phe Ile Pro Val Asp Asp Gly Trp Gly Glu Arg Pro Leu
                   1285                1290                1295
   Lys Val Asp Lys Leu Ala Ala Ala Leu Glu His His His His His His
               1300                1305                1310
<210> 114
   <211> 4005
   <212> DNA
   <213> Artificial Sequence
<220>
   <223> His-BoNT/A, unmodified
<400> 114
   atgcggggtt ctcatcatca tcatcatcat ggtatggcta gcatgactgg tggacagcaa 60
   atgggtcggg atctgtacga cgatgacgat aaggatccga attcgagctc ccatatgcca 120
   tttgttaata aacaatttaa ttataaagat cctgtaaatg gtgttgatat tgcttatata 180
   aaaattccaa atgcaggaca aatgcaacca gtaaaagctt ttaaaattca taataaaata 240
   tgggttattc cagaaagaga tacatttaca aatcctgaag aaggagattt aaatccacca 300
   ccagaagcaa aacaagttcc agtttcatat tatgattcaa catatttaag tacagataat 360
   gaaaaagata attatttaaa gggagttaca aaattatttg agagaattta ttcaactgat 420
   cttggaagaa tgttgttaac atcaatagta aggggaatac cattttgggg tggaagtaca 480
   atagatacag aattaaaagt tattgatact aattgtatta atgtgataca accagatggt 540
   agttatagat cagaagaact taatctagta ataataggac cctcagctga tattatacag 600
   tttgaatgta aaagctttgg acatgaagtt ttgaatctta cgcgaaatgg ttatggctct 660
   actcaataca ttagatttag cccagatttt acatttggtt ttgaggagtc acttgaagtt 720
   gatacaaatc ctcttttagg tgcaggcaaa tttgctacag atccagcagt aacattagca 780
   catgaactta tacatgctgg acatagatta tatggaatag caattaatcc aaatagggtt 840
   tttaaagtaa atactaatgc ctattatgaa atgagtgggt tagaagtaag ctttgaggaa 900
   cttagaacat ttgggggaca tgatgcaaag tttatagata gtttacagga aaacgaattt 960
   cgtctatatt attataataa gtttaaagat atagcaagta cacttaataa agctaaatca 1020
   atagtaggta ctactgcttc attacagtat atgaaaaatg tttttaaaga gaaatatctc 1080
   ctatctgaag atacatctgg aaaatttccg gtagataaat taaaatttga taagttatac 1140
   aaaatgttaa cagagattta cacagaggat aattttgtta agttttttaa agtacttaac 1200
   agaaaaacat atttgaattt tgataaagcc gtatttaaga taaatatagt acctaaggta 1260
   aattacacaa tatatgatgg atttaattta agaaatacaa atttagcagc aaactttaat 1320
   ggtcaaaata cagaaattaa taatatgaat tttactaaac taaaaaattt tactggattg 1380
   tttgaatttt ataagttgct atgtgtaaga gggataataa cttctaaaac taaatcatta 1440
   gataaaggat acaataaggc attaaatgat ttatgtatca aagttaataa ttgggacttg 1500
   ttttttagtc cttcagaaga taattttact aatgatctaa ataaaggaga agaaattaca 1560
   tctgatacta atatagaagc agcagaagaa aatattagtt tagatttaat acaacaatat 1620
   tatttaacct ttaattttga taatgaacct gaaaatattt caatagaaaa tctttcaagt 1680
   gacattatag gccaattaga acttatgcct aatatagaaa gatttcctaa tggaaaaaag 1740
   tatgagttag ataaatatac tatgttccat tatcttcgtg ctcaagaatt tgaacatggt 1800
   aaatctagga ttgctttaac aaattctgtt aacgaagcat tattaaatcc tagtcgtgtt 1860
   tatacatttt tttcttcaga ctatgtaaag aaagttaata aagctacgga ggcagctatg 1920
   tttttaggct gggtagaaca attagtatat gattttaccg atgaaactag cgaagtaagt 1980
   actacggata aaattgcgga tataactata attattccat atataggacc tgctttaaat 2040
   ataggtaata tgttatataa agatgatttt gtaggtgctt taatattttc aggagctgtt 2100
   attctgttag aatttatacc agagattgca atacctgtat taggtacttt tgcacttgta 2160
   tcatatattg cgaataaggt tctaaccgtt caaacaatag ataatgcttt aagtaaaaga 2220
   aatgaaaaat gggatgaggt ctataaatat atagtaacaa attggttagc aaaggttaat 2280
   acacagattg atctaataag aaaaaaaatg aaagaagctt tagaaaatca agcagaagca 2340
   acaaaggcta taataaacta tcagtataat caatatactg aggaagagaa aaataatatt 2400
   aattttaata ttgatgattt aagttcgaaa cttaatgagt ctataaataa agctatgatt 2460
   aatataaata aatttttgaa tcaatgctct gtttcatatt taatgaattc tatgatccct 2520
   tatggtgtta aacggttaga agattttgat gctagtctta aagatgcatt attaaagtat 2580
   atatatgata atagaggaac tttaattggt caagtagata gattaaaaga taaagttaat 2640
   aatacactta gtacagatat accttttcag ctttccaaat acgtagataa tcaaagatta 2700
   ttatctacat ttactgaata tattaagaat attattaata cttctatatt gaatttaaga 2760
   tatgaaagta atcatttaat agacttatct aggtatgcat caaaaataaa tattggtagt 2820
   aaagtaaatt ttgatccaat agataaaaat caaattcaat tatttaattt agaaagtagt 2880
   aaaattgagg taattttaaa aaatgctatt gtatataata gtatgtatga aaattttagt 2940
   actagctttt ggataagaat tcctaagtat tttaacagta taagtctaaa taatgaatat 3000
   acaataataa attgtatgga aaataattca ggatggaaag tatcacttaa ttatggtgaa 3060
   ataatctgga ctttacagga tactcaggaa ataaaacaaa gagtagtttt taaatacagt 3120
   caaatgatta atatatcaga ttatataaac agatggattt ttgtaactat cactaataat 3180
   agattaaata actctaaaat ttatataaat ggaagattaa tagatcaaaa accaatttca 3240
   aatttaggta atattcatgc tagtaataat ataatgttta aattagatgg ttgtagagat 3300
   acacatagat atatttggat aaaatatttt aatctttttg ataaggaatt aaatgaaaaa 3360
   gaaatcaaag atttatatga taatcaatca aattcaggta ttttaaaaga cttttggggt 3420
   gattatttac aatatgataa accatactat atgttaaatt tatatgatcc aaataaatat 3480
   gtcgatgtaa ataatgtagg tattagaggt tatatgtatc ttaaagggcc tagaggtagc 3540
   gtaatgacta caaacattta tttaaattca agtttgtata gggggacaaa atttattata 3600
   aaaaaatatg cttctggaaa taaagataat attgttagaa ataatgatcg tgtatatatt 3660
   aatgtagtag ttaaaaataa agaatatagg ttagctacta atgcgtcaca ggcaggcgta 3720
   gaaaaaatac taagtgcatt agaaatacct gatgtaggaa atctaagtca agtagtagta 3780
   atgaagtcaa aaaatgatca aggaataaca aataaatgca aaatgaattt acaagataat 3840
   aatgggaatg atataggctt tataggattt catcagttta ataatatagc taaactagta 3900
   gcaagtaatt ggtataatag acaaatagaa agatctagta ggactttggg ttgctcatgg 3960
   gaatttattc ctgtagatga tggatgggga gaaaggccac tgtaa                 4005
<210> 115
   <211> 3939
   <212> DNA
   <213> Artificial Sequence
<220>
   <223> BoNT/A-KHis, unmodified
<400> 115
   atgccatttg ttaataaaca atttaattat aaagatcctg taaatggtgt tgatattgct 60
   tatataaaaa ttccaaatgc aggacaaatg caaccagtaa aagcttttaa aattcataat 120
   aaaatatggg ttattccaga aagagataca tttacaaatc ctgaagaagg agatttaaat 180
   ccaccaccag aagcaaaaca agttccagtt tcatattatg attcaacata tttaagtaca 240
   gataatgaaa aagataatta tttaaaggga gttacaaaat tatttgagag aatttattca 300
   actgatcttg gaagaatgtt gttaacatca atagtaaggg gaataccatt ttggggtgga 360
   agtacaatag atacagaatt aaaagttatt gatactaatt gtattaatgt gatacaacca 420
   gatggtagtt atagatcaga agaacttaat ctagtaataa taggaccctc agctgatatt 480
   atacagtttg aatgtaaaag ctttggacat gaagttttga atcttacgcg aaatggttat 540
   ggctctactc aatacattag atttagccca gattttacat ttggttttga ggagtcactt 600
   gaagttgata caaatcctct tttaggtgca ggcaaatttg ctacagatcc agcagtaaca 660
   ttagcacatg aacttataca tgctggacat agattatatg gaatagcaat taatccaaat 720
   agggttttta aagtaaatac taatgcctat tatgaaatga gtgggttaga agtaagcttt 780
   gaggaactta gaacatttgg gggacatgat gcaaagttta tagatagttt acaggaaaac 840
   gaatttcgtc tatattatta taataagttt aaagatatag caagtacact taataaagct 900
   aaatcaatag taggtactac tgcttcatta cagtatatga aaaatgtttt taaagagaaa 960
   tatctcctat ctgaagatac atctggaaaa ttttcggtag ataaattaaa atttgataag 1020
   ttatacaaaa tgttaacaga gatttacaca gaggataatt ttgttaagtt ttttaaagta 1080
   cttaacagaa aaacatattt gaattttgat aaagccgtat ttaagataaa tatagtacct 1140
   aaggtaaatt acacaatata tgatggattt aatttaagaa atacaaattt agcagcaaac 1200
   tttaatggtc aaaatacaga aattaataat atgaatttta ctaaactaaa aaattttact 1260
   ggattgtttg aattttataa gttgctatgt gtaagaggga taataacttc taaaactaaa 1320
   tcattagata aaggatacaa taaggcatta aatgatttat gtatcaaagt taataattgg 1380
   gacttgtttt ttagtccttc agaagataat tttactaatg atctaaataa aggagaagaa 1440
   attacatctg atactaatat agaagcagca gaagaaaata ttagtttaga tttaatacaa 1500
   caatattatt taacctttaa ttttgataat gaacctgaaa atatttcaat agaaaatctt 1560
   tcaagtgaca ttataggcca attagaactt atgcctaata tagaaagatt tcctaatgga 1620
   aaaaagtatg agttagataa atatactatg ttccattatc ttcgtgctca agaatttgaa 1680
   catggtaaat ctaggattgc tttaacaaat tctgttaacg aagcattatt aaatcctagt 1740
   cgtgtttata catttttttc ttcagactat gtaaagaaag ttaataaagc tacggaggca 1800
   gctatgtttt taggctgggt agaacaatta gtatatgatt ttaccgatga aactagcgaa 1860
   gtaagtacta cggataaaat tgcggatata actataatta ttccatatat aggacctgct 1920
   ttaaatatag gtaatatgtt atataaagat gattttgtag gtgctttaat attttcagga 1980
   gctgttattc tgttagaatt tataccagag attgcaatac ctgtattagg tacttttgca 2040
   cttgtatcat atattgcgaa taaggttcta accgttcaaa caatagataa tgctttaagt 2100
   aaaagaaatg aaaaatggga tgaggtctat aaatatatag taacaaattg gttagcaaag 2160
   gttaatacac agattgatct aataagaaaa aaaatgaaag aagctttaga aaatcaagca 2220
   gaagcaacaa aggctataat aaactatcag tataatcaat atactgagga agagaaaaat 2280
   aatattaatt ttaatattga tgatttaagt tcgaaactta atgagtctat aaataaagct 2340
   atgattaata taaataaatt tttgaatcaa tgctctgttt catatttaat gaattctatg 2400
   atcccttatg gtgttaaacg gttagaagat tttgatgcta gtcttaaaga tgcattatta 2460
   aagtatatat atgataatag aggaacttta attggtcaag tagatagatt aaaagataaa 2520
   gttaataata cacttagtac agatatacct tttcagcttt ccaaatacgt agataatcaa 2580
   agattattat ctacatttac tgaatatatt aagaatatta ttaatacttc tatattgaat 2640
   ttaagatatg aaagtaatca tttaatagac ttatctaggt atgcatcaaa aataaatatt 2700
   ggtagtaaag taaattttga tccaatagat aaaaatcaaa ttcaattatt taatttagaa 2760
   agtagtaaaa ttgaggtaat tttaaaaaat gctattgtat ataatagtat gtatgaaaat 2820
   tttagtacta gcttttggat aagaattcct aagtatttta acagtataag tctaaataat 2880
   gaatatacaa taataaattg tatggaaaat aattcaggat ggaaagtatc acttaattat 2940
   ggtgaaataa tctggacttt acaggatact caggaaataa aacaaagagt agtttttaaa 3000
   tacagtcaaa tgattaatat atcagattat ataaacagat ggatttttgt aactatcact 3060
   aataatagat taaataactc taaaatttat ataaatggaa gattaataga tcaaaaacca 3120
   atttcaaatt taggtaatat tcatgctagt aataatataa tgtttaaatt agatggttgt 3180
   agagatacac atagatatat ttggataaaa tattttaatc tttttgataa ggaattaaat 3240
   gaaaaagaaa tcaaagattt atatgataat caatcaaatt caggtatttt aaaagacttt 3300
   tggggtgatt atttacaata tgataaacca tactatatgt taaatttata tgatccaaat 3360
   aaatatgtcg atgtaaataa tgtaggtatt agaggttata tgtatcttaa agggcctaga 3420
   ggtagcgtaa tgactacaaa catttattta aattcaagtt tgtatagggg gacaaaattt 3480
   attataaaaa aatatgcttc tggaaataaa gataatattg ttagaaataa tgatcgtgta 3540
   tatattaatg tagtagttaa aaataaagaa tataggttag ctactaatgc gtcacaggca 3600
   ggcgtagaaa aaatactaag tgcattagaa atacctgatg taggaaatct aagtcaagta 3660
   gtagtaatga agtcaaaaaa tgatcaagga ataacaaata aatgcaaaat gaatttacaa 3720
   gataataatg ggaatgatat aggctttata ggatttcatc agtttaataa tatagctaaa 3780
   ctagtagcaa gtaattggta taatagacaa atagaaagat ctagtaggac tttgggttgc 3840
   tcatgggaat ttattcctgt agatgatgga tggggagaaa ggccactgaa agtcgacaag 3900
   cttgcggccg cactcgagca ccaccaccac caccactga 3939
<210> 116
   <211> 3891
   <212> DNA
   <213> Artificial Sequence
<220>
   <223> iBoNT/A (H227Y), E-coli-modified
<400> 116
   atgccgttcg taaacaaaca gttcaactat aaagacccag tcaacggcgt ggacattgcc 60
   tatatcaaaa tcccgaatgc gggtcaaatg cagcccgtga aagcatttaa aatccataac 120
   aaaatttggg tgatcccgga gcgcgatacg ttcacgaacc cggaagaagg agatttaaac 180
   ccaccgcctg aggctaaaca ggtcccggtg tcttactatg atagcacata cctgagtacc 240
   gacaatgaaa aggacaacta cctgaaaggt gttaccaaac tgttcgagcg catttattcg 300
   acagatctcg gtcgcatgtt gctgacttct attgtgcgcg gcattccgtt ttggggtggt 360
   agcaccatcg atacagaact caaagtgatt gacaccaact gcatcaatgt gattcagcct 420
   gatgggagct accggtccga agagcttaac ctcgtaatca ttggcccgag cgcggatatt 480
   atccaattcg aatgtaaatc ttttgggcat gaagtcctga atctgacgcg gaatggctat 540
   ggatcgacgc agtatattcg tttttctcca gatttcacat ttggatttga agaaagcctc 600
   gaagttgata cgaaccctct tttaggcgcg ggaaaattcg cgacggaccc agcggtgacc 660
   ttggcacatg aacttattta tgccgggcat cgcttgtatg gaatcgccat taacccgaac 720
   cgtgttttca aggtgaatac gaacgcgtat tacgagatgt cgggcttaga agtgtccttt 780
   gaagaactgc gcacgtttgg cggtcatgat gcaaaattta ttgatagtct gcaagaaaac 840
   gaatttcggc tgtactatta caataaattc aaagacattg catcaacctt aaacaaggcg 900
   aaaagcattg tgggtaccac ggctagctta caatatatga aaaacgtttt caaagaaaaa 960
   tacctcctta gcgaagacac ttccggcaaa ttctctgtcg ataaactgaa atttgataaa 1020
   ctgtataaaa tgctcaccga gatctacaca gaggataact ttgtcaaatt cttcaaggtc 1080
   ttgaatcgga aaacctatct gaacttcgat aaagccgtct ttaagatcaa catcgtaccg 1140
   aaagttaact acaccatcta tgatggcttt aatctgcgca atacgaatct ggcggcgaac 1200
   tttaacggcc agaacaccga aatcaacaac atgaacttta ctaaactgaa aaattttacc 1260
   ggcttgtttg aattttataa gctcctgtgt gtccgcggta ttatcaccag caaaaccaaa 1320
   tccttggata agggctataa caaggcgctc aatgatttat gcatcaaggt gaacaactgg 1380
   gacttgtttt tctctccatc tgaagataat tttactaacg acttgaacaa aggagaggaa 1440
   attacttccg ataccaacat cgaagcagcg gaagagaata ttagcctgga tcttattcaa 1500
   caatattacc tgacctttaa ttttgataac gagcctgaga acatttccat tgagaatctc 1560
   agctctgaca tcatcggcca gctggaactg atgccgaata tcgaacgctt tcctaatgga 1620
   aagaaatatg aattggacaa atacaccatg ttccactatc tccgcgcgca ggagtttgag 1680
   cacggcaagt ctcgtattgc tctgaccaat tcggtaaacg aagccctttt aaatccttcg 1740
   cgtgtgtaca cctttttctc aagcgattat gttaaaaaag tgaacaaggc gaccgaagcg 1800
   gcgatgtttt tgggatgggt ggaacaactg gtatatgact ttacggatga aacttctgaa 1860
   gtctcgacca ccgacaaaat tgccgatatt accattatca ttccctatat tggccctgca 1920
   ctgaacattg gtaacatgct gtataaagat gattttgtgg gcgccctgat cttttcaggc 1980
   gctgttatcc tgctggaatt tatcccggaa atcgccattc cagtactcgg tacctttgcg 2040
   ctggtgtcct atatcgcaaa caaagttttg actgtccaga cgatcgacaa cgcgctcagt 2100
   aaacgtaacg aaaaatggga tgaggtgtat aagtatattg ttaccaactg gctcgctaaa 2160
   gtaaacaccc agattgacct gattcgcaag aagatgaaag aagcgctgga aaaccaagca 2220
   gaagcgacca aagctattat caactatcaa tataaccagt acacagagga agaaaagaat 2280
   aacatcaact tcaacatcga cgacttatct tcaaagctga atgaatctat taacaaagcg 2340
   atgattaata ttaacaagtt cttgaaccaa tgtagtgtca gctatctgat gaactcgatg 2400
   atcccatatg gtgtgaaacg tctggaagac ttcgatgcaa gccttaaaga tgcccttctg 2460
   aagtatattt acgataatcg cggaactctt attggccaag tggatcgctt aaaagataaa 2520
   gtcaacaaca cgctgagtac agacatccct tttcagctgt ctaaatatgt ggacaatcag 2580
   cgcctgctgt ccacgtttac ggaatacatc aaaaacatca tcaacactag tattctgaac 2640
   ttgcgttacg agagtaacca tctgattgat ctgagccgtt acgcatctaa aatcaacatc 2700
   ggctcgaagg tgaacttcga tcctatcgac aaaaaccaga ttcaattgtt caacttagaa 2760
   tcgtcaaaga ttgaagttat cttaaaaaat gcgattgtat ataattcaat gtacgaaaat 2820
   ttctctacga gcttttggat tcgtattccg aaatatttca acagtatctc tttaaacaac 2880
   gagtatacta tcatcaattg tatggagaat aacagcgggt ggaaagtgag ccttaactat 2940
   ggtgaaatca tctggactct gcaggacact caagaaatta aacaacgcgt ggtgtttaaa 3000
   tactcacaga tgattaacat ctcggattat attaatcgct ggatttttgt gacaattact 3060
   aacaaccggc tgaacaacag caaaatttac attaacggtc gcctgatcga tcagaaacca 3120
   atcagtaatc tcggtaacat tcacgcatcg aataatatca tgttcaaact ggatggttgt 3180
   cgcgacacgc accgttacat ttggatcaaa tacttcaatt tattcgacaa agaactcaac 3240
   gaaaaggaga ttaaggatct ttatgacaat cagtctaatt cgggtattct gaaagacttt 3300
   tggggtgatt accttcagta cgataaaccg tattatatgt taaacttata tgatccgaat 3360
   aaatacgttg acgtcaacaa cgttggcatt cgtggctata tgtatctgaa agggccgcgt 3420
   ggcagcgtga tgaccactaa catttactta aactcctccc tctatcgcgg tactaaattt 3480
   attatcaaga aatatgcctc tggcaacaag gacaatatcg tacgcaataa cgatcgcgtc 3540
   tacattaacg tggtggtgaa gaataaagaa tatcgtctgg cgaccaatgc tagtcaggcg 3600
   ggcgtggaga aaattctgtc tgcacttgaa atcccggatg tgggtaattt atcccaggtg 3660
   gttgtgatga aaagtaaaaa tgaccaaggg atcaccaata aatgcaaaat gaatctgcaa 3720
   gataacaacg gcaacgacat tggttttatc ggcttccacc aattcaataa tatcgcgaaa 3780
   ctggtggcct caaattggta caaccgtcag attgagcgca gctcccgcac tttaggctgt 3840
   agctgggagt tcattccggt agatgacggt tggggagaac gcccattgta a 3891
<210> 117
   <211> 1296
   <212> PRT
   <213> Artificial Sequence
<220>
   <223> iBoNT/A (H227Y) , E-coli-modified
<400> 117
   Met Pro Phe Val Asn Lys Gln Phe Asn Tyr Lys Asp Pro Val Asn Gly
   1               5                   10                  15
   Val Asp Ile Ala Tyr Ile Lys Ile Pro Asn Ala Gly Gln Met Gln Pro
               20                  25                  30
   Val Lys Ala Phe Lys Ile His Asn Lys Ile Trp Val Ile Pro Glu Arg
           35                  40                  45
   Asp Thr Phe Thr Asn Pro Glu Glu Gly Asp Leu Asn Pro Pro Pro Glu
       50                  55                  60
   Ala Lys Gln Val Pro Val Ser Tyr Tyr Asp Ser Thr Tyr Leu Ser Thr
   65                  70                  75                  80
   Asp Asn Glu Lys Asp Asn Tyr Leu Lys Gly Val Thr Lys Leu Phe Glu
                   85                  90                  95
   Arg Ile Tyr Ser Thr Asp Leu Gly Arg Met Leu Leu Thr Ser Ile Val
               100                 105                 110
   Arg Gly Ile Pro Phe Trp Gly Gly Ser Thr Ile Asp Thr Glu Leu Lys
           115                 120                 125
   Val Ile Asp Thr Asn Cys Ile Asn Val Ile Gln Pro Asp Gly Ser Tyr
       130                 135                 140
   Arg Ser Glu Glu Leu Asn Leu Val Ile Ile Gly Pro Ser Ala Asp Ile
   145                 150                 155                 160
   Ile Gln Phe Glu Cys Lys Ser Phe Gly His Glu Val Leu Asn Leu Thr
                   165                 170                 175
   Arg Asn Gly Tyr Gly Ser Thr Gln Tyr Ile Arg Phe Ser Pro Asp Phe
               180                 185                 190
   Thr Phe Gly Phe Glu Glu Ser Leu Glu Val Asp Thr Asn Pro Leu Leu
           195                 200                 205
   Gly Ala Gly Lys Phe Ala Thr Asp Pro Ala Val Thr Leu Ala His Glu
       210                 215                 220
   Leu Ile Tyr Ala Gly His Arg Leu Tyr Gly Ile Ala Ile Asn Pro Asn
   225                 230                 235                 240
   Arg Val Phe Lys Val Asn Thr Asn Ala Tyr Tyr Glu Met Ser Gly Leu
                   245                 250                 255
   Glu Val Ser Phe Glu Glu Leu Arg Thr Phe Gly Gly His Asp Ala Lys
               260                 265                 270
   Phe Ile Asp Ser Leu Gln Glu Asn Glu Phe Arg Leu Tyr Tyr Tyr Asn
           275                 280                 285
   Lys Phe Lys Asp Ile Ala Ser Thr Leu Asn Lys Ala Lys Ser Ile Val
       290                 295                 300
   Gly Thr Thr Ala Ser Leu Gln Tyr Met Lys Asn Val Phe Lys Glu Lys
   305                 310                 315                 320
   Tyr Leu Leu Ser Glu Asp Thr Ser Gly Lys Phe Ser Val Asp Lys Leu
                   325                 330                 335
   Lys Phe Asp Lys Leu Tyr Lys Met Leu Thr Glu Ile Tyr Thr Glu Asp
               340                 345                 350
   Asn Phe Val Lys Phe Phe Lys Val Leu Asn Arg Lys Thr Tyr Leu Asn
           355                 360                 365
   Phe Asp Lys Ala Val Phe Lys Ile Asn Ile Val Pro Lys Val Asn Tyr
       370                 375                 380
   Thr Ile Tyr Asp Gly Phe Asn Leu Arg Asn Thr Asn Leu Ala Ala Asn
   385 390 395 400
   Phe Asn Gly Gln Asn Thr Glu Ile Asn Asn Met Asn Phe Thr Lys Leu
   405 410 415
   Lys Asn Phe Thr Gly Leu Phe Glu Phe Tyr Lys Leu Leu Cys Val Arg
   420 425 430
   Gly Ile Ile Thr Ser Lys Thr Lys Ser Leu Asp Lys Gly Tyr Asn Lys
   435 440 445
   Ala Leu Asn Asp Leu Cys Ile Lys Val Asn Asn Trp Asp Leu Phe Phe
   450 455 460
   Ser Pro Ser Glu Asp Asn Phe Thr Asn Asp Leu Asn Lys Gly Glu Glu
   465 470 475 480
   Ile Thr Ser Asp Thr Asn Ile Glu Ala Ala Glu Glu Asn Ile Ser Leu
   485 490 495
   Asp Leu Ile Gln Gln Tyr Tyr Leu Thr Phe Asn Phe Asp Asn Glu Pro
   500 505 510
   Glu Asn Ile Ser Ile Glu Asn Leu Ser Ser Asp Ile Ile Gly Gln Leu
   515 520 525
   Glu Leu Met Pro Asn Ile Glu Arg Phe Pro Asn Gly Lys Lys Tyr Glu
   530 535 540
   Leu Asp Lys Tyr Thr Met Phe His Tyr Leu Arg Ala Gln Glu Phe Glu
   545 550 555 560
   His Gly Lys Ser Arg Ile Ala Leu Thr Asn Ser Val Asn Glu Ala Leu
   565 570 575
   Leu Asn Pro Ser Arg Val Tyr Thr Phe Phe Ser Ser Asp Tyr Val Lys
   580 585 590
   Lys Val Asn Lys Ala Thr Glu Ala Ala Met Phe Leu Gly Trp Val Glu
   595 600 605
   Gln Leu Val Tyr Asp Phe Thr Asp Glu Thr Ser Glu Val Ser Thr Thr
   610 615 620
   Asp Lys Ile Ala Asp Ile Thr Ile Ile Ile Pro Tyr Ile Gly Pro Ala
   625 630 635 640
   Leu Asn Ile Gly Asn Met Leu Tyr Lys Asp Asp Phe Val Gly Ala Leu
   645 650 655
   Ile Phe Ser Gly Ala Val Ile Leu Leu Glu Phe Ile Pro Glu Ile Ala
   660 665 670
   Ile Pro Val Leu Gly Thr Phe Ala Leu Val Ser Tyr Ile Ala Asn Lys
   675 680 685
   Val Leu Thr Val Gln Thr Ile Asp Asn Ala Leu Ser Lys Arg Asn Glu
   690 695 700
   Lys Trp Asp Glu Val Tyr Lys Tyr Ile Val Thr Asn Trp Leu Ala Lys
   705 710 715 720
   Val Asn Thr Gln Ile Asp Leu Ile Arg Lys Lys Met Lys Glu Ala Leu
   725 730 735
   Glu Asn Gln Ala Glu Ala Thr Lys Ala Ile Ile Asn Tyr Gln Tyr Asn
   740 745 750
   Gln Tyr Thr Glu Glu Glu Lys Asn Asn Ile Asn Phe Asn Ile Asp Asp
   755 760 765
   Leu Ser Ser Lys Leu Asn Glu Ser Ile Asn Lys Ala Met Ile Asn Ile
   770 775 780
   Asn Lys Phe Leu Asn Gln Cys Ser Val Ser Tyr Leu Met Asn Ser Met
   785 790 795 800
   Ile Pro Tyr Gly Val Lys Arg Leu Glu Asp Phe Asp Ala Ser Leu Lys
   805 810 815
   Asp Ala Leu Leu Lys Tyr Ile Tyr Asp Asn Arg Gly Thr Leu Ile Gly
   820 825 830
   Gln Val Asp Arg Leu Lys Asp Lys Val Asn Asn Thr Leu Ser Thr Asp
   835 840 845
   Ile Pro Phe Gln Leu Ser Lys Tyr Val Asp Asn Gln Arg Leu Leu Ser
       850                 855                 860
   Thr Phe Thr Glu Tyr Ile Lys Asn Ile Ile Asn Thr Ser Ile Leu Asn
   865                 870                 875                 880
   Leu Arg Tyr Glu Ser Asn His Leu Ile Asp Leu Ser Arg Tyr Ala Ser
                   885                 890                 895
   Lys Ile Asn Ile Gly Ser Lys Val Asn Phe Asp Pro Ile Asp Lys Asn
               900                 905                 910
   Gln Ile Gln Leu Phe Asn Leu Glu Ser Ser Lys Ile Glu Val Ile Leu
           915                 920                 925
   Lys Asn Ala Ile Val Tyr Asn Ser Met Tyr Glu Asn Phe Ser Thr Ser
       930                 935                 940
   Phe Trp Ile Arg Ile Pro Lys Tyr Phe Asn Ser Ile Ser Leu Asn Asn
   945                 950                 955                 960
   Glu Tyr Thr Ile Ile Asn Cys Met Glu Asn Asn Ser Gly Trp Lys Val
                   965                 970                 975
   Ser Leu Asn Tyr Gly Glu Ile Ile Trp Thr Leu Gln Asp Thr Gln Glu
               980                 985                 990
   Ile Lys Gln Arg Val Val Phe Lys Tyr Ser Gln Met Ile Asn Ile Ser
           995                 1000                1005
   Asp Tyr Ile Asn Arg Trp Ile Phe Val Thr Ile Thr Asn Asn Arg Leu
       1010                1015                1020
   Asn Asn Ser Lys Ile Tyr Ile Asn Gly Arg Leu Ile Asp Gln Lys Pro
   1025                1030                1035                1040
   Ile Ser Asn Leu Gly Asn Ile His Ala Ser Asn Asn Ile Met Phe Lys
                   1045                1050                1055
   Leu Asp Gly Cys Arg Asp Thr His Arg Tyr Ile Trp Ile Lys Tyr Phe
               1060                1065                1070
   Asn Leu Phe Asp Lys Glu Leu Asn Glu Lys Glu Ile Lys Asp Leu Tyr
           1075                1080                1085
   Asp Asn Gln Ser Asn Ser Gly Ile Leu Lys Asp Phe Trp Gly Asp Tyr
       1090                1095                1100
   Leu Gln Tyr Asp Lys Pro Tyr Tyr Met Leu Asn Leu Tyr Asp Pro Asn
   1105                1110                1115                1120
   Lys Tyr Val Asp Val Asn Asn Val Gly Ile Arg Gly Tyr Met Tyr Leu
                   1125                1130                1135
   Lys Gly Pro Arg Gly Ser Val Met Thr Thr Asn Ile Tyr Leu Asn Ser
               1140                1145                1150
   Ser Leu Tyr Arg Gly Thr Lys Phe Ile Ile Lys Lys Tyr Ala Ser Gly
           1155                1160                1165
   Asn Lys Asp Asn Ile Val Arg Asn Asn Asp Arg Val Tyr Ile Asn Val
       1170                1175                1180
   Val Val Lys Asn Lys Glu Tyr Arg Leu Ala Thr Asn Ala Ser Gln Ala
   1185                1190                1195                1200
   Gly Val Glu Lys Ile Leu Ser Ala Leu Glu Ile Pro Asp Val Gly Asn
                   1205                1210                1215
   Leu Ser Gln Val Val Val Met Lys Ser Lys Asn Asp Gln Gly Ile Thr
               1220                1225                1230
   Asn Lys Cys Lys Met Asn Leu Gln Asp Asn Asn Gly Asn Asp Ile Gly
           1235                1240                1245
   Phe Ile Gly Phe His Gln Phe Asn Asn Ile Ala Lys Leu Val Ala Ser
       1250                1255                1260
   Asn Trp Tyr Asn Arg Gln Ile Glu Arg Ser Ser Arg Thr Leu Gly Cys
   1265                1270                1275                1280
   Ser Trp Glu Phe Ile Pro Val Asp Asp Gly Trp Gly Glu Arg Pro Leu
                   1285                1290                1295
<210> 118
   <211> 4005
   <212> DNA
   <213> Artificial Sequence
<220>
   <223> His-iBoNT/A (H265Y), E. coli-modified
<400> 118
   atgcggggtt ctcatcatca tcatcatcat ggtatggcta gcatgactgg tggacagcaa 60
   atgggtcggg atctgtacga cgatgacgat aaggatccga attcgagctc ccatatgccg 120
   ttcgtaaaca aacagttcaa ctataaagac ccagtcaacg gcgtggacat tgcctatatc 180
   aaaatcccga atgcgggtca aatgcagccc gtgaaagcat ttaaaatcca taacaaaatt 240
   tgggtgatcc cggagcgcga tacgttcacg aacccggaag aaggagattt aaacccaccg 300
   cctgaggcta aacaggtccc ggtgtcttac tatgatagca catacctgag taccgacaat 360
   gaaaaggaca actacctgaa aggtgttacc aaactgttcg agcgcattta ttcgacagat 420
   ctcggtcgca tgttgctgac ttctattgtg cgcggcattc cgttttgggg tggtagcacc 480
   atcgatacag aactcaaagt gattgacacc aactgcatca atgtgattca gcctgatggg 540
   agctaccggt ccgaagagct taacctcgta atcattggcc cgagcgcgga tattatccaa 600
   ttcgaatgta aatcttttgg gcatgaagtc ctgaatctga cgcggaatgg ctatggatcg 660
   acgcagtata ttcgtttttc tccagatttc acatttggat ttgaagaaag cctcgaagtt 720
   gatacgaacc ctcttttagg cgcgggaaaa ttcgcgacgg acccagcggt gaccttggca 780
   catgaactta tttatgccgg gcatcgcttg tatggaatcg ccattaaccc gaaccgtgtt 840
   ttcaaggtga atacgaacgc gtattacgag atgtcgggct tagaagtgtc ctttgaagaa 900
   ctgcgcacgt ttggcggtca tgatgcaaaa tttattgata gtctgcaaga aaacgaattt 960
   cggctgtact attacaataa attcaaagac attgcatcaa ccttaaacaa ggcgaaaagc 1020
   attgtgggta ccacggctag cttacaatat atgaaaaacg ttttcaaaga aaaatacctc 1080
   cttagcgaag acacttccgg caaattctct gtcgataaac tgaaatttga taaactgtat 1140
   aaaatgctca ccgagatcta cacagaggat aactttgtca aattcttcaa ggtcttgaat 1200
   cggaaaacct atctgaactt cgataaagcc gtctttaaga tcaacatcgt accgaaagtt 1260
   aactacacca tctatgatgg ctttaatctg cgcaatacga atctggcggc gaactttaac 1320
   ggccagaaca ccgaaatcaa caacatgaac tttactaaac tgaaaaattt taccggcttg 1380
   tttgaatttt ataagctcct gtgtgtccgc ggtattatca ccagcaaaac caaatccttg 1440
   gataagggct ataacaaggc gctcaatgat ttatgcatca aggtgaacaa ctgggacttg 1500
   tttttctctc catctgaaga taattttact aacgacttga acaaaggaga ggaaattact 1560
   tccgatacca acatcgaagc agcggaagag aatattagcc tggatcttat tcaacaatat 1620
   tacctgacct ttaattttga taacgagcct gagaacattt ccattgagaa tctcagctct 1680
   gacatcatcg gccagctgga actgatgccg aatatcgaac gctttcctaa tggaaagaaa 1740
   tatgaattgg acaaatacac catgttccac tatctccgcg cgcaggagtt tgagcacggc 1800
   aagtctcgta ttgctctgac caattcggta aacgaagccc ttttaaatcc ttcgcgtgtg 1860
   tacacctttt tctcaagcga ttatgttaaa aaagtgaaca aggcgaccga agcggcgatg 1920
   tttttgggat gggtggaaca actggtatat gactttacgg atgaaacttc tgaagtctcg 1980
   accaccgaca aaattgccga tattaccatt atcattccct atattggccc tgcactgaac 2040
   attggtaaca tgctgtataa agatgatttt gtgggcgccc tgatcttttc aggcgctgtt 2100
   atcctgctgg aatttatccc ggaaatcgcc attccagtac tcggtacctt tgcgctggtg 2160
   tcctatatcg caaacaaagt tttgactgtc cagacgatcg acaacgcgct cagtaaacgt 2220
   aacgaaaaat gggatgaggt gtataagtat attgttacca actggctcgc taaagtaaac 2280
   acccagattg acctgattcg caagaagatg aaagaagcgc tggaaaacca agcagaagcg 2340
   accaaagcta ttatcaacta tcaatataac cagtacacag aggaagaaaa gaataacatc 2400
   aacttcaaca tcgacgactt atcttcaaag ctgaatgaat ctattaacaa agcgatgatt 2460
   aatattaaca agttcttgaa ccaatgtagt gtcagctatc tgatgaactc gatgatccca 2520
   tatggtgtga aacgtctgga agacttcgat gcaagcctta aagatgccct tctgaagtat 2580
   atttacgata atcgcggaac tcttattggc caagtggatc gcttaaaaga taaagtcaac 2640
   aacacgctga gtacagacat cccttttcag ctgtctaaat atgtggacaa tcagcgcctg 2700
   ctgtccacgt ttacggaata catcaaaaac atcatcaaca ctagtattct gaacttgcgt 2760
   tacgagagta accatctgat tgatctgagc cgttacgcat ctaaaatcaa catcggctcg 2820
   aaggtgaact tcgatcctat cgacaaaaac cagattcaat tgttcaactt agaatcgtca 2880
   aagattgaag ttatcttaaa aaatgcgatt gtatataatt caatgtacga aaatttctct 2940
   acgagctttt ggattcgtat tccgaaatat ttcaacagta tctctttaaa caacgagtat 3000
   actatcatca attgtatgga gaataacagc gggtggaaag tgagccttaa ctatggtgaa 3060
   atcatctgga ctctgcagga cactcaagaa attaaacaac gcgtggtgtt taaatactca 3120
   cagatgatta acatctcgga ttatattaat cgctggattt ttgtgacaat tactaacaac 3180
   cggctgaaca acagcaaaat ttacattaac ggtcgcctga tcgatcagaa accaatcagt 3240
   aatctcggta acattcacgc atcgaataat atcatgttca aactggatgg ttgtcgcgac 3300
   acgcaccgtt acatttggat caaatacttc aatttattcg acaaagaact caacgaaaag 3360
   gagattaagg atctttatga caatcagtct aattcgggta ttctgaaaga cttttggggt 3420
   gattaccttc agtacgataa accgtattat atgttaaact tatatgatcc gaataaatac 3480
   gttgacgtca acaacgttgg cattcgtggc tatatgtatc tgaaagggcc gcgtggcagc 3540
   gtgatgacca ctaacattta cttaaactcc tccctctatc gcggtactaa atttattatc 3600
   aagaaatatg cctctggcaa caaggacaat atcgtacgca ataacgatcg cgtctacatt 3660
   aacgtggtgg tgaagaataa agaatatcgt ctggcgacca atgctagtca ggcgggcgtg 3720
   gagaaaattc tgtctgcact tgaaatcccg gatgtgggta atttatccca ggtggttgtg 3780
   atgaaaagta aaaatgacca agggatcacc aataaatgca aaatgaatct gcaagataac 3840
   aacggcaacg acattggttt tatcggcttc caccaattca ataatatcgc gaaactggtg 3900
   gcctcaaatt ggtacaaccg tcagattgag cgcagctccc gcactttagg ctgtagctgg 3960
   gagttcattc cggtagatga cggttgggga gaacgcccat tgtaa                 4005
<210> 119
   <211> 1334
   <212> PRT
   <213> Artificial Sequence
<220>
   <223> His-iBoNT/A (H265Y), E. coli-modified
<400> 119
   Met Arg Gly Ser His His His His His His Gly Met Ala Ser Met Thr
    1               5                  10                  15
   Gly Gly Gln Gln Met Gly Arg Asp Leu Tyr Asp Asp Asp Asp Lys Asp
               20                  25                  30
   Pro Asn Ser Ser Ser His Met Pro Phe Val Asn Lys Gln Phe Asn Tyr
           35                  40                  45
   Lys Asp Pro Val Asn Gly Val Asp Ile Ala Tyr Ile Lys Ile Pro Asn
       50                  55                  60
   Ala Gly Gln Met Gln Pro Val Lys Ala Phe Lys Ile His Asn Lys Ile
   65                  70                  75                  80
   Trp Val Ile Pro Glu Arg Asp Thr Phe Thr Asn Pro Glu Glu Gly Asp
                   85                  90                  95
   Leu Asn Pro Pro Pro Glu Ala Lys Gln Val Pro Val Ser Tyr Tyr Asp
               100                 105                 110
   Ser Thr Tyr Leu Ser Thr Asp Asn Glu Lys Asp Asn Tyr Leu Lys Gly
           115                 120                 125
   Val Thr Lys Leu Phe Glu Arg Ile Tyr Ser Thr Asp Leu Gly Arg Met
       130                 135                 140
   Leu Leu Thr Ser Ile Val Arg Gly Ile Pro Phe Trp Gly Gly Ser Thr
   145                 150                 155                 160
   Ile Asp Thr Glu Leu Lys Val Ile Asp Thr Asn Cys Ile Asn Val Ile
                   165                 170                 175
   Gln Pro Asp Gly Ser Tyr Arg Ser Glu Glu Leu Asn Leu Val Ile Ile
               180                 185                 190
   Gly Pro Ser Ala Asp Ile Ile Gln Phe Glu Cys Lys Ser Phe Gly His
           195                 200                 205
   Glu Val Leu Asn Leu Thr Arg Asn Gly Tyr Gly Ser Thr Gln Tyr Ile
       210                 215                 220
   Arg Phe Ser Pro Asp Phe Thr Phe Gly Phe Glu Glu Ser Leu Glu Val
   225                 230                 235                 240
   Asp Thr Asn Pro Leu Leu Gly Ala Gly Lys Phe Ala Thr Asp Pro Ala
                   245                 250                 255
   Val Thr Leu Ala His Glu Leu Ile Tyr Ala Gly His Arg Leu Tyr Gly
               260                 265                 270
   Ile Ala Ile Asn Pro Asn Arg Val Phe Lys Val Asn Thr Asn Ala Tyr
   275 280 285
   Tyr Glu Met Ser Gly Leu Glu Val Ser Phe Glu Glu Leu Arg Thr Phe
   290 295 300
   Gly Gly His Asp Ala Lys Phe Ile Asp Ser Leu Gln Glu Asn Glu Phe
   305 310 315 320
   Arg Leu Tyr Tyr Tyr Asn Lys Phe Lys Asp Ile Ala Ser Thr Leu Asn
   325 330 335
   Lys Ala Lys Ser Ile Val Gly Thr Thr Ala Ser Leu Gln Tyr Met Lys
   340 345 350
   Asn Val Phe Lys Glu Lys Tyr Leu Leu Ser Glu Asp Thr Ser Gly Lys
   355 360 365
   Phe Ser Val Asp Lys Leu Lys Phe Asp Lys Leu Tyr Lys Met Leu Thr
   370 375 380
   Glu Ile Tyr Thr Glu Asp Asn Phe Val Lys Phe Phe Lys Val Leu Asn
   385 390 395 400
   Arg Lys Thr Tyr Leu Asn Phe Asp Lys Ala Val Phe Lys Ile Asn Ile
   405 410 415
   Val Pro Lys Val Asn Tyr Thr Ile Tyr Asp Gly Phe Asn Leu Arg Asn
   420 425 430
   Thr Asn Leu Ala Ala Asn Phe Asn Gly Gln Asn Thr Glu Ile Asn Asn
   435 440 445
   Met Asn Phe Thr Lys Leu Lys Asn Phe Thr Gly Leu Phe Glu Phe Tyr
   450 455 460
   Lys Leu Leu Cys Val Arg Gly Ile Ile Thr Ser Lys Thr Lys Ser Leu
   465 470 475 480
   Asp Lys Gly Tyr Asn Lys Ala Leu Asn Asp Leu Cys Ile Lys Val Asn
   485 490 495
   Asn Trp Asp Leu Phe Phe Ser Pro Ser Glu Asp Asn Phe Thr Asn Asp
   500 505 510
   Leu Asn Lys Gly Glu Glu Ile Thr Ser Asp Thr Asn Ile Glu Ala Ala
   515 520 525
   Glu Glu Asn Ile Ser Leu Asp Leu Ile Gln Gln Tyr Tyr Leu Thr Phe
   530 535 540
   Asn Phe Asp Asn Glu Pro Glu Asn Ile Ser Ile Glu Asn Leu Ser Ser
   545 550 555 560
   Asp Ile Ile Gly Gln Leu Glu Leu Met Pro Asn Ile Glu Arg Phe Pro
   565 570 575
   Asn Gly Lys Lys Tyr Glu Leu Asp Lys Tyr Thr Met Phe His Tyr Leu
   580 585 590
   Arg Ala Gln Glu Phe Glu His Gly Lys Ser Arg Ile Ala Leu Thr Asn
   595 600 605
   Ser Val Asn Glu Ala Leu Leu Asn Pro Ser Arg Val Tyr Thr Phe Phe
   610 615 620
   Ser Ser Asp Tyr Val Lys Lys Val Asn Lys Ala Thr Glu Ala Ala Met
   625 630 635 640
   Phe Leu Gly Trp Val Glu Gln Leu Val Tyr Asp Phe Thr Asp Glu Thr
   645 650 655
   Ser Glu Val Ser Thr Thr Asp Lys Ile Ala Asp Ile Thr Ile Ile Ile
   660 665 670
   Pro Tyr Ile Gly Pro Ala Leu Asn Ile Gly Asn Met Leu Tyr Lys Asp
   675 680 685
   Asp Phe Val Gly Ala Leu Ile Phe Ser Gly Ala Val Ile Leu Leu Glu
   690 695 700
   Phe Ile Pro Glu Ile Ala Ile Pro Val Leu Gly Thr Phe Ala Leu Val
   705 710 715 720
   Ser Tyr Ile Ala Asn Lys Val Leu Thr Val Gln Thr Ile Asp Asn Ala
   725 730 735
   Leu Ser Lys Arg Asn Glu Lys Trp Asp Glu Val Tyr Lys Tyr Ile Val
   740 745 750
   Thr Asn Trp Leu Ala Lys Val Asn Thr Gln Ile Asp Leu Ile Arg Lys
   755 760 765
   Lys Met Lys Glu Ala Leu Glu Asn Gln Ala Glu Ala Thr Lys Ala Ile
   770 775 780
   Ile Asn Tyr Gln Tyr Asn Gln Tyr Thr Glu Glu Glu Lys Asn Asn Ile
   785 790 795 800
   Asn Phe Asn Ile Asp Asp Leu Ser Ser Lys Leu Asn Glu Ser Ile Asn
   805 810 815
   Lys Ala Met Ile Asn Ile Asn Lys Phe Leu Asn Gln Cys Ser Val Ser
   820 825 830
   Tyr Leu Met Asn Ser Met Ile Pro Tyr Gly Val Lys Arg Leu Glu Asp
   835 840 845
   Phe Asp Ala Ser Leu Lys Asp Ala Leu Leu Lys Tyr Ile Tyr Asp Asn
   850 855 860
   Arg Gly Thr Leu Ile Gly Gln Val Asp Arg Leu Lys Asp Lys Val Asn
   865 870 875 880
   Asn Thr Leu Ser Thr Asp Ile Pro Phe Gln Leu Ser Lys Tyr Val Asp
   885 890 895
   Asn Gln Arg Leu Leu Ser Thr Phe Thr Glu Tyr Ile Lys Asn Ile Ile
   900 905 910
   Asn Thr Ser Ile Leu Asn Leu Arg Tyr Glu Ser Asn His Leu Ile Asp
   915 920 925
   Leu Ser Arg Tyr Ala Ser Lys Ile Asn Ile Gly Ser Lys Val Asn Phe
   930 935 940
   Asp Pro Ile Asp Lys Asn Gln Ile Gln Leu Phe Asn Leu Glu Ser Ser
   945 950 955 960
   Lys Ile Glu Val Ile Leu Lys Asn Ala Ile Val Tyr Asn Ser Met Tyr
   965 970 975
   Glu Asn Phe Ser Thr Ser Phe Trp Ile Arg Ile Pro Lys Tyr Phe Asn
   980 985 990
   Ser Ile Ser Leu Asn Asn Glu Tyr Thr Ile Ile Asn Cys Met Glu Asn
   995 1000 1005
   Asn Ser Gly Trp Lys Val Ser Leu Asn Tyr Gly Glu Ile Ile Trp Thr
   1010 1015 1020
   Leu Gln Asp Thr Gln Glu Ile Lys Gln Arg Val Val Phe Lys Tyr Ser
   1025 1030 1035 1040
   Gln Met Ile Asn Ile Ser Asp Tyr Ile Asn Arg Trp Ile Phe Val Thr
   1045 1050 1055
   Ile Thr Asn Asn Arg Leu Asn Asn Ser Lys Ile Tyr Ile Asn Gly Arg
   1060 1065 1070
   Leu Ile Asp Gln Lys Pro Ile Ser Asn Leu Gly Asn Ile His Ala Ser
   1075 1080 1085
   Asn Asn Ile Met Phe Lys Leu Asp Gly Cys Arg Asp Thr His Arg Tyr
   1090 1095 1100
   Ile Trp Ile Lys Tyr Phe Asn Leu Phe Asp Lys Glu Leu Asn Glu Lys
   1105 1110 1115 1120
   Glu Ile Lys Asp Leu Tyr Asp Asn Gln Ser Asn Ser Gly Ile Leu Lys
   1125 1130 1135
   Asp Phe Trp Gly Asp Tyr Leu Gln Tyr Asp Lys Pro Tyr Tyr Met Leu
   1140 1145 1150
   Asn Leu Tyr Asp Pro Asn Lys Tyr Val Asp Val Asn Asn Val Gly Ile
   1155 1160 1165
   Arg Gly Tyr Met Tyr Leu Lys Gly Pro Arg Gly Ser Val Met Thr Thr
   1170 1175 1180
   Asn Ile Tyr Leu Asn Ser Ser Leu Tyr Arg Gly Thr Lys Phe Ile Ile
   1185 1190 1195 1200
   Lys Lys Tyr Ala Ser Gly Asn Lys Asp Asn Ile Val Arg Asn Asn Asp
   1205 1210 1215
   Arg Val Tyr Ile Asn Val Val Val Lys Asn Lys Glu Tyr Arg Leu Ala
                     1220                     1225                     1230
   Thr Asn Ala Ser Gln Ala Gly Val Glu Lys Ile Leu Ser Ala Leu Glu
               1235                     1240                     1245
   Ile Pro Asp Val Gly Asn Leu Ser Gln Val Val Val Met Lys Ser Lys
           1250                     1255                     1260
   Asn Asp Gln Gly Ile Thr Asn Lys Cys Lys Met Asn Leu Gln Asp Asn
   1265                     1270                     1275                     1280
   Asn Gly Asn Asp Ile Gly Phe Ile Gly Phe His Gln Phe Asn Asn Ile
                         1285                     1290                     1295
   Ala Lys Leu Val Ala Ser Asn Trp Tyr Asn Arg Gln Ile Glu Arg Ser
                     1300                     1305                     1310
   Ser Arg Thr Leu Gly Cys Ser Trp Glu Phe Ile Pro Val Asp Asp Gly
               1315                     1320                     1325
   Trp Gly Glu Arg Pro Leu
           1330
<210> 120
   <211> 3939
   <212> DNA
   <213> Artificial Sequence
<220>
   <223> iBoNT/A-KHis (H227Y), E. coli-modified
<400> 120
   atgccgttcg taaacaaaca gttcaactat aaagacccag tcaacggcgt ggacattgcc 60
   tatatcaaaa tcccgaatgc gggtcaaatg cagcccgtga aagcatttaa aatccataac 120
   aaaatttggg tgatcccgga gcgcgatacg ttcacgaacc cggaagaagg agatttaaac 180
   ccaccgcctg aggctaaaca ggtcccggtg tcttactatg atagcacata cctgagtacc 240
   gacaatgaaa aggacaacta cctgaaaggt gttaccaaac tgttcgagcg catttattcg 300
   acagatctcg gtcgcatgtt gctgacttct attgtgcgcg gcattccgtt ttggggtggt 360
   agcaccatcg atacagaact caaagtgatt gacaccaact gcatcaatgt gattcagcct 420
   gatgggagct accggtccga agagcttaac ctcgtaatca ttggcccgag cgcggatatt 480
   atccaattcg aatgtaaatc ttttgggcat gaagtcctga atctgacgcg gaatggctat 540
   ggatcgacgc agtatattcg tttttctcca gatttcacat ttggatttga agaaagcctc 600
   gaagttgata cgaaccctct tttaggcgcg ggaaaattcg cgacggaccc agcggtgacc 660
   ttggcacatg aacttattta tgccgggcat cgcttgtatg gaatcgccat taacccgaac 720
   cgtgttttca aggtgaatac gaacgcgtat tacgagatgt cgggcttaga agtgtccttt 780
   gaagaactgc gcacgtttgg cggtcatgat gcaaaattta ttgatagtct gcaagaaaac 840
   gaatttcggc tgtactatta caataaattc aaagacattg catcaacctt aaacaaggcg 900
   aaaagcattg tgggtaccac ggctagctta caatatatga aaaacgtttt caaagaaaaa 960
   tacctcctta gcgaagacac ttccggcaaa ttctctgtcg ataaactgaa atttgataaa 1020
   ctgtataaaa tgctcaccga gatctacaca gaggataact ttgtcaaatt cttcaaggtc 1080
   ttgaatcgga aaacctatct gaacttcgat aaagccgtct ttaagatcaa catcgtaccg 1140
   aaagttaact acaccatcta tgatggcttt aatctgcgca atacgaatct ggcggcgaac 1200
   tttaacggcc agaacaccga aatcaacaac atgaacttta ctaaactgaa aaattttacc 1260
   ggcttgtttg aattttataa gctcctgtgt gtccgcggta ttatcaccag caaaaccaaa 1320
   tccttggata agggctataa caaggcgctc aatgatttat gcatcaaggt gaacaactgg 1380
   gacttgtttt tctctccatc tgaagataat tttactaacg acttgaacaa aggagaggaa 1440
   attacttccg ataccaacat cgaagcagcg gaagagaata ttagcctgga tcttattcaa 1500
   caatattacc tgacctttaa ttttgataac gagcctgaga acatttccat tgagaatctc 1560
   agctctgaca tcatcggcca gctggaactg atgccgaata tcgaacgctt tcctaatgga 1620
   aagaaatatg aattggacaa atacaccatg ttccactatc tccgcgcgca ggagtttgag 1680
   cacggcaagt ctcgtattgc tctgaccaat tcggtaaacg aagccctttt aaatccttcg 1740
   cgtgtgtaca cctttttctc aagcgattat gttaaaaaag tgaacaaggc gaccgaagcg 1800
   gcgatgtttt tgggatgggt ggaacaactg gtatatgact ttacggatga aacttctgaa 1860
   gtctcgacca ccgacaaaat tgccgatatt accattatca ttccctatat tggccctgca 1920
   ctgaacattg gtaacatgct gtataaagat gattttgtgg gcgccctgat cttttcaggc 1980
   gctgttatcc tgctggaatt tatcccggaa atcgccattc cagtactcgg tacctttgcg 2040
   ctggtgtcct atatcgcaaa caaagttttg actgtccaga cgatcgacaa cgcgctcagt 2100
   aaacgtaacg aaaaatggga tgaggtgtat aagtatattg ttaccaactg gctcgctaaa 2160
   gtaaacaccc agattgacct gattcgcaag aagatgaaag aagcgctgga aaaccaagca 2220
   gaagcgacca aagctattat caactatcaa tataaccagt acacagagga agaaaagaat 2280
   aacatcaact tcaacatcga cgacttatct tcaaagctga atgaatctat taacaaagcg 2340
   atgattaata ttaacaagtt cttgaaccaa tgtagtgtca gctatctgat gaactcgatg 2400
   atcccttacg gtgtgaaacg tctggaagac ttcgatgcaa gccttaaaga tgcccttctg 2460
   aagtatattt acgataatcg cggaactctt attggccaag tggatcgctt aaaagataaa 2520
   gtcaacaaca cgctgagtac agacatccct tttcagctgt ctaaatatgt ggacaatcag 2580
   cgcctgctgt ccacgtttac ggaatacatc aaaaacatca tcaacactag tattctgaac 2640
   ttgcgttacg agagtaacca tctgattgat ctgagccgtt acgcatctaa aatcaacatc 2700
   ggctcgaagg tgaacttcga tcctatcgac aaaaaccaga ttcaattgtt caacttagaa 2760
   tcgtcaaaga ttgaagttat cttaaaaaat gcgattgtat ataattcaat gtacgaaaat 2820
   ttctctacga gcttttggat tcgtattccg aaatatttca acagtatctc tttaaacaac 2880
   gagtatacta tcatcaattg tatggagaat aacagcgggt ggaaagtgag ccttaactat 2940
   ggtgaaatca tctggactct gcaggacact caagaaatta aacaacgcgt ggtgtttaaa 3000
   tactcacaga tgattaacat ctcggattat attaatcgct ggatttttgt gacaattact 3060
   aacaaccggc tgaacaacag caaaatttac attaacggtc gcctgatcga tcagaaacca 3120
   atcagtaatc tcggtaacat tcacgcatcg aataatatca tgttcaaact ggatggttgt 3180
   cgcgacacgc accgttacat ttggatcaaa tacttcaatt tattcgacaa agaactcaac 3240
   gaaaaggaga ttaaggatct ttatgacaat cagtctaatt cgggtattct gaaagacttt 3300
   tggggtgatt accttcagta cgataaaccg tattatatgt taaacttata tgatccgaat 3360
   aaatacgttg acgtcaacaa cgttggcatt cgtggctata tgtatctgaa agggccgcgt 3420
   ggcagcgtga tgaccactaa catttactta aactcctccc tctatcgcgg tactaaattt 3480
   attatcaaga aatatgcctc tggcaacaag gacaatatcg tacgcaataa cgatcgcgtc 3540
   tacattaacg tggtggtgaa gaataaagaa tatcgtctgg cgaccaatgc tagtcaggcg 3600
   ggcgtggaga aaattctgtc tgcacttgaa atcccggatg tgggtaattt atcccaggtg 3660
   gttgtgatga aaagtaaaaa tgaccaaggg atcaccaata aatgcaaaat gaatctgcaa 3720
   gataacaacg gcaacgacat tggttttatc ggcttccacc aattcaataa tatcgcgaaa 3780
   ctggtggcct caaattggta caaccgtcag attgagcgca gctcccgcac tttaggctgt 3840
   agctgggagt tcattccggt agatgacggt tggggagaac gcccattgaa agtcgacaag 3900
   cttgcggccg cactcgagca ccaccaccac caccactga                        3939
<210> 121
   <211> 1312
   <212> PRT
   <213> Artificial Sequence
<220>
   <223> iBoNT/A-KHis (H227Y), E. coli-modified
<400> 121
   Met Pro Phe Val Asn Lys Gln Phe Asn Tyr Lys Asp Pro Val Asn Gly
   1               5                   10                  15
   Val Asp Ile Ala Tyr Ile Lys Ile Pro Asn Ala Gly Gln Met Gln Pro
               20                  25                  30
   Val Lys Ala Phe Lys Ile His Asn Lys Ile Trp Val Ile Pro Glu Arg
           35                  40                  45
   Asp Thr Phe Thr Asn Pro Glu Glu Gly Asp Leu Asn Pro Pro Pro Glu
       50                  55                  60
   Ala Lys Gln Val Pro Val Ser Tyr Tyr Asp Ser Thr Tyr Leu Ser Thr
   65                  70                  75                  80
   Asp Asn Glu Lys Asp Asn Tyr Leu Lys Gly Val Thr Lys Leu Phe Glu
                   85                  90                  95
   Arg Ile Tyr Ser Thr Asp Leu Gly Arg Met Leu Leu Thr Ser Ile Val
               100                 105                 110
   Arg Gly Ile Pro Phe Trp Gly Gly Ser Thr Ile Asp Thr Glu Leu Lys
           115                 120                 125
   Val Ile Asp Thr Asn Cys Ile Asn Val Ile Gln Pro Asp Gly Ser Tyr
       130                 135                 140
   Arg Ser Glu Glu Leu Asn Leu Val Ile Ile Gly Pro Ser Ala Asp Ile
   145                 150                 155                 160
   Ile Gln Phe Glu Cys Lys Ser Phe Gly His Glu Val Leu Asn Leu Thr
                   165                 170                 175
   Arg Asn Gly Tyr Gly Ser Thr Gln Tyr Ile Arg Phe Ser Pro Asp Phe
               180                 185                 190
   Thr Phe Gly Phe Glu Glu Ser Leu Glu Val Asp Thr Asn Pro Leu Leu
           195                 200                 205
   Gly Ala Gly Lys Phe Ala Thr Asp Pro Ala Val Thr Leu Ala His Glu
       210                 215                 220
   Leu Ile Tyr Ala Gly His Arg Leu Tyr Gly Ile Ala Ile Asn Pro Asn
   225                 230                 235                 240
   Arg Val Phe Lys Val Asn Thr Asn Ala Tyr Tyr Glu Met Ser Gly Leu
                   245                 250                 255
   Glu Val Ser Phe Glu Glu Leu Arg Thr Phe Gly Gly His Asp Ala Lys
               260                 265                 270
   Phe Ile Asp Ser Leu Gln Glu Asn Glu Phe Arg Leu Tyr Tyr Tyr Asn
           275                 280                 285
   Lys Phe Lys Asp Ile Ala Ser Thr Leu Asn Lys Ala Lys Ser Ile Val
       290                 295                 300
   Gly Thr Thr Ala Ser Leu Gln Tyr Met Lys Asn Val Phe Lys Glu Lys
   305                 310                 315                 320
   Tyr Leu Leu Ser Glu Asp Thr Ser Gly Lys Phe Ser Val Asp Lys Leu
                   325                 330                 335
   Lys Phe Asp Lys Leu Tyr Lys Met Leu Thr Glu Ile Tyr Thr Glu Asp
               340                 345                 350
   Asn Phe Val Lys Phe Phe Lys Val Leu Asn Arg Lys Thr Tyr Leu Asn
           355                 360                 365
   Phe Asp Lys Ala Val Phe Lys Ile Asn Ile Val Pro Lys Val Asn Tyr
       370                 375                 380
   Thr Ile Tyr Asp Gly Phe Asn Leu Arg Asn Thr Asn Leu Ala Ala Asn
   385                 390                 395                 400
   Phe Asn Gly Gln Asn Thr Glu Ile Asn Asn Met Asn Phe Thr Lys Leu
                   405                 410                 415
   Lys Asn Phe Thr Gly Leu Phe Glu Phe Tyr Lys Leu Leu Cys Val Arg
               420                 425                 430
   Gly Ile Ile Thr Ser Lys Thr Lys Ser Leu Asp Lys Gly Tyr Asn Lys
           435                 440                 445
   Ala Leu Asn Asp Leu Cys Ile Lys Val Asn Asn Trp Asp Leu Phe Phe
       450                 455                 460
   Ser Pro Ser Glu Asp Asn Phe Thr Asn Asp Leu Asn Lys Gly Glu Glu
   465                 470                 475                 480
   Ile Thr Ser Asp Thr Asn Ile Glu Ala Ala Glu Glu Asn Ile Ser Leu
                   485                 490                 495
   Asp Leu Ile Gln Gln Tyr Tyr Leu Thr Phe Asn Phe Asp Asn Glu Pro
               500                 505                 510
   Glu Asn Ile Ser Ile Glu Asn Leu Ser Ser Asp Ile Ile Gly Gln Leu
           515                 520                 525
   Glu Leu Met Pro Asn Ile Glu Arg Phe Pro Asn Gly Lys Lys Tyr Glu
       530                 535                 540
   Leu Asp Lys Tyr Thr Met Phe His Tyr Leu Arg Ala Gln Glu Phe Glu
   545                 550                 555                 560
   His Gly Lys Ser Arg Ile Ala Leu Thr Asn Ser Val Asn Glu Ala Leu
                   565                 570                 575
   Leu Asn Pro Ser Arg Val Tyr Thr Phe Phe Ser Ser Asp Tyr Val Lys
               580                 585                 590
   Lys Val Asn Lys Ala Thr Glu Ala Ala Met Phe Leu Gly Trp Val Glu
           595                 600                 605
   Gln Leu Val Tyr Asp Phe Thr Asp Glu Thr Ser Glu Val Ser Thr Thr
   610 615 620
   Asp Lys Ile Ala Asp Ile Thr Ile Ile Ile Pro Tyr Ile Gly Pro Ala
   625 630 635 640
   Leu Asn Ile Gly Asn Met Leu Tyr Lys Asp Asp Phe Val Gly Ala Leu
   645 650 655
   Ile Phe Ser Gly Ala Val Ile Leu Leu Glu Phe Ile Pro Glu Ile Ala
   660 665 670
   Ile Pro Val Leu Gly Thr Phe Ala Leu Val Ser Tyr Ile Ala Asn Lys
   675 680 685
   Val Leu Thr Val Gln Thr Ile Asp Asn Ala Leu Ser Lys Arg Asn Glu
   690 695 700
   Lys Trp Asp Glu Val Tyr Lys Tyr Ile Val Thr Asn Trp Leu Ala Lys
   705 710 715 720
   Val Asn Thr Gln Ile Asp Leu Ile Arg Lys Lys Met Lys Glu Ala Leu
   725 730 735
   Glu Asn Gln Ala Glu Ala Thr Lys Ala Ile Ile Asn Tyr Gln Tyr Asn
   740 745 750
   Gln Tyr Thr Glu Glu Glu Lys Asn Asn Ile Asn Phe Asn Ile Asp Asp
   755 760 765
   Leu Ser Ser Lys Leu Asn Glu Ser Ile Asn Lys Ala Met Ile Asn Ile
   770 775 780
   Asn Lys Phe Leu Asn Gln Cys Ser Val Ser Tyr Leu Met Asn Ser Met
   785 790 795 800
   Ile Pro Tyr Gly Val Lys Arg Leu Glu Asp Phe Asp Ala Ser Leu Lys
   805 810 815
   Asp Ala Leu Leu Lys Tyr Ile Tyr Asp Asn Arg Gly Thr Leu Ile Gly
   820 825 830
   Gln Val Asp Arg Leu Lys Asp Lys Val Asn Asn Thr Leu Ser Thr Asp
   835 840 845
   Ile Pro Phe Gln Leu Ser Lys Tyr Val Asp Asn Gln Arg Leu Leu Ser
   850 855 860
   Thr Phe Thr Glu Tyr Ile Lys Asn Ile Ile Asn Thr Ser Ile Leu Asn
   865 870 875 880
   Leu Arg Tyr Glu Ser Asn His Leu Ile Asp Leu Ser Arg Tyr Ala Ser
   885 890 895
   Lys Ile Asn Ile Gly Ser Lys Val Asn Phe Asp Pro Ile Asp Lys Asn
   900 905 910
   Gln Ile Gln Leu Phe Asn Leu Glu Ser Ser Lys Ile Glu Val Ile Leu
   915 920 925
   Lys Asn Ala Ile Val Tyr Asn Ser Met Tyr Glu Asn Phe Ser Thr Ser
   930 935 940
   Phe Trp Ile Arg Ile Pro Lys Tyr Phe Asn Ser Ile Ser Leu Asn Asn
   945 950 955 960
   Glu Tyr Thr Ile Ile Asn Cys Met Glu Asn Asn Ser Gly Trp Lys Val
   965 970 975
   Ser Leu Asn Tyr Gly Glu Ile Ile Trp Thr Leu Gln Asp Thr Gln Glu
   980 985 990
   Ile Lys Gln Arg Val Val Phe Lys Tyr Ser Gln Met Ile Asn Ile Ser
   995 1000 1005
   Asp Tyr Ile Asn Arg Trp Ile Phe Val Thr Ile Thr Asn Asn Arg Leu
   1010 1015 1020
   Asn Asn Ser Lys Ile Tyr Ile Asn Gly Arg Leu Ile Asp Gln Lys Pro
   1025 1030 1035 1040
   Ile Ser Asn Leu Gly Asn Ile His Ala Ser Asn Asn Ile Met Phe Lys
   1045 1050 1055
   Leu Asp Gly Cys Arg Asp Thr His Arg Tyr Ile Trp Ile Lys Tyr Phe
   1060 1065 1070
   Asn Leu Phe Asp Lys Glu Leu Asn Glu Lys Glu Ile Lys Asp Leu Tyr
   1075 1080 1085
   Asp Asn Gln Ser Asn Ser Gly Ile Leu Lys Asp Phe Trp Gly Asp Tyr
   1090 1095 1100
   Leu Gln Tyr Asp Lys Pro Tyr Tyr Met Leu Asn Leu Tyr Asp Pro Asn
   1105 1110 1115 1120
   Lys Tyr Val Asp Val Asn Asn Val Gly Ile Arg Gly Tyr Met Tyr Leu
   1125 1130 1135
   Lys Gly Pro Arg Gly Ser Val Met Thr Thr Asn Ile Tyr Leu Asn Ser
   1140 1145 1150
   Ser Leu Tyr Arg Gly Thr Lys Phe Ile Ile Lys Lys Tyr Ala Ser Gly
   1155 1160 1165
   Asn Lys Asp Asn Ile Val Arg Asn Asn Asp Arg Val Tyr Ile Asn Val
   1170 1175 1180
   Val Val Lys Asn Lys Glu Tyr Arg Leu Ala Thr Asn Ala Ser Gln Ala
   1185 1190 1195 1200
   Gly Val Glu Lys Ile Leu Ser Ala Leu Glu Ile Pro Asp Val Gly Asn
   1205 1210 1215
   Leu Ser Gln Val Val Val Met Lys Ser Lys Asn Asp Gln Gly Ile Thr
   1220 1225 1230
   Asn Lys Cys Lys Met Asn Leu Gln Asp Asn Asn Gly Asn Asp Ile Gly
   1235 1240 1245
   Phe Ile Gly Phe His Gln Phe Asn Asn Ile Ala Lys Leu Val Ala Ser
   1250 1255 1260
   Asn Trp Tyr Asn Arg Gln Ile Glu Arg Ser Ser Arg Thr Leu Gly Cys
   1265 1270 1275 1280
   Ser Trp Glu Phe Ile Pro Val Asp Asp Gly Trp Gly Glu Arg Pro Leu
   1285 1290 1295
   Lys Val Asp Lys Leu Ala Ala Ala Leu Glu His His His His His His
   1300 1305 1310
<210> 122
   <211> 3891
   <212> DNA
   <213> Artificial Sequence
<220>
   <223> BoNT/A, E. coli-modified 1-3
<400> 122
   atgccgttcg ttaacaaaca gttcaactac aaggatccgg ttaacggcgt tgacatcgct 60
   tacatcaaaa tcccgaacgc tggccagatg cagccggtta aagcattcaa gattcacaac 120
   aaaatctggg taatcccgga acgtgacacc ttcaccaacc cggaagaagg cgacctgaac 180
   ccgccgccgg aagctaaaca ggttccggtt tcctactacg actccaccta cctgtccacc 240
   gacaacgaga aggacaacta cctgaaaggc gttaccaaac tgttcgaacg tatctactcc 300
   accgacctgg gccgtatgct gctgacctcc atcgttcgtg gcatcccgtt ctggggcggc 360
   tccaccatcg acaccgaact gaaagttatc gacaccaact gcatcaacgt tatccagccg 420
   gacggctcct accgttccga agaactgaac ctggttatca tcggcccgtc cgctgacatc 480
   atccagttcg aatgcaaatc cttcggccac gaagttctga acctgacccg taacggctac 540
   ggctccaccc agtacatccg tttctccccg gacttcacct tcggcttcga agaatccctg 600
   gaagttgaca ccaacccgct gctgggcgct ggcaaattcg ctaccgaccc ggctgttacc 660
   ctggctcacg aactgatcca cgctggccac cgtctgtacg gcatcgctat caacccgaac 720
   cgtgtattta aagttaacac caacgcttac tacgaaatgt ccggcctgga agtttccttc 780
   gaagaactgc gtaccttcgg cggccacgac gctaaattca tcgactccct gcaagagaac 840
   gaatttcgtc tgtactatta caacaaattc aaagacatcg cttccaccct gaacaaagct 900
   aaatccatcg ttggcaccac cgcttccctg caatacatga agaacgtttt caaagagaag 960
   tacctgctgt ccgaagacac ctccggcaaa ttctccgttg acaaactgaa attcgacaaa 1020
   ctgtacaaaa tgctgaccga aatctacacc gaagacaact tcgttaaatt cttcaaagtt 1080
   ctgaaccgta agacttacct gaacttcgac aaagctgttt tcaagattaa catcgttccg 1140
   aaagttaact acaccatcta cgacggcttc aacctgcgta acaccaacct ggctgctaac 1200
   ttcaacggcc agaacaccga aatcaacaac atgaacttca ccaaactgaa gaacttcacc 1260
   ggtctgttcg aattttacaa actgctgtgc gttcgtggca tcatcacctc caagactaaa 1320
   tccctggaca aaggctacaa caaagctctg aacgacctgt gcatcaaagt taacaactgg 1380
   gacctgttct tctccccgtc cgaagacaac ttcaccaacg acctgaacaa aggcgaagaa 1440
   atcacctccg acaccaacat cgaagctgct gaagagaaca tctccctgga cctgatccag 1500
   cagtactacc tgaccttcaa cttcgacaac gaaccggaga acatctccat cgaaaacctg 1560
   tcctccgaca tcatcggcca gctggaactg atgccgaaca tcgaacgttt cccgaacggc 1620
   aagaagtatg aactggacaa atacaccatg ttccactacc tgcgtgctca ggaattcgaa 1680
   cacggcaaat cccgtatcgc tctgaccaac tccgttaacg aagctctgct gaacccgtcc 1740
   cgtgtttaca ccttcttctc ctccgactac gttaagaagg ttaacaaagc taccgaagct 1800
   gctatgttcc tgggctgggt tgaacagctg gtttacgact tcaccgacga aacctccgaa 1860
   gtttccacca ccgacaagat tgctgacatc accatcatta tcccgtacat cggcccggct 1920
   ctgaacatcg gcaacatgct gtataaagac gacttcgttg gcgctctgat cttctccggc 1980
   gctgttatcc tgctggaatt tatcccggaa atcgctatcc cggttctggg caccttcgct 2040
   ctggtttcct acatcgctaa caaagttctg accgttcaga ccatcgacaa cgctctgtcc 2100
   aaacgtaacg agaagtggga cgaagtttac aaatacatcg ttaccaactg gctggctaaa 2160
   gttaacaccc agatcgacct gatccgtaag aagatgaaag aagctctgga aaaccaggct 2220
   gaagctacca aagctatcat caactaccag tacaaccagt acaccgaaga ggaaaagaac 2280
   aacatcaact tcaacatcga cgacctgagc tccaaactga acgaatccat caacaaagct 2340
   atgatcaaca tcaacaaatt cctgaaccag tgctccgttt cctacctgat gaactccatg 2400
   atcccgtacg gcgttaaacg tctggaagac ttcgacgctt ccctgaaaga cgctctgctg 2460
   aaatacatct acgacaaccg cggcaccctg atcggccagg ttgaccgtct gaaagacaaa 2520
   gttaacaaca ccctgtccac cgacatcccg ttccagctgt ccaaatacgt agacaaccag 2580
   cgtctgctgt ccaccttcac cgaatacatc aagaacatca tcaacacctc catcctgaac 2640
   ctgcgttacg aatccaacca cctgatcgac ctgtcccgtt acgctagcaa aatcaacatc 2700
   ggctccaaag ttaacttcga cccgatcgac aagaaccaga tccagctgtt caacctggaa 2760
   tcctccaaga ttgaagttat cctgaagaac gcaattgttt acaactccat gtacgagaac 2820
   ttctccacct ccttctggat tcgtatcccg aaatacttca actccatctc cctgaacaac 2880
   gaatacacca tcatcaactg catggagaac aactccggct ggaaagtttc cctgaactac 2940
   ggcgaaatca tctggaccct gcaagacacc caggaaatca aacagcgtgt tgttttcaaa 3000
   tactcccaga tgatcaacat ctccgactac atcaaccgtt ggatcttcgt taccatcacc 3060
   aacaaccgtc tgaacaactc caaaatctac atcaacggcc gtctgatcga ccagaaaccg 3120
   atctccaacc tgggcaacat ccacgcttcc aacaacatca tgttcaaact ggacggctgc 3180
   cgtgacaccc accgttacat ctggatcaaa tacttcaacc tgttcgacaa agaactgaac 3240
   gagaaggaaa tcaaagacct gtacgacaac cagtccaact ccggcatcct gaaagacttc 3300
   tggggcgact acctgcagta tgacaaaccg tactacatgc tgaacctgta cgacccgaac 3360
   aaatacgttg acgttaacaa cgttggcatc cgtggctaca tgtacctgaa aggcccgcgt 3420
   ggctccgtta tgaccaccaa catctacctg aactcctccc tgtaccgtgg caccaaattc 3480
   atcatcaaga agtacgcttc cggcaacaaa gacaacatcg ttcgtaacaa cgaccgtgtt 3540
   tacatcaacg ttgtagttaa gaacaaagaa taccgtctgg ctaccaacgc ttcccaggct 3600
   ggcgttgaga agattctgtc cgctctggaa atcccggacg ttggcaacct gagccaggtt 3660
   gtagttatga aatccaagaa cgaccagggc atcaccaaca aatgcaaaat gaacctgcaa 3720
   gacaacaacg gcaacgacat cggcttcatc ggcttccacc agttcaacaa catcgctaaa 3780
   ctggttgctt ccaactggta caaccgtcag atcgaacgtt cctcccgtac cctgggctgc 3840
   tcctgggaat ttatcccggt tgacgacggc tggggcgaac gtccgctgta a          3891
<210> 123
   <211> 3891
   <212> DNA
   <213> Artificial Sequence
<220>
   <223> BoNT/A, E. coli-modified 2-3
<400> 123
   atgccgttcg ttaacaaaca gttcaactac aaagacccgg ttaacggcgt tgacatcgct 60
   tacatcaaaa tcccgaacgc tggccagatg cagccggtta aagcattcaa gattcacaac 120
   aaaatctggg taatcccgga acgtgacacc ttcaccaacc cggaagaagg cgacctgaac 180
   ccgccgccgg aagctaaaca ggttccggtt tcctactacg actccaccta cctgtccacc 240
   gacaacgaga aggacaacta cctgaaaggc gttaccaaac tgttcgaacg tatctactcc 300
   accgacctgg gccgtatgct gctgactagc atcgttcgtg gcatcccgtt ctggggcggc 360
   tccaccatcg acaccgaact gaaagttatc gacaccaact gcatcaacgt tatccagccg 420
   gacggctcct accgttccga agaactgaac ctggttatca tcggcccgtc cgctgacatc 480
   atccagttcg aatgcaaatc cttcggccac gaagttctga acctgacccg taacggctac 540
   ggctccaccc agtacatccg tttctccccg gacttcacct tcggcttcga agaatccctg 600
   gaagttgaca ccaacccgct gctgggcgct ggcaaattcg ctaccgaccc ggctgttacc 660
   ctggctcacg aactgatcca cgctggccac cgtctgtacg gcatcgctat caacccgaac 720
   cgtgtattca aagttaacac caacgcttac tacgaaatgt ccggcctgga agtttccttc 780
   gaagaactgc gtaccttcgg cggccacgac gctaaattca tcgactccct gcaagaaaac 840
   gaattccgtc tgtactatta caacaaattc aaagacatcg cttccaccct gaacaaagct 900
   aaatccatcg ttggcaccac cgcttccctg cagtacatga agaacgtttt caaagagaag 960
   tacctgctgt ccgaagacac ctccggcaaa ttctccgttg acaaactgaa attcgacaaa 1020
   ctgtacaaga tgctgaccga aatctacacc gaagacaact tcgttaaatt cttcaaagtt 1080
   ctgaaccgta aaacctacct gaacttcgac aaagctgttt tcaagattaa catcgttccg 1140
   aaagttaact acaccatcta cgacggcttc aacctgcgta acaccaacct ggctgctaac 1200
   ttcaacggcc agaacaccga aatcaacaac atgaacttca ccaaactgaa gaacttcacc 1260
   ggtctgttcg aattctacaa actgctgtgc gttcgtggca tcatcacctc caaaaccaaa 1320
   tccctggaca aaggctacaa caaagctctg aacgacctgt gcatcaaagt taacaactgg 1380
   gacctgttct tctctccgtc cgaagacaac ttcaccaacg acctgaacaa aggcgaagaa 1440
   atcacctccg acaccaacat cgaagctgct gaagagaaca tctccctgga cctgatccag 1500
   cagtactacc tgaccttcaa cttcgacaac gaaccggaaa acatctccat cgagaacctg 1560
   tcctccgaca tcatcggcca gctggaactg atgccgaaca tcgaacgttt cccgaacggc 1620
   aagaagtatg aactggacaa atacaccatg ttccactacc tgcgtgctca ggaattcgaa 1680
   cacggcaaat cccgtatcgc tctgaccaac tccgttaacg aagctctgct gaacccgtcc 1740
   cgtgtttaca ccttcttctc ctccgactac gttaagaagg ttaacaaagc taccgaagct 1800
   gctatgttcc tgggctgggt tgaacagctg gtttacgact tcaccgacga aacctccgaa 1860
   gtttccacca ccgacaaaat cgctgacatc accatcatta tcccgtacat cggtccggct 1920
   ctgaacatcg gcaacatgct gtataaagac gacttcgttg gcgctctgat cttctccggc 1980
   gctgttatcc tgctggaatt catcccggaa atcgctatcc cggttctggg caccttcgct 2040
   ctggtttcct acatcgctaa caaagttctg accgttcaga ccatcgacaa cgctctgtcc 2100
   aaacgtaacg agaagtggga cgaagtttac aaatacatcg ttaccaactg gctggctaaa 2160
   gttaacaccc agatcgacct gatccgtaag aagatgaaag aagctctgga gaaccaggct 2220
   gaagctacca aagctatcat caactaccag tacaaccagt acaccgaaga agagaagaac 2280
   aacatcaact tcaacatcga cgacctgtcc tccaaactga acgaatccat caacaaagct 2340
   atgatcaaca tcaacaaatt cctgaaccag tgctccgttt cctacctgat gaactccatg 2400
   atcccgtacg gcgttaaacg tctggaagac ttcgacgctt ccctgaaaga cgctctgctg 2460
   aaatacatct acgacaaccg cggcaccctg atcggccagg ttgaccgtct gaaagacaaa 2520
   gttaacaaca ccctgtccac cgacatcccg ttccagctgt ccaaatacgt agacaaccag 2580
   cgtctgctgt ccaccttcac cgaatacatc aagaacatca tcaacacctc catcctgaac 2640
   ctgcgttacg aatccaacca cctgatcgac ctgtcccgtt acgctagcaa aatcaacatc 2700
   ggctccaaag ttaacttcga cccgatcgac aagaaccaga tccagctgtt caacctggaa 2760
   tcctccaaga ttgaagttat cctgaagaac gcaattgttt acaactccat gtacgagaac 2820
   ttctccacct ccttctggat ccgtatcccg aaatacttca actccatctc cctgaacaac 2880
   gaatacacca tcatcaactg catggagaac aactccggct ggaaagtttc cctgaactac 2940
   ggcgaaatca tctggaccct gcaagacacc caggaaatca aacagcgtgt tgtttttaaa 3000
   tactcccaga tgatcaacat ctccgactac atcaaccgtt ggatcttcgt taccatcacc 3060
   aacaaccgtc tgaacaactc caaaatctac atcaacggcc gtctgatcga ccagaaaccg 3120
   atctccaacc tgggcaacat ccacgcttcc aacaacatca tgttcaaact ggacggctgc 3180
   cgtgacaccc accgttacat ctggatcaaa tacttcaacc tgttcgacaa agaactgaac 3240
   gagaaggaaa tcaaagacct gtacgacaac cagtccaact ccggcatcct gaaagacttc 3300
   tggggcgact acctgcaata tgacaaaccg tactacatgc tgaacctgta cgacccgaac 3360
   aaatacgttg acgttaacaa cgttggcatc cgtggctaca tgtacctgaa aggcccgcgt 3420
   ggctccgtta tgaccaccaa catctacctg aactcctccc tgtaccgtgg caccaaattc 3480
   atcatcaaga agtacgcttc cggcaacaaa gacaacatcg ttcgtaacaa cgaccgtgtt 3540
   tacatcaacg ttgtagttaa gaacaaagaa taccgtctgg ctaccaacgc ttcccaggct 3600
   ggcgttgaga agattctgtc cgctctggaa atcccggacg ttggcaacct gtcccaagtt 3660
   gtagttatga aatccaagaa cgaccagggc atcaccaaca agtgcaagat gaacctgcaa 3720
   gacaacaacg gcaacgacat cggcttcatc ggcttccacc agttcaacaa catcgctaaa 3780
   ctggttgctt ccaactggta caaccgtcag atcgaacgtt cctcccgtac cctgggctgc 3840
   tcctgggaat tcatcccggt tgacgacggc tggggcgaac gtccgctgta a          3891
<210> 124
   <211> 3891
   <212> DNA
   <213>. Artificial Sequence
<220>
   <223> BoNT/A, E. coli-modified 3-3
<400> 124
   atgccgttcg ttaacaaaca gttcaactac aaagacccgg ttaacggcgt tgacatcgct 60
   tacatcaaaa tcccgaacgc tggccagatg cagccggtta aagcattcaa gatccacaac 120
   aaaatctggg taatcccgga acgtgacacc ttcaccaacc cggaagaagg cgacctgaac 180
   ccgccgccgg aagctaaaca ggttccggtt tcctactacg actccaccta cctgagcacc 240
   gacaacgaga aggacaacta cctgaaaggc gttaccaaac tgttcgaacg tatctactcc 300
   accgacctgg gccgtatgct gctgacctcc atcgttcgtg gcatcccgtt ctggggcggt 360
   agcaccatcg acaccgaact gaaagttatc gacaccaact gcatcaacgt tatccagccg 420
   gacggctcct accgttccga agaactgaac ctggttatca tcggcccgtc cgctgacatc 480
   atccagttcg aatgcaaatc cttcggccac gaagttctga acctgacccg taacggctac 540
   ggctccaccc agtacatccg tttctccccg gacttcacct tcggcttcga agaatccctg 600
   gaagttgaca ccaacccgct gctgggcgct ggcaaattcg ctaccgaccc ggctgttacc 660
   ctggctcacg aactgatcca cgctggccac cgtctgtacg gcatcgctat caacccgaac 720
   cgtgttttca aagttaacac caacgcttac tacgaaatgt ccggcctgga agtttccttc 780
   gaagaactgc gtaccttcgg cggccacgac gctaaattca tcgactccct gcaggagaac 840
   gaattccgtc tgtactatta caacaaattc aaagacatcg cttccaccct gaacaaagct 900
   aaatccatcg ttggcaccac cgcttccctg cagtacatga agaacgtttt caaagagaag 960
   tacctgctgt ccgaagacac ctccggcaaa ttctccgttg acaaactgaa attcgacaaa 1020
   ctgtacaaga tgctgaccga aatctacacc gaagacaact tcgttaaatt cttcaaagtt 1080
   ctgaaccgta aaacctacct gaacttcgac aaagctgttt tcaagatcaa catcgttccg 1140
   aaagttaact acaccatcta cgacggcttc aacctgcgta acaccaacct ggctgctaac 1200
   ttcaacggcc agaacaccga aatcaacaac atgaacttca ccaaactgaa gaacttcacc 1260
   ggcctgttcg aattctacaa actgctgtgc gttcgtggca tcatcacctc caaaaccaaa 1320
   tccctggaca aaggctacaa caaagctctg aacgacctgt gcatcaaagt taacaactgg 1380
   gacctgttct tctctccgtc cgaagacaac ttcaccaacg acctgaacaa aggcgaagaa 1440
   atcacctccg acaccaacat cgaagctgca gaagagaaca tctccctgga cctgatccag 1500
   cagtactacc tgaccttcaa cttcgacaac gaaccggaga acatctccat cgagaacctg 1560
   tcctccgaca tcatcggcca gctggaactg atgccgaaca tcgaacgttt cccgaacggc 1620
   aagaagtatg aactggacaa atacaccatg ttccactacc tgcgtgctca ggaattcgaa 1680
   cacggcaaat cccgtatcgc tctgaccaac tccgttaacg aagctctgct gaacccgtcc 1740
   cgtgtttaca ccttcttctc ctccgactac gttaagaagg ttaacaaagc taccgaagct 1800
   gctatgttcc tgggctgggt tgaacagctg gtttacgact tcaccgacga aacctccgaa 1860
   gtttccacca ccgacaagat cgctgacatc accatcatca tcccgtacat cggtccggct 1920
   ctgaacatcg gcaacatgct gtataaagac gacttcgttg gcgctctgat cttctccggc 1980
   gctgttatcc tgctggaatt catcccggaa atcgctatcc cggttctggg caccttcgct 2040
   ctggtttcct acatcgctaa caaagttctg accgttcaga ccatcgacaa cgctctgtcc 2100
   aaacgtaacg agaagtggga cgaagtttac aaatacatcg ttaccaactg gctggctaaa 2160
   gttaacaccc agatcgacct gatccgtaag aagatgaaag aagctctgga gaaccaggct 2220
   gaagctacca aagctatcat caactaccag tacaaccagt acaccgaaga agagaagaac 2280
   aacatcaact tcaacatcga cgacctgtcc tccaaactga acgaatccat caacaaagct 2340
   atgatcaaca tcaacaaatt cctgaaccag tgctccgttt cctacctgat gaactccatg 2400
   atcccgtacg gcgttaaacg tctggaagac ttcgacgctt ccctgaaaga cgctctgctg 2460
   aaatacatct acgacaaccg cggcaccctg atcggccagg ttgaccgtct gaaagacaaa 2520
   gttaacaaca ccctgtccac cgacatcccg ttccagctgt ccaaatacgt agacaaccag 2580
   cgtctgctgt ccaccttcac cgaatacatc aagaacatca tcaacacctc catcctgaac 2640
   ctgcgttacg aatccaacca cctgatcgac ctgtcccgtt acgctagcaa gatcaacatc 2700
   ggctccaaag ttaacttcga cccgatcgac aagaaccaga tccagctgtt caacctggaa 2760
   tcctccaaga tcgaagttat cctgaagaac gctatcgttt acaactccat gtacgagaac 2820
   ttctccacct ccttctggat ccgtatcccg aaatacttca actccatctc cctgaacaac 2880
   gaatacacca tcatcaactg catggaaaac aactccggct ggaaagtttc cctgaactac 2940
   ggcgaaatca tctggaccct gcaggacacc caggaaatca aacagcgtgt tgtttttaaa 3000
   tactcccaga tgatcaacat ctccgactac atcaaccgtt ggatcttcgt taccatcacc 3060
   aacaaccgtc tgaacaactc caagatctac atcaacggcc gtctgatcga ccagaaaccg 3120
   atctccaacc tgggcaacat ccacgcttcc aacaacatca tgttcaaact ggacggctgc 3180
   cgtgacaccc accgttacat ctggatcaaa tacttcaacc tgttcgacaa agaactgaac 3240
   gagaaggaaa tcaaagacct gtacgacaac cagtccaact ccggcatcct gaaagacttc 3300
   tggggcgact acctgcagta tgacaaaccg tactacatgc tgaacctgta cgacccgaac 3360
   aaatacgttg acgttaacaa cgttggcatc cgtggctaca tgtacctgaa aggcccgcgt 3420
   ggctccgtta tgaccaccaa catctacctg aactcctccc tgtaccgtgg caccaaattc 3480
   atcatcaaga agtacgcttc cggcaacaaa gacaacatcg ttcgtaacaa cgaccgtgtt 3540
   tacatcaacg ttgtagttaa gaacaaagaa taccgtctgg ctaccaacgc ttcccaggct 3600
   ggcgttgaga agatcctgtc cgctctggaa atcccggacg taggcaacct gtcccaggtt 3660
   gtagttatga aatccaagaa cgaccagggc atcaccaaca aatgcaaaat gaacctgcag 3720
   gacaacaacg gcaacgacat cggcttcatc ggcttccacc agttcaacaa catcgctaaa 3780
   ctggttgctt ccaactggta caaccgtcag atcgaacgtt cctcccgtac cctgggctgc 3840
   tcctgggaat tcatcccggt tgacgacggc tggggcgaac gtccgctgta a          3891
<210> 125
   <211> 3891
   <212> DNA
   <213> Artificial Sequence
<220>
   <223> BoNT/A, E. coli-modified 4-3
<400> 125
   atgccgttcg ttaacaaaca gttcaactac aaagacccgg ttaacggcgt tgacatcgct 60
   tacatcaaaa tcccgaacgc tggccagatg cagccggtta aagcattcaa gattcacaac 120
   aaaatctggg taatcccgga acgtgacacc ttcaccaacc cggaagaagg cgacctgaac 180
   ccgccgccgg aagctaaaca ggttccggtt tcctactacg actccaccta cctgtccacc 240
   gacaacgaga aggacaacta cctgaaaggc gttaccaaac tgttcgaacg tatctactcc 300
   accgacctgg gccgtatgct gctgacctcc atcgttcgtg gcatcccgtt ctggggcggc 360
   tccaccatcg acaccgaact gaaagttatc gacaccaact gcatcaacgt tatccagccg 420
   gacggctcct accgttccga agaactgaac ctggttatca tcggcccgtc cgctgacatc 480
   atccagttcg aatgcaaatc cttcggccac gaagttctga acctgacccg taacggctac 540
   ggctccaccc agtacatccg tttctccccg gacttcacct tcggcttcga agaatccctg 600
   gaagttgaca ccaacccgct gctgggcgct ggcaaattcg ctaccgaccc ggctgttacc 660
   ctggctcacg aactgatcca cgctggccac cgtctgtacg gcatcgctat caacccgaac 720
   cgtgttttca aagttaacac caacgcttac tacgaaatgt ccggcctgga agtttccttc 780
   gaagaactgc gtaccttcgg cggccacgac gctaaattca tcgactccct gcaagagaac 840
   gaattccgtc tgtactatta caacaaattc aaagacatcg cttccaccct gaacaaagct 900
   aaatccatcg ttggcaccac cgcttccctg caatacatga agaacgtttt caaagagaag 960
   tacctgctgt ccgaagacac ctccggcaaa ttctccgttg acaaactgaa attcgacaaa 1020
   ctgtacaaga tgctgaccga aatctacacc gaagacaact tcgttaaatt ctttaaagtt 1080
   ctgaaccgta aaacctacct gaacttcgac aaagctgttt tcaagattaa catcgttccg 1140
   aaagttaact acaccatcta cgacggcttc aacctgcgta acaccaacct ggctgctaac 1200
   ttcaacggcc agaacaccga aatcaacaac atgaacttca ccaaactgaa gaacttcacc 1260
   ggtctgttcg aattctacaa actgctgtgc gtacgtggca tcatcacctc caaaaccaaa 1320
   tccctggaca aaggctacaa caaagctctg aacgacctgt gcatcaaagt taacaactgg 1380
   gacctgttct tctccccgtc cgaagacaac ttcaccaacg acctgaacaa aggcgaagaa 1440
   atcacctccg acaccaacat cgaagctgct gaagagaaca tctccctgga cctgatccag 1500
   cagtactacc tgaccttcaa cttcgacaac gaaccggaga acatctccat cgagaacctg 1560
   tcctccgaca tcatcggcca gctggaactg atgccgaaca tcgaacgttt cccgaacggc 1620
   aagaagtatg aactggacaa atacaccatg ttccactacc tgcgtgctca ggaattcgaa 1680
   cacggcaaat cccgtatcgc tctgaccaac tccgttaacg aagctctgct gaacccgtcc 1740
   cgtgtttaca ccttcttctc ctccgactac gttaagaagg ttaacaaagc taccgaagct 1800
   gctatgttcc tgggctgggt tgaacagctg gtttacgact tcaccgacga aacctccgaa 1860
   gtttccacca ccgacaagat tgctgacatc accatcatta tcccgtacat cggtccggct 1920
   ctgaacatcg gcaacatgct gtataaagac gacttcgttg gcgctctgat cttctccggc 1980
   gctgttatcc tgctggaatt catcccggaa atcgctatcc cggttctggg caccttcgct 2040
   ctggtttcct acatcgctaa caaagttctg accgttcaga ccatcgacaa cgctctgtcc 2100
   aaacgtaacg agaagtggga cgaagtttac aaatacatcg ttaccaactg gctggctaaa 2160
   gttaacaccc agatcgacct gatccgtaag aagatgaaag aagctctgga gaaccaggct 2220
   gaagctacca aagctatcat caactaccag tacaaccagt acaccgaaga ggaaaagaac 2280
   aacatcaact tcaacatcga cgacctgtcc tccaaactga acgaatccat caacaaagct 2340
   atgatcaaca tcaacaaatt cctgaaccag tgctccgttt cctacctgat gaactccatg 2400
   atcccgtatg gcgttaaacg tctggaagac ttcgacgcgt ccctgaaaga cgctctgctg 2460
   aaatacatct acgacaaccg cggcaccctg atcggccagg ttgaccgtct gaaagacaaa 2520
   gttaacaaca ccctgtccac cgacatcccg ttccagctgt ccaaatacgt agacaaccag 2580
   cgtctgctgt ccaccttcac cgaatacatc aagaacatca tcaacacctc catcctgaac 2640
   ctgcgttacg aatccaacca cctgatcgac ctgtcccgtt acgctagcaa aatcaacatc 2700
   ggctccaaag ttaacttcga cccgatcgac aagaaccaga tccagctgtt caacctggaa 2760
   tcctccaaaa tcgaagttat cctgaagaac gcaattgttt acaactccat gtacgagaac 2820
   ttctccacct ccttctggat ccgtatcccg aaatacttca actccatctc cctgaacaac 2880
   gaatacacca tcatcaactg catggagaac aactccggct ggaaagtttc cctgaactac 2940
   ggcgaaatca tctggaccct gcaggacacc caggaaatca aacagcgtgt tgttttcaaa 3000
   tactcccaga tgatcaacat ctccgactac atcaaccgtt ggatcttcgt taccatcacc 3060
   aacaaccgtc tgaacaactc caaaatctac atcaacggcc gtctgatcga ccagaaaccg 3120
   atctccaacc tgggcaacat ccacgcttcc aacaacatca tgttcaaact ggacggctgc 3180
   cgtgacaccc accgttacat ctggatcaaa tacttcaacc tgttcgacaa agaactgaac 3240
   gagaaggaaa tcaaagacct gtacgacaac cagtccaact ccggcatcct gaaagacttc 3300
   tggggcgact acctgcaata tgacaaaccg tactacatgc tgaacctgta cgacccgaac 3360
   aaatacgttg acgttaacaa cgttggcatc cgtggctata tgtacctgaa aggcccgcgt 3420
   ggctccgtta tgaccaccaa catctacctg aactcctccc tgtaccgtgg caccaaattc 3480
   atcatcaaga agtacgcttc cggcaacaaa gacaacatcg ttcgtaacaa cgaccgtgtt 3540
   tacatcaacg ttgtagttaa gaacaaagaa taccgtctgg ctaccaacgc ttcccaggct 3600
   ggcgttgaga agattctgtc cgctctggaa atcccggacg ttggcaacct gagccaagtt 3660
   gtagttatga aatccaagaa cgaccagggc atcaccaaca agtgcaagat gaacctgcaa 3720
   gacaacaacg gcaacgacat cggcttcatc ggcttccacc agttcaacaa catcgctaaa 3780
   ctggttgctt ccaactggta caaccgtcag atcgaacgtt cctcccgtac cctgggctgc 3840
   tcctgggaat tcatcccggt tgacgacggc tggggcgaac gtccgctgta a          3891